# EUROPEAN PATENT SPECIFICATION

(11) **EP 2 190 822 B1**
(45) Date of publication and mention of the grant of the patent: **31.12.2014**
(21) Application number: 08834057.5
(22) Date of filing: 24.09.2008
(51) Int. Cl.: C07D 231/18, C07D 233/24, C07D 277/36, C07D 307/64, C07D 333/34, C07D 401/04, C07D 405/12, C07D 409/04, C07D 409/12, C07D 417/04, C07D 417/14, A61K 31/381, A61K 31/41, A61K 31/44, A61P 1/00

(54) **5-MEMBERED HETEROCYCLIC COMPOUNDS AS PROTON PUMP INHIBITORS**
5-GLIEDRIGE HETEROCYCLISCHE VERBINDUNGEN ALS PROTONENPUMPENHEMMER
COMPOSÉS HÉTÉROCYCLIQUES À 5 CHAÎNONS COMME INHIBITEURS DE POMPE À PROTONS

(30) Priority: 28.09.2007 JP 2007256274; 27.08.2008 JP 2008218076
(43) Date of publication of application: 02.06.2010
(73) Proprietor: Takeda Pharmaceutical Company Limited, Osaka 541-0045 (JP)
(72) Inventor: NISHIDA, Haruyuki, Fujisawa Kanagawa 251-8555 (JP); ARIKAWA, Yasuyoshi, Fujisawa Kanagawa 251-8555 (JP); HIRASE, Keizo, Fujisawa Kanagawa 251-8555 (JP)
(74) Representative: Jones, Nicholas Andrew
(86) International application number: PCT/JP2008/067784
(87) International publication number: WO 2009/041705

(56) References cited:
- EP-A- 1 803 709
- WO-A-2007/026916
- WO-A-2007/114338

## Description

### Technical Field of the Invention

The present invention relates to 5-membered heterocycle compounds having an acid secretion suppressive activity.

### Background of the Invention

Proton pump inhibitors represented by omeprazole, which suppress secretion of gastric acid for the treatment of peptic ulcer, reflux esophagitis, have been widely used in clinical situations. However, the existing proton pump inhibitors are associated with problems in terms of effect and side effects. To be specific, since the existing proton pump inhibitors are unstable under acidic conditions, they are often formulated as enteric preparations, in which case several hours are required before expression of the effect, and about 5 days to exhibit maximum efficacy by consecutive administration. In addition, since the existing proton pump inhibitors show dispersion of treatment effects due to metabolic enzyme polymorphism and drug interaction with pharmaceutical agents such as diazepam, an improvement has been desired.

As pyrrole compounds having a proton pump inhibitory action, WO 2006/036024 describes a compound represented by the formula: 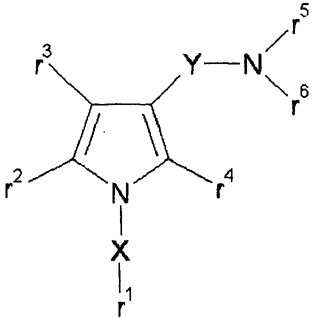 wherein X and Y are the same or different and each is a bond or a spacer having 1 to 20 atoms in the main chain, r¹ is an optionally substituted hydrocarbon group or an optionally substituted heterocyclic group, r², r³ and r⁴ are the same or different and each is a hydrogen atom, an optionally substituted hydrocarbon group, an optionally substituted thienyl group, an optionally substituted benzo[b]thienyl group, an optionally substituted furyl group, an optionally substituted pyridyl group, an optionally substituted pyrazolyl group, an optionally substituted pyrimidinyl group, an acyl group, a halogen atom, a cyano group or a nitro group, and r⁵ and r⁶ are the same or different and each is a hydrogen atom or an optionally substituted hydrocarbon group.

As pyrrole compounds having a proton pump inhibitory action, WO 2007/026916 describes a compound represented by the formula: 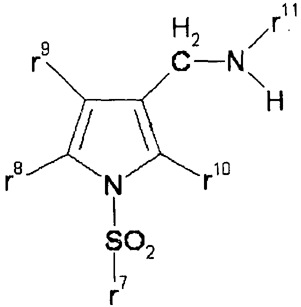 wherein r⁷ is an monocyclic nitrogen-containing heterocyclic group optionally condensed with a benzene ring or heterocycle, which optionally has substituent(s), r⁸ is an optionally substituted C₆₋₁₄ aryl group, an optionally substituted thienyl group or an optionally substituted pyridyl group, r⁹ and r¹⁰ are the same or different and each is a hydrogen atom, or one of r⁹ and r¹⁰ is a hydrogen atom, and the other is an optionally substituted lower alkyl group, an acyl group, a halogen atom, a cyano group or a nitro group, and r¹¹ is an alkyl group.

As a therapeutic drug for neoplastic diseases or autoimmune diseases, WO 2004/103968 describes a compound represented by the formula: 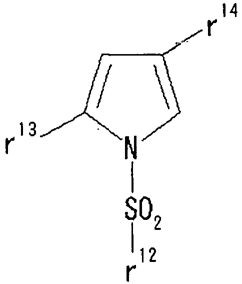 wherein r¹² is aryl, aralkyl, heteroaryl, r¹³ is aryl, heteroaryl, and r¹⁴ is aryl, heteroaryl, optionally substituted aminomethyl.

As compounds having a proton pump inhibitory action, WO 2007/114338 describes a compound represented by the formula: 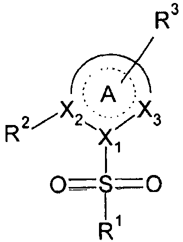 wherein
ring A is a saturated or unsaturated 5-membered ring optionally containing, as a ring-constituting atom besides carbon atoms, 1 to 4 heteroatoms selected from a nitrogen atom, an oxygen atom and a sulfur atom, and the ring-constituting atoms X₁ and X₂ are the same or different and each is a carbon atom or a nitrogen atom,
R¹ is an optionally substituted aryl group or an optionally substituted heteroaryl group,
R² is an optionally substituted alkyl group, an optionally substituted aryl group or an optionally substituted heteroaryl group, and
R³ is a substituent on the ring-constituting atom other than X₁, X₂ and X₃, which optionally has substituent(s) selected from a lower alkyl group, a halogen atom, a cyano group and oxo.

### Disclosure of the Invention

A pharmaceutical agent that effectively suppresses gastric acid secretion as known proton pump inhibitors, which is improved in instability under acidic conditions, dispersion of effects due to metabolic enzyme polymorphism and drug interaction, which are problems of known proton pump inhibitors, is expected to show more superior treatment effect on peptic ulcer, reflux esophagitis. As the situation stands, however, a proton pump inhibitor capable of sufficiently satisfying these requirements has not been found. It is therefore an object of the present invention to provide a compound having a superior acid secretion suppressive effect (particularly, proton pump inhibitory effect), which has been improved in these problems.

The present inventors have conducted various studies and found that a compound represented by the formula (I): 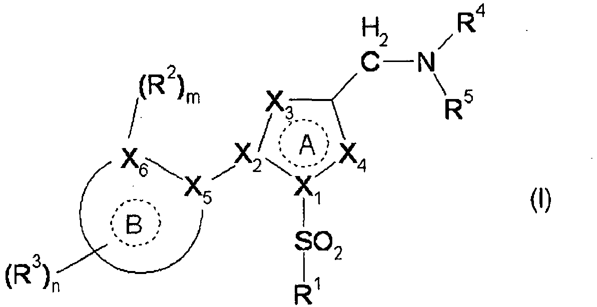 wherein
each symbol is as defined herein,
or a salt thereof [hereinafter to be sometimes abbreviated as compound (I)] unexpectedly has a very strong proton pump inhibitory effect, and is fully satisfactory as a pharmaceutical agent, which resulted in the completion of the present invention.

Accordingly, the present invention relates to
[1] A compound represented by the formula (I): 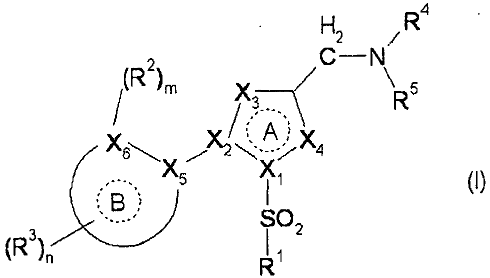 which is selected from
   (1) a compound represented by the formula (Ia-20): 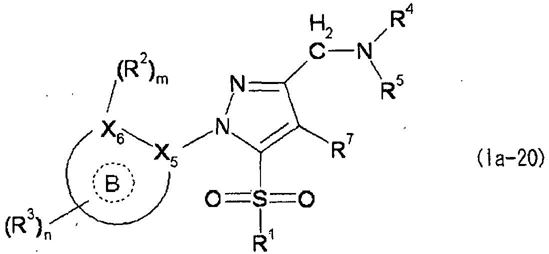 wherein
      ring B is a phenyl group, a cyclopentyl group, a cyclohexyl group, a pyrrolyl group, a pyrazolyl group, a thiazolyl group, an imidazolyl group, an oxazolyl group, a thienyl group, a furyl group, a pyridyl group, a pyrrolidinyl group or a piperidyl group;
      X₅ is a carbon atom or a nitrogen atom, and X₆ is a carbon atom, a nitrogen atom, an oxygen atom or a sulfur atom; R¹ is a phenyl group, a cyclopentyl group, a cyclohexyl group,
      a pyrrolyl group, a pyrazolyl group, a thiazolyl group, an imidazolyl group, an oxazolyl group, a thienyl group, a furyl group, a pyridyl group, a pyrrolidinyl group or a piperidyl group, each of which is optionally substituted by 1 to 3 substituents selected from (i) a halogen atom, (ii) hydroxy, (iii) cyano, (iv) C₁₋₆ alkyl optionally substituted by 1 to 5 halogen atoms, (v) C₁₋₆ alkoxy optionally substituted by 1 to 5 halogen atoms, (vi) amino optionally mono- or di-substituted by C₁₋₆ alkyl, (vii) oxo, (viii) carbamoyl, (ix) mono-C₁₋₆ alkyl-carbamoyl, (x) di-C₁₋₆ alkyl-carbamoyl, (xi) C₁₋₆ alkylsulfonyl and (xii) C₁₋₆ alkyl-carbonylamino;
      R² is a halogen atom, a cyano group, a trifluoromethyl group, a methyl group, an ethyl group, a methoxy group or an ethoxy group;
      R³ is a halogen atom, a cyano group, a trifluoromethyl group, a methyl group, an ethyl group or a methoxy group; R⁴ and R⁵ are the same or different and each is a hydrogen atom or a methyl group;
      R⁷ is a hydrogen atom, a halogen atom, a methyl group or a cyano group;
      m is 0 or 1, provided that when ring B is a phenyl group, a pyrrolyl group, a pyrazolyl group, a thiazolyl group, an imidazolyl group, an oxazolyl group, a thienyl group, a furyl group or a pyridyl group, then m should be 1; and n is an integer of 0 to 3;
   (2) a compound represented by the formula (Ia-1): 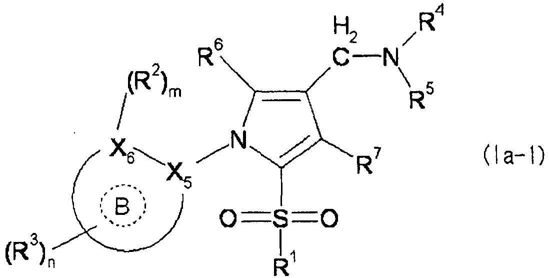 wherein
      ring B is a phenyl group, a cyclopentyl group, a cyclohexyl group, a pyrrolyl group, a pyrazolyl group, a thiazolyl group, an imidazolyl group, an oxazolyl group, a thienyl group, a furyl group, a pyridyl group, a pyrrolidinyl group or a piperidyl group;
      X₅ is a carbon atom or a nitrogen atom, and X₆ is a carbon atom, a nitrogen atom, an oxygen atom or a sulfur atom; R¹ is a phenyl group, a cyclopentyl group, a cyclohexyl group,
      a pyrrolyl group, a pyrazolyl group, a thiazolyl group, an imidazolyl group, an oxazolyl group, a thienyl group, a furyl group, a pyridyl group, a pyrrolidinyl group or a piperidyl group, each of which is optionally substituted by 1 to 3 substituents selected from (i) a halogen atom, (ii) hydroxy, (iii) cyano, (iv) C₁₋₆ alkyl optionally substituted by 1 to 5 halogen atoms, (v) C₁₋₆ alkoxy optionally substituted by 1 to 5 halogen atoms, (vi) amino optionally mono- or di-substituted by C₁₋₆ alkyl, (vii) oxo, (viii) carbamoyl, (ix) mono-C₁₋₆ alkyl-carbamoyl, (x) di-C₁₋₆ alkyl-carbamoyl, (xi) C₁₋₆ alkylsulfonyl and (xii) C₁₋₆ alkyl-carbonylamino;
      R² is a halogen atom, a cyano group, a trifluoromethyl group, a methyl group, an ethyl group, a methoxy group or an ethoxy group;
      R³ is a halogen atom, a cyano group, a trifluoromethyl group, a methyl group, an ethyl group or a methoxy group; R⁴ and R⁵ are the same or different and each is a hydrogen atom or a methyl group;
      R⁶ and R⁷ are the same or different and each is a hydrogen atom, a halogen atom, a methyl group or a cyano group; m is 0 or 1, provided that when ring B is a phenyl group, a pyrrolyl group, a pyrazolyl group, a thiazolyl group, an imidazolyl group, an oxazolyl group, a thienyl group, a furyl group or a pyridyl group, then m should be 1; and n is an integer of 0 to 3;
   (3) a compound represented by the formula (Ia-9): 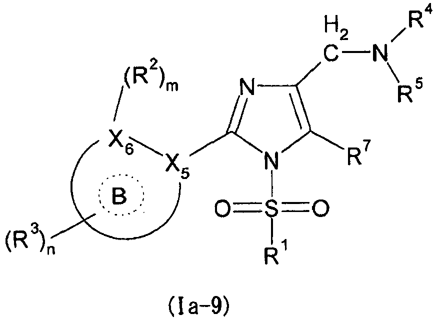 wherein
      ring B is a phenyl group, a cyclopentyl group, a cyclohexyl group, a pyrrolyl group, a pyrazolyl group, a thiazolyl group, an imidazolyl group, an oxazolyl group, a thienyl group, a furyl group, a pyridyl group, a pyrrolidinyl group or a piperidyl group;
      X₅ is a carbon atom or a nitrogen atom, and X₆ is a carbon atom, a nitrogen atom, an oxygen atom or a sulfur atom; R¹ is a phenyl group, a cyclopentyl group, a cyclohexyl group,
      a pyrrolyl group, a pyrazolyl group, a thiazolyl group, an imidazolyl group, an oxazolyl group, a thienyl group, a furyl group, a pyridyl group, a pyrrolidinyl group or a piperidyl group, each of which is optionally substituted by 1 to 3 substituents selected from (i) a halogen atom, (ii) hydroxy, (iii) cyano, (iv) C₁₋₆ alkyl optionally substituted by 1 to 5 halogen atoms, (v) C₁₋₆ alkoxy optionally substituted by 1 to 5 halogen atoms, (vi) amino optionally mono- or di-substituted by C₁₋₆ alkyl, (vii) oxo, (viii) carbamoyl, (ix) mono-C₁₋₆ alkyl-carbamoyl, (x) di-C₁₋₆ alkyl-carbamoyl, (xi) C₁₋₆ alkylsulfonyl and (xii) C₁₋₆ alkyl-carbonylamino;
      R² is a halogen atom, a cyano group, a trifluoromethyl group, a methyl group, an ethyl group, a methoxy group or an ethoxy group;
      R³ is a halogen atom, a cyano group, a trifluoromethyl group, a methyl group, an ethyl group or a methoxy group; R⁴ and R⁵ are the same or different and each is a hydrogen atom or a methyl group;
      R⁷ is a hydrogen atom, a halogen atom, a methyl group or a cyano group;
      m is 0 or 1, provided that when ring B is a phenyl group, a pyrrolyl group, a pyrazolyl group, a thiazolyl group, an imidazolyl group, an oxazolyl group, a thienyl group, a furyl group or a pyridyl group, then m should be 1; and n is an integer of 0 to 3;
   (4) a compound represented by the formula (Ia-30): 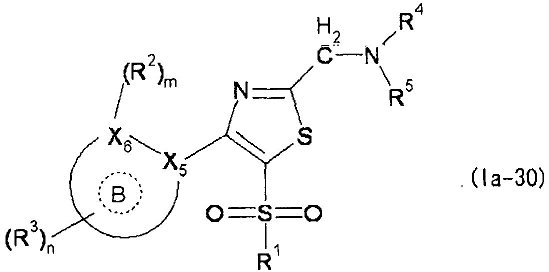 wherein
      ring B is a phenyl group, a cyclopentyl group, a cyclohexyl group, a pyrrolyl group, a pyrazolyl group, a thiazolyl group, an imidazolyl group, an oxazolyl group, a thienyl group, a furyl group, a pyridyl group, a pyrrolidinyl group or a piperidyl group;
      X₅ is a carbon atom or a nitrogen atom, and X₆ is a carbon atom, a nitrogen atom, an oxygen atom or a sulfur atom; R¹ is a phenyl group, a cyclopentyl group, a cyclohexyl group,
      a pyrrolyl group, a pyrazolyl group, a thiazolyl group, an imidazolyl group, an oxazolyl group, a thienyl group, a furyl group, a pyridyl group, a pyrrolidinyl group or a piperidyl group, each of which is optionally substituted by 1 to 3 substituents selected from (i) a halogen atom, (ii) hydroxy, (iii) cyano, (iv) C₁₋₆ alkyl optionally substituted by 1 to 5 substituents selected from a halogen atom and a non-aromatic heterocyclic group, (v) C₁₋₆ alkoxy optionally substituted by 1 to 5 halogen atoms, (vi) amino optionally mono- or di-substituted by Alkyl, (vii) oxo, (viii) carbamoyl, (ix) mono-C₁₋₆ alkyl-carbamoyl, (x) di-C₁₋₆ alkyl-carbamoyl, (xi) C₁₋₆ alkylsulfonyl, (xii) C₁₋₆ alkyl-carbonylamino, (xiii) a non-aromatic heterocyclic group optionally substituted by oxo and (xiv) a 5- or 10-membered heterocyclyl-carbonyl containing, besides carbon atoms, 1 or 2 kinds of 1 to 4 heteroatoms selected from a nitrogen atom, a sulfur atom and an oxygen atom;
      R² is a halogen atom, a cyano group, a trifluoromethyl group, a methyl group, an ethyl group, a methoxy group or an ethoxy group;
      R³ is a halogen atom, a cyano group, a trifluoromethyl group, a methyl group, an ethyl group or a methoxy group; R⁴ and R⁵ are the same or different and each is a hydrogen atom or a methyl group;
      m is 0 or 1, provided that when ring B is a phenyl group, a pyrrolyl group, a pyrazolyl group, a thiazolyl group, an imidazolyl group, an oxazolyl group, a thienyl group, a furyl group or a pyridyl group, then m should be 1; and n is an integer of 0 to 3;
   (5) a compound represented by the formula (Ia-31): 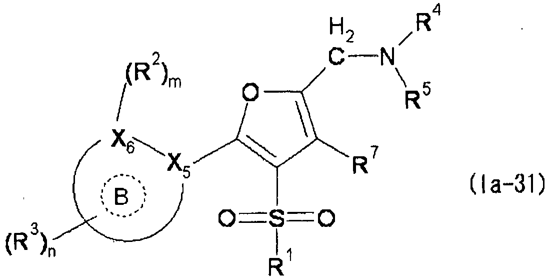 wherein
      ring B is a phenyl group, a cyclopentyl group, a cyclohexyl group, a pyrrolyl group, a pyrazolyl group, a thiazolyl group, an imidazolyl group, an oxazolyl group, a thienyl group, a furyl group, a pyridyl group, a pyrrolidinyl group or a piperidyl group;
      X₅ is a carbon atom or a nitrogen atom, and X₆ is a carbon atom, a nitrogen atom, an oxygen atom or a sulfur atom; R¹ is a phenyl group, a cyclopentyl group, a cyclohexyl group,
      a pyrrolyl group, a pyrazolyl group, a thiazolyl group, an imidazolyl group, an oxazolyl group, a thienyl group, a furyl group, a pyridyl group, a pyrrolidinyl group or a piperidyl group, each of which is optionally substituted by 1 to 3 substituents selected from (i) a halogen atom, (ii) hydroxy, (iii) cyano, (iv) C₁₋₆ alkyl optionally substituted by 1 to 5 halogen atoms, (v) C₁₋₆ alkoxy optionally substituted by 1 to 5 halogen atoms, (vi) amino optionally mono- or di-substituted by C₁₋₆ alkyl, (vii) oxo, (viii) carbamoyl, (ix) mono-C₁₋₆ alkyl-carbamoyl, (x) di-C₁₋₆ alkyl-carbamoyl, (xi) C₁₋₆ alkylsulfonyl and (xii) C₁₋₆ alkyl-carbonylamino;
      R² is a halogen atom, a cyano group, a trifluoromethyl group, a methyl group, an ethyl group, a methoxy group or an ethoxy group;
      R³ is a halogen atom, a cyano group, a trifluoromethyl group, a methyl group, an ethyl group or a methoxy group; R⁴ and R⁵ are the same or different and each is a hydrogen atom or a methyl group;
      R⁷ is a hydrogen atom, a halogen atom, a methyl group or a cyano group;
      m is 0 or 1, provided that when ring B is a phenyl group, a pyrrolyl group, a pyrazolyl group, a thiazolyl group, an imidazolyl group, an oxazolyl group, a thienyl group, a furyl group or a pyridyl group, then m should be 1; and n is an integer of 0 to 3;
   (6) a compound represented by the formula (Ia-33): 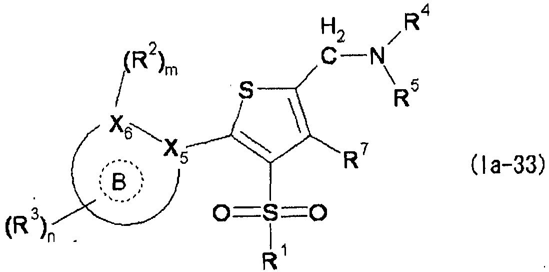 wherein
      ring B is a phenyl group, a cyclopentyl group, a cyclohexyl group, a pyrrolyl group, a pyrazolyl group, a thiazolyl group, an imidazolyl group, an oxazolyl group, a thienyl group, a furyl group, a pyridyl group, a pyrrolidinyl group or a piperidyl group;
      X₅ is a carbon atom or a nitrogen atom, and X₆ is a carbon atom, a nitrogen atom, an oxygen atom or a sulfur atom;
      R¹ is a phenyl group, a cyclopentyl group, a cyclohexyl group, a pyrrolyl group, a pyrazolyl group, a thiazolyl group, an imidazolyl group, an oxazolyl group, a thienyl group, a furyl group, a pyridyl group, a pyrrolidinyl group or a piperidyl group, each of which is optionally substituted by 1 to 3 substituents selected from (i) a halogen atom, (ii) hydroxy, (iii) cyano, (iv) C₁₋₆ alkyl optionally substituted by 1 to 5 halogen atoms, (v) C₁₋₆ alkoxy optionally substituted by 1 to 5 halogen atoms, (vi) amino optionally mono- or di-substituted by C₁₋₆ alkyl, (vii) oxo, (viii) carbamoyl, (ix) mono-C₁₋₆ alkyl-carbamoyl, (x) di-C₁₋₆ alkyl-carbamoyl, (xi) C₁₋₆ alkylsulfonyl and (xii) C₁₋₆ alkyl-carbonylamino;
      R² is a halogen atom, a cyano group, a trifluoromethyl group, a methyl group, an ethyl group, a methoxy group or an ethoxy group;
      R³ is a halogen atom, a cyano group, a trifluoromethyl group, a methyl group, an ethyl group or a methoxy group; R⁴ and R⁵ are the same or different and each is a hydrogen atom or a methyl group;
      R⁷ is a hydrogen atom, a halogen atom, a methyl group or a cyano group;
      m is 0 or 1, provided that when ring B is a phenyl group, a pyrrolyl group, a pyrazolyl group, a thiazolyl group, an imidazolyl group, an oxazolyl group, a thienyl group, a furyl group or a pyridyl group, then m should be 1; and n is an integer of 0 to 3; and
   (7) a compound represented by the formula (Ia-34): 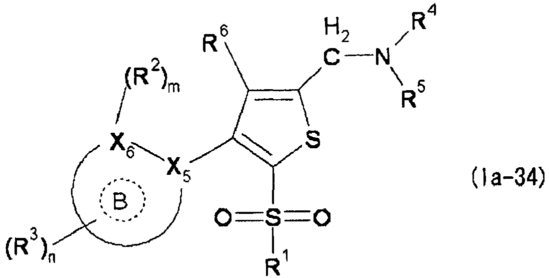 wherein
      ring B is a phenyl group, a cyclopentyl group, a cyclohexyl group, a pyrrolyl group, a pyrazolyl group, a thiazolyl group, an imidazolyl group, an oxazolyl group, a thienyl group, a furyl group, a pyridyl group, a pyrrolidinyl group or a piperidyl group;
      X₅ is a carbon atom or a nitrogen atom, and X₆ is a carbon atom, a nitrogen atom, an oxygen atom or a sulfur atom; R¹ is a phenyl group, a cyclopentyl group, a cyclohexyl group,
      a pyrrolyl group, a pyrazolyl group, a thiazolyl group, an imidazolyl group, an oxazolyl group, a thienyl group, a furyl group, a pyridyl group, a pyrrolidinyl group or a piperidyl group, each of which is optionally substituted by 1 to 3 substituents selected from (i) a halogen atom, (ii) hydroxy, (iii) cyano, (iv) C₁₋₆ alkyl optionally substituted by 1 to 5 substituents selected from a halogen atom and hydroxy, (v) C₁₋₆ alkoxy optionally substituted by 1 to 5 halogen atoms, (vi) amino optionally mono- or di-substituted by C₁₋₆ alkyl, (vii) oxo, (viii) carbamoyl, (ix) mono-C₁₋₆ alkyl-carbamoyl, (x) di-C₁₋₆ alkyl-carbamoyl, (xi) C₁₋₆ alkylsulfonyl and (xii) C₁₋₆ alkyl-carbonylamino;
      R² is a halogen atom, a cyano group, a trifluoromethyl group, a methyl group, an ethyl group, a methoxy group or an ethoxy group;
      R³ is a halogen atom, a cyano group, a trifluoromethyl group, a methyl group, an ethyl group or a methoxy group; R⁴ and R⁵ are the same or different and each is a hydrogen atom or a methyl group;
      R⁶ is a hydrogen atom, a halogen atom, a methyl group or a cyano group;
      m is 0 or 1, provided that when ring B is a phenyl group, a pyrrolyl group, a pyrazolyl group, a thiazolyl group, an imidazolyl group, an oxazolyl group, a thienyl group, a furyl group or a pyridyl group, then m should be 1; and n is an integer of 0 to 3;
      or a salt thereof; ,
[2] The compound of the above-mentioned [1], wherein R⁶ and R⁷ are the same or different and each is a hydrogen atom or a halogen atom;
[3] The compound of the above-mentioned [1], wherein R² is a halogen atom, a cyano group, a trifluoromethyl group, a methyl group or an ethyl group;
[4] The compound or salt of the above-mentioned [1], which is 1-[4-(2-fluoropyridin-3-yl)-5-(pyridin-3-ylsulfonyl)thiophen-2-yl]-N-methylmethanamine or a salt thereof;
[5] The compound or salt of the above-mentioned [1], which is 1-[5-(2-fluoropyridin-3-yl)-4-(pyridin-3-ylsulfonyl)thiophen-2-yl]-N-methylmethanamine or a salt thereof;
[6] The compound or salt of the above-mentioned [1], which is 1-[1-(2-fluoropyridin-3-yl)-5-(phenylsulfonyl)-1H-pyrazol-3-yl]-N-methylmethanamine or a salt thereof;
[7] The compound or salt of the above-mentioned [1], which is 1-[1-(2-fluorophenyl)-5-(pyridin-3-ylsulfonyl)-1H-pyrazol-3-yl]-N-methylmethanamine or a salt thereof;
[8] The compound or salt of the above-mentioned [1], which is 1-[1-(2-chlorophenyl)-5-(pyridin-3-ylsulfonyl)-1H-pyrazol-3-yl]-N-methylmethanamine or a salt thereof;
[9] The compound or salt of the above-mentioned [1], which is 1-{1-(2-chlorophenyl)-5-[(6-methylpyridin-3-yl)sulfonyl]-1H-pyrazol-3-yl}-N-methylmethanamine or a salt thereof;
[10] The compound or salt of the above-mentioned [1], which is 1-[1-(2,3-difluorophenyl)-5-(pyridin-3-ylsulfonyl)-1H-pyrazol-3-yl]-N-methylmethanamine or a salt thereof;
[11] The compound or salt of the above-mentioned [1], which is 1-{1-(2,3-difluorophenyl)-5-[(6-methylpyridin-3-yl)sulfonyl]-1H-pyrazol-3-yl}-N-methylmethanamine or a salt thereof;
[12] A pharmaceutical agent comprising the compound or salt of any of the above-mentioned [1] to [11] ;
[13] The pharmaceutical agent of the above-mentioned [12], for use in the prophylaxis or treatment of peptic ulcer, Zollinger-Ellison syndrome, gastritis, reflux esophagitis, symptomatic gastroesophageal reflux disease (symptomatic GERD), Barrettesophagus, functional dyspepsia, gastric cancer, stomach MALT lymphoma, or ulcer caused by non-steroidal anti-inflammatory agent, gastric hyperacidity or ulcer due to postoperative stress; or as an inhibitor of upper gastrointestinal hemorrhage due to peptic ulcer, acute stress ulcer, hemorrhagic gastritis or invasive stress;
[14] Use of the compound or salt of any of the above-mentioned [1] to [11] for the production of an agent for the prophylaxis or treatment of peptic ulcer, Zollinger-Ellison syndrome, gastritis, reflux esophagitis, symptomatic gastroesophageal reflux disease (symptomatic GERD), Barrettesophagus, functional dyspepsia, gastric cancer, stomach MALT lymphoma, or ulcer caused by non-steroidal anti-inflammatory agent, gastric hyperacidity or ulcer due to postoperative stress; or for the inhibition of upper gastrointestinal hemorrhage due to peptic ulcer, acute stress ulcer, hemorrhagic gastritis or invasive stress.

### Effect of the Invention

Compound (I) of the present invention shows a superior proton pump inhibitory effect. Conventional proton pump inhibitors such as omeprazole, lansoprazole are converted to active forms in an acidic environment of stomach wall cells and form a covalent bond with a cysteine residue of H⁺/K⁺-ATPase, and irreversibly inhibit the enzyme activity. In contrast, compound (I) inhibits proton pump (H⁺/K⁺-ATPase) activity in a reversible and K⁺ antagonist-like inhibitory manner, and consequently suppresses acid secretion. Therefore, it is sometimes called a potassium-competitive acid blocker (P-CAB), or an acid pump antagonist (APA). Compound (I) rapidly expresses the action and shows the maximum efficacy from the initial administration. Furthermore, it characteristically shows less influence of metabolic polymorphism (variation between patients) and long duration of action. Accordingly, the present invention can provide a clinically useful agent for the prophylaxis or treatment of peptic ulcer (e.g., gastric ulcer, duodenal ulcer, anastomotic ulcer, ulcer caused by non-steroidal anti-inflammatory agent, ulcer due to postoperative stress etc.), Zollinger-Ellison syndrome, gastritis, erosive esophagitis, reflux esophagitis, symptomatic gastroesophageal reflux disease (Symptomatic GERD), Barrettesophagus, functional dyspepsia, gastric cancer, stomach MALT lymphoma or hyperacidity; or a suppressant of upper gastrointestinal hemorrhage due to peptic ulcer, acute stress ulcer, hemorrhagic gastritis or invasive stress. Since compound (I) shows low toxicity and is superior in water-solubility, in vivo kinetics and efficacy expression, it is useful as a pharmaceutical composition. Since compound (I) is stable even under acidic conditions, it can be administered orally as a conventional tablet without formulating into an enteric-coated preparation. This has an advantageous consequence that the preparation (tablet) can be made smaller, and can be easily swallowed by patients having difficulty in swallowing, particularly the elderly and children. In addition, since it is free of a sustained release effect afforded by enteric-coated preparations, a gastric acid secretion-suppressive action is expressed rapidly, and symptoms such as pain can be alleviated rapidly.

### [DETAILED DESCRIPTION OF THE INVENTION]

Examples of "C₁₋₆ alkyl" include methyl, ethyl, n-propyl, isopropyl, n-butyl, isobutyl, sec-butyl, tert-butyl, n-pentyl, sec-pentyl, isopentyl, neopentyl, n-hexyl, isohexyl etc.

Examples of the "halogen atom" include a fluorine atom, a chlorine atom, a bromine atom and an iodine atom.

Examples of "C₁₋₆ alkoxy optionally having 1 to 5 halogen atoms" include methoxy, ethoxy, propoxy, isopropoxy, butoxy, isobutoxy, sec-butoxy, pentyloxy, hexyloxy, fluoromethoxy etc.

Examples of "amino optionally mono- or di-substituted by C₁₋₆ alkyl," include methylamino, ethylamino, dimethylamino, diethylamino etc.

Examples of "mono-C₁₋₆ alkyl-carbamoyl" include methylcarbamoyl, ethylcarbamoyl etc.

Examples of "di-C₁₋₆ alkyl-carbamoyl" include dimethylcarbamoyl, diethylcarbamoyl, ethylmethylcarbamoyl etc.

Examples of "C₁₋₆ alkylsulfonyl" include methylsulfonyl, ethylsulfonyl etc.

Examples of "C₁₋₆ alkyl-carbonylamino" include acetylamino etc.

Examples of "C₁₋₆ alkyl optionally having 1 to 5 substituents selected from a halogen atom", "C₁₋₆ alkyl optionally having 1 to 5 substituents selected from a halogen atom and hydroxy", and "C₁₋₆ alkyl optionally having 1 to 5 substituents selected from a halogen atom and a non-aromatic heterocyclic group" include methyl, ethyl, n-propyl, isopropyl, n-butyl, isobutyl, sec-butyl, tert-butyl, n-pentyl, sec-pentyl, isopentyl, neopentyl, n-hexyl, isohexyl, hydroxylmethyl, 1-pyrrolidinylmethyl etc.

Examples of "5- or 10-membered heterocyclyl-carbonyl containing, besides carbon atoms, 1 or 2 kinds of 1 to 4 heteroatoms selected from a nitrogen atom, a sulfur atom and an oxygen atom" include 4-morpholinocarbonyl, 1-pyrrolidinylcarbonyl etc.

The partial structure of compound (I): 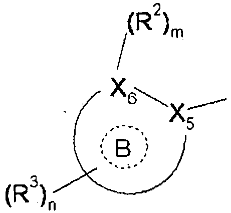 is preferably 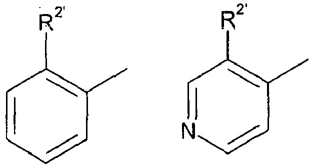 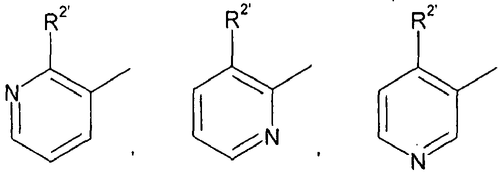 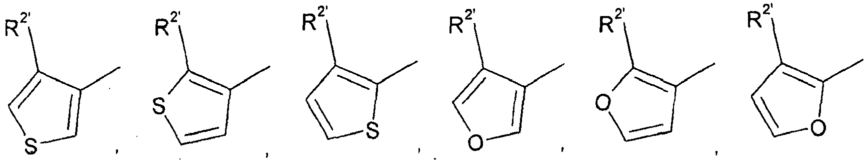 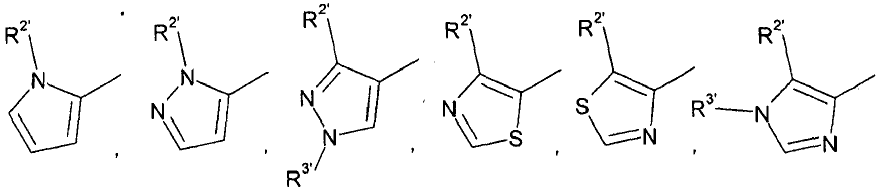 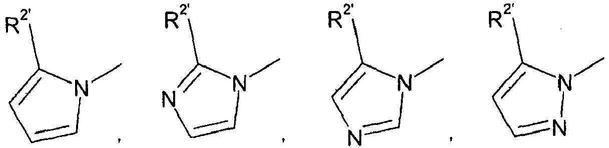 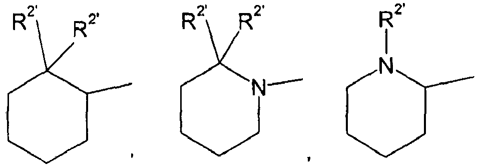 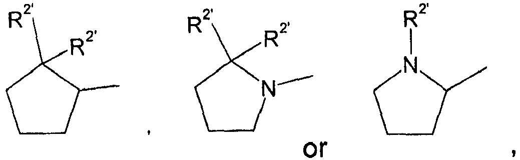 wherein R²_{,} is a hydrogen atom or R², and R³' is a hydrogen atom or R³, more preferably 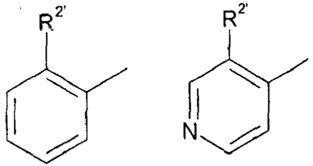 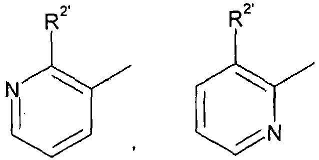 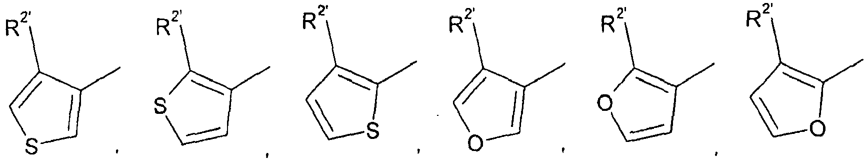 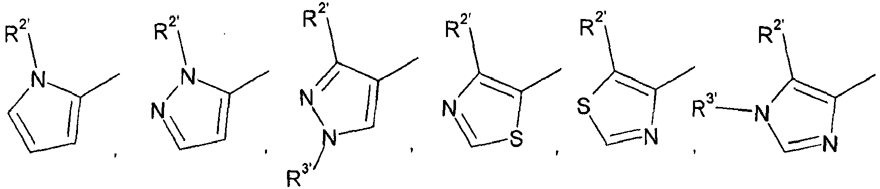 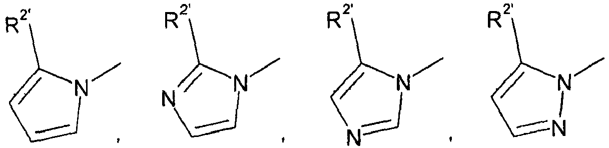 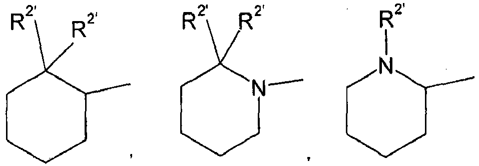 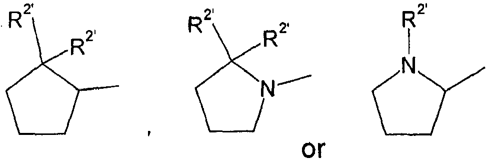 wherein R²' is a hydrogen atom or R², and R³' is a hydrogen atom or R³.

The partial structure of compound (I): 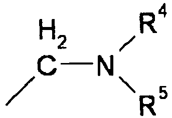 is preferably aminomethyl (-CH₂-NH₂), methylaminomethyl (-CH₂-NH(CH₃)) or dimethylaminomethyl (-CH₂-N(CH₃)₂), particularly preferably methylaminomethyl.

Preferable embodiments of compounds (Ia-1), (Ia-9), (Ia-20), (Ia-30), (Ia-31), (Ia-33) and (Ia-34) are shown in the following.
(1) Compound (Ia-1) 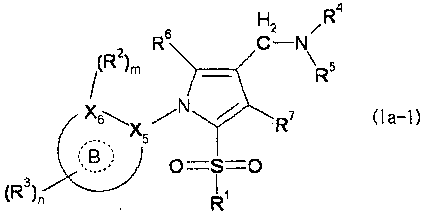 wherein
   ring B is a phenyl group, a cyclopentyl group, a cyclohexyl group, a pyrrolyl group (e.g., 1-, 2- or 3-pyrrolyl), a pyrazolyl group (e.g., 1-, 3-, 4- or 5-pyrazolyl), a thiazolyl group (e.g., 2-, 4- or 5-thiazolyl), an imidazolyl group (e.g., 1-, 2-, 4- or 5-imidazolyl), an oxazolyl group (e.g., 2-, 4-or 5-oxazolyl), a thienyl group (e.g., 2- or 3-thienyl), a furyl group (e.g., 2- or 3-furyl), a pyridyl group (e.g., 1-, 2-, 3- or 4-pyridyl), a pyrrolidinyl group (e.g., 1-, 2- or 3-pyrrolidinyl) or a piperidyl group (e.g., 1-, 2-, 3- or 4-piperidyl);
   R¹ is a phenyl group, a cyclopentyl group, a cyclohexyl group, a pyrrolyl group (e.g., 1-, 2- or 3-pyrrolyl), a pyrazolyl group (e.g., 1-, 3-, 4- or 5-pyrazolyl), a thiazolyl group (e.g., 2-, 4- or 5-thiazolyl), an imidazolyl group (e.g., 1-, 2-, 4- or 5-imidazolyl), an oxazolyl group (e.g., 2-, 4- or 5-oxazolyl), a thienyl group (e.g., 2- or 3-thienyl), a furyl group (e.g., 2- or 3-furyl), a pyridyl group (e.g., 1-, 2-, 3-or 4-pyridyl), a pyrrolidinyl group (e.g., 1-, 2- or 3-pyrrolidinyl) or a piperidyl group (e.g., 1-, 2-, 3- or 4-piperidyl), each of which is optionally substituted by 1 to 3 substituents selected from (i) a halogen atom, (ii) hydroxy, (iii) cyano, (iv) C₁₋₆ alkyl optionally substituted by 1 to 5 (preferably 1 to 3) halogen atoms, (v) C₁₋₆ alkoxy optionally substituted by 1 to 5 (preferably 1 to 3) halogen atoms, (vi) amino optionally mono- or di-substituted by C₁₋₆ alkyl, (vii) oxo, (viii) carbamoyl, (ix) mono-C₁₋₆ alkyl-carbamoyl, (x) di-C₁₋₆ alkyl-carbamoyl, (xi) C₁₋₆ alkylsulfonyl and (xii) C₁₋₆ alkyl-carbonylamino;
   R² is a halogen atom, a cyano group, a trifluoromethyl group, a methyl group, an ethyl group, a methoxy group or an ethoxy group, particularly preferably a halogen atom, a cyano group, a trifluoromethyl group, a methyl group or an ethyl group; R³ is a halogen atom, a cyano group, a trifluoromethyl group, a methyl group, an ethyl group or a methoxy group; R⁴ and R⁵ are the same or different and each is a hydrogen atom or a methyl group;
   R⁶ and R⁷ are the same or different and each is a hydrogen atom, a halogen atom, a methyl group or a cyano group; m is 0 or 1, provided that when ring B is a phenyl group, a pyrrolyl group, a pyrazolyl group, a thiazolyl group, an imidazolyl group, an oxazolyl group, a thienyl group, a furyl group or a pyridyl group, then m should be 1; and n is an integer of 0 to 3,
   or a salt thereof.
(2) Compound (Ia-9) 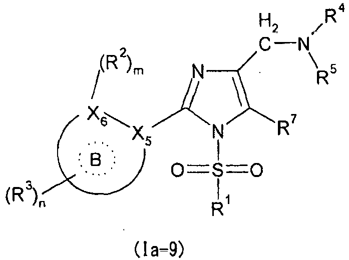 wherein
   ring B is a phenyl group, a cyclopentyl group, a cyclohexyl group, a pyrrolyl group (e.g., 1-, 2- or 3-pyrrolyl), a pyrazolyl group (e.g., 1-, 3-, 4- or 5-pyrazolyl), a thiazolyl group (e.g., 2-, 4- or 5-thiazolyl), an imidazolyl group (e.g., 1-, 2-, 4- or 5-imidazolyl), an oxazolyl group (e.g., 2-, 4-or 5-oxazolyl), a thienyl group (e.g., 2- or 3-thienyl), a furyl group (e.g., 2- or 3-furyl), a pyridyl group (e.g., 1-, 2-, 3- or 4-pyridyl), a pyrrolidinyl group (e.g., 1-, 2- or 3-pyrrolidinyl) or a piperidyl group (e.g., 1-, 2-, 3- or 4-piperidyl);
   R¹ is a phenyl group, a cyclopentyl group, a cyclohexyl group, a pyrrolyl group (e.g., 1-, 2- or 3-pyrrolyl), a pyrazolyl group (e.g., 1-, 3-, 4- or 5-pyrazolyl), a thiazolyl group (e.g., 2-, 4- or 5-thiazolyl), an imidazolyl group (e.g., 1-, 2-, 4- or 5-imidazolyl), an oxazolyl group (e.g., 2-, 4- or 5-oxazolyl), a thienyl group (e.g., 2- or 3-thienyl), a furyl group (e.g., 2- or 3-furyl), a pyridyl group (e.g., 1-, 2-, 3-or 4-pyridyl), a pyrrolidinyl group (e.g., 1-, 2- or 3-pyrrolidinyl) or a piperidyl group (e.g., 1-, 2-, 3- or 4-piperidyl), each of which is optionally substituted by 1 to 3 substituents selected from (i) a halogen atom, (ii) hydroxy, (iii) cyano, (iv) C₁₋₆ alkyl optionally substituted by 1 to 5 (preferably 1 to 3) halogen atoms, (v) C₁₋₆ alkoxy optionally substituted by 1 to 5 (preferably 1 to 3) halogen atoms, (vi) amino optionally mono- or di-substituted by C₁₋₆ alkyl, (vii) oxo, (viii) carbamoyl, (ix) mono-C₁₋₆ alkyl-carbamoyl, (x) di-C₁₋₆ alkyl-carbamoyl, (xi) C₁₋₆ alkylsulfonyl and (xii) C₁₋₆ alkyl-carbonylamino;
   R² is a halogen atom, a cyano group, a trifluoromethyl group, a methyl group, an ethyl group, a methoxy group or an ethoxy group, particularly preferably a halogen atom, a cyano group, a trifluoromethyl group, a methyl group or an ethyl group; R³ is a halogen atom, a cyano group, a trifluoromethyl group, a methyl group, an ethyl group or a methoxy group; R⁴ and R⁵ are the same or different and each is a hydrogen atom or a methyl group;
   R⁷ is a hydrogen atom, a halogen atom, a methyl group or a cyano group;
   m is 0 or 1, provided that when ring B is a phenyl group, a pyrrolyl group, a pyrazolyl group, a thiazolyl group, an imidazolyl group, an oxazolyl group, a thienyl group, a furyl group or a pyridyl group, then m should be 1; and n is an integer of 0 to 3,
   or a salt thereof.
(3) Compound (Ia-20) 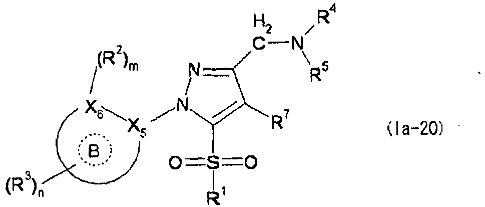 wherein
   ring B is a phenyl group, a cyclopentyl group, a cyclohexyl group, a pyrrolyl group (e.g., 1-, 2- or 3-pyrrolyl), a pyrazolyl group (e.g., 1-, 3-, 4- or 5-pyrazolyl), a thiazolyl group (e.g., 2-, 4- or 5-thiazolyl), an imidazolyl group (e.g., 1-, 2-, 4- or 5-imidazolyl), an oxazolyl group (e.g., 2-, 4-or 5-oxazolyl), a thienyl group (e.g., 2- or 3-thienyl), a furyl group (e.g., 2- or 3-furyl), a pyridyl group (e.g., 1-, 2-, 3- or 4-pyridyl), a pyrrolidinyl group (e.g., 1-, 2- or 3-pyrrolidinyl) or a piperidyl group (e.g., 1-, 2-, 3- or 4-piperidyl);
   R¹ is a phenyl group, a cyclopentyl group, a cyclohexyl group, a pyrrolyl group (e.g., 1-, 2- or 3-pyrrolyl), a pyrazolyl group (e.g., 1-, 3-, 4- or 5-pyrazolyl), a thiazolyl group (e.g., 2-, 4- or 5-thiazolyl), an imidazolyl group (e.g., 1-, 2-, 4- or 5-imidazolyl), an oxazolyl group (e.g., 2-, 4- or 5-oxazolyl), a thienyl group (e.g., 2- or 3-thienyl), a furyl group (e.g., 2- or 3-furyl), a pyridyl group (e.g., 1-, 2-, 3-or 4-pyridyl), a pyrrolidinyl group (e.g., 1-, 2- or 3-pyrrolidinyl) or a piperidyl group (e.g., 1-, 2-, 3- or 4-piperidyl), each of which is optionally substituted by 1 to 3 substituents selected from (i) a halogen atom, (ii) hydroxy, (iii) cyano, (iv) C₁₋₆ alkyl optionally substituted by 1 to 5 (preferably 1 to 3) halogen atoms, (v) C₁₋₆ alkoxy optionally substituted by 1 to 5 (preferably 1 to 3) halogen atoms, (vi) amino optionally mono- or di-substituted by C₁₋₆ alkyl, (vii) oxo, (viii) carbamoyl, (ix) mono-C₁₋₆ alkyl-carbamoyl, (x) di-C₁₋₆ alkyl-carbamoyl, (xi) C₁₋₆ alkylsulfonyl and (xii) C₁₋₆ alkyl-carbonylamino;
   R² is a halogen atom, a cyano group, a trifluoromethyl group, a methyl group, an ethyl group, a methoxy group or an ethoxy group, particularly preferably a halogen atom, a cyano group, a trifluoromethyl group, a methyl group or an ethyl group; R³ is a halogen atom, a cyano group, a trifluoromethyl group, a methyl group, an ethyl group or a methoxy group; R⁴ and R⁵ are the same or different and each is a hydrogen atom or a methyl group;
   R⁷ is a hydrogen atom, a halogen atom, a methyl group or a cyano group;
   m is 0 or 1, provided that when ring B is a phenyl group, a pyrrolyl group, a pyrazolyl group, a thiazolyl group, an imidazolyl group, an oxazolyl group, a thienyl group, a furyl group or a pyridyl group, then m should be 1; and n is an integer of 0 to 3,
   or a salt thereof.
(4) Compound (Ia-30) 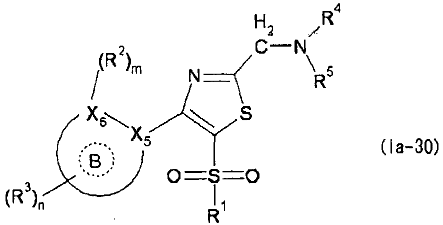 wherein
   ring B is a phenyl group, a cyclopentyl group, a cyclohexyl group, a pyrrolyl group (e.g., 1-, 2- or 3-pyrrolyl), a pyrazolyl group (e.g., 1-, 3-, 4- or 5-pyrazolyl), a thiazolyl group (e.g., 2-, 4- or 5-thiazolyl), an imidazolyl group (e.g., 1-, 2-, 4- or 5-imidazolyl), an oxazolyl group (e.g., 2-, 4-or 5-oxazolyl), a thienyl group (e.g., 2- or 3-thienyl), a furyl group (e.g., 2- or 3-furyl), a pyridyl group (e.g., 1-, 2-, 3- or 4-pyridyl), a pyrrolidinyl group (e.g., 1-, 2- or 3-pyrrolidinyl) or a piperidyl group (e.g., 1-, 2-, 3- or 4-piperidyl);
   R¹ is a phenyl group, a cyclopentyl group, a cyclohexyl group, a pyrrolyl group (e.g., 1-, 2- or 3-pyrrolyl), a pyrazolyl group (e.g., 1-, 3-, 4- or 5-pyrazolyl), a thiazolyl group (e.g., 2-, 4- or 5-thiazolyl), an imidazolyl group (e.g., 1-, 2-, 4- or 5-imidazolyl), an oxazolyl group (e.g., 2-, 4- or 5-oxazolyl), a thienyl group (e.g., 2- or 3-thienyl), a furyl group (e.g., 2- or 3-furyl), a pyridyl group (e.g., 1-, 2-, 3-or 4-pyridyl), a pyrrolidinyl group (e.g., 1-, 2- or 3-pyrrolidinyl) or a piperidyl group (e.g., 1-, 2-, 3- or 4-piperidyl), each of which is optionally substituted by 1 to 3 substituents selected from (i) a halogen atom, (ii) hydroxy, (iii) cyano, (iv) C₁₋₆ alkyl optionally substituted by 1 to 5 (preferably 1 to 3) substituents selected from a halogen atom and a non-aromatic heterocyclic group (e.g., 1-pyrrolidinyl), (v) C₁₋₆ alkoxy optionally substituted by 1 to 5 (preferably 1 to 3) halogen atoms, (vi) amino optionally mono- or di-substituted by C₁₋₆ alkyl, (vii) oxo, (viii) carbamoyl, (ix) mono-C₁₋₆ alkyl-carbamoyl, (x) di-C₁₋₆ alkyl-carbamoyl, (xi) C₁₋₆ alkylsulfonyl, (xii) C₁₋₆ alkyl-carbonylamino, (xiii) a non-aromatic heterocyclic group optionally substituted by oxo (e.g., 1-pyrrolidinyl, 2-oxo-1-pyrrolidinyl) and (xiv) a 5- or 10-membered heterocyclyl-carbonyl containing, besides carbon atoms, 1 or 2 kinds of 1 to 4 heteroatoms selected from a nitrogen atom, a sulfur atom and an oxygen atom (e.g., 1-pyrrolidinyl-carbonyl);
   R² is a halogen atom, a cyano group, a trifluoromethyl group, a methyl group, an ethyl group, a methoxy group or an ethoxy group, particularly preferably a halogen atom, a cyano group, a trifluoromethyl group, a methyl group or an ethyl group; R³ is a halogen atom, a cyano group, a trifluoromethyl group, a methyl group, an ethyl group or a methoxy group; R⁴ and R⁵ are the same or different and each is a hydrogen atom or a methyl group;
   m is 0 or 1, provided that when ring B is a phenyl group, a pyrrolyl group, a pyrazolyl group, a thiazolyl group, an imidazolyl group, an oxazolyl group, a thienyl group, a furyl group or a pyridyl group, then m should be 1; and n is an integer of 0 to 3,
   or a salt thereof.
(4) As another embodiment, compound (Ia-30) 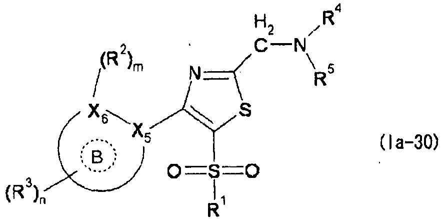 wherein
   ring B is a phenyl group, a cyclopentyl group, a cyclohexyl group, a pyrrolyl group (e.g., 1-, 2- or 3-pyrrolyl), a pyrazolyl group (e.g., 1-, 3-, 4- or 5-pyrazolyl), a thiazolyl group (e.g., 2-, 4- or 5-thiazolyl), an imidazolyl group (e.g., 1-, 2-, 4- or 5-imidazolyl), an oxazolyl group (e.g., 2-, 4-or 5-oxazolyl), a thienyl group (e.g., 2- or 3-thienyl), a furyl group (e.g., 2- or 3-furyl), a pyridyl group (e.g., 1-, 2-, 3- or 4-pyridyl), a pyrrolidinyl group (e.g., 1-, 2- or 3-pyrrolidinyl) or a piperidyl group (e.g., 1-, 2-, 3- or 4-piperidyl);
   R¹ is a phenyl group, a cyclopentyl group, a cyclohexyl group, a pyrrolyl group (e.g., 1-, 2- or 3-pyrrolyl), a pyrazolyl group (e.g., 1-, 3-, 4- or 5-pyrazolyl), a thiazolyl group (e.g., 2-, 4- or 5-thiazolyl), an imidazolyl group (e.g., 1-, 2-, 4- or 5-imidazolyl), an oxazolyl group (e.g., 2-, 4- or 5-oxazolyl), a thienyl group (e.g., 2- or 3-thienyl), a furyl group (e.g., 2- or 3-furyl), a pyridyl group (e.g., 1-, 2-, 3-or 4-pyridyl), a pyrrolidinyl group (e.g., 1-, 2- or 3-pyrrolidinyl) or a piperidyl group (e.g., 1-, 2-, 3- or 4-piperidyl), each of which is optionally substituted by 1 to 3 substituents selected from (i) a halogen atom, (ii) hydroxy, (iii) cyano, (iv) C₁₋₆ alkyl optionally substituted by 1 to 5 (preferably 1 to 3) halogen atoms, (v) C₁₋₆ alkoxy optionally substituted by 1 to 5 (preferably 1 to 3) halogen atoms, (vi) amino optionally mono- or di-substituted by C₁₋₆ alkyl, (vii) oxo, (viii) carbamoyl, (ix) mono-C₁₋₆ alkyl-carbamoyl, (x) diC₁₋₆ alkyl-carbamoyl, (xi) C₁₋₆ alkylsulfonyl and (xii) C₁₋₆ alkyl-carbonylamino;
   R² is a halogen atom, a cyano group, a trifluoromethyl group, a methyl group, an ethyl group, a methoxy group or an ethoxy group, particularly preferably a halogen atom, a cyano group, a trifluoromethyl group, a methyl group or an ethyl group; R³ is a halogen atom, a cyano group, a trifluoromethyl group, a methyl group, an ethyl group or a methoxy group; R⁴ and R⁵ are the same or different and each is a hydrogen atom or a methyl group;
   m is 0 or 1, provided that when ring B is a phenyl group, a pyrrolyl group, a pyrazolyl group, a thiazolyl group, an imidazolyl group, an oxazolyl group, a thienyl group, a furyl group or a pyridyl group, then m should be 1; and n is an integer of 0 to 3,
   or a salt thereof.
(5) Compound (Ia-31) 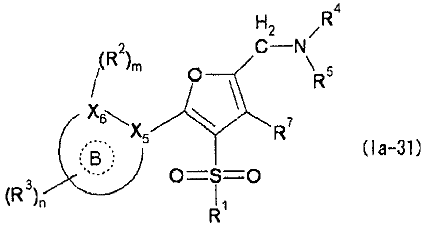 wherein
   ring B is a phenyl group, a cyclopentyl group, a cyclohexyl group, a pyrrolyl group (e.g., 1-, 2- or 3-pyrrolyl), a pyrazolyl group (e.g., 1-, 3-, 4- or 5-pyrazolyl), a thiazolyl group (e.g., 2-, 4- or 5-thiazolyl), an imidazolyl group (e.g., 1-, 2-, 4- or 5-imidazolyl), an oxazolyl group (e.g., 2-, 4-or 5-oxazolyl), a thienyl group (e.g., 2- or 3-thienyl), a furyl group (e.g., 2- or 3-furyl), a pyridyl group (e.g., 1-, 2-, 3- or 4-pyridyl), a pyrrolidinyl group (e.g., 1-, 2- or 3-pyrrolidinyl) or a piperidyl group (e.g., 1-, 2-, 3- or 4-piperidyl);
   R¹ is a phenyl group, a cyclopentyl group, a cyclohexyl group, a pyrrolyl group (e.g., 1-, 2- or 3-pyrrolyl), a pyrazolyl group (e.g., 1-, 3-, 4- or 5-pyrazolyl), a thiazolyl group (e.g., 2-, 4- or 5-thiazolyl), an imidazolyl group (e.g., 1-, 2-, 4- or 5-imidazolyl), an oxazolyl group (e.g., 2-, 4- or 5-oxazolyl), a thienyl group (e.g., 2- or 3-thienyl), a furyl group (e.g., 2- or 3-furyl), a pyridyl group (e.g., 1-, 2-, 3-or 4-pyridyl), a pyrrolidinyl group (e.g., 1-, 2- or 3-pyrrolidinyl) or a piperidyl group (e.g., 1-, 2-, 3- or 4-piperidyl), each of which is optionally substituted by 1 to 3 substituents selected from (i) a halogen atom, (ii) hydroxy, (iii) cyano, (iv) C₁₋₆ alkyl optionally substituted by 1 to 5 (preferably 1 to 3) halogen atoms, (v) C₁₋₆ alkoxy optionally substituted by 1 to 5 (preferably 1 to 3) halogen atoms, (vi) amino optionally mono- or di-substituted by C₁₋₆ alkyl, (vii) oxo, (viii) carbamoyl, (ix) mono-C₁₋₆ alkyl-carbamoyl, (x) di-C₁₋₆ alkyl-carbamoyl, (xi) C₁₋₆ alkylsulfonyl and (xii) C₁₋₆ alkyl-carbonylamino;
   R² is a halogen atom, a cyano group, a trifluoromethyl group, a methyl group, an ethyl group, a methoxy group or an ethoxy group, particularly preferably a halogen atom, a cyano group, a trifluoromethyl group, a methyl group or an ethyl group; R³ is a halogen atom, a cyano group, a trifluoromethyl group, a methyl group, an ethyl group or a methoxy group; R⁴ and R⁵ are the same or different and each is a hydrogen atom or a methyl group;
   R⁷ is a hydrogen atom, a halogen atom, a methyl group or a cyano group;
   m is 0 or 1, provided that when ring B is a phenyl group, a pyrrolyl group, a pyrazolyl group, a thiazolyl group, an imidazolyl group, an oxazolyl group, a thienyl group, a furyl group or a pyridyl group, then m should be 1; and n is an integer of 0 to 3,
   or a salt thereof.
(6) Compound (Ia-33) 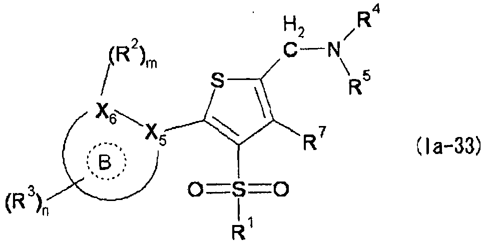 wherein
   ring B is a phenyl group, a cyclopentyl group, a cyclohexyl group, a pyrrolyl group (e.g., 1-, 2- or 3-pyrrolyl), a pyrazolyl group (e.g., 1-, 3-, 4- or 5-pyrazolyl), a thiazolyl group (e.g., 2-, 4- or 5-thiazolyl), an imidazolyl group (e.g., 1-, 2-, 4- or 5-imidazolyl), an oxazolyl group (e.g., 2-, 4-or 5-oxazolyl), a thienyl group (e.g., 2- or 3-thienyl), a furyl group (e.g., 2- or 3-furyl), a pyridyl group (e.g., 1-, 2-, 3- or 4-pyridyl), a pyrrolidinyl group (e.g., 1-, 2- or 3-pyrrolidinyl) or a piperidyl group (e.g., 1-, 2-, 3- or 4-piperidyl);
   R¹ is a phenyl group, a cyclopentyl group, a cyclohexyl group, a pyrrolyl group (e.g., 1-, 2- or 3-pyrrolyl), a pyrazolyl group (e.g., 1-, 3-, 4- or 5-pyrazolyl), a thiazolyl group (e.g., 2-, 4- or 5-thiazolyl), an imidazolyl group (e.g., 1-, 2-, 4- or 5-imidazolyl), an oxazolyl group (e.g., 2-, 4- or 5-oxazolyl), a thienyl group (e.g., 2- or 3-thienyl), a furyl group (e.g., 2- or 3-furyl), a pyridyl group (e.g., 1-, 2-, 3-or 4-pyridyl), a pyrrolidinyl group (e.g., 1-, 2- or 3-pyrrolidinyl) or a piperidyl group (e.g., 1-, 2-, 3- or 4-piperidyl), each of which is optionally substituted by 1 to 3 substituents selected from (i) a halogen atom, (ii) hydroxy, (iii) cyano, (iv) C₁₋₆ alkyl optionally substituted by 1 to 5 (preferably 1 to 3) halogen atoms, (v) C₁₋₆ alkoxy optionally substituted by 1 to 5 (preferably 1 to 3) halogen atoms, (vi) amino optionally mono- or di-substituted by C₁₋₆ alkyl, (vii) oxo, (viii) carbamoyl, (ix) mono-C₁₋₆ alkyl-carbamoyl, (x) di-C₁₋₆ alkyl-carbamoyl, (xi) C₁₋₆ alkylsulfonyl and (xii) C₁₋₆ alkyl-carbonylamino;
   R² is a halogen atom, a cyano group, a trifluoromethyl group, a methyl group, an ethyl group, a methoxy group or an ethoxy group, particularly preferably a halogen atom, a cyano group, a trifluoromethyl group, a methyl group or an ethyl group; R³ is a halogen atom, a cyano group, a trifluoromethyl group, a methyl group, an ethyl group or a methoxy group; R⁴ and R⁵ are the same or different and each is a hydrogen atom or a methyl group;
   R⁷ is a hydrogen atom, a halogen atom, a methyl group or a cyano group;
   m is 0 or 1, provided that when ring B is a phenyl group, a pyrrolyl group, a pyrazolyl group, a thiazolyl group, an imidazolyl group, an oxazolyl group, a thienyl group, a furyl group or a pyridyl group, then m should be 1; and n is an integer of 0 to 3,
   or a salt thereof.
(7) Compound (Ia-34) 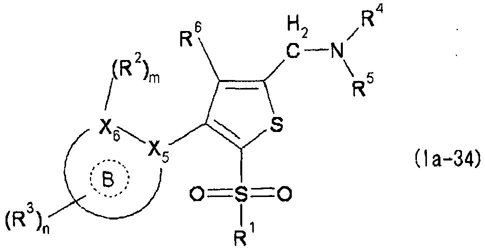 wherein
   ring B is a phenyl group, a cyclopentyl group, a cyclohexyl group, a pyrrolyl group (e.g., 1-, 2- or 3-pyrrolyl), a pyrazolyl group (e.g., 1-, 3-, 4- or 5-pyrazolyl), a thiazolyl group (e.g., 2-, 4- or 5-thiazolyl), an imidazolyl group (e.g., 1-, 2-, 4- or 5-imidazolyl), an oxazolyl group (e.g., 2-, 4-or 5-oxazolyl), a thienyl group (e.g., 2- or 3-thienyl), a furyl group (e.g., 2- or 3-furyl), a pyridyl group (e.g., 1-, 2-, 3- or 4-pyridyl), a pyrrolidinyl group (e.g., 1-, 2- or 3-pyrrolidinyl) or a piperidyl group (e.g., 1-, 2-, 3- or 4-piperidyl);
   R¹ is a phenyl group, a cyclopentyl group, a cyclohexyl group, a pyrrolyl group (e.g., 1-, 2- or 3-pyrrolyl), a pyrazolyl group (e.g., 1-, 3-, 4- or 5-pyrazolyl), a thiazolyl group (e.g., 2-, 4- or 5-thiazolyl), an imidazolyl group (e.g., 1-, 2-, 4- or 5-imidazolyl), an oxazolyl group (e.g., 2-, 4- or 5-oxazolyl), a thienyl group (e.g., 2- or 3-thienyl), a furyl group (e.g., 2- or 3-furyl), a pyridyl group (e.g., 1-, 2-, 3-or 4-pyridyl, N-oxido-4-pyridyl), a pyrrolidinyl group (e.g., 1-, 2- or 3-pyrrolidinyl) or a piperidyl group (e.g., 1-, 2-, 3- or 4-piperidyl), each of which is optionally substituted by 1 to 3 substituents selected from (i) a halogen atom, (ii) hydroxy, (iii) cyano, (iv) C₁₋₆ alkyl optionally substituted by 1 to 5 (preferably 1 to 3) substituents selected from a halogen atom and hydroxy, (v) C₁₋₆ alkoxy optionally substituted by 1 to 5 (preferably 1 to 3) halogen atoms, (vi) amino optionally mono- or di-substituted by alkyl, (vii) oxo, (viii) carbamoyl, (ix) mono-C₁₋₆ alkyl-carbamoyl, (x) di-C₁₋₆ alkyl-carbamoyl, (xi) C₁₋₆ alkylsulfonyl and (xii) Alkyl-carbonylamino;
   R² is a halogen atom, a cyano group, a trifluoromethyl group, a methyl group, an ethyl group, a methoxy group or an ethoxy group, particularly preferably a halogen atom, a cyano group, a trifluoromethyl group, a methyl group or an ethyl group;
   R³ is a halogen atom, a cyano group, a trifluoromethyl group, a methyl group, an ethyl group or a methoxy group;
   R⁴ and R⁵ are the same or different and each is a hydrogen atom or a methyl group;
   R⁶ is a hydrogen atom, a halogen atom, a methyl group or a cyano group;
   m is 0 or 1, provided that when ring B is a phenyl group, a pyrrolyl group, a pyrazolyl group, a thiazolyl group, an imidazolyl group, an oxazolyl group, a thienyl group, a furyl group or a pyridyl group, then m should be 1; and
   n is an integer of 0 to 3,
   or a salt thereof.
(7) As another embodiment, compound (Ia-34) 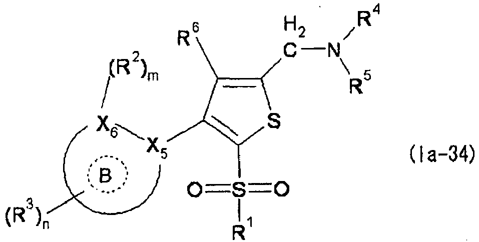 wherein
   ring B is a phenyl group, a cyclopentyl group, a cyclohexyl group, a pyrrolyl group (e.g., 1-, 2- or 3-pyrrolyl), a pyrazolyl group (e.g., 1-, 3-, 4- or 5-pyrazolyl), a thiazolyl group (e.g., 2-, 4- or 5-thiazolyl), an imidazolyl group (e.g., 1-, 2-, 4- or 5-imidazolyl), an oxazolyl group (e.g., 2-, 4-or 5-oxazolyl), a thienyl group (e.g., 2- or 3-thienyl), a furyl group (e.g., 2- or 3-furyl), a pyridyl group (e.g., 1-, 2-, 3- or 4-pyridyl), a pyrrolidinyl group (e.g., 1-, 2- or 3-pyrrolidinyl) or a piperidyl group (e.g., 1-, 2-, 3- or 4-piperidyl);
   R¹ is a phenyl group, a cyclopentyl group, a cyclohexyl group, a pyrrolyl group (e.g., 1-, 2- or 3-pyrrolyl), a pyrazolyl group (e.g., 1-, 3-, 4- or 5-pyrazolyl), a thiazolyl group (e.g., 2-, 4- or 5-thiazolyl), an imidazolyl group (e.g., 1-, 2-, 4- or 5-imidazolyl), an oxazolyl group (e.g., 2-, 4- or 5-oxazolyl), a thienyl group (e.g., 2- or 3-thienyl), a furyl group (e.g., 2- or 3-furyl), a pyridyl group (e.g., 1-, 2-, 3-or 4-pyridyl), a pyrrolidinyl group (e.g., 1-, 2- or 3-pyrrolidinyl) or a piperidyl group (e.g., 1-, 2-, 3- or 4-piperidyl), each of which is optionally substituted by 1 to 3 substituents selected from (i) a halogen atom, (ii) hydroxy, (iii) cyano, (iv) C₁₋₆ alkyl optionally substituted by 1 to 5 (preferably 1 to 3) halogen atoms, (v) C₁₋₆ alkoxy optionally substituted by 1 to 5 (preferably 1 to 3) halogen atoms, (vi) amino optionally mono- or di-substituted by C₁₋₆ alkyl, (vii) oxo, (viii) carbamoyl, (ix) mono-C₁₋₆ alkyl-carbamoyl, (x) di-C₁₋₆ alkyl-carbamoyl, (xi) C₁₋₆ alkylsulfonyl and (xii) C₁₋₆ alkyl-carbonylamino;
   R² is a halogen atom, a cyano group, a trifluoromethyl group, a methyl group, an ethyl group, a methoxy group or an ethoxy group, particularly preferably a halogen atom, a cyano group, a trifluoromethyl group, a methyl group or an ethyl group; R³ is a halogen atom, a cyano group, a trifluoromethyl group, a methyl group, an ethyl group or a methoxy group; R⁴ and R⁵ are the same or different and each is a hydrogen atom or a methyl group;
   R⁶ is a hydrogen atom, a halogen atom, a methyl group or a cyano group;
   m is 0 or 1, provided that when ring B is a phenyl group, a pyrrolyl group, a pyrazolyl group, a thiazolyl group, an imidazolyl group, an oxazolyl group, a thienyl group, a furyl group or a pyridyl group, then m should be 1; and n is an integer of 0 to 3,
   or a salt thereof.

In the present invention, the following compounds are particularly preferable.
1-[4-(2-Fluoropyridin-3-yl)-5-(pyridin-3-ylsulfonyl)thiophen-2-yl]-N-methylmethanamine or a salt thereof (Example 48).
1-[5-(2-Fluoropyridin-3-yl)-4-(pyridin-3-ylsulfonyl)thiophen-2-yl]-N-methylmethanamine or a salt thereof (Example 65).
1-[1-(2-Fluoropyridin-3-yl)-5-(phenylsulfonyl)-1H-pyrazol-3-yl]-N-methylmethanamine or a salt thereof (Example 79).
1-[1-(2-Fluorophenyl)-5-(pyridin-3-ylsulfonyl)-1H-pyrazol-3-yl]-N-methylmethanamine or a salt thereof (Example 81).
1-[1-(2-Chlorophenyl)-5-(pyridin-3-ylsulfonyl)-1H-pyrazol-3-yl]-N-methylmethanamine or a salt thereof (Example 87).
1-{1-(2-Chlorophenyl)-5-[(6-methylpyridin-3-yl)sulfonyl]-1H-pyrazol-3-yl}-N-methylmethanamine or a salt thereof (Example 89).
1-[1-(2,3-Difluorophenyl)-5-(pyridin-3-ylsulfonyl)-1H-pyrazol-3-yl]-N-methylmethanamine or a salt thereof (Example 98).
1-{1-(2,3-Difluorophenyl)-5-[(6-methylpyridin-3-yl)sulfonyl]-1H-pyrazol-3-yl}-N-methylmethanamine or a salt thereof (Example 99).

Examples of the salt of compound (I) include metal salts, ammonium salts, salts with organic bases, salts with inorganic bases, salts with organic acids, salts with basic or acidic amino acids. Preferable examples of metal salt include alkali metal salts such as sodium salt, potassium salt; alkaline earth metal salts such as calcium salt, magnesium salt, barium salt; aluminum salt. Preferable examples of the salt with organic base include a salt with trimethylamine, triethylamine, pyridine, picoline, 2,6-lutidine, ethanolamine, diethanolamine, triethanolamine, cyclohexylamine, dicyclohexylamine, N,N'-dibenzylethylenediamine. Preferable examples of the salt with inorganic acid include a salt with hydrochloric acid, hydrobromic acid, nitric acid, sulfuric acid, phosphoric acid. Preferable examples of the salt with organic acid include a salt with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid. Preferable examples of the salt with basic amino acid include a salt with arginine, lysin, ornithine. Preferable examples of the salt with acidic amino acid include a salt with aspartic acid, glutamic acid.

Of these, pharmaceutically acceptable salts are preferable. For example, when a compound contains an acidic functional group, inorganic salts such as alkali metal salt (e.g., sodium salt, potassium salt etc.), alkaline earth metal salt (e.g., calcium salt, magnesium salt, barium salt etc.), ammonium salts; and when a compound contains a basic functional group, for example, salts with inorganic acid such as hydrochloric acid, hydrobromic acid, nitric acid, sulfuric acid, phosphoric acid, or salts with organic acid such as acetic acid, phthalic acid, fumaric acid, oxalic acid, tartaric acid, maleic acid, citric acid, succinic acid, methanesulfonic acid, p-toluenesulfonic acid.

The production methods of compound (I) of the present invention are explained.

The compounds (Ia)-(XVII) in the schemes may form salts, and as such salts, for example, those similar to the salts of compound (I) can be mentioned.

p is an integer of 0, 1 or 2. A compound wherein p is 0 or 1 is can be converted to a compound wherein p is 2 by oxidization using a suitable oxidant. Compound (I) is compound (Ia) wherein p is 2.

While the compounds obtained in respective steps can be used for the next reaction in the form of a reaction mixture or a crude product, they can also be easily isolated and purified from the reaction mixture by a known separation and purification means, such as recrystallization, distillation, chromatography.

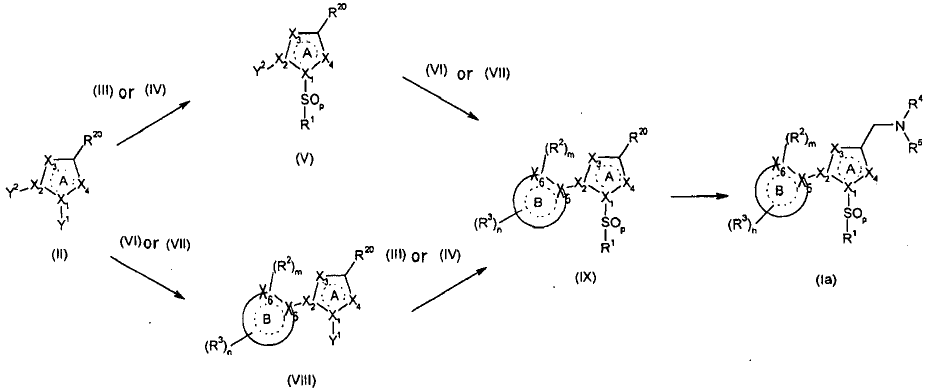

Compound (II) wherein ring A, X₁, X₂, X₃ and X₄ are as defined above, Y¹ and Y² are the same or different and each is a hydrogen atom; a leaving group such as a halogen atom (e.g., a fluorine atom, a chlorine atom, a bromine atom, an iodine atom), methanesulfonyloxy, p-toluenesulfonyloxy, trifluoromethanesulfonyloxy; a hydroxyl group; an amino group or a mercapto group, and R²⁰ is a hydrogen atom, a formyl group, a carboxyl group, an ester group, a cyano group, an alkylaminocarbonyl group, a dialkylamino group, may be commercially available, or can be produced according to a method known per se, for example, the methods described in Journal of the Chemical Society Chemical Communications (J. C. S. Chem. Commun.), page 26 (1983), Heterocycles, vol. 46, page 489 (1997), or a method analogous thereto.

When Y¹ is a hydrogen atom or a leaving group such as a halogen atom (e.g., a fluorine atom, a chlorine atom, a bromine atom, an iodine atom), methanesulfonyloxy, p-toluenesulfonyloxy, trifluoromethanesulfonyloxy, compound (V) wherein each symbol is as defined above, can be produced by compound (II) with compound (III) 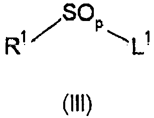 wherein R¹ and p is as defined above, and L¹ is a hydrogen atom, a leaving group such as a halogen atom (e.g., a fluorine atom, a chlorine atom, a bromine atom, an iodine atom), or a metal atom such as sodium, potassium. When Y¹ is a hydroxyl group, an amino group or a mercapto group, compound (V) can be produced by compound (II) with compound (IV)

R¹-L² (IV)

wherein R¹ is as defined above, and L² is a hydrogen atom or a leaving group such as halogen atom (e.g., a fluorine atom, a chlorine atom, a bromine atom, an iodine atom), methanesulfonyl, p-toluenesulfonyl.

The amount of compound (III) to be used is about 1 to about 10 mol, preferably about 1 to about 3 mol, per 1 mol of compound (II).

The amount of compound (IV) to be used is about 1 to about 10 mol, preferably about 1 to about 3 mol, per 1 mol of compound (II).

This reaction is advantageously carried out using a solvent inert to the reaction. While the solvent is not particularly limited as long as the reaction proceeds, and hydrocarbons such as benzene, toluene, ethers such as tetrahydrofuran, amides such as N,N-dimethylformamide, N,N-dimethylacetamide, and a mixed solvent thereof are preferable.

The reaction is advantageously carried out using a base. Examples of the base include inorganic bases such as sodium hydride, sodium hydroxide, potassium hydroxide, basic salts such as sodium carbonate, potassium carbonate, cesium carbonate, sodium hydrogen carbonate, metal bases such as potassium ethoxide, potassium tert-butoxide, sodium methoxide, sodium ethoxide, aromatic amines such as pyridine, lutidine, tertiary amines such as triethylamine, N-diisopropylethylamine, tripropylamine, tributylamine, cyclohexyldimethylamine, 4-dimethylaminopyridine, N,N-dimethylaniline, N-methylpiperidine, N-methylpyrrolidine, N-methylmorpholine. The amount of the base to be used is about 1 to about 10 mol, preferably about 1 to about 5 mol, per 1 mol of compound (II).

This reaction can be carried out in the presence of a crown ether or a halogenating agent. Examples of the crown ether include 15-crown-5-ether, 18-crown-6-ether. Examples of the halogenating agent include N-iodosuccinimide, N-bromosuccinimide, N-chlorosuccinimide, bromine. The amount of the crown ether or halogenating agent to be used is about 1 to about 10 mol, preferably about 1 to about 5 mol, per 1 mol of compound (II), respectively.

Alternatively, this reaction can also be carried out in the presence of a metal catalyst such as palladium catalyst. Examples of the palladium catalyst include tetrakis(triphenylphosphine)palladium (0), tris(dibenzylideneacetone)dipalladium, palladium acetate. In this case, this reaction can be carried out in the co-presence of a phosphine, if desired. Examples of the phosphine include 9,9-dimethyl-4,5-bis(diphenylphosphino)xanthine (XANTOPHOS), 2,2'-bis(diphenylphosphino)-1,1'-binaphthyl(BINAP). The amount of the palladium catalyst and phosphine to be used is about 0.01 to about 0.5 mol, preferably about 0.01 to about 0.3 mol, per 1 mol of compound (II), respectively.

While the reaction time varies depending on the reagents and solvent to be used, it is generally about 30 min to about 24 hr, preferably about 30 min to about 18 hr.

The reaction temperature is generally about 0°C to about 150°C, preferably about 10°C to about 120°C.

Compound (VIII) wherein ach symbol is as defined above, may be commercially available, or can be produced according to a method known per se, for example, the methods described in Journal of Bioorganic and Medicinal Chemistry Letters, vol. 16, page 731 (2006), Chemical and Pharmaceutical Bulletin, vol. 31, page 1228 (1981), WO 2004/98589, or a method analogous thereto. Alternatively, compound (VIII) can also be produced by compound (II) with compound (VI) (or a various ester derivative of compound (VI)) 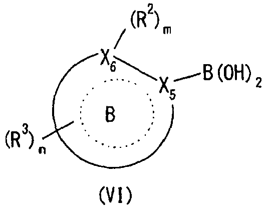 wherein each symbol is as defined above, according to the method described in Synthetic Communications, vol. 11, page 513 (1981), or a method analogous thereto.

In addition, compound (VIII) can also be produced by compound (II) with compound (VII) 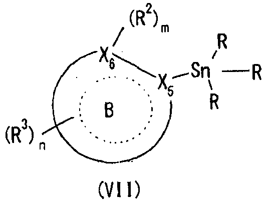 wherein R is an alkyl group or an allyl group, and R² and R³ are as defined above, according to the method described in Synthesis, vol. 7, pages 564-565 (1986), or a method analogous thereto.

The amount of compound (VI) to be used is about 1 to about 10 mol, preferably about 1 to about 3 mol, per 1 mol of compound (II).

The amount of compound (VII) to be used is about 1 to about 10 mol, preferably about 1 to about 3 mol, per 1 mol of compound (II).

Compound (IX) can be produced from compound (V) in the same manner as in the production method of compound (VIII) from compound (II), or a method analogous thereto. Alternatively, compound (IX) can also be produced from compound (VIII) in the same manner as in the production method of compound (V) from compound (II), or a method analogous thereto.

When R²⁰ is a formyl group, compound (Ia) can be produced by subjecting compound (IX) to reductive amination reaction using compound (X) 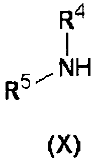 wherein each symbol is as defined above, according to the methods described in Shin Jikken Kagaku Kouza, vol. 14-III, pages 1380-1385 (Maruzen Press), or a method analogous thereto.

The amount of compound (X) to be used is about 1 to about 20 mol, preferably about 1 to about 10 mol, per 1 mol of compound (IX).

When R²⁰ is a hydrogen atom, compound (Ia) can be produced by subjecting compound (IX) to formylation, for example, according to the methods described in Jikken Kagaku Kouza, 4th edition, vol. 21, pages 106-124 (1991) (Maruzen Press), or a method analogous thereto, and then subjecting the resulting compound to the above-mentioned reductive amination reaction.

When R²⁰ is an ester group, compound (Ia) can be produced by reducing the ester group of compound (IX) using a reducing agent such as lithium aluminum hydride, diisobutylaluminum hydride, sodium borohydride, calcium bis(borohydride), and oxidizing the resulting hydroxyl group using an oxidant such as chrome acid-pyridine complex, pyridinium chlorochromate, manganese dioxide, sulfur trioxide-pyridine complex, tetra-n-propylammonium perruthenate to convert the hydroxyl group to a formyl group, and by subjecting the resulting compound to the above-mentioned reductive amination reaction.

The reducing agent is particularly preferably diisobutylaluminum hydride. The amount of the reducing agent to be used is about 0.75 to about 10 equivalent, preferably about 1 to about 5 equivalent, per 1 mol of compound (IX).

The oxidant is preferably manganese dioxide, sulfur trioxide-pyridine complex or tetra-n-propylammonium perruthenate. The amount of the oxidant to be used is about 0.01 to 30 equivalent, preferably about 0.05 to about 10 equivalent, per 1 mol of compound (IX). The oxidative reaction is carried out, for example, according to the method described in Synthesis, page 639 (1994).

This reaction is advantageously carried out using a solvent inert to the reaction. While the solvent is not particularly limited as long as the reaction proceeds, and hydrocarbons (e.g., benzene, toluene), ethers (e.g., tetrahydrofuran, diethyl ether), and a mixed solvent thereof are preferable.

While the reaction time varies depending on the reagent or solvent to be used, it is generally about 30 min to about 24 hr, preferably about 30 min to about 8 hr.

The reaction temperature is generally about -78°C to about 100°C, preferably about -78°C to about 25°C.

When R²⁰ is a cyano group, compound (Ia) can be produced by reducing the cyano group of compound (IX) using a reducing agent such as diisobutylaluminum hydride to convert the cyano group to a formyl group, by subjecting the resulting compound to the above-mentioned reductive amination reaction.

The reducing agent is particularly preferably diisobutylaluminum hydride. The amount of the reducing agent to be used is about 0.75 to about 10 equivalent, preferably about 1 to about 5 equivalent, per 1 mol of compound (IX).

This reaction is advantageously carried out using a solvent inert to the reaction. While the solvent is not particularly limited as long as the reaction proceeds, and hydrocarbons (e.g., benzene, toluene), ethers (e.g., tetrahydrofuran, diethyl ether), and a mixed solvent thereof are preferable.

While the reaction time varies depending on the reagent or solvent to be used, it is generally about 30 min to about 24 hr, preferably about 30 min to about 8 hr.

The reaction temperature is generally about -78°C to about 100°C, preferably about -78°C to about 25°C.

When R²⁰ is an aminocarbonyl group or a dialkylaminocarbonyl group, compound (Ia) can be produced by reducing compound (IX) using a reducing agent.

Examples of the reducing agent include metal hydrides such as sodium borohydride, lithium aluminum hydride, boranes such as borane-tetrahydrofuran complex. The amount of the reducing agent to be used is about 0.5 to about 10 mol, preferably about 1 to about 5 mol, per 1 mol of compound (IX). If desired, an acid catalyst can be added together with a reducing agent.

Examples of the acid catalyst include Lewis acids such as trifluoroborane-diethyl ether complex, aluminum chloride. The amount of the acid catalyst to be used is about 0.5 to about 10 mol, preferably about 1.0 to about 5.0 mol, per 1 mol of compound (IX).

This reaction is advantageously carried out without solvent or using a solvent inert to the reaction. While the solvent is not particularly limited as long as the reaction proceeds, and examples thereof include alcohols (e.g., methanol, ethanol, propanol), hydrocarbons (e.g., cyclohexane, hexane, benzene, toluene, xylene, mesitylene), organic acids (e.g., formic acid, acetic acid), ethers (e.g., tetrahydrofuran, dioxane, 1,2-dimethoxyethane, diethyl ether, diisopropyl ether), anilines (e.g., N,N-dimethylaniline, N,N-diethylaniline), halogenated hydrocarbons (e.g., dichloromethane, chloroform, carbon tetrachloride, 1,2-dichloroethane), and a mixed solvent thereof.

The reaction time is generally about 10 min to about 24 hr, preferably about 30 min to 12 hr. The reaction temperature is generally about 0 to about 120°C, preferably about 25 to about 100°C.

When R²⁰ is an ester group or a carboxyl group, compound (Ia) can be produced by condensing compound (IX) with compound (X), and subjecting the resulting compound to the above-mentioned reduction.

The aforementioned reaction can be carried out in the presence of a suitable condensing agent.

Examples of the condensing agent include N,N'-dicarboimides such as N,N'-dicyclohexylcarbodiimide, 1-ethyl-3-(3-dimethylaminopropyl)carbodiimide (WSC) hydrochloride; azolites such as N,N'-carbonyldiimidazole; dehydrating agents such as N-ethoxycarbonyl-2-ethoxy-1,2-dihydroquinoline, phosphorus oxychloride, acetic anhydride; 2-halogenopyridinium salts such as 2-chloromethylpyridinium iodide, 2-fluoro-1-chloromethylpyridinium iodide. The amount of the condensing agent to be used is about 1 to about 5 mol, preferably about 2 to 3 mol, per 1 mol of compound (IX).

The reaction can be carried out in the co-presence of a base together with a condensing agent, if desired. Examples of the base include basic salts such as potassium acetate, sodium acetate, 1-hydroxy-1H-benzotriazole (HOBt) monohydrate. The amount of the base to be used is about 1 to about 5 mol, preferably about 2 to about 3 mol, per 1 mol of compound (IX).

This reaction is advantageously carried out using a solvent inert to the reaction. While the solvent is not particularly limited as long as the reaction proceeds, and alcohols (e.g., methanol, ethanol, propanol), and hydrocarbons (e.g., cyclohexane, hexane, benzene, toluene, xylene), ethers (e.g., tetrahydrofuran, dioxane, 1,2-dimethoxyethane, diethyl ether, diisopropyl ether), amides (e.g., N,N-dimethylformamide, N,N-dimethylacetamide, hexamethylphosphoric triamide), sulfoxides (e.g., dimethyl sulfoxide), halogenated hydrocarbons (e.g., dichloromethane, chloroform, carbon tetrachloride, 1,2-dichloroethane), acid anhydrides (e.g., acetic anhydride), and a mixed solvent thereof are preferable.

The reaction time is generally about 30 min to about 48 hr, preferably about 30 min to about 24 hr. The reaction temperature is generally about 0 to about 120°C, preferably about 25 to about 100°C.

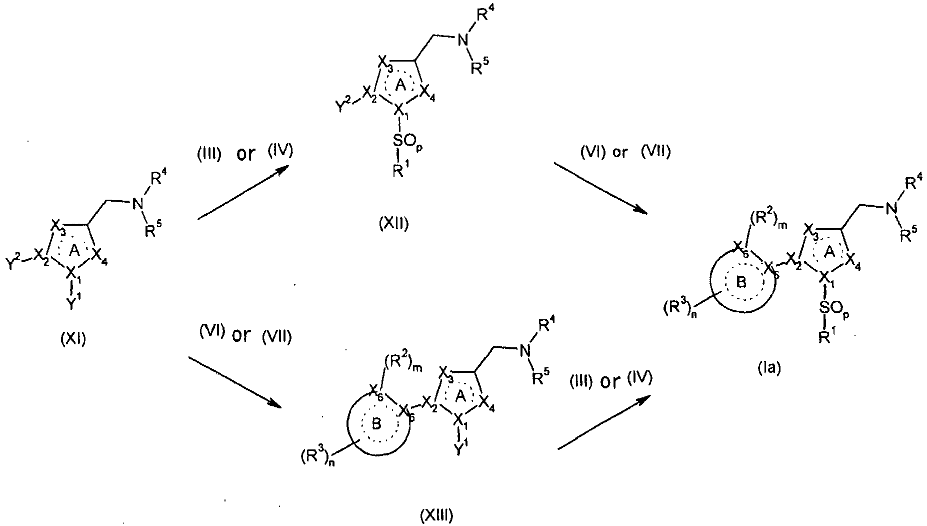

Compound (XI) wherein each symbol is as defined above, may be commercially available, or can be produced according to a method known per se, for example, the methods described in Journal of American Chemical Society, vol. 72, page 745 (1950), or a method analogous thereto.

Compound (XII) can be produced from compound (XI) in the same manner as in the production method of compound (V) from compound (II), or a method analogous thereto.

Compound (XIII) can be produced from compound (XI) in the same manner as in the production method of compound (VIII) from compound (II), or a method analogous thereto.

Alternatively, In the same manner as in the production method of compound (Ia) from compound (IX), or a method analogous thereto, compound (XI) can be produced form compound (II), compound (XII) can be produced from compound (V), and compound (XIII) can be also produced from compound (VIII).

Compound (Ia) can be produced from compound (XII) in the same manner as in the production method of compound (VIII) from compound (II), or a method analogous thereto, or from compound (XIII) in the same manner as in the production method of compound (V) from compound (II), or a method analogous thereto.

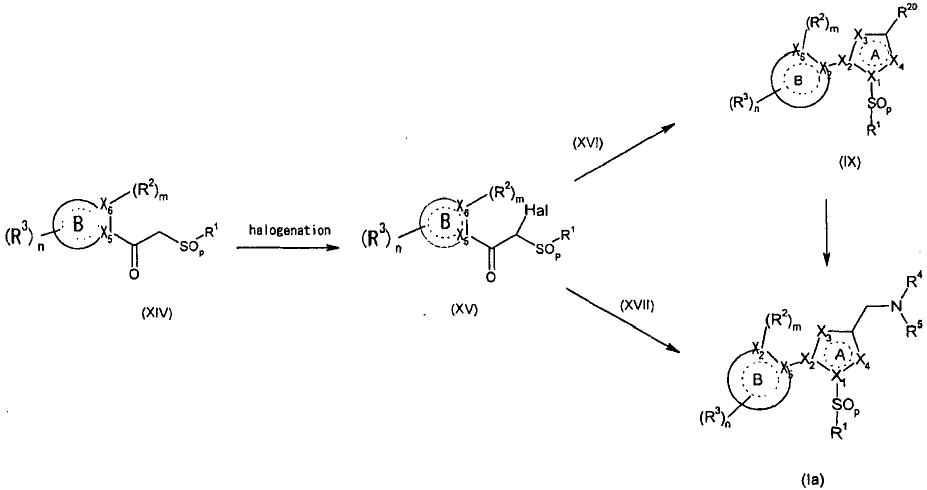

Compound (XIV) wherein each symbol is as defined above, may be commercially available, or can be produced according to a method known per se, for example, the methods described in Journal of Organic Chemistry, vol. 46, page 2596 (1981), Organic letters, vol. 3, page 1261 (2001), or a method analogous thereto.

Compound (XV) wherein each symbol is as defined above, and Hal is a leaving group such as a halogen atom (e.g., a fluorine atom, a chlorine atom, a bromine atom, an iodine atom)) can be produced by treating compound (XIV) with a halogen such as chlorine, bromine, iodine, or a metal halide such as copper(II) bromide, copper(II) chloride. The amount of the halogen or metal halide to be used is about 1 to about 5 mol, preferably about 1 to about 2 mol, per 1 mol of compound (XIV).

This reaction is advantageously carried out without solvent or using a solvent inert to the reaction. While the solvent is not particularly limited as long as the reaction proceeds, and examples thereof include ethers, esters, aromatic hydrocarbons, aliphatic hydrocarbons, amides, halogenated hydrocarbons, nitriles, sulfoxides, organic acids, aromatic amines, and a mixture of two or more solvents.

This reaction can be carried out in the presence of an acid or a base.

Examples of the acid include inorganic acids such as hydrochloric acid, hydrobromic acid. Examples of the base include hydroxides such as sodium hydroxide, potassium hydroxide, lithium hydroxide; basic salts such as sodium carbonate, potassium carbonate, cesium carbonate, sodium hydrogen carbonate, sodium acetate; aromatic amines such as pyridine, lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine. The amount of the acid to be used is about 0.01 to about 3 mol, preferably about 0.01 to about 1 mol, per 1 mol of compound (XIV). The amount of the base to be used is about 1 to about 10 mol, preferably about 1 to about 3 mol, per 1 mol of compound (XIV).

While the reaction time varies depending on the reagents and solvent to be used, it is generally about 5 min to about 24 hr, preferably about 10 min to about 5 hr.

The reaction temperature is generally about -20°C to about 150°C, preferably about 0°C to about 100°C.

Compound (IX) can be produced by condensing compound (XV) with compound (XVI) 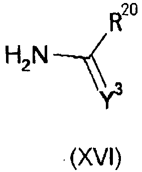 wherein R²⁰ is as defined above, and Y³ is an oxygen atom, a sulfur atom or a nitrogen atom (NH).

Compound (XVI) may be commercially available, or can be produced according to a method known per se or a method analogous thereto. The amount of compound (XVI) to be used is about 0.5 to about 3 mol, preferably about 0.8 to about 2 mol, per 1 mol of compound (XV).

This reaction is advantageously carried out without solvent or using a solvent inert to the reaction. While the solvent is not particularly limited as long as the reaction proceeds, and examples thereof include halogenated hydrocarbons, aliphatic hydrocarbons, aromatic hydrocarbons, ethers, amides, alcohols, nitriles, and a mixture of two or more solvents.

This reaction can be carried out in the presence of a base, if desired. Examples of the base include basic salts such as sodium carbonate, potassium carbonate, cesium carbonate, sodium hydrogen carbonate, sodium acetate; aromatic amines such as pyridine, lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine. The amount of the base to be used is about 1 to about 30 mol, preferably about 1 to about 10 mol, per 1 mol of compound (XV).

While the reaction time varies depending on the reagents and solvent to be used, it is generally about 5 min to about 72 hr, preferably about 0.5 hr to about 30 hr.

The reaction temperature is generally about -5°C to about 200°C, preferably about 5°C to about 150°C.

Compound (Ia) can be produced by condensing compound (XV) with compound (XVII) 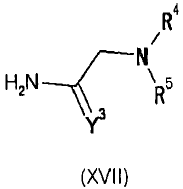 wherein each symbol is as defined above.

Compound (XVII) may be commercially available, or can be produced according to a method known per se or a method analogous thereto.

The amount of compound (XVII) to be used is about 0.5 to about 3 mol, preferably about 0.8 to about 2 mol, per 1 mol of compound (XV).

This reaction is advantageously carried out without solvent or using a solvent inert to the reaction. While the solvent is not particularly limited as long as the reaction proceeds, and examples thereof include halogenated hydrocarbons, aliphatic hydrocarbons, aromatic hydrocarbons, ethers, amides, alcohols, nitriles, and a mixture of two or more solvents.

This reaction can be carried out in the presence of a base, if desired. Examples of the base include basic salts such as sodium carbonate, potassium carbonate, cesium carbonate, sodium hydrogen carbonate, sodium acetate; aromatic amines such as pyridine, lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine. The amount of the base to be used is about 1 to about 30 mol, preferably about 1 to about 10 mol, per 1 mol of compound (XV).

While the reaction time varies depending on the reagents and solvent to be used, it is generally about 5 min to about 72 hr, preferably about 0.5 hr to about 30 hr.

The reaction temperature is generally about -5°C to about 200°C, preferably about 5°C to about 150°C.

In each of the aforementioned reactions, when the starting compound has an amino group, a carboxyl group or a hydroxy group as a substituent, a protecting group generally used in peptide chemistry may be introduced into these groups. By removing the protecting group as necessary after the reaction, the objective compound can be obtained. Introduction or removal of these protective groups may be carried out according to a method known per se, for example, the method disclosed in Theodora W. Greene and Peter G. M. Wuts, "Protective Groups in Organic Synthesis, 3rd Ed.", Wiley-Interscience (1999), or the like.

Compounds (Ia)-(XVII) can be produced by further carrying out one or more of known deprotection reaction, acylation reaction, alkylation reaction, hydrogenation reaction, oxidation reaction, reduction reaction, carbon chain extension reaction, substituent exchange reaction, as desired.

When compounds (Ia)-(XVII) are obtained as a free compound, they can be converted to a desired salt by a method known per se or a method analogous thereto; conversely, when compounds (Ia)-(XVII) are obtained as a salt, they can be converted into a free form or another desired salt by a method known per se or a method analogous thereto.

Compound (I) can be isolated and purified by a known means such as phase transfer, concentration, solvent extraction, fractionation, liquid conversion, crystallization, recrystallization, chromatography.

When compound (I) is obtained as a free compound, it can be converted to a desired salt by a method known per se or a method analogous thereto; conversely, when compound (I) is obtained as a salt, it can be converted into a free form or another desired salt by a method known per se or a method analogous thereto.

Compound (I) may be used as a prodrug. The prodrug of compound (I) means a compound which is converted to compound (I) under the physiological condition in the body by a reaction with an enzyme, gastric acid, or the like, that is, a compound which is converted to compound (I) by enzymatic oxidation, reduction, hydrolysis; a compound which is converted to compound (I) by hydrolysis with gastric acid.

Examples of the prodrug of compound (I) include a compound wherein the amino group of compound (I) is modified with acyl, alkyl or phosphoryl (e.g., a compound wherein the amino group of compound (I) is modified with eicosanoyl, alanyl, pentylaminocarbonyl, (5-methyl-2-oxo-1,3-dioxolen-4-yl)methoxycarbonyl, tetrahydrofuranyl, pyrrolidylmethyl, pivaloyloxymethyl or t-butyl, etc.); a compound wherein the hydroxy group of compound (I) is modified with acyl, alkyl, phosphoric acid or boric acid (e.g., a compound wherein the hydroxy group of compound (I) is modified with acetyl, palmitoyl, propanoyl, pivaloyl, succinyl, fumaryl, alanyl or dimethylaminomethylcarbonyl, etc.); a compound wherein a carboxyl group of compound (I) is modified to ester or amide (e.g., a compound wherein a carboxyl group of compound (I) is modified to ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester or methylamide, etc.). These compounds can be produced from compound (I) by a method known *per se.*

In addition, the prodrug of compound (I) may be a compound, which is converted to compound (I) under the physiological conditions, as described in Pharmaceutical Research and Development, Vol. 7 (Molecule Design), pp. 163-198 (1990), published by Hirokawa Publishing Co.

When compound (I) contains an optical isomer, a stereoisomer, a regioisomer or a rotamer, either isomer and a mixture of these are also encompassed in compound (I). For example, when compound (I) has an optical isomer, an optical isomer resolved from a racemate is also encompassed in compound (I). These isomers can be obtained as single products according to synthesis and separation methods known per se (concentration, solvent extraction, column chromatography, recrystallization, etc.)

The compound (I) may be a crystal, and both a single crystal and crystal mixtures are encompassed in compound (I). Crystals can be produced by crystallization according to crystallization methods known *per se.*

The compound (I) may be a solvate (e.g., hydrate etc.) or a non-solvate, both of which are encompassed in the compound (I).

A compound labeled with an isotope (e.g., ³H, ¹⁴C, ³⁵S, ¹²⁵I) and a deuterium conversion form wherein ¹H has been converted to ²H(D) are also encompassed in the compound (I).

Compound (I) of the present invention (hereinafter sometimes to be abbreviated as the compound of the present invention) has a proton pump inhibitory effect and effectively suppress gastric acid secretion. In addition, since it shows low toxicity (e.g., acute toxicity, chronic toxicity, genetic toxicity, reproductive toxicity, cardiotoxicity, drug interaction, carcinogenicity) and high water-solubility, and are superior in the stability, in vivo kinetics (absorbability, distribution, metabolism, excretion), and efficacy expression, it is useful as a pharmaceutical agent.

The compound of the present invention is useful for the prophylaxis or treatment of peptic ulcer (e.g., gastric ulcer, duodenal ulcer, anastomotic ulcer, ulcer caused by non-steroidal anti-inflammatory agent, ulcer due to postoperative stress etc.); Zollinger-Ellison syndrome; gastritis; erosive esophagitis; reflux esophagitis such as erosive reflux esophagitis; symptomatic gastroesophageal reflux disease (Symptomatic GERD) such as nonerosive esophageal reflux, esophageal reflux unaccompanied by esophagitis; Barrettesophagus; functional dyspepsia; gastric cancer (including gastric cancer associated with promoted production of interleukin-1β due to gene polymorphism of interleukin-1); stomach MALT lymphoma; hyperacidity; upper gastrointestinal hemorrhage caused by peptic ulcer, acute stress ulcer, hemorrhagic gastritis, invasive stress (e.g., stress caused by major surgery requiring post-operative intensive management, or cerebrovascular disorder, head trauma, multiple organ failure or extensive burn requiring intensive treatment); airway disorders; asthma; in mammals (e.g., human, monkey, sheep, bovine, horse, dog, cat, rabbit, rat, mouse etc.), pre-anesthetic administration, eradication.or assistant to eradication of Helicobacter pylori.

As used herein, the above-mentioned reflux esophagitis and symptomatic gastroesophageal reflux disease (symptomatic GERD) are sometimes collectively referred to simply as GERD.

The content of a compound of the present invention in the pharmaceutical composition of the present invention is about 0.01 to 100% by weight relative to the entire composition. Though subject to change depending on the administration target, administration route, target disease, its dose is about 0.5 to 1,500 mg/day, preferably about 5 to 150 mg/day, based on the active ingredient, when, for example, the compound is orally administered as an anti-ulcer agent to an adult human (60 kg). The compound of the present invention may be administered once daily or in 2 or 3 divided portions per day.

The compound of the present invention shows low toxicity and can be safely administered orally or parenterally (e.g., topical, rectal, intravenous administrations) as it is or as a preparation containing a pharmaceutical composition containing a pharmacologically acceptable carrier admixed according to a method known per se, such as tablets (including sugar-coated tablets and film-coated tablets), powder, granule, capsule (including soft capsule), orally disintegrating tablet, orally disintegrating film, liquid, injection, suppository, sustained-release preparation, plaster. Particularly, the compound of the present invention is preferably administered as an oral preparation in the form of tablet, granule, capsule.

Examples of the pharmacologically acceptable carrier that may be used to produce the pharmaceutical composition of the present invention include various organic or inorganic carrier substances in common use as pharmaceutical materials, including excipients, lubricants, binders, disintegrants, aqueous polymers and basic inorganic salts for solid preparations; and solvents, solubilizing agents, suspending agents, isotonizing agents, buffers and soothing agents for liquid preparations. Other ordinary pharmaceutical additives such as preservatives, anti-oxidants, colorants, sweetening agents, souring agents, bubbling agents and flavorings may also be used as necessary.

Examples of the "excipients" include lactose, sucrose, D-mannitol, starch, cornstarch, crystalline cellulose, light anhydrous silicic acid, titanium oxide.

Examples of the "lubricants" include magnesium stearate, sucrose fatty acid esters, polyethylene glycol, talc, stearic acid.

Examples of the "binders" include hydroxypropyl cellulose, hydroxypropylmethyl cellulose, crystalline cellulose, starch, polyvinylpyrrolidone, gum arabic powder, gelatin, pullulan, low-substituted hydroxypropyl cellulose.

Examples of the "disintegrants" include (1) crosspovidone, (2) what is called super-disintegrants such as crosscarmellose sodium (FMC-Asahi Chemical) and carmellose calcium (Gotoku Yakuhin) etc, (3) sodium carboxymethyl starch (e.g., product of Matsutani Chemical), (4) low-substituted hydroxypropyl cellulose (e.g., product of Shin-Etsu Chemical), (5) corn starch, and so forth. Said "crosspovidone" may be any crosslinked polymer having the chemical name 1-ethenyl-2-pyrrolidinone homopolymer, including polyvinylpyrrolidone (PVPP) and 1-vinyl-2-pyrrolidinone homopolymer, and is exemplified by Colidon CL (produced by BASF), Polyplasdon XL (produced by ISP), Polyplasdon XL-10 (produced by ISP), Polyplasdon INF-10 (produced by ISP).

Examples of the "aqueous polymers" include ethanol-soluble aqueous polymers [e.g., cellulose derivatives such as hydroxypropyl cellulose (hereinafter also referred to as HPC) etc, polyvinylpyrrolidone], ethanol-insoluble aqueous polymers [e.g., cellulose derivatives such as hydroxypropylmethyl cellulose (hereinafter also referred to as HPMC), methyl cellulose, carboxymethyl cellulose sodium, sodium polyacrylate, polyvinyl alcohol, sodium alginate, guar gum].

Examples of the "basic inorganic salts" include basic inorganic salts of sodium, potassium, magnesium and/or calcium. Preferred are basic inorganic salts of magnesium and/or calcium. More preferred are basic inorganic salts of magnesium. Examples of the basic inorganic salts of sodium include sodium carbonate, sodium hydrogen carbonate, disodium hydrogenphosphate. Examples of the basic inorganic salts of potassium include potassium carbonate, potassium hydrogencarbonate. Examples of the basic inorganic salts of magnesium include heavy magnesium carbonate, magnesium carbonate, magnesium oxide, magnesium hydroxide, magnesium aluminometasilicate, magnesium silicate, magnesium aluminate, synthetic hydrotalcite [Mg₆Al₂(OH)₁₆ CO₃ 4H₂O], and aluminum magnesium hydroxide. Preferred are heavy magnesium carbonate, magnesium carbonate, magnesium oxide, magnesium hydroxide. Examples of the basic inorganic salts of calcium include precipitated calcium carbonate, calcium hydroxide.

Examples of the "solvents" include water for injection, alcohol, propylene glycol, macrogol, sesame oil, corn oil, olive oil.

Examples of the "solubilizing agents" include polyethylene glycol, propylene glycol, D-mannitol, benzyl benzoate, ethanol, trisaminomethane, cholesterol, triethanolamine, sodium carbonate, sodium citrate.

Examples of the "suspending agents" include surfactants such as stearyltriethanolamine, sodium lauryl sulfate, laurylaminopropionic acid, lecithin, benzalkonium chloride, benzethonium chloride, glyceryl monostearate etc; hydrophilic polymers such as polyvinyl alcohol, polyvinylpyrrolidone, carboxymethyl cellulose sodium, methyl cellulose, hydroxymethyl cellulose, hydroxyethyl cellulose, hydroxypropyl cellulose.

Examples of the "isotonizing agents" include glucose, D-sorbitol, sodium chloride, glycerol, D-mannitol.

Examples of the "buffers" include buffer solutions of phosphates, acetates, carbonates, citrates.

Examples of the "soothing agents" include benzyl alcohol.

Examples of the "preservatives" include p-oxybenzoic acid esters, chlorobutanol, benzyl alcohol, phenethyl alcohol, dehydroacetic acid, sorbic acid.

Examples of the "antioxidants" include sulfites, ascorbic acid, α-tocopherol.

Examples of the "colorants" include food colors such as Food Color Yellow No. 5, Food Color Red No. 2, Food Color Blue No. 2; food lake colors, red ferric oxide.

Examples of the "sweetening agents" include saccharin sodium, dipotassium glycyrrhizinate, aspartame, stevia, thaumatin.

Examples of the "souring agents" include citric acid (citric anhydride), tartaric acid, malic acid.

Examples of the "bubbling agents" include sodium bicarbonate.

The "flavorings" may be synthetic substances or naturally occurring substances, and examples thereof include lemon, lime, orange, menthol, strawberry.

The compound of the present invention may be prepared as a preparation for oral administration in accordance with a commonly-known method, by, for example, compression-shaping with a carrier such as an excipient, a disintegrant, a binder, a lubricant, or the like, and subsequently coating the preparation as necessary by a commonly known method for the purpose of taste masking, enteric dissolution or sustained release. For an enteric preparation, an intermediate layer may be provided by a commonly known method between the enteric layer and the drug-containing layer for the purpose of separation of the two layers.

For preparing the compound of the present invention as an orally disintegrating tablet, available methods include a method in which a core containing crystalline cellulose and lactose is coated with the compound of the present invention and, where necessary, a basic inorganic salt, and then further coated with a coating layer containing an aqueous polymer to give a composition, which is coated with an enteric coating layer containing polyethylene glycol, further coated with an enteric coating layer containing triethyl citrate, still further coated with an enteric coating layer containing polyethylene glycol, and finally coated with mannitol to give fine granules, which are mixed with additives and shaped.

Examples of the above-mentioned "enteric coating layer" include a layer consisting of a mixture of one or more kinds from aqueous enteric polymer substrates such as cellulose acetate phthalate (CAP), hydroxypropylmethyl cellulose phthalate, hydroxymethyl cellulose acetate succinate, methacrylic acid copolymers (e.g., Eudragit L30D-55 (trade name; produced by Rohm), Colicoat MAE30DP (trade name; produced by BASF), Polyquid PA30 (trade name; produced by Sanyo Chemical) etc.), carboxymethylethyl cellulose, shellac; sustained-release substrates such as methacrylic acid copolymers (e.g., Eudragit NE30D (trade name), Eudragit RL30D (trade name), Eudragit RS30D (trade name), etc.); aqueous polymers; plasticizers such as triethyl citrate, polyethylene glycol, acetylated monoglycerides, triacetin, castor oil.

Examples of the above-mentioned "additive" include aqueous sugar alcohols (e.g., sorbitol, mannitol, maltitol, reduced starch saccharides, xylitol, reduced palatinose, erythritol, etc.), crystalline cellulose (e.g., Ceolas KG 801, Avicel PH 101, Avicel PH 102, Avicel PH 301, Avicel PH 302, Avicel RC-591 (crystalline cellulose-carmellose sodium) etc.), low-substituted hydroxypropyl cellulose (e.g., LH-22, LH-32, LH-23, LH-33 (Shin-Etsu Chemical), mixtures thereof etc.). Furthermore, binders, souring agents, bubbling agents, sweetening agents, flavorings, lubricants, colorants, stabilizers, excipients, disintegrants are also used.

The compound of the present invention may be used in combination with 1 to 3 other active ingredients.

Examples of the "other active ingredients" include anti-*Helicobacter pylori* active substances, imidazole compounds, bismuth salts, quinolone compounds, and so forth.

Examples of the "anti-Helicobacter pylori active substance" include penicillin antibiotic (e.g., amoxicillin, benzylpenicillin, piperacillin, mecillinam, ampicillin, temocillin, bacampicillin, aspoxicillin, sultamicillin, lenampicillin etc.), cephem antibiotic (e.g., cefixime, cefaclor etc.), macrolide antibiotic (e.g., erythromycin, clarithromycin, roxithromycin, rokitamycin, flurithromycin, telithromycin etc.), tetracycline antibiotic (e.g., tetracycline, minocycline, streptomycin etc.), aminoglycoside antibiotic (e.g., gentamicin, amikacin etc.), imipenem. Of these, penicillin antibiotic, macrolide antibiotic are preferable.

Examples of the "imidazole compounds" include metronidazole, miconazole.

Examples of the "bismuth salts" include bismuth acetate, bismuth citrate, bismuth subsalicylate.

Examples of the "quinolone compounds" include ofloxacin, ciploxacin.

For eradication of *Helicobacter pylori,* a compound (I) or a salt thereof of the present invention with antibiotic penicillin (e.g., amoxicillin) and antibiotic erythromycin (e.g., clarithromycin) is preferably used.

For the purpose of eradication of *Helicobacter pylori,* while the compound of the present invention has an anti-H. pylori action (bacteriostatic action or eradication action) by itself, it can enhance antibacterial action of other antibiotics based on the pH controlling action in the stomach, and also provides an assistant effect such as an eradication effect based on the action of the antibiotics to be used in combination.

The "other active ingredients" and the compound (I) or a salt thereof of the present invention may be mixed, prepared as a single pharmaceutical composition [e.g., tablets, powders, granules, capsules (including soft capsules), liquids, injectable preparations, suppositories, sustained-release preparations, etc.], in accordance with a commonly known method, and used in combination, and may also be prepared as separate preparations and administered to the same subject simultaneously or at a time interval.

In addition, the compound of the present invention may be used in combination with a gastric motility enhancer, a drug acting on lower esophageal sphincter (e.g., temporary lower esophageal sphincter relaxation suppressant etc.), ClC-2 channel opener (intestinal juice secretion enhancer), a histamine H2 receptor antagonist, an antacid, a sedative, a stomachic digestant or a non-steroidal anti-inflammatory drug (NSAID).

Examples of the "gastric motility enhancer" include domperidone, metoclopramide, mosapride, itopride, tegaserod.

Examples of the "a drug acting on lower esophageal sphincter" include GABA-B receptor agonists such as baclofen, an optically active form thereof, glutamine receptor antagonists.

Examples of the "ClC-2 channel opener (intestinal juice secretion enhancer)" include lubiprostone.

Examples of the "histamine H2 receptor antagonist" include cimetidine, ranitidine, famotidine, roxatidine, nizatidine, lafutidine.

Examples of the "antacid" include sodium hydrogen carbonate, aluminum hydroxide.

Examples of the "sedatives" include diazepam, chlordiazepoxide.

Examples of the "stomachic digestant" include gentiana, swertia japonica, diastase.

Examples of the "non-steroidal anti-inflammatory drug" include aspirin, indomethacin, ibuprofen, mefenamic acid, diclofenac, etodorac, piroxicam, celecoxib.

A gastric motility enhancer, a drug acting on lower esophageal sphincter, a C1C-2 channel opener (intestinal juice secretion enhancer), a histamine H2 receptor antagonist, an antacid, a sedative, a stomachic digestant or a non-steroidal anti-inflammatory drug and compound (I) or a salt thereof of the present invention may be mixed, prepared as a single pharmaceutical composition [e.g., tablets, powders, granules, capsules (including soft capsules), liquids, injections, suppositories, sustained-release preparations, etc.] according to a method known per se for combined use, or may also be prepared as separate preparations and administered to the same subject simultaneously or in a staggered manner.

The compound of the present invention may be used in combination with the following drugs.
(i) proton pump inhibitor, for example, omeprazole, esomeprazole, pantoprazole, rabeprazole, tenatoprazole, ilaprazole and lansoprazole;
(ii) oral antacid combination agent, for example, Maalox, Aludrox and Gaviscon;
(iii) mucous membrane protector, for example, polaprezinc, ecabe sodium, rebamipide, teprenone, cetraxate, sucralfate, chloropylline-copper and plaunotol;
(iv) antigastric agent, for example, anti-gastrin vaccine, itriglumide and Z-360;
(v) 5-HT₃ antagonist, for example, dolasetron, palonosetron, alosetron, azasetron, ramosetron, mitrazapine, granisetron, tropisetron, E-3620, ondansetron and indisetron;
(vi) 5-HT₄ agonist, for example, tegaserod, mosapride, cinitapride and oxtriptane;
(vii) laxative agent, for example, Trifyba, Fybogel, Konsyl, Isogel, Regulan, Celevac and Normacol;
(viii) GABA_{B} agonist, for example, baclofen and AZD-3355;
(ix) GABA_{B} antagonist, for example, GAS-360 and SGS-742;
(x) calcium channel blocker, for example, aranidipine, lacidipine, falodipine, azelnidipine, clinidipine, lomerizine, diltiazem, gallopamil, efonidipine, nisoldipine, amlodipine, lercanidipine, bevantolol, nicardipine, isradipine, benidipine, verapamil, nitrendipine, barnidipine, propafenone, manidipine, bepridil, nifedipine, nilvadipine, nimodipine and fasudil;
(xi) dopamine antagonist, for example, metoclopramide, domperidone and levosulpiride;
(xii) tachykinin (NK) antagonist, particularly, NK-3, NK-2 and NK-1 antagonist, for example, nepadutant, saredutant, talnetant, (αR,9R)-7-[3,5-bis(trifluoromethyl)benzyl]-8,9,10,11-tetrahydro-9-methyl-5-(4-methylphenyl)-7H-[1,4]diazocino[2,1-g][1,7]naphthyridine-6-13-dione (TAK-637), 5-[[(2R,3S)-2-[(1R)-1-[3,5-bis(trifluoromethyl)phenyl]ethoxy-3-(4-fluorophenyl)-4-morpholinyl]methyl]-1,2-dihydro-3H-1,2,4-triazol-3-one (MK-869), lanepitant, dapitant and 3-[[2-methoxy-5-(trifluoromethoxy)phenyl]methylamino]-2-phenylpiperidine(2S,3S);
(xiii) nitric monoxide synthase inhibitor, for example, GW-274150, tilarginine, P54, guanidioethyldisulfide and nitroflurbiprofen;
(xiv) vanilloid receptor 1 antagonist, for example, AMG-517 and GW-705498;
(xv) ghrelin agonist, for example, capromorelin and TZP-101;
(xvi) AchE release stimulant, for example, Z-338 and KW-5092.

The above-mentioned drugs (i)-(xvi) and compound (I) or a salt thereof of the present invention may be mixed, prepared as a single pharmaceutical composition [e.g., tablets, powders, granules, capsules (including soft capsules), liquids, injections, suppositories, sustained-release preparations, etc.] according to a method known *per se* for combined use, or may also be prepared as separate preparations and administered to the same subject simultaneously or in a staggered manner.

### Examples

The present invention is explained in detail in the following by referring to Reference Examples, Examples and Experimental Examples, which are not to be construed as limitative.

In the following Reference Examples and Examples, the "room temperature" generally means about 10°C to about 35°C, but it is not particularly strictly limited. The mixing ratio of liquids shows a volume ratio. Unless otherwise specified, "%" means weight %. The yield is in mol/mol%. Silica gel column chromatography was performed using silica gel 60 (0.063-0.200 mm) manufactured by MERCK, Fuji Silysia Chemical Ltd. Chromatorex (trade name) NH (described as basic silica gel column chromatography) or Purif-Pack manufactured by MORITEX (described as silica gel column chromatography or basic silica gel column chromatography). The melting point was measured using Yanagimoto trace melting point measurement apparatus or Buechi trace melting point measurement apparatus (B-545), and shown without amendment. For ¹H-NMR spectrum, tetramethylsilane was used as the internal standard, and Varian Gemini-200 (200MHz), Mercury-300 (300MHz) spectrometer, Bruker AVANCE AV300 (300MHz) and JNM-AL400 (400MHz) nuclear magnetic resonance apparatuses JEOL DATUM (JEOL DATUM LTD.) were used for the measurement. The following abbreviations are used for showing the measurement results.
s: singlet, d: doublet, dd: double doublet, ddd: triple doublet, dt: double triplet, t: triplet, q: quartet, dq: double quartet, m: multiplet, br: broad, brs: broad singlet, J: coupling constant, Hz: Hertz.

### Reference Example 1

### 4-bromo-5-(phenylthio)thiophene-2-carbaldehyde

To a solution of 4,5-dibromothiophene-2-carbaldehyde (1.0 g) in N,N-dimethylformamide (10 mL) were added potassium carbonate (665 mg) and thiophenol (448 mg) at room temperature. After stirring overnight at room temperature, water was added to the reaction mixture, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=19:1→7:1) to give the title compound as a pale-yellow oil (1.1 g, yield 99%).
¹H-NMR(CDCl₃)δ: 7.39-7.43(3H,m), 7.50-7.53(2H,m), 7.60(1H,s), 9.67(1H,s).

### Reference Example 2

### 4-bromo-5-[(3-methoxyphenyl)thio]thiophene-2-carbaldehyde

To a solution of 4,5-dibromothiophene-2-carbaldehyde (1.0 g) in N,N-dimethylformamide (10 mL) were added potassium carbonate (665 mg) and 3-methoxybenzenethiol (571 mg) at room temperature. After stirring overnight at room temperature, water was added to the reaction mixture, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate= 19:1→7:1) to give the crude title compound as a pale-yellow oil (1.30 g, quantitative).
¹H-NMR(CDCl₃)δ: 3.81(3H,s), 6.92-6.96(1H,m), 7.06-7.10(2H,m), 7.28-7.34(1H,m), 7.61(1H,s), 9.68(1H,s).

### Reference Example 3

### 4-bromo-5-[(3-fluorophenyl)thio]thiophene-2-carbaldehyde

To a solution of 4,5-dibromothiophene-2-carbaldehyde (1.0 g) in N,N-dimethylformamide (5 mL) were added potassium carbonate (665 mg) and 3-fluorobenzenethiol (522 mg) at room temperature. After stirring at room temperature for 6 hr, water was added to the reaction mixture, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=19:1→9:1) to give the title compound as a pale-yellow oil (1.17 g, yield 99%).
¹H-NMR(CDCl₃)δ: 7.03-7.15(2H,m), 7.20-7.25(1H,m), 7.32-7.39(1H,m), 7.65(1H,s), 9.73(1H,s).

### Reference Example 4

### 4-bromo-5-(pyridin-3-ylsulfonyl)thiophene-2-carbaldehyde

To a solution of 4,5-dibromothiophene-2-carbaldehyde (500 mg) in N,N-dimethylformamide (10 mL) were added pyridine (171 mg) and sodium pyridine-3-sulfinate (397 mg) at room temperature, and the mixture was stirred overnight at 70°C. After the reaction mixture was allowed to cool to room temperature, water was added to the reaction mixture, and the mixture was extracted with ethyl acetate. The extract was washed successively with water and saturated brine, dried over anhydrous sodium sulfate, and concentrated under reduced pressure. The residue was purified by silica gel column chromatography (eluent: hexane-ethyl acetate=4:1→2:1) to give the title compound as a pale-yellow crude solid (480 mg, yield 78%).
¹H-NMR(CDCl₃)δ: 7.51-7.55(1H,m), 7.65(1H,s), 8.33-8.37(1H,m), 8.87-8.89(1H,m), 9.28-9.29(1H,m), 9.90(1H,s).

### Reference Example 5

### methyl 4-bromo-3-methyl-5-(phenylthio)thiophene-2-carboxylate

To a solution of methyl 4,5-dibromo-3-methylthiophene-2-carboxylate (3.14 g) in N,N-dimethylformamide (31 mL) were added potassium carbonate (1.8 g) and thiophenol (1.21 g) at room temperature. After stirring at room temperature for 3 hr, water was added to the reaction mixture, and the mixture was stirred for 18 hr. The precipitate was collected by filtration, washed with water, and dried under reduced pressure to give the title compound as a colorless solid (3.16 g, yield 92%).
¹H-NMR(CDCl₃)δ: 2.56(3H,s), 3.82(3H,s), 7.31-7.39(3H,m), 7.39-7.46(2H,m).

### Reference Example 6

### 4-(2-fluoropyridin-3-yl)thiophene-2-carbaldehyde

A suspension of 4-bromothiophene-2-carbaldehyde (10.0 g), 2-fluoro-3-pyridineboronic acid (9.1 g), tetrakis(triphenylphosphine)palladium (0) (3.1 g) and sodium carbonate (13.7 g) in a mixed solvent of 1,2-dimethoxyethane (100 mL) and water (50 mL) was stirred at 80°C for 20 hr under a nitrogen atmosphere. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=2:1) to give the title compound as a pale-yellow solid (5.8 g, yield 53%).
¹H-NMR(CDCl₃)δ: 7.28-7.32(1H,m), 7.96-8.11(3H,m), 8.19-8.22(1H,m), 9.99(1H,d,J=1.2Hz).

### Reference Example 7

### tert-butyl {[4-(2-fluoropyridin-3-yl)-2-thienyl]methyl}methylcarbamate

To a solution of 4-(2-fluoropyridin-3-yl)thiophene-2-carbaldehyde (1.3 g) in tetrahydrofuran (15 mL) were added 40% methylamine-methanol solution (7 mL) and methanol (15 mL), and the mixture was stirred at room temperature for 12 hr. The reaction mixture was concentrated under reduced pressure. The residue was dissolved in methanol (15 mL), and sodium borohydride (1.6 g) was added at 0°C. The mixture was stirred at room temperature for 4 hr, and concentrated under reduced pressure. Water was added to the residue, and the mixture was extracted with ethyl acetate. The extract was washed successively with saturated aqueous sodium hydrogen carbonate solution, water and saturated brine, dried over anhydrous magnesium sulfate, and concentrated under reduced pressure. The residue was dissolved in ethyl acetate (10 mL), and di-tert-butyl bicarbonate (1.2 mL) was added. The mixture was stirred at room temperature for 12 hr, and concentrated under reduced pressure. The residue was purified by silica gel column chromatography (eluent: hexane-ethyl acetate=4:1) to give the title compound as a pale-yellow solid (1.1 g, yield in 2 steps 57%).
¹H-NMR(CDCl₃)δ: 1.51(9H,brs), 2.90(3H,brs), 4.56(2H,brs), 7.20-7.26(2H,m), 7.56-7.57(1H,m), 7.91-7.97(1H,m), 8.11-8.12(1H,m).

### Reference Example 8

### tert-butyl {[5-bromo-4-(2-fluoropyridin-3-yl)-2-thienyl]methyl}methylcarbamate

To a solution of tert-butyl {[4-(2-fluoropyridin-3-yl)-2-thienyl]methyl}methylcarbamate (267 mg) in N,N-dimethylformamide (5 mL) was added N-bromosuccinimide (163 mg), and the mixture was stirred at room temperature for 2 hr. Saturated aqueous sodium hydrogen carbonate solution was added to the reaction mixture, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=4:1) to give the title compound as a pale-yellow solid (260 mg, yield 79%).
¹H-NMR(CDCl₃) δ: 1.51 (9H,brs), 2.89(3H,s), 4.47 (2H,brs), 6.89-6.90(1H,m), 7.25-7.29(1H,m), 7.90-7.96(1H,m), 8.21-8.22(1H,m).

### Reference Example 9

### tert-butyl {[4-bromo-5-(phenylthio)-2-thienyl]methyl}methylcarbamate

4-Bromo-5-(phenylthio)thiophene-2-carbaldehyde (1.1 g) was dissolved in a mixed solvent of tetrahydrofuran (10 mL) and methanol (10 mL), and 40% methylamine-methanol solution (3.8 mL) was added. After stirring at room temperature for 1 hr, sodium borohydride (835 mg) was added, and the mixture was stirred overnight. The solvent was evaporated under reduced pressure, water was added to the residue, and the mixture was extracted with ethyl acetate. The extract was washed with saturated brine, dried over anhydrous sodium sulfate, and concentrated under reduced pressure. The residue was dissolved in tetrahydrofuran (10 mL), and di-tert-butyl bicarbonate (883 mg) was added at room temperature. After stirring for 30 min, the reaction mixture was concentrated under reduced pressure. The residue was purified by silica gel column chromatography (eluent: hexane-ethyl acetate=19:1→9:1) to give the title compound as a colorless oil (1.12 g, yield 73%). ¹H-NMR(CDCl₃) δ: 1.47 (9H,s), 2.87(3H,brs), 4.46(2H,brs), 6.91(1H,brs), 7.18-7.29(5H,m).

### Reference Example 10

### tert-butyl ({4-bromo-5-[(3-methoxyphenyl)thio]-2-thienyl}methyl)methylcarbamate

Crude 4-bromo-5-[(3-methoxyphenyl)thio]thiophene-2-carbaldehyde (1.3 g) was dissolved in a mixed solvent of tetrahydrofuran (5 mL) and methanol (5 mL), and 40% methylamine-methanol solution (3.8 mL) was added. After stirring at room temperature for 3 hr, sodium borohydride (840 mg) was added, and the mixture was further stirred for 3 hr. The solvent was evaporated under reduced pressure, water was added to the residue, and the mixture was extracted with ethyl acetate. The extract was washed with saturated brine, dried over anhydrous sodium sulfate, and concentrated under reduced pressure. The residue was dissolved in tetrahydrofuran (10 mL), and di-tert-butyl bicarbonate (888 mg) was added at room temperature. After stirring for 10 min, the reaction mixture was concentrated under reduced pressure. The residue was purified by silica gel column chromatography (eluent: hexane-ethyl acetate=19:1→6:1) to give the title compound as a colorless oil (1.28 g, yield in 2 steps 78%).
¹H-NMR(CDCl₃) δ: 1.47(9H,s), 2.87 (3H,brs), 3.75(3H,s), 4.45(2H,br), 6.71-6.79(3H,m), 6.91 (1H,brs), 7.13(1H,t,J=7.8Hz).

### Reference Example 11

### tert-butyl ({4-bromo-5-[(3-fluorophenyl)thio]-2-thienyl}methyl)methylcarbamate

4-Bromo-5-[(3-fluorophenyl)thio]thiophene-2-carbaldehyde (1.2 g) was dissolved in a mixed solvent of tetrahydrofuran (5 mL) and methanol (2 mL), and 40% methylamine-methanol solution (3.8 mL) was added. After stirring at room temperature for 3 hr, the excess methylamine was evaporated under reduced pressure. The residue was dissolved in methanol (5 mL), sodium borohydride (840 mg) was added, and the mixture was further stirred for 3 hr. The solvent was evaporated under reduced pressure, water was added to the residue, and the mixture was extracted with ethyl acetate. The extract was washed with saturated brine, dried over anhydrous sodium sulfate, and concentrated under reduced pressure. The residue was dissolved in tetrahydrofuran (10 mL), and di-tert-butyl bicarbonate (886 mg) was added at room temperature. After stirring for 10 min, the reaction mixture was concentrated under reduced pressure. The residue was purified by silica gel column chromatography (eluent: hexane-ethyl acetate=19:1→9:1) to give crude the title compound as a pale-yellow oil (1.63 g).
¹H-NMR(CDCl₃)δ: 1.48(9H,s), 2.89(3H,brs), 4.48(2H,brs), 6.81-6.89(2H,m), 6.94-6.97(2H,m), 7.19-7.26(1H,m).

### Reference Example 12

### tert-butyl {[4-bromo-5-(phenylsulfonyl)-2-thienyl]methyl}methylcarbamate

To a solution of tert-butyl {[4-bromo-5-(phenylthio)-2-thienyl]methyl}methylcarbamate (1.12 g) in ethyl acetate (15 mL) was added 3-chloroperbenzoic acid (1.86 g), and the mixture was stirred for 4 hr. The reaction mixture was treated with aqueous sodium thiosulfate solution, and the mixture was extracted with ethyl acetate. The extract was washed successively with water, saturated aqueous sodium hydrogen carbonate solution and saturated brine, dried over anhydrous sodium sulfate, and concentrated under reduced pressure. The residue was purified by silica gel column chromatography (eluent: hexane-ethyl acetate=19:1→7:1) to give the title compound as a pale-yellow oil (1.1 g, yield 91%).
¹H-NMR (CDCl₃) δ: 1.49(9H,s), 2.88(3H,s), 4.49(2H,s), 6.86(1H,brs),7.51-7.62(3H,m), 8.04-8.07(2H,m).

### Reference Example 13

### tert-butyl ({4-bromo-5-[(3-methoxyphenyl)sulfonyl]-2-thienyl}methyl)methylcarbamate

To a solution of tert-butyl ({4-bromo-5-[(3-methoxyphenyl)thio]-2-thienyl}methyl)methylcarbamate (1.28 g) in ethyl acetate (15 mL) was added 3-chloroperbenzoic acid (1.99 g), and the mixture was stirred for 2 hr. The reaction mixture was treated with aqueous sodium thiosulfate solution, and the mixture was extracted with ethyl acetate. The extract was washed successively with water, saturated aqueous sodium hydrogen carbonate solution and saturated brine, dried over anhydrous sodium sulfate, and concentrated under reduced pressure. The residue was purified by silica gel column chromatography (eluent: hexane-ethyl acetate=19:1→6:1) to give the title compound as a colorless oil (1.23 g, yield 90%).
¹H-NMR(CDCl₃) δ: 1.48(9H,s), 2.88(3H,s); 3.86(3H,s), 4.48(2H,brs), 6.86(1H,s), 7.11-7.15(1H,m), 7.43(1H,t,J=7.5Hz), 7.56-7.64(2H,m).

### Reference Example 14

### tert-butyl ({4-bromo-5-[(3-fluorophenyl)sulfonyl]-2-thienyl}methyl)methylcarbamate

To a solution of crude tert-butyl ({4-bromo-5-[(3-fluorophenyl)thio]-2-thienyl}methyl)methylcarbamate (1.63 g) in ethyl acetate (15 mL) was added 3-chloroperbenzoic acid (3.54 g), and the mixture was stirred overnight. The reaction mixture was treated with aqueous sodium thiosulfate solution, and the mixture was extracted with ethyl acetate. The extract was washed successively with water, saturated aqueous sodium hydrogen carbonate solution and,saturated brine, dried over anhydrous sodium sulfate, and concentrated under reduced pressure. The residue was purified by silica gel column chromatography (eluent: hexane-ethyl acetate=6:1→3:1) to give the title compound as a colorless oil (1.39 g, yield in 2 steps 81%).
¹H-NMR(CDCl₃)δ: 1.49(9H,s), 2.88(3H,s), 4.50(2H,brs), 6.88(1H,brs), 7.28-7.35(1H,m), 7.49-7.56(1H,m), 7.74-7.76(1H,m), 7.83-7.86(1H,m).

### Reference Example 15

### methyl 4-bromo-3-methyl-5-(phenylsulfonyl)thiophene-2-carboxylate

To a solution of methyl 4-bromo-3-methyl-5-(phenylthio)thiophene-2-carboxylate (1.72 g) in ethyl acetate (35 mL) was added 3-chloroperbenzoic acid (3.45 g), and the mixture was stirred for 18 hr. The reaction mixture was treated with aqueous sodium thiosulfate solution, and the mixture was extracted with ethyl acetate. The extract was washed successively with water, saturated aqueous sodium hydrogen carbonate solution and saturated brine, dried over anhydrous magnesium sulfate, and concentrated under reduced pressure. The residue was purified by silica gel column chromatography (eluent: hexane-ethyl acetate=19:1→1:1) to give the title compound as a colorless solid (1.72 g, yield 91%).
¹H-NMR(CDCl₃)δ: 2.50(3H,s), 3.91(3H,s), 7.50-7.60(2H,m), 7.60-7.70(1H,m), 8.04-8.11(2H,m).

### Reference Example 16

### 4-bromo-N,3-dimethyl-5-(phenylsulfonyl)thiophene-2-carboxamide

To methyl 4-bromo-3-methyl-5-(phenylsulfonyl)thiophene-2-carboxylate (470 mg) was added 40%'methylamine-methanol solution (7 mL). After stirring at room temperature for 3 hr, the mixture was concentrated under reduced pressure. The residue was purified by silica gel column chromatography (eluent: hexane-ethyl acetate=3:1→1:3) and basic silica gel column chromatography (eluent: hexane-ethyl acetate=7:3→35:65) to give the title compound as a colorless solid (451 mg, yield 96%).
¹H-NMR(CDCl₃)δ: 2.40(3H,s), 3.00(3H,d,J=4.9Hz), 5.90(1H,brs), 7.51-7.59(2H,m), 7.60-7.69(1H,m), 8.02-8.10(2H,m).

### Reference Example 17

### tert-butyl {[4-bromo-3-methyl-5-(phenylsulfonyl)-2-thienyl]methyl}methylcarbamate

To a suspension of lithium aluminum hydride (34 mg) in tetrahydrofuran (3 mL) was added aluminum chloride (40 mg) under ice-cooling under an argon atmosphere, and the mixture was stirred at room temperature for 30 min. A solution of 4-bromo-N,3-dimethyl-5-(phenylsulfonyl)thiophene-2-carboxamide (112 mg) in tetrahydrofuran (1 mL) was added to the reaction mixture, and the mixture was stirred for 2 hr under ice-cooling. 15% Aqueous sodium hydroxide solution (0.074 mL), water (0.074 mL) and 15% aqueous sodium hydroxide solution (0.222 mL) were added to the reaction mixture under ice-cooling, celite and magnesium sulfate were added, and the mixture was stirred at room temperature for 30 min. The insoluble material was filtered off, and washed with ethyl acetate, and the filtrate was concentrated under reduced pressure. The residue was dissolved in tetrahydrofuran (3 mL), and di-tert-butyl bicarbonate (131 mg) was added at room temperature. After stirring at room temperature for 18 hr, the reaction mixture was concentrated under reduced pressure. The residue was purified by silica gel column chromatography (eluent: hexane-ethyl acetate=85:15→45:55) to give the title compound as a colorless solid (74.9 mg, yield 54%).
¹H-NMR(CDCl₃)δ: 1.49(9H,s), 2.14(3H,s), 2.89(3H,s), 4.55(2H,brs), 7.48-7.65(3H,m), 8.02-8.08(2H,m).

### Reference Example 18

### 4-(2-fluorophenyl)-5-(pyridin-3-ylsulfonyl)thiophene-2-carbaldehyde

4-Bromo-5-(pyridin-3-ylsulfonyl)thiophene-2-carbaldehyde (397 mg), (2-fluorophenyl)boronic acid (202 mg), sodium hydrogen carbonate (305 mg) and tetrakis(triphenylphosphine)palladium (0) (139 mg) were suspended in a mixed solvent of 1,2-dimethoxyethane (10 mL) and water (4 mL), and the suspension was stirred at 105°C for 4 hr under a nitrogen atmosphere. After the reaction mixture was allowed to cool to room temperature, water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=7:1→3:1) to give the title compound as a pale-yellow solid (361 mg, yield 87%).
¹H-NMR(CDCl₃)δ: 7.00-7.06(1H,m), 7.24-7.31(2H,m), 7.38-7.51(2H,m), 7.64(1H,s) 7.74-7.78(1H,m), 8.61-8.62(1H,m),8.72-8.75(1H,m), 9.96(1H,s).

### Reference Example 19

### tert-butyl ({4-(2-fluoropyridin-3-yl)-5-[(3-methoxyphenyl)thio]-2-thienyl}methyl)methylcarbamate

tert-Butyl {[5-bromo-4-(2-fluoropyridin-3-yl)-2-thienyl]methyl}methylcarbamate (255 mg), 3-methoxythiophenol (0.10 mL), tris(dibenzylideneacetone)dipalladium (18 mg), 9,9-dimethyl-4,5-bis(diphenylphosphino)xanthine (23 mg) and N-ethyldiisopropylamine (0.22 mL) were stirred in toluene (8 mL) at 105°C for 12 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=4:1) to give the title compound as a yellow oil (287 mg, yield 97%).
¹H-NMR(CDCl₃)δ: 1.49(9H,s), 2.92(3H,s), 3.72(3H,s), 4.53(2H,brs), 6.60-6.61(1H,m), 6.65-6.69(2H,m), 7.09-7.19(3H,m), 7.80-7.86(1H,m), 8.15-8.16(1H,m).

### Reference Example 20

### tert-butyl {[4-(2-fluoropyridin-3-yl)-5-(phenylsulfonyl)-2-thienyl]methyl}methylcarbamate

tert-Butyl {[4-bromo-5-(phenylsulfonyl)-2-thienyl]methyl}methylcarbamate (282 mg), (2-fluoropyridin-3-yl)boronic acid (110 mg), sodium carbonate (161 mg) and tetrakis(triphenylphosphine)palladium (0) (73 mg) were suspended in a mixed solvent of 1,2-dimethoxyethane (10 mL) and water (4 mL), and the suspension was stirred at 105°C for 4 hr under a nitrogen atmosphere. After the reaction mixture was allowed to cool to room temperature, water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=9:1→4:1) and basic silica gel column chromatography (eluent: hexane-ethyl acetate=9:1→4:1) to give the title compound as a colorless oil (165 mg, yield 57%).
¹H-NMR(CDCl₃)δ: 1.49(9H,s), 2.92(3H,s), 4.54(2H,brs), 6.86(1H,brs), 7.26-7.39(3H,m), 7.49-7.54(3H,m), 7.90-7.99(1H,m), 8.23-8.25(1H,m).

### Reference Example 21

### tert-butyl {[4-(2-chloropyridin-3-yl)-5-(phenylsulfonyl)-2-thienyl]methyl}methylcarbamate

tert-Butyl {[4-bromo-5-(phenylsulfonyl)-2-thienyl]methyl}methylcarbamate (532 mg), (2-chloropyridin-3-yl)boronic acid (225 mg), sodium carbonate (303 mg) and tetrakis(triphenylphosphine)palladium (0) (138 mg) were suspended in a mixed solvent of 1,2-dimethoxyethane (10 mL) and water (4 mL), and the suspension was stirred at 105°C for 4 hr under a nitrogen atmosphere. After the reaction mixture was allowed to cool to room temperature, water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=9:1→3:1) to give the title compound as a red oil (433 mg, yield 76%).
¹H-NMR(CDCl₃)δ: 1.50(9H,s), 2.91(3H,s), 4.54(2H,br), 6.84(1H,s), 7.21-7.25(1H,m), 7.33-7.44(4H,m), 7.49-7.55(1H,m), 7.77-7.83(1H,m), 8.41-8.43(1H,m).

### Reference Example 22

### tert-butyl {[4-(2-cyanopyridin-3-yl)-5-(phenylsulfonyl)-2-thienyl]methyl}methylcarbamate

To a solution of tert-butyl {[4-(2-chloropyridin-3-yl)-5-(phenylsulfonyl)-2-thienyl]methyl}methylcarbamate (433 mg) in N,N-dimethylformamide (10 mL) were added zinc cyanide (213 mg) and tetrakis(triphenylphosphine)palladium (0) (105 mg), and the mixture was stirred at 105°C for 4 hr under a nitrogen atmosphere. After the reaction mixture was allowed to cool to room temperature, water and ethyl acetate were added, and the mixture was filtered through celite. The organic layer of the filtrate was washed successively with water and saturated brine, dried over anhydrous sodium sulfate, and concentrated under reduced pressure. The residue was purified by silica gel column chromatography (eluent: hexane-ethyl acetate=6:1→3:1) to give the title compound as a colorless oil (238 mg, yield 56%).
¹H-NMR(CDCl₃)δ: 1.50(9H,s), 2.92(3H,s), 4.58(2H,brs), 6.93(1H,s), 7.35-7.46(4H,m), 7.53-7.62(2H,m), 7.97-8.00(1H,m), 8.70-8.72(1H,m).

### Reference Example 23

### tert-butyl ({4-(2-fluorophenyl)-5-[(3-methoxyphenyl)sulfonyl]-2-thienyl}methyl)methylcarbamate

tert-Butyl ({4-bromo-5-[(3-methoxyphenyl)sulfonyl]-2-thienyl}methyl)methylcarbamate (324 mg), (2-fluorophenyl)boronic acid (114 mg), sodium carbonate (173 mg) and tetrakis(triphenylphosphine)palladium (0) (79 mg) were suspended in a mixed solvent of 1,2-dimethoxyethane (10 mL) and water (4 mL), and the suspension was stirred at 105°C for 3 hr under a nitrogen atmosphere. After the reaction mixture was allowed to cool to room temperature, water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=6:1→3:1) to give the title compound as a pale-yellow oil (308 mg, yield 92%).
¹H-NMR(CDCl₃) δ: 1.49(9H,s), 2.91(3H,s), 3.69(3H,s), 4.53(2H,brs), 6.83(1H,brs), 6.95-7.02(3H,m), 7.10-7.25(3H,m), 7.33-7.40(2H,m).

### Reference Example 24

### tert-Butyl ({5-[(3-fluorophenyl)sulfonyl]-4-(2-fluoropyridin-3-yl)-2-thienyl}methyl)methylcarbamate

tert-Butyl ({4-bromo-5-[(3-fluorophenyl)sulfonyl]-2-thienyl}methyl)methylcarbamate (300 mg), (2-fluoropyridin-3-yl)boronic acid (109 mg), sodium carbonate (164 mg) and tetrakis(triphenylphosphine)palladium (0) (75 mg) were suspended in a mixed solvent of 1,2-dimethoxyethane (10 mL) and water (4 mL), and the suspension was stirred overnight at 105°C under a nitrogen atmosphere. After the reaction mixture was allowed to cool to room temperature, water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=4:1→2:1) and basic silica gel column chromatography (eluent: hexane-ethyl acetate=3:1→1:1) to give the title compound as a colorless oil (140 mg, yield 45%).
¹H-NMR(CDCl₃)δ: 1.49(9H,s), 2.93(3H,s), 4.55(2H,brs), 6.88(1H,brs), 7.19-7.40(5H,m), 7.89-7.95(1H,m), 8.25-8.27(1H,m).

### Reference Example 25

### tert-butyl ({4-(2-chloropyridin-3-yl)-5-[(3-fluorophenyl)sulfonyl]-2-thienyl}methyl)methylcarbamate

tert-Butyl ({4-bromo-5-[(3-fluorophenyl)sulfonyl]-2-thienyl}methyl)methylcarbamate (557 mg), (2-chloropyridin-3-yl)boronic acid (227 mg), sodium carbonate (305 mg) and tetrakis(triphenylphosphine)palladium (0) (139 mg) were suspended in a mixed solvent of 1,2-dimethoxyethane (10 mL) and water (4 mL), and the suspension was stirred overnight at 105°C under a nitrogen atmosphere. After the reaction mixture was allowed to cool to room temperature, water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=7:1→3:1) and basic silica gel column chromatography (eluent: hexane-ethyl acetate=7:1→3:1) to give the title compound as a colorless oil (186 mg, yield 31%).
¹H-NMR(CDCl₃)δ: 1.50(9H,s), 2.92(3H,s), 4.56(2H,brs), 6.86(1H,s), 7.12-7.23(2H,m), 7.31-7.38(3H,m), 7.79-7.82(1H,m), 8.43-8.45(1H,m).

### Reference Example 26

### tert-butyl {[4-(2-fluorophenyl)-3-methyl-5-(phenylsulfonyl)-2-thienyl]methyl}methylcarbamate

tert-Butyl {[4-bromo-3-methyl-5-(phenylsulfonyl)-2-thienyl]methyl}methylcarbamate (138 mg), (2-fluorophenyl)boronic acid (126 mg), sodium carbonate (143 mg) and tetrakis(triphenylphosphine)palladium (0) (69 mg) were suspended in a mixed solvent of 1,2-dimethoxyethane (10 mL) and water (4 mL), and the suspension was stirred at 105°C for 18 hr under a nitrogen atmosphere. After the reaction mixture was allowed to cool to room temperature, water was added, and the mixture was extracted with ethyl acetate. The extract was washed with saturated brine, dried over anhydrous magnesium sulfate, and concentrated under reduced pressure. The residue was purified by silica gel column chromatography (eluent: hexane-ethyl acetate=85:15→1:1) to give the title compound as a colorless solid (143 mg, yield 100%).
¹H-NMR(CDCl₃)δ: 1.50(9H,s), 1.84(3H,s), 2.94(3H,s), 4.56(2H,brs), 6.94(1H,t,J=8.9Hz), 7.15-7.23(2H,m), 7.27-7.34(2H,m), 7.34-7.53(4H,m).

### Reference Example 27

### tert-butyl methyl{[3-methyl-4-(2-methylphenyl)-5-(phenylsulfonyl)-2-thienyl]methyl}carbamate

tert-Butyl {[4-bromo-3-methyl-5-(phenylsulfonyl)-2-thienyl]methyl}methylcarbamate (138 mg), (2-methylphenyl)boronic acid (122 mg), sodium carbonate (143 mg) and tetrakis(triphenylphosphine)palladium (0) (69 mg) were suspended in a mixed solvent of 1,2-dimethoxyethane (10 mL) and water (4 mL), and the suspension was stirred at 105°C for 18 hr under a nitrogen atmosphere. After the reaction mixture was allowed to cool to room temperature, water was added, and the mixture was extracted with ethyl acetate. The extract was washed with saturated brine, dried over anhydrous magnesium sulfate, and concentrated under reduced pressure. The residue was purified by silica gel column chromatography (eluent: hexane-ethyl acetate=19:1→6:4) to give the title compound as a colorless oil (138 mg, yield 98%).
¹H-NMR(CDCl₃)δ: 1.51(9H,s), 1.53(3H,s), 1.75(3H,s), 2.93(3H,s), 4.57(2H,brs), 6.86(1H,d,J=6.4Hz) 7.06-7.21(2H,m), 7.23-7.33(3H,m), 7.34-7.41(2H,m), 7.43-7.52(1H,m).

### Reference Example 28

### tert-butyl {[4-(2-fluoropyridin-3-yl)-3-methyl-5-(phenylsulfonyl)-2-thienyl]methyl}methylcarbamate

tert-Butyl {[4-bromo-3-methyl-5-(phenylsulfonyl)-2-thienyl]methyl}methylcarbamate (70 mg), (2-fluoropyridin-3-yl)boronic acid (26 mg), sodium carbonate (39 mg) and tetrakis(triphenylphosphine)palladium (0) (18 mg) were suspended in a mixed solvent of 1,2-dimethoxyethane (10 mL) and water (4 mL), and the suspension was stirred at 105°C for 18 hr under a nitrogen atmosphere. After the reaction mixture was allowed to cool to room temperature, water was added, and the mixture was extracted with ethyl acetate. The extract was washed with saturated brine, dried over anhydrous magnesium sulfate, and concentrated under reduced pressure. The residue was purified by silica gel column chromatography (eluent: hexane-ethyl acetate=10:0→1:1) to give the title compound as a pale-yellow solid (51 mg, yield 70%).
¹H-NMR(CDCl₃)δ: 1.50(9H,s), 1.86(3H,s), 2.94(3H,s), 4.56(2H,brs), 7.28-7.45(5H,m), 7.47-7.55(1H,m), 7.75-7.85(1H,m), 8.25-8.30(1H,m).

### Reference Example 29

### tert-butyl {[4-(2-chloropyridin-3-yl)-3-methyl-5-(phenylsulfonyl)-2-thienyl]methyl}methylcarbamate

tert-Butyl {[4-bromo-3-methyl-5-(phenylsulfonyl)-2-thienyl]methyl}methylcarbamate (230 mg), (2-chloropyridin-3-yl)boronic acid (236 mg), sodium carbonate (238 mg) and tetrakis(triphenylphosphine)palladium (0) (116 mg) were suspended in a mixed solvent of 1,2-dimethoxyethane (10 mL) and water (4 mL), and the suspension was stirred at 105°C for 18 hr under a nitrogen atmosphere. After the reaction mixture was allowed to cool to room temperature, water was added, and the mixture was extracted with ethyl acetate. The extract was washed with saturated brine, dried over anhydrous magnesium sulfate, and concentrated under reduced pressure. The residue was purified by silica gel column chromatography (eluent: hexane-ethyl acetate=75:25→45:55) to give the title compound as a colorless oil (55 mg, yield 22%).
¹H-NMR(CDCl₃)δ: 1.51(9H,s), 1.86(3H,s), 2.93(3H,s), 4.57(2H,brs), 7.31-7.44(5H,m), 7.48-7.57(1H,m), 7.73(1H,dd,J=7.6,1.9Hz), 8.46(1H,dd,J=4.9,1.9Hz).

### Reference Example 30

### tert-butyl ({4-(2-fluoropyridin-3-yl)-5-[(3-methoxyphenyl)sulfonyl]-2-thienyl}methyl)methylcarbamate

To a solution of tert-butyl ({4-(2-fluoropyridin-3-yl)-5-[(3-methoxyphenyl)thio]-2-thienyl}methyl)methylcarbamate (281 mg) in acetic acid (5 mL) was added 3-chloroperbenzoic acid (728 mg), and the mixture was stirred at room temperature for 3 hr. The reaction mixture was concentrated under reduced pressure. The residue was basified with saturated aqueous sodium hydrogen carbonate solution, and the mixture was extracted with ethyl acetate. The extract was washed successively with saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=2:1) to give the title compound as a yellow oil (232 mg, yield 77%).
¹H-NMR(CDCl₃)δ: 1.49(9H,s), 2.92(3H,s), 3.73(3H,s), 4.53(2H,brs), 6.86(1H,brs), 6.99-7.11(3H,m), 7.24-7.30(2H,m), 7.92-7.97(1H,m), 8.23-8.25(1H,m).

### Reference Example 31

### 1-(2-fluoropyridin-3-yl)ethanol

To a solution of diisopropylamine (54.7 g) in tetrahydrofuran (1.0 L) was added dropwise 1.6 mol/L n-butyllithium-hexane solution (355 mL) over 1 hr under ice-cooling under a nitrogen atmosphere, and the mixture was stirred at the same temperature for 1 hr. The reaction mixture was cooled to -78°C, a solution of 2-fluoropyridine (50.0 g) in tetrahydrofuran (100 mL) was added dropwise over 1 hr, and the mixture was stirred at the same temperature for 2 hr. A solution of acetaldehyde (28.4 g) in tetrahydrofuran (100 mL) was added dropwise over 1 hr at the same temperature to the obtained reaction mixture. While the mixture was allowed to warm to room temperature, the mixture was stirred for 20 hr. Water was added to the reaction mixture, and the mixture was extracted with ethyl acetate. The extract was dried over anhydrous magnesium sulfate, and concentrated under reduced pressure. The residue was purified by silica gel column chromatography (eluent: hexane-ethyl acetate=1:1) to give the title compound as a red oil (39.9 g, yield 56%).
¹H-NMR(CDCl₃)δ: 1.52(3H,d,J=6.6Hz), 2.50(1H,brs), 5.14(1H,q,J=6.6Hz), 7.21(1H,ddd,J=7.3,4.9,1.7Hz), 7.97(1H,dddd,J=9.8,7.3,1.9,0.8Hz), 8.09(1H,ddd,J=4.9,1.9,1.1Hz).

### Reference Example 32

### 1-(2-fluoropyridin-3-yl)ethanone

To a solution of 1-(2-fluoropyridin-3-yl)ethanol (39.9 g) in dimethylsulfoxide (200 mL) was added triethylamine (200 mL) under ice-cooling, and then added slowly dropwise a suspension of sulfur trioxide pyridine complex (90.0 g) in dimethylsulfoxide (200 mL) at the same temperature. The reaction mixture was allowed to warm to room temperature, and stirred at the same temperature for 20 hr. Water was added to the obtained reaction mixture, and the mixture was extracted with ethyl acetate. The extract was dried over anhydrous magnesium sulfate, and concentrated under reduced pressure. The residue was purified by silica gel column chromatography (eluent: hexane-ethyl acetate=1:2) to give the title compound as a red oil (33.8 g, yield 86%).
¹H-NMR(CDCl₃)δ: 2.69(3H,dd,J=4.9,1.1Hz), 7.34(1H,ddd,J=7.6,4.9,2.3Hz), 8.34(1H,dddd,J=9.5,7.6,2.3,1.1Hz), 8.40(1H,ddd,J=4.9,2.3,1.1Hz).

### Reference Example 33

### 2-bromo-1-(2-fluorophenyl)ethanone

To a solution of 1-(2-fluorophenyl)ethanone (15.1 g) in acetic acid (150 mL) was added bromine (5.8 mL). The mixture was stirred at room temperature for 2 hr, and concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous magnesium sulfate, and concentrated under reduced pressure to give the title compound as a pale-yellow oil (22.91 g, yield 97%).
¹H-NMR(CDCl₃)δ: 4.53(2H,d,J=2.4Hz), 7.13-7.20(1H,m), 7.27-7.30(1H,m), 7.54-7.61(1H,m), 7.91-7.96(1H,m).

### Reference Example 34

### 2-bromo-1-(2-fluoropyridin-3-yl)ethanone hydrobromide

1-(2-Fluoropyridin-3-yl)ethanone (3.0 g) was dissolved in 25% hydrogen bromide-acetic acid solution (10 mL), bromine (1.2 mL) was added dropwise at 0°C. The reaction mixture was allowed to warm to room temperature, and the mixture was stirred at the same temperature for 2 hr. The obtained reaction mixture was concentrated under reduced pressure, and the obtained solid was washed with ethyl acetate to give the title compound as a brown powder (3.5 g, yield 54%).
¹H-NMR(CDCl₃)δ: 4.90(2H,d,J=1.9Hz), 7.57(1H,ddd,J=7.6,4.9,2.3Hz), 8.43-8.50(1H,m), 8.48-8.53(1H,m), 10.18(1H,brs).

### Reference Example 35

### 1-(2-fluorophenyl)-2-(phenylthio)ethanone

To a suspension of 2-bromo-1-(2-fluorophenyl)ethanone (3.0 g) and potassium carbonate (2.0 g) in ethanol (30 mL) was added dropwise thiophenol (1.4 mL) at 0°C, and the mixture was stirred at room temperature for 8 hr. The insoluble material was filtered off, and the filtrate was concentrated under reduced pressure. Water was added to the residue, and the mixture was extracted with ethyl acetate. The extract was washed successively with saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=15:1) to give the title compound as a pale-yellow solid (2.2 g, yield 64%). ¹H-NMR(CDCl₃)δ: 4.26(2H,d,J=2.1Hz), 7.10-7.35(7H,m), 7.50-7.57(1H,m), 7.79-7.85(1H,m).

### Reference Example 36

### 2-bromo-1-(2-fluorophenyl)-2-(phenylthio)ethanone

To a solution of 1-(2-fluorophenyl)-2-(phenylthio)ethanone (413 mg) in ethyl acetate (6 mL) were added copper(II) bromide (419 mg) and 25% hydrogen bromide-acetic acid solution (2 drops), and the mixture was stirred at 80°C for 2 hr. The reaction mixture was allowed to cool to room temperature, and concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous magnesium sulfate, and concentrated under reduced pressure to give the title compound as a brown oil (503 mg, yield 92%).
¹H-NMR(CDCl₃)δ: 6.61(1H,d,J=3.0Hz), 7.08-7.63(8H,m), 7.88-7.96(1H,m).

### Reference Example 37

### (1,3-dioxo-1,3-dihydro-2H-isoindol-2-yl)acetonitrile

To a solution of bromoacetonitrile (22 g) in N,N-dimethylformamide (200 mL) was added potassium phthalimide (34 g) under ice-cooling, and the mixture was stirred at room temperature for 3 hr. Water was added to the reaction mixture, and the mixture was extracted with ethyl acetate. The extract was washed successively with saturated aqueous sodium hydrogen carbonate solution, water and saturated brine, dried over anhydrous magnesium sulfate, and concentrated under reduced pressure. The residue was recrystallized from ethanol to give the title compound as white crystals (27 g, yield 80%).
¹H-NMR(CDCl₃)δ: 4.59(2H,s), 7.79-7.85(2H,m), 7.790-7.97(2H,m).

### Reference Example 38

### 2-(1,3-dioxo-1,3-dihydro-2H-isoindol-2-yl)ethanethioamide

To a mixture of (1,3-dioxo-1,3-dihydro-2H-isoindol-2-yl)acetonitrile (15 g), 4 mol/L hydrogen chloride-ethyl acetate solution (40 mL) and tetrahydrofuran (50 mL) was added O,O-diethyl dithiophosphate (15 mL), and the mixture was stirred at room temperature for 5 hr. Water was added to the reaction mixture, and the mixture was extracted with ethyl acetate and tetrahydrofuran. The extract was washed successively with water (twice), saturated brine and saturated aqueous sodium hydrogen carbonate solution, dried over anhydrous magnesium sulfate, and concentrated under reduced pressure. The residue was recrystallized from ethanol to give the title compound as white crystals (9.0 g, yield 51%).
¹H-NMR(CDCl₃)δ: 4.69(2H,s), 7.25(1H,brs), 7.47(1H,brs), 7.75-7.79(2H,m), 7.88-7.92(2H,s).

### Reference Example 39

### tert-butyl (2-amino-2-oxoethyl)methylcarbamate

To 40% methylamine-methanol solution (50 mL) was added slowly 2-chloroacetamide (18.3 g) at 0°C, and the mixture was stirred at room temperature for 18 hr. The reaction mixture was concentrated under reduced pressure, and the residue was washed with ethanol to give a solid (26.5 g). The solid was dissolved in a mixed solvent of methanol (200 mL) and water (100 mL), and triethylamine (30 mL) was added dropwise at 0°C. The obtained solution was stirred at room temperature for 30 min, and di-tert-butyl bicarbonate (43.7 g) was added dropwise at 0°C. After stirring at room temperature for 3 hr, the mixture was concentrated under reduced pressure. Water was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was recrystallized from ethyl acetate-hexane to give the title compound as white crystals (13.8 g, yield 38%)
¹H-NMR(CDCl₃)δ: 1.47(9H,s), 2.96(3H,s), 3.86(2H,s), 5.70(1H,brs), 6.08(1H,brs).

### Reference Example 40

### tert-butyl (2-amino-2-thioxoethyl)methylcarbamate

To a suspension of tert-butyl (2-amino-2-oxoethyl)methylcarbamate (2.0 g) in tetrahydrofuran (30 mL) was added the Lawesson's reagent (2.5 g) at 0°C, and the mixture was stirred at room temperature for 12 hr. The reaction mixture was concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was recrystallized from ethyl acetate-hexane to give the title compound as white crystals (1.6 g, yield 71%).
¹H-NMR(CDCl₃)δ: 1.47(9H,s), 2.96(3H,s), 7.27(2H,s), 7.48-8.08(2H,m).

### Reference Example 41

### 2-{[4-(2-fluorophenyl)-5-(phenylthio)-1,3-thiazol-2-yl]methyl}-1H-isoindole-1,3(2H)-dione

To a solution of 2-bromo-1-(2-fluorophenyl)-2-(phenylthio)ethanone (496 mg) in N,N-dimethylformamide (5 mL) was added 2-(1,3-dioxo-1,3-dihydro-2H-isoindol-2-yl)ethanethioamide (357 mg), and the mixture was stirred at room temperature for 12 hr. Aqueous sodium hydrogen carbonate solution was added to the reaction mixture, and the mixture was extracted with ethyl acetate. The extract was washed successively with saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=4:1→2:1) to give the title compound as a pale-yellow oil (351 mg, yield 51%).
¹H-NMR(CDCl₃)δ: 5.19(2H,s), 7.08-7.24(7H,m), 7.31-7.39(1H,m), 7.43-7.49(1H,m), 7.72-7.77(2H,m), 7.86-7.92(2H,m).

### Reference Example 42

### tert-butyl {[4-(2-fluorophenyl)-1,3-thiazol-2-yl]methyl}methylcarbamate

To a solution of 2-bromo-1-(2-fluorophenyl)ethanone (2.2 g) in N,N-dimethylformamide (20 mL) was added tert-butyl (2-amino-2-thioxoethyl)methylcarbamate (2.1 g), and the mixture was stirred at room temperature for 2 days. Aqueous sodium hydrogen carbonate solution was added to the reaction mixture, and the mixture was extracted with ethyl acetate. The extract was washed successively with saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=5:1) to give the title compound as a pale-yellow oil (2.3 g, yield 70%).
¹H-NMR(CDCl₃)δ: 1.50(9H,s), 3.01(3H,brs), 4.72-4.76(2H,m), 7.10-7.32(3H,m), 7.70(1H,d,J=2.1Hz), 8.14-8.20(1H,m).

### Reference Example 43

### tert-butyl {[4-(2-fluoropyridin-3-yl)-1,3-thiazol-2-yl]methyl}methylcarbamate

To a solution of 2-bromo-1-(2-fluoropyridin-3-yl)ethanone hydrobromide (3.1 g) in N,N-dimethylformamide (40 mL) was added tert-butyl (2-amino-2-thioxoethyl)methylcarbamate (2.1 g), and the mixture was stirred at room temperature for 2 days. Aqueous sodium hydrogen carbonate solution was added to the reaction mixture, and the mixture was extracted with ethyl acetate. The extract was washed successively with saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=4:1) to give the title compound as a pale-yellow oil (2.8 g, yield 82%).
¹H-NMR(CDCl₃)δ: 1.50(9H,s), 3.01(3H,brs), 4.71(2H,brs), 7.26-7.31(1H,m), 7.80(1H,d,J=2.4Hz), 8.13-8.15(1H,m), 8.59-8.65(1H,m).

### Reference Example 44

### tert-butyl {[5-bromo-4-(2-fluorophenyl)-1,3-thiazol-2-yl]methyl}methylcarbamate

To a solution of tert-butyl {[4-(2-fluorophenyl)-1,3-thiazol-2-yl]methyl}methylcarbamate (2.3 g) in N,N-dimethylformamide (20 mL) was added N-bromosuccinimide (2.7 g) at 0°C, and the mixture was stirred at room temperature for 3 hr. Water was added to the reaction mixture, and the mixture was extracted with ethyl acetate. The extract was washed successively with saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=4:1) to give the title compound as a pale-yellow solid (2.5 g, yield 88%).
¹H-NMR(CDCl₃)δ: 1.52(9H,s), 2.97(3H,brs), 4.15(2H,brs), 7.14-7.24(2H,m), 7.37-7.44(1H,m), 7.49-7.54(1H,m).

### Reference Example 45

### tert-butyl {[5-bromo-4-(2-fluoropyridin-3-yl)-1,3-thiazol-2-yl]methyl}methylcarbamate

To a solution of tert-butyl {[4-(2-fluoropyridin-3-yl)-1,3-thiazol-2-yl]methyl}methylcarbamate (1.5 g) in N,N-dimethylformamide (20 mL) was added N-bromosuccinimide (2.5 g), and the mixture was stirred at 50°C for 12 hr. The reaction mixture was allowed to cool to room temperature, saturated aqueous sodium hydrogen carbonate solution was added, and the mixture was extracted with ethyl acetate. The extract was washed successively with saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=3:1) to give the title compound as a pale-yellow solid (0.97 g, yield 51%).
¹H-NMR(CDCl₃)δ: 1.51(9H,s), 2.98(3H,s), 4.65(2H,s), 7.27-7.32(1H,m), 7.96-8.02(1H,m), 8.28-7.30(1H,m).

### Reference Example 46

### 1-[4-(2-fluorophenyl)-5-(phenylthio)-1,3-thiazol-2-yl]methanamine

To a solution of 2-{[4-(2-fluorophenyl)-5-(phenylthio)-1,3-thiazol-2-yl]methyl}-1H-isoindole-1,3(2H)-dione (345 mg) in ethanol (5 mL) was added hydrazine monohydrate (0.05 mL), and the mixture was stirred at 70°C for 3 hr. The reaction mixture was allowed to cool to room temperature, saturated aqueous sodium hydrogen carbonate solution was added, and the mixture was extracted with ethyl acetate. The extract was washed successively with saturated aqueous sodium hydrogen carbonate solution, water and saturated brine, dried over anhydrous sodium sulfate, and concentrated under reduced pressure to give the title compound as a yellow oil (209 mg, yield 86%).
¹H-NMR(CDCl₃)δ: 4.20(2H,s), 7.10-7.27(7H,m), 7.33-7.40(1H,m), 7.44-7.49(1H,m),2H not detected.

### Reference Example 47

### tert-butyl {[4-(2-fluorophenyl)-5-(phenylthio)-1,3-thiazol-2-yl]methyl}carbamate

To a solution of 1-[4-(2-fluorophenyl)-5-(phenylthio)-1,3-thiazol-2-yl]methanamine (208 mg) in ethyl acetate (5 mL) was added di-tert-butyl bicarbonate (0.19 mL), and the mixture was stirred at room temperature for 2 days. Saturated aqueous sodium hydrogen carbonate solution was added to the reaction mixture, and the mixture was extracted with ethyl acetate. The extract was washed successively with saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=4:1) to give the title compound as a yellow oil (280 mg, quantitative).
¹H-NMR(CDCl₃)δ: 1.46(9H,s), 4.62(2H,brd,J=5.7Hz), 5.25(1H,brs), 7.10-7.26(7H,m), 7.33-7.41(1H, 7.43-7.48(1H,m).

### Reference Example 48

### tert-butyl {[4-(2-fluorophenyl)-5-(phenylthio)-1,3-thiazol-2-yl]methyl}methylcarbamate

Sodium hydride (79 mg) was washed twice with hexane, and suspended in N,N-dimethylformamide (8 mL). A solution of tert-butyl {[4-(2-fluorophenyl)-5-(phenylthio)-1,3-thiazol-2-yl]methyl}carbamate (642 mg) in N,N-dimethylformamide (5 mL) was added dropwise at 0°C to the suspension, and the mixture was stirred at the same temperature for 15 min. Methyl iodide (0.13 mL) was added at 0°C to the mixture, and the mixture was stirred at the same temperature for 15 min. Water was added to the reaction mixture, and the mixture was extracted with ethyl acetate. The extract was washed successively with saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=4:1) to give the title compound as a yellow oil (364 mg, yield 55%).
¹H-NMR(CDCl₃)δ: 1.46(9H,brs), 3.00(3H,brs), 4.65-4.71(2H,m), 7.11-7.27(7H,m), 7.34-7.38(1H,m), 7.44-7.49(1H,m).

### Reference Example 49

### tert-butyl ({4-(2-fluorophenyl)-5-[(3-methoxyphenyl)thio]-1,3-thiazol-2-yl}methyl)methylcarbamate

tert-Butyl {[5-bromo-4-(2-fluorophenyl)-1,3-thiazol-2-yl]methyl}methylcarbamate (224 mg), 3-methoxythiophenol (90 mg), tris(dibenzylideneacetone)dipalladium (13 mg), 9,9-dimethyl-4,5-bis(diphenylphosphino)xanthine (16 mg) and N-ethyldiisopropylamine (0.20 mL) were stirred in toluene (6 mL) at 110°C for 8 hr. The reaction mixture was allowed to cool to room temperature, saturated aqueous sodium hydrogen carbonate solution was added, and the mixture was extracted with ethyl acetate. The extract was washed successively with saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=4:1) to give the title compound as a yellow oil (234 mg, yield 91%).
¹H-NMR (CDCl₃)δ: 1.47(9H,s), 3.00(3H,brs), 3.73(3H,s), 4.66-4.72(2H,m), 6.70-6.78(3H,m), 7.11-7.20(3H,m), 7.36-7.38(1H,m), 7.44-7.49(1H,m).

### Reference Example 50

### tert-butyl {[4-(2-fluoropyridin-3-yl)-5-(phenylthio)-1,3-thiazol-2-yl]methyl}methylcarbamate

tert-Butyl {[5-bromo-4-(2-fluoropyridin-3-yl)-1,3-thiazol-2-yl]methyl}methylcarbamate (305 mg), thiophenol (0.09 mL), tris(dibenzylideneacetone)dipalladium (21 mg), 9,9-dimethyl-4,5-bis(diphenylphosphino)xanthine (27 mg) and N-ethyldiisopropylamine (0.26 mL) were stirred in toluene (10 mL) at 105°C for 12 hr. The reaction mixture was allowed to cool to room temperature, saturated aqueous sodium hydrogen carbonate solution was added, and the mixture was extracted with ethyl acetate. The extract was washed successively with saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=3:1) to give the title compound as a yellow oil (316 mg, yield 96%).
¹H-NMR(CDCl₃):δ 1.46(9H,s), 2.99(3H,brs), 4.67(2H,brd,J=15.6Hz), 7.17-7.28(6H,m), 7.88-7.94(1H,m) 8.24-8.25(1H,m).

### Reference Example 51

### tert-butyl ({4-(2-fluoropyridin-3-yl)-5-[(3-methoxyphenyl)thio]-1,3-thiazol-2-yl}methyl)methylcarbamate

tert-Butyl {[5-bromo-4-(2-fluoropyridin-3-yl)-1,3-thiazol-2-yl]methyl}methylcarbamate (253 mg), 3-methoxythiophenol (107 mg), tris(dibenzylideneacetone)dipalladium (17 mg), 9,9-dimethyl-4,5-bis(diphenylphosphino)xanthine (22 mg) and N-ethyldiisopropylamine (0.22 mL) were stirred in toluene (8 mL) at 105°C for 12 hr. The reaction mixture was allowed to cool to room temperature, saturated aqueous sodium hydrogen carbonate solution was added, and the mixture was extracted with ethyl acetate. The extract was washed successively with saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=2:1) to give the title compound as a yellow oil (285 mg, yield 98%).
¹H-NMR(CDCl₃)δ: 1.47(9H,brs), 2.99(3H,brs), 3.74(3H,s), 4.68(2H,brd,J=13.2Hz), 6.71-6.77(3H,s), 7.13-7.25(2H,m), 7.88-7.94(1H,m), 8.24-8.25(1H,m).

### Reference Example 52

### tert-butyl {[5-[(3-chlorophenyl)thio]-4-(2-fluoropyridin-3-yl)-1,3-thiazol-2-yl]methyl}methylcarbamate

tert-Butyl {[5-bromo-4-(2-fluoropyridin-3-yl)-1,3-thiazol-2-yl]methyl}methylcarbamate (226 mg), 3-chlorothiophenol (124 mg), tris(dibenzylideneacetone)dipalladium (26 mg), 9,9-dimethyl-4,5-bis(diphenylphosphino)xanthine (33 mg) and N-ethyldiisopropylamine (0.20 mL) were stirred in toluene (3 mL) at 140°C for 1 hr, while irradiating microwave. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=3:1) to give the title compound as a yellow oil (170 mg, yield 65%).
¹H-NMR(CDl₃)δ: 1.48(9H,s), 3.01(3H,brs), 4.67-4.71(2H,m), 7.02-7.04(1H,m), 7.13-7.21(2H,m), 7.23-7.27(2H,m), 7.87-7.93(1H,m), 8.25-8.27(1H,m).

### Reference Example 53

### tert-butyl {[4-(2-fluorophenyl)-5-(phenylsulfonyl)-1,3-thiazol-2-yl]methyl}methylcarbamate

To a solution of tert-butyl {[4-(2-fluorophenyl)-5-(phenylthio)-1,3-thiazol-2-yl]methyl}methylcarbamate (360 mg) in acetic acid (4 mL) was added 3-chloroperbenzoic acid (810 mg), and the mixture was stirred at room temperature for 2 hr. The reaction mixture was basified with aqueous sodium thiosulfate solution and 8 mol/L aqueous sodium hydroxide solution, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=3:1) to give the title compound as a yellow oil (251 mg, yield 65%).
¹H-NMR(CDCl₃)δ: 1.48-1.52(9H,m), 3.00(3H,s), 4.64-4.69(2H,m), 6.99-7.05(1H,m), 7.34-7.51(4H,m), 7.53-7.58(3H,m).

### Reference Example 54

### tert-butyl ({4-(2-fluorophenyl)-5-[(3-methoxyphenyl)sulfonyl]-1,3-thiazol-2-yl}methyl)methylcarbamate

To a solution of tert-butyl ({4-(2-fluorophenyl)-5-[(3-methoxyphenyl)thio]-1,3-thiazol-2-yl}methyl)methylcarbamate (232 mg) in acetic acid (3 mL) was added 3-chloroperbenzoic acid (509 mg), and the mixture was stirred at room temperature for 4 hr. The reaction mixture was basified with aqueous sodium thiosulfate solution and 8 mol/L aqueous sodium hydroxide solution, and the mixture was extracted with ethyl acetate. The extract was washed successively with saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=3:1) to give the title compound as a yellow oil (182 mg, yield 74%).
¹H-NMR(CDCl₃)δ: 1.47-1.52(9H,m), 3.00(3H,s), 3.72(3H,s), 4.64-4.96(2H,m), 7.02-7.26(4H,m), 7.18-7.31(2H,m), 7.41(2H,brs).

### Reference Example 55

### tert-butyl {[4-(2-fluoropyridin-3-yl)-5-(phenylsulfonyl)-1,3-thiazol-2-yl]methyl}methylcarbamate

To a solution of tert-butyl {[4-(2-fluoropyridin-3-yl)-5-(phenylthio)-1,3-thiazol-2-yl]methyl}methylcarbamate (315 mg) in acetic acid (5 mL) was added 3-chloroperbenzoic acid (713 mg), and the mixture was stirred at room temperature for 5 hr. The reaction mixture was concentrated under reduced pressure. The residue was basified with saturated aqueous sodium hydrogen carbonate solution, and the mixture was extracted with ethyl acetate. The extract was washed successively with saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=2:1) to give the title compound as a yellow oil (212 mg, yield 63%).
¹H-NMR(CDCl₃)δ: 1.47-1.51(9H,m), 3.00(3H,s), 4.60-4.68(2H,m), 7.29-7.33(1H,m) 7.40-7.45(2H,m), 7.55-7.63(3H,m), 7.93-7.98(1H,m), 8.31-8.32(1H,m).

### Reference Example 56

### tert-butyl ({4-(2-fluoropyridin-3-yl)-5-[(3-methoxyphenyl)sulfonyl]-1,3-thiazol-2-yl}methyl)methylcarbamate

To a solution of tert-butyl ({4-(2-fluoropyridin-3-yl)-5-[(3-methoxyphenyl)thio]-1,3-thiazol-2-yl}methyl)methylcarbamate (276 mg) in acetic acid (5 mL) was added 3-chloroperbenzoic acid (590 mg), and the mixture was stirred at room temperature for 3 hr. The reaction mixture was concentrated under reduced pressure. The residue was basified with saturated aqueous sodium hydrogen carbonate solution, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=1:1) to give the title compound as a yellow oil (203 mg, yield 68%).
¹H-NMR(CDCl₃)δ: 1.47-1.51(9H,m), 3.00(3H,s), 3.77(3H,s), 4.60-4.67(2H,m), 7.08-7.10(1H,m), 7.20-7.25(2H,m), 7.31-7.36(2H,m), 7.96-7.98(1H,m), 8.31-8.32(1H,m).

### Reference Example 57

### tert-butyl {[5-[(3-chlorophenyl)sulfonyl]-4-(2-fluoropyridin-3-yl)-1,3-thiazol-2-yl]methyl}methylcarbamate

To a solution of tert-butyl {[5-[(3-chlorophenyl)thio]-4-(2-fluoropyridin-3-yl)-1,3-thiazol-2-yl]methyl}methylcarbamate (168 mg) in acetic acid (3 mL) was added 3-chloroperbenzoic acid (426 mg), and the mixture was stirred at room temperature for 5 hr. The reaction mixture was concentrated under reduced pressure. The residue was basified with saturated aqueous sodium hydrogen carbonate solution, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=1:1) to give the title compound as a yellow oil (131 mg, yield 79%).
¹H-NMR(CDCl₃)δ: 1.48-1.52(9H,m), 3.01(3H,s), 4.68(2H,brs), 7.31-7.40(2H,m), 7.48-7.57(3H,m), 7.91-7.96(1H,m), 8.33-8.35(1H,m).

### Reference Example 58

### tert-butyl {[5-[(3-fluorophenyl)sulfonyl]-4-(2-fluoropyridin-3-yl)-1,3-thiazol-2-yl]methyl}methylcarbamate

tert-Butyl {[5-bromo-4-(2-fluoropyridin-3-yl)-1,3-thiazol-2-yl]methyl}methylcarbamate (260 mg), 3-fluorothiophenol (0.07 mL), tris(dibenzylideneacetone)dipalladium (18 mg), 9,9-dimethyl-4,5-bis(diphenylphosphino)xanthine (23 mg) and N-ethyldiisopropylamine (0.23 mL) were stirred in toluene (8 mL) at 105°C for 16 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=2:1) to give a mixture of the title compound, tert-butyl {[5-bromo-4-(2-fluoropyridin-3-yl)-1,3-thiazol-2-yl]methyl}methylcarbamate (260 mg) and 3-fluorothiophenol as a yellow oil (140 mg). To a solution of the obtained mixture (136 mg) in acetic acid (2 mL) was added 3-chloroperbenzoic acid (360 mg), and the mixture was stirred at room temperature for 12 hr. The reaction mixture was concentrated under reduced pressure. The residue was basified with saturated aqueous sodium hydrogen carbonate solution, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=2:1) to give the title compound as a yellow oil (71 mg, yield in 2 steps 23%).
¹H-NMR(CDCl₃)δ: 1.52(9H,brs), 3.01(3H,s), 4.68(2H,brs), 7.28-7.35(3H,m), 7.39-7.47(2H,m), 7.92-7.97(1H,m), 8.33-8.34(1H,m).

### Reference Example 59

### [2-(2-fluorophenyl)-1H-imidazol-4-yl]methanol

A mixture of 2-fluorobenzamidine hydrochloride (5 g), dihydroxyacetone dimer (5.16 g), ammonium chloride (7.66 g) and 25% aqueous ammonia (50 mL) was stirred at 80°C for 30 min. Water was added to the reaction mixture, and the mixture was extracted with ethyl acetate. The extract was dried over anhydrous magnesium sulfate, and concentrated under reduced pressure. The residue was crystallized from diethyl ether to give the title compound as colorless crystals (2.78 g, yield 51%).
¹H-NMR(DMSO-d₆)δ: 4.44(2H,brs), 4.80-5.15(1H,m), 6.84-7.10(1H,m), 7.20-7.52(3H,m), 7.90-8.05(1H,m), 12.00(1H,brs).

### Reference Example 60

### 2-(2-fluorophenyl)-1H-imidazole-4-carbaldehyde

To a solution of [2-(2-fluorophenyl)-1H-imidazol-4-yl]methanol (170 mg) in tetrahydrofuran (30 mL) was added manganese dioxide (770 mg), and the mixture was stirred at room temperature for 15 hr. The reaction mixture was filtered, and the filtrate was concentrated under reduced pressure. The residue was crystallized from isopropyl ether to give the title compound as colorless crystals (160 mg, yield 95%).
¹H-NMR(DMSO-d₆)δ: 7.28-7.45(2H,m), 7.48-7.60(1H,m), 7.94-8.05(1H,m), 8.13(1H,s), 9.81(1H,s), 13.09(1H,brs).

### Reference Example 61

### 2-(2-fluorophenyl)-1-(2-thienylsulfonyl)-1H-imidazole-4-carbaldehyde

To a solution of 2-(2-fluorophenyl)-1H-imidazole-4-carbaldehyde (140 mg) in tetrahydrofuran (30 mL) was added sodium hydride (60% in oil, 148 mg) at room temperature, and the mixture was stirred for 10 min. 15-crown-5 (811 mg) was added dropwise, and the mixture was stirred for 5 min. Thiophene-2-sulfonyl chloride (404 mg) was added, and the mixture was further stirred for 30 min. The reaction mixture was diluted with water, and the mixture was extracted with ethyl acetate. The extract was washed with saturated brine, dried over anhydrous magnesium sulfate, and concentrated under reduced pressure. The residue was purified by silica gel column chromatography (eluent: hexane-ethyl acetate=1:1→3:7) to give the title compound as a colorless oil (200 mg, yield 81%).
¹H-NMR(CDCl₃): 7.03-7.08(1H,m), 7.10-7.17(1H,m), 7.22-7.29(1H,m), 7.35-7.42(2H,m), 7.49-7.60(1H,m),

### 7.78(1H,dd,J=4.9,1.1Hz), 8.26(1H,s),9.94(1H,s). Reference Example 62

### 5-bromo-4-(2-fluoropyridin-3-yl)thiophene-2-carbaldehyde

4-(2-Fluoropyridin-3-yl)thiophene-2-carbaldehyde (3.35 g) was dissolved in a mixed solvent of acetic acid (20 mL) and N,N-dimethylformamide (20 mL), and bromine (7.77 g) was added at room temperature. The reaction mixture was stirred overnight, and concentrated under reduced pressure. The residue was weakly basified with saturated aqueous sodium hydrogen carbonate solution, and the mixture was extracted with ethyl acetate. The extract was washed successively with saturated aqueous sodium thiosulfate solution, saturated aqueous sodium hydrogen carbonate solution and saturated brine, dried over anhydrous sodium sulfate, and concentrated under reduced pressure. The residue was purified by silica gel column chromatography (eluent: hexane:ethyl acetate=6:1→3:1), and the obtained solid was washed with diisopropyl ether to give the title compound as a pale-yellow powder (1.79 g, yield 39%).
¹H-NMR(CDCl₃)δ: 7.30-7.35(1H,m),7.71(1H,d,J=2.1Hz), 7.92-7.99(1H,m),8.28-8.31(1H,m),9.83(1H,s).

### Reference Example 63

### 5-bromo-4-(2-fluoropyridin-3-yl)thiophene-2-carboxylic acid

5-Bromo-4-(2-fluoropyridin-3-yl)thiophene-2-carbaldehyde (1.0 g), sodium chlorite (594 mg) and sodium dihydrogen phosphate (420 mg) were suspended in a mixed solvent of 2-methyl-2-propanol (15 mL), tetrahydrofuran (7 mL) and water (7 mL), and 2-methyl-2-butene (982 mg) was added at 0°C. The reaction mixture was stirred at room temperature for 6 hr, and concentrated under reduced pressure. 1 mol/L Hydrochloric acid was added to the residue, and the mixture was extracted with ethyl acetate. The extract was extracted with 1 mol/L aqueous sodium hydroxide solution. The aqueous layer was acidified with 1 mol/L hydrochloric acid. The resulting solid was collected by filtration, washed with water, and concentrated under reduced pressure to give the title compound as a white solid (400 mg, yield 38%).
¹H-NMR(DMSO-d₆)δ: 7.48-7.52(1H,m),7.77(1H,s),8.07-8.14(1H,m),8.32-8.34(1H,m),13.6(1H,br).

### Reference Example 64

### [5-bromo-4-(2-fluoropyridin-3-yl)thiophen-2-yl](1,1-dideutero)methanol

To a solution of 5-bromo-4-(2-fluoropyridin-3-yl)thiophene-2-carboxylic acid (400 mg) in tetrahydrofuran (10 mL) were added oxalyl chloride (254 mg) and N,N-dimethylformamide (a several drops) under ice-cooling. The reaction mixture was stirred at room temperature for 30 min, concentrated under reduced pressure, and azeotroped with toluene. The residue was dissolved in tetrahydrofuran (5 mL), and deuterated sodium borohydride (166 mg) and deuterated methanol (1 mL) were added at room temperature. The reaction mixture was stirred for 3 hr, and concentrated under reduced pressure. 1 mol/L Hydrochloric acid was added to the residue, and the mixture was extracted with ethyl acetate. The extract was washed successively with saturated aqueous sodium hydrogen carbonate solution and saturated brine, dried over anhydrous sodium sulfate, and concentrated under reduced pressure. The residue was purified by silica gel column chromatography (eluent: hexane-ethyl acetate=7:1→3:1) to give the title compound as colorless crystals (242 mg, yield 63%).
¹H-NMR(CDCl₃)δ: 2.05(1H,s),6.96(1H,d,J=2.7Hz),7.25-7.30(1H,m),7.90-7.96(1H,m),8.21-8.23(1H,m).

### Reference Example 65

### 5-bromo-4-(2-fluoropyridin-3-yl)thiophene-2-deuterocarbaldehyde

[5-Bromo-4-(2-fluoropyridin-3-yl)thiophen-2-yl](1,1-dideutero)methanol (242 mg) and manganese dioxide (483 mg) were suspended in toluene (5 mL), and the suspension was stirred at 90°C for 1 hr, allowed to cool to room temperature, and filtered through celite. The filtrate was concentrated under reduced pressure to give the title compound as a white powder (223 mg, yield 93%).
¹H-NMR(CDCl₃)δ: 7.30-7.34(1H,m),7.71(1H,d,J=2.1Hz),7.93-7.99(1H,m),8.28-8.31(1H,m)

### Reference Example 66

### 5-(benzylthio)-2-methylpyridine

5-Bromo-2-methylpyridine (10 g), phenylmethanethiol (7.22 g), N-ethyldiisopropylamine (15 g), tris(dibenzylideneacetone)dipalladium(0) (2.67 g) and 4,5-bis(diphenylphosphino)-9,9-dimethylxanthene (3.36 g) were mixed in toluene (200 mL), and the mixture was stirred at 110°C for 3 hr under an argon atmosphere. The reaction mixture was allowed to cool, and water was added. The mixture was filtered, and the filtrate was extracted with ethyl acetate. The extract was washed successively with saturated sodium hydrogen carbonate solution and saturated brine, dried over anhydrous magnesium sulfate, and concentrated under reduced pressure. The residue was purified by silica gel column chromatography (eluent: hexane-ethyl acetate=7:3) to give the title compound as a pale-yellow oil (11.7 g, yield 94%).
¹H-NMR(CDCl₃)δ: 2.51(3H,s),4.04(2H,s),7.01(H,d,J=8.0Hz),7.18-7.33(5H,m),7.44(1H,dd,J=8.0, 2.3Hz),8.41(1H,d,J=2.3Hz).

### Reference Example 67

### 6-methylpyridine-3-sulfonyl chloride

5-(Benzylsulfanyl)-2-methylpyridine (11.7 g) was dissolved in a mixed solvent of acetic acid (120 mL)-water (40 mL), N-chlorosuccinimide (29.0 g) was added, and the mixture was stirred at room temperature for 3 hr. The reaction mixture was concentrated under reduced pressure, saturated aqueous sodium hydrogen carbonate solution was added to the residue, and the mixture was extracted with ethyl acetate. The extract was washed with saturated brine, dried over anhydrous magnesium sulfate, and concentrated under reduced pressure. The residue was purified by silica gel column chromatography (eluent: hexane-ethyl acetate=4:1→1:1) to give the title compound as a pale-brown solid (7.87 g, yield 76%).
¹H-NMR(CDCl₃)δ: 2.73(3H,s),7.43(1H,d,J=8.3Hz),8.19(1H,dd,J=8.3,2.7Hz),9.12(1H, d,J=2.7Hz).

### Reference Example 68

### sodium 3-(methylsulfonyl)benzenesulfinate

A solution of sodium sulfite (990 mg) and sodium hydrogen carbonate (660 mg) in water (3 mL) was heated to 80°C, and a solution of 3-(methylsulfonyl)benzenesulfonyl chloride (1.0 g) in 1,4-dioxane (3 mL) was added. The reaction mixture was stirred at the same temperature for 1 hr, and concentrated under reduced pressure. Ethanol was added to the residue, and the mixture was further refluxed for 1 hr. The supernatant was separated while the reaction mixture was hot, ethanol was added to the residue, and the mixture was stirred at room temperature. The reaction mixture was filtered, the filtrate was combined with the supernatant previously separated, and ethanol was evaporated under reduced pressure to give the crude title compound as a white solid (569 mg).

### Reference Example 69

### sodium 6-methoxypyridine-2-sulfinate

A solution of sodium sulfite (607 mg) and sodium hydrogen carbonate (405 mg) in water (3 mL) was heated to 80°C, and 6-methoxypyridine-2-sulfonyl chloride (500 mg) was added. The reaction mixture was stirred at the same temperature for 1 hr, and concentrated under reduced pressure. Ethanol was added to the residue, and the mixture was further refluxed for 2 hr. The supernatant was separated while the reaction mixture was hot, ethanol was added to the residue, and the mixture was stirred at room temperature. The reaction mixture was filtered, the filtrate was combined with the supernatant previously separated, and ethanol was evaporated under reduced pressure. The residue was crystallized from 2-propanol to give the title compound as a white powder (439 mg, yield 93%). ¹H-NMR(DMSO-d₆)δ: 3.83(3H,s),6.60-6.64(1H,m),7.24-7.27(1H,m),7.64-7.70(3H,m).

### Reference Example 70

### sodium 6-methylpyridine-3-sulfinate

Anhydrous sodium sulfite (2.65 g) and sodium hydrogen carbonate (1.77 g) were suspended in water (10 mL), 6-methylpyridine-3-sulfonyl chloride (2.0 g) was added, and the mixture was stirred at 80°C for 1 hr. The reaction mixture was concentrated under reduced pressure, ethanol (50 mL) was added to the obtained residue, and the mixture was stirred at 80°C for 30 min. The reaction mixture was filtered to remove the insoluble material, and the filtrate was concentrated under reduced pressure. Ethyl acetate was added to the residue, and the insoluble solid was collected by filtration to give the title compound as a pale-yellow powder (1.4 g, yield 75%).
¹H-NMR(DMSO-d₆)δ: 2.44(3H,s),7.18(1H,d,J=8.0Hz),7.68(1H,dd,J=8.0,1.9Hz),8.46(1H, s).

### Reference Example 71

### sodium 6-methoxypyridine-3-sulfinate

A solution of sodium sulfite (607 mg) and sodium hydrogen carbonate (405 mg) in water (3 mL) was heated to 80°C, and a solution of 6-methoxypyridine-3-sulfonyl chloride (500 mg) in 1,4-dioxane (3 mL) was added. The reaction mixture was stirred at the same temperature for 1 hr, and concentrated under reduced pressure. Ethanol was added to the residue, and the mixture was further refluxed for 1 hr. The supernatant was separated while the reaction mixture was hot, ethanol was added to the residue, and the mixture was stirred at room temperature. The reaction mixture was filtered, the filtrate was combined with the supernatant previously separated, and ethanol was evaporated under reduced pressure to give the title compound as a white powder (452 mg, yield 96%).
¹H-NMR(DMSO-d₆) δ: 3.83(3H,s),6.70-6.73(1H,m),7.67-7.71(1H,m),8.11(1H,d,J=2.4Hz).

### Reference Example 72

### sodium 1-methyl-1H-pyrazole-4-sulfinate

A solution of sodium sulfite (698 mg) and sodium hydrogen carbonate (465 mg) in water (3 mL) was heated to 80°C, and a solution of 1-methyl-1H-pyrazole-4-sulfonyl chloride (500 mg) in 1,4-dioxane (3 mL) was added. The reaction mixture was stirred at the same temperature for 1 hr, and concentrated under reduced pressure. Ethanol was added to the residue, and the mixture was further refluxed for 1 hr. The supernatant was separated while the reaction mixture was hot, ethanol was added to the residue, and the mixture was stirred at room temperature. The reaction mixture was filtered, the filtrate was combined with the supernatant previously separated, and ethanol was evaporated under reduced pressure to give the crude title compound as a white solid (550 mg).
¹H-NMR(DMSO-d₆)δ: 3.75(3H,s),7.20(1H,s),7.40(1H,s).

### Reference Example 73

### 5-[(3-bromophenyl)thio]-4-(2-fluoropyridin-3-yl)thiophene-2-carbaldehyde

To a solution of 5-bromo-4-(2-fluoropyridin-3-yl)thiophene-2-carbaldehyde (300 mg) in N,N-dimethylformamide (5 mL) were added potassium carbonate (189 mg) and 3-bromobenzenethiol (218 mg) at room temperature, and the mixture was stirred at room temperature for 1 hr. Water was added to the reaction mixture, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=19:1→9:1) to give the title compound as a pale-yellow oil (394 mg, yield 95%).
¹H-NMR(CDCl₃)δ: 7.16-7.30(3H,m),7.41-7.46(2H,m),7.81-7.91(2H,m),8.24-8.27(1H,m),9.84(1H,s).

### Reference Example 74

### 4-(2-fluoropyridin-3-yl)-5-[(pyridin-2-yl)]thiophene-2-carbaldehyde

A suspension of 5-bromo-4-(2-fluoropyridin-3-yl)thiophene-2-carbaldehyde (297 mg), potassium carbonate (171 mg) and 2-mercaptopyridine (129 mg) in N,N-dimethylformamide (5 mL) was stirred at room temperature for 2 days. Water was added to the reaction mixture, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was washed with diisopropyl ether to give the title compound as a pale-yellow solid (281 mg, yield 85%).
¹H-NMR(CDCl₃)δ: 7.06-7.11(2H,m),7.19-7.24(1H,m),7.52-7.57(1H,m),7.89-7.97(2H,m),8.19-8.22(1H,m),8.40-8.43(1H,m),9.91(1H,s).

### Reference Example 75

### 4-(2-fluoropyridin-3-yl)-5-[(thiophen-3-yl)thio]thiophene-2-carbaldehyde

To a solution of 5-bromo-4-(2-fluoropyridin-3-yl)thiophene-2-carbaldehyde (286 mg) in N,N-dimethylformamide (2 mL) were added potassium carbonate (276 mg) and thiophene-3-thiol (151 mg) at room temperature, and the mixture was stirred at room temperature for 18 hr. The reaction mixture was diluted with water, and extracted with ethyl acetate. The extract was washed with saturated brine, dried over anhydrous magnesium sulfate, and concentrated under reduced pressure to give a residue. The obtained residue was purified by silica gel column chromatography (eluent: hexane-ethyl acetate=4:1→1:1) to give the title compound as a pale-brown powder (173 mg, yield 54%).
¹H-NMR(CDCl₃)δ: 7.09(1H,dd,J=5.1,1.3Hz),7.29-7.35(1H,m),7.43(1H,dd,J=5.1,3.0Hz),7.54(1H,dd,J=3.0,1.3Hz),7.7 4(1H,d,J=2.3Hz),7.93-8.01(1H,m),8.25-8.29(1H,m),9.77(1H,s).

### Reference Example 76

### 4-(2-fluoropyridin-3-yl)-5-[(2-methylfuran-3-yl)thio]thiophene-2-carbaldehyde

In the same manner as in Reference Example 75 and using 5-bromo-4-(2-fluoropyridin-3-yl)thiophene-2-carbaldehyde (250 mg) as a starting material and 2-methyl-3-furanthiol (0.114 mL) and potassium carbonate (193 mg), the title compound was obtained as a pale-yellow oil (271 mg, yield 97%).
¹H-NMR(CDCl₃)δ: 2.37(3H,s),6.38(1H,d,J=1.9Hz),7.31-7.37(1H,m),7.39(1H,d,J=1.9Hz),7.74(1H,d,J=2.3Hz),7.98-8.06(1H,m),8.25-8.30(1H,m),9.74(1H,s).

### Reference Example 77

### 4-(2-fluoropyridin-3-yl)-5-[(1,3-thiazol-2-yl)thio]thiophene-2-carbaldehyde

In the same manner as in Reference Example 75 and using 5-bromo-4-(2-fluoropyridin-3-yl)thiophene-2-carbaldehyde (250 mg) as a starting material and 2-mercaptothiazole (134 mg) and potassium carbonate (193 mg), the title compound was obtained as a white powder (282 mg, yield 100%).
¹H-NMR(CDCl₃)δ: 7.27-7.33(1H,m),7.34(1H,d,J=3.4Hz),7.74(1H,d,J=3.2Hz),7.87(1H,d,J=2 .3Hz),7.98-8.05(1H,m),8.26-8.30(1H,m),9.92(1H,s).

### Reference Example 78

### 4-(2-fluoropyridin-3-yl)-5-[(1H-imidazol-2-yl)thio]thiophene-2-carbaldehyde

In the same manner as in Reference Example 75 and using 5-bromo-4-(2-fluoropyridin-3-yl)thiophene-2-carbaldehyde (500 mg) as a starting material and 2-mercaptoimidazole (227 mg) and potassium carbonate (484 mg), the title compound was obtained as a pale-yellow powder (216 mg, yield 40%).
¹H-NMR(CDCl₃)δ: 7.15(1H,brs),7.22(1H,brs),7.31-7.37(1H,m),7.74(1H,d,J=1.9Hz),8.01-8.10(1H,m),8.26-8.30(1H,m),9.80(1H,s),9.88(1H,brs).

### Reference Example 79

### 4-(2-fluoropyridin-3-yl)-5-[(pyridin-4-yl)thio]thiophene-2-carbaldehyde

In the same manner as in Reference Example 75 and using 5-bromo-4-(2-fluoropyridin-3-yl)thiophene-2-carbaldehyde (400 mg) as a starting material and 4-mercaptopyridine (171 mg) and potassium carbonate (251 mg), the title compound was obtained as a colorless oil (256 mg, yield 58%).
¹H-NMR(CDCl₃)δ: 6.98(2H,dd,J=4.6,1.6Hz),7.20-7.28(1H,m),7.77-7.86(1H,m),7.95(1H,d,J=2.1Hz),8.22-8.28(1H,m),8.43(2H,dd,J=4.6,1.6Hz),9.96(1H,s).

### Reference Example 80

### 5-[(2-chloropyridin-4-yl)thio]-4-(2-fluoropyridin-3-yl)thiophene-2-carbaldehyde

In the same manner as in Reference Example 75 and using 5-bromo-4-(2-fluoropyridin-3-yl)thiophene-2-carbaldehyde (411 mg) as a starting material and sodium 2-chloropyridine-4-thiolate (240 mg) and potassium carbonate (199 mg), the title compound was obtained as a colorless oil (264 mg, yield 52%).
¹H-NMR(CDCl₃)δ: 6.87(1H,dd,J=5.5,1.7Hz),6.97(1H,d,J=1.1Hz),7.23-7.30(1H,m),7.75-7.83(1H,m),7.96(1H,d,J=2.1Hz),8.19(1H,d,J=4.9Hz),8.25-8.29(1H,m),9.98(1H,s).

### Reference Example 81

### 5-[(6-chloropyridin-3-yl)thio]-4-(2-fluoropyridin-3-yl)thiophene-2-carbaldehyde

In the same manner as in Reference Example 75 and using 5-bromo-4-(2-fluoropyridin-3-yl)thiophene-2-carbaldehyde (750 mg) as a starting material and sodium 6-chloropyridine-3-thiolate (658 mg) and potassium carbonate (724 mg), the title compound was obtained as a pale-yellow oil (556 mg, yield 61%).
¹H-NMR(CDCl₃)δ: 7.28-7.35(2H,m),7.57-7.62(1H,m),7.81(1H,d,J=1.9Hz),7.85-7.92(1H,m),8.28-8.31(1H,m),8.31-8.34(1H,m),9.85(1H,s).

### Reference Example 82

### 4-bromo-5-(phenylsulfonyl)thiophene-2-carbaldehyde

To a solution of 4,5-dibromothiophene-2-carbaldehyde (1.0 g) in N,N-dimethylformamide (10 mL) were added pyridine (342 mg) and sodium benzenesulfinate dihydrate (790 mg) at room temperature, and the mixture was stirred at 70°C for 18 hr. Water was added to the reaction mixture, and the mixture was extracted with ethyl acetate. The extract was washed successively with water and saturated brine, dried over anhydrous sodium sulfate, and concentrated under reduced pressure. The residue was purified by silica gel column chromatography (eluent: hexane-ethyl acetate=6:1→3:1) to give the title compound as a pale-yellow solid (1.1 g, yield 89%).
¹H-NMR(CDCl₃)δ: 7.55-7.70(4H,m),8.07-8.10(2H,m),9.88(1H,s). Reference Example 83

### 4-(2-fluorophenyl)-5-(phenylsulfonyl)thiophene-2-carbaldehyde

4-Bromo-5-(phenylsulfonyl)thiophene-2-carbaldehyde (1.1 g), (2-fluorophenyl)boronic acid (552 mg), sodium carbonate (837 mg) and tetrakis(triphenylphosphine) palladium(0) (380 mg) was suspended in a mixed solvent of 1,2-dimethoxyethane (10 mL) and water (4 mL), and the suspension was stirred at 105°C for 6 hr under a nitrogen atmosphere. The reaction mixture was allowed to cool to room temperature, water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=7:1→3:1) to give the title compound as a brown solid (1.1 g, yield 93%).
¹H-NMR(CDCl₃)δ: 6.96-7.02(1H,m),7.19-7.25(1H,m),7.30-7.55(7H,m),7.61-7.62(1H,m),9.94(1H,s)

### Reference Example 84

### 4-(2-bromophenyl)-5-(pyridin-3-ylsulfonyl)thiophene-2-carbaldehyde

4-Bromo-5-(pyridin-3-ylsulfonyl)thiophene-2-carbaldehyde (334 mg), (2-bromophenyl)boronic acid (243 mg), sodium carbonate (257 mg) and tetrakis(triphenylphosphine) palladium(0) (117 mg) were suspended in a mixed solvent of 1,2-dimethoxyethane (10 mL) and water (4 mL), and the suspension was stirred at 105°C for 4 hr under a nitrogen atmosphere. The reaction mixture was allowed to cool to room temperature, water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=7:1→3:1) to give the title compound as a pale-yellow solid (300 mg, yield 73%).
¹H-NMR(CDCl₃)δ: 7.26-7.38(2H,m),7.44-7.50(3H,m),7.62(1H,s),7.65-7.69(1H,m),8.50-8.51(1H,m),8.74-8.76(1H,m),9.97(1H,s)

### Reference Example 85

### 4-(2-fluoropyridin-3-yl)-5-(pyridin-3-ylsulfonyl)thiophene-2-carbaldehyde

4-Bromo-5-(pyridin-3-ylsulfonyl)thiophene-2-carbaldehyde (250 mg), (2-fluoropyridin-3-yl)boronic acid (127 mg), sodium carbonate (192 mg) and tetrakis(triphenylphosphine) palladium(0) (87 mg) were suspended in a mixed solvent of 1,2-dimethoxyethane (10 mL) and water (4 mL), and the suspension was stirred at 105°C for 4 hr under a nitrogen atmosphere. The reaction mixture was allowed to cool to room temperature, water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=4:1→1:1) to give the title compound as a pale-yellow solid (214 mg, yield 41%).
¹H-NMR(CDCl₃) δ: 7.34-7.40(2H,m),7.67(1H,s),7.75-7.80(1H,m),7.95-8.01(1H,m),8.33-8.35(1H,m),8.72-8.73(1H,m),8.78-8.80(1H,m),9.97(1H,s).

### Reference Example 86

### 4-(2-chloropyridin-3-yl)-5-(pyridin-3-ylsulfonyl)thiophene-2-carbaldehyde

4-Bromo-5-(pyridin-3-ylsulfonyl)thiophene-2-carbaldehyde (300 mg), (2-chloropyridin-3-yl)boronic acid (171 mg), sodium carbonate (230 mg) and tetrakis(triphenylphosphine) palladium(0) (104 mg) were suspended in a mixed solvent of 1,2-dimethoxyethane (10 mL) and water (4 mL), and the suspension was stirred at 105°C for 2 hr under a nitrogen atmosphere. The reaction mixture was allowed to cool to room temperature, water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=3:1→1:2) to give the title compound as a pale-yellow oil (136 mg, yield 41%).
¹H-NMR(CDCl₃)δ: 7.31-7.34(1H,m),7.41-7.46(1H,m),7.64-7.68(2H,m),7.84-7.87(1H,m),8.51-8.53(1H,m),8.64-8.65(1H,m),8.78-8.80(1H,m),9.98(1H,s).

### Reference Example 87

### 4-(2-fluoropyridin-3-yl)-5-(phenylsulfonyl)thiophene-2-carbaldehyde

To a solution of 5-bromo-4-(2-fluoropyridin-3-yl)thiophene-2-carbaldehyde (1 g) in N,N-dimethylformamide (10 mL) were added pyridine (0.367 mL) and sodium benzenesulfinate dihydrate (909 mg) at room temperature, and the mixture was stirred at 60°C for 48 hr. Water was added to the reaction mixture, and the mixture was stirred for 30 min. The precipitate was collected by filtration, washed with water, and dried under reduced pressure to give the title compound as a white solid (1.01 g, yield 83%).
¹H-NMR(CDCl₃)δ: 7.32-7.44(3H,m),7.49-7.61(3H,m),7.65(1H,d,J=1.3Hz),7.96-8.04(1H,m),8.29-8.33(1H,m),9.96(1H,s).

### Reference Example 88

### 4-(2-fluoropyridin-3-yl)-5-{[3-(methylsulfonyl)phenyl]sulfonyl}thiophene-2-carbaldehyde

To a solution of 5-bromo-4-(2-fluoropyridin-3-yl)thiophene-2-carbaldehyde (300 mg) in N,N-dimethylformamide (5 mL) were added pyridine (97 mg) and crude sodium 3-(methylsulfonyl)benzenesulfinate (330 mg), and the mixture was stirred at 70°C for 18 hr. Water was added to the reaction mixture, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=1:1→1:3) to give the title compound as a pale-yellow solid (297 mg, yield 66%).
¹H-NMR (CDCl₃) δ: 3.08(3H,s),7.38-7.44(1H,m),7.63-7.70(2H,m),7.85-7.88(1H,m),7.93-8.01(2H,m),8.12-8.15(1H,m),8.33-8.35(1H,m),9.97(1H,s).

### Reference Example 89

### 4-(2-fluoropyridin-3-yl)-5-[(6-methoxypyridin-2-yl)sulfonyl]thiophene-2-carbaldehyde

To a solution of 5-bromo-4-(2-fluoropyridin-3-yl)thiophene-2-carbaldehyde (350 mg) in N,N-dimethylformamide (5 mL) were added pyridine (113 mg) and sodium 6-methoxypyridine-2-sulfinate (310 mg), and the mixture was stirred at 70°C for 18 hr. Water was added to the reaction mixture, and the mixture was extracted with ethyl acetate. The extract was washed successively with water and saturated brine, dried over anhydrous sodium sulfate, and concentrated under reduced pressure. The residue was purified by silica gel column chromatography (eluent: hexane-ethyl acetate=3:1→1:1) to give the title compound as colorless crystals (363 mg, yield 79%).
¹H-NMR(CDCl₃)δ: 3.81(3H,s),6.88-6.91(1H,m),7.25-7.31(1H,m),7.40-7.41(1H,m),7.62-7.67(1H,m),7.71(1H,s),7.97-8.03(1H,m),8.24-8.26(1H,m),9.99(1H,s).

### Reference Example 90

### 4-(2-fluoropyridin-3-yl)-5-[(6-methylpyridin-3-yl)sulfonyl]thiophene-2-carbaldehyde

5-Bromo-4-(2-fluoropyridin-3-yl)thiophene-2-carbaldehyde (700 mg) and pyridine (390 mg) were dissolved in N,N-dimethylformamide (30 mL), sodium 6-methylpyridine-3-sulfinate (530 mg) was added, and the mixture was stirred at 80°C for 3 hr. The reaction mixture was allowed to cool, saturated aqueous sodium hydrogen carbonate solution was added, and the mixture was extracted with ethyl acetate. The extract was washed with saturated brine, dried over anhydrous magnesium sulfate, and concentrated under reduced pressure. The residue was purified by silica gel column chromatography (eluent: hexane-ethyl acetate=1:1→1:4) to give the title compound as colorless crystals (700 mg, yield 79%).
¹H-NMR (CDCl₃) δ: 2.61(3H,s),7.20(1H,d,J=8.3Hz),7.38(1H,ddd,J=7.3,5.1,1.8Hz),7.6 5(1H,dd,J=8.3,2.5Hz),7.68(1H,d,J=1.1Hz),8.00(1H,ddd,J=9.5,7.4, 1.9Hz),8.33-8.37(1H,m),8.59(1H,d,J=2.5Hz),9.98(1H,s).

### Reference Example 91

### 4-(2-fluoropyridin-3-yl)-5-[(6-methoxypyridin-3-yl)sulfonyl]thiophene-2-carbaldehyde

To a solution of 5-bromo-4-(2-fluoropyridin-3-yl)thiophene-2-carbaldehyde (350 mg) in N,N-dimethylformamide (5 mL) were added pyridine (113 mg) and sodium 6-methoxypyridine-3-sulfinate (310 mg), and the mixture was stirred at 70°C for 18 hr. Water was added to the reaction mixture, and the mixture was extracted with ethyl acetate. The extract was washed successively with water and saturated brine, dried over anhydrous sodium sulfate, and concentrated under reduced pressure. The residue was purified by silica gel column chromatography (eluent: hexane-ethyl acetate=6:1→2:1) to give the title compound as colorless crystals (515 mg, yield quantitative).
¹H-NMR(CDCl₃)δ: 3.97(3H,s),6.68-6.71(1H,m),7.35-7.39(1H,m),7.57-7.61(1H,m),7.67(1H,s),7.98-8.04(1H,m),8.28-8.35(2H,m),9.96(1H,s).

### Reference Example 92

### 4-(2-fluoropyridin-3-yl)-5-[(1-methyl-1H-pyrazol-4-yl)sulfonyl]thiophene-2-carbaldehyde

To a solution of 5-bromo-4-(2-fluoropyridin-3-yl)thiophene-2-carbaldehyde (300 mg) in N,N-dimethylformamide (5 mL) were added pyridine (97 mg) and crude sodium 1-methyl-1H-pyrazole-4-sulfinate (230 mg), and the mixture was stirred at 70°C for 18 hr. Water was added to the reaction mixture, and the mixture was extracted with ethyl acetate. The extract was washed successively with water and saturated brine, dried over anhydrous sodium sulfate, and concentrated under reduced pressure. The residue was purified by silica gel column chromatography (eluent: hexane-ethyl acetate=3:1→1:3) to give the title compound as colorless crystals (294 mg, yield 80%).
¹H-NMR(CDCl₃)δ: 3.87(3H,s),7.35-7.39(1H,m),7.43-7.47(2H,m),7.66(1H,s),8.00-8.06(1H,m),8.33-8.35(1H,m),9.96(1H,s).

### Reference Example 93

### 4-(2-fluoropyridin-3-yl)-5-(pyridin-3-ylsulfonyl)thiophene-2-deuterocarbaldehyde

5-Bromo-4-(2-fluoropyridin-3-yl)thiophene-2-deuterocarbaldehyde (223 mg), sodium pyridine-3-sulfinate (167 mg) and pyridine (72 mg) were dissolved in N,N-dimethylformamide (5 mL), and the solution was stirred at 80°C for 18 hr. The reaction mixture was allowed to cool, water was added to the reaction mixture, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=4:1→1:1) to give the title compound as a yellow solid (257 mg, yield 95%).
¹H-NMR(CDCl₃)δ: 7.34-7.40(1H,m),7.67(1H,s),7.76-7.80(1H,m),7.94-8.01(1H,m),8.33-8.36(1H,m),8.72-8.73(1H,m),8.78-8.80(1H,m).

### Reference Example 94

### 1-[(3-bromothiophen-2-yl)sulfonyl]-1H-pyrrole

To a solution of pyrrole (385 mg) in tetrahydrofuran (15 mL) was added sodium hydride (60% in oil, 306 mg) at room temperature, and the mixture was stirred for 10 min. A solution of 3-bromothiophene-2-sulfonyl chloride (1.00 g) in tetrahydrofuran (5 mL) was added, and the mixture was further stirred for 30 min. The reaction mixture was diluted with water, and extracted with ethyl acetate. The extract was washed with saturated brine, dried over anhydrous magnesium sulfate, and concentrated under reduced pressure. The residue was purified by silica gel column chromatography (eluent: hexane-ethyl acetate=4:1) to give a colorless solid. Hexane was added thereto, and the solid was collected by filtration to give the title compound as colorless crystals (970 mg, yield 87%).
¹H-NMR(CDCl₃)δ: 6.30-6.38(2H,m),7.07(1H,d,J=5.3Hz),7.28-7.32(2H,m),7.58(1H,d,J=5.3Hz).

### Reference Example 95

### 2-fluoro-3-[2-(1H-pyrrol-1-ylsulfonyl)thiophen-3-yl]pyridine

1-[(3-Bromothiophen-2-yl)sulfonyl]-1H-pyrrole (450 mg), (2-fluoropyridin-3-yl)boronic acid (434 mg), sodium hydrogen carbonate (388 mg) and tetrakis(triphenylphosphine) palladium(0) (89 mg) were added to a mixed solvent of 1,2-dimethoxyethane (10 mL) and water (5 mL), and the mixture was refluxed for 4 hr under an argon atmosphere. The reaction mixture was allowed to cool, water was added, and the mixture was extracted with ethyl acetate. The extract was washed with saturated brine, dried over anhydrous magnesium sulfate, and concentrated under reduced pressure. The residue was purified by silica gel column chromatography (eluent: hexane-ethyl acetate=7:3), and crystallized from hexane to give the title compound as colorless crystals (130 mg, yield 27%).
¹H-NMR(CDCl₃)δ: 6.19-6.22(2H,m),6.76-6.78(2H,m),7.06(1H,dd,J=5.1,1.3Hz),7.27-7.33(1H,m),7.68(1H,d,J=5.3Hz),7.82-7.89(1H,m),8.29-8.33(1H,m).

### Reference Example 96

### 4-(2-fluoropyridin-3-yl)-5-(1H-pyrrol-1-ylsulfonyl)thiophene-2-carbaldehyde

A solution of 2-fluoro-3-[2-(1H-pyrrol-1-ylsulfonyl)thiophen-3-yl]pyridine (230 mg) in tetrahydrofuran (10 mL) was cooled to -70°C, 1.6 mol/L n-butyllithium hexane solution (1.5 mL) was added dropwise, and the mixture was stirred for 30 min. N,N-Dimethylformamide was added at the same temperature, and the mixture was stirred for 30 min. Saturated aqueous ammonium chloride solution was added, and the mixture was extracted with ethyl acetate. The extract was washed with saturated brine, dried over anhydrous magnesium sulfate, and concentrated under reduced pressure. The residue was purified by silica gel column chromatography (eluent: hexane-ethyl acetate=7:3), and crystallized from hexane-diisopropyl ether (1:1) to give the title compound as colorless crystals (150 mg, yield 60%).
¹H-NMR(CDCl₃)δ: 6.21-6.31(2H,m),6.70-6.82(2H,m),7.33-7.38(1H,m),7.66(1H,d,J=1.3Hz),7.87-7.95(1H,m),8.33-8.39(1H,m)9.96(1H,s).

### Reference Example 97

### 1-{4-(2-fluoropyridin-3-yl)-5-[(pyridin-2-yl)thio]thiophen-2-yl}-N-methylmethanamine

To a solution of 4-(2-fluoropyridin-3-yl)-5-[(pyridin-2-yl)thio]thiophene-2-carbaldehyde (270 mg) in tetrahydrofuran (2 mL) were added 40% methylamine-methanol solution (0.9 mL) and methanol (2 mL), and the mixture was stirred at room temperature for 2 days, and concentrated under reduced pressure. The residue was dissolved in methanol (3 mL), and sodium borohydride (222 mg) was added at 0°C. The mixture was stirred at room temperature for 1 days, and concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous magnesium sulfate, and concentrated under reduced pressure to give the title compound as a pale-yellow oil (286 mg, yield quantitative).
¹H-NMR(CDCl₃) δ: 2.54(3H,s),4.00(2H,d,J=1.2Hz),6.86-6.88(1H,m), 6.96-7.01(1H,m), 7.13-7.18(2H,m), 7.44-7.50(1H,m),7.85-7.91(1H,m),8.12-8.15(1H,m),8.35-8.37(1H,m),1H: not detected.

### Reference Example 98

### tert-butyl ({5-[(3-bromophenyl)thio]-4-(2-fluoropyridin-3-yl)thiophen-2-yl}methyl)methylcarbamate

5-[(3-Bromophenyl)thio]-4-(2-fluoropyridin-3-yl)thiophene-2-carbaldehyde (394 mg) was dissolved in a mixed solvent of tetrahydrofuran (3 mL) and methanol (1 mL), 40% methylamine-methanol solution (1.0 mL) was added, and the mixture was stirred at room temperature for 3 days. The reaction mixture was concentrated under reduced pressure, the residue was dissolved again in methanol (3 mL), and sodium borohydride (22 mg) was added under ice-cooling. The mixture was stirred at room temperature for 4 hr, and concentrated under reduced pressure. Water was added to the residue, and the mixture was extracted with ethyl acetate. The extract was washed with saturated brine, dried over anhydrous sodium sulfate, and concentrated under reduced pressure. The residue was dissolved in tetrahydrofuran (3 mL), di-tert-butyl bicarbonate (262 mg) was added, and the mixture was stirred for 30 min. The reaction mixture was concentrated under reduced pressure, and the residue was purified by silica gel column chromatography (eluent: hexane-ethyl acetate=9:1→4:1) to give the title compound as a colorless oil (508 mg, yield 99%).
¹H-NMR(CDCl₃)δ: 1.49(9H,s),2.93(3H,s),4.54(2H,br),6.95-6.98(1H,m),7.05-7.10(2H,m),7.16-7.25(3H,m),7.76-7.81(1H,m),8.17-8.18(1H,m).

### Reference Example 99

### tert-butyl ({4-(2-fluoropyridin-3-yl)-5-[(thiophen-3-yl)thio]thiophen-2-yl}methyl)methylcarbamate

4-(2-Fluoropyridin-3-yl)-5-[(thiophen-3-yl)thio]thiophene-2-carbaldehyde (173 mg) was dissolved in a mixed solvent of tetrahydrofuran (2 mL) and methanol (1 mL), 40% methylamine-methanol solution (0.552 mL) was added, and the mixture was stirred at room temperature for 18 hr. The reaction mixture was concentrated under reduced pressure, and the residue was dissolved in methanol (2 mL). Sodium borohydride (102 mg) was added, and the mixture was stirred at room temperature for 6 hr. The solvent was evaporated under reduced pressure, 1 mol/L hydrochloric acid was added to the residue, and the mixture was stirred at room temperature for 1 hr. The reaction mixture was concentrated under reduced pressure, and the residue was dissolved in ethyl acetate (5 mL) and saturated aqueous sodium hydrogen carbonate solution (5 mL). Di-tert-butyl bicarbonate (161 mg) was added at room temperature, and the mixture was stirred for 1.5 hr. The reaction mixture was extracted with ethyl acetate, and the extract was washed successively with saturated aqueous sodium hydrogen carbonate solution and saturated brine, dried over anhydrous sodium sulfate, and concentrated under reduced pressure. The residue was purified by silica gel column chromatography (eluent: hexane-ethyl acetate=17:3→1:1) to give the title compound as a colorless oil (213 mg, yield 91%).
¹H-NMR(CDCl₃)δ: 1.48(9H,s),2.90(3H,s),4.49(2H,brs),6.87(1H,dd,J=5.1,1.3Hz),7.0 1(1H,d,J=2.4Hz),7.05(1H,brs),7.18-7.32(2H,m),7.81-7.95(1H,m),8.16-8.24(1H,m).

### Reference Example 100

### tert-butyl {4-(2-fluoropyridin-3-yl)-5-[(2-methylfuran-3-yl)thio]thiophen-2-yl}methyl)methylcarbamate

In the same manner as in Reference Example 99 and using 4-(2-fluoropyridin-3-yl)-5-[(2-methylfuran-3-yl)thio]thiophene-2-carbaldehyde (279 mg) as a starting material and 40% methylamine-methanol solution (0.899 mL), sodium borohydride (166 mg) and di-tert-butyl bicarbonate (0.261 mL), the title compound was obtained as a colorless oil (206 mg, yield 54%).
¹H-NMR (CDCl₃)δ: 1.48(9H,s),2.24(3H,s),2.87(3H,s),4.43(2H,brs),6.19-6.22(1H,m),6.89-6.94(1H,m),7.19-7.32(2H,m),7.87-7.98(1H,m),8.18-8.25(1H,m).

### Reference Example 101

### tert-butyl {[4-(2-fluoropyridin-3-yl)-5-[(1,3-thiazol-2-yl)thio]thiophen-2-yl]methyl}methylcarbamate

In the same manner as in Reference Example 99 and using 4-(2-fluoropyridin-3-yl)-5-[(1,3-thiazol-2-yl)thio]thiophene-2-carbaldehyde (282 mg) as a starting material and 40% methylamine-methanol solution (0.899 mL), sodium borohydride (166 mg) and di-tert-butyl bicarbonate (0.261 mL), the title compound was obtained as a colorless oil (185 mg, yield 48%).
¹H-NMR (CDCl₃) δ: 1.50(9H,s),2.93(3H,s),4.57(2H,brs),7.14(1H,d,J=2.6Hz),7.19(1H, d,J=3.4Hz),7.21-7.30(1H,m),7.62(1H,d,J=3.4Hz),7.92-8.01(1H,m),8.18-8.24(1H,m).

### Reference Example 102

### tert-butyl ({4-(2-fluoropyridin-3-yl)-5-[(1H-imidazol-2-yl)thio]thiophen-2-yl}methyl)methylcarbamate

In the same manner as in Reference Example 99 and using 4-(2-fluoropyridin-3-yl)-5-[(1H-imidazol-2-yl)thio]thiophene-2-carbaldehyde (216 mg) as a starting material and 40% methylamine-methanol solution (0.723 mL), sodium borohydride (134 mg) and di-tert-butyl bicarbonate (185 mg), the title compound was obtained as a white powder (237 mg, yield 80%).
¹H-NMR (CDCl₃) δ: 1.48(9H,s),2.89(3H,s),4.48(2H,brs),6.99(1H,d,J=2.3Hz),7.00-7.12(2H,m),7.23-7.31(1H,m),8.02-8.12(1H,m),8.17-8.23(1H,m),9.79(1H,brs).

### Reference Example 103

### tert-butyl ({4-(2-fluoropyridin-3-yl)-5-[(pyridin-4-yl)thio]thiophen-2-yl}methyl)methylcarbamate

In the same manner as in Reference Example 99 and using 4-(2-fluoropyridin-3-yl)-5-[(pyridin-4-yl)thio]thiophene-2-carbaldehyde (256 mg) as a starting material and 40% methylamine-methanol solution (0.833 mL), sodium borohydride (245 mg) and di-tert-butyl bicarbonate (0.242 mL), the title compound was obtained as a colorless oil (112 mg, yield 32%).
¹H-NMR(CDCl₃)δ: 1.50(9H,s),2.95(3H,s),4.59(2H,brs),6.88-6.93(2H,m),7.14-7.21(2H,m),7.70-7.79(1H,m),8.16-8.21(1H,m),8,33-8.39(2H,m).

### Reference Example 104

### tert-butyl ({5-[(2-chloropyridin-4-yl)thio]-4-(2-fluoropyridin-3-yl)thiophen-2-yl}methyl)methylcarbamate

In the same manner as in Reference Example 99 and using 5-[(2-chloropyridin-4-yl)thio]-4-(2-fluoropyridin-3-yl)thiophene-2-carbaldehyde (264 mg) as a starting material and 40% methylamine-methanol solution (0.772 mL), sodium borohydride (143 mg) and di-tert-butyl bicarbonate (0.237 mL), the title compound was obtained as a colorless oil (317 mg, yield 90%).
¹H-NMR (CDCl₃)δ: 1.50(9H,s),2.96(3H,s),4.60(2H,brs),6.82(1H,dd,J=5.3,1.5 Hz),6.90-6.93(1H,m),7.15-7.24(2H,m),7.68-7.77(1H,m),8.14(1H,d,J=5.3Hz),8.18-8.23(1H,m).

### Reference Example 105

### tert-butyl ({5-[(6-chloropyridin-3-yl)thio]-4-(2-fluoropyridin-3-yl)thiophen-2-yl}methyl)methylcarbamate

In the same manner as in Reference Example 99 and using 5-[(6-chloropyridin-3-yl)thio]-4-(2-fluoropyridin-3-yl)thiophene-2-carbaldehyde (556 mg) as a starting material and 40% methylamine-methanol solution (1.62 mL), sodium borohydride (300 mg) and di-tert-butyl bicarbonate (0.498 mL), the title compound was obtained as a colorless oil (683 mg, yield 92%).
¹H-NMR(CDCl₃)δ: 1.49(9H,s),2.92(3H,s),4.54(2H,brs),7.06-7.09(1H,m),7.15-7.28(2H,m),7.33(1H,dd,J=8.3,2.7Hz),7.74-7.84(1H,m),8.11(1H,d,J=2.3Hz),8.19-8.24(1H,m).

### Reference Example 106

### tert-butyl ({4-(2-fluoropyridin-3-yl)-5-[(pyridin-2-yl)thio]thiophen-2-yl}methyl)methylcarbamate

To a solution of 1-{4-(2-fluoropyridin-3-yl)-5-[(pyridin-2-yl)thio]thiophen-2-yl}-N-methylmethanamine (286 mg) in ethyl acetate (3 mL) was added di-tert-butyl bicarbonate (0.23 mL), and the mixture was stirred at room temperature for 2 days. The reaction mixture was concentrated under reduced pressure, and the residue was purified by silica gel column chromatography (eluent: hexane-ethyl acetate=1:1) to give the title compound as a yellow oil (332 mg, yield 91%).
¹H-NMR(CDCl₃)δ: 1.50(9H,brs),2.94(3H,s),4.56(2H,brs),6.85-6.88(1H,m),6.98-7.02(1H,m),7.14-7.18(2H,m),7.46-7.52(1H,m),7.85-7.92(1H,m),8.13-8.15(1H,m),8.35-8.37(1H,m).

### Reference Example 107

### tert-butyl {[4-(2-fluoro-3-formylphenyl)-5-(phenylsulfonyl)thiophen-2-yl]methyl}methylcarbamate

tert-Butyl {[4-bromo-5-(phenylsulfonyl)-2-thienyl]methyl}methylcarbamate (257 mg), (2-fluoro-3-formylphenyl)boronic acid (116 mg), sodium carbonate (152 mg) and tetrakis(triphenylphosphine) palladium(0) (66 mg) were suspended in a mixed solvent of 1,2-dimethoxyethane (5 mL) and water (2 mL), and the suspension was stirred under a nitrogen atmosphere at 105°C for 18 hr. The reaction mixture was allowed to cool to room temperature, water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=9:1→4:1) to give the title compound as a colorless amorphous solid (147 mg, yield 52%).
¹H-NMR(CDCl₃)δ: 1.50(9H,s),2.93(3H,s),4.56(2H,br),6.86(1H,s),7.29-7.35(3H,m),7.47-7.52(3H,m),7.58-7.62(1H,m),7.88-7.93(1H,m),10.19(1H,s).

### Reference Example 108

### tert-butyl ({4-[2-fluoro-3-(hydroxymethyl)phenyl]-5-(phenylsulfonyl)thiophen-2-yl}methyl)methylcarbamate

To a solution of tert-butyl {[4-(2-fluoro-3-formylphenyl)-5-(phenylsulfonyl)thiophen-2-yl]methyl}methylcarbamate (147 mg) in tetrahydrofuran (2 mL) were added sodium borohydride (14 mg) and methanol (1 mL) at room temperature. The mixture was stirred for 1 hr, water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=7:1→2:1) to give the title compound as a colorless oil (137 mg, yield 93%).
¹H-NMR (CDCl₃) δ: 1.49(9H,s),1.73(1H,brt,J=6.0Hz),2.91(3H,s),4.53(2H,br),4.64(2H ,d,J=6.0Hz),6.82(1H,s),7.15-7.34(4H,m),7.44-7.50(4H,m).

### Reference Example 109

### tert-butyl methyl{[4-(1-methyl-1H-pyrazol-5-yl)-5-(phenylsulfonyl)thiophen-2-yl]methyl}carbamate

tert-Butyl {[4-bromo-5-(phenylsulfonyl)-2-thienyl]methyl}methylcarbamate (189 mg), 1-methyl-5-(tributylstannyl)-1H-pyrazole (247 mg) and tetrakis(triphenylphosphine) palladium(0) (51 mg) were dissolved in toluene (3 mL), and the solution was degassed, and stirred at 110°C for 3 hr. The reaction mixture was concentrated under reduced pressure, and the residue was purified by silica gel column chromatography (eluent: hexane-ethyl acetate=1:1→1:3) and basic silica gel column chromatography (eluent: hexane-ethyl acetate=3:1→1:2) to give the title compound as a colorless oil (169 mg, yield 85%).
¹H-NMR(CDCl₃) δ: 1.50(9H,s),2.92(3H,s),3.32(3H,s),4.56(2H,br),6.19(1H,brs),6.79 (1H,s),7.36-7.41(2H,m),7.48-7.56(4H,m).

### Reference Example 110

### tert-butyl methyl{[4-(1-methyl-1H-imidazol-2-yl)-5-(phenylsulfonyl)thiophen-2-yl]methyl}carbamate

tert-Butyl {[4-bromo-5-(phenylsulfonyl)-2-thienyl]methyl}methylcarbamate (496 mg), 1-methyl-2-(tributylstannyl)-1H-imidazole (619 mg) and tetrakis(triphenylphosphine) palladium(0) (129 mg) were dissolved in toluene (10 mL), and the solution was degassed, and stirred at 160°C for 30 min under microwave irradiation. The reaction mixture was concentrated under reduced pressure, and the residue was purified by silica gel column chromatography (eluent: hexane-ethyl acetate=1:1→ethyl acetate) to give the title compound as a colorless oil (313 mg, yield 63%).
¹H-NMR (CDCl₃) δ: 1.48(9H,s),2.86(3H,s),3.56(3H,s),4.52(2H,br),6.89(1H,brs),7.03 (1H,brs),7.10(1H,brs),7.40-7.55(3H,m),7.66-7.70(2H,m).

### Reference Example 111

### tert-butyl methyl{[4-(1-methyl-1H-imidazol-5-yl)-5-(phenylsulfonyl)thiophen-2-yl]methyl}carbamate

tert-Butyl {[4-bromo-5-(phenylsulfonyl)-2-thienyl]methyl}methylcarbamate (285 mg), 1-methyl-5-(tributylstannyl)-1H-imidazole (356 mg) and tetrakis(triphenylphosphine) palladium(0) (74 mg) were dissolved in toluene (5 mL), and the solution was degassed, and stirred at 160°C for 30 min under microwave irradiation. The reaction mixture was concentrated under reduced pressure, and the residue was purified by basic silica gel column chromatography (eluent: hexane-ethyl acetate=1:1→ethyl acetate) to give the title compound as a colorless oil (295 mg, yield quantitative)
¹H-NMR(CDCl₃)δ: 1.50(9H,s),2.91(3H,s),3.27(3H,brs),4.55(2H,br),6.77(1H,brs),6. 82(1H,br),7.37-7.43(2H,m),7.49-7.58(4H,m).

### Reference Example 112

### tert-butyl methyl{[4-(2-oxopiperidin-1-yl)-5-(phenylsulfonyl)thiophen-2-yl]methyl}carbamate

tert-Butyl {[4-bromo-5-(phenylsulfonyl)-2-thienyl]methyl}methylcarbamate (525 mg), piperidin-2-one (233 mg), cesium carbonate (769 mg), tris(dibenzylideneacetone)dipalladium(0) (54 mg) and 4,5-bis(diphenylphosphino)-9,9-dimethylxanthine (68 mg) were suspended in toluene (5 mL), and the suspension was stirred at 170°C for 4 hr under microwave irradiation. The mixture was allowed to cool to room temperature, water was added, and the mixture was extracted with ethyl acetate. The extract was washed with saturated brine, dried over anhydrous sodium sulfate, and concentrated under reduced pressure. The residue was purified by silica gel column chromatography (eluent: ethyl acetate→ethyl acetate-methanol=20:1) and basic silica gel column chromatography (eluent: hexane-ethyl acetate=1:1→1:4) to give the title compound as a pale-yellow oil (394 mg, yield 72%).
¹H-NMR (CDCl₃) δ: 1.47(9H,s),1.86-2.03(4H,m),2.39(2H,t,J=6.0Hz),2.89(3H,s),3.63-3.67(2H,m),4.47(2H,brs),6.71(1H,s),7.47-7.60(3H,m),7.91-7.94(2H,m).

### Reference Example 113

### tert-butyl {[4-(2-bromophenyl)-5-(pyridin-3-ylsulfonyl)thiophen-2-yl]methyl}methylcarbamate

4-(2-Bromophenyl)-5-(pyridin-3-ylsulfonyl)thiophene-2-carbaldehyde (246 mg) was dissolved in a mixed solvent of tetrahydrofuran (3 mL) and methanol (1 mL), 40% methylamine-methanol solution (0.6 mL) was added, and the mixture was stirred at room temperature for 18 hr. The reaction mixture was concentrated under reduced pressure, and the residue was dissolved again in a mixed solvent of tetrahydrofuran (5 mL) and methanol (2 mL). Sodium borohydride (68 mg) was added under ice-cooling, and the mixture was stirred at room temperature for 2 hr, treated with 1 mol/L hydrochloric acid, and concentrated under reduced pressure. Saturated aqueous sodium hydrogen carbonate solution and ethyl acetate were added to the residue, di-tert-butyl bicarbonate (158 mg) was added, and the mixture was stirred for 1 hr. The ethyl acetate layer of the reaction mixture was washed with saturated brine, dried over anhydrous sodium sulfate, and concentrated under reduced pressure. The residue was purified by silica gel column chromatography (eluent: hexane-ethyl acetate=4:1→2:1) to give the title compound as a pale-yellow oil (214 mg, yield 68%).
¹H-NMR (CDCl₃) δ: 1.51(9H,s),2.91(3H,s),4.54(2H,br),6.83(1H,brs),7.23-7.31(2H,m),7.41-7.47(3H,m),7.64-7.68(1H,m),8.54-8.55(1H,m),8.70-8.72(1H,m).

### Reference Example 114

### tert-butyl ({4-(2-cyanopyridin-3-yl)-5-[(3-fluorophenyl)sulfonyl]thiophen-2-yl}methyl)methylcarbamate

tert-Butyl ({4-(2-chloropyridin-3-yl)-5-[(3-fluorophenyl)sulfonyl]thiophen-2-yl}methyl)methylcarbamate (305 mg), zinc cyanide (108 mg) and tetrakis(triphenylphosphine) palladium(0) (142 mg) were suspended in N,N-dimethylformamide (10 mL), and the suspension was stirred at 80°C for 8 hr. The reaction mixture was allowed to cool to room temperature, water and ethyl acetate were added, and the mixture was filtered through celite. The organic layer of the filtrate was washed successively with water and saturated brine, dried over anhydrous sodium sulfate, and concentrated under reduced pressure. The residue was purified by silica gel column chromatography (eluent: hexane-ethyl acetate=17:1→3:1) to give the crude title compound as a pale-yellow oil (198 mg, yield 66%).
¹H-NMR (CDCl₃) δ: 1.51(9H,s),2.92(3H,s),4.59(2H,brs),6.95(1H,brs),7.12-7.15(1H,m),7.23-7.28(2H,m),7.36-7.43(1H,m),7.60-7.64(1H,m),7.96-7.98(1H,m),8.74-8.75(1H,m).

### Reference Example 115

### tert-butyl {[4-(2-chloropyridin-3-yl)-5-(pyridin-3-ylsulfonyl)thiophen-2-yl]methyl}methylcarbamate

4-(2-Chloropyridin-3-yl)-5-(pyridin-3-ylsulfonyl)thiophene-2-carbaldehyde (136 mg) was dissolved in a mixed solvent of tetrahydrofuran (3 mL) and methanol (1 mL), 40% methylamine-methanol solution (0.4 mL) was added, and the mixture was stirred at room temperature for 18 hr. The reaction mixture was concentrated under reduced pressure, and the residue was dissolved again in a mixed solvent of tetrahydrofuran (5 mL) and methanol (2 mL). Sodium borohydride (22 mg) was added under ice-cooling, and the mixture was stirred at room temperature for 2 hr, treated with 1 mol/L hydrochloric acid, and concentrated under reduced pressure. Saturated aqueous sodium hydrogen carbonate solution was added to the residue, and the mixture was extracted with ethyl acetate. The extract was washed with saturated brine, dried over anhydrous sodium sulfate, and concentrated under reduced pressure. The residue was dissolved in tetrahydrofuran (3 mL), di-tert-butyl bicarbonate (97 mg) was added, and the mixture was stirred for 10 min. The reaction mixture was concentrated under reduced pressure, and the residue was purified by silica gel column chromatography (eluent: hexane-ethyl acetate=1:1→1:3) to give the title compound as a colorless oil (67 mg, yield 38%).
¹H-NMR (CDCl₃) δ: 1.50(9H,s),2.92(3H,s),4.57(2H,br),6.87(1H,brs),7.28-7.40(2H,m),7.62-7.66(1H,m),7.80-7.83(1H,m),8.45-8.47(1H,m),8.68-8.69(1H,m),8.73-8.75(1H,m).

### Reference Example 116

### tert-butyl {[4-(2-cyanophenyl)-5-(pyridin-3-ylsulfonyl)thiophen-2-yl]methyl}methylcarbamate

tert-Butyl {[4-(2-bromophenyl)-5-(pyridin-3-ylsulfonyl)thiophen-2-yl]methyl}methylcarbamate (214 mg), zinc cyanide (96 mg) and tetrakis(triphenylphosphine) palladium (0) (47 mg) were suspended in N,N-dimethylformamide (5 mL), and the suspension was stirred at 140°C for 30 min under microwave irradiation. The reaction mixture was allowed to cool to room temperature, water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=3:1→1:1) to give the crude title compound as a colorless oil (150 mg, yield 78%).
¹H-NMR(CDCl₃)δ: 1.51(9H,s),2.92(3H,s),4.59(2H,brs),6.95(1H,brs),7.12-7.15(1H,m),7.23-7.28(2H,m),7.36-7.43(1H,m),7.60-7.64(1H,m),7.96-7.98(1H,m),8.74-8.75(1H,m).

### Reference Example 117

### tert-butyl ({5-[(3-bromophenyl)sulfonyl]-4-(2-fluoropyridin-3-yl)thiophen-2-yl}methyl)methylcarbamate

To a solution of tert-butyl ({5-[(3-bromophenyl)thio]-4-(2-fluoropyridin-3-yl)thiophen-2-yl}methyl)methylcarbamate (508 mg) in ethyl acetate (10 mL) was added 3-chloroperbenzoic acid (955 mg), and the mixture was stirred at room temperature for 18 hr, treated with saturated aqueous sodium thiosulfate solution, and extracted with ethyl acetate. The extract was washed successively with water, saturated aqueous sodium hydrogen carbonate solution and saturated brine, dried over anhydrous sodium sulfate, and concentrated under reduced pressure. The residue was purified by basic silica gel column chromatography (eluent: hexane-ethyl acetate=9:1→4:1) to give the title compound as a colorless amorphous solid (419 mg, yield 78%).
¹H-NMR (CDCl₃) δ: 1.50(9H,brs),2.93(3H,brs),4.55(2H,br),6.88(1H,s),7.22-7.34(2H,m),7.41-7.45(1H,m),7.58-7.65(2H,m),7.91(1H,br),8.27-8.29(1H,m).

### Reference Example 118

### tert-butyl ({5-[(3-cyanophenyl)sulfonyl]-4-(2-fluoropyridin-3-yl)thiophen-2-yl}methyl)methylcarbamate

tert-Butyl ({5-[(3-bromophenyl)sulfonyl]-4-(2-fluoropyridin-3-yl)thiophen-2-yl}methyl)methylcarbamate (419 mg), zinc cyanide (136 mg) and tetrakis(triphenylphosphine) palladium(0) (89 mg) were suspended in N,N-dimethylformamide (5 mL), and the suspension was stirred at 140°C for 30 min under microwave irradiation. The reaction mixture was allowed to cool to room temperature, water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=4:1→2:1) to give the crude title compound as a colorless oil (335 mg, yield 89%).
¹H-NMR (CDCl₃) δ: 1.50(9H,s),2.94(3H,s),4.57(2H,brs),6.89(1H,s),7.32-7.36(1H,m),7.49-7.55(1H,m),7.68-7.7.81(3H,m),7.89-7.7.94(1H,m),8.29-8.31(1H,m).

### Reference Example 119

### tert-butyl ({4-(2-fluoropyridin-3-yl)-5-[(3-formylphenyl)sulfonyl]thiophen-2-yl}methyl)methylcarbamate

A solution of tert-butyl ({5-[(3-cyanophenyl)sulfonyl]-4-(2-fluoropyridin-3-yl)thiophen-2-yl}methyl)methylcarbamate (143 mg) in tetrahydrofuran (2 mL) was cooled to -78°C, and 1.5 mol/L diisobutylaluminum hydride-toluene solution (5.8 mL) was added dropwise. The reaction mixture was stirred at room temperature for 2 hr, treated with 1 mol/L hydrochloric acid, and extracted with ethyl acetate. The extract was washed successively with water, saturated aqueous sodium hydrogen carbonate solution and saturated brine, dried over anhydrous sodium sulfate, and concentrated under reduced pressure. The residue was purified by silica gel column chromatography (eluent: hexane-ethyl acetate=6:1→3:1) to give the title compound as a colorless oil (30 mg, yield 21%).
¹H=NMR(CDCl₃) δ: 1.49(9H,s),2.93(3H,s),4.56(2H,br),6.88(1H,s),7.31-7.35(1H,m),7.54-7.59(1H,s),7.73-7.76(1H,m),7.93-8.04(3H,m),8.24-8.25(1H,m),9.92(1H,s).

### Reference Example 120

### tert-butyl {[4-(2-fluoropyridin-3-yl)-5-{[3-(hydroxymethyl)phenyl]sulfonyl}thiophen-2-yl]methyl}methylcarbamate

To a solution of tert-butyl ({4-(2-fluoropyridin-3-yl)-5-[(3-formylphenyl)sulfonyl]thiophen-2-yl}methyl)methylcarbamate (253 mg) in tetrahydrofuran (3 mL) were added sodium borohydride (24 mg) and ethanol (1 mL) at room temperature, and the mixture was stirred for 1 hr, treated with 1 mol/L hydrochloric acid, and extracted with ethyl acetate. The extract was washed successively with water, saturated aqueous sodium hydrogen carbonate solution and saturated brine, dried over anhydrous sodium sulfate, and concentrated under reduced pressure. The residue was purified by silica gel column chromatography (eluent: hexane-ethyl acetate=1:1→1:3) to give the title compound as a colorless oil (220 mg, yield 86%).
¹H-NMR(CDCl₃)δ: 1.49(9H,s),2.06(1H,t,J=6.0Hz),2.92(3H,s),4.53(2H,br),4.60(2H,d ,J=6.0Hz),6.85(1H,s),7.25-7.40(3H,m),7.47-7.60(2H,s),7.90-8.00(1H,m),8.25-8.26(1H,m).

### Reference Example 121

### tert-butyl {[4-(2-fluoropyridin-3-yl)-5-(thiophen-3-ylsulfonyl)thiophen-2-yl]methyl}methylcarbamate

To a solution of tert-butyl ({4-(2-fluoropyridin-3-yl)-5-[(thiophen-3-yl)thio]thiophen-2-yl}methyl)methylcarbamate (210 mg) in ethyl acetate (5 mL) was added 3-chloroperbenzoic acid (356 mg), and the mixture was stirred for 18 hr. Potassium carbonate, anhydrous sodium sulfate and celite were added to the reaction mixture, and the mixture was stirred for 30 min. The insoluble material was filtered off, and the filtrate was concentrated under reduced pressure. The residue was purified by basic silica gel column chromatography (eluent: hexane-ethyl acetate=75:25→20:80) to give the title compound as a colorless solid (212 mg, yield 94%).
¹H-NMR (CDCl₃) δ: 1.49(9H,s),2.93(3H,s),4.56(2H,brs),6.90(1H,brs),7.03(1H,dd,J=5 .2,1.2Hz),7.28-7.33(2H,m),7.63(1H,dd,J=3.0,1.3Hz),7.96(1H,t,J=8.0Hz),8.23-8.29(1H,m).

### Reference Example 122

### tert-butyl ({4-(2-fluoropyridin-3-yl)-5-[(2-methylfuran-3-yl)sulfonyl]thiophen-2-yl}methyl)methylcarbamate

In the same manner as in Reference Example 121 and using tert-butyl ({4-(2-fluoropyridin-3-yl)-5-[(2-methylfuran-3-yl)thio]thiophen-2-yl}methyl)methylcarbamate (206 mg) and 3-chloroperbenzoic acid (350 mg), the title compound was obtained as a colorless oil (187 mg, yield 85%).
¹H-NMR(CDCl₃)δ: 1.50(9H,s),2.27(3H,s),2.93(3H,s),4.56(2H,brs),6.13(1H,d,J=2.3H z),6.91(1H,s),7.15(1H,d,J=1.9Hz),7.23-7.35(1H,m),7.89-8.04(1H,m),8.26(1H,d,J=4.5Hz).

### Reference Example 123

### tert-butyl {[4-(2-fluoropyridin-3-yl)-5-(1,3-thiazol-2-ylsulfonyl)thiophen-2-yl]methyl}methylcarbamate

In the same manner as in Reference Example 121 and using tert-butyl ({4-(2-fluoropyridin-3-yl)-5-[(1,3-thiazol-2-yl)thio]thiophen-2-yl}methyl)methylcarbamate (185 mg) and 3-chloroperbenzoic acid (314 mg), the title compound was obtained as a white powder (86 mg, yield 43%).
¹H-NMR(CDCl₃)δ: 1.49(9H,s),2.94(3H,s),4.58(2H,brs),6.97(1H,s),7.27-7.34(1H,m),7.62(1H,d,J=3.0Hz),7.93(1H,d,J=3.0Hz),8.04-8.12(1H,m),8.24-8.29(1H,m).

### Reference Example 124

### tert-butyl {[4-(2-fluoropyridin-3-yl)-5-(1H-imidazol-2-ylsulfonyl)thiophen-2-yl]methyl}methylcarbamate

In the same manner as in Reference Example 121 and using tert-butyl ({4-(2-fluoropyridin-3-yl)-5-[(1H-imidazol-2-yl)thio]thiophen-2-yl}methyl)methylcarbamate (126 mg) and 3-chloroperbenzoic acid (259 mg), the title compound was obtained as a white powder (121 mg, yield 89%).
¹H-NMR(CDCl₃)δ: 1.48(9H,s),2.93(3H,s),4.57(2H,brs),6.91(1H,brs),7.10(1H,brs),7 .19-7.31(2H,m),7.85-8.00(1H,m),8.18(1H,dd,J=4.9,1.1Hz),11.53(1H,brs).

### Reference Example 125

### tert-butyl ({4-(2-fluoropyridin-3-yl)-5-[(1-methyl-1H-imidazol-2-yl)thio]thiophen-2-yl}methyl)methylcarbamate

To a solution of tert-butyl {[4-(2-fluoropyridin-3-yl)-5-(1H-imidazol-2-ylsulfonyl)thiophen-2-yl]methyl}methylcarbamate (40 mg) in N,N-dimethylformamide (1 mL) were added potassium carbonate (18.3 mg) and iodomethane (19 mg) at room temperature, and the mixture was stirred at room temperature for 48 hr. The reaction mixture was diluted with water, and extracted with ethyl acetate. The extract was washed with saturated brine, dried over anhydrous magnesium sulfate, and concentrated under reduced pressure to give a residue. The obtained residue was purified by silica gel column chromatography (eluent: hexane-ethyl acetate=1:1→ethyl acetate) to give the title compound as a white powder (29.7 mg, yield 72%).
¹H-NMR(CDCl₃)δ: 1.50(9H,s),2.94(3H,s),3.76(3H,s),4.58(2H,brs),6.92(1Hb,rs),6.9 6(1H,brs),7.04(1H,d,J=0.9Hz),7.22-7.31(1H,m),7.94-8.06(1H,m),8.20-8.27(1H,m).

### Reference Example 126

### tert-butyl ({5-[(2-chloropyridin-4-yl)sulfonyl]-4-(2-fluoropyridin-3-yl)thiophen-2-yl}methyl)methylcarbamate

In the same manner as in Reference Example 121 and using tert-butyl ({5-[(2-chloropyridin-4-yl)thio]-4-(2-fluoropyridin-3-yl)thiophen-2-yl}methyl)methylcarbamate (317 mg) and 3-chloroperbenzoic acid (503 mg), the title compound was obtained as a white powder (296 mg, yield 87%).
¹H-NMR(CDCl₃)δ: 1.50(9H,s),2.94(3H,s),4.58(2H,brs),6.94(1H,s),7.24-7.42(3H,m),7.88(1H,t,J=8.0Hz),8.33(1H,d,J=4.5Hz),8.50(1H,d,J=4 .9Hz).

### Reference Example 127

### tert-butyl {[4-(2-fluoropyridin-3-yl)-5-(pyridin-4-ylsulfonyl)thiophen-2-yl]methyl}methylcarbamate

To a solution of tert-butyl ({5-[(2-chloropyridin-4-yl)sulfonyl]-4-(2-fluoropyridin-3-yl)thiophen-2-yl}methyl)methylcarbamate (105 mg) in methanol (3 mL) was added triethylamine (0.044 mL). 10% Palladium-carbon (50% water-containing product, 21 mg) was added under a nitrogen atmosphere, and the mixture was stirred for 48 hr under a hydrogen atmosphere. The reaction mixture was filtered, and the filtrate was concentrated under reduced pressure to give a residue. The obtained residue was purified by silica gel column chromatography ((eluent: hexane-ethyl acetate=3:2→ethyl acetate) to give the title compound as a colorless oil (80 mg, yield 82%).
¹H-NMR(CDCl₃)δ: 1.49(9H,s),2.93(3H,s),4.57(2H,brs),6.92(1H,s),7.29-7.35(1H,m),7.38(2H,d,J=5.8Hz),7.85-7.96(1H,m),8.30(1H,d,J=4.7Hz),8.71-8.77(2H,m).

### Reference Example 128

### tert-butyl ({4-(2-fluoropyridin-3-yl)-5-[(1-oxidepyridin-4-yl)sulfonyl]thiophen-2-yl}methyl)methylcarbamate

In the same manner as in Reference Example 121 and using tert-butyl ({4-(2-fluoropyridin-3-yl)-5-[(pyridin-4-yl)thio]thiophen-2-yl}methyl)methylcarbamate (112 mg) and 3-chloroperbenzoic acid (150 mg), the title compound was obtained as a colorless oil (16.7 mg, yield 13%).
¹H-NMR(CDCl₃) δ: (9H,s),2.94(3H,s),4.57(2H,brs),6.93(1H,s),7.29-7.41(3H,m),7.86-7.96(1H,m),8.06-8.14(2H,m),8.32(1H,d,J=4.5Hz).

### Reference Example 129

### tert-butyl ({5-[(6-chloropyridin-3-yl)sulfonyl]-4-(2-fluoropyridin-3-yl)thiophen-2-yl}methyl)methylcarbamate

In the same manner as in Reference Example 121 and using tert-butyl ({5-[(6-chloropyridin-3-yl)thio]-4-(2-fluoropyridin-3-yl)thiophen-2-yl}methyl)methylcarbamate (683 mg) and 3-chloroperbenzoic acid (1.08 g), the title compound was obtained as a white powder (650 mg, yield 89%).
¹H-NMR (CDCl₃) δ: 1.50(9H,s),2.93(3H,s),4.57(2H,brs),6.92(1H,s),7.29-7.38(2H,m),7.71(1H,dd,J=8.4,2.5Hz),7.87-7.97(1H,m),8.28-8.33(1H,m),8.53(1H,d,J=2.3Hz).

### Reference Example 130

### tert-butyl {[4-(2-fluoropyridin-3-yl)-5-(pyridin-2-ylsulfonyl)thiophen-2-yl]methyl}methylcarbamate

To a solution of tert-butyl ({4-(2-fluoropyridin-3-yl)-5-[(pyridin-2-yl)thio]thiophen-2-yl}methyl)methylcarbamate (326 mg) in acetic acid (4 mL) was added 3-chloroperbenzoic acid (384 mg), and the mixture was stirred at room temperature for 18 hr, and concentrated under reduced pressure. The residue was basified with saturated aqueous sodium hydrogen carbonate solution, and the mixture was extracted with ethyl acetate. The extract was washed successively with saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=1:1) to give the title compound as a yellow oil (231 mg, yield 65%).
¹H-NMR(CDCl₃)δ: 1.49(9H,s),2.93(3H,s),4.56(2H,brs),6.92(1H,s),7.22-7.28(1H,m),7.40-7.45(1H,m),7.73-7.82(2H,m),7.98-8.03(1H,m),8.21-8.22(1H,m),8.60-8.62(1H,m).

### Reference Example 131

### tert-butyl {dideutero[4-(2-fluoropyridin-3-yl)-5-(pyridin-3-ylsulfonyl)thiophen-2-yl]methyl}methylcarbamate

4-(2-Fluoropyridin-3-yl)-5-(pyridin-3-ylsulfonyl)thiophene-2-deuterocarbaldehyde (696 mg) was dissolved in a mixed solvent of tetrahydrofuran (5 mL) and methanol (1 mL), and 40% methylamine-methanol solution (2.1 mL) was added at room temperature. The reaction mixture was stirred for 18 hr, and concentrated under reduced pressure. The residue was dissolved in tetrahydrofuran (3 mL), deuterated sodium borohydride (103 mg) and deuterated methanol (2 mL) were added under ice-cooling, and the mixture was further stirred at room temperature for 18 hr. Water was added to the reaction mixture, and then ethyl acetate was added. Di-tert-butyl bicarbonate (237 mg) was added, and the mixture was stirred for 1 hr. The organic layer of the reaction mixture was washed with saturated brine, dried over anhydrous sodium sulfate, and concentrated under reduced pressure. The residue was purified by basic silica gel column chromatography (eluent: hexane-ethyl acetate=9:1→4:1) to give the title compound as a colorless oil (134 mg, yield 14%).
¹H-NMR(CDCl₃)δ:1.50(9H,s),2.92(3H,br),6.90(1H,s),7.30-7.35(2H,m),7.74-7.77(1H,m),7.88-7.95(1H,m),8.27-8.29(1H,m),8.73-8.75(2H,m).

### Reference Example 132

### 2-ethylhexyl 3-{[2-{[(tert-butoxycarbonyl)(methyl)amino]methyl}-4-(2-fluorophenyl)-1,3-thiazol-5-yl]thio}propanoate

tert-Butyl {[5-bromo-4-(2-fluorophenyl)-1,3-thiazol-2-yl]methyl}methylcarbamate (1.18 g), 2-ethylhexyl 3-mercaptopropanoate (965 mg), tris(dibenzylideneacetone)dipalladium(0) (134 mg), 9,9-dimethyl-4,5-bis(diphenylphosphino)xanthine (173 mg) and cesium carbonate (1.92 g) were stirred in toluene (15 mL) at 105°C for 12 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=6:1) to give the title compound as a yellow oil (1.46 g, yield 92%).
¹H-NMR(CDCl₃)δ: 0.84-0.91(6H,m),1.23-1.37(9H,m),1.51(9H,s),2.53(2H,t,J=7.2Hz),2.94-2.99(5H,m),3.89-3.96(2H,m),4.65-4.69(2H,m),7.11-7.22(2H,m),7.35-7.50(2H,m).

### Reference Example 133

### 2-ethylhexyl 3-{[2-{[(tert-butoxycarbonyl)(methyl)amino]methyl}-4-(2-fluoropyridin-3-yl)-1,3-thiazol-5-yl]thio}propanoate

tert-Butyl {[5-bromo-4-(2-fluoropyridin-3-yl)-1,3-thiazol-2-yl]methyl}methylcarbamate (1.77 g), 2-ethylhexyl 3-mercaptopropanoate (1.46 g), tris(dibenzylideneacetone)dipalladium(0) (202 mg), 9,9-dimethyl-4,5-bis(diphenylphosphino)xanthine (255 mg) and cesium carbonate (2.87 g) were stirred in toluene (20 mL) at 105°C for 14 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=3:1) to give the title compound as a yellow oil (1.97 g, yield 83%).
¹H-NMR(CDCl₃)δ: 0.84-0.89(6H,m), 1.24-1.37(9H,m),1.51(9H,s),2.56(2H,t,J=7.2Hz),2.98-3.03(5H,m),3.94-3,97(2H,m),4.62-4.68(2H,m),7.25-7.29(1H,m),7.91-7.97(1H,m),8.26(1H,brs).

### Reference Example 134

### 1-(3-iodobenzyl)pyrrolidine

To pyrrolidine (0.2 mL) in methanol (10 mL) was added a solution of 3-iodobenzaldehyde (565 mg) in tetrahydrofuran (5 mL), and the mixture was stirred at room temperature for 12 hr. Sodium borohydride (109 mg) was added at 0°C, and the mixture was stirred at room temperature for 2 hr, and concentrated under reduced pressure. Water was added to the residue, and the mixture was extracted with ethyl acetate. 1 mol/L Hydrochloric acid was added to the extract, and the aqueous layer was washed with ethyl acetate. The obtained aqueous layer was basified with 1 mol/L aqueous sodium hydroxide solution, and the mixture was extracted with ethyl acetate. The extract was washed successively with saturated aqueous sodium hydrogen carbonate solution, water and saturated brine, dried over anhydrous sodium sulfate, and concentrated under reduced pressure to give the title compound as a yellow oil (382 mg, yield 55%).
¹H-NMR(CDCl₃)δ: 1.76-1.82(4H,m),2.46-2.51(4H,m),3.54(2H,s),7.03(1H,t,J=7.5Hz),7.27-7.30(1H,m),7.55-7.58(1H,m),7.69-7.70(1H,m).

### Reference Example 135

### tert-butyl ({5-[(3-bromophenyl)thio]-4-(2-fluorophenyl)-1,3-thiazol-2-yl}methyl)methylcarbamate

tert-Butyl {[5-bromo-4-(2-fluorophenyl)-1,3-thiazol-2-yl]methyl}methylcarbamate (1.0 g), 3-bromothiophenol (0.32 mL), tris(dibenzylideneacetone)dipalladium(0) (119 mg), 9,9-dimethyl-4,5-bis(diphenylphosphino)xanthine (150 mg) and N-ethyldiisopropylamine (0.88 mL) were stirred in toluene (15 mL) at 105°C for 14 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=5:1) to give the title compound as a yellow oil (645 mg, yield 49%).
¹H-NMR(CDCl₃)δ: 1.42(9H,brs),2.99(3H,brs),4.67-4.72(2H,m),7.04-7.48(8H,m).

### Reference Example 136

### tert-butyl ({4-(2-fluorophenyl)-5-[(3-formylphenyl)thio]-1,3-thiazol-2-yl}methyl)methylcarbamate

To a solution of 2-ethylhexyl 3-{[2-{[(tert-butoxycarbonyl)(methyl)amino]methyl}-4-(2-fluorophenyl)-1,3-thiazol-5-yl]thio}propanoate (749 mg) in ethanol (10 mL) was added sodium ethoxide (381 mg) at 0°C, and the mixture was stirred at room temperature for 4 hr, and concentrated under reduced pressure. A mixture of the residue, 3-iodobenzaldehyde (489 mg), tris(dibenzylideneacetone)dipalladium(0) (35 mg) and 9,9-dimethyl-4,5-bis(diphenylphosphino)xanthine (81 mg) in toluene (10 mL) was stirred at 80°C for 3 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=4:1) to give the title compound as a yellow oil (676 mg, yield quantitative).
¹H-NMR(CDCl₃)δ: 1.47(9H,brs),3.00(3H,brs),4.69(2H,brs),7.10-7.20(3H,m),7.34-7.50(3H,m),7.65-7.68(2H,m),9.90(1H,s).

### Reference Example 137

### tert-butyl {[5-{[3-(dimethoxymethyl)phenyl]thio}-4-(2-fluorophenyl)-1,3-thiazol-2-yl]methyl}methylcarbamate

To a solution of tert-butyl ({4-(2-fluorophenyl)-5-[(3-formylphenyl)thio]-1,3-thiazol-2-yl}methyl)methylcarbamate (665 mg) in methanol (10 mL) was added ruthenium (III) chloride (3.7 mg), and the mixture was stirred at room temperature for 12 hr, and concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=4:1) to give the title compound as a yellow oil (617 mg, yield 84%).
¹H-NMR(CDCl₃)δ: 1.47(9H,brs),2.98(3H,brs),3.72(6H,s),4.60-4.70(2H,m),5.30(1H,s),7.10-7.28(5H,m),7.31-7.40(2H,m),7.44-7.49(1H,m).

### Reference Example 138

### tert-butyl {[4-(2-fluorophenyl)-5-{[3-(pyrrolidin-1-ylmethyl)phenyl]thio}-1,3-thiazol-2-yl]methyl}methylcarbamate

To a solution of 2-ethylhexyl 3-{[2-{[(tert-butoxycarbonyl)(methyl)amino]methyl}-4-(2-fluorophenyl)-1,3-thiazol-5-yl]thio}propanoate (696 mg) in ethanol (10 mL) was added sodium ethoxide (177 mg) at 0°C, and the mixture was stirred at room temperature for 1 hr, and concentrated under reduced pressure. A mixture of the residue, 1-(3-iodobenzyl)pyrrolidine (379 mg), tris(dibenzylideneacetone)dipalladium(0) (60 mg) and 9,9-dimethyl-4,5-bis(diphenylphosphino)xanthine (75 mg) was stirred in toluene (10 mL) at 105°C for 4 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by basic silica gel column chromatography (eluent: hexane-ethyl acetate=5:1) to give the title compound as a yellow oil (569 mg, yield 86%).
¹H-NMR(CDCl₃)δ: 1.46(9H,brs),1.74-1.81(4H,m),2.42-2.47(4H,m),2.98(3H,brs),3.53(2H,s),4.65-4.71(2H,m),7.03-7.21(6H,m),7.33-7.40(1H,m),7.44-7.50(1H,m).

### Reference Example 139

### tert-butyl ({5-[(6-chloropyridin-3-yl)thio]-4-(2-fluorophenyl)-1,3-thiazol-2-yl}methyl)methylcarbamate

To a solution of 2-ethylhexyl 3-{[2-{[(tert-butoxycarbonyl)(methyl)amino]methyl}-4-(2-fluorophenyl)-1,3-thiazol-5-yl]thio}propanoate (1.28 g) in ethanol (15 mL) was added sodium ethoxide (328 mg) at 0°C, and the mixture was stirred at room temperature for 1 hr, and concentrated under reduced pressure. A mixture of the residue, 2-chloro-5-iodopyridine (606 mg), tris(dibenzylideneacetone)dipalladium(0) (110 mg) and 9,9-dimethyl-4,5-bis(diphenylphosphino)xanthine (139 mg) was stirred in toluene (15 mL) at 80°C for 3 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by basic silica gel column chromatography (eluent: hexane-ethyl acetate=5:1) to give the title compound as a yellow oil (968 mg, yield 87%). ¹H-NMR(CDCl₃)δ: 1.48(9H,brs),2.99(3H,s),4.67-4.70(2H,m),7.12-7.25(3H,m),7.37-7.48(3H,m),8.17-8.18(1H,m).

### Reference Example 140

### tert-butyl ({4-(2-fluorophenyl)-5-[(1-methyl-1H-pyrazol-4-yl)thio]-1,3-thiazol-2-yl}methyl)methylcarbamate

To a solution of 2-ethylhexyl 3-{[2-{[(tert-butoxycarbonyl)(methyl)amino]methyl}-4-(2-fluorophenyl)-1,3-thiazol-5-yl]thio}propanoate (356 mg) in ethanol (4 mL) was added sodium ethoxide (89 mg) at 0°C, and the mixture was stirred at room temperature for 1 hr, and concentrated under reduced pressure. A mixture of the residue, 4-iodo-1-methyl-1H-pyrazole (155 mg), tris(dibenzylideneacetone)dipalladium(0) (32 mg) and 9,9-dimethyl-4,5-bis(diphenylphosphino)xanthine (39 mg) was stirred in toluene (5 mL) at 80°C for 2 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by basic silica gel column chromatography (eluent: hexane-ethyl acetate=1:1) to give the title compound as a yellow oil (208 mg, yield 27%).
¹H-NMR(CDCl₃)δ: 1.46(9H,brs),2.94(3H,brs),3.85(3H,s),4.58-4.62(2H,m),7.15-7.26(2H,m),7.37-7.44(3H,m),7.48-7.53(1H,m).

### Reference Example 141

### tert-butyl ({5-[(3,4-dimethoxyphenyl)thio]-4-(2-fluoropyridin-3-yl)-1,3-thiazol-2-yl}methyl)methylcarbamate

tert-Butyl {[5-bromo-4-(2-fluoropyridin-3-yl)-1,3-thiazol-2-yl]methyl}methylcarbamate (236 mg), 3,4-dimethoxythiophenol (152 mg), tris(dibenzylideneacetone)dipalladium(0) (17 mg), 9,9-dimethyl-4,5-bis(diphenylphosphino)xanthine (21 mg) and N-ethyldiisopropylamine (0.20 mL) were stirred in toluene (6 mL) at 110°C for 1 hr under microwave irradiation. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=1:1) to give the title compound as a yellow oil (223 mg, yield 77%).
¹H-NMR(CDCl₃)δ: 1.44-1.47(9H,m),2.96(3H,brs),3.80(3H,s),3.85(3H,s),4.63(2H,brd,J=14 .7Hz),6.75-6.81(2H,m),6.88-6.91(1H,m),6.88-6.91(1H,m),7.25-7.29(1H,m),7.90-7.97(1H,m),8.26-7.28(1H,m).

### Reference Example 142

### tert-butyl ({5-[(6-chloropyridin-3-yl)thio]-4-(2-fluoropyridin-3-yl)-1,3-thiazol-2-yl}methyl)methylcarbamate

To a solution of 2-ethylhexyl 3-{[2-{[(tert-butoxycarbonyl)(methyl)amino]methyl}-4-(2-fluoropyridin-3-yl)-1,3-thiazol-5-yl]thio}propanoate (1.97 g) in ethanol (20 mL) was added sodium ethoxide (500 mg) at 0°C, and the mixture was stirred at room temperature for 1 hr, and concentrated under reduced pressure. A mixture of the residue, 2-chloro-5-iodopyridine (970 mg), tris(dibenzylideneacetone)dipalladium(0) (169 mg) and 9,9-dimethyl-4,5-bis(diphenylphosphino)xanthine (214 mg) was stirred in toluene (20 mL) at 80°C for 3 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by basic silica gel column chromatography (eluent: hexane-ethyl acetate=4:1) to give the title compound as a yellow oil (1.45 g, yield 85%).
¹H-NMR(CDCl₃)δ: 1.48(9H,brs),2.99(3H,s),4.69(2H,brs),7.20-7.31(2H,m),7.43-7.47(1H,m),7.89-7.96(1H,m),8.19-8.20(1H,m),8.18-8.19(1H,m).

### Reference Example 143

### tert-butyl ({5-[(2-chloropyridin-4-yl)thio]-4-(2-fluoropyridin-3-yl)-1,3-thiazol-2-yl}methyl)methylcarbamate

To a solution of 2-ethylhexyl 3-{[2-{[(tert-butoxycarbonyl)(methyl)amino]methyl}-4-(2-fluoropyridin-3-yl)-1,3-thiazol-5-yllthiolpropanoate (1.01 g) in ethanol (15 mL) was added sodium ethoxide (287 mg) at 0°C, and the mixture was stirred at room temperature for 2 hr, and concentrated under reduced pressure. A mixture of the residue, 2-chloro-4-iodopyridine (499 mg), tris(dibenzylideneacetone)dipalladium(0) (86 mg) and 9,9-dimethyl-4,5-bis(diphenylphosphino)xanthine (108 mg) was stirred in toluene (15 mL) at 80°C for 2 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=1:1) to give the title compound as a yellow oil (687 mg, yield 79%).
¹H-NMR(CDCl₃)δ: 1.50(9H,brs),3.04(3H,brs),4.70-4.76(2H,m),6.87-6.89(1H,m),6.96(1H,s),7.25-7.29(1H,m),7.87-7.93(1H,m),8.16-8.18(1H,m),8.26-8.28(1H,m).

### Reference Example 144

### tert-butyl ({5-[(3-bromophenyl)sulfonyl]-4-(2-fluorophenyl)-1,3-thiazol-2-yl}methyl)methylcarbamate

To a solution of tert-butyl ({5-[(3-bromophenyl)thio]-4-(2-fluorophenyl)-1,3-thiazol-2-yl}methyl)methylcarbamate (835 mg) in acetic acid (10 mL) was added 3-chloroperbenzoic acid (1.76 g), and the mixture was stirred at room temperature for 14 hr. Aqueous sodium thiosulfate solution was added to the reaction mixture, and the mixture was concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=3:1) to give the title compound as a yellow oil (424 mg, yield 48%).
¹H-NMR(CDCl₃)δ: 1.49-1.52(9H,m),3.01(3H,s),4.65-4.70(2H,m),7.03-7.09(1H,m),7.22-7.28(2H,m),7.37-7.54(3H,m),7.58-7.60(1H,m),7.64-7.66(1H,m).

### Reference Example 145

### tert-butyl {[4-(2-fluorophenyl)-5-{[3-(2-oxopyrrolidin-1-yl)phenyl]sulfonyl}-1,3-thiazol-2-yl]methyl}methylcarbamate

tert-Butyl ({5-[(3-bromophenyl)sulfonyl]-4-(2-fluorophenyl)-1,3-thiazol-2-yl}methyl)methylcarbamate (124 mg), 2-pyrrolidone(0.02 mL), tris(dibenzylideneacetone)dipalladium(0) (5.3 mg), 9,9-dimethyl-4,5-bis(diphenylphosphino)xanthine (6.8 mg) and cesium carbonate (152 mg) were stirred in toluene (2 mL) at 120°C for 1 hr under microwave irradiation. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=1:1) to give the title compound as a yellow oil (84 mg, yield 67%).
¹H-NMR(CDCl₃)δ: 1.51(9H,brs),2.12-2.20(2H,m),2.62(2H,t,J=8.1Hz),2.99(3H,s),3.73(2H,t,J=7.2Hz),4. 68(2H,brs),7.01-7.07(1H,m),7.18-7.23(1H,m),7.32-7.45(4H,m),7.52(1H,brs),8.19-8.22(1H,m).

### Reference Example 146

### tert-butyl ({4-(2-fluorophenyl)-5-[(3-pyrrolidin-1-ylphenyl)sulfonyl]-1,3-thiazol-2-yl}methyl)methylcarbamate

To a suspension of aluminum chloride (106 mg) in tetrahydrofuran (6 mL) was slowly added lithium aluminum hydride (31 mg) at 0°C, and the mixture was stirred at the same temperature for 15 min. A solution of tert-butyl {[4-(2-fluorophenyl)-5-{[3-(2-oxopyrrolidin-1-yl)phenyl]sulfonyl}-1,3-thiazol-2-yl]methyl}methylcarbamate (209 mg) in tetrahydrofuran (2 mL) was added dropwise to the obtained mixture at 0°C, and the obtained mixture was stirred at the same temperature for 30 min. 1 mol/L Aqueous sodium hydroxide solution was added to the reaction mixture, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=2:1) to give the title compound as a yellow oil (177 mg, yield 87%).
¹H-NMR(CDCl₃)δ: 1.47-1.51(9H,m),1.98-2.02(4H,m),2.99(3H,s),3.16-3.21(4H,m),4.62-4.68(2H,m),6.62-6.65(1H,m),6.69(1H,s),6.83-6.86(1H,m),7.02-7.08(1H,m),7.16-7.21(2H,m),7.40-7.41(2H,m).

### Reference Example 147

### tert-butyl {[4-(2-fluorophenyl)-5-{[3-(pyrrolidin-1-ylcarbonyl)phenyl]sulfonyl}-1,3-thiazol-2-yl]methyl}methylcarbamate

To a solution of tert-butyl {[5-{[3-(dimethoxymethyl)phenyl]thio}-4-(2-fluorophenyl)-1,3-thiazol-2-yl]methyl}methylcarbamate (600 mg) in acetic acid (10 mL) was added 3-chloroperbenzoic acid (1.18 g), and the mixture was stirred at room temperature for 14 hr. Aqueous sodium thiosulfate solution and 1 moL/L hydrochloric acid were added to the reaction mixture, and the mixture was extracted with ethyl acetate. The extract was washed successively with water and saturated brine, dried over anhydrous magnesium sulfate, and concentrated under reduced pressure. To a solution of the residue in N,N-dimethylformamide (10 mL) were added 1-ethyl-3-(dimethylaminopropyl)carbodiimide hydrochloride (1.37 g), 1-hydroxy-1H-benzotriazolehydrate (1.09 g) and pyrrolidine (0.6 mL), and the mixture was added for 10 hr. Water was added to the reaction mixture, and the mixture was extracted with ethyl acetate. The extract was washed successively with saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=2:1→ethyl acetate) to give the title compound as a yellow oil (439 mg, yield 66%).
¹H-NMR(CDCl₃)δ: 1.52(9H,brs), 1.83-1.99(4H,m),3.00(3H,s),3.28(2H,t,J=6.6Hz),3.62(2H,t,J=6.6Hz),4. 69(2H,brs),7.04(1H,t,J=8.7Hz),7.19-7.26(1H,m),7.36-7.44(3H,m),7.56-7.59(1H,m),7.70-7.73(2H,m).

### Reference Example 148

### tert-butyl {[4-(2-fluorophenyl)-5-{[3-(pyrrolidin-1-ylmethyl)phenyl]sulfonyl}-1,3-thiazol-2-yl]methyl}methylcarbamate

To a suspension of tert-butyl {[4-(2-fluorophenyl)-5-{[3-(pyrrolidin-1-ylmethyl)phenyl]thio}-1,3-thiazol-2-yl]methyl}methylcarbamate (490 mg) in a mixed solvent of acetonitrile (8 mL) and water (8 mL) was added sodium percarbonate (1.08 g), and the mixture was stirred at room temperature for 1 hr. Aqueous sodium thiosulfate solution was added to the reaction mixture, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by basic silica gel column chromatography (eluent: hexane-ethyl acetate=4:1) to give the title compound as a yellow oil (127 mg, yield 25%).
¹H-NMR(CDCl₃)δ: 1.48-1.52(9H,m),1.75-1.83(4H,m),2.41-2.46(4H,m),3.00(3H,s),3.54(2H,s),4.65-4.70(2H,m),6.99-7.05(1H,m),7.18-7.33(2H,m),7.39-7.46(3H,m),7.52-7.60(2H,m).

### Reference Example 149

### tert-butyl ({5-[(6-chloropyridin-3-yl)sulfonyl]-4-(2-fluorophenyl)-1,3-thiazol-2-yl}methyl)methylcarbamate

To a solution of tert-butyl ({5-[(6-chloropyridin-3-yl)thio]-4-(2-fluorophenyl)-1,3-thiazol-2-yl}methyl)methylcarbamate (902 mg) in acetic acid (10 mL) was added 3-chloroperbenzoic acid (1.65 g), and the mixture was stirred at room temperature for 14 hr. Aqueous sodium thiosulfate solution was added to the reaction mixture, and the mixture was concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=4:1) to give the title compound as a yellow oil (681 mg, yield 70%).
¹H-NMR(CDCl₃)δ: 1.52(9H,brs),3.01(3H,s),4.70(2H,brs),7.06-7.12(1H,m),7.24-7.28(1H,m),7.33-7.36(1H,m),7.39-7.50(2H,m),7.75-7.82(1H,m),8.49-8.50(1H,m).

### Reference Example 150

### tert-butyl {[4-(2-fluorophenyl)-5-(pyridin-3-ylsulfonyl)-1,3-thiazol-2-yl]methyl}methylcarbamate

To a solution of tert-butyl ({5-[(6-chloropyridin-3-yl)sulfonyl]-4-(2-fluorophenyl)-1,3-thiazol-2-yl}methyl)methylcarbamate (590 mg) in a mixed solvent of ethanol (20 mL) and tetrahydrofuran (5 mL) was added 10% palladium-carbon (50% water-containing product, 220 mg), and the mixture was stirred at room temperature for 2 hr under a hydrogen atmosphere. The insoluble material was filtered off, and the filtrate was concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=3:1→1:1) to give the title compound as a yellow oil (188 mg, yield 34%).
¹H-NMR(CDCl₃)δ: 1.52(9H,brs),3.01(3H,s),4.65-4.70(2H,m),7.05-7.08(1H,m),7.25-7.30(1H,m),7.32-7.35(1H,m),7.39-7.48(2H,m),7.82-7.85(1H,m),8.74-8.76(2H,m).

### Reference Example 151

### tert-butyl ({4-(2-fluorophenyl)-5-[(1-methyl-1H-pyrazol-4-yl)sulfonyl]-1,3-thiazol-2-yl}methyl)methylcarbamate

To a solution of tert-butyl ({4-(2-fluorophenyl)-5-[(1-methyl-1H-pyrazol-4-yl)thio]-1,3-thiazol-2-yl}methyl)methylcarbamate (203 mg) in acetic acid (5 mL) was added 3-chloroperbenzoic acid (450 mg), and the mixture was stirred at room temperature for 12 hr. Aqueous sodium thiosulfate solution was added to the reaction mixture, and the mixture was concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=1:1) to give the title compound as a yellow oil (181 mg, yield 83%).
¹H-NMR(CDCl₃)δ: 1.51(9H,brs),3.00(3H,s),3.84(3H,s),4.60-4.69(2H,m),7.08-7.14(1H,m),7.23-7.27(2H,m),7.43-7.51(3H,m).

### Reference Example 152

### tert-butyl ({5-[(3,4-dimethoxyphenyl)sulfonyl]-4-(2-fluoropyridin-3-yl)-1,3-thiazol-2-yl}methyl)methylcarbamate

To a solution of tert-butyl ({5-[(3,4-dimethoxyphenyl)thio]-4-(2-fluoropyridin-3-yl)-1,3-thiazol-2-yl}methyl)methylcarbamate (219 mg) in acetic acid (4 mL) was added 3-chloroperbenzoic acid (500 mg), and the mixture was stirred at room temperature for 10 hr. Aqueous sodium thiosulfate solution was added to the reaction mixture, and the mixture was concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=1:1) to give the title compound as a yellow oil (204 mg, yield 89%).
¹H-NMR(CDCl₃)δ: 1.47-1.50(9H,m),2.99(3H,s),3.82(3H,s),3.92(3H,s),4.66(2H,brs),6.83-7.86(1H,m),7.06(1H,d,J=2.1Hz),7.26-7.34(2H,m),7.95-8.00(1H,m),8.31-8.32(1H,m).

### Reference Example 153

### tert-butyl ({5-[(6-chloropyridin-3-yl)sulfonyl]-4-(2-fluoropyridin-3-yl)-1,3-thiazol-2-yl}methyl)methylcarbamate

To a solution of tert-butyl ({5-[(6-chloropyridin-3-yl)thio]-4-(2-fluoropyridin-3-yl)-1,3-thiazol-2-yl}methyl)methylcarbamate (1.43 g) in acetic acid (20 mL) was added 3-chloroperbenzoic acid (2.93 g), and the mixture was stirred at room temperature for 12 hr, and concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=2:1→1:1) to give the title compound as a colorless solid (1.15 g, yield 75%).
¹H-NMR(CDCl₃)δ: 1.51(9H,brs),3.01(3H,s),4.69(2H,brs),7.33-7.37(1H,m),7.41-7.44(1H,m),7.85-7.89(1H,m),7.92-7.98(1H,m),8.35-8.37(1H,m),8.66-8.70(1H,m).

### Reference Example 154

### tert-butyl {[4-(2-fluoropyridin-3-yl)-5-(pyridin-3-ylsulfonyl)-1,3-thiazol-2-yl]methyl}methylcarbamate

To a solution of tert-butyl ({5-[(6-chloropyridin-3-yl)sulfonyl]-4-(2-fluoropyridin-3-yl)-1,3-thiazol-2-yl}methyl)methylcarbamate (469 mg) and triethylamine (0.15 mL) in a mixed solvent of ethanol (10 mL) and tetrahydrofuran (10 mL) was added 10% palladium-carbon (50% water-containing product, 153 mg). The mixture was stirred at room temperature for 2 hr under a hydrogen atmosphere, and the insoluble material was filtered off. Saturated aqueous sodium hydrogen carbonate solution was added to the filtrate, the mixture was concentrated under reduced pressure, and the residue was extracted with ethyl acetate. The extract was washed successively with saturated aqueous sodium hydrogen carbonate solution, water and saturated brine, dried over anhydrous sodium sulfate, and concentrated under reduced pressure. The residue was purified by basic silica gel column chromatography (eluent: hexane-ethyl acetate=2:1) to give the title compound as a yellow oil (371 mg, yield 85%).
¹H-NMR (CDCl₃)δ: 1.1(9H,brs),3.01(3H,s),4.69(2H,brs),7.24-7.42(2H,m),7.89-7.99(2H,m),8.34-8.35(1H,m),8.79-8.81(1H,m),8.87-8.88(1H,m).

### Reference Example 155

### tert-butyl ({5-[(2-chloropyridin-4-yl)sulfonyl]-4-(2-fluoropyridin-3-yl)-1,3-thiazol-2-yl}methyl)methylcarbamate

To a solution of tert-butyl ({5-[(2-chloropyridin-4-yl)thio]-4-(2-fluoropyridin-3-yl)-1,3-thiazol-2-yl}methyl)methylcarbamate (646 mg) in acetic acid (10 mL) was added 3-chloroperbenzoic acid (1.34 g), and mixture was stirred at room temperature for 20 hr. Aqueous sodium thiosulfate solution was added to the reaction mixture, and the mixture was concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=1:1) to give the title compound as a yellow oil (312 mg, yield 45%).
¹H-NMR(CDCl₃)δ: 1.52(9H,brs),3.03(3H,s),4.70(2H,brs),7.34-7.38(1H,m),7.42-7.44(1H,m),7.53(1H,s),7.90-7.96(1H,m),8.37-8.39(1H,m),8.56-8.58(1H,m).

### Reference Example 156

### 5-(2-fluorophenyl)furan-2-carbaldehyde

To a mixture of 5-bromofuran-2-carbaldehyde (15.0 g), tetrakis(triphenylphosphine) palladium(0) (9.9 g) and 2-fluorophenylboronic acid (18.0 g) in a mixed solvent of toluene (196 mL) and ethanol (49 mL) was added a solution of sodium carbonate (24.9 g) in water (98 mL) at room temperature, and the mixture was refluxed for 10 hr under a nitrogen atmosphere. The reaction mixture was allowed to cool to room temperature, water was added, and the mixture was extracted with ethyl acetate. The extract was dried over anhydrous sodium sulfate, and concentrated under reduced pressure. The residue was purified by silica gel column chromatography (eluent: hexane-ethyl acetate=20:1) to give the title compound as a pale-yellow solid (14.0 g, yield 86%).
¹H-NMR(CDCl₃)δ: 7.04(1H,t,J=3.6Hz),7.18(1H,ddd,J=11.2,8.4,1.2Hz),7.24-7.35(1H,m),7.35-7.41(2H,m),8.03(1H,td,J=7.6,1.6Hz),9.69(1H,s).

### Reference Example 157

### 5-(2-methylphenyl)furan-2-carbaldehyde

To a mixture of 5-bromofuran-2-carbaldehyde (15.0 g), tetrakis(triphenylphosphine) palladium(0) (9.9 g) and 2-methylphenylboronic acid (17.5 g) in a mixed solvent of toluene (196 mL) and ethanol (49 mL) was added a solution of sodium carbonate (24.9 g) in water (98 mL) at room temperature, and the mixture was refluxed for 6 hr under a nitrogen atmosphere. The reaction mixture was allowed to cool to room temperature, water was added, and the mixture was extracted with ethyl acetate. The extract was dried over anhydrous sodium sulfate, and concentrated under reduced pressure. The residue was purified by silica gel column chromatography (eluent: hexane-ethyl acetate=20:1) to give the title compound as a pale-yellow solid (13.8 g, yield 86%).
¹H-NMR(CDCl₃)δ: 2.56(3H,s),6.75(1H,d,J=3.6Hz),7.25-7.34(3H,m),7.35(1H,d,J=4.0Hz),7.78-7.82(1H,m),9.68(1H,s).

### Reference Example 158

### 4-bromo-5-(2-fluorophenyl)furan-2-carbaldehyde

To a solution of 5-(2-fluorophenyl)furan-2-carbaldehyde (4.00 g) in chloroform (42 mL) was added bromine (1.08 mL) at room temperature. Bromine (0.54 mL) was added five times over 3 days at 12 hr intervals. The reaction mixture was washed successively with saturated aqueous sodium hydrogen carbonate solution and saturated brine, dried over anhydrous sodium sulfate, and concentrated under reduced pressure. The residue was purified by silica gel column chromatography (eluent: hexane-ethyl acetate=20:1→10:1) to give the title compound as a yellow solid (3.80 g, yield 67%).
¹H-NMR(CDCl₃)δ: 7.20-7.30(2H,m),7.37(1H,s),7.47-7.52(1H,m),7.74(1H,td,J=7.6,2.6Hz),9.68(1H,s).

### Reference Example 159

### 4-bromo-5-(2-methylphenyl)furan-2-carbaldehyde

To a solution of 5-(2-methylphenyl)furan-2-carbaldehyde (13.8 g) in acetonitrile (106 mL) was added N-bromosuccinimide (14.5 g) at room temperature, and the reaction mixture was stirred for 20 hr, and concentrated under reduced pressure. Water was added to the residue, and the mixture was extracted with ethyl acetate. The extract was washed successively with saturated aqueous sodium hydrogen carbonate solution and saturated brine, dried over anhydrous sodium sulfate, and concentrated under reduced pressure. The residue was purified by silica gel column chromatography (eluent: hexane-ethyl acetate=20:1→10:1) to give the title compound as a yellow solid (15.2 g, yield 77%).
¹H-NMR(CDCl₃)δ: 2.39(3H,s),7.27-7.30(2H,m),7.36-7.40(2H,m),7.54(1H,d,J=7.6Hz),9.65(1H,s).

### Reference Example 160

### 5-(2-fluorophenyl)-4-(phenylthio)furan-2-carbaldehyde

To a solution of 4-bromo-5-(2-fluorophenyl)furan-2-carbaldehyde (3.80 g) in N,N-dimethylformamide (35.5 mL) were added thiophenol (1.45 mL), potassium carbonate (3.90 g) and copper powder (0.897 g) at room temperature, and the mixture was stirred at 100°C for 30 hr under a nitrogen atmosphere. The reaction mixture was allowed to cool to room temperature, and water and ethyl acetate were added. The insoluble material was filtered off, and the filtrate was extracted with ethyl acetate. The extract was washed successively with water and saturated brine, dried over anhydrous magnesium sulfate, and concentrated under reduced pressure. The residue was purified by silica gel column chromatography (eluent: hexane-ethyl acetate=19:1→4:1) to give the title compound as a pale-yellow oil (0.700 g, yield 17%).
¹H-NMR(CDCl₃)δ: 7.18-7.32(8H,m),7.43-7.49(1H,m),7.72(1H,td,J=7.6,2.0Hz),9.67(1H,s).

### Reference Example 161

### 5-(2-methylphenyl)-4-(phenylthio)furan-2-carbaldehyde

To a solution of 4-bromo-5-(2-methylphenyl)furan-2-carbaldehyde (8.00 g) in N,N-dimethylformamide (76 mL) were added thiophenol (4.65 mL), potassium carbonate (8.34 g) and copper powder (1.92 g) at room temperature, and the mixture was stirred at 100°C for 25 hr under a nitrogen atmosphere. The reaction mixture was allowed to cool to room temperature, and water and ethyl acetate were added. The insoluble material was filtered off, and the filtrate was extracted with ethyl acetate. The extract was washed successively with water and saturated brine, dried over anhydrous magnesium sulfate, and concentrated under reduced pressure. The residue was purified by silica gel column chromatography (eluent: hexane-ethyl acetate=19:1→4:1) to give the title compound as a pale-yellow oil (2.20 g, yield 25%).
¹H-NMR(CDCl₃)δ: 2.41(3H,s),7.24-7.33(8H,m),7.34-7.38(1H,m),7.47(1H,d,J=7.6Hz),9.65(1H,s).

### Reference Example 162

### tert-butyl {[5-(2-fluorophenyl)-4-(phenylthio)furan-2-yl]methyl}methylcarbamate

5-(2-Fluorophenyl)-4-(phenylthio)furan-2-carbaldehyde (320 mg) was dissolved in a mixed solvent of tetrahydrofuran (10 mL) and methanol (3 mL), 40% methylamine-methanol solution (1.1 mL) was added, and the mixture was stirred at room temperature for 18 hr. The reaction mixture was concentrated under reduced pressure, and the residue was dissolved again in a mixed solvent of tetrahydrofuran (5 mL) and methanol (2 mL). Sodium borohydride (61 mg) was added under ice-cooling, and the mixture was stirred at room temperature for 30 min, treated with 1 mol/L hydrochloric acid, and basified with saturated aqueous sodium hydrogen carbonate solution. Di-tert-butyl bicarbonate (280 mg) was added, the mixture was stirred for 30 min, and concentrated under reduced pressure, and the residue was extracted with ethyl acetate. The extract was washed with saturated brine, dried over anhydrous sodium sulfate, and concentrated under reduced pressure. The residue was purified by silica gel column chromatography (eluent: hexane-ethyl acetate=19:1→9:1) to give the title compound as a pale-yellow oil (343 mg, yield 78%).
¹H-NMR(CDCl₃)δ: 1.47(9H,s),2.94(3H,br),4.40(2H,br),6.25(1H,br),7.10-7.36(8H,m),7.53-7.63(1H,m).

### Reference Example 163

### tert-butyl methyl{[5-(2-methylphenyl)-4-(phenylthio)furan-2-yl]methyl}carbamate

5-(2-Methylphenyl)-4-(phenylthio)furan-2-carbaldehyde (310 mg) was dissolved in a mixed solvent of tetrahydrofuran (5 mL) and methanol (2 mL), 40% methylamine-methanol solution (1.1 mL) was added, and the mixture was stirred at room temperature for 18 hr. The reaction mixture was concentrated under reduced pressure, the residue was dissolved again in a mixed solvent of tetrahydrofuran (5 mL) and methanol (2 mL), and sodium borohydride (61 mg) was added under ice-cooling. The mixture was stirred at room temperature for 30 min, treated with 1 mol/L hydrochloric acid, and concentrated under reduced pressure. Saturated aqueous sodium hydrogen carbonate solution and ethyl acetate were added to the residue, di-tert-butyl bicarbonate (280 mg) was added, and the mixture was stirred for 1 hr. The ethyl acetate layer of the reaction mixture was washed with saturated brine, dried over anhydrous sodium sulfate, and concentrated under reduced pressure. The residue was purified by silica gel column chromatography (eluent: hexane-ethyl acetate=19:1→9:1) to give the crude title compound as a pale-yellow oil (345 mg).
¹H-NMR(CDCl₃)δ: 1.45(9H,brs),2.34(3H,s),2.93(3H,br),4.42(2H,br),6.29(1H,br),7. 10-7.27(8H,m),7.37-7.39(1H,m).

### Reference Example 164

### tert-butyl {[5-(2-fluorophenyl)-4-(phenylsulfonyl)furan-2-yl]methyl}methylcarbamate

To a solution of tert-butyl {[5-(2-fluorophenyl)-4-(phenylthio)furan-2-yl]methyl}methylcarbamate (343 mg) in ethyl acetate (3 mL) was added 3-chloroperbenzoic acid (795 mg). The mixture was stirred at room temperature for 18 hr, treated with saturated aqueous sodium thiosulfate solution, and extracted with ethyl acetate. The extract was washed successively with water, saturated aqueous sodium hydrogen carbonate solution and saturated brine, dried over anhydrous sodium sulfate, and concentrated under reduced pressure. The residue was purified by basic silica gel column chromatography (eluent: hexane-ethyl acetate=9:1→4:1) to give the title compound as a colorless oil (326 mg, yield 88%).
¹H-NMR(CDCl₃)δ: 1.42(9H,brs),2.90(3H,brs),4.35(2H,br),6.59(1H,br),7.01-7.13(1H,m),7.20-7.25(1H,m),7.39-7.62(5H,m),7.74-7.78(2H,m).

### Reference Example 165

### tert-butyl methyl{[5-(2-methylphenyl)-4-(phenylsulfonyl)furan-2-yl]methyl}carbamate

To a solution of crude tert-butyl methyl{[5-(2-methylphenyl)-4-(phenylthio)furan-2-yl]methyl}carbamate (345 mg) in ethyl acetate (10 mL) was added 3-chloroperbenzoic acid (806 mg). The mixture was stirred at room temperature for 18 hr, treated with saturated aqueous sodium thiosulfate solution, and extracted with ethyl acetate. The extract was washed successively with water, saturated aqueous sodium hydrogen carbonate solution and saturated brine, dried over anhydrous sodium sulfate, and concentrated under reduced pressure. The residue was purified by basic silica gel column chromatography (eluent: hexane-ethyl acetate=9:1→4:1) to give the title compound as a pale-yellow oil (284 mg, 2 step yield 77%). ¹H-NMR(CDCl₃)δ:
1.43(9H,brs),1.89(3H,s),2.88(3H,brs),4.37(2H,br),6.67(1H,br),7 .14-7.17(1H,m),7.21-7.25(1H,m),7.30-7.38(4H,m),7.45-7.57(3H,m).

### Reference Example 166

### 1-(4-bromothiophen-2-yl)-N-methylmethanamine

To a solution of 4-bromothiophene-2-carbaldehyde (5.1 g) in a mixed solvent of tetrahydrofuran (30 mL) and methanol (30 mL) was added 40% methylamine-methanol solution (27 mL) at 0°C, and the mixture was stirred at room temperature for 16 hr, and concentrated under reduced pressure. The residue was dissolved in methanol (50 mL), sodium borohydride (6.9 g) was added at 0°C, and the mixture was stirred at room temperature for 6 hr, and concentrated under reduced pressure. Water was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure to give the title compound as a yellow oil (5.4 g, yield 98%).
¹H-NMR(CDCl₃)δ: 2.47(3H,s),3.90(2H,s),6.83-6.84(1H,m),7.10-7.11(1H,m),1H: not detected.

### Reference Example 167

### tert-butyl [(4-bromothiophen-2-yl)methyl]methylcarbamate

To a solution of 1-(4-bromothiophen-2-yl)-N-methylmethanamine (8.46) in ethyl acetate (100 mL) was added di-tert-butyl bicarbonate (9.8 mL) at 0°C, and the mixture was stirred at room temperature for 12 hr, and concentrated under reduced pressure. Water was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=9:1) to give the title compound as a yellow oil (10.3 g, yield 81%).
¹H-NMR(CDCl₃)δ: 1.49(9H,s),2.85(3H,brs),4.48(2H,brs),6.83(1H,s),7.11-7.12(1H,s).

### Reference Example 168

### 2-ethylhexyl 3-[(5-{[(tert-butoxycarbonyl)(methyl)amino]methyl}thiophen-3-yl)thio]propanoate

A mixture of tert-butyl [(4-bromothiophen-2-yl)methyl]methylcarbamate (3.0 g), 2-ethylhexyl 3-mercaptopropanoate (2.4 mL), tris(dibenzylideneacetone)dipalladium(0) (363 mg), 9,9-dimethyl-4,5-bis(diphenylphosphino)xanthine (460 mg) and N-ethyldiisopropylamine (3.4 mL) was stirred in toluene (30 mL) at 105°C for 7 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=8:1) to give the title compound as a yellow oil (4.3 g, yield 96%).
¹H-NMR(CDCl₃)δ: 0.86-0.91(6H,m),1.23-1.37(9H,m),1.49(9H,s),2.60(2H,t,J=7.5Hz),2.85(3H,brs),3.05(2H, t,J=7.5Hz),3.99-4.02(2H,m),4.47(2H,brs),6.85(1H,brs),7.09-7.10(1H,m).

### Reference Example 169

### tert-butyl methyl({[4-(pyridin-3-yl)thio]thiophen-2-yl}methyl)carbamate

To a solution of 2-ethylhexyl 3-[(5-{[(tert-butoxycarbonyl)(methyl)amino]methyl}thiophen-3-yl)thio]propanoate (2.0 g) in ethanol (25 mL) was added sodium ethoxide (618 mg) at 0°C, and the mixture was stirred at room temperature for 2 hr, and concentrated under reduced pressure. A mixture of the residue, 3-iodopyridine (970 mg), tris(dibenzylideneacetone)dipalladium(0) (166 mg) and 9,9-dimethyl-4,5-bis(diphenylphosphino)xanthine (210 mg) was stirred in toluene (25 mL) at 80°C for 2 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=2:1) to give the title compound as a yellow oil (1.2 g, yield 81%).
¹H-NMR(CDCl₃)δ: 1.48(9H,s),2.85(3H,s),4.48(2H,brs),6.84(1H,brs),7.14-7.19(1H,m),7.32(1H,d,J=1.5Hz),7.45-7.49(1H,m),8.38-8.40(1H,m),8.45(1H,d,J=2.1Hz).

### Reference Example 170

### tert-butyl ({5-bromo-4-[(pyridin-3-yl)thio]thiophen-2-yl}methyl)methylcarbamate

To a solution of tert-butyl methyl({4-[(pyridin-3-yl)thio]thiophen-2-yl}methyl)carbamate (1.2 g) in N,N-dimethylformamide (15 mL) was added N-bromosuccinimide (1.3 g) at 0°C, and the mixture was stirred at room temperature for 5 hr. Saturated aqueous sodium hydrogen carbonate solution was added to the reaction mixture, and the mixture was extracted with ethyl acetate. The extract was washed successively with saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=1:1) to give the title compound as a pale-yellow solid (1.0 g, yield 68%).
¹H-NMR(CDCl₃)δ: 1.48(9H,s),2.84(3H,s),4.40(2H,brs),6.71(1H,brs),7.17-7.21(1H,m),7.47-7.51(1H,m),8.41-8.43(1H,m),8.46(1H,d,J=1.5Hz).

### Reference Example 171

### tert-butyl {[5-bromo-4-(pyridin-3-ylsulfonyl)thiophen-2-yl]methyl}methylcarbamate

To a suspension of tert-butyl ({5-bromo-4-[(pyridin-3-yl)thio]thiophen-2-yl}methyl)methylcarbamate (814 mg) in a mixed solvent of acetonitrile (8 mL) and water (8 mL) was added sodium percarbonate (4.02 g), and the mixture was stirred at room temperature for 3 hr. Aqueous sodium thiosulfate solution was added to the reaction mixture, and the mixture was extracted with ethyl acetate. The extract was washed successively with saturated aqueous sodium hydrogen carbonate solution, water and saturated brine, dried over anhydrous magnesium sulfate, and concentrated under reduced pressure. The residue was washed with diisopropyl ether to give the title compound as a colorless solid (593 mg, yield 68%).
¹H-NMR(CDCl₃) δ: 1.48(9H,s),2.86(3H,s),4.42(2H,s),7.29(1H,s),7.46-7.50(1H,m),8.27-8.31(1H,m),8.82-8.84(1H,m),9.19-9.20(1H,m).

### Reference Example 172

### tert-butyl {[5-(2-fluorophenyl)-4-(pyridin-3-ylsulfonyl)thiophen-2-yl]methyl}methylcarbamate

A suspension of tert-butyl {[5-bromo-4-(pyridin-3-ylsulfonyl)thiophen-2-yl]methyl}methylcarbamate (153 mg), 2-fluorophenylboronic acid (63 mg), tetrakis(triphenylphosphine) palladium(0) (41 mg) and sodium carbonate (75 mg) in a mixed solvent of 1,2-dimethoxyethane (3 mL) and water (1.5 mL) was stirred at 105°C for 4 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=4:1→1:1) to give the title compound as a yellow oil (145 mg, yield 92%).
¹H-NMR(CDCl₃)δ: 1.47(9H,s),2.92(3H,s),4.51(2H,brs),7.03(1H,t,J=8.4Hz),7.17-7.22(1H,m),7.28-7.36(3H,m),7.41-7.47(2H,m),7.80-7.84(1H,m),8.70-8.73(1H,m).

### Reference Example 173

### tert-butyl {[5-(2-fluoropyridin-3-yl)-4-(pyridin-3-ylsulfonyl)thiophen-2-yl]methyl}methylcarbamate

A suspension of tert-butyl {[5-bromo-4-(pyridin-3-ylsulfonyl)thiophen-2-yl]methyl}methylcarbamate (257 mg), 2-fluoro-3-pyridineboronic acid (123 mg), tetrakis(triphenylphosphine) palladium(0) (66 mg) and sodium carbonate (133 mg) in a mixed solvent of 1,2-dimethoxyethane (4 mL) and water (2 mL) was stirred at 105°C for 3 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by basic silica gel column chromatography (eluent: hexane-ethyl acetate=1:1) to give the title compound as a yellow oil (148 mg, yield 56%).
¹H-NMR (CDCl₃)δ: 1.47(9H,s),2.91(3H,s),4.52(2H,s),7.25-7.39(3H,m),7.86-7.89(2H,m),8.30-8.32(1H,m),8.75-8.78(1H,m),8.84(1H,d,J=2.4Hz).

### Reference Example 174

### tert-butyl {[5-(2-chloropyridin-3-yl)-4-(pyridin-3-ylsulfonyl)thiophen-2-yl]methyl}methylcarbamate

A suspension of tert-butyl {[5-bromo-4-(pyridin-3-ylsulfonyl)thiophen-2-yl]methyl}methylcarbamate (168 mg), 2-chloro-3-pyridineboronic acid (90 mg), tetrakis(triphenylphosphine) palladium(0) (44 mg) and sodium carbonate (80 mg) in a mixed solvent of 1,2-dimethoxyethane (3 mL) and water (1.5 mL) was stirred at 105°C for 4 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by basic silica gel column chromatography (eluent: hexane-ethyl acetate=1:1) to give the title compound as a yellow oil (82 mg, yield 45%).
¹H-NMR(CDCl₃)δ: 1.47(9H,s),2.93(3H,s),4.54(2H,brs),7.31-7.39(2H,m),7.42(1H,t,J=0.9Hz),7.72-7.76(1H,m),7.81-7.84(1H,m),8.48-8.50(1H,m),8.75-8.77(2H,m).

### Reference Example 175

### [2-(2,3-difluorophenyl)-1H-imidazol-4-yl]methanol

A mixture of 2,3-difluorobenzamidine hydrochloride (3.0 g), dihydroxyacetone dimer (2.81 g), ammonium chloride (4.17 g) and 25% aqueous ammonia (30 mL) was stirred at 85°C for 1 hr. The reaction mixture was allowed to cool, and the resulting insoluble material was collected by filtration to give the title compound as pale-brown crystals (2.0 g, yield 61%).
¹H-NMR(DMSO-d₆)δ: 4.35-4.53(2H,m),4.83-5.13(1H,m),6.91-7.18(1H,m),7.21-7.33(1H,m),7.34-7.49(1H,m),7.75(1H,t,J=7.2Hz),12.03-12.43(1H,m).

### Reference Example 176

### 2-(2,3-difluorophenyl)-1H-imidazole-4-carbaldehyde

To a solution of [2-(2,3-difluorophenyl)-1H-imidazol-4-yl]methanol (1.80 g) in tetrahydrofuran (90 mL) was added manganese dioxide (7.50 g), and the mixture was stirred at room temperature for 4 hr. The reaction mixture was filtered, and the filtrate was concentrated under reduced pressure. Diisopropyl ether was added to the residue, and the insoluble material was collected by filtration to give the title compound as pale-yellow crystals (1.62 g, yield 91%).
¹H-NMR(DMSO-d₆)δ: 7.31-7.42(1H,m),7.51-7.60(1H,m),7.76-7.84(1H,m),8.18(1H,s),9.83(1H,s),13.30(1H,brs).

### Reference Example 177

### 2-(2-fluorophenyl)-1-(phenylsulfonyl)-1H-imidazole-4-carbaldehyde

To a solution of 2-(2-fluorophenyl)-1H-imidazole-4-carbaldehyde (191 mg) in tetrahydrofuran (40 mL) was added sodium hydride (60% in oil, 81 mg) at room temperature, and the mixture was stirred for 10 min. Benzenesulfonyl chloride (270 mg) was added, and the mixture was stirred for 1 hr. The reaction mixture was diluted with saturated aqueous sodium hydrogen carbonate solution, and extracted with ethyl acetate. The extract was washed with saturated brine, dried over anhydrous magnesium sulfate, and concentrated under reduced pressure. The residue was purified by silica gel column chromatography (eluent: hexane-ethyl acetate=1:1) to give the title compound as a colorless oil (310 mg, yield 93%).
¹H-NMR(CDCl₃)δ: 7.07(1H,t,J=9.0Hz),7.17-7.34(2H,m),7.42-7.58(5H,m),7.63-7.73(1H,m),8.31(1H,s),9.94(1H,s).

### Reference Example 178

### 2-(2-fluorophenyl)-1-(thiophen-3-ylsulfonyl)-1H-imidazole-4-carbaldehyde

To a solution of 2-(2-fluorophenyl)-1H-imidazole-4-carbaldehyde (200 mg) in tetrahydrofuran (10 mL) was added sodium hydride (60% in oil, 210 mg) at room temperature, and the mixture was stirred for 10 min. 15-Crown-5 (1.16 g) was added dropwise, and the mixture was stirred for 1 min. Thiophene-3-sulfonyl chloride (576 mg) was added, and the mixture was further stirred for 1 hr. The reaction mixture was diluted with water, and extracted with ethyl acetate. The extract was washed with saturated brine, dried over anhydrous magnesium sulfate, and concentrated under reduced pressure. The residue was purified by silica gel column chromatography (eluent: hexane-ethyl acetate=1:1→2:3) to give the title compound as a colorless oil (280 mg, yield 79%).
¹H-NMR(CDCl₃)δ: 7.07-7.16(2H,m),7.20-7.28(1H,m),7.30-7.38(1H,m),7.43(1H,dd,J=5.1,3.2Hz),7.47-7.59(1H,m),7.74(1H,dd,J=3.2,1.3Hz),8.28(1H,s),9.95(1H,s).

### Reference Example 179

### 2-(2-fluorophenyl)-1-[(5-methylthiophen-2-yl)sulfonyl]-1H-imidazole-4-carbaldehyde

To a solution of 2-(2-fluorophenyl)-1H-imidazole-4-carbaldehyde (191 mg) in tetrahydrofuran (20 mL) was added sodium hydride (60% in oil, 81 mg) at room temperature, and the mixture was stirred for 15 min. 15-Crown-5 (449 mg) was added dropwise, and the mixture was stirred for 1 min. 5-Methylthiophene-2-sulfonyl chloride (297 mg) was added, and the mixture was further stirred for 1 hr. The reaction mixture was diluted with saturated aqueous sodium hydrogen carbonate solution, and extracted with ethyl acetate. The extract was washed with saturated brine, dried over anhydrous magnesium sulfate, and concentrated under reduced pressure. The residue was purified by silica gel column chromatography (eluent: hexane-ethyl acetate=7:3→1:1) to give the title compound as a pale-brown oil (340 mg, yield 97%).
¹H-NMR(CDCl₃)δ: 2.54(3H,d,J=0.8Hz),6.72(1H,dd,J=4.0,1.0Hz),7.10-7.18(2H,m),7.21-7.28(1H,m),7.39(1H,td,J=7.4,1.9Hz),7.50-7.58(1H,m),8.23(1H,s),9.94(1H,s).

### Reference Example 180

### 2-(2-fluorophenyl)-1-(furan-3-ylsulfonyl)-1H-imidazole-4-carbaldehyde

To a solution of 2-(2-fluorophenyl)-1H-imidazole-4-carbaldehyde (200 mg) in tetrahydrofuran (20 mL) was added sodium hydride (60% in oil, 84 mg) at room temperature, and the mixture was stirred for 10 min. 15-Crown-5 (464 mg) was added dropwise, and the mixture was stirred for 1 min. Furan-3-sulfonyl chloride (576 mg) was added, and the mixture was further stirred for 30 min. The reaction mixture was diluted with water, and extracted with ethyl acetate. The extract was washed with saturated brine, dried over anhydrous magnesium sulfate, and concentrated under reduced pressure. The residue was purified by silica gel column chromatography (eluent: hexane-ethyl acetate=1:1) to give the title compound as a colorless solid (290 mg, yield 86%).
¹H-NMR(CDCl₃)δ: 6.47(1H,dd,J=2.1,1.0Hz),7.11-7.19(1H,m),7.23-7.30(1H,m),7.38-7.45(1H,m),7.50(1H,t,J=1.7Hz),7.52-7.60(1H,m),7.63(1H,brs),8.24(1H,s),9.96(1H,s).

### Reference Example 181

### 2-(2-fluorophenyl)-1-[(1-methyl-1H-pyrazol-5-yl)sulfonyl]-1H-imidazole-4-carbaldehyde

To a solution of 2-(2-fluorophenyl)-1H-imidazole-4-carbaldehyde (191 mg) in tetrahydrofuran (20 mL) was added sodium hydride (60% in oil, 81 mg) at room temperature, and the mixture was stirred for 15 min. 15-Crown-5 (450 mg) was added dropwise, and the mixture was stirred for 1 min. 1-Methyl-1H-pyrazole-5-sulfonyl chloride (576 mg) was added, and the mixture was further stirred for 30 min. The reaction mixture was diluted with saturated aqueous sodium hydrogen carbonate solution, and extracted with ethyl acetate. The extract was washed with saturated brine, dried over anhydrous magnesium sulfate, and concentrated under reduced pressure. The residue was purified by silica gel column chromatography (eluent: hexane-ethyl acetate=1:1) to give the title compound as a colorless oil (330 mg, yield 98%).
¹H-NMR(CDCl₃)δ: 3.82(3H,s),6.40(1H,d,J=2.3Hz),7.05-7.12(1H,m),7.20-7.26(1H,m),7.29-7.36(1H,m),7.40(1H,d,J=2.3Hz),7.48-7.58(1H,m),8.32(1H,s),9.97(1H,s).

### Reference Example 182

### 2-(2-fluorophenyl)-1-[(3-methylpiperidin-1-yl)sulfonyl]-1H-imidazole-4-carbaldehyde

To a solution of 2-(2-fluorophenyl)-1H-imidazole-4-carbaldehyde (191 mg) in dimethylformamide (15 mL) was added potassium carbonate (700 mg) at room temperature, 3-methylpiperidine-1-sulfonyl chloride (1.0 g) was added dropwise, and the mixture was stirred for 24 hr. The reaction mixture was diluted with saturated aqueous sodium hydrogen carbonate solution, and extracted with ethyl acetate. The extract was washed with saturated brine, dried over anhydrous magnesium sulfate, and concentrated under reduced pressure. The residue was purified by silica gel column chromatography (eluent: hexane-ethyl acetate=1:1) to give the title compound as a colorless oil (140 mg, yield 40%).
¹H-NMR(CDCl₃)δ: 0.83(3H, d,J=6.8Hz) ,1.42-1.80(5H,m),2.14(1H,t,J=11.4Hz),2.47(1H,td,J=12.0,2.8Hz),3.29-3.51(2H,m),7.09-7.33(2H,m),7.46-7.57(2H,m),8.08(1H,s),9.96(1H,s).

### Reference Example 183

### 2-(2,3-difluorophenyl)-1-[(5-methylthiophen-2-yl)sulfonyl]-1H-imidazole-4-carbaldehyde

To a solution of 2-(2,3-difluorophenyl)-1H-imidazole-4-carbaldehyde (209 mg) in tetrahydrofuran (20 mL) was added sodium hydride (60% in oil, 81 mg) at room temperature, and the mixture was stirred for 5 min. 15-Crown-5 (449 mg) was added dropwise, and the mixture was stirred for 1 min. 5-Methylthiophene-2-sulfonyl chloride (297 mg) was added, and the mixture was further stirred for 1 hr. The reaction mixture was diluted with saturated aqueous sodium hydrogen carbonate solution, and extracted with ethyl acetate. The extract was washed with saturated brine, dried over anhydrous magnesium sulfate, and concentrated under reduced pressure. The residue was purified by silica gel column chromatography (eluent: hexane-ethyl acetate=7:3→1:1) to give the title compound as a pale-brown oil (200 mg, yield 97%).
¹H-NMR(CDCl₃)δ: 2.55(3H,s),6.76(1H,d,J=4.9Hz),7.12-7.29(3H,m),7.32-7.44(1H,m),8.23(1H,s),9.94(1H,s).

### Reference Example 184

### (2,3-difluorophenyl)hydrazine hydrochloride

To a solution of sodium nitrite (27.8 g) in water (100 mL) was added dropwise a solution of 2,3-difluoroaniline (40 g) in concentrated hydrochloric acid (620 mL) at -20°C, and the mixture was stirred at the same temperature for 1 hr. A solution of tin(II) chloride (117 g) in concentrated hydrochloric acid (200 mL) was added dropwise at -20°C, and the mixture was stirred at 0°C for 2 hr. The resulting solid was collected by filtration, washed with water and hexane, and dried concentrated under reduced pressure to give the title compound as a white solid (32.6g, yield 73%).
¹H-NMR(DMSO-d₆)δ: 6.96-7.19(3H,m),8.70(1H,brs),10.66(3H,brs).

### Reference Example 185

### (2-fluoro-3-methylphenyl)hydrazine

To a solution of sodium nitrite (28.7 g) in water (100 mL) was added dropwise a solution of 2-fluoro-3-methylaniline (40 g) in concentrated hydrochloric acid (640 mL) at -20°C, and the mixture was stirred at the same temperature for 1 hr. A solution of tin(II) chloride (121 g) in concentrated hydrochloric acid (200 mL) was added dropwise at -20°C, and the mixture was stirred at 0°C for 2 hr. The resulting solid was collected by filtration, and washed with water and hexane. The obtained solid was dissolved in water (500 mL), the solution was adjusted to pH 12 with 2 mol/L aqueous sodium hydroxide solution, and extracted twice with dichloromethane. The extract was dried over anhydrous sodium sulfate, and concentrated under reduced pressure to give the title compound as a white solid (28.4 g, yield 63%).
¹H-NMR(CDCl₃)δ: 2.24(3H,d,J=2.4Hz),3.54(2H,brs),5.30(1H,brs),6.59-6.63(1H,m),6.89(1H,dt,J=7.8,2.OHz),6.94-6.99(1H,m).

### Reference Example 186

### (2-fluoro-4-methylphenyl)hydrazine

To a solution of sodium nitrite (28.7 g) in water (100 mL) was added dropwise a solution of 2-fluoro-4-methylaniline (40 g) in concentrated hydrochloric acid (640 mL) at -20°C, and the mixture was stirred at the same temperature for 1 hr. A solution of tin(II) chloride (121 g) in concentrated hydrochloric acid (200 mL) was added dropwise at -20°C, and the mixture was stirred at 0°C for 2 hr. The resulting solid was collected by filtration, and washed with water and hexane. The obtained solid was dissolved in water (500 mL), the solution was adjusted to pH 12 with 2 mol/L aqueous sodium hydroxide solution, and extracted twice with dichloromethane. The extract was dried over anhydrous magnesium sulfate, and concentrated under reduced pressure to give the title compound as a white solid (28.4g, yield 63%).
¹H-NMR(CDCl₃)δ: 2.26(3H,s),3.53(2H,brs),5.35(1H,brs),6.80(1H,dd,J=12.4,1.2Hz), 6.87(1H,d,J=8.0Hz),6.96(1H,t,J=8.4Hz).

### Reference Example 187

### (2-fluoro-5-methylphenyl)hydrazine

To a solution of sodium nitrite (14.3 g) in water (60 mL) was added dropwise a solution of 2-fluoro-5-methylaniline (20 g) in concentrated hydrochloric acid (320 mL) at -20°C, and the mixture was stirred at the same temperature for 1 hr. A solution of tin(II) chloride (60.6 g) in concentrated hydrochloric acid (100 mL) was added dropwise at -20°C, and the mixture was stirred at 0°C for 1 hr. The resulting solid was collected by filtration, and washed with water and hexane. The obtained solid was dissolved in 6 mol/L aqueous sodium hydroxide solution, and the solution was extracted tree times with dichloromethane. The extract was dried over anhydrous magnesium sulfate, and concentrated under reduced pressure to give the title compound as a yellow oil (13.4g, yield 61%).
¹H-NMR(CDCl₃)δ: 2.30(3H,s),3.45(2H,brs),6.50-6.54(2H,m),6.82-6.90(2H,m),1H: not detected.

### Reference Example 188

### (3-fluoro-2-methylphenyl)hydrazine hydrochloride

To a solution of sodium nitrite (1.2 g) in water (10 mL) was gradually added dropwise a solution of 3-fluoro-2-methylaniline (2.0 g) in 6 mol/L hydrochloric acid (10 mL) at 8°C or less under ice-cooling, and the mixture was stirred at the same temperature for 1 hr. A solution of tin(II) chloride (6.06 g) in 6 mol/L hydrochloric acid (9 mL) was gradually added dropwise under ice-cooling, and the mixture was stirred at 0°C for 1 hr. Celite and 8 mol/L aqueous sodium hydroxide solution (20 mL) were added to the reaction mixture, and the mixture was stirred for 1 hr. The precipitate was filtered through celite, and the filtrate was extracted with ethyl acetate. The separated aqueous layer was extracted again with ethyl acetate. The combined organic layers were washed with saturated brine, dried over magnesium sulfate, and concentrated under reduced pressure. The residue was purified by silica gel column chromatography (eluent: hexane-ethyl acetate=19:1→4:1) to give the title compound as a brown solid (1.32 g, yield 59%).
¹H-NMR(DMSO-d₆)δ: 2.02(3H,d,J=1.5Hz),6.54(1H,t,J=9.0Hz),6.76(1H,d,J=8.3Hz),7.03-7.20(1H,m)3H: not detected.

### Reference Example 189

### (2-chloro-3-fluorophenyl)hydrazine hydrochloride

To a solution of sodium nitrite (3.1 g) in water (10 mL) was added dropwise a solution of 2-chloro-3-fluoroaniline (5.0 g) in concentrated hydrochloric acid (70 mL) at -20°C, and the mixture was stirred at the same temperature for 1.5 hr. A solution of tin(II) chloride (13 g) in concentrated hydrochloric acid (20 mL) was added dropwise at -20°C, and the mixture was stirred at 0°C for 1 hr. The reaction mixture was filtered, and the obtained solid was washed with water and hexane, and dried under reduced pressure to give the title compound as a yellow powder (4.3g, yield 64%).
¹H-NMR(DMSO-d₆)δ: 6.91-7.02(2H,m),7.32-7.40(1H,m),8.35(1H,brs),10.23(2H,brs),1H: not detected.

### Reference Example 190

### 2-fluoro-3-hydrazinopyridine

To a solution of sodium nitrite (48.0 g) in water (100 mL) was added dropwise a solution of 2-fluoropyridin-3-amine (60 g) in concentrated hydrochloric acid (892 mL) at -20°C, and the mixture was stirred at the same temperature for 1 hr. A solution of tin(II) chloride (203 g) in concentrated hydrochloric acid (200 mL) was added dropwise at -20°C, and the mixture was stirred at 0°C for 1 hr. The resulting solid was collected by filtration, and washed with water and hexane. The obtained solid was dissolved in 2 mol/L aqueous sodium hydroxide solution, and the solution was extracted three times with dichloromethane. The extract was dried over anhydrous magnesium sulfate, and concentrated under reduced pressure to give the title compound as a yellow oil (30.0 g, yield 44%).
¹H-NMR(DMSO-d₆)δ: 6.96(1H,brs),7.09-7.13(1H,m),7.30-7.32(1H,m),7.42-7.47(1H,m),2H not detected.

### Reference Example 191

### potassium (1Z)-1-cyano-3-ethoxy-3-oxoprop-1-en-2-olate

To a solution of diethyl oxalate (50 g) in acetonitrile (342 mL) was added potassium tert-butoxide (38.4 g) at room temperature, and the mixture was stirred at the same temperature for 1 hr. The reaction mixture was filtered, and the solid was washed with acetonitrile to give the title compound as a yellow solid (35.0 g, yield 73%).
¹H-NMR(DMSO-d₆)δ: 1.18(3H,t,J=7.0Hz),4.01(2H,q,J=7.0Hz),4.09(1H,d,J=2.8 Hz).

### Reference Example 192

### ethyl 1-(2-fluorophenyl)-5-hydroxy-1H-pyrazole-3-carboxylate

To a solution of 2-fluorophenylhydrazine hydrochloride (1.62 g) in ethanol (40 mL) were added potassium carbonate (2.76 g) and diethyl but-2-ynedioate (1.7 g) at room temperature, and the mixture was stirred for 4 hr with heating under reflux. The reaction mixture was allowed to cool, 1 mol/L hydrochloric acid (70 mL) and water (70 mL) were added, and the mixture was stirred for 1 hr. The precipitate was collected by filtration, washed with water, and dried under reduced pressure to give the title compound as a colorless solid (2.11 g, yield 84%).
¹H-NMR(DMSO-d₆)δ: 1.24-1.31(3H,m),4.21-4.31(2H,m),5.92(1H,s),7.32-7.40(1H,m),7.41-7.50(1H,m),7.50-7.60(2H,m),11.94(1H,s).

### Reference Example 193

### ethyl 5-bromo-1-(2-fluorophenyl)-1H-pyrazole-3-carboxylate

Ethyl 1-(2-fluorophenyl)-5-hydroxy-1H-pyrazole-3-carboxylate (2 g) and phosphorus oxybromide (6.88 g) were mixed, and the mixture was stirred at 110°C for 2 hr under an argon atmosphere. The reaction mixture was allowed to cool to about room temperature. The reaction mixture was diluted with ethyl acetate, ice water was carefully added. The reaction mixture was basified with sodium hydrogen carbonate, and extracted with ethyl acetate. The obtained ethyl acetate layer was washed with saturated brine, dried over anhydrous magnesium sulfate, and concentrated under reduced pressure. The residue was purified by silica gel column chromatography (eluent: hexane-ethyl acetate=19:1→2:3) to give the title compound as a white powder (466 mg, yield 19%).
¹H-NMR (CDCl₃)δ: 1.40(3H,t,J=7.2Hz),4.43(2H,q,J=7.2Hz),7.02(1H,s),7.22-7.33(2H,m),7.44-7.56(2H,m).

### Reference Example 194

### ethyl 5-hydroxy-1-(2-methylphenyl)-1H-pyrazole-3-carboxylate

A mixture of 2-methylphenylhydrazine hydrochloride (25.3 g), diethyl but-2-ynedioate (27.2 g) and potassium carbonate (44.26 g) was stirred in ethanol (300 mL) at 90°C for 16 hr. The reaction mixture was allowed to cool to room temperature, acidified with 6 mol/L hydrochloric acid, and concentrated under reduced pressure. Water was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was washed with ethyl acetate-diisopropyl ether to give the title compound as an orange solid (25.8 g, yield 66%).
¹H-NMR(DMSO-d₆)δ: 1.27(3H,t,J=7.2Hz),2.07(3H,s),4.24(2H,q,J=7.2Hz),5.91(1H,s),7. 26-7.43(4H,m),11.64(1H,brs).

### Reference Example 195

### ethyl 5-amino-1-(2-methylphenyl)-1H-pyrazole-3-carboxylate

To a solution of 2-methylphenylhydrazine (23.2 g) in water (450 mL) was added concentrated sulfuric acid (10 mL) at room temperature, and then a solution of potassium (1Z)-1-cyano-3-ethoxy-3-oxoprop-1-en-2-olate (34 g) in chloroform (450 mL) was added. The mixture was stirred at room temperature for 18 hr, the organic layer was separated, and the aqueous layer was extracted with dichloromethane (300 mL). The extract was combined with the organic layer previously separated, washed with saturated aqueous sodium hydrogen carbonate solution, dried over anhydrous sodium sulfate, and concentrated under reduced pressure. The obtained residue was dissolved in ethanol (450 mL), and the solution was refluxed for 18 hr, and allowed to cool to room temperature. Triethylamine (79 mL) was added, and the mixture was further stirred for 1 hr, and concentrated under reduced pressure. The residue was diluted with ethyl acetate, and the organic layer was washed with water, dried over anhydrous sodium sulfate, and concentrated under reduced pressure. The obtained residual solid was recrystallized from a mixed solvent of ethyl acetate and hexane to give the title compound as a yellow solid (28.3 g, yield 61%).
¹H-NMR(CDCl₃)δ: 1.38(3H,t,J=7.0Hz),2.14(3H,s),3.67(2H,s),4.39(2H,q,J=7.2Hz),6. 13(1H,s) ,7.28-7.41(4H,m).

### Reference Example 196

### ethyl 5-amino-1-(2,6-difluorophenyl)-1H-pyrazole-3-carboxylate

To a solution of (2,6-difluorophenyl)phenylhydrazine (33.7 g) in water (1.17L) was added concentrated sulfuric acid (12.5 mL) at room temperature, and then a solution of potassium (1Z)-1-cyano-3-ethoxy-3-oxoprop-1-en-2-olate (41.9 g) in chloroform (1.17L) was added. The mixture was stirred at room temperature for 18 hr, the organic layer was separated, and the aqueous layer was extracted with dichloromethane (300 mL). The extract was combined with the organic layer previously separated, washed with saturated aqueous sodium hydrogen carbonate solution, dried over anhydrous sodium sulfate, and concentrated under reduced pressure. The obtained residue was dissolved in ethanol (700 mL), and the solution was refluxed for 18 hr, and allowed to cool to room temperature. Triethylamine (98 mL) was added, and the mixture was further stirred for 1 hr, and concentrated under reduced pressure. The residue was diluted with ethyl acetate, and the organic layer was washed with water, dried over anhydrous sodium sulfate, and concentrated under reduced pressure. The obtained residual solid was recrystallized from a mixed solvent of ethyl acetate and hexane to give the title compound as a yellow solid (37.0 g, yield 59%).
¹H-NMR(CDCl₃)δ: 1.39(3H,t,J=7.2Hz),3.74(2H,s),4.39(2H,q,J=7.2Hz),6.20(1H,s),7. 10(2H,dd,J=8.4,7.2Hz),7.43-7.51(1H,m).

### Reference Example 197

### ethyl 5-iodo-1-(2-methylphenyl)-1H-pyrazole-3-carboxylate

Ethyl 5-amino-1-(2-methylphenyl)-1H-pyrazole-3-carboxylate (2.0 g) was suspended in water (50 mL), and concentrated sulfuric acid (50 mL) and potassium iodide (1.6 g) were added at 0°C. Then a solution of sodium nitrite (675 mg) in water (20 mL) was added dropwise to the reaction mixture, and the mixture was stirred at the same temperature for 2 hr, and extracted with ethyl acetate. The extract was washed with saturated aqueous sodium thiosulfate solution, dried over anhydrous sodium sulfate, and concentrated under reduced pressure. The residue was purified by silica gel column chromatography (eluent: hexane-ethyl acetate=15:1) to give the title compound as a yellow oil (816 mg, yield 28%).
¹H-NMR(CDCl₃)δ: 1.40(3H,t,J=7.0Hz),2.04(3H,s),4.43(2H,q,J=7.2Hz),7.15(1H,s),7. 24-7.27(1H,m),7.29-7.34(2H,s),7.42(1H,td,J=7.4,1.6Hz).

### Reference Example 198

### ethyl 1-(2,6-difluorophenyl)-5-iodo-1H-pyrazole-3-carboxylate

Ethyl 5-amino-1-(2,6-difluorophenyl)-1H-pyrazole-3-carboxylate (10.0 g) was suspended in water (200 mL), and concentrated sulfuric acid (200 mL) and potassium iodide (7.45 g) were added at 0°C. Then a solution of sodium nitrite (3.10 mg) in water (50 mL) was added dropwise to the reaction mixture, and the mixture was stirred at the same temperature for 2 hr, and extracted with ethyl acetate. The extract was washed with saturated aqueous sodium thiosulfate solution, dried over anhydrous sodium sulfate, and concentrated under reduced pressure. The residue was purified by silica gel column chromatography (eluent: hexane-ethyl acetate=15:1) to give the title compound as a yellow oil (2.20 g, yield 16%).
¹H-NMR(CDCl₃)δ: 1.40(3H,t,J=7.0Hz),4.43(2H,q,J=7.0Hz),7.10(2H,t,J=8.2Hz),7.18( 1H,s),7.48-7.46(1H,m).

### Reference Example 199

### ethyl 5-hydroxy-1-(2-chlorophenyl)-1H-pyrazole-3-carboxylate

A mixture of 2-chlorophenylhydrazine hydrochloride (10 g), diethyl but-2-ynedioate (9.5 g) and potassium carbonate (15.5 g) was stirred in ethanol (200 mL) at 90°C for 24 hr. The reaction mixture was allowed to cool to room temperature, acidified with 6 mol/L hydrochloric acid, and concentrated under reduced pressure. Water was added to the residue, and the mixture was extracted with ethyl acetate. The extract was washed with saturated brine, dried over anhydrous sodium sulfate, and concentrated under reduced pressure. The residue was washed with ethyl acetate-diisopropyl ether to give the title compound as an orange solid (9.6 g, yield 64%).
¹H-NMR(DMSO-d₆)δ: 1.28(3H,t,J=7.2Hz),4.25(2H,q,J=7.2Hz),5.90(1H,s),7.47-7.66(3H,m),7.67(1H,d,J=7.5Hz),11.82(1H,brs).

### Reference Example 200

### ethyl 1-(2,3-difluorophenyl)-5-hydroxy-1H-pyrazole-3-carboxylate

To a solution of (2,3-difluorophenyl)hydrazine hydrochloride (32.6 g) in ethanol (452 mL) were added potassium carbonate (62.5 g) and diethyl but-2-ynedioate (38.5 g), and the mixture was refluxed for 18 hr, and concentrated under reduced pressure. The residue was treated with 2 mol/L hydrochloric acid, and the mixture was extracted twice with ethyl acetate. The extract was dried over anhydrous sodium sulfate, and concentrated under reduced pressure. The residue was suspended in diethyl ether, and the obtained solid was collected by filtration, and dried under reduced pressure to give the title compound as a pale-yellow solid (26.0 g, yield 43%).
1H-NMR(DMSO-d₆)δ: 1.29(3H,t,J=7.2Hz),4.28(2H,q,J=7.2Hz),5.96(1H,s),7.36-7.45(2H,m),7.59-7.66(1H,m),12.2(1H,s).

### Reference Example 201

### ethyl 1-(2,4-difluorophenyl)-5-hydroxy-1H-pyrazole-3-carboxylate

To a solution of (2,4-difluorophenyl)hydrazine (28.0 g) in ethanol (310 mL) were added potassium carbonate (42.9 g) and diethyl but-2-ynedioate (26.4 g), and the mixture was refluxed for 18 hr, and concentrated under reduced pressure. The residue was treated with 2 mol/L hydrochloric acid, and the mixture was extracted twice with ethyl acetate. The extract was dried over anhydrous sodium sulfate, and concentrated under reduced pressure. The residue was suspended in diethyl ether, and the obtained solid was collected by filtration, and dried under reduced pressure to give the title compound as a pale-yellow solid (15.7g, yield 38%).
¹H-NMR(DMSO-d₆)δ: 1.28(3H,t,J=7.2Hz),4.26(2H,q,J=7.2Hz),5.93(1H,s),7.24-7.30(1H,m),7.53-7.58(1H,m),7.61-7.67(1H,m),12.03(1H,s).

### Reference Example 202

### ethyl 1-(2,5-difluorophenyl)-5-hydroxy-1H-pyrazole-3-carboxylate

To a solution of (2,5-difluorophenyl)hydrazine hydrochloride (25.0 g) in ethanol (500 mL) were added potassium carbonate (38.3 g) and diethyl but-2-ynedioate (23.6 g), and the mixture was refluxed for 18 hr, cooled to 0°C, and acidified with 6 mol/L hydrochloric acid. Ethanol was evaporated under reduced pressure, and the residue was extracted with ethyl acetate. The extract was washed with saturated brine, dried over anhydrous sodium sulfate, and concentrated under reduced pressure. The residue was washed with a mixed solvent of ethyl acetate and diisopropyl ether, and the obtained solid was collected by filtration, and dried under reduced pressure to give the title compound as a pale-yellow solid (22.7g, yield 61%).
¹H-NMR(DMSO-d₆)δ: 1.28(3H,t,J=7.2Hz),4.26(2H,q,J=7.2Hz),5.91(1H,s),7.40-7.56(3H,m),12.05(1H,br).

### Reference Example 203

### ethyl 1-(2-fluoro-3-methylphenyl)-5-hydroxy-1H-pyrazole-3-carboxylate

To a solution of (2-fluoro-3-methylphenyl)hydrazine (28.4 g) in ethanol (405 mL) were added potassium carbonate (56 g) and diethyl but-2-ynedioate (34.5 g), and the mixture was refluxed for 18 hr, allowed to cool to room temperature, treated with 2 mol/L hydrochloric acid, and extracted twice with ethyl acetate. The extract was dried over anhydrous sodium sulfate, and concentrated under reduced pressure. The residue was suspended in diethyl ether, and the obtained solid was collected by filtration, and dried under reduced pressure to give the title compound as a pale-yellow solid (14.8g, yield 28%).
¹H-NMR(DMSO-d₆)δ: 1.28(3H,t,J=7.2Hz),2.32(3H,d,J=2.0Hz),4.26(2H,q,J=7.2Hz),5.92( 1H,s),7.24(1H,t,J=7.8Hz),7.32-7.36(1H,m),7.41-7.46(1H,m),11.90(1H,s).

### Reference Example 204

### ethyl 1-(2-fluoro-4-methylphenyl)-5-hydroxy-1H-pyrazole-3-carboxylate

To a solution of (2-fluoro-4-methylphenyl)hydrazine (28.4 g) in ethanol (405 mL) were added potassium carbonate (56 g) and diethyl but-2-ynedioate (34.5 g), and the mixture was refluxed for 18 hr, allowed to cool to room temperature, treated with 2 mol/L hydrochloric acid, and extracted twice with ethyl acetate. The extract was dried over anhydrous sodium sulfate, and concentrated under reduced pressure. The residue was suspended in diethyl ether, and the obtained solid was collected by filtration, and dried under reduced pressure to give the title compound as a pale-yellow solid (32.0 g, yield 60%).
¹H-NMR (DMSO-d₆)δ: 1.26(3H,t,J=7.2Hz) ,2.38(3H,s) 4.24(2H,q,J=7.2Hz)5.90(1H,s), 7. 13-7.15(1H,m),7.26(1H,dd,J=11.2,1.2Hz),7.38(1H,t,J=8.2Hz),11.85(1 H, s) .

### Reference Example 205

### ethyl 1-(2-fluoro-5-methylphenyl)-5-hydroxy-1H-pyrazole-3-carboxylate

To a solution of (2-fluoro-5-methylphenyl)hydrazine (13.8 g) in ethanol (197 mL) were added potassium carbonate (27.2 g) and diethyl but-2-ynedioate (16.8 g), and the mixture was refluxed for 18 hr, allowed to cool to room temperature, treated with 2 mol/L hydrochloric acid, and extracted twice with ethyl acetate. The extract was dried over anhydrous sodium sulfate, and concentrated under reduced pressure. The residue was suspended in diethyl ether, and the obtained solid was collected by filtration, and dried under reduced pressure to give the title compound as a yellow solid (15.0 g, yield 58%) .
¹H-NMR(DMSO-d₆)δ: 1.28(3H,t,J=7.2Hz),2.50(3H,s),4.26(2H,q,J=7.2Hz),5.76(1H,s),7. 29-7.36(3H,m),1H: not detected.

### Reference Example 206

### ethyl 1-(3-fluoro-2-methylphenyl)-5-hydroxy-1H-pyrazole-3-carboxylate

To a solution of (3-fluoro-2-methylphenyl)hydrazine hydrochloride (1.31 g) in ethanol (45 mL) were added potassium carbonate (1.29 g) and diethyl but-2-ynedioate (1.59 g), and the mixture was refluxed for 4 hr, and concentrated under reduced pressure. 1 mol/L Hydrochloric acid was added to the residue, and the mixture was extracted with ethyl acetate. The separated aqueous layer was extracted again with ethyl acetate. The combined organic layers were washed with saturated brine, dried over anhydrous sodium sulfate, and concentrated under reduced pressure to give the crude title compound as a brown solid (2.51 g).
¹H-NMR(DMSO-d₆)δ: 1.28(3H,t,J=7.2Hz),1.98(3H,d,J=2.1Hz),4.25(2H,q,J=7.1Hz),5.93( 1H,s),7.20(1H,dd,J=6.8,2.1Hz),7.28-7.51(2H,m),11.84(1H,brs).

### Reference Example 207

### ethyl 1-(5-fluoro-2-methylphenyl)-5-hydroxy-1H-pyrazole-3-carboxylate

To a solution of (5-fluoro-2-methylphenyl)hydrazine hydrochloride (25.0 g) in ethanol (500 mL) were added potassium carbonate (39.1 g) and diethyl but-2-ynedioate (24.1 g), and the mixture was refluxed for 18 hr, cooled to 0°C, and acidified with 6 mol/L hydrochloric acid. Ethanol was evaporated under reduced pressure, and the residue was extracted with ethyl acetate. The extract was washed with saturated brine, dried over anhydrous sodium sulfate, and concentrated under reduced pressure. The residue was purified by silica gel column chromatography (eluent: hexane-ethyl acetate=9:1→4:1), and solidified with a mixed solvent of ethyl acetate and diisopropyl ether. The obtained solid was collected by filtration, and dried under reduced pressure to give the title compound as a white solid (7.8g, yield 21%).
¹H-NMR(DMSO-d₆)δ: 1.27(3H,t,J=7.2Hz),2.04(3H,s),4.24(2H,q,J=7.2Hz),5.91(1H,s),7. 21-7.32(2H,m),7.40-7.56(1H,m),11.81(1H,s).

### Reference Example 208

### ethyl 1-(2-chloro-3-fluorophenyl)-5-hydroxy-1H-pyrazole-3-carboxylate

To a solution of (2-chloro-3-fluorophenyl)hydrazine hydrochloride (4.3 g) in ethanol (80 mL) were added potassium carbonate (6.1 g) and diethyl but-2-ynedioate (3.7 g), and the mixture was refluxed for 14 hr, cooled to 0°C, and acidified with 6 mol/L hydrochloric acid. Ethanol was evaporated under reduced pressure, and the residue was extracted with ethyl acetate. The extract was washed with saturated brine, dried over anhydrous sodium sulfate, and concentrated under reduced pressure. The residue was washed with diethyl ether, collected by filtration, and dried under reduced pressure to give the title compound as a pale-yellow powder (3.0 g, yield 47%).
¹H-NMR(DMSO-d₆)δ: 1.28(3H,t,J=7.2Hz),4.24(2H,q,J=7.2Hz),5.90(1H,s),7.42-7.45(1H,m),7.52-7.66(2H,m),12.06(1H,s).

### Reference Example 209

### ethyl 1-(2-chloro-5-fluorophenyl)-5-hydroxy-1H-pyrazole-3-carboxylate

To a solution of (2-chloro-5-fluorophenyl)hydrazine hydrochloride (10 g) in ethanol (200 mL) were added potassium carbonate (14.0 g) and diethyl but-2-ynedioate (8.6 g), and the mixture was refluxed for 24 hr, allowed to cool to room temperature, and treated with 6 mol/L hydrochloric acid. Ethanol was evaporated under reduced pressure, water was added to the residue, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=4:1→1:2) to give the title compound as a white powder (985 mg, yield 6.8%).
¹H-NMR(DMSO-d₆)δ: 1.28(3H,t,J=7.2Hz),4.25(2H,q,J=7.2Hz),5.90(1H,s),7.45-7.51(1H,m),7.56-7.60(1H,m),7.71-7.75(1H,m),11.99(1H,brs).

### Reference Example 210

### ethyl 1-(2-fluoropyridin-3-yl)-5-hydroxy-1H-pyrazole-3-carboxylate

To a solution of 2-fluoro-3-hydrazinopyridine (30.0 g) in ethanol (472 mL) were added sodium carbonate (65.2 g) and diethyl but-2-ynedioate (40.2 g), and the mixture was refluxed for 18 hr, allowed to cool to room temperature, treated with 2 mol/L hydrochloric acid, and extracted twice with ethyl acetate. The extract was dried over anhydrous sodium sulfate, and concentrated under reduced pressure. The residue was suspended in diethyl ether, and the obtained solid was collected by filtration, and dried under reduced pressure to give the title compound as a yellow solid (20.0 g, yield 34%). ¹H-NMR (DMSO-d₆)δ: 1.28(3H,t,J=7.2Hz),4.23(2H,q,J=7.2Hz),5.94(1H,s),7.57(1H,ddd,J =7.6,4.8,1.2Hz),7.49(1H,ddd,J=9.6,7.6,1.6Hz),8.39(1H,dt,J=4.8, 1.6Hz),12.3(1H,brs).

### Reference Example 211

### ethyl 1-(2-fluorophenyl)-5-{[(trifluoromethyl)sulfonyl]oxy}-1H-pyrazole-3-carboxylate

To a solution of ethyl 1-(2-fluorophenyl)-5-hydroxy-1H-pyrazole-3-carboxylate (4.35 g) in tetrahydrofuran (90 mL) were added N-phenylbis(trifluoromethanesulfonimide) (7.45 g) and triethylamine (2.9 mL) at 5°C, and the mixture was stirred at room temperature for 2 hr. Water was added to the reaction mixture, and the mixture was extracted with ethyl acetate. The separated aqueous layer was extracted again with ethyl acetate. The combined organic layers were washed with saturated brine, dried over anhydrous magnesium sulfate, and concentrated under reduced pressure. The residue was purified by silica gel column chromatography (eluent: hexane-ethyl acetate=19:1→3:2) to give the title compound as a colorless oil (6.65 g, yield 100%).
-NMR(CDCl₃)δ: 1.42(3H,t,J=7.1Hz),4.45(2H,q,J=7.2Hz),6.86(1H,s),7.22-7.44(2H,m),7.48-7.60(2H,m).

### Reference Example 212 ethyl 1-(2-methylphenyl)-5-{[(trifluoromethyl)sulfonyl]oxy}-1H-pyrazole-3-carboxylate

To a solution of ethyl 5-hydroxy-1-(2-methylphenyl)-1H-pyrazole-3-carboxylate (5.03 g) in tetrahydrofuran (60 mL) were added N-phenylbis(trifluoromethanesulfonimide) (8.01 g) and triethylamine (3.4 mL) at 0°C, and the mixture was stirred at room temperature for 1 hr. Water was added to the reaction mixture, and the mixture was extracted with ethyl acetate. The extract was washed successively with saturated aqueous sodium hydrogen carbonate solution, water and saturated brine, dried over anhydrous sodium sulfate, and concentrated under reduced pressure to give the title compound as a brown oil (9.6 g, yield quantitative).
¹H-NMR(CDCl₃)δ: 1.41(3H,t,J=7.2Hz),2.13(3H,s),4.44(2H,q,J=7.2Hz),6.83(1H,s),7. 27-7.45(4H,s).

### Reference Example 213

### ethyl 1-(2-chlorophenyl)-5-{[(trifluoromethyl)sulfonyl]oxy}-1H-pyrazole-3-carboxylate

To a solution of ethyl 1-(2-chlorophenyl)-5-hydroxy-1H-pyrazole-3-carboxylate (2.0 g) in tetrahydrofuran (15 mL) were added triethylamine (917 mg) and N-phenylbis(trifluoromethanesulfonimide) (3.2 g), and the mixture was stirred at room temperature for 1 hr. Water was added to the reaction mixture, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=19:1→7:1) to give the title compound as a yellow oil (2.85 g, yield 95%).
¹H-NMR(CDCl₃)δ: 1.42(3H,t,J=7.2Hz) ,4.44(2H,q,J= 7.2Hz),6.84(1H,s),7.25-7.58(4H,m).

### Reference Example 214

### ethyl 1-(2,3-difluorophenyl)-5-{[(trifluoromethyl)sulfonyl]oxy}-1H-pyrazole-3-carboxylate

To a solution of ethyl 1-(2,3-difluorophenyl)-5-hydroxy-1H-pyrazole-3-carboxylate (2.0 g) in tetrahydrofuran (20 mL) were added triethylamine (905 mg) and N-phenylbis(trifluoromethanesulfonimide) (3.2 g), and the mixture was stirred at room temperature for 30 min. Water was added to the reaction mixture, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=19:1→9:1) to give the title compound as a yellow oil (3.0 g, yield quantitative).
¹H-NMR(CDCl₃)δ: 1.42(3H,t,J=7.2Hz),4.45(2H,q,J=7.2Hz),6.86(1H,s),7.23-7.43(4H,m).

### Reference Example 215

### ethyl 1-(2,4-difluorophenyl)-5-{[(trifluoromethyl)sulfonyl]oxy}-1H-pyrazole-3-carboxylate

To a solution of ethyl 1-(2,4-difluorophenyl)-5-hydroxy-1H-pyrazole-3-carboxylate (2.0 g) in tetrahydrofuran (20 mL) were added triethylamine (905 mg) and N-phenylbis(trifluoromethanesulfonimide) (3.2 g), and the mixture was stirred at room temperature for 30 min. Water was added to the reaction mixture, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=19:1→9:1) to give the title compound as a yellow oil (2.8 g, yield 94%).
¹H-NMR(CDCl₃)δ: 1.42(3H,t,J=7.2Hz),4.44(2H,q,J=7.2Hz),6.84(1H,s),7.01-7.09(2H,m),7.50-7.57(1H,m).

### Reference Example 216

### ethyl 1-(2,5-difluorophenyl)-5-{[(trifluoromethyl)sulfonyl]oxy}-1H-pyrazole-3-carboxylate

To a solution of ethyl 1-(2,5-difluorophenyl)-5-hydroxy-1H-pyrazole-3-carboxylate (2.0 g) in tetrahydrofuran (20 mL) were added triethylamine (905 mg) and N-phenylbis(trifluoromethanesulfonimide) (3.2 g), and the mixture was stirred at room temperature for 30 min. Water was added to the reaction mixture, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=19:1→9:1) to give the title compound as a yellow oil (3.4 g, yield quantitative).
¹H-NMR(CDCl₃)δ: 1.42(3H,t,J=7.2Hz),4.45(2H,q,J=7.2Hz),6.85(1H,s),7.23-7.42(4H,m).

### Reference Example 217

### ethyl 1-(2-fluoro-3-methylphenyl)-5-{[(trifluoromethyl)sulfonyl]oxy}-1H-pyrazole-3-carboxylate

To a solution of ethyl 1-(2-fluoro-3-methylphenyl)-5-hydroxy-1H-pyrazole-3-carboxylate (1.0 g) in tetrahydrofuran (10 mL) were added triethylamine (459 mg) and N-phenylbis(trifluoromethanesulfonimide) (1.6 g), and the mixture was stirred at room temperature for 1 hr. Water was added to the reaction mixture, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=19:1→7:1) to give the title compound as a yellow oil (1.6 g, yield quantitative).
¹H-NMR(CDCl₃)δ: 1.41(3H,t,J=7.2Hz),2.35(1H,d,J=2.1Hz),4.44(2H,q,J=7.2Hz),6.84( 1H,s),7.15-7.21(1H,m),7.23-7.43(3H,m).

### Reference Example 218

### ethyl 1-(2-fluoro-4-methylphenyl)-5-{[(trifluoromethyl)sulfonyl]oxy}-1H-pyrazole-3-carboxylate

To a solution of ethyl 1-(2-fluoro-4-methylphenyl)-5-hydroxy-1H-pyrazole-3-carboxylate (1.0 g) in tetrahydrofuran (10 mL) were added triethylamine (459 mg) and N-phenylbis(trifluoromethanesulfonimide) (1.6 g), and the mixture was stirred at room temperature for 30 min. Water was added to the reaction mixture, and the mixture was extracted with ethyl acetate. The extract was washed with saturated brine, dried over anhydrous sodium sulfate, and concentrated under reduced pressure. The residue was purified silica gel column chromatography (eluent: hexane-ethyl acetate=19:1→9:1) to give the title compound as a yellow oil (1.6 g, yield quantitative).
¹H-NMR (CDCl₃) δ: 1.41(3H,t,J=7.2Hz),2.44(3H,s),4.44(2H,q,J=7.2Hz),6.83(1H,s),7. 07-7.41(4H,m).

### Reference Example 219

### ethyl 1-(2-fluoro-5-methylphenyl)-5-{[(trifluoromethyl)sulfonyl]oxy}-1H-pyrazole-3-carboxylate

To a solution of ethyl 1-(2-fluoro-5-methylphenyl)-5-hydroxy-1H-pyrazole-3-carboxylate (1.0 g) in tetrahydrofuran (10 mL) were added triethylamine (458 mg) and N-phenylbis(trifluoromethanesulfonimide) (1.6 g), and the mixture was stirred at room temperature for 1 hr. Water was added to the reaction mixture, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=99:1→9:1) to give the title compound as a yellow oil (1.8 g, yield quantitative).
¹H-NMR(CDCl₃)δ: 1.42(3H,t,J=7.2Hz),2.38(3H,s),4.44(2H,q,J=7.2Hz),6.83(1H,s),7. 11-7.17(1H,m),7.25-7.42(2H,m).

### Reference Example 220

### ethyl 1-(3-fluoro-2-methylphenyl)-5-{[(trifluoromethyl)sulfonyl]oxy}-1H-pyrazole-3-carboxylate

To a solution of crude ethyl 1-(3-fluoro-2-methylphenyl)-5-hydroxy-1H-pyrazole-3-carboxylate (2.51 g) in tetrahydrofuran (45 mL) were added triethylamine (946 mg) and N-phenylbis(trifluoromethanesulfonimide) (3.34 g), and the mixture was stirred at room temperature for 10 min, and concentrated under reduced pressure. Water was added to the residue, and the mixture was extracted with ethyl acetate. The separated aqueous layer was extracted again with ethyl acetate. The combined organic layers were washed with saturated brine, dried over anhydrous sodium sulfate, and concentrated under reduced pressure. The residue was purified by silica gel column chromatography (eluent: hexane-ethyl acetate=49:1→9:1) to give the title compound an orange oil (3.56 g, yield in two step 96%).
¹H-NMR(CDCl₃)δ: 1.42(3H,t,J=7.2Hz),2.05(3H,d,J=2.3Hz),4.45(2H,q,J=7.2Hz),7.15( 1H,d,J=7.7Hz),7.19-7.26(1H,m),7.28-7.32(1H,m),7.32-7.45(1H,m).

### Reference Example 221

### ethyl 1-(5-fluoro-2-methylphenyl)-5-{[(trifluoromethyl)sulfonyl]oxy}-1H-pyrazole-3-carboxylate

To a solution of ethyl 1-(5-fluoro-2-methylphenyl)-5-hydroxy-1H-pyrazole-3-carboxylate (7.8 g) in tetrahydrofuran (100 mL) were added triethylamine (3.5 g) and N-phenylbis(trifluoromethanesulfonimide) (11.5 g), and the mixture was stirred at room temperature for 15 min, and concentrated under reduced pressure. Water was added to the residue, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=19:1→9:1) to give the crude title compound as a yellow oil (13.9 g).
¹H-NMR(CDCl₃)δ: 1.42(3H,t,J=7.2Hz),2.11(3H,s),4.44(2H,q,J=7.2Hz),6.84(1H,s),7. 05-7.09(1H,m),7.14-7.20(1H,m),7.25-7.41(1H,m).

### Reference Example 222

### ethyl 1-(2-chloro-3-fluorophenyl)-5-{[(trifluoromethyl)sulfonyl]oxy}-1H-pyrazole-3-carboxylate

To a solution of ethyl 1-(2-chloro-3-fluorophenyl)-5-hydroxy-1H-pyrazole-3-carboxylate (3.0 g) in tetrahydrofuran (40 mL) were added triethylamine (1.3 g) and N-phenylbis(trifluoromethanesulfonimide) (4.1 g), and the mixture was stirred at room temperature for 10 min, and concentrated under reduced pressure. Water was added to the residue, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=19:1→9:1) to give the title compound as a colorless oil (4.1 g, yield 96%).
¹H-NMR (CDCl₃) δ: 1.42(3H,t,J=7.2Hz),4.45(2H,q,J=7.2Hz),6.85(1H,s),7.26-7.47(3H,m).

### Reference Example 223

### ethyl 1-(2-chloro-5-fluorophenyl)-5-{[(trifluoromethyl)sulfonyl]oxy}-1H-pyrazole-3-carboxylate

To a solution of ethyl 1-(2-chloro-5-fluorophenyl)-5-hydroxy-1H-pyrazole-3-carboxylate (985 mg) in tetrahydrofuran (12 mL) were added triethylamine (423 mg) and N-phenylbis(trifluoromethanesulfonimide) (1.5 g), and the mixture was stirred at room temperature for 30 min. Water was added to the reaction mixture, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=19:1→9:1) to give the title compound as a yellow oil (1.39 g, yield 96%).
¹H-NMR(CDCl₃)δ: 1.42(3H,t,J=7.2Hz),4.44(2H,q,J=7.2Hz),6.85(1H,s),7.22-7.30(2H,m),7.52-7.56(1H,m).

### Reference Example 224

### ethyl 1-(2-fluoropyridin-3-yl)-5-{[(trifluoromethyl)sulfonyl]oxy}-lH-pyrazole-3-carboxylate

To a solution of ethyl 1-(2-fluoropyridin-3-yl)-5-hydroxy-1H-pyrazole-3-carboxylate (2.0 g) in tetrahydrofuran (20 mL) were added triethylamine (966 mg) and N-phenylbis(trifluoromethanesulfonimide) (3.1 g), and the mixture was stirred at room temperature for 15 min. Water was added to the reaction mixture, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=19:1→9:1) to give the title compound as a yellow oil (2.1 g, yield 70%).
¹H-NMR(CDCl₃)δ: 1.43(3H,t,J=7.2Hz),4.46(2H,q,J=7.2Hz),6.88(1H,s),7.40-7.45(1H,m),7.99-8.06(1H,m),8.40-8.43(1H,m).

### Reference Example 225

### ethyl 5-({3-[(2-ethylhexyl)oxy]-3-oxopropyl}thio)-1-(2-fluorophenyl)-1H-pyrazole-3-carboxylate

To a solution of ethyl 1-(2-fluorophenyl)-5-{[(trifluoromethyl)sulfonyl]oxy}-1H-pyrazole-3-carboxylate (6.65 g) in toluene (100 mL) were added 2-ethylhexyl 3-mercaptopropionate (5.69 g), tris(dibenzylideneacetone)dipalladium(0) (1.59 g), 4,5-bis(diphenylphosphino)-9,9-dimethylxanthine (2.02 g) and cesium carbonate (11.37 g), and the mixture was heated under reflux for 2 hr. The reaction mixture was allowed to cool to about room temperature, anhydrous magnesium sulfate and celite were added, and the mixture was stirred for 30 min. The insoluble material was filtered off, and the filtrate was concentrated under reduced pressure. The residue was purified by silica gel column chromatography (eluent: hexane-ethyl acetate=19→11:9) to give the title compound as a pale-brown oil (3.54 g, yield 45%).
¹H-NMR(CDCl₃)δ: 0.82-0.93(6H,m),1.17-1.60 (12H,m),2.56(2H,t,J=7.4Hz),2.94(2H,t,J=7.2Hz),3.91-4.03(2H,m),4.43(2H,q,J=7.2Hz),7.05(1H,s),7.19-7.34(2H,m),7.42-7.55(2H,m).

### Reference Example 226

### ethyl 5-({3-[(2-ethylhexyl)oxy]-3-oxopropyl}thio)-1-(2-methylphenyl)-1H-pyrazole-3-carboxylate

Ethyl 1-(2-methylphenyl)-5-{[(trifluoromethyl)sulfonyl]oxy}-1H-pyrazole-3-carboxylate (1.07 g), 2-ethylhexyl 3-mercaptopropanoate (618 mg), tris(dibenzylideneacetone)dipalladium(0) (66 mg), 9,9-dimethyl-4,5-bis(diphenylphosphino)xanthine (83 mg) and N-ethyldiisopropylamine (1.0 mL) were stirred in toluene (15 mL) at 105°C for 2 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous magnesium sulfate, and concentrated under reduced pressure to give the title compound as a brown oil (1.31 g, yield quantitative).
¹H-NMR(CDCl₃)δ: 0.85-0.92(6H,m),1.23-1.35(9H,m),1.40(3H,t,J=7.2Hz),2.05(3H,s),2.56(2H,t,J=7.2Hz),2. 92(3H,t,J=7.2Hz),3.96-3.99(2H,m),4.42(2H,q,J=7.2Hz),6.98(1H,s),7.24-7.41(4H,m).

### Reference Example 227

### ethyl 1-(2-chlorophenyl)-5-({3-[(2-ethylhexyl)oxy]-3-oxopropyl}thio)-1H-pyrazole-3-carboxylate

A solution of ethyl 1-(2-chlorophenyl)-5-{[(trifluoromethyl)sulfonyl]oxy}-1H-pyrazole-3-carboxylate (2.85 g), 2-ethylhexyl 3-mercaptopropionate (2.34 g) and N-ethyldiisopropylamine (1.85 g) in toluene (30 mL) was degassed, tris(dibenzylideneacetone)dipalladium(0) (327 mg) and 4,5-bis(diphenylphosphino)-9,9-dimethylxanthine (414 mg) were added, and the mixture was degassed. The mixture was stirred at 110°C for 4 hr under an argon atmosphere, and allowed to cool to room temperature. Water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=19:1→7:1) to give the title compound as a yellow oil (2.87 g, yield 86%).
¹H-NMR(CDCl₃)δ: 0.85-0.91(6H,m),1.23-1.38(8H,m),1.41(3H,t,J=7.2Hz),1.50-1.60(1H,m),2.57(2H,t,J=7.5Hz),2.93(2H,t,J=7.5Hz),3.92-4.02(2H,m),4.43(2H,q,J=7.2Hz),7.04(1H,s),7.36-7.55(4H,m).

### Reference Example 228

### ethyl 1-(2,3-difluorophenyl)-5-({3-[(2-ethylhexyl)oxy]-3-oxopropyl}thio)-1H-pyrazole-3-carboxylate

A solution of ethyl 1-(2,3-difluorophenyl)-5-{[(trifluoromethyl)sulfonyl]oxy}-1H-pyrazole-3-carboxylate (3.0 g), 2-ethylhexyl 3-mercaptopropionate (2.4 g) and N-ethyldiisopropylamine (1.9 g) in toluene (30 mL) was degassed, tris(dibenzylideneacetone)dipalladium(0) (342 mg) and 4,5-bis(diphenylphosphino)-9,9-dimethylxanthine (432 mg) were added, and the mixture was degassed. The mixture was stirred at 110°C for 1.5 hr under an argon atmosphere, and allowed to cool to room temperature. Water and ethyl acetate were added, and the mixture was filtered through celite. The organic layer of the filtrate was washed with saturated brine, dried over anhydrous sodium sulfate, and concentrated under reduced pressure. The residue was purified by silica gel column chromatography (eluent: hexane-ethyl acetate=19:1→9:1→4:1) to give the title compound as a yellow oil (2.61 g, yield 75%). ¹H-NMR(CDCl₃)δ: 0.81-0.90(6H,m) ,1.23-1.43(11H,m),1.50-1.60(1H,m),2.57(2H,t,J=7.2Hz),2.96(2H,t,J=7.2Hz),3.93-4.02(2H,m),4.43(2H,q,J=7.2Hz),7.05(1H,s),7.17-7.38(3H,m).

### Reference Example 229

### ethyl 1-(2,4-difluorophenyl)-5-({3-[(2-ethylhexyl)oxy]-3-oxopropyl}thio)-1H-pyrazole-3-carboxylate

A solution of ethyl 1-(2,4-difluorophenyl)-5-{[(trifluoromethyl)sulfonyl]oxy}-1H-pyrazole-3-carboxylate (2.8 g), 2-ethylhexyl 3-mercaptopropionate (2.3 g) and N-ethyldiisopropylamine (1.8 g) in toluene (30 mL) was degassed, tris(dibenzylideneacetone)dipalladium(0) (322 mg) and 4,5-bis(diphenylphosphino)-9,9-dimethylxanthine (406 mg) were added, and the mixture was degassed. The mixture was stirred under an argon atmosphere at 110°C for 1.5 hr, and allowed to cool to room temperature. Water and ethyl acetate were added, and the mixture was filtered through celite. The organic layer of the filtrate was washed with saturated brine, dried over anhydrous sodium sulfate, and concentrated under reduced pressure. The residue was purified by silica gel column chromatography (eluent: hexane-ethyl acetate=19:1→9:1) to give the title compound as a yellow oil (1.9 g, yield 59%).
¹H-NMR(CDCl₃)δ: 0.85-0.91(6H,m),1.24-1.43(11H,m),1.50-1.60(1H,m),2.57(2H,t,J=7.2Hz),2.96(2H,t,J=7.2Hz),3.92-4.02(2H,m),4.43(2H,q,J=7.2Hz),6.95-7.08(3H,m),7.42-7.49(1H,m).

### Reference Example 230

### ethyl 1-(2,5-difluorophenyl)-5-({3-[(2-ethylhexyl)oxy]-3-oxopropyl}thio)-lH-pyrazole-3-carboxylate

A solution of ethyl 1-(2,5-difluorophenyl)-5-{[(trifluoromethyl)sulfonyl]oxy}-1H-pyrazole-3-carboxylate (3.4 g), 2-ethylhexyl 3-mercaptopropionate (2.4 g) and N-ethyldiisopropylamine (1.9 g) in toluene (30 mL) was degassed, tris(dibenzylideneacetone)dipalladium(0) (342 mg) and 4,5-bis(diphenylphosphino)-9,9-dimethylxanthine (432 mg) were added, and the mixture was degassed. The mixture was stirred at 110°C for 2 hr under an argon atmosphere, and allowed to cool to room temperature. Water and ethyl acetate were added, and the mixture was filtered through celite. The organic layer of the filtrate was washed with saturated brine, dried over anhydrous sodium sulfate, and concentrated under reduced pressure. The residue was purified by silica gel column chromatography (eluent: hexane-ethyl acetate=19:1→7:1) to give the title compound as a yellow oil (1.8 g, yield 53%).
¹H-NMR(CDCl₃)δ: 0.85-0.91(6H,m),1.24-1.43(11H,m),1.50-1.60(1H,m),2.57(2H,t,J=7.2Hz),2.97(2H,t,J=7.2Hz),3.93-4.03(2H,m),4.43(2H,q,J=7.2Hz),7.04(1H,s),7.04(1H,s),7.17-7.26(3H,m).

### Reference Example 231

### ethyl 5-({3-[(2-ethylhexyl)oxy]-3-oxopropyl}thio)-1-(2-fluoro-3-methylphenyl)-1H-pyrazole-3-carboxylate

A solution of ethyl 1-(2-fluoro-3-methylphenyl)-5-{[(trifluoromethyl)sulfonyl]oxy}-1H-pyrazole-3-carboxylate (1.6 g), 2-ethylhexyl 3-mercaptopropionate (1.2 g) and N-ethyldiisopropylamine (977 mg) in toluene (15 mL) was degassed, tris(dibenzylideneacetone)dipalladium(0) (173 mg) and 4,5-bis(diphenylphosphino)-9,9-dimethylxanthine (219 mg) were added, and the mixture was degassed. The mixture was stirred at 110°C for 1.5 hr under an argon atmosphere, and allowed to cool to room temperature. Water and ethyl acetate were added, and the mixture was filtered through celite. The organic layer of the filtrate was washed with saturated brine, dried over anhydrous sodium sulfate, and concentrated under reduced pressure. The residue was purified by silica gel column chromatography (eluent: hexane-ethyl acetate= 9:1→4:1) to give the title compound as a yellow oil (1.5 g, yield 85%).
¹H-NMR(CDCl₃)δ: 0.85-0.91(6H,m),1.24-1.43(11H,m),1.43-1.60(1H,m),2.34-2.35(3H,m),2.56(2H,t,J=7.2Hz),2.94(2H,t,J=7.2Hz),3.92-4.02(2H,m),4.42(2H,q,J=7.2Hz),7.03(1H,s),7.10-7.16(1H,m),7.23-7.35(2H,m).

### Reference Example 232

### ethyl 5-({3-[(2-ethylhexyl)oxy]-3-oxopropyl}thio)-1-(2-fluoro-4-methylphenyl)-1H-pyrazole-3-carboxylate

A solution of ethyl 1-(2-fluoro-4-methylphenyl)-5-{[(trifluoromethyl)sulfonyl]oxy}-lH-pyrazole-3-carboxylate (1.6 g), 2-ethylhexyl 3-mercaptopropionate (1.2 g) and N-ethyldiisopropylamine (977 mg) in toluene (15 mL) was degassed, tris(dibenzylideneacetone)dipalladium(0) (173 mg) and 4,5-bis(diphenylphosphino)-9,9-dimethylxanthine (219 mg) were added, and the mixture was degassed. The mixture was stirred at 110°C for 1 hr under an argon atmosphere, and allowed to cool to room temperature. Water and ethyl acetate were added, and the mixture was filtered through celite. The organic layer of the filtrate was washed with saturated brine, dried over anhydrous sodium sulfate, and concentrated under reduced pressure. The residue was purified by silica gel column chromatography (eluent: hexane-ethyl acetate=19:1→6:1) to give the title compound as a yellow oil (1.3 g, yield 73%).
¹H-NMR(CDCl₃)δ: 0.85-0.90(6H,m),1.23-1.43(11H,m),1.48-1.60(1H,m),2.43(3H,s),2.55(2H,t,J=7.2Hz),2.93(2H,t,J=7.2Hz),3. 92-4.03(2H,m),4.42(2H,q,J=7.2Hz),7.02-7.06(3H,m),7.28-7.33(1H,m).

### Reference Example 233

### ethyl 5-({3-[(2-ethylhexyl)oxy]-3-oxopropyl}thio)-1-(2-fluoro-5-methylphenyl)-1H-pyrazole-3-carboxylate

A solution of ethyl 1-(2-fluoro-5-methylphenyl)-5-{[(trifluoromethyl)sulfonyl]oxy}-1H-pyrazole-3-carboxylate (1.8 g), 2-ethylhexyl 3-mercaptopropionate (1.2 g) and N-ethyldiisopropylamine (977 mg) in toluene (20 mL) was degassed, tris(dibenzylideneacetone)dipalladium(0) (173 mg) and 4,5-bis(diphenylphosphino)-9,9-dimethylxanthine (219 mg) were added, and the mixture was degassed. The mixture was stirred at 110°C for 1.5 hr under an argon atmosphere, and allowed to cool to room temperature. Water and ethyl acetate were added, and the mixture was filtered through celite. The organic layer of the filtrate was washed with saturated brine, dried over anhydrous sodium sulfate, and concentrated under reduced pressure. The residue was purified by silica gel column chromatography (eluent: hexane-ethyl acetate=19:1→9:1) to give the title compound as a yellow oil (1.7 g, yield 94%).
¹H-NMR(CDCl₃)δ: 0.85-0.91(6H,m),1.23-1.43(12H,m),1.50-1.60(1H,m),2.37(3H,s),2.56(2H,t,J=7.2Hz),2.95(2H,t,J=7.2Hz),3. 93-4.03(2H,m),4.42(2H,q,J=7.2Hz),7.03(1H,s),7.07-7.13(1H,m),7.23-7.28(2H,m).

### Reference Example 234

### ethyl 5-({3-[(2-ethylhexyl)oxy]-3-oxopropyl}thio)-1-(3-fluoro-2-methylphenyl)-1H-pyrazole-3-carboxylate

A solution of ethyl 1-(3-fluoro-2-methylphenyl)-5-{[(trifluoromethyl)sulfonyl]oxy}-1H-pyrazole-3-carboxylate (4.23 g), 2-ethylhexyl 3-mercaptopropionate (2.80 g) and N-ethyldiisopropylamine (2.07 g) in toluene (30 mL) was degassed, tris(dibenzylideneacetone)dipalladium(0) (98 mg) and 4,5-bis(diphenylphosphino)-9,9-dimethylxanthine (123 mg) were added, and the mixture was degassed. The mixture was stirred at 80°C for 3 hr under an argon atmosphere, and filtered through silica gel, and the filtrate was concentrated under reduced pressure. The residue was purified by silica gel column chromatography (eluent: hexane-ethyl acetate=19:1→17:3) to give the title compound as a yellow oil (3.91 g, yield 79%).
¹H-NMR(CDCl₃)δ: 0.80-0.96(6H,m),1.20-1.36(8H,m),1.41(3H,t,J=7.2Hz),1.48-1.60(1H,m),1.96(3H,d,J=2.3Hz),2.53-2.63(2H,m),2.94(2H,t),3.91-4.05(2H,m),4.43(2H,q,J=7.2Hz),6.99(1H,s),7.11(1H,d,J=7.7Hz),7. 14-7.22(1H,m),7.22-7.41(1H,m).

### Reference Example 235

### ethyl 5-({3-[(2-ethylhexyl)oxy]-3-oxopropyl}thio)-1-(5-fluoro-2-methylphenyl)-1H-pyrazole-3-carboxylate

A solution of ethyl 1-(5-fluoro-2-methylphenyl)-5-{[(trifluoromethyl)sulfonyl]oxy}-1H-pyrazole-3-carboxylate (13.9 g), 2-ethylhexyl 3-mercaptopropionate (7.8 g) and N-ethyldiisopropylamine (5.9 g) in toluene (150 mL) was degassed, tris(dibenzylideneacetone)dipalladium(0) (219 mg) and 4,5-bis(diphenylphosphino)-9,9-dimethylxanthine (277 mg) were added, and the mixture was degassed. The mixture was stirred at 110°C for 3 hr under an argon atmosphere, and allowed to cool to room temperature. Water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=19:1→7:1) to give the title compound as a yellow oil (11.7 g, yield 86%).
¹H-NMR(CDCl₃)δ: 0.85-0.90(6H,m),1.24-1.42(11H,m),1.50-1.60(1H,m),2.02(3H,s),2.58(2H,t,J=7.2Hz),2.95(2H,t,J=7.2Hz),3. 93-4.03(2H,m),4.42(2H,q,J=7.2Hz),6.98(1H,s),7.02-7.05(1H,m),7.09-7.15(1H,m),7.25-7.30(1H,m).

### Reference Example 236

### ethyl 1-(2-chloro-3-fluorophenyl)-5-({3-[(2-ethylhexyl)oxy]-3-oxopropyl}thio)-1H-pyrazole-3-carboxylate

A solution of ethyl 1-(2-chloro-3-fluorophenyl)-5-{[(trifluoromethyl)sulfonyl]oxy}-1H-pyrazole-3-carboxylate (4.1 g), 2-ethylhexyl 3-mercaptopropionate (2.6 g) and N-ethyldiisopropylamine (1.9 g) in toluene (40 mL) was degassed, tris(dibenzylideneacetone)dipalladium(0) (91 mg) and 4,5-bis(diphenylphosphino)-9,9-dimethylxanthine (115 mg) were added, and the mixture was degassed. The mixture was stirred at 110°C for 1.5 hr under an argon atmosphere, and allowed to cool to room temperature. Water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=19:1→7:1) to give the title compound as a yellow oil (3.9 g, yield 80%).
¹H-NMR(CDCl₃)δ: 0.85-0.91(6H,m),1.24-1.43(11H,m),1.50-1.60(1H,m),2.58(2H,t,J=7.2Hz),2.94(2H,t,J=7.2Hz),3.93-4.02(2H,m),4.43(2H,q,J=7.2Hz),7.04(1H,s),7.26-7.42(3H,m).

### Reference Example 237

### ethyl 1-(2-chloro-5-fluorophenyl)-5-({3-[(2-ethylhexyl)oxy]-3-oxopropyl}thio)-1H-pyrazole-3-carboxylate

A solution of ethyl 1-(2-chloro-5-fluorophenyl)-5-{[(trifluoromethyl)sulfonyl]oxy}-1H-pyrazole-3-carboxylate (1.4 g), 2-ethylhexyl 3-mercaptopropionate (1.1 g) and N-ethyldiisopropylamine (861 mg) in toluene (15 mL) was degassed, tris(dibenzylideneacetone)dipalladium(0) (153 mg) and 4,5-bis(diphenylphosphino)-9,9-dimethylxanthine (193 mg) were added, and the mixture was degassed. The mixture was stirred at 110°C for 4 hr under an argon atmosphere, and allowed to cool to room temperature. Water and ethyl acetate were added, and the mixture was filtered through celite. The organic layer of the filtrate was washed with saturated brine, dried over anhydrous sodium sulfate, and concentrated under reduced pressure. The residue was purified by silica gel column chromatography (eluent: hexane-ethyl acetate=19:1→9:1) to give the title compound as a yellow oil (1.34 g, yield 83%).
¹H-NMR(CDCl₃)δ: 0.85-0.91(6H,m),1.24-1.43(11H,m),1.50-1.60(1H,m),2.59(2H,t,J=7.2Hz),2.96(2H,t,J=7.2Hz),3.95-4.02(2H,m),4.43(2H,q,J=7.2Hz),7.03(1H,s),7.17-7.25(3H,m),7.46-7.52(1H,m).

### Reference Example 238

### ethyl 1-(2-fluorophenyl)-5-(phenylthio)-1H-pyrazole-3-carboxylate

To a solution of ethyl 5-bromo-1-(2-fluorophenyl)-1H-pyrazole-3-carboxylate (210 mg) in N,N-dimethylformamide (4 mL) were added potassium carbonate (463 mg) and thiophenol (0.344 mL) at room temperature under an argon atmosphere, and the mixture was stirred at 120°C for 6 hr. The reaction mixture was diluted with water, and extracted with ethyl acetate. The extract was washed with saturated brine, dried over anhydrous magnesium sulfate, and concentrated under reduced pressure to give a residue. The obtained residue was purified by basic silica gel column chromatography (eluent: hexane-ethyl acetate=19:1→3:7) to give the title compound as a colorless oil (76.1 mg, yield 33%).
¹H-NMR(CDCl₃)δ: 1.40(3H,t,J=7.1Hz) ,4.38-4.48(2H,m) 7.05-7.55(10H,m).

### Reference Example 239

### ethyl 5-[(3-methoxyphenyl)thio]-1-(2-methylphenyl)-1H-pyrazole-3-carboxylate

To a solution of ethyl 5-iodo-1-(2-methylphenyl)-1H-pyrazole-3-carboxylate (4.9 g) in tetrahydrofuran (100 mL) was added dropwise 2.5 mol/L n-butyllithium-hexane solution (6.6 mL) at -78°C, and the mixture was stirred at the same temperature for 1 hr. A solution of 1,2-bis(3-methoxyphenyl)disulfide (4.6 g) in tetrahydrofuran (38 mL) was added, and the mixture was further stirred at the same temperature for 1 hr. The reaction mixture was treated with aqueous ammonium chloride solution (100 mL), and extracted with ethyl acetate. The extract was dried over anhydrous sodium sulfate, and concentrated under reduced pressure. The residue was purified by silica gel column chromatography (eluent: hexane-ethyl acetate=15:1) to give the title compound as a yellow oil (1.3 g, yield 25%),
¹H-MMR(CDCl₃)δ: 1.40(3H,t,J=7.0Hz),1.95(3H,s),3.71(3H,s),4.42(2H,q,J=7.2Hz),6. 61(1H,t,J=2.2Hz),6.70(1H,dq,J=7.6,1.6Hz),6.75(1H,ddd,J=8.2,2.4 ,0.8Hz),7.07(1H,dd,J=8.0,1.2Hz),7.11(1H,s),7.15-7.19(1H,m),7.24(1H,d,J=7.2Hz),7.31-7.36(2H,m).

### Reference Example 240

### ethyl 1-(2,6-difluorophenyl)-5-(phenylthio)-1H-pyrazole-3-carboxylate

To a solution of ethyl 1-(2,6-difluorophenyl)-5-iodo-1H-pyrazole-3-carboxylate (300 mg) in tetrahydrofuran (5.9 mL) was added dropwise 2.5 mol/L n-butyllithium-hexane solution (0.635 mL) at -78°C, and the mixture was stirred at the same temperature for 1 hr. A solution of diphenyldisulfide (210 mg) in tetrahydrofuran (2 mL) was added, and the mixture was further stirred at the same temperature for 1 hr. The reaction mixture was treated with aqueous ammonium chloride solution (100 mL), and extracted with ethyl acetate. The extract was dried over anhydrous sodium sulfate, and concentrated under reduced pressure. The residue was purified by silica gel column chromatography (eluent: hexane-ethyl acetate=15:1) to give the title compound as a yellow oil (116 mg, yield 41%).
¹H-NMR (CDCl₃) δ: 1.40(3H,t,J=7.0Hz),4.43(2H,q,J=7.0Hz),6.98(2H,t,J=2.2Hz),7.09-7.12(2H,m),7.17(1H,s),7.20-7.21(3H,m),7.38-7.45(1H,m).

### Reference Example 241

### ethyl 1-(2-fluorophenyl)-5-[(3-methoxyphenyl)thio]-1H-pyrazole-3-carboxylate

To a solution of ethyl 5-bromo-1-(2-fluorophenyl)-1H-pyrazole-3-carboxylate (500 mg) in N,N-dimethylformamide (8 mL) were added potassium carbonate (662 mg) and 3-methoxybenzenethiol (0.594 mL) at room temperature under an argon atmosphere, and the mixture was stirred at 120°C for 3 hr. The reaction mixture was diluted with water, and extracted with ethyl acetate. The extract was washed with saturated brine, dried over anhydrous magnesium sulfate, and concentrated under reduced pressure to give a residue. The obtained residue was purified by basic silica gel column chromatography (eluent: hexane-ethyl acetate=19:1→13:7) to give the title compound as a colorless oil (including impurity) (154 mg, yield 26%).
LC-MS(ESI),m/z,372.9(M+H).

### Reference Example 242

### ethyl 5-[(6-chloropyridin-3-yl)thio]-1-(2-fluorophenyl)-1H-pyrazole-3-carboxylate

To a solution of ethyl 5-({3-[(2-ethylhexyl)oxy]-3-oxopropyl}thio)-1-(2-fluorophenyl)-1H-pyrazole-3-carboxylate (3.54 g) in ethanol (35 mL) was added sodium ethoxide (1.07 g) under ice-cooling, and the mixture was stirred at room temperature for 2 hr, and concentrated under reduced pressure to give sodium 3-(ethoxycarbonyl)-1-(2-fluorophenyl)-1H-pyrazole-5-thiolate as a pale-yellow solid. The pale-yellow solid was suspended in toluene (90 mL), and 2-chloro-5-iodopyridine (2.44 g), tris(dibenzylideneacetone)dipalladium(0) (720 mg) and 4,5-bis(diphenylphosphino)-9,9-dimethylxanthine (909 mg) were mixed therewith. The mixture was heated under reflux for 5 hr, and allowed to cool to about room temperature. Anhydrous magnesium sulfate and celite were added, and the mixture was stirred for 30 min. The insoluble material was filtered off, and the filtrate was concentrated under reduced pressure. The residue was purified by silica gel column chromatography (eluent: hexane-ethyl acetate=9:1→3:7) to give the title compound as a colorless oil (including impurity) (1.93 g, yield 65%).
LC-MS(ESI),m/z,377.89(M+H).

### Reference Example 243

### ethyl 5-[(6-chloropyridin-3-yl)thio]-1-(2-methylphenyl)-1H-pyrazole-3-carboxylate

To a solution of ethyl 5-({3-[(2-ethylhexyl)oxy]-3-oxopropyl}thio)-1-(2-methylphenyl)-1H-pyrazole-3-carboxylate (1.31 g) in ethanol (15 mL) was added sodium ethoxide (387 mg) at 0°C, and the mixture was stirred at room temperature for 1 hr, and concentrated under reduced pressure. A mixture of the residue, 2-chloro-5-iodopyridine (679 mg), tris(dibenzylideneacetone)dipalladium(0) (66 mg) and 9,9-dimethyl-4,5-bis(diphenylphosphino)xanthine (83 mg) was stirred in toluene (10 mL) at 90°C for 2 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=6:1) to give the title compound as a yellow oil (479 mg, yield 45%).
¹H-NMR(CDCl₃)δ: 1.40(3H,t,J=7.2Hz),1.92(3H,s),4.42(2H,q,J=7.2Hz),7.01-7.04(1H,m),7.15-7.27(4H,m),7.32-7.39(2H,m),8.00-8.01(1H,m).

### Reference Example 244

### ethyl 5-[(3'-bromophenyl)thio]-1-(2-chlorophenyl)-1H-pyrazole-3-carboxylate

A mixture of ethyl 1-(2-chlorophenyl)-5-({3-[(2-ethylhexyl)oxy]-3-oxopropyl}thio)-1H-pyrazole-3-carboxylate (1.21 g), sodium ethoxide (362 mg), 1-bromo-3-iodobenzene (775 mg), tris(dibenzylideneacetone)dipalladium(0) (97 mg), 9,9-dimethyl-4,5-bis(diphenylphosphino)xanthine (123 mg) and N-ethyldiisopropylamine (0.88 mL) was stirred in a mixed solvent of ethanol (10 mL) and toluene (15 mL) at 80°C for 12 hr. The reaction mixture was allowed to cool to room temperature, and concentrated under reduced pressure. Water was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=4:1) to give the title compound as a yellow oil (689 mg, yield 61%).
¹H-NMR(CDCl₃)δ: 1.42(3H,t,J=7.2Hz),4.44(2H,q,J=7.2Hz),6.98-7.33(7H,m),7.38-7.49(2H,m).

### Reference Example 245

### ethyl 1-(2-chlorophenyl)-5-[(pyridin-3-yl)thio]-1H-pyrazole-3-carboxylate

To a solution of ethyl 1-(2-chlorophenyl)-5-({3-[(2-ethylhexyl)oxy]-3-oxopropyl}thio)-1H-pyrazole-3-carboxylate (500 mg) in ethanol (5 mL) was added sodium ethoxide (109 mg), and the mixture was stirred at room temperature for 2 hr. About half volume of ethanol was evaporated under reduced pressure, and the residue was dissolved in toluene (5 mL). 3-Iodopyridine (241 mg) was added, and the mixture was degassed. Tris(dibenzylideneacetone)dipalladium(0) (49 mg) and 4,5-bis(diphenylphosphino)-9,9-dimethylxanthine (62 mg) were added, and the mixture was further degassed. The mixture was stirred at 110°C for 2 hr under an argon atmosphere, and allowed to cool to room temperature. Water and ethyl acetate were added, and the mixture was filtered through celite. The organic layer of the filtrate was washed with saturated brine, dried over anhydrous sodium sulfate, and concentrated under reduced pressure. The residue was purified by silica gel column chromatography (eluent: hexane-ethyl acetate=4:1→1:1) to give the title compound as a purple oil (316 mg, yield 82%).
¹H-NMR(CDCl₃)δ: 1.41(3H,t,J=7.2Hz), 4.43(2H,q,J=7.2Hz) ,7.13-7.17(2H,m),7.17-7.33(2H,m),7.39-7.50(3H,m),8.28-8.30(1H,m),8.43-8.45(1H,m).

### Reference Example 246

### ethyl 1-(2-chlorophenyl)-5-[(5-fluoropyridin-3-yl)thio]-1H-pyrazole-3-carboxylate

To a solution of ethyl 1-(2-chlorophenyl)-5-({3-[(2-ethylhexyl)oxy]-3-oxopropyl}thio)-1H-pyrazole-3-carboxylate (500 mg) in ethanol (5 mL) was added sodium ethoxide (109 mg), and the mixture was stirred at room temperature for 1 hr. About half volume of ethanol was evaporated under reduced pressure, and the residue was dissolved in toluene (5 mL). 3-Bromo-5-fluoropyridine (207 mg) was added, and the mixture was degassed. Tris(dibenzylideneacetone)dipalladium(0) (49 mg) and 4,5-bis(diphenylphosphino)-9,9-dimethylxanthine (62 mg) were added, and the mixture was further degassed. The mixture was stirred at 110°C for 3 hr under an argon atmosphere, and allowed to cool to room temperature. Water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate= 4:1→2:1) to give the title compound as a pale-yellow oil (290 mg, yield 72%).
¹H-NMR(CDCl₃)δ: 1.42(3H,t,J=7.2Hz),4.44(2H,q,J=7.2Hz),7.08-7.13(1H,m),7.23-7.34(3H,m),7.40-7.50(2H,m) 8.08(1H,s),8.28-8.29(1H,m).

### Reference Example 247

### ethyl 1-(2-chlorophenyl)-5-[(6-chloropyridin-3-yl)thio]-1H-pyrazole-3-carboxylate

To a solution of ethyl 1-(2-chlorophenyl)-5-({3-[(2-ethylhexyl)oxy]-3-oxopropyl}thio)-1H-pyrazole-3-carboxylate (2.87 g) in ethanol (30 mL) was added sodium ethoxide (1.67 g), and the mixture was stirred at room temperature for 2 hr. About half volume of ethanol was evaporated under reduced pressure, and the residue was dissolved in toluene (10 mL). 2-Chloro-5-iodopyridine (1.62 g) was added, and the mixture was degassed. Tris(dibenzylideneacetone)dipalladium(0) (282 mg) and 4,5-bis(diphenylphosphino)-9,9-dimethylxanthine (356 mg) were added, and the mixture was further degassed. The mixture was stirred at 110°C for 2 hr under an argon atmosphere, and allowed to cool to room temperature. Water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=19:1→7:1) to give the title compound as a purple oil (2.06 g, yield 85%).
¹H-NMR(CDCl₃)δ: 1.41(3H,t,J=7.2Hz),4.43(2H,q,J=7.2Hz),7.16-7.26(2H,m),7.30-7.52(5H,m),8.03-8.04(1H,m).

### Reference Example 248

### ethyl 1-(2-chlorophenyl)-5-[(pyridin-4-yl)thio]-1H-pyrazole-3-carboxylate

To a solution of ethyl 1-(2-chlorophenyl)-5-({3-[(2-ethylhexyl)oxy]-3-oxopropyl}thio)-1H-pyrazole-3-carboxylate (500 mg) in ethanol (5 mL) was added sodium ethoxide (87 mg), and the mixture was stirred at room temperature for 1.5 hr. About half volume of ethanol was evaporated under reduced pressure, and the residue was dissolved in toluene (5 mL). 4-Iodopyridine (329 mg) was added, and the mixture was degassed. Tris(dibenzylideneacetone)dipalladium(0) (49 mg) and 4,5-bis(diphenylphosphino)-9,9-dimethylxanthine (62 mg) were added, and the mixture was further degassed. The mixture was stirred at 110°C for 2 hr under an argon atmosphere, and allowed to cool to room temperature. Water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=2:1→1:2) to give the title compound as a yellow oil (124 mg, yield 32%).
¹H-NMR(CDCl₃)δ: 1.43(3H,t,J=7.2Hz),4.46(2H,q,J=7.2Hz),6.86-6.88(1H,m),7.24-7.27(2H,m), 7.33(1H,s),7.37-7.43(1H,m),7.47-7.50(1H,m),8.35-8.37(1H,m).

### Reference Example 249

### ethyl 1-(2-chlorophenyl)-5-[(2-methylpyridin-4-yl)thio]-1H-pyrazole-3-carboxylate

To a solution of ethyl 1-(2-chlorophenyl)-5-({3-[(2-ethylhexyl)oxy]-3-oxopropyl}thio)-1H-pyrazole-3-carboxylate (500 mg) in ethanol (5 mL) was added sodium ethoxide (87 mg), and the mixture was stirred at room temperature for 1.5 hr. About half volume of ethanol was evaporated under reduced pressure, and the residue was dissolved in toluene (5 mL). 4-Bromo-2-methylpyridine (276 mg) was added, and the mixture was degassed. Tris(dibenzylideneacetone)dipalladium(0) (49 mg) and 4,5-bis(diphenylphosphino)-9,9-dimethylxanthine (62 mg) were added, and the mixture was further degassed. The mixture was stirred at 110°C for 2 hr under an argon atmosphere, and allowed to cool to room temperature. Water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=4:1→1:1) to give the title compound as a pale-yellow oil (286 mg, yield 71%).
¹H-NMR (CDCl₃) δ: 1.43(3H,t,J=7.2Hz),2.44(3H,s),4.46(2H,q,J=7.2Hz),6.65-6.68(1H,m),6.71-6.72(1H,m),7.23-7.31(3H,m),7.36-7.42(1H,m),7.46-7.49(1H,m),8.23(1H,d,J=5.4Hz).

### Reference Example 250

### ethyl 1-(2-chlorophenyl)-5-[(2-methoxypyridin-4-yl)thio]-1H-pyrazole-3-carboxylate

To a solution of ethyl 1-(2-chlorophenyl)-5-({3-[(2-ethylhexyl)oxy]-3-oxopropyl}thio)-1H-pyrazole-3-carboxylate (500 mg) in ethanol (5 mL) was added sodium ethoxide (87 mg), and the mixture was stirred at room temperature for 1 hr. About half volume of ethanol was evaporated under reduced pressure, and the residue was dissolved in toluene (5 mL). 4-Bromo-2-methoxypyridine (303 mg) was added, and the mixture was degassed. Tris(dibenzylideneacetone)dipalladium(0) (49 mg) and 4,5-bis(diphenylphosphino)-9,9-dimethylxanthine (62 mg) were added, and the mixture was further degassed. The mixture was stirred at 110°C for 1.5 hr under an argon atmosphere, and allowed to cool to room temperature. Water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate= 9:1→4:1) to give the title compound as a pale-yellow oil (325 mg, yield 78%).
¹H-NMR(CDCl₃)δ: 1.43(3H,t,J=7.2Hz),3.87(3H,s),4.46(2H,q,J=7.2Hz),6.27-6.28(1H,m),6.46-6.49(1H,m),7.26-7.30(3H,m),7.37-7.43(1H,m),7.47-7.50(1H,m),7.92(1H,d,J=5.4Hz).

### Reference Example 251

### ethyl 1-(2-chlorophenyl)-5-[(6-methylpyridin-2-yl)thio]-1H-pyrazole-3-carboxylate

To a solution of ethyl 1-(2-chlorophenyl)-5-({3-[(2-ethylhexyl)oxy]-3-oxopropyl}thio)-1H-pyrazole-3-carboxylate (1.09 g) in ethanol (10 mL) was added sodium ethoxide (320 mg) at 0°C, and the mixture was stirred at room temperature for 1 hr, and concentrated under reduced pressure. A mixture of the residue, 2-bromo-6-methylpyridine (456 mg), tris(dibenzylideneacetone)dipalladium(0) (86 mg) and 9,9-dimethyl-4,5-bis(diphenylphosphino)xanthine (108 mg) was stirred in toluene (10 mL) at 80°C for 3 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=4:1→3:1) to give the title compound as a yellow oil (645 mg, yield 74%).
¹H-NMR(CDCl₃) δ: 1.42(3H,t,J=7.2Hz),2.42(3H,s),4.45(2H,q,J=7.2Hz),6.68(1H,d,J=7 .8Hz),6.86(1H,d,J=7.8Hz),7.21-7.26(2H,m),7.32-7.39(3H,m),7.44-7.47 (1H,m).

### Reference Example 252

### ethyl 1-(2-chlorophenyl)-5-[(5-methylpyridin-2-yl)thio]-1H-pyrazole-3-carboxylate

To a solution of ethyl 1-(2-chlorophenyl)-5-({3-[(2-ethylhexyl)oxy]-3-oxopropyl}thio)-1H-pyrazole-3-carboxylate (1.15 g) in ethanol (15 mL) was added sodium ethoxide (337 mg) at 0°C, and the mixture was stirred at room temperature for 1 hr, and concentrated under reduced pressure. A mixture of the residue, 2-bromo-5-methylpyridine (452 mg), tris(dibenzylideneacetone)dipalladium(0) (92 mg) and 9,9-dimethyl-4,5-bis(diphenylphosphino)xanthine (115 mg) was stirred in toluene (10 mL) at 90°C for 6 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=2:1) to give the title compound as a yellow oil (668 mg, yield 73%).
¹H-NMR(CDCl₃)δ: 1.42(3H,t,J=7.2Hz),2.25(3H,s),4.44(2H,q,J=7.2Hz),6.81-6.84(1H,m),7.20-7.39(5H,m),7.40-7.47(1H,m),8.16-8.17(1H,m).

### Reference Example 253

### ethyl 1-(2-chlorophenyl)-5-[(6-methoxypyridin-2-yl)thio]-1H-pyrazole-3-carboxylate

To a solution of ethyl 1-(2-chlorophenyl)-5-({3-[(2-ethylhexyl)oxy]-3-oxopropyl}thio)-1H-pyrazole-3-carboxylate (1.06 g) in ethanol (10 mL) was added sodium ethoxide (314 mg) at 0°C, and the mixture was stirred at room temperature for 1 hr, and concentrated under reduced pressure. A mixture of the residue, 2-bromo-6-methoxypyridine (462 mg), tris(dibenzylideneacetone)dipalladium(0) (83 mg) and 9,9-dimethyl-4,5-bis(diphenylphosphino)xanthine (106 mg) was stirred in toluene (10 mL) at 80°C for 4 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=4:1) to give the title compound as a yellow oil (599 mg, yield 68%).
¹H-NMR(CDCl₃)δ: 1.42(3H,t,J=7.2Hz),3.75(3H,s),4.45(2H,q,J=7.2Hz),6.44-6.50(2H,m),7.22-7.41(5H,m),7.46-7.49(1H,m).

### Reference Example 254

### ethyl 5-[(6-bromopyridin-2-yl)thio]-1-(2-chlorophenyl)-1H-pyrazole-3-carboxylate

To a solution of ethyl 1-(2-chlorophenyl)-5-({3-[(2-ethylhexyl)oxy]-3-oxopropyl}thio)-1H-pyrazole-3-carboxylate (1.38 g) in ethanol (15 mL) was added sodium ethoxide (410 mg) at 0°C, and the mixture was stirred at room temperature for 1 hr, and concentrated under reduced pressure. A mixture of the residue, 2,6-dibromopyridine (738 mg), tris(dibenzylideneacetone)dipalladium(0) (108 mg) and 9,9-dimethyl-4,5-bis(diphenylphosphino)xanthine (137 mg) was stirred in toluene (15 mL) at 80°C for 3 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=3:1) to give the title compound as a yellow oil (538 mg, yield 42%).
¹H-NMR(CDCl₃)δ: 1.43(3H,t,J=7.2Hz),4.45(2H,q,J=7.2Hz),6.84-6.87(1H,m),7.16-7.19(1H,m),7.25-7.48(6H,m).

### Reference Example 255

### ethyl 5-[(6-chloropyridin-3-yl)thio]-1-(2,3-difluorophenyl)-1H-pyrazole-3-carboxylate

To a solution of ethyl 1-(2,3-difluorophenyl)-5-({3-[(2-ethylhexyl)oxy]-3-oxopropyl}thio)-1H-pyrazole-3-carboxylate (800 mg) in ethanol (10 mL) was added sodium ethoxide (139 mg), and the mixture was stirred at room temperature for 1 hr. About half volume of ethanol was evaporated under reduced pressure, and the residue was dissolved in toluene (10 mL). 2-Chloro-5-iodopyridine (450 mg) was added, and the mixture was degassed. Tris(dibenzylideneacetone)dipalladium(0) (78 mg) and 4,5-bis(diphenylphosphino)-9,9-dimethylxanthine (99 mg) were added, and the mixture was further degassed. The mixture was stirred at 110°C for 2 hr under an argon atmosphere, and allowed to cool to room temperature. Water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=19:1→9:1) to give the title compound as a pale-yellow oil (528 mg, yield 78%).
¹H-NMR(CDCl₃)δ: 1.41(3H,t,J=7.2Hz),4,43(2H,q,J=7.2Hz),7.07-7.22(4H,m),7.28-7.39(2H,m),8.07-8.08(1H,m).

### Reference Example 256

### ethyl 5-[(6-chloropyridin-3-yl)thio]-1-(2,4-difluorophenyl)-1H-pyrazole-3-carboxylate

To a solution of ethyl 1-(2,4-difluorophenyl)-5-({3-[(2-ethylhexyl)oxy]-3-oxopropyl}thio)-1H-pyrazole-3-carboxylate (500 mg) in ethanol (10 mL) was added sodium ethoxide (87 mg), and the mixture was stirred at room temperature for 1 hr. About half volume of ethanol was evaporated under reduced pressure, and the residue was dissolved in toluene (10 mL). 2-Chloro-5-iodopyridine (279 mg) was added, and the mixture was degassed. Tris(dibenzylideneacetone)dipalladium(0) (49 mg) and 4,5-bis(diphenylphosphino)-9,9-dimethylxanthine (61 mg) were added, and the mixture was further degassed. The mixture was stirred at 110°C for 1.5 hr under an argon atmosphere, and allowed to cool to room temperature. Water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=9:1→4:1) to give the title compound as a pale-yellow oil (348 mg, yield 83%).
¹H-NMR(CDCl₃)δ: 1.41(3H,t,J=7.2Hz),4.43(2H,q,J=7.2Hz),6.92-6.99(2H,m),7.18-7.22(2H,m),7.25-7.38(2H,m),8.06-8.07(1H,m).

### Reference Example 257

### ethyl 5-[(3-bromophenyl)thio]-1-(2,5-difluorophenyl)-1H-pyrazole-3-carboxylate

To a solution of ethyl 1-(2,5-difluorophenyl)-5-({3-[(2-ethylhexyl)oxy]-3-oxopropyl}thio)-1H-pyrazole-3-carboxylate (1.95 g) in ethanol (20 mL) was added sodium ethoxide (569 mg) at 0°C, and the mixture was stirred at room temperature for 1 hr, and concentrated under reduced pressure. A mixture of the residue, 1-bromo-3-iodobenzene (1.20 g), tris(dibenzylideneacetone)dipalladium(0) (38 mg) and 9,9-dimethyl-4,5-bis(diphenylphosphino)xanthine (49 mg) was stirred in toluene (20 mL) at 80°C for 2 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=6:1) to give the title compound as a yellow oil (2.04 g, yield quantitative).
¹H-NMR(CDCl₃)δ: 1.41(3H,t,J=7.2Hz),4.44(2H,q,J=7.2Hz),6.98-7.19(7H,m),7.32-7.35(1H,m).

### Reference Example 258

### ethyl 5-[(6-chloropyridin-3-yl)thio]-1-(2,5-difluorophenyl)-1H-pyrazole-3-carboxylate

To a solution of ethyl 1-(2,5-difluorophenyl)-5-({3-[(2-ethylhexyl)oxy]-3-oxopropyl}thio)-1H-pyrazole-3-carboxylate (820 mg) in ethanol (10 mL) was added sodium ethoxide (143 mg), and the mixture was stirred at room temperature for 1 hr. About half volume of ethanol was evaporated under reduced pressure, and the residue was dissolved in toluene (10 mL). 2-Chloro-5-iodopyridine (461 mg) was added, and the mixture was degassed. Tris(dibenzylideneacetone)dipalladium(0) (80 mg) and 4,5-bis(diphenylphosphino)-9,9-dimethylxanthine (101 mg) were added, and the mixture was further degassed. The mixture was stirred at 90°C for 18 hr under an argon atmosphere, and allowed to cool to room temperature. Water was added, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=9:1→4:1) to give the title compound as a pale-yellow oil (471 mg, yield 68%).
¹H-NMR(CDCl₃)δ: 1.41(3H,t,J=7.2Hz),4.43(2H,q,J=7.2Hz),7.08-7.25(5H,m),7.36-7.40(1H,m),8.10-8.11(1H,m).

### Reference Example 259

### ethyl 5-[(6-chloropyridin-3-yl)thio]-1-(2-fluoro-3-methylphenyl)-1H-pyrazole-3-carboxylate

To a solution of ethyl 5-({3-[(2-ethylhexyl)oxy]-3-oxopropyl}thio)-1-(2-fluoro-3-methylphenyl)-1H-pyrazole-3-carboxylate (1.5 g) in ethanol (15 mL) was added sodium ethoxide (264 mg), and the mixture was stirred at room temperature for 1 hr. About half volume of ethanol was evaporated under reduced pressure, and the residue was dissolved in toluene (15 mL). 2-Chloro-5-iodopyridine (851 mg) was added, and the mixture was degassed. Tris(dibenzylideneacetone)dipalladium(0) (148 mg) and 4,5-bis(diphenylphosphino)-9,9-dimethylxanthine (187 mg) were added, and the mixture was further degassed. The mixture was stirred at 110°C for 1 hr under an argon atmosphere, and allowed to cool to room temperature. Water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=19:1→9:1) to give the title compound as a yellow oil (988 mg, yield 78%).
¹H-NMR(CDCl₃)δ: 1.40(3H,t,J=7.2Hz),2.28(3H,d,J=1.8Hz),4.43(2H,q,J=7.2Hz),7.06-7.19(4H,m),7.27-7.37(2H,m),8.02-8.03(1H,m).

### Reference Example 260

### ethyl 5-[(6-chloropyridin-3-yl)thio]-1-(2-fluoro-4-methylphenyl)-1H-pyrazole-3-carboxylate

To a solution of ethyl 5-({3-[(2-ethylhexyl)oxy]-3-oxopropyl}thio)-1-(2-fluoro-4-methylphenyl)-1H-pyrazole-3-carboxylate (1.3 g) in ethanol (15 mL) was added sodium ethoxide (225 mg), and the mixture was stirred at room temperature for 2 hr. About half volume of ethanol was evaporated under reduced pressure, and the residue was dissolved in toluene (15 mL). 2-Chloro-5-iodopyridine (727 mg) was added, and the mixture was degassed. Tris(dibenzylideneacetone)dipalladium(0) (126 mg) and 4,5-bis(diphenylphosphino)-9,9-dimethylxanthine (160 mg) were added, and the mixture was further degassed. The mixture was stirred at 110°C for 1 hr under an argon atmosphere, and allowed to cool to room temperature. Water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=9:1→4:1) to give the title compound as a pale-yellow oil (968 mg, yield 90%).
¹H-NMR (CDCl₃) δ: 1.40(3H,t,J=7.2Hz),2.42(3H,s),4.42(2H,q,J=7.2Hz),6.98-7.01(2H,m),7.14-7.19(3H,m),7.33-7.37(1H,m),8,04-8.05(1H,m).

### Reference Example 261

### ethyl 5-[(6-chloropyridin-3-yl)thio]-1-(2-fluoro-5-methylphenyl)-1H-pyrazole-3-carboxylate

To a solution of ethyl 5-({3-[(2-ethylhexyl)oxy]-3-oxopropyl}thio)-1-(2-fluoro-5-methylphenyl)-1H-pyrazole-3-carboxylate (1.7 g) in ethanol (15 mL) was added sodium ethoxide (290 mg), and the mixture was stirred at room temperature for 1 hr. About half volume of ethanol was evaporated under reduced pressure, and the residue was dissolved in toluene (15 mL). 2-Chloro-5-iodopyridine (935 mg) was added, and the mixture was degassed. Tris(dibenzylideneacetone)dipalladium(0) (163 mg) and 4,5-bis(diphenylphosphino)-9,9-dimethylxanthine (205 mg) were added, and the mixture was further degassed. The mixture was stirred at 110°C for 4 hr under an argon atmosphere, and allowed to cool to room temperature. Water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=19:1→7:1) to give the title compound as a pale-yellow oil (1.1 g, yield 75%).
¹H-NMR(CDCl₃)δ: 1.43(3H,t,J=7.2Hz),2.31(3H,s),4.43(2H,q,J=7.2Hz),7.03-7.09(2H,m),7.15-7.26(3H,m),7.34-7.46(1H,m),8.04-8.05(1H,m).

### Reference Example 262

### ethyl 5-[(6-chloropyridin-3-yl)thio]-1-(3-fluoro-2-methylphenyl)-1H-pyrazole-3-carboxylate

To a solution of ethyl 5-({3-[(2-ethylhexyl)oxy]-3-oxopropyl}thio)-1-(3-fluoro-2-methylphenyl)-1H-pyrazole-3-carboxylate (3.91 g) in ethanol (25 mL) was added sodium ethoxide (857 mg), and the mixture was stirred at room temperature for 24 hr. About half volume of ethanol was evaporated under reduced pressure, and the residue was dissolved in toluene (30 mL). 2-Chloro-5-iodopyridine (2.22 g) was added, and the mixture was degassed. Tris(dibenzylideneacetone)dipalladium(0) (77 mg) and 4,5-bis(diphenylphosphino)-9,9-dimethylxanthine (97 mg) were added, and the mixture was further degassed. The mixture was stirred at 110°C for 2.5 hr under an argon atmosphere, allowed to cool to room temperature, and filtered through silica gel, and the filtrate was concentrated under reduced pressure. The residue was purified by silica gel column chromatography (eluent: hexane-ethyl acetate=19:1→4:1) to give the title compound as a pale-yellow oil (2.57 g, yield 78%).
¹H-NMR(CDCl₃)δ: 1.41(3H,t,J=7.1Hz),1.86(3H,d,J=2.3Hz),4.43(2H,q,J=7.2Hz),6.79-6.96(1H,m),7.07-7.25(4H,m),7.36(1H,dd,J=8.3,2.4Hz),8.05(1H,d,J=2.3Hz).

### Reference Example 263

### ethyl 5-[(6-chloropyridin-3-yl)thio]-1-(5-fluoro-2-methylphenyl)-1H-pyrazole-3-carboxylate

To a solution of ethyl 5-({3-[(2-ethylhexyl)oxy]-3-oxopropyl}thio)-1-(5-fluoro-2-methylphenyl)-1H-pyrazole-3-carboxylate (4.0 g) in ethanol (40 mL) was added sodium ethoxide (879 mg), and the mixture was stirred at room temperature for 2 hr. About half volume of ethanol was evaporated under reduced pressure, and the residue was dissolved in toluene (10 mL). 2-Chloro-5-iodopyridine (2.3 g) was added, and the mixture was degassed. Tris(dibenzylideneacetone)dipalladium(0) (79 mg) and 4,5-bis(diphenylphosphino)-9,9-dimethylxanthine (100 mg) were added, and the mixture was further degassed. The mixture was stirred at 110°C for 3 hr under an argon atmosphere, and allowed to cool to room temperature. Water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=9:1→4:1) to give the title compound as a pale-yellow oil (2.9 g, yield 86%).
¹H-NMR(CDCl₃) δ: 1.40(3H,t,J=7.2Hz),1.89(3H,s),4.43(2H,q,J=7.2Hz),6.81-6.85(1H,m),7.08-7.14(2H,m),7.18-7.27(2H,m),7.36-7.39(1H,m),8.06-8.07(1H,m).

### Reference Example 264

### ethyl 1-(2-chloro-3-fluorophenyl)-5-[(pyridin-3-yl)thio]-1H-pyrazole-3-carboxylate

To a solution of ethyl 1-(2-chloro-3-fluorophenyl)-5-({3-[(2-ethylhexyl)oxy]-3-oxopropyl}thio)-1H-pyrazole-3-carboxylate (2.0 g) in ethanol (20 mL) was added sodium ethoxide (413 mg), and the mixture was stirred at room temperature for 2 hr. About half volume of ethanol was evaporated under reduced pressure, and the residue was dissolved in toluene (20 mL). 3-Iodopyridine (911 mg) was added, and the mixture was degassed. Tris(dibenzylideneacetone)dipalladium(0) (37 mg) and 4,5-bis(diphenylphosphino)-9,9-dimethylxanthine (47 mg) were added, and the mixture was further degassed. The mixture was stirred at 110°C for 2 hr under an argon atmosphere, and allowed to cool to room temperature. Water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=4:1→2:1) to give the title compound as a yellow oil (1.3 g, yield 87%).
¹H-NMR(CDCl₃)δ: 1.41(3H,t,J=7.2Hz),4.43(2H,q,J=7.2Hz),7.05-7.10(1H,m),7.14-7.19(2H,m),7.24-7.33(2H,m),7.42-7.45(1H,m),8.30-8.31(1H,m),8.44-8.46(1H,m),

### Reference Example 265

### ethyl 1-(2-chloro-3-fluorophenyl)-5-[(6-chloropyridin-3-yl)thio]-1H-pyrazole-3-carboxylate

To a solution of ethyl 1-(2-chloro-3-fluorophenyl)-5-({3-[(2-ethylhexyl)oxy]-3-oxopropyl}thio)-1H-pyrazole-3-carboxylate (1.9 g) in ethanol (20 mL) was added sodium ethoxide (400 mg), and the mixture was stirred at room temperature for 1 hr. About half volume of ethanol was evaporated under reduced pressure, and the residue was dissolved in toluene (10 mL). 2-Chloro-5-iodopyridine (1.0 g) was added, and the mixture was degassed. Tris(dibenzylideneacetone)dipalladium(0) (36 mg) and 4,5-bis(diphenylphosphino)-9,9-dimethylxanthine (45 mg) were added, and the mixture was further degassed. The mixture was stirred at 110°C for 2 hr under an argon atmosphere, and allowed to cool to room temperature. Water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=9:1→4:1) to give the title compound as a pale-yellow oil (1.5 g, yield 91%).
¹H-NMR(CDCl₃) δ: 1.41(3H,t,J=7.2Hz),4.43(2H,q,J=7.2Hz),7.04-7.11(1H,m),7.18-7.21(2H,m),7.27-7.41(3H,m),8.05-8.06(1H,m).

### Reference Example 266

### ethyl 1-(2-chloro-5-fluorophenyl)-5-[(6-chloropyridin-3-yl)thio]-1H-pyrazole-3-carboxylate

To a solution of ethyl 1-(2-chloro-5-fluorophenyl)-5-({3-[(2-ethylhexyl)oxy]-3-oxopropyl}thio)-1H-pyrazole-3-carboxylate (500 mg) in ethanol (5 mL) was added sodium ethoxide (84.1 mg), and the mixture was stirred at room temperature for 1 hr. About half volume of ethanol was evaporated under reduced pressure, and the residue was dissolved in toluene (5 mL). 2-Chloro-5-iodopyridine (271 mg) was added, and the mixture was degassed. Tris(dibenzylideneacetone)dipalladium(0) (47 mg) and 4,5-bis(diphenylphosphino)-9,9-dimethylxanthine (60 mg) were added, and the mixture was further degassed. The mixture was stirred at 110°C for 1.5 hr under an argon atmosphere, and allowed to cool to room temperature. Water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=19:1→9:1) to give the title compound as a pale-yellow oil (327 mg, yield 77%).
¹H-NMR(CDCl₃)δ: 1.41(3H,t,J=7.2Hz),4.43(2H,q,J=7.2Hz),7.03-7.07(1H,m),7.16-7.23(3H,m),7.39-7.49(2H,m),8.08-8.09(1H,m).

### Reference Example 267

### ethyl 1-(2-fluoropyridin-3-yl)-5-(phenylthio)-1H-pyrazole-3-carboxylate

A solution of ethyl 1-(2-fluoropyridin-3-yl)-5-{[(trifluoromethyl)sulfonyl]oxy}-1H-pyrazole-3-carboxylate (2.4 g), thiophenol (1.0 g) and cesium carbonate (4.1 g) in toluene (30 mL) was degassed, tris(dibenzylideneacetone)dipalladium(0) (288 mg) and 4,5-bis(diphenylphosphino)-9,9-dimethylxanthine (364 mg) were added, and the mixture was further degassed. The mixture was stirred at 110°C for 6 hr under an argon atmosphere, and allowed to cool to room temperature. Water and ethyl acetate were added, and the mixture was filtered through celite. The organic layer of the filtrate was washed with saturated brine, dried over anhydrous sodium sulfate, and concentrated under reduced pressure. The residue was purified by silica gel column chromatography (eluent: hexane-ethyl acetate=6:1→3:1) to give the crude title compound as a yellow oil (1.32 g).
¹H-NMR(CDCl₃)δ: 1.41(3H,t,J=7.2Hz),4.43(2H,q,J=7.2Hz),7.04-7.10(2H,m),7.15(1H,s),7.18-7.28(4H,m),7.66-7.72(1H,m),8.27-8.29(1H,m).

### Reference Example 268

### ethyl 1-(2-fluorophenyl)-5-(phenylsulfonyl)-1H-pyrazole-3-carboxylate

To a solution of ethyl 1-(2-fluorophenyl)-5-(phenylthio)-1H-pyrazole-3-carboxylate (75 mg) in ethyl acetate (3 mL) was added 3-chloroperbenzoic acid (151 mg), and the mixture was stirred for 18 hr. The reaction mixture was treated with aqueous sodium thiosulfate solution, and extracted with ethyl acetate. The extract was washed successively with water, saturated aqueous sodium hydrogen carbonate solution and saturated brine, dried over anhydrous magnesium sulfate, and concentrated under reduced pressure. The residue was purified by silica gel column chromatography (eluent: hexane-ethyl acetate=9:1→9:11) to give the title compound as a white powder (46 mg, yield 56%).
¹H-NMR(CDCl₃)δ: 1.40(3H,t,J=7.2Hz),4.43(2H,q,J=7.2Hz),7.01-7.10(1H,m),7.18-7.26(1H,m),7.29-7.45(3H,m),7.46-7.56(3H,m),7.56-7.64(2H,m).

### ethyl Reference Example 269

### 1-(2-fluorophenyl)-5-[(3-methoxyphenyl)sulfonyl]-1H-pyrazole-3-carboxylate

To a solution of ethyl 1-(2-fluorophenyl)-5-[(3-methoxyphenyl)thio]-1H-pyrazole-3-carboxylate (154 mg (including impurity)) in ethyl acetate (4 mL) was added 3-chloroperbenzoic acid (285 mg), and the mixture was stirred for 18 hr. The reaction mixture was treated with aqueous sodium thiosulfate solution, and extracted with ethyl acetate. The extract was washed successively with water, saturated aqueous sodium hydrogen carbonate solution and saturated brine, dried over anhydrous magnesium sulfate, and concentrated under reduced pressure. The residue was purified by silica gel column chromatography (eluent: hexane-ethyl acetate=3:1→1:1) to give the title compound as a colorless oil (145 mg, yield 87%).
¹H-NMR (CDCl₃) δ: 1.40(3H,t,J=7.0Hz),3.74(3H,s),4.43(2H,q,J=7.2Hz),6.95-6.99(1H,m),7.02-7.15(3H,m),7.19-7.37(3H,m),7.46-7.56(1H,m),7.62(1H,s).

### Reference Example 270

### ethyl 5-[(3-methoxyphenyl)sulfonyl]-1-(2-methylphenyl)-1H-pyrazole-3-carboxylate

To a solution of ethyl 5-[(3-methoxyphenyl)thio]-1-(2-methylphenyl)-1H-pyrazole-3-carboxylate (1.3 g) in ethyl acetate (35 mL) was added 3-chloroperbenzoic acid (2.6 g) at room temperature, and the mixture was stirred at the same temperature for 18 hr. The reaction mixture was treated with saturated aqueous sodium thiosulfate solution, and extracted with ethyl acetate. The extract was dried over anhydrous sodium sulfate, and concentrated under reduced pressure. The residue was purified by silica gel column chromatography (eluent: hexane-ethyl acetate=15:1) to give the title compound as a white solid (1.1 g, yield 75%).
¹H-NMR(CDCl₃)δ: 1.41(3H,t,J=7.0Hz),1.61(3H,s),3.71(3H,s),4.44(2H,q,J=7.0Hz),6. 84(1H,t,J=2.2Hz),7.05-7.11(3H,m),7.16-7.23(2H,m),7.28(1H,t,J=7.4Hz),7.41(1H,td,J=7.5,1.2Hz),7.66(1H, s).

### Reference Example 271

### ethyl 1-(2,6-difluorophenyl)-5-(phenylsulfonyl)-1H-pyrazole-3-carboxylate

To a solution of ethyl 1-(2,6-difluorophenyl)-5-(phenylthio)-1H-pyrazole-3-carboxylate (1.14 g) in ethyl acetate (31.6 mL) was added 3-chloroperbenzoic acid (2.34 g) at room temperature, and the mixture was stirred at the same temperature for 18 hr. The reaction mixture was treated with saturated aqueous sodium thiosulfate solution, and extracted with ethyl acetate. The extract was dried over anhydrous sodium sulfate, and concentrated under reduced pressure. The residue was purified by silica gel column chromatography (eluent: hexane-ethyl acetate=15:1) to give the title compound as a white solid (0.87 g, yield 70%).
¹H-NMR (CDCl₃) δ: 1.40(3H,t,J=7.0Hz),4.43(2H,q,J=7.0Hz),6.99(2H,dd,J=8.4,7.2Hz), 7.44-7.49(2H,m),7.50-7.56(1H,m),7.59-7.66(4H,m).

### Reference Example 272

### ethyl 1-(2-fluoropyridin-3-yl)-5-(phenylsulfonyl)-1H-pyrazole-3-carboxylate

To a solution of ethyl 1-(2-fluoropyridin-3-yl)-5-(phenylthio)-1H-pyrazole-3-carboxylate (1.3 g) in ethyl acetate (15 mL) was added 3-chloroperbenzoic acid (3.7 g), and the mixture was stirred at room temperature for 3 hr. The reaction mixture was treated with saturated aqueous sodium thiosulfate solution, and extracted with ethyl acetate. The extract was washed successively with water, saturated aqueous sodium hydrogen carbonate solution and saturated brine, dried over anhydrous sodium sulfate, and concentrated under reduced pressure. The residue was purified by basic silica gel column chromatography (eluent: hexane-ethyl acetate=7:1→3:1) to give the title compound as a colorless oil (988 mg, 2 step yield 42%).
¹H-NMR(CDCl₃)δ: 1.40(3H,t,J=7.2Hz),4.44(2H,q,J=7.2Hz) ,7.34-7.39(1H,m),7.43-7.49(2H,m),7.54-7.57(2H,m),7.60(1H,s),7.61-7.67(1H,m),7.87-7.93(1H,m),8.36-8.39(1H,m)

### Reference Example 273

### 5-[(6-chloropyridin-3-yl)thio]-1-(2-fluorophenyl)-N-methyl-1H-pyrazole-3-carboxamide

To a solution of ethyl 5-[(6-chloropyridin-3-yl)thio]-1-(2-fluorophenyl)-1H-pyrazole-3-carboxylate (1.73 g (including impurity)) in methanol (20 mL) was added 40% methylamine-methanol solution (20 mL) under ice-cooling. The mixture was stirred at room temperature for 1 hr, and concentrated under reduced pressure. The residue was purified by silica gel column chromatography (eluent: hexane-ethyl acetate=9:1→methyl acetate) to give the title compound as a pale-yellow solid (1.56 g, yield 94%).
¹H-NMR(CDCl₃)δ: 2.98(3H,d,J=4.9Hz),6.79-6.90(1H,m),7.13-7.32(5H,m),7.37(1H,dd,J=8.3,2.7Hz),7.45-7.55(1H,m),8.02(1H,d,J=1.9Hz).

### Reference Example 274

### 5-[(6-chloropyridin-3-yl)thio]-N-methyl-1-(2-methylphenyl)-1H-pyrazole-3-carboxamide

To a solution of ethyl 5-[(6-chloropyridin-3-yl)thio]-1-(2-methylphenyl)-1H-pyrazole-3-carboxylate (471 mg) in methanol (6 mL) was added 40% methylamine-methanol solution (1.2 mL) at 0°C. The reaction mixture was stirred at room temperature for 14 hr, and concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was washed with diisopropyl ether to give the title compound as a white solid (272 mg, yield 82%).
¹H-NMR(CDCl₃) δ: 1.91(3H,s),2.97(3H,d,J=5.1Hz),6.85(1H,brs),7.01-7.04(2H,m),7.14-7.17(2H,m),7.21-7.42(4H,m),7.97-7.98(1H,m).

### Reference Example 275

### 5-[(3-bromophenyl)thio]-1-(2-chlorophenyl)-N-methyl-1H-pyrazole-3-carboxamide

To a solution of ethyl 5-[(3-bromophenyl)thio]-1-(2-chlorophenyl)-1H-pyrazole-3-carboxylate (687 mg) in methanol (8 mL) was added 40% methylamine-methanol solution (1.6 mL) at 0°C. The reaction mixture was stirred at room temperature for 3 hr, and concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure to give the title compound as a yellow oil (600 mg, yield 90%).
¹H-NMR(CDCl₃)δ: 2.97(3H,d,J=5.1Hz),6.86(1H,brs),6.98-7.33(7H,m),7.38-7.51(2H,m).

### Reference Example 276

### 1-(2-chlorophenyl)-5-[(6-chloropyridin-3-yl)thiol-N-methyl-1H-pyrazole-3-carboxamide

To a solution of ethyl 1-(2-chlorophenyl)-5-[(6-chloropyridin-3-yl)thiol-1H-pyrazole-3-carboxylate (2.06 g) in methanol (4 mL) was added 40% methylamine-methanol solution (6 mL) at room temperature. The mixture was stirred for 2 hr, and concentrated under reduced pressure, and the residue was purified by silica gel column chromatography (eluent: hexane-ethyl acetate=9:1→4:1) to give the title compound as a pale-yellow amorphous solid (1.75 g, yield 88%).
¹H-NMR(CDCl₃)δ: 2.97(3H,d,J=5.1Hz),6.85(1H,br),7.16-7.26(2H,m),7.33-7.53(5H,m),7.99-8.01(1H,m).

### Reference Example 277

### 5-[(6-bromopyridin-2-yl)thio]-1-(2-chlorophenyl)-N-methyl-1H-pyrazole-3-carboxamide

To a solution of ethyl 5-[(6-bromopyridin-2-yl)thio]-1-(2-chlorophenyl)-1H-pyrazole-3-carboxylate (1.38 g) in methanol (6 mL) was added 40% methylamine-methanol solution (1.5 mL) at 0°C. The mixture was stirred at room temperature for 3 hr, and concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure to give the title compound as a yellow oil (466 mg, yield 93%).
¹H-NMR(CDCl₃)δ: 3.00(3H,d,J=5.4Hz),6.85-6.90(2H,m),7.16-7.18(1H,m),7.28-7.34(3H,m),7.38-7.43(2H,m),7.47-7.51(1H,m).

### Reference Example 278

### 5-[(6-chloropyridin-3-yl)thio]-1-(2,3-difluorophenyl)-N-methyl-1H-pyrazole-3-carboxamide

To a solution of ethyl 5-[(6-chloropyridin-3-yl)thio]-1-(2,3-difluorophenyl)-1H-pyrazole-3-carboxylate (528 mg) in methanol (5 mL) was added 40% methylamine-methanol solution (5 mL) at room temperature. The mixture was stirred for 1 hr, and concentrated under reduced pressure, and the residue was purified by silica gel column chromatography (eluent: hexane-ethyl acetate=3:1→1:2) to give the title compound as a pale-yellow solid (478 mg, yield 94%).
¹H-NMR(CDCl₃)δ: 2.98(3H,d,J=4.8Hz),6.83(1H,br),7.04-7.10(1H,m),7.15-7.23(3H,m),7.30-7.40(2H,m),8.04-8.06(1H,m).

### Reference Example 279

### 5-[(6-chloropyridin-3-yl)thio]-1-(2,4-difluorophenyl)-N-methyl-1H-pyrazole-3-carboxamide

To a solution of ethyl 5-[(6-chloropyridin-3-yl)thio]-1-(2,4-difluorophenyl)-1H-pyrazole-3-carboxylate (348 mg) in methanol (5 mL) was added 40% methylamine-methanol solution (5 mL) at room temperature. The mixture was stirred for 1 hr, and concentrated under reduced pressure, and the residue was purified by silica gel column chromatography (eluent: hexane-ethyl acetate=3:1→1:1) to give the title compound as a pale-yellow solid (312 mg, yield 93%).
¹H-NMR(CDCl₃)δ: 2.98(3H,d,J=5.4Hz),6.82(1H,br),6.94-7.01(2H,m),7.18-7.21(2H,m),7.24-7.32(1H,m),7.35-7.39(1H,m),8.04-8.06(1H,m).

### Reference Example 280

### 5-[(3-bromophenyl)thio]-1-(2,5-difluorophenyl)-N-methyl-1H-pyrazole-3-carboxamide

To a solution of ethyl 5-[(3-bromophenyl)thio]-1-(2,5-difluorophenyl)-1H-pyrazole-3-carboxylate (2.04 g) in methanol (2 mL) was added 40% methylamine-methanol solution (4 mL) at 0°C. The mixture was stirred at room temperature for 3 hr, and concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=2:1) to give the title compound as a yellow oil (1.35 g, yield 76%).
¹H-NMR(CDCl₃)δ: 2.99(3H,d,J=5.1Hz),6.86(1H,brs),6.98-7.10(3H,m),7.14-7.20(4H,m),7.30-7.34(1H,m).

### Reference Example 281

### 5-[(6-chloropyridin-3-yl)thio]-1-(2,5-difluorophenyl)-N-methyl-1H-pyrazole-3-carboxamide

To a solution of ethyl 5-[(6-chloropyridin-3-yl)thio]-1-(2,5-difluorophenyl)-1H-pyrazole-3-carboxylate (471 mg) in methanol (3 mL) was added 40% methylamine-methanol solution (5 mL) at room temperature. The mixture was stirred for 1 hr, and concentrated under reduced pressure, and the residue was purified by silica gel column chromatography (eluent: hexane-ethyl acetate=2:1→1:1) to give the title compound as colorless crystals (457 mg, yield quantitative).
¹H-NMR(CDCl₃)δ: 2.98(3H,d,J=4.8Hz), 6.83(1H,br), 7.04-7.10(1H,m),7.17-7.27(4H,m),7.36-7.40(1H,m),8.07-8.08(1H,m).

### Reference Example 282

### 5-[(6-chloropyridin-3-yl)thio]-1-(2-fluoro-3-methylphenyl)-N-methyl-1H-pyrazole-3-carboxamide

To a solution of ethyl 5-[(6-chloropyridin-3-yl)thio]-1-(2-fluoro-3-methylphenyl)-1H-pyrazole-3-carboxylate (988 mg) in methanol (5 mL) was added 40% methylamine-methanol solution (5 mL) at room temperature. The mixture was stirred for 2 hr, and concentrated under reduced pressure, and the residue was purified by silica gel column chromatography (eluent: hexane-ethyl acetate=4:1→1:1) to give the title compound as colorless crystals (862 mg, yield 90%).
¹H-NMR(CDCl₃)δ: 2.29-2.30(3H,m),2.97(3H,d,J=5.1Hz),6.84(1H,br),7.06-7.19(4H,m),7.29-7.38(2H,m),7.99-8.00(1H,m).

### Reference Example 283

### 5-[(6-chloropyridin-3-yl)thio]-1-(2-fluoro-4-methylphenyl)-N-methyl-1H-pyrazole-3-carboxamide

To a solution of ethyl 5-[(6-chloropyridin-3-yl)thio]-1-(2-fluoro-4-methylphenyl)-1H-pyrazole-3-carboxylate (968 mg) in methanol (5 mL) was added 40% methylamine-methanol solution (5 mL) at room temperature. The mixture was stirred for 1 hr, and concentrated under reduced pressure, and the residue was purified by silica gel column chromatography (eluent: hexane-ethyl acetate=4:1→1:1) to give the title compound as colorless crystals (843 mg, yield 91%).
¹H-NMR(CDCl₃)δ: 2.43(3H,s),2.97(3H,d,J=5.1Hz),6.83(1H,br),7.00-7.01(1H,m),7.04(1H,s),7.11-7.19(3H,m),7.34-7.38(2H,m),8.03-8.04(1H,m)

### Reference Example 284

### 5-[(6-chloropyridin-3-yl)thio]-1-(2-fluoro-5-methylphenyl)-N-methyl-1H-pyrazole-3-carboxamide

To a solution of ethyl 5-[(6-chloropyridin-3-yl)thio]-1-(2-fluoro-5-methylphenyl)-1H-pyrazole-3-carboxylate (1.1 g) in methanol (5 mL) was added 40% methylamine-methanol solution (5 mL) at room temperature. The mixture was stirred for 1 hr, and concentrated under reduced pressure, and the residue was purified by silica gel column chromatography (eluent: hexane-ethyl acetate=3:1→1:2) to give the title compound as a colorless amorphous solid (956 mg, yield 93%).
¹H-NMR(CDCl₃) δ: 2.34(3H,s),2.97(3H,d,J=5.1Hz),6.83(1H,br),7.01-7.11(2H,m),7.16-7.19(2H,m),7.24-7.28(1H,m),7.35-7.39(1H,m),8.02-8.03(1H,m).

### Reference Example 285

### 5-[(6-chloropyridin-3-yl)thio]-1-(3-fluoro-2-methylphenyl)-N-methyl-1H-pyrazole-3-carboxamide

To a solution of ethyl 5-[(6-chloropyridin-3-yl)thio]-1-(3-fluoro-2-methylphenyl)-1H-pyrazole-3-carboxylate (2.9 g) in methanol (10 mL) was added 40% methylamine-methanol solution (10 mL) at room temperature. The mixture was stirred for 2 hr, and concentrated under reduced pressure, and the residue was purified by silica gel column chromatography (eluent: hexane-ethyl acetate=2:1→1:2) to give the title compound as colorless crystals (2.7 g, yield 95%).
¹H-NMR(CDCl₃)δ: 1.87(3H,s),2.97(3H,d,J=5.1Hz),6.81-6.85(2H,m),7.10-7.29(4H,m),7.37-7.41(1H,m),8.03-8.04(1H,m).

### Reference Example 286

### 5-[(6-chloropyridin-3-yl)thio]-1-(5-fluoro-2-methylphenyl)-N-methyl-1H-pyrazole-3-carboxamide

To a solution of ethyl 5-[(6-chloropyridin-3-yl)thio]-1-(5-fluoro-2-methylphenyl)-1H-pyrazole-3-carboxylate (2.57 g) in methanol (3 mL) was added 40% methylamine-methanol solution (15 mL) at room temperature. The mixture was stirred for 1.5 hr, and concentrated under reduced pressure, and the residue was azeotroped with toluene to give the title compound as a pale-yellow oil (2.47 g, yield quantitative).
¹H-NMR(CDCl₃)δ: 1.85(3H,d,J=2.3Hz),2.98(3H,d,J=5.1Hz),6.77-6.90(2H,m),7.14-7.25(4H,m),7.38(1H,dd,J=8.3,2.6Hz),8.03(1H,d,J=1.9Hz).

### Reference Example 287

### 1-(2-chloro-3-fluorophenyl)-5-[(6-chloropyridin-3-yl)thio]-N-methyl-1H-pyrazole-3-carboxamide

To a solution of ethyl 1-(2-chloro-3-fluorophenyl)-5-[(6-chloropyridin-3-yl)thio]-1H-pyrazole-3-carboxylate (1.5 g) in methanol (5 mL) was added 40% methylamine-methanol solution (5 mL) at room temperature. The mixture was stirred for 2 hr, and concentrated under reduced pressure, and the residue was washed with diisopropyl ether to give the title compound as colorless crystals (1.2 g, yield 82%).
¹H-NMR(CDCl₃)δ: 2.98(3H,d,J=5.1Hz),6.83(1H,br),7.04-7.09(1H,m),7.18-7.21(2H,m),7.32-7.42(3H,m),8.03-8.04(1H,m).

### Reference Example 288

### 1-(2-chloro-5-fluorophenyl)-5-[(6-chloropyridin-3-yl)thio]-N-methyl-1H-pyrazole-3-carboxamide

To a solution of ethyl 1-(2-chloro-5-fluorophenyl)-5-[(6-chloropyridin-3-yl)thio]-1H-pyrazole-3-carboxylate (327 mg) in methanol (3 mL) was added 40% methylamine-methanol solution (3 mL) at room temperature. The mixture was stirred for 2 hr, and concentrated under reduced pressure, and the residue was purified by silica gel column chromatography (eluent: hexane-ethyl acetate=3:1→1:1) to give the title compound as a pale-yellow solid (343 g, yield quantitative).
¹H-NMR(CDCl₃)δ; 2.97(3H,d,J=5.4Hz),6.82(1H,br),7.01-7.05(1H,m),7.17-7.25(3H,m),7.39-7.50(2H,m),8.05-8.06(1H,m).

### Reference Example 289

### {1-(2-chlorophenyl)-5-[(5-fluoropyridin-3-yl)thio]-1H-pyrazol-3-yl}methanol

A solution of ethyl 1-(2-chlorophenyl)-5-[(5-fluoropyridin-3-yl)thio]-1H-pyrazole-3-carboxylate (290 mg) in tetrahydrofuran (5 mL) was cooled to -78°C, and 1.5 mol/L diisobutylaluminum hydride-toluene solution (2 mL) was added dropwise. The reaction mixture was stirred for 2 hr under ice-cooling, sodium sulfate 10 hydrate was added, and the mixture was further stirred at room temperature for 1 hr. The reaction mixture was filtered through celite, and the filtrate was concentrated under reduced pressure. The residue was purified by silica gel column chromatography (eluent: hexane-ethyl acetate=4:1→1:1) to give the title compound as a colorless oil (223 mg, yield 87%).
¹H-NMR(CDCl₃)δ: 2.14(1H,br),4.78(2H,d,J=5.7Hz),6.75(1H,s),7.06-7.11(1H,m),7.21-7.33(2H,m),7.37-7.49(2H,m),8.06-8.07(1H,m),8.24-8.25(1H,m).

### Reference Example 290

### 1-(2-chlorophenyl)-5-[(pyridin-3-yl)thio]-1H-pyrazole-3-carbaldehyde

A solution of ethyl 1-(2-chlorophenyl)-5-[(pyridin-3-yl)thio]-1H-pyrazole-3-carboxylate (282 mg) in tetrahydrofuran (5 mL) was cooled to -78°C, and 1.5 mol/L diisobutylaluminum hydride-toluene solution (2.1 mL) was added dropwise. The reaction mixture was stirred for 1 hr under ice-cooling, sodium sulfate 10 hydrate was added, and the mixture was further stirred at room temperature for 30 min. The reaction mixture was filtered through celite, and the filtrate was concentrated under reduced pressure. The obtained residue was dissolved in toluene (5 mL), manganese dioxide (682 mg) was added, and the mixture was stirred at 110°C for 4 hr. The reaction mixture was allowed to cool to room temperature, and filtered through celite. The filtrate was concentrated under reduced pressure, and the residue was purified by silica gel column chromatography (eluent: hexane-ethyl acetate=4:1→1:1) to give the title compound as a pale-yellow oil (152 mg, 2 step yield 61%).
¹H-NMR(CDCl₃)δ: 7.12(1H,s),7.14-7.25(1H,m),7.25-7.28(1H,m),7.33-7.39(1H,m),7.44-7.55(3H,m),8.28-8.29(1H,m),8.45-8.47(1H,m),9.99(1H,s).

### Reference Example 291

### 1-(2-chlorophenyl)-5-[(5-fluoropyridin-3-yl)thio]-1H-pyrazole-3-carbaldehyde

{1-(2-Chlorophenyl)-5-[(5-fluoropyridin-3-yl)thio]-1H-pyrazol-3-yl}methanol (223 mg) was dissolved in toluene (3 mL), manganese dioxide (577 mg) was added, and the mixture was stirred at 110°C for 1 hr. The reaction mixture was allowed to cool to room temperature, and filtered through celite. The filtrate was concentrated under reduced pressure to give the title compound as a pale-yellow oil (181 mg, yield 82%).
¹H-NMR(CDCl₃)δ: 7.09-7.13(1H,m),7.20(1H,s),7.25-7.29(1H,m),7.34-7.39(1H,m),7.45-7.54(2H,m),8.08(1H,s),8.30-8.31(1H,m),10.0(1H,s)

### Reference Example 292

### 1-(2-chlorophenyl)-5-[(pyridin-4-yl)thio]-1H-pyrazole-3-carbaldehyde

A solution of ethyl 1-(2-chlorophenyl)-5-[(pyridin-4-yl)thio]-1H-pyrazole-3-carboxylate (124 mg) in tetrahydrofuran (5 mL) was cooled to -78°C, and 1.5 mol/L diisobutylaluminum hydride-toluene solution (0.9 mL) was added dropwise. The reaction mixture was stirred at room temperature for 4 hr, sodium sulfate 10 hydrate was added under ice-cooling, and the mixture was further stirred at room temperature for 30 min. The reaction mixture was filtered through celite, and the filtrate was concentrated under reduced pressure. The obtained residue was dissolved in toluene (5 mL), manganese dioxide (300 mg) was added, and the mixture was stirred at 80°C for 15 min. The reaction mixture was allowed to cool to room temperature, and filtered through celite. The filtrate was concentrated under reduced pressure, and the residue was purified by silica gel column chromatography (eluent: hexane-ethyl acetate=3:1→1:1) to give the title compound as a yellow oil (71.8 mg, yield 66%).
¹H-NMR(CDCl₃)δ: 6.86-6.88(2H,m),7.24-7.34(3H,m),7.42-7.54(2H,m),8.35-8.38(2H,m),10.06(1H,s).

### Reference Example 293

### 1-(2-chlorophenyl)-5-[(2-methylpyridin-4-yl)thio]-1H-pyrazole-3-carbaldehyde

A solution of ethyl 1-(2-chlorophenyl)-5-[(2-methylpyridin-4-yl)thio]-1H-pyrazole-3-carboxylate (286 mg) in tetrahydrofuran (5 mL) was cooled to -78°C, and 1.5 mol/L diisobutylaluminum hydride-toluene solution (2.0 mL) was added dropwise. The reaction mixture was stirred for 1 hr under ice-cooling, sodium sulfate 10 hydrate was added, and the mixture was further stirred at room temperature for 30 min. The reaction mixture was filtered through celite, and the filtrate was concentrated under reduced pressure. The obtained residue was dissolved in toluene (5 mL), manganese dioxide (664 mg) was added, and the mixture was stirred at 110°C for 2 hr. The reaction mixture was allowed to cool to room temperature, and filtered through celite. The filtrate was concentrated under reduced pressure, and the residue was purified by silica gel column chromatography (eluent: hexane-ethyl acetate=1:1→1:3) to give the title compound as a colorless oil (94.2 mg, yield 37%).
¹H-NMR(CDCl₃)δ: 2.44(3H,s),6.66-6.69(1H,m),6.72-6.73(1H,m),7.24-7.35(3H,m),7.41-7.47(1H,m),7.50 7.53(1H,m),8.24(1H,d,J=5.4Hz),10.06(1H,s).

### Reference Example 294

### 1-(2-chlorophenyl)-5-[(2-methoxypyridin-4-yl)thio]-1H-pyrazole-3-carbaldehyde

A solution of ethyl 1-(2-chlorophenyl)-5-[(2-methoxypyridin-4-yl)thio]-1H-pyrazole-3-carboxylate (325 mg) in tetrahydrofuran (5 mL) was cooled to -78°C, and 1.5 mol/L diisobutylaluminum hydride-toluene solution (2.2 mL) was added dropwise. The reaction mixture was stirred for 1 hr under ice-cooling, sodium sulfate 10 hydrate was added, and the mixture was further stirred at room temperature for 30 min. The reaction mixture was filtered through celite, and the filtrate was concentrated under reduced pressure. The obtained residue was dissolved in toluene (5 mL), manganese dioxide (682 mg) was added, and the mixture was stirred at 110°C for 1 hr. The reaction mixture was allowed to cool to room temperature, and filtered through celite. The filtrate was concentrated under reduced pressure, and the residue was purified by silica gel column chromatography (eluent: hexane-ethyl acetate=9:1→4:1) to give the title compound as a colorless oil (228 mg, yield 79%) .
¹H-NMR(CDCl₃)δ: 3.87(3H,s),6.28-6.29(1H,m),6.47-6.49(1H,m),7.25-7.35(3H,m),7.42-7.48(1H,m),7.52-7.55(1H,m),7.93(1H,d,J=5.4Hz),10.0(1H,s).

### Reference Example 295

### 1-(2-chlorophenyl)-5-[(6-methylpyridin-2-yl)thio]-1H-pyrazole-3-carbaldehyde

To a solution of ethyl 1-(2-chlorophenyl)-5-[(6-methylpyridin-2-yl)thio]-1H-pyrazole-3-carboxylate (640 mg) in tetrahydrofuran (10 mL) was added 1.5 mol/L diisobutylaluminum hydride-toluene solution (4.6 mL) at -78°C, and the mixture was stirred at the same temperature for 30 min. 1 mol/L Aqueous sodium hydroxide solution was added to the reaction mixture, the insoluble material was filtered off, and the filtrate was extracted with ethyl acetate. The extract was washed successively with water and saturated brine, dried over anhydrous sodium sulfate, and concentrated under reduced pressure. To a solution of the residue in toluene (7 mL) was added manganese dioxide (968 mg), and the mixture was stirred at 80°C for 1 hr. The reaction mixture was allowed to cool to room temperature, and filtered, and the filtrate was concentrated under reduced pressure. The residue was purified by silica gel column chromatography (eluent: hexane-ethyl acetate=2:1) to give the title compound as a yellow oil (420 mg, yield 74%).
¹H-NMR(CDCl₃)δ: 2.42(3H,s),6.71(1H,d,J=7.8Hz),6.88(1H,d,J=7.8Hz),7.22-7.43(5H,m),7.49-7.52(1H,m),10.05(1H,s).

### Reference Example 296

### 1-(2-chlorophenyl)-5-[(5-methylpyridin-2-yl)thio]-1H-pyrazole-3-carbaldehyde

To a solution of ethyl 1-(2-chlorophenyl)-5-[(5-methylpyridin-2-yl)thio]-1H-pyrazole-3-carboxylate (642 mg) in tetrahydrofuran (10 mL) was added 1.5 mol/L diisobutylaluminum hydride-toluene solution (3.4 mL) at -78°C, and the mixture was stirred at 0°C for 1 hr. 1 mol/L Aqueous sodium hydroxide solution was added to the reaction mixture, and the mixture was extracted with ethyl acetate. The extract was washed successively with water, saturated aqueous sodium hydrogen carbonate solution and saturated brine, dried over anhydrous sodium sulfate, and concentrated under reduced pressure. To a solution of the residue in toluene (8 mL) was added manganese dioxide (1.38 g), and the mixture was stirred at 80°C for 3 hr. The reaction mixture was allowed to cool to room temperature, and filtered, and the filtrate was concentrated under reduced pressure. The residue was purified by silica gel column chromatography (eluent: hexane-ethyl acetate=3:1) to give the title compound as a yellow oil (426 mg, yield 80%).
¹H-NMR(CDCl₃)δ: 2.25(3H,s),6.85-6.87(1H,m),7.24-7.43(5H,m),7.49-7.52(1H,m),8.17-8.18(1H,m),10.03(1H,s).

### Reference Example 297

### 1-(2-chlorophenyl)-5-[(6-methoxypyridin-2-yl)thio]-1H-pyrazole-3-carbaldehyde

To a solution of ethyl 1-(2-chlorophenyl)-5-[(6-methoxypyridin-2-yl)thio]-1H-pyrazole-3-carboxylate (594 mg) in tetrahydrofuran (6 mL) was added 1.5 mol/L diisobutylaluminum hydride-toluene solution (3 mL) at -78°C, and the mixture was stirred at the same temperature for 30 min, and then at -30°C for 1 hr. 1 mol/L Aqueous sodium hydroxide solution was added to the reaction mixture, and the mixture was extracted with ethyl acetate. The extract was washed successively with water and saturated brine, dried over anhydrous sodium sulfate, and concentrated under reduced pressure. To a solution of the residue in toluene (8 mL) was added manganese dioxide (1.79 g), and the mixture was stirred at 80°C for 1 hr. The reaction mixture was allowed to cool to room temperature, and filtered, and the filtrate was concentrated under reduced pressure. The residue was purified by silica gel column chromatography (eluent: hexane-ethyl acetate=4:1) to give the title compound as a yellow oil (406 mg, yield 77%).
¹H-NMR(CDCl₃)δ: 3.73(3H,s),6.45-6.53(2H,m),7.27-7.45(5H,m),7.51-7.54(1H,m),10.04(1H,s).

### Reference Example 298

### 1-(2-chloro-3-fluorophenyl)-5-[(pyridin-3-yl)thio]-1H-pyrazole-3-carbaldehyde

A solution of ethyl 1-(2-chloro-3-fluorophenyl)-5-[(pyridin-3-yl)thio]-1H-pyrazole-3-carboxylate (1.3 g) in tetrahydrofuran (15 mL) was cooled to -78°C, and 1.5 mol/L diisobutylaluminum hydride-toluene solution (9.4 mL) was added dropwise. The reaction mixture was stirred for 3 hr under ice-cooling, sodium sulfate 10 hydrate was added, and the mixture was further stirred at room temperature for 30 min. The reaction mixture was filtered through celite, and the filtrate was concentrated under reduced pressure. The obtained residue was dissolved in toluene (15 mL), manganese dioxide (2.0 g) was added, and the mixture was stirred at 110°C for 2 hr. The reaction mixture was allowed to cool to room temperature, and filtered through celite. The filtrate was concentrated under reduced pressure, and the residue was purified by silica gel column chromatography (eluent: hexane-ethyl acetate=4:1→2:1) to give the title compound as a yellow oil (588 mg, 2 step yield 50%).
¹H-NMR(CDCl₃)δ: 7.09-7.19(3H,m),7.33-7.38(2H,m),7.45-7.48(1H,m),8.30-8.31(1H,m),8.47-8.48(1H,m),9.98(1H,s).

### Reference Example 299

### 1-{5-[(3-bromophenyl)thio]-1-(2-chlorophenyl)-1H-pyrazol-3-yl}-N-methylmethanamine

To a suspension of aluminum chloride (763 mg) in tetrahydrofuran (8 mL) was slowly added lithium aluminum hydride (217 mg) at 0°C, and the mixture was stirred at the same temperature for 30 min. A solution of 5-[(3-bromophenyl)thio]-1-(2-chlorophenyl)-N-methyl-1H-pyrazole-3-carboxamide (595 mg) in tetrahydrofuran (2 mL) was added dropwise at 0°C to the obtained suspension, and the mixture was stirred at room temperature for 1 hr. 8 mol/L Aqueous sodium hydroxide solution was added to the reaction mixture, and the mixture was extracted with ethyl acetate. The extract was washed successively with saturated aqueous sodium hydrogen carbonate solution, water and saturated brine, dried over anhydrous sodium sulfate, and concentrated under reduced pressure to give the title compound as a yellow oil (522 mg, yield 91%).
¹H-NMR(CDCl₃)δ: 2.51(3H,s),3.86(2H,s),6.63(1H,s),6.96-7.29(6H,m),7.32-7.39(1H,m),7.44-7.47(1H,m),1H: not detected.

### Reference Example 300

### 1-{1-(2-chlorophenyl)-5-[(6-methylpyridin-2-yl)thio]-1H-pyrazol-3-yl}-N-methylmethanamine

To a solution of 1-(2-chlorophenyl)-5-[(6-methylpyridin-2-yl)thio]-1H-pyrazole-3-carbaldehyde (415 mg) in tetrahydrofuran (4 mL) were added 40% methylamine-methanol solution (1.5 mL) and methanol (4 mL) at 0°C, and the mixture was stirred at room temperature for 4 hr. Sodium borohydride (905 mg) was added at 0°C to the reaction mixture, and the mixture was stirred at room temperature for 1 hr, and concentrated under reduced pressure. Water was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure to give the title compound as a yellow oil (450 mg, yield quantitative).
¹H-NMR(CDCl₃)δ: 2.42(3H,s),2.53(3H,s),3.89(2H,s),6.66(1H,d,J=7.8Hz),6.72(1H,s) ,6.83(1H,d,J=7.8Hz),7.20-7.26(1H,m),7.30-7.36(3H,m),7.42-7.46(1H,m),1H: not detected.

### Reference Example 301

### 1-{1-(2-chlorophenyl)-5-[(5-methylpyridin-2-yl)thio]-1H-pyrazol-3-yl}-N-methylmethanamine

To a solution of 1-(2-chlorophenyl)-5-[(5-methylpyridin-2-yl)thio]-1H-pyrazole-3-carbaldehyde (422 mg) in tetrahydrofuran (4 mL) were added 40% methylamine-methanol solution (1.3 mL) and methanol (4 mL) at 0°C, and the mixture was stirred at room temperature for 16 hr, and concentrated under reduced pressure. The residue was dissolved in methanol (4 mL), and sodium borohydride (60 mg) was added at 0°C. The mixture was stirred at room temperature for 1 hr, and concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure to give the title compound as a yellow oil (433 mg, yield 98%).
¹H-NMR (CDCl₃) δ: 2.23(3H,s),2.52(3H,s),3.88(2H,s),6.70(1H,s),6.79-6.81(1H,m),7.19-7.35(4H,m),7.43-7.46(1H,m),8.15-8.16(1H,m),1H: not detected.

### Reference Example 302

### 1-{1-(2-chlorophenyl)-5-[(6-methoxypyridin-2-yl)thio]-1H-pyrazol-3-yl}-N-methylmethanamine

To a solution of 1-(2-chlorophenyl)-5-[(6-methoxypyridin-2-yl)thio]-1H-pyrazole-3-carbaldehyde (404 mg) in tetrahydrofuran (4 mL) were added 40% methylamine-methanol solution (1.5 mL) and methanol (4 mL) at 0°C, and the mixture was stirred at room temperature for 14 hr, and concentrated under reduced pressure. The residue was dissolved in methanol (4 mL), and sodium borohydride (76 mg) was added at 0°C. The mixture was stirred at room temperature for 1 hr, and concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure to give the title compound as a yellow oil (436 mg, yield quantitative).
¹H-NMR(CDCl₃)δ: 2.52(3H,s),3.78(3H,s),3.89(2H,s),6.41-6.47(2H,m),6.72(1H,s),7.21-7.37(4H,m),7.45-7.48(1H,m),1H: not detected.

### Reference Example 303

### 1-{5-[(3-bromophenyl)thio]-1-(2,5-difluorophenyl)-1H-pyrazol-3-yl}-N-methylmethanamine

To a suspension of aluminum chloride (1.28 g) in tetrahydrofuran (15 mL) was slowly added lithium aluminum hydride (364 mg) at 0°C, and the mixture was stirred at the same temperature for 15 min. A solution of 5-[(3-bromophenyl)thio]-1-(2,5-difluorophenyl)-N-methyl-1H-pyrazole-3-carboxamide (1.35 g) in tetrahydrofuran (7 mL) was added dropwise at 0°C to the obtained suspension, and the mixture was stirred at room temperature for 1 hr. 8 mol/L Aqueous sodium hydroxide solution was added to the reaction mixture, and the mixture was extracted with ethyl acetate. The extract was washed successively with saturated aqueous sodium hydrogen carbonate solution, water and saturated brine, dried over anhydrous sodium sulfate, and concentrated under reduced pressure to give the title compound as a yellow oil (1.18 g, yield 90%).
¹H-NMR(CDCl₃)δ: 2.51(3H,s),3.84(2H,s),6.63(1H,s),6.94-7.17(6H,m),7.25-7.30(1H,m),1H: not detected.

### Reference Example 304

### tert-butyl ({5-[(3-bromophenyl)thio]-1-(2-chlorophenyl)-1H-pyrazol-3-yl}methyl)methylcarbamate

To a solution of 1-{5-[(3-bromophenyl)thio]-1-(2-chlorophenyl)-1H-pyrazol-3-yl}-N-methylmethanamin (519 mg) in ethyl acetate (6 mL) was added di-tert-butyl bicarbonate (0.35 mL), and the mixture was stirred at room temperature for 12 hr. Saturated aqueous sodium hydrogen carbonate solution was added to the reaction mixture, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=4:1) to give the title compound as a yellow oil (533 mg, yield 82%).
¹H-NMR(CDCl₃)δ: 1.50(9H,s),2.91(3H,brs),4.46(2H,brs),6.56-6.63(1H,m),6.96-7.29(6H,m),7.32-7.40(1H,m),7.44-7.47(1H,m).

### Reference Example 305

### tert-butyl ({1-(2-chlorophenyl)-5-[(6-methylpyridin-2-yl)thio]-1H-pyrazol-3-yl}methyl)methylcarbamate

To a solution of 1-{1-(2-chlorophenyl)-5-[(6-methylpyridin-2-yl)thio]-1H-pyrazol-3-yl}-N-methylmethanamine (450 mg) in ethyl acetate (4 mL) was added di-tert-butyl bicarbonate (0.3 mL), and the mixture was stirred at room temperature for 12 hr. Water was added to the reaction mixture, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=3:1) to give the title compound as a yellow oil (462 mg, yield 82%).
¹H-NMR(CDCl₃)δ: 1.50(9H,s),2.42(3H,s),2.89-2.94(3H,m),4.50-4.80(2H,m),6.64-6.70(2H,m),6.84(1H,d,J=7.8Hz),7.21-7.26(1H,m),7.29-7.37(3H,m),7.43-7.46(1H,m).

### Reference Example 306

### tert-butyl ({1-(2-chlorophenyl)-5-[(5-methylpyridin-2-yl)thio]-1H-pyrazol-3-yl}methyl)methylcarbamate

To a solution of 1-{1-(2-chlorophenyl)-5-[(5-methylpyridin-2-yl)thio]-1H-pyrazol-3-yl}-N-methylmethanamine (425 mg) in ethyl acetate (5 mL) was added di-tert-butyl bicarbonate (0.3 mL) at 0°C, and the mixture was stirred at room temperature for 1 hr. Water was added to the reaction mixture, and the mixture was extracted with ethyl acetate. The extract was washed successively with saturated aqueous sodium hydrogen carbonate solution, water and saturated brine, dried over anhydrous sodium sulfate, and concentrated under reduced pressure to give the title compound as a yellow oil (654 mg, yield quantitative).
¹H-NMR(CDCl₃)δ: 1.50(9H,brs),2.24(3H,s),2.93(3H,brs),4.49(2H,brs),6.66(1H,brs) ,6.78-6.81(1H,m),7.20-7.36(4H,m),7.43-7.46(1H,m),8.15-8.16(1H,m).

### Reference Example 307

### tert-butyl ({1-(2-chlorophenyl)-5-[(6-methoxypyridin-2-yl)thio]-1H-pyrazol-3-yl}methyl)methylcarbamate

To a solution of 1-{1-(2-chlorophenyl)-5-[(6-methoxypyridin-2-yl)thio]-1H-pyrazol-3-yl}-N-methylmethanamine (436 mg) in ethyl acetate (5 mL) was added di-tert-butyl bicarbonate (0.28 mL), and the mixture was stirred at room temperature for 1 hr. Saturated aqueous sodium hydrogen carbonate solution was added to the reaction mixture, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=4:1) to give the title compound as a yellow oil (418 mg, yield 77%).
¹H-NMR(CDCl₃)δ: 1.50(9H,s),2.88-2.92(3H,m),3.77(3H,s),4.49-4.54(2H,m),6.42-6.47(2H,m),6.68-6.72(1H,m),7.22-7.38(4H,m),7.45-7.48(1H,m).

### Reference Example 308

### tert-butyl ({5-[(3-bromophenyl)thio]-1-(2,5-difluorophenyl)-1H-pyrazol-3-yl}methyl)methylcarbamate

To a solution of 1-{5-[(3-bromophenyl)thio]-1-(2,5-difluorophenyl)-1H-pyrazol-3-yl}-N-methylmethanamine (1.17 g) in ethyl acetate (15 mL) was added di-tert-butyl bicarbonate (0.7 mL) at 0°C, and the mixture was stirred at room temperature for 1 hr. Saturated aqueous sodium hydrogen carbonate solution was added to the reaction mixture, and the mixture was extracted with ethyl acetate. The extract was washed successively with saturated aqueous sodium hydrogen carbonate solution, water and saturated brine, dried over anhydrous sodium sulfate, and concentrated under reduced pressure to give the title compound as a yellow oil (1.52 g, yield quantitative).
¹H-NMR(CDCl₃)δ: 1.48(9H,brs),2.91(3H,brs),4.46(2H,brs),6.55-6.63(1H,m),6.94-7.18(6H,m),7.27-7.30(1H,m).

### Reference Example 309

### tert-butyl ({5-[(6-chloropyridin-3-yl)thio]-1-(2-fluorophenyl)-1H-pyrazol-3-yl}methyl)methylcarbamate

To a suspension of lithium aluminum hydride (972 mg) in tetrahydrofuran (20 mL) was added aluminum chloride (1.14 g) under ice-cooling under an argon atmosphere, and the mixture was stirred at room temperature for 30 min. 5-[(6-Chloropyridin-3-yl)thio]-1-(2-fluorophenyl)-N-methyl-1H-pyrazole-3-carboxamide (1.55 g) was added under ice-cooling to the reaction mixture, and the mixture was stirred for 1 hr under ice-cooling. Water (2.11 mL), 15% aqueous sodium hydroxide solution (2.11 mL) and water (6.33 mL) were successively added under ice-cooling to the reaction mixture, celite and anhydrous magnesium sulfate were added, and the mixture was stirred at room temperature for 30 min. The insoluble material was filtered, and washed with ethyl acetate, and the filtrate was concentrated under reduced pressure. The residue was dissolved in tetrahydrofuran (15 mL), and di-tert-butyl bicarbonate (1.96 mL) was added at room temperature. The mixture was stirred at room temperature for 2 hr, and concentrated under reduced pressure. The residue was purified by silica gel column chromatography (eluent: hexane-ethyl acetate=19:1→11:9) to give the title compound as a pale-yellow oil (1.19 g, yield 62%).
¹H-NMR(CDCl₃)δ: 1.49(9H,s),2.90(3H,brs),4.45(2H,brs),7.11-7.36(6H,m),7.37-7.52(1H,m),8.01(1H,d,J=2.3Hz).

### Reference Example 310

### tert-butyl ({1-(2-chlorophenyl)-5-[(6-chloropyridin-3-yl)thio]-1H-pyrazol-3-yl}methyl)methylcarbamate

To a suspension of lithium aluminum hydride (350 mg) in tetrahydrofuran (40 mL) was added aluminum chloride (3.69 g) under ice-cooling, and the mixture was stirred at the same temperature for 15 min. A solution of 1-(2-chlorophenyl)-5-[(6-chloropyridin-3-yl)thio]-N-methyl-1H-pyrazole-3-carboxamide (1.75 g) in tetrahydrofuran (30 mL) was added dropwise under ice-cooling, and the mixture was stirred at room temperature for 2 hr. The reaction mixture was cooled again, treated with 8 mol/L aqueous sodium hydroxide solution, dried over anhydrous sodium sulfate, and filtered. The filtrate was concentrated under reduced pressure, and ethyl acetate and saturated aqueous sodium hydrogen carbonate solution were added to the residue. Di-tert-butyl bicarbonate (1.21 g) was added, and the mixture was stirred for 10 min. The ethyl acetate layer was washed with saturated brine, dried over anhydrous sodium sulfate, and concentrated under reduced pressure. The residue was purified by silica gel column chromatography (eluent: hexane-ethyl acetate=9:1→4:1) to give the title compound as a colorless oil (1.32 g, yield 62%).
¹H-NMR(CDCl₃)δ: 1.50(9H,s),2.88(3H,br),4.45(2H,br),6.60(1H,brd),7.14-7.50(6H,m),7.99-8.00(1H,m).

### Reference Example 311

### tert-butyl ({1-(2-chlorophenyl)-5-[(pyridin-2-yl)thio]-1H-pyrazol-3-yl}methyl)methylcarbamate

To a suspension of aluminum chloride (594 mg) in tetrahydrofuran (8 mL) was slowly added lithium aluminum hydride (175 mg) at 0°C, and the mixture was stirred at the same temperature for 15 min. A solution of 5-[(pyridin-2-yl)thio]-1-(2-chlorophenyl)-N-methyl-1H-pyrazole-3-carboxamide (464 mg) in tetrahydrofuran (2 mL) was added dropwise at 0°C to the obtained suspension, and the mixture was stirred at room temperature for 6 hr. 8 mol/L Aqueous sodium hydroxide solution was added to the reaction mixture, and the mixture was extracted with ethyl acetate. The extract was washed successively with saturated aqueous sodium hydrogen carbonate solution, water and saturated brine, dried over anhydrous sodium sulfate, and concentrated under reduced pressure. To a solution of the residue in ethyl acetate (5 mL) was added di-tert-butyl bicarbonate (0.25 mL), and the mixture was stirred at room temperature for 12 hr. Saturated aqueous sodium hydrogen carbonate solution was added to the reaction mixture, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=3:1→1:1) to give the title compound as a yellow oil (198 mg, yield 35%).
¹H-NMR(CDCl₃)δ: 1.50(9H,s),2.90-2.94(3H,m),4.50-4.54(2H,m),6.70-6.75(1H,m),6.88(1H,d,J=7.8Hz),6.97-7.01(1H,m),7.20-7.36(3H,m),7.43-7.60(2H,m),8.31-8.33(1H,m).

### Reference Example 312

### tert-butyl ({5-[(6-chloropyridin-3-yl)thio]-1-(2,3-difluorophenyl)-1H-pyrazol-3-yl}methyl)methylcarbamate

To a suspension of lithium aluminum hydride (96 mg) in tetrahydrofuran (10 mL) was added aluminum chloride (1.0 g) under ice-cooling, and the mixture was stirred at the same temperature for 15 min. A solution of 5-[(6-chloropyridin-3-yl)thio]-1-(2,3-difluorophenyl)-N-methyl-1H-pyrazole-3-carboxamide (478 mg) in tetrahydrofuran (10 mL) was added dropwise under ice-cooling, and the mixture was stirred at room temperature for 2 hr. The reaction mixture was cooled again, treated with 8 mol/L aqueous sodium hydroxide solution, dried over anhydrous sodium sulfate, and filtered. The filtrate was concentrated under reduced pressure, and ethyl acetate and saturated aqueous sodium hydrogen carbonate solution were added to the residue. Di-tert-butyl bicarbonate (330 mg) was added, and the mixture was stirred for 15 min. The ethyl acetate layer was washed with saturated brine, dried over anhydrous sodium sulfate, and concentrated under reduced pressure. The residue was purified by silica gel column chromatography (eluent: hexane-ethyl acetate=9:1→4:1) to give the title compound as a colorless oil (475 mg, yield 81%).
¹H-NMR(CDCl₃)δ: 1.49(9H,s),2.90(3H,brs),4.47(2H,br),6.62(1H,br),7.05-7.19(3H,m),7.24-7.35(2H,m),8.03-8.04(1H,m).

### Reference Example 313

### tert-butyl ({5-[(6-chloropyridin-3-yl)thio]-1-(2,4-difluorophenyl)-1H-pyrazol-3-yl}methyl)methylcarbamate

To a suspension of lithium aluminum hydride (62 mg) in tetrahydrofuran (5 mL) was added aluminum chloride (655 mg) under ice-cooling, and the mixture was stirred at the same temperature for 15 min. A solution of 5-[(6-chloropyridin-3-yl)thio]-1-(2,4-difluorophenyl)-N-methyl-1H-pyrazole-3-carboxamide (312 mg) in tetrahydrofuran (5 mL) was added dropwise under ice-cooling, and the mixture was stirred at room temperature for 2 hr. The reaction mixture was cooled again, treated with 8 mol/L aqueous sodium hydroxide solution, dried over anhydrous sodium sulfate, and filtered. The filtrate was concentrated under reduced pressure, and ethyl acetate and saturated aqueous sodium hydrogen carbonate solution were added to the residue. Di-tert-butyl bicarbonate (215 mg) was added, and the mixture was stirred for 30 min. The ethyl acetate layer was washed with saturated brine, dried over anhydrous sodium sulfate, and concentrated under reduced pressure. The residue was purified by silica gel column chromatography (eluent: hexane-ethyl acetate=9:1→4:1) to give the title compound as a colorless oil (328 mg, yield 86%).
¹H-NMR(CDCl₃)δ: 1.49(9H,s),2.89(3H,brs),4.45(2H,br),6.60(1H,br),6.91-6.99(2H,m),7.16-7.19(1H,m),7.23-7.35(2H,m),8.01-8.02(1H,m).

### Reference Example 314

### tert-butyl ({5-[(6-chloropyridin-3-yl)thio]-1-(2,5-difluorophenyl)-1H-pyrazol-3-yl}methyl)methylcarbamate

To a suspension of lithium aluminum hydride (90 mg) in tetrahydrofuran (10 mL) was added aluminum chloride (952 mg) under ice-cooling, and the mixture was stirred at the same temperature for 15 min. A suspension of 5-[(6-chloropyridin-3-yl)thio]-1-(2,5-difluorophenyl)-N-methyl-1H-pyrazole-3-carboxamide (457 mg) in tetrahydrofuran (10 mL) was added dropwise under ice-cooling, and the mixture was stirred at room temperature for 2 hr. The reaction mixture was cooled again, treated with 8 mol/L aqueous sodium hydroxide solution, dried over anhydrous sodium sulfate, and filtered. The filtrate was concentrated under reduced pressure, and ethyl acetate and saturated aqueous sodium hydrogen carbonate solution were added to the residue. Di-tert-butyl bicarbonate (312 mg) was added, and the mixture was stirred for 1 hr. The ethyl acetate layer was washed with saturated brine, dried over anhydrous sodium sulfate, and concentrated under reduced pressure. The residue was purified by silica gel column chromatography (eluent: hexane-ethyl acetate=4:1→2:1) to give the title compound as a colorless oil (530 mg, yield 95%).
¹H-NMR(CDCl₃)δ: 1.48(9H,s),2.89(3H,brs),4.46(2H,br),6.59(1H,br),7.04-7.19(4H,m),7.26-7.35(1H,m),8.05-8.06(1H,m).

### Reference Example 315

### tert-butyl ({5-[(6-chloropyridin-3-yl)thio]-1-(2-fluoro-3-methylphenyl)-1H-pyrazol-3-yl}methyl)methylcarbamate

To a suspension of lithium aluminum hydride (174 mg) in tetrahydrofuran (15 mL) was added aluminum chloride (1.8 g) under ice-cooling, and the mixture was stirred at the same temperature for 15 min. A suspension of 5-[(6-chloropyridin-3-yl)thio]-1-(2-fluoro-3-methylphenyl)-N-methyl-1H-pyrazole-3-carboxamide (862 mg) in tetrahydrofuran (10 mL) was added dropwise under ice-cooling, and the mixture was stirred at room temperature for 2 hr. The reaction mixture was cooled again, treated with 8 mol/L aqueous sodium hydroxide solution, dried over anhydrous sodium sulfate, and filtered. The filtrate was concentrated under reduced pressure, and ethyl acetate and saturated aqueous sodium hydrogen carbonate solution were added to the residue. Di-tert-butyl bicarbonate (600 mg) was added, and the mixture was stirred for 15 min. The ethyl acetate layer was washed with saturated brine, dried over anhydrous sodium sulfate, and concentrated under reduced pressure. The residue was purified by silica gel column chromatography (eluent: hexane-ethyl acetate=7:1→3:1) to give the title compound as a colorless oil (1.0 g, yield 94%).
¹H-NMR(CDCl₃)δ: 1.49(9H,s),2.27-2.28(3H,m),2.89(3H,brs),4.46(2H,br),6.59(1H,br),7.04-7.16(3H,m),7.23-7.33(2H,m),7.99-8.00(1H, m).

### Reference Example 316

### tert-butyl ({5-[(6-chloropyridin-3-yl)thio]-1-(2-f luoro-4-methylphenyl)-1H-pyrazol-3-yl}methyl)methylcarbamate

To a suspension of lithium aluminum hydride (170 mg) in tetrahydrofuran (15 mL) was added aluminum chloride (1.0 g) under ice-cooling, and the mixture was stirred at the same temperature for 15 min. A solution of 5-[(6-chloropyridin-3-yl)thio]-1-(2-fluoro-4-methylphenyl)-N-methyl-1H-pyrazole-3-carboxamide (843 mg) in tetrahydrofuran (10 mL) was added dropwise under ice-cooling, and the mixture was stirred at room temperature for 3 hr. The reaction mixture was cooled again, treated with 8 mol/L aqueous sodium hydroxide solution, dried over anhydrous sodium sulfate, and filtered. The filtrate was concentrated under reduced pressure, and ethyl acetate and saturated aqueous sodium hydrogen carbonate solution were added to the residue. Di-tert-butyl bicarbonate (587 mg) was added, and the mixture was stirred for 15 min. The ethyl acetate layer was washed with saturated brine, dried over anhydrous sodium sulfate, and concentrated under reduced pressure. The residue was purified by silica gel column chromatography (eluent: hexane-ethyl acetate=9:1→4:1) to give the title compound as a colorless oil (882 mg, yield 85%).
¹H-NMR(CDCl₃)δ: 1.48(9H,s),2.40(3H,s),2.89(3H,brs),4.45(2H,br),6.58(1H,br),6.9 6-7.00(2H,m),7.11-7.17(2H,m),7.29-7.33(1H,m),8.01-8.02(1H,m).

### Reference Example 317

### tert-butyl ({5-[(6-chloropyridin-3-yl)thio]-1-(2-fluoro-5-methylphenyl)-1H-pyrazol-3-yl}methyl)methylcarbamate

To a suspension of lithium aluminum hydride (193 mg) in tetrahydrofuran (10 mL) was added aluminum chloride (2.0 g) under ice-cooling, and the mixture was stirred at the same temperature for 10 min. A solution of 5-[(6-chloropyridin-3-yl)thio]-1-(2-fluoro-5-methylphenyl)-N-methyl-1H-pyrazole-3-carboxamide (956 mg) in tetrahydrofuran (10 mL) was added dropwise under ice-cooling, and the mixture was stirred at room temperature for 5 hr. The reaction mixture was cooled again, treated with 8 mol/L aqueous sodium hydroxide solution, dried over anhydrous sodium sulfate, and filtered. The filtrate was concentrated under reduced pressure, and ethyl acetate and saturated aqueous sodium hydrogen carbonate solution were added to the residue. Di-tert-butyl bicarbonate (665 mg) was added, and the mixture was stirred for 15 min. The ethyl acetate layer was washed with saturated brine, dried over anhydrous sodium sulfate, and concentrated under reduced pressure. The residue was purified by silica gel column chromatography (eluent: hexane-ethyl acetate=9:1→4:1) to give the title compound as a colorless oil (978 mg, yield 83%).
¹H-NMR(CDCl₃)δ: 1.48(9H,s),2.31(3H,s),2.88(3H,brs),4.45(2H,br),6.57(1H,br),7.0 1-7.07(2H,m),7.14-7.25(2H,m),7.30-7.34(1H,m),8.01-8.02(1H,m).

### Reference Example 318

### tert-butyl ({5-[(6-chloropyridin-3-yl)thio]-1-(3-fluoro-2-methylphenyl)-1H-pyrazol-3-yl}methyl)methylcarbamate

To a suspension of lithium aluminum hydride (497 mg) in tetrahydrofuran (30 mL) was added aluminum chloride (5.24 g) under ice-cooling, and the mixture was stirred at the same temperature for 30 min. A solution of 5-[(6-chloropyridin-3-yl)thio]-1-(3-fluoro-2-methylphenyl)-N-methyl-1H-pyrazole-3-carboxamide (2.47 g) in tetrahydrofuran (15 mL) was added dropwise under ice-cooling, and the mixture was stirred at room temperature for 2 hr. 8 mol/L Aqueous sodium hydroxide solution and ethyl acetate were added to the residue, di-tert-butyl bicarbonate (1.43 g) was added, and the mixture was stirred for 1 hr. 6 mol/L Hydrochloric acid was added to the reaction mixture, and the aqueous layer and the organic layer were separated. The separated aqueous layer was extracted again with ethyl acetate. The combined organic layers were washed with saturated brine, dried over sodium sulfate, and concentrated under reduced pressure. The residue was purified by silica gel column chromatography (eluent: hexane-ethyl acetate=19:1→4:1) to give the title compound as a colorless oil (2.84 g, yield 93%).
¹H-NMR(CDCl₃)δ: 1.50(9H,s),1.85(3H,d,J=1.9Hz),2.88(3H,brs),4.46(2H,brs),6.58(1 H,d,J=18.9Hz),6.88(1H,d,J=6.8Hz),7.07-7.23(3H,m),7.32(1H,dd,J=8.3,2.7Hz),8.02(1H,d,J=2.3Hz).

### Reference Example 319

### tert-butyl ({5-[(6-chloropyridin-3-yl)thio]-1-(5-fluoro-2-methylphenyl)-1H-pyrazol-3-yl}methyl)methylcarbamate

To a suspension of lithium aluminum hydride (534 mg) in tetrahydrofuran (30 mL) was added aluminum chloride (5.6 g) under ice-cooling, and the mixture was stirred at the same temperature for 15 min. A solution of 5-[(6-chloropyridin-3-yl)thio]-1-(5-fluoro-2-methylphenyl)-N-methyl-1H-pyrazole-3-carboxamide (2.7 g) in tetrahydrofuran (20 mL) was added dropwise under ice-cooling, and the mixture was stirred at room temperature for 3 hr. The reaction mixture was cooled again, treated with 8 mol/L aqueous sodium hydroxide solution, dried over anhydrous sodium sulfate, and filtered. The filtrate was concentrated under reduced pressure, and ethyl acetate and saturated aqueous sodium hydrogen carbonate solution were added to the residue. Di-tert-butyl bicarbonate (1.8 g) was added, and the mixture was stirred for 10 min. The ethyl acetate layer was washed with saturated brine, dried over anhydrous sodium sulfate, and concentrated under reduced pressure. The residue was purified by silica gel column chromatography (eluent: hexane-ethyl acetate=9:1→4:1) to give the title compound as a colorless oil (3.0 g, yield 93%).
¹H-NMR(CDCl₃)δ: 1.50(9H,s),1.87(3H,s),2.88(3H,brs),4.44(2H,br),6.56(1H,br),6.8 1-6.84(1H,m),7.04-7.10(1H,m),7.15-7.25(2H,m),7.31-7.35(1H,m),8.02(1H,br).

### Reference Example 320

### tert-butyl ({1-(2-chloro-3-fluorophenyl)-5-[(6-chloropyridin-3-yl)thio]-1H-pyrazol-3-yl}methyl)methylcarbamate

To a suspension of lithium aluminum hydride (222 mg) in tetrahydrofuran (10 mL) was added aluminum chloride (2.3 g) under ice-cooling, and the mixture was stirred at the same temperature for 15 min. A suspension of 1-(2-chloro-3-fluorophenyl)-5-[(6-chloropyridin-3-yl)thio]-N-methyl-1H-pyrazole-3-carboxamide (1.2 g) in tetrahydrofuran (10 mL) was added dropwise under ice-cooling, and the mixture was stirred at room temperature for 4 hr. The reaction mixture was cooled again, treated with 8 mol/L aqueous sodium hydroxide solution, dried over anhydrous sodium sulfate, and filtered. The filtrate was concentrated under reduced pressure, and ethyl acetate and saturated aqueous sodium hydrogen carbonate solution were added to the residue. Di-tert-butyl bicarbonate (765 mg) was added, and the mixture was stirred for 15 min. The ethyl acetate layer was washed with saturated brine, dried over anhydrous sodium sulfate, and concentrated under reduced pressure. The residue was purified by silica gel column chromatography (eluent: hexane-ethyl acetate=9:1→4:1) to give the title compound as a colorless oil (1.3 g, yield 90%).
¹H-NMR(CDCl₃)δ: 1.49(9H,s),2.88(3H,brs),4.45(2H,br),6.62(1H,br),7.02-7.09(1H,m),7.16-7.18(1H,m),7.25-7.36(3H,m),8.01-8.02(1H,m).

### Reference Example 321

### tert-butyl ({1-(2-chloro-5-fluorophenyl)-5-[(6-chloropyridin-3-yl)thio]-1H-pyrazol-3-yl}methyl)methylcarbamate

To a suspension of lithium aluminum hydride (60 mg) in tetrahydrofuran (10 mL) was added aluminum chloride (635 mg) under ice-cooling, and the mixture was stirred at the same temperature for 5 min. A solution of 1-(2-chloro-5-fluorophenyl)-5-[(6-chloropyridin-3-yl)thio]-N-methyl-1H-pyrazole-3-carboxamide (343 mg) in tetrahydrofuran (10 mL) was added dropwise under ice-cooling, and the mixture was stirred at room temperature for 3 hr. The reaction mixture was cooled again, treated with 8 mol/L aqueous sodium hydroxide solution, dried over anhydrous sodium sulfate, and filtered. The filtrate was concentrated under reduced pressure, and ethyl acetate and saturated aqueous sodium hydrogen carbonate solution were added to the residue. Di-tert-butyl bicarbonate (208 mg) was added, and the mixture was stirred for 15 min. The ethyl acetate layer was washed with saturated brine, dried over anhydrous sodium sulfate, and concentrated under reduced pressure. The residue was purified by silica gel column chromatography (eluent: hexane-ethyl acetate=9:1→4:1) to give the title compound as a colorless oil (258 mg, yield 67%).
¹H-NMR(CDCl₃)δ: 1.49(9H,s),2.88(3H,brs),4.46(2H,brs),6.60(1H,br),7.00-7.05(1H,m),7.12-7.19(2H,m),7.34-7.46(2H,m),8.03-8.05(1H,m).

### Reference Example 322

### tert-butyl ({1-(2-chlorophenyl)-5-[(pyridin-3-yl)thio]-1H-pyrazol-3-yl}methyl)methylcarbamate

1-(2-Chlorophenyl)-5-[(pyridin-3-yl)thiol-1H-pyrazole-3-carbaldehyde (152 mg) was dissolved in a mixed solvent of tetrahydrofuran (3 mL) and methanol (1 mL), 40% methylamine-methanol solution (0.5 mL) was added, and the mixture was stirred at room temperature for 1 hr. The reaction mixture was concentrated under reduced pressure, the residue was dissolved again in a mixed solvent of tetrahydrofuran (3 mL) and methanol (1 mL), and sodium borohydride (55 mg) was added under ice-cooling. The mixture was stirred at room temperature for 3 hr, water was added, and the solvent was evaporated under reduced pressure. Ethyl acetate and saturated aqueous sodium hydrogen carbonate solution were added to the residue, di-tert-butyl bicarbonate (126 mg) was added, and the mixture was stirred for 10 min. The ethyl acetate layer was washed with saturated brine, dried over anhydrous sodium sulfate, and concentrated under reduced pressure. The residue was purified by silica gel column chromatography (eluent: hexane-ethyl acetate=4:1→2:1) to give the title compound as a pale-yellow oil (190 mg, yield 92%).
¹H-NMR(CDCl₃)δ: 1.49(9H,s),2.89(3H,br),4.45(2H,br),6.58(1H,br),7.10-7.15(1H,m),7.20-7.33(2H,m),7.35-7.47(3H,m),8.25-8.26(1H,m),8.39-8.40(1H,m).

### Reference Example 323

### tert-butyl ({1-(2-chlorophenyl)-5-[(5-fluoropyridin-3-yl)thio]-1H-pyrazol-3-yl}methyl)methylcarbamate

1-(2-Chlorophenyl)-5-[(5-fluoropyridin-3-yl)thio]-1H-pyrazole-3-carbaldehyde (181 mg) was dissolved in a mixed solvent of tetrahydrofuran (3 mL) and methanol (1 mL), 40% methylamine-methanol solution (0.5 mL) was added at room temperature, and the mixture was stirred for 1 hr. Sodium borohydride was added under ice-cooling, and the mixture was further stirred at room temperature for 3 hr. The solvent was evaporated under reduced pressure, and sodium hydrogen carbonate and ethyl acetate were added to the residue. Di-tert-butyl bicarbonate (142 mg) was added, and the mixture was stirred for 1 hr. The ethyl acetate layer was washed with saturated brine, dried over anhydrous sodium sulfate, and concentrated under reduced pressure. The residue was purified by silica gel column chromatography (eluent: hexane-ethyl acetate=4:1→2:1) to give the title compound as a colorless oil (172 mg, yield 71%).
¹H-NMR(CDCl₃)δ: 1.50(9H,s),2.91(3H,brs),4.48(2H,brs),6.67(1H,br),7.04-7.08(1H,m),7.22-7.48(4H,m),8.05-8.06(1H,m),8.24-8.25(1H,m).

### Reference Example 324

### tert-butyl ({1-(2-chlorophenyl)-5-[(pyridin-4-yl)thio]-1H-pyrazol-3-yl}methyl)methylcarbamate

1-(2-Chlorophenyl)-5-[(pyridin-4-yl)thio]-1H-pyrazole-3-carbaldehyde (270 mg) was dissolved in a mixed solvent of tetrahydrofuran (3 mL) and methanol (1 mL), 40% methylamine-methanol solution (3 mL) was added at room temperature, and the mixture was stirred for 2 hr. Sodium borohydride (97 mg) was added under ice-cooling, and the mixture was further stirred at room temperature for 3 hr. The solvent was evaporated under reduced pressure, and sodium hydrogen carbonate and ethyl acetate were added to the residue. Di-tert-butyl bicarbonate (224 mg) was added, and the mixture was stirred for 15 min. The ethyl acetate layer was washed with saturated brine, dried over anhydrous sodium sulfate, and concentrated under reduced pressure. The residue was purified by silica gel column chromatography (eluent: hexane-ethyl acetate=2:1→1:2) to give the title compound as a pale-yellow oil (279 mg, yield 76%).
¹H-NMR(CDCl₃)δ: 1.50(9H,s),2.94(3H,br),4.52(2H,br),6.70(1H,br),6.85-6.88(2H,m),7.19-7.23(2H,m),7.34-7.40(1H,m), 7.45-7.48(1H,m),8.33-8.36(1H,m).

### Reference Example 325

### tert-butyl ({1-(2-chlorophenyl)-5-[(2-methylpyridin-4-yl)thio]-1H-pyrazol-3-yl}methyl)methylcarbamate

1-(2-Chlorophenyl)-5-[(2-methylpyridin-4-yl)thio]-1H-pyrazole-3-carbaldehyde (94 mg) was dissolved in a mixed solvent of tetrahydrofuran (3 mL) and methanol (1 mL), 40% methylamine-methanol solution (0.3 mL) was added at room temperature, and the mixture was stirred for 3 hr. Sodium borohydride was added under ice-cooling, and the mixture was further stirred at room temperature for 2 hr. The solvent was evaporated under reduced pressure, and sodium hydrogen carbonate and ethyl acetate were added to the residue. Di-tert-butyl bicarbonate (75 mg) was added, and the mixture was stirred for 30 min. The ethyl acetate layer was washed with saturated brine, dried over anhydrous sodium sulfate, and concentrated under reduced pressure. The residue was purified by silica gel column chromatography (eluent: hexane-ethyl acetate=4:1→1:1) to give the title compound as a colorless oil (78.4 mg, yield 62%).
¹H-NMR(CDCl₃)δ: 1.50(9H,s),2.43(3H,s),2.94(3H,br),4.52(2H,br),6.65-6.67(1H,m),6.72-6.73(1H,m),7.21-7.30(3H,m),7.33-7.39(1H,m),7.45-7.48(1H,m),8.22(1H,d,J=5.4Hz).

### Reference Example 326

### tert-butyl ({1-(2-chlorophenyl)-5-[(2-methoxypyridin-4-yl)thio]-1H-pyrazol-3-yl}methyl)methylcarbamate

1-(2-Chlorophenyl)-5-[(2-methoxypyridin-4-yl)thio]-1H-pyrazole-3-carbaldehyde (228 mg) was dissolved in a mixed solvent of tetrahydrofuran (3 mL) and methanol (1 mL), 40% methylamine-methanol solution (0.7 mL) was added at room temperature, and the mixture was stirred for 2 hr. Sodium borohydride was added under ice-cooling, and the mixture was further stirred at room temperature for 1 hr. The solvent was evaporated under reduced pressure, and sodium hydrogen carbonate and ethyl acetate were added to the residue. Di-tert-butyl bicarbonate (173 mg) was added, and the mixture was stirred for 1 hr. The ethyl acetate layer was washed with saturated brine, dried over anhydrous sodium sulfate, and concentrated under reduced pressure. The residue was purified by silica gel column chromatography (eluent: hexane-ethyl acetate=9:1→3:1) to give the title compound as a colorless oil (237 mg, yield 78%).
¹H-NMR(CDCl₃)δ: 1.50(9H,m),2.94(3H,br),3.86.(3H,s),4.51(2H,br),6.28-6.29(1H,m),6.46-6.48(1H,m)6.70(1H,br),7.23-7.30(2H,m),7.34-7.40(1H,m),7.46-7.49(1H,m),7.91(1H,d,J=5.4Hz).

### Reference Example 327

### tert-butyl ({1-(2-chloro-3-fluorophenyl)-5-[(pyridin-3-yl)thio]-1H-pyrazol-3-yl}methyl)methylcarbamate

1-(2-Chloro-3-fluorophenyl)-5-[(pyridin-3-yl)thio]-1H-pyrazole-3-carbaldehyde (588 mg) was dissolved in a mixed solvent of tetrahydrofuran (5 mL) and methanol (2 mL), 40% methylamine-methanol solution (1.8 mL) was added, and the mixture was stirred at room temperature for 1 hr. The reaction mixture was concentrated under reduced pressure, and the residue was dissolved again in a mixed solvent of tetrahydrofuran (3 mL) and methanol (1 mL). Sodium borohydride (200 mg) was added under ice-cooling, and the mixture was stirred at room temperature for 3 hr. Water was added, and the solvent was evaporated under reduced pressure. Ethyl acetate and saturated aqueous sodium hydrogen carbonate solution were added to the residue, di-tert-butyl bicarbonate (461 mg) was added, and the mixture was stirred for 30 min. The ethyl acetate layer was washed with saturated brine, dried over anhydrous sodium sulfate, and concentrated under reduced pressure. The residue was purified by silica gel column chromatography (eluent: hexane-ethyl acetate=7:1→2:1) to give the title compound as a pale-yellow oil (528 mg, yield 68%).
¹H-NMR(CDCl₃)δ: 1.49(9H,s),2.89(3H,br),4.45(2H,br),6.61(1H,br),7.03-7.07(1H,m),7.12-7.16(1H,m),7.24-7.28(2H,m),7.37-7.41(1H,m),8.27-8.28(1H,m),8.41-8.42(1H,m).

### Reference Example 328

### 1-(2-fluorophenyl)-N-methyl-5-(phenylsulfonyl)-1H-pyrazole-3-carboxamide

To ethyl 1-(2-fluorophenyl)-5-(phenylsulfonyl)-1H-pyrazole-3-carboxylate (46 mg) was added 40% methylamine-methanol solution (3 mL), and the mixture was stirred at room temperature for 3 hr, and concentrated under reduced pressure to give the title compound as a white solid (40.2 mg, yield 91%).
¹H-NMR(CDCl₃)δ: 2.96(3H,d,J=4.9Hz),6.79(1H,brs),7.09(1H,t,J=8.9Hz),7.22-7.31(1H,m),7.31-7.46(3H,m),7.48-7.64(5H,m).

### Reference Example 329

### 1-(2-fluorophenyl)-5-[(3-methoxyphenyl)sulfonyl]-N-methyl-1H-pyrazole-3-carboxamide

To ethyl 1-(2-fluorophenyl)-5-[(3-methoxyphenyl)sulfonyl]-1H-pyrazole-3-carboxylate (144 mg) was added 40% methylamine-methanol solution (3 mL), and the mixture was stirred at room temperature for 2 hr, and concentrated under reduced pressure to give the title compound as a white solid (136 mg, yield 98%).
¹H-NMR(CDCl₃)δ: 2.96(3H,d,J=4.9Hz),3.75(3H,s),6.75-6.84(1H,m),6.98-7.03(1H,m),7.06-7.16(3H,m),7.22-7.41(3H,m),7.48-7.59(2H,m).

### Reference Example 330

### 5-[(3-methoxyphenyl)sulfonyl]-N-methyl-1-(2-methylphenyl)-1H-pyrazole-3-carboxamide

To a solution of ethyl 5-[(3-methoxyphenyl)sulfonyl]-1-(2-methylphenyl)-1H-pyrazole-3-carboxylate (500 mg) in methanol (2 mL) was added 40% methylamine-methanol solution (5 mL) at room temperature, and the mixture was stirred for 4 hr. The reaction mixture was concentrated under reduced pressure, and the residue was purified by silica gel column chromatography (eluent: hexane-ethyl acetate=4:1→2:1) to give the title compound as a pale-yellow amorphous solid (489 mg, yield quantitative).
¹H-NMR(CDCl₃)δ: 1.61(3H,s),2.96(3H,d,J=5.1Hz),3.70(3H,s),6.79(1H,br),6.86-6.88(1H,m),7.05-7.11(3H,m),7.19-7.30(3H,m),7.40-7.45(1H,m),7.61(1H,s).

### Reference Example 331

### 1-(2,6-difluorophenyl)-N-methyl-5-(phenylsulfonyl)-1H-pyrazole-3-carboxamide

To a solution of ethyl 1-(2,6-difluorophenyl)-5-(phenylsulfonyl)-1H-pyrazole-3-carboxylate (300 mg) in methanol (1 mL) was added 40% methylamine-methanol solution (1 mL), and the mixture was stirred at room temperature for 2 hr, and concentrated under reduced pressure to give the title compound as a white solid (279 mg, yield 97%).
¹H-NMR(CDCl₃)δ: 2.96(3H,d,J=4.9Hz),6.73-6.80(1H,m),6.98-7.06(2H,m),7.42-7.50(2H,m),7.51-7.57(1H,m),7.58(1H,s),7.59-7.67(3H,m).

### Reference Example 332

### 5-[(6-chloropyridin-3-yl)sulfonyl]-N-methyl-1-(2-methylphenyl)-1H-pyrazole-3-carboxamide

To a solution of 5-[(6-chloropyridin-3-yl)thio]-N-methyl-1-(2-methylphenyl)-1H-pyrazole-3-carboxamide (356 mg) in ethyl acetate (10 mL) was added 3-chloroperbenzoic acid (732 mg) at 0°C, and the mixture was stirred at room temperature for 14 hr. Aqueous sodium thiosulfate solution was added to the reaction mixture, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was washed with diisopropyl ether to give the title compound as a colorless solid (362 mg, yield 94%).
¹H-NMR(CDCl₃)δ: 1.68(3H,s),2.97(3H,d,J=5.1Hz),6.78(1H,brs),7.06-7.08(1H,m),7.25-7.24(3H,m),7.46-7.52(1H,m),7.63-7.67(2H,m),8.25-8.26(1H,m).

### Reference Example 333

### N-methyl-1-(2-methylphenyl)-5-[(6-methylpyridin-3-yl)sulfonyl]-1H-pyrazole-3-carboxamide

A suspension of 5-[(6-chloropyridin-3-yl)sulfonyl]-N-methyl-1-(2-methylphenyl)-1H-pyrazole-3-carboxamide (352 mg), methylboronic acid (537 mg), tetrakis(triphenylphosphine) palladium(0) (50 mg) and potassium carbonate (151 mg) in a mixed solvent of cyclopentylmethyl ether (5 mL) and tetrahydrofuran (5 mL) was stirred at 80°C for 14 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=1:1) to give the title compound as a colorless solid (155 mg, yield 46%).
¹H-NMR(CDCl₃)δ: 1.65(3H,s),2.63(s,3H),2.96(3H,d,J=4.8Hz),6.79(1H,brs),7.07-7.10(1H,m),7.14-7.17(1H,m),7.23-7.30(2H,m),7.44-7.52(1H,m),7.58-7.62(2H,m),8.38-8.39(1H,m).

### Reference Example 334

### [1-(2-fluoropyridin-3-yl)-5-(phenylsulfonyl)-1H-pyrazol-3-yl]methanol

A solution of ethyl 1-(2-fluoropyridin-3-yl)-5-(phenylsulfonyl)-1H-pyrazole-3-carboxylate (1.1 g) in tetrahydrofuran (10 mL) was cooled to -78°C, and 1.5 mol/L diisobutylaluminum hydride-toluene solution (5.8 mL) was added dropwise. The reaction mixture was stirred at -20°C for 1 hr, treated with 1 mol/L hydrochloric acid, and extracted with ethyl acetate. The extract was washed successively with water, saturated aqueous sodium hydrogen carbonate solution and saturated brine, dried over anhydrous sodium sulfate, and concentrated under reduced pressure. The residue was purified by silica gel column chromatography (eluent: hexane-ethyl acetate=4:1→1:1) to give the title compound as a colorless oil (970 mg, yield 99%).
¹H-NMR(CDCl₃)δ: 2.03-2.08(1H,m),4.76(2H,d,J=6.0Hz),7.15(1H,s),7.33-7.37(1H,m),7.41-7.47(2H,m),7.52-7.56(2H,m),7.58-7.64(1H,m),7.85-7.91(1H,m),8.33-8.35(1H,m).

### Reference Example 335

### 1-(2-fluoropyridin-3-yl)-5-(phenylsulfonyl)-1H-pyrazole-3-carbaldehyde

[1-(2-Fluoropyridin-3-yl)-5-(phenylsulfonyl)-1H-pyrazol-3-yl]methanol (970 mg) was dissolved in toluene (10 mL), manganese dioxide (1.7 g) was added, and the mixture was stirred at 90°C for 1 hr. The reaction mixture was allowed to cool to room temperature, and filtered through celite. The filtrate was concentrated under reduced pressure, and the residue was purified by silica gel column chromatography (eluent: hexane-ethyl acetate=3:1→1:1) to give the title compound as a colorless oil (900 mg, yield 93%).
¹H-NMR(CDCl₃)δ: 7.40-7.50(3H,m),7.53-7.58(3H,m),7.63-7.68(1H,m),7.93-7,99(1H,m),8.40-8.43(1H,m),9.99(1H,s).

### Reference Example 336

### tert-butyl ({5-[(6-chloropyridin-3-yl)sulfonyl]-1-(2-fluorophenyl)-1H-pyrazol-3-yl}methyl)methylcarbamate

In the same manner as in Reference Example 268 and using tert-butyl ({5-[(6-chloropyridin-3-yl)thio]-1-(2-fluorophenyl)-1H-pyrazol-3-yl}methyl)methylcarbamate (1.17 g) and 3-chloroperbenzoic acid (1.93 g), the title compound was obtained as a white powder (1.13 g, yield 90%).
¹H-NMR(CDCl₃)δ: 1.50(9H,s),2.89(3H,brs),4.48(2H,brs),7.04-7.19(2H,m),7.23-7.46(3H,m),7.47-7.63(1H,m),7.75(1H,d,J=6.8Hz),8.36(1H,d,J=2.3Hz).

### Reference Example 337

### tert-butyl {[1-(2-fluorophenyl)-5-(pyridin-3-ylsulfonyl)-1H-pyrazol-3-yl]methyl}methylcarbamate

To a solution of tert-butyl ({5-[(6-chloropyridin-3-yl)sulfonyl]-1-(2-fluorophenyl)-1H-pyrazol-3-yl}methyl)methylcarbamate (250 mg) in methanol (5 mL) was added triethylamine (0.145 mL), 10% palladium-carbon (50% water-containing product, 50 mg) was added under a nitrogen atmosphere, and the mixture was stirred for 4 hr under a hydrogen atmosphere. The reaction mixture was filtered, and the filtrate was concentrated under reduced pressure to give a residue. The obtained residue was purified by silica gel column chromatography (eluent: hexane-ethyl acetate=7:3→ethyl acetate) to give the title compound as a colorless oil (199 mg, yield 86%).
¹H-NMR(CDCl₃)δ: 1.50(9H,s),2.89(3H,brs),4.48(2H,brs),7.02-7.19(2H,m),7.22-7.31(1H,m),7.32-7.42(2H,m),7.47-7.59(1H,m),7.81(1H,d,J=8.1Hz),8.62(1H,d,J=1.7Hz),8.79(1H,dd,J= 4.9,1.5Hz).

### Reference Example 338

### tert-butyl ({1-(2-fluorophenyl)-5-[(6-methylpyridin-3-yl)sulfonyl]-1H-pyrazol-3-yl}methyl)methylcarbamate

tert-Butyl ({5-[(6-chloropyridin-3-yl)sulfonyl]-1-(2-fluorophenyl)-1H-pyrazol-3-yl}methyl)methylcarbamate (170 mg), methylboronic acid (27.5 mg), potassium carbonate (146 mg) and tetrakis(triphenylphosphine) palladium(0) (41 mg) were suspended in 1,4-dioxane(4 mL), and the suspension was stirred at 80°C for 18 hr under a nitrogen atmosphere. The reaction mixture was allowed to cool to room temperature, anhydrous sodium sulfate and celite was added, and the mixture was stirred for 30 min. The insoluble material was filtered off, and the filtrate was concentrated under reduced pressure. The residue was purified by silica gel column chromatography (eluent: hexane-ethyl acetate=9:1→1:3) to give the title compound as a colorless oil (75 mg, yield 46%).
¹H-NMR(CDCl₃)δ: 1.50(9H,s),2.77(3H,s),2.89(3H,brs),4.47(2H,brs),7.03-7.14(2H,m),7.19(1H,d,J=8.3Hz),7.23-7.31(1H,m),7.33-7.43(1H,m),7.48-7.57(1H,m),7.63-7.73(1H,m),8.49(1H,d,J=2.3Hz).

### Reference Example 339

### tert-butyl ({1-(2-fluorophenyl)-5-[(6-methoxypyridin-3-yl)sulfonyl]-1H-pyrazol-3-yl}methyl)methylcarbamate

To a solution of tert-butyl ({5-[(6-chloropyridin-3-yl)sulfonyl]-1-(2-fluorophenyl)-1H-pyrazol-3-yl}methyl)methylcarbamate (72 mg) in methanol (1 mL) was added sodium methoxide (32.4 mg), and the mixture was stirred at room temperature for 0.5 hr, and concentrated under reduced pressure. The residue was purified by silica gel column chromatography (eluent: hexane-ethyl acetate=9:1→2:3) to give the title compound as a colorless oil (66.5 mg, yield 93%).
¹H-NMR(CDCl₃)δ: 1.57(9H,s),2.90(3H,brs),3.98(3H,s),4.40-4.51(2H,m),6.67-6.72(1H,m),6.99-7.15(2H,m),7.23-7.32(1H,m),7.35-7.43(1H,m),7.47-7.62(2H,m),8.21(1H,d,J=2.1Hz).

### Reference Example 340

### tert-butyl ({5-[(6-ethoxypyridin-3-yl)sulfonyl]-1-(2-fluorophenyl)-1H-pyrazol-3-yl}methyl)methylcarbamate

To a solution of tert-butyl ({5-[(6-chloropyridin-3-yl)sulfonyl]-1-(2-fluorophenyl)-1H-pyrazol-3-yl}methyl)methylcarbamate (110 mg) in ethanol (1.5 mL) was added sodium ethoxide (62.4 mg), and the mixture was stirred at room temperature for 18 hr, and concentrated under reduced pressure. The residue was purified by silica gel column chromatography (eluent: hexane-ethyl acetate=97:3→1:1) to give the title compound as a colorless oil (108 mg, yield 96%).
¹H-NMR (CDCl₃)δ: 1.40(3H,t,J=7.2Hz),1.54(9H,s),2.89(3H,brs),4.41(2H,q,J=7.1Hz), 4.48(2H,brs),6.66(1H,d,J=8.7Hz),6.97-7.14(2H,m),7.20-7.32(1H,m),7.34-7.44(1H,m),7.45-7.64(2H,m),8.17(1H,d,J=2.3Hz).

### Reference Example 341

### tert-butyl ({5-[(6-cyanopyridin-3-yl)sulfonyl]-1-(2-fluorophenyl)-1H-pyrazol-3-yl}methyl)methylcarbamate

To a mixture of tert-butyl ({5-[(6-chloropyridin-3-yl)sulfonyl]-1-(2-fluorophenyl)-1H-pyrazol-3-yl}methyl)methylcarbamate (120 mg) in a mixed solvent of dimethylsulfoxide (0.75 mL) and water (0.15 mL) were added sodium cyanide (14.7 mg) and 1,4-diazabicyclo[2.2.2]octane (5.6 mg), and the mixture was stirred at room temperature for 1 hr. The reaction mixture was diluted with water, and extracted with ethyl acetate. The extract was washed with saturated brine, dried over anhydrous magnesium sulfate, and concentrated under reduced pressure. The residue was purified by silica gel column chromatography (eluent: hexane-ethyl acetate=17:3→1:3) to give the title compound as a white powder (74 mg, yield 62%).
¹H-NMR(CDCl₃)δ: 1.50(9H,s),2.90(3H,brs),4.49(2H,brs),7.06-7.23(2H,m),7.27-7.44(2H,m),7.51-7.62(1H,m),7.74(1H,dd,J=8.2,0.8Hz),7.92-8.01(1H,m),8.68(1H,d,J=1.5Hz).

### Reference Example 342

### tert-butyl ({5-[(3-bromophenyl)sulfonyl]-1-(2-chlorophenyl)-1H-pyrazol-3-yl}methyl)methylcarbamate

To a solution of tert-butyl ({5-[(3-bromophenyl)thio]-1-(2-chlorophenyl)-1H-pyrazol-3-yl}methyl)methylcarbamate (523 mg) in ethyl acetate (6 mL) was added 3-chloroperbenzoic acid (838 mg) at 0°C, and the mixture was stirred at room temperature for 12 hr. Aqueous sodium thiosulfate solution was added to the reaction mixture, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=3:1) to give the title compound as a yellow oil (326 mg, yield 58%).
¹H-NMR (CDCl₃)δ: 1.51(9H,s),2.88(3H,brs),4.49(2H,brs),7.07(1H,brs),7.23-7.52(7H,m),7.66-7.69(1H,m).

### Reference Example 343

### tert-butyl ({1-(2-chlorophenyl)-5-[(3-cyanophenyl)sulfonyl]-1H-pyrazol-3-yl}methyl)methylcarbamate

A mixture of tert-butyl ({5-[(3-bromophenyl)sulfonyl]-1-(2-chlorophenyl)-1H-pyrazol-3-yl}methyl)methylcarbamate (318 mg), zinc cyanide (40 mg) and tetrakis(triphenylphosphine) palladium(0) (70 mg) was stirred in N,N-dimethylformamide (5 mL) at 100°C for 3 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=2:1) to give the title compound as a colorless solid (227 mg, yield 79%).
¹H-NMR(CDCl₃)δ: 1.51(9H,s),2.88(3H,brs),4.48(2H,brs),7.15(1H,brs),7.31-7.35(1H,m),7.45-7.56(5H,m),7.76-7.84(2H,m).

### Reference Example 344

### tert-butyl {[1-(2-chlorophenyl)-5-(pyridin-3-ylsulfonyl)-1H-pyrazol-3-yl]methyl}methylcarbamate

tert-Butyl ({1-(2-chlorophenyl)-5-[(pyridin-3-yl)thio]-1H-pyrazol-3-yl}methyl)methylcarbamate (190 mg) was suspended in a mixed solvent of acetonitrile (5 mL) and water (5 mL), sodium percarbonate (1.0 g) was added at room temperature, and the mixture was stirred for 2 hr. Sodium percarbonate (3.0 g) was added again, and the mixture was stirred for 3 hr. Acetonitrile was evaporated under reduced pressure, and the residue was extracted with ethyl acetate. The extract was washed successively with saturated aqueous sodium thiosulfate solution and saturated brine, dried over anhydrous sodium sulfate, and concentrated under reduced pressure. The residue was purified by basic silica gel column chromatography (eluent: hexane-ethyl acetate=9:1→4:1) to give the title compound as a colorless oil (123 mg, yield 60%).
¹H-NMR(CDCl₃) δ: 1.51(9H,s),2.88(3H,brs),4.50(2H,br),7.13(1H,br),7.30-7.34(2H,m),7.41-7.51(3H,m),7.73-7.76(1H,m),8.53-8.54(1H,m),8.76-8.78(1H,m).

### Reference Example 345

### tert-butyl ({1-(2-chlorophenyl)-5-[(5-fluoropyridin-3-yl)sulfonyl]-1H-pyrazol-3-yl}methyl)methylcarbamate

tert-Butyl ({1-(2-chlorophenyl)-5-[(5-fluoropyridin-3-yl)thio]-1H-pyrazol-3-yl}methyl)methylcarbamate (172 mg) was suspended in a mixed solvent of acetonitrile (5 mL) and water (10 mL), sodium percarbonate (3.6 g) was added at room temperature, and the mixture was stirred for 18 hr. Sodium percarbonate (7.2 g) was added again, and the mixture was stirred for 8 hr. Acetonitrile was evaporated under reduced pressure, and the residue was extracted with ethyl acetate. The extract was washed successively with saturated aqueous sodium thiosulfate solution and saturated brine, dried over anhydrous sodium sulfate, and concentrated under reduced pressure. The residue was purified by basic silica gel column chromatography (eluent: hexane-ethyl acetate=19:1→7:1) to give the title compound as a colorless oil (117 mg, yield 64%).
¹H-NMR (CDCl₃) δ: 1.51(9H,s),2.88(3H,s),4.50(2H,br),7.16(1H,br),7.32-7.36(2H,m),7.45-7.55(3H,m),8.41(1H,s),8.63-8.64(1H,m).

### Reference Example 346

### tert-butyl ({1-(2-chlorophenyl)-5-[(6-chloropyridin-3-yl)sulfonyl]-1H-pyrazol-3-yl}methyl)methylcarbamate

To a solution of tert-butyl ({1-(2-chlorophenyl)-5-[(6-chloropyridin-3-yl)thio]-1H-pyrazol-3-yl}methyl)methylcarbamate (1.32 g) in ethyl acetate (15 mL) was added 3-chloroperbenzoic acid (2.72 g). The mixture was stirred at room temperature for 2 hr, treated with saturated aqueous sodium thiosulfate solution, and extracted with ethyl acetate. The extract was washed successively with water, saturated aqueous sodium hydrogen carbonate solution and saturated brine, dried over anhydrous sodium sulfate, and concentrated under reduced pressure. The residue was purified by basic silica gel column chromatography (eluent: hexane-ethyl acetate=9:1→6:1) to give the title compound as a colorless amorphous solid (1.32 g, yield 62%).
¹H-NMR(CDCl₃)δ: 1.51(9H,s),2.88(3H,s),4.49(2H,s),7.14(1H,br),7.32-7.38(2H,m),7.42-7.54(3H,m),7.66-7.70(1H,m),8.26(1H,d,J=2.4Hz).

### Reference Example 347

### tert-butyl ({1-(2-chlorophenyl)-5-[(6-methylpyridin-3-yl)sulfonyl]-1H-pyrazol-3-yl}methyl)methylcarbamate

tert-Butyl ({1-(2-chlorophenyl)-5-[(6-chloropyridin-3-yl)sulfonyl]-1H-pyrazol-3-yl}methyl)methylcarbamate (414 mg), trimethylboroxin (104 mg), potassium carbonate (173 mg) and tetrakis(triphenylphosphine) palladium(0) (96.1 mg) were suspended in tetrahydrofuran (10 mL), and the suspension was refluxed for 72 hr. The reaction mixture was allowed to cool to room temperature, water was added, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=9:1→3:1) to give the title compound as a colorless oil (76.6 mg, yield 19%).
¹H-NMR(CDCl₃)δ: 1.51(9H,s),2.62(3H,s),2.87(3H,br),4.49(2H,br),7.08(1H,br),7.16 (1H,d,J=8.4Hz),7.32-7.35(1H,m),7.40-7.52(3H,m),7.60-7.64(1H,m),8.40(1H,d,J=2.7Hz).

### Reference Example 348

### tert-butyl ({1-(2-chlorophenyl)-5-[(6-methoxypyridin-3-yl)sulfonyl]-1H-pyrazol-3-yl}methyl)methylcarbamate

To a solution of tert-butyl ({1-(2-chlorophenyl)-5-[(6-chloropyridin-3-yl)sulfonyl]-1H-pyrazol-3-yl}methyl)methylcarbamate (209 mg) in methanol (2 mL) was added 28% sodium methoxide-methanol solution (2 mL) at room temperature, and the mixture was stirred for 1 hr. The solvent was evaporated under reduced pressure. Water was added to the residue, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=7:1→3:1) to give the title compound as a colorless oil (204 mg, yield 98%).
¹H-NMR(CDCl₃) δ: 1.51(9H,s),2.87(3H,brs),3.97(3H,s),4.49(2H,brs),6.64-6.68(1H,m),7.05(1H,br),7.32-7.36(1H,m),7.39-7.54(4H,m),8.12(1H,d,J=2.4Hz).

### Reference Example 349

### tert-butyl {[1-(2-chlorophenyl)-5-(pyridin-4-ylsulfonyl)-1H-pyrazol-3-yl]methyl}methylcarbamate

tert-Butyl ({1-(2-chlorophenyl)-5-[(pyridin-4-yl)thio]-1H-pyrazol-3-yl}methyl)methylcarbamate (279 mg) was suspended in a mixed solvent of acetonitrile (5 mL) and water (5 mL), sodium percarbonate (6.1 g) was added at room temperature, and the mixture was stirred for 4 hr. Sodium percarbonate (6.1 g) was added again, and the mixture was stirred for 18 hr. Acetonitrile was evaporated under reduced pressure, and the residue was extracted with ethyl acetate. The extract was washed successively with saturated aqueous sodium thiosulfate solution and saturated brine, dried over anhydrous sodium sulfate, and concentrated under reduced pressure. The residue was purified by basic silica gel column chromatography (eluent: hexane-ethyl acetate=4:1→1:1) to give the title compound as a colorless oil (152 mg, yield 51%).
¹H-NMR(CDCl₃)δ: 1.51(9H,s),2.88(3H,br),4.50(2H,br),7.16(1H,br),7.26-7.35(3H,m),7.39-7.51(3H,m),8.69-8.71(2H,m).

### Reference Example 350

### tert-butyl ({1-(2-chlorophenyl)-5-[(2-methylpyridin-4-yl)sulfonyl]-lH-pyrazol-3-yl}methyl)methylcarbamate

tert-Butyl ({1-(2-chlorophenyl)-5-[(2-methylpyridin-4-yl)thio]-1H-pyrazol-3-yl}methyl)methylcarbamate (78 mg) was suspended in a mixed solvent of acetonitrile (5 mL) and water (5 mL), sodium percarbonate (1.7 g) was added at room temperature, and the mixture was stirred for 4 hr. Sodium percarbonate (1.7 g) was added again, and the mixture was stirred for 18 hr. Acetonitrile was evaporated under reduced pressure, and the residue was extracted with ethyl acetate. The extract was washed successively with saturated aqueous sodium thiosulfate solution and saturated brine, dried over anhydrous sodium sulfate, and concentrated under reduced pressure. The residue was purified by basic silica gel column chromatography (eluent: hexane-ethyl acetate=4:1→2:1) to give the title compound as a colorless oil (60 mg, yield 71%).
¹H-NMR(CDCl₃)δ: 1.51(9H,s),2.54(3H,s),2.88(3H,brs),4.49(2H,brs),7.07-7.15(3H,m),7.32-7.35(1H,m),7.42-7.50(3H,m),8.56(1H,d,J=5.1Hz).

### Reference Example 351

### tert-butyl ({1-(2-chlorophenyl)-5-[(2-methoxypyridin-4-yl)sulfonyl]-1H-pyrazol-3-yl}methyl)methylcarbamate

To a solution of tert-butyl ({1-(2-chlorophenyl)-5-[(2-methoxypyridin-4-yl)thio]-1H-pyrazol-3-yl}methyl)methylcarbamate (237 mg) in ethyl acetate (10 mL) was added 3-chloroperbenzoic acid (494 mg). The mixture was stirred at room temperature for 48 hr, treated with saturated aqueous sodium thiosulfate solution, and extracted with ethyl acetate. The extract was washed successively with water, saturated aqueous sodium hydrogen carbonate solution and saturated brine, dried over anhydrous sodium sulfate, and concentrated under reduced pressure. The residue was purified by basic silica gel column chromatography (eluent: hexane-ethyl acetate=9:1→4:1) to give the title compound as a colorless oil (195 mg, yield 77%).
¹H-NMR(CDCl₃)δ: 1.51(9H,s),2.89(3H,br),3.94(3H,s),4.49(2H,br),6.66(1H,brs),6.8 9-6.91(1H,m),7.12(1H,br),7.28-7.49(4H,m),8.20(1H,d,J=6.0Hz).

### Reference Example 352

### tert-butyl {[1-(2-chlorophenyl)-5-(pyridin-2-ylsulfonyl)-1H-pyrazol-3-yl]methyl}methylcarbamate

To a solution of tert-butyl ({1-(2-chlorophenyl)-5-[(pyridin-2-yl)thio]-1H-pyrazol-3-yl}methyl)methylcarbamate (124 mg) in ethyl acetate (3 mL) was added 3-chloroperbenzoic acid (213 mg) at 0°C, and the mixture was stirred at room temperature for 2 days. Aqueous sodium thiosulfate solution was added to the reaction mixture, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=1:1) to give the title compound as a yellow oil (106 mg, yield 79%).
¹H-NMR (CDCl₃) δ: 1.50(9H,s),2.89(3H,brs),4.46(2H,brs),7.20(1H,s),7.29-7.49(5H,m),7.56-7.60(1H,m),7.72-7.78(1H,m),8.65-8.67(1H,m).

### Reference Example 353

### tert-butyl ({1-(2-chlorophenyl)-5-[(6-methylpyridin-2-yl)sulfonyl]-1H-pyrazol-3-yl}methyl)methylcarbamate

To a solution of tert-butyl ({1-(2-chlorophenyl)-5-[(6-methylpyridin-2-yl)thio]-1H-pyrazol-3-yl}methyl)methylcarbamate (459 mg) in ethyl acetate (5 mL) was added 3-chloroperbenzoic acid (747 mg) at 0°C, and the mixture was stirred at room temperature for 16 hr. Aqueous sodium thiosulfate solution was added to the reaction mixture, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=1:1) to give the title compound as a yellow oil (381 mg, yield 78%). ¹H-NMR(CDCl₃) δ: 1.50(9H,s), 2.56(3H,s) ,2.80-2.90(3H,m),4.64(2H,brs),7.16(1H,brs),7.27-7.49(6H,m),7.57-7.63(1H,m).

### Reference Example 354

### tert-butyl ({1-(2-chlorophenyl)-5-[(5-methylpyridin-2-yl)sulfonyl]-1H-pyrazol-3-yl}methyl)methylcarbamate

To a solution of tert-butyl ({1-(2-chlorophenyl)-5-[(5-methylpyridin-2-yl)thio]-1H-pyrazol-3-yl}methyl)methylcarbamate (654 mg) in ethyl acetate (6 mL) was added 3-chloroperbenzoic acid (904 mg) at 0°C, and the mixture was stirred at room temperature for 14 hr. Aqueous sodium thiosulfate solution was added to the reaction mixture, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by basic silica gel column chromatography (eluent: hexane-ethyl acetate=2:1) to give the title compound as a yellow oil (424 mg, yield 72%).
¹H-NMR (CDCl₃) δ: 1.50(9H,s),2,42(3H,s),2.88(3H,brs),4.46(2H,brs),7.16(1H,brs),7 .25-7.53(6H,m),8.47(1H,s).

### Reference Example 355

### tert-butyl ({1-(2-chlorophenyl)-5-[(6-methoxypyridin-2-yl)sulfonyl]-1H-pyrazol-3-yl}methyl)methylcarbamate

To a solution of tert-butyl ({1-(2-chlorophenyl)-5-[(6-methoxypyridin-2-yl)thio]-1H-pyrazol-3-yl}methyl)methylcarbamate (412 mg) in ethyl acetate (5 mL) was added 3-chloroperbenzoic acid (649 mg) at 0°C, and the mixture was stirred at room temperature for 14 hr. Aqueous sodium thiosulfate solution was added to the reaction mixture, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=2:1) to give the title compound as a yellow oil (406 mg, yield 92%).
¹H-NMR(CDCl₃)δ: 1.50(9H,s),2.89(3H,brs),3.85(3H,s),4.52(2H,brs),6.86-6.89(1H,m),7.13-7.25(2H,m),7.28-7.41(4H,m),7.52-7.57(1H,m).

### Reference Example 356

### tert-butyl ({5-[(6-chloropyridin-3-yl)sulfonyl]-1-(2,3-difluorophenyl)-1H-pyrazol-3-yl}methyl)methylcarbamate

To a solution of tert-butyl ({5-[(6-chloropyridin-3-yl)thio]-1-(2,3-difluorophenyl)-1H-pyrazol-3-yl}methyl)methylcarbamate (475 mg) in ethyl acetate (10 mL) was added 3-chloroperbenzoic acid (978 mg). The mixture was stirred at room temperature for 18 hr, treated with saturated aqueous sodium thiosulfate solution, and extracted with ethyl acetate. The extract was washed successively with water, saturated aqueous sodium hydrogen carbonate solution and saturated brine, dried over anhydrous sodium sulfate, and concentrated under reduced pressure. The residue was purified by silica gel column chromatography (eluent: hexane-ethyl acetate=4:1→2:1) to give the title compound as a colorless oil (488 mg, yield 96%).
¹H-NMR(CDCl₃) δ: 1.49(9H,s),2.89(3H,brs),4.46(2H,br),7.03-7.28(3H,m),7.33-7.43(2H,m),7.77-7.81(1H,m),8.43-8.44(1H,m).

### Reference Example 357

### tert-butyl {[1-(2,3-difluorophenyl)-5-(pyridin-3-ylsulfonyl)-1H-pyrazol-3-yl]methyl}methylcarbamate

To a solution of tert-butyl ({5-[(6-chloropyridin-3-yl)sulfonyl]-1-(2,3-difluorophenyl)-1H-pyrazol-3-yl}methyl)methylcarbamate (222 mg) and triethylamine (0.1 mL) in a mixed solvent of ethanol (3 mL) and tetrahydrofuran (3 mL) was added 10% palladium-carbon (50% water-containing product, 25 mg). The mixture was stirred at room temperature for 1 hr under a hydrogen atmosphere, the insoluble material was filtered off, and the filtrate was concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure to give the title compound as a yellow oil (210 mg, yield quantitative).
¹H-NMR(CDCl₃) δ: 1.49(9H,brs),2.89(3H,brs),4.46(2H,brs),7.11-7.24(3H,m),7.32-7.42(2H,m),7.82-7.85(1H,m),8.67-8.68(1H,m),8.80-8.82(1H,m).

### Reference Example 358

### tert-butyl ({5-[(6-methylpyridin-3-yl)sulfonyl]-1-(2,3-difluorophenyl)-1H-pyrazol-3-yl}methyl)methylcarbamate

tert-Butyl ({5-[(6-chloropyridin-3-yl)sulfonyl]-1-(2,3-difluorophenyl)-1H-pyrazol-3-yl}methyl)methylcarbamate (488 mg), potassium carbonate (162 mg) and methylboronic acid (176 mg) were suspended in cyclopentylmethyl ether (5 mL), and the suspension was degassed under an argon atmosphere. Tetrakistriphenylphosphine palladium (0) (113 mg) was added, and the mixture was further degassed. The mixture was stirred at 110°C for 1.5 hr, and allowed to cool to room temperature. Water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=4:1→2:1) to give the title compound as a colorless oil (71 mg, yield 15%).
¹H-NMR(CDCl₃) δ: 1.49(9H,s),2.64(3H,s),2.88(3H,brs),4.45(2H,br),7.08(1H,br),7.1 6-7.38(4H,m),7.67-7.73(1H,m),8.52-8.53(1H,m).

### Reference Example 359

### tert-butyl ({5-[(6-chloropyridin-3-yl)sulfonyl]-1-(2,4-difluorophenyl)-1H-pyrazol-3-yl}methyl)methylcarbamate

To a solution of tert-butyl ({5-[(6-chloropyridin-3-yl)thio]-1-(2,4-difluorophenyl)-1H-pyrazol-3-yl}methyl)methylcarbamate (328 mg) in ethyl acetate (10 mL) was added 3-chloroperbenzoic acid (674 mg). The mixture was stirred at room temperature for 18 hr, treated with saturated aqueous sodium thiosulfate solution, and extracted with ethyl acetate. The extract was washed successively with water, saturated aqueous sodium hydrogen carbonate solution and saturated brine, dried over anhydrous sodium sulfate, and concentrated under reduced pressure. The residue was purified by silica gel column chromatography (eluent: hexane-ethyl acetate=9:1→4:1) to give the title compound as a colorless oil (340 mg, yield 97%).
¹H-NMR(CDCl₃)δ: 1.49(9H,s),2.89(3H,brs),4.45(2H,br),6.86-6.93(1H,m),6.99-7.13(2H,m),7.33-7.43(2H,m),7.73-7.81(1H,m),8.43-8.45(1H,m).

### Reference Example 360

### tert-butyl ({5-[(6-methylpyridin-3-yl)sulfonyl]-1-(2,4-difluorophenyl)-1H-pyrazol-3-yl}methyl)methylcarbamate

tert-Butyl ({5-[(6-chloropyridin-3-yl)sulfonyl]-1-(2,4-difluorophenyl)-1H-pyrazol-3-yl}methyl)methylcarbamate (340 mg), potassium carbonate (141 mg) and methylboronic acid (204 mg) were suspended in cyclopentylmethyl ether (5 mL), and the suspension was degassed under an argon atmosphere. Tetrakistriphenylphosphine palladium (0) (79 mg) was added, and the mixture was further degassed. The mixture was stirred at 110°C for 2 hr, and allowed to cool to room temperature. Water was added, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=9:1→4:1) to give the title compound as a colorless oil (129 mg, yield 39%).
¹H-NMR(CDCl₃) δ: 1.49(9H,s),2.64(3H,s),2.88(3H,brs),4.44(2H,br),6.82-6.89(1H,m),6.97-7.10(2H,m),7.21-7.25(1H,m),7.32-7.39(1H,m),7.70-7.73(1H,m),8.55-8.56(1H,m).

### Reference Example 361

### tert-butyl ({5-[(3-bromophenyl)sulfonyl]-1-(2,5-difluorophenyl)-1H-pyrazol-3-yl}methyl)methylcarbamate

To a solution of tert-butyl ({5-[(3-bromophenyl)thio]-1-(2,5-difluorophenyl)-1H-pyrazol-3-yl}methyl)methylcarbamate (1.52 g) in ethyl acetate (15 mL) was added 3-chloroperbenzoic acid (2.74 g) at 0°C, and the mixture was stirred at room temperature for 14 hr. Aqueous sodium thiosulfate solution was added to the reaction mixture, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=4:1) to give the title compound as a yellow oil (1.38 g, yield 89%).
¹H-NMR(CDCl₃)δ: 1.49(9H,brs),2.89(3H,brs),4.46(2H,brs),7.03-7.11(3H,m),7.19-7.28(1H,m),7.30-7.35(1H,m),7.54-7.60(2H,m),7.71-7.73(1H,m).

### Reference Example 362

### tert-butyl {[1-(2,5-difluorophenyl)-5-(phenylsulfonyl)-1H-pyrazol-3-yl]methyl}methylcarbamate

To a solution of tert-butyl ({5-[(3-bromophenyl)sulfonyl]-1-(2,5-difluorophenyl)-1H-pyrazol-3-yl}methyl)methylcarbamate (747 mg) and triethylamine (0.2 mL) in ethanol (10 mL) was added 10% palladium-carbon (50% water-containing product, 81 mg), and the mixture was stirred at room temperature for 14 hr under a hydrogen atmosphere. The insoluble material was filtered off, and the filtrate was concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=4:1) to give the title compound as a yellow oil (572 mg, yield 89%).
¹H-NMR(CDCl₃)δ: 1.48(9H,brs),2.88(3H,brs),4.43(2H,brs),6.97-7.06(3H,m),7.14-7.22(1H,m),7.40-7.45(2H,m),7.56-7.62(3H,m).

### Reference Example 363

### tert-butyl ({5-[(3-cyanophenyl)sulfonyl]-1-(2,5-difluorophenyl)-1H-pyrazol-3-yl}methyl)methylcarbamate

A mixture of tert-butyl ({5-[(3-bromophenyl)sulfonyl]-1-(2,5-difluorophenyl)-1H-pyrazol-3-yl}methyl)methylcarbamate (578 mg), zinc cyanide (68 mg) and tetrakis(triphenylphosphine) palladium(0) (123 mg) was stirred in N,N-dimethylformamide (6 mL) at 100°C for 3 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=2:1) to give the title compound as a yellow oil (462 mg, yield 88%).
¹H-NMR(CDCl₃)δ: 1.49(9H,brs),2.89(3H,brs),4.46(2H,brs),7.04-7.13(3H,m),7.25-7.30(1H,m),7.58-7.63(1H,m),7.73(1H,s),7.83-7.88(2H,m).

### Reference Example 364

### tert-butyl ({5-[(6-chloropyridin-3-yl)sulfonyl]-1-(2,5-difluorophenyl)-1H-pyrazol-3-yl}methyl)methylcarbamate

To a solution of tert-butyl ({5-[(6-chloropyridin-3-yl)thiol-1-(2,5-difluorophenyl)-1H-pyrazol-3-yl}methyl)methylcarbamate (530 mg) in ethyl acetate (10 mL) was added 3-chloroperbenzoic acid (1.1 g). The mixture was stirred at room temperature for 18 hr, treated with saturated aqueous sodium thiosulfate solution, and extracted with ethyl acetate. The extract was washed successively with water, saturated aqueous sodium hydrogen carbonate solution and saturated brine, dried over anhydrous sodium sulfate, and concentrated under reduced pressure. The residue was purified by silica gel column chromatography (eluent: hexane-ethyl acetate=4:1→2:1) to give the title compound as a colorless oil (529 mg, yield 93%).
¹H-NMR(CDCl₃)δ: 1.49(9H,s),2.89(3H,brs),4.45(2H,br),7.06-7.30(4H,m),7.40-7.43(1H,m),7.81-7.83(1H,m),8.47-8.48(1H,m).

### Reference Example 365

### tert-butyl {[1-(2,5-difluorophenyl)-5-(pyridin-3-ylsulfonyl)-1H-pyrazol-3-yl]methyl}methylcarbamate

To a solution of tert-butyl ({5-[(6-chloropyridin-3-yl)sulfonyl]-1-(2,5-difluorophenyl)-1H-pyrazol-3-yl}methyl)methylcarbamate (272 mg) and triethylamine (0.1 mL) in a mixed solvent of ethanol (5 mL) and tetrahydrofuran (5 mL) was added 10% palladium-carbon (50% water-containing product, 38 mg), and the mixture was stirred at room temperature for 1 hr under a hydrogen atmosphere. The insoluble material was filtered off, and the filtrate was concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was dissolved in toluene (10 mL), manganese dioxide (530 mg) was added, and the mixture was stirred at 90°C for 1 hr. The reaction mixture was allowed to cool to room temperature, and filtered, and the filtrate was concentrated under reduced pressure to give the title compound as a yellow oil (218 mg, yield 85%).
¹H-NMR(CDCl₃)δ: 1.49(9H,brs),2.89(3H,brs),4.46(2H,brs),7.02-7.27(4H,m),7.37-7.42(1H,m),7.86-7.89(1H,m),8.71-8.72(1H,m),8.80-8.82(1H,m).

### Reference Example 366

### tert-butyl ({5-[(6-methylpyridin-3-yl)sulfonyl]-1-(2,5-difluorophenyl)-1H-pyrazol-3-yl}methyl)methylcarbamate

tert-Butyl ({5-[(6-chloropyridin-3-yl)sulfonyl]-1-(2,5-difluorophenyl)-1H-pyrazol-3-yl}methyl)methylcarbamate (529 mg), potassium carbonate (176 mg) and methylboronic acid (317 mg) were suspended in a mixed solvent of cyclopentylmethyl ether (10 mL) and tetrahydrofuran (5 mL), and the suspension was degassed under an argon atmosphere. Tetrakistriphenylphosphine palladium (0) (123 mg) was added, and the mixture was futher degassed. The mixture was stirred at 110°C for 1.5 hr, and allowed to cool to room temperature. Water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=4:1→2:1) and basic silica gel column chromatography (eluent: hexane-ethyl acetate=9:1→4:1) to give the title compound as a colorless oil (154 mg, yield 30%).
¹H-NMR(CDCl₃)δ: 1.49(9H,s),2.64(3H,s),2.88(3H,brs),4.46(2H,br),7.00-7.11(3H,m),7.19-7.25(2H,m),7.73-7.77(1H,m),8.58-8.59(1H,m).

### Reference Example 367

### tert-butyl ({5-[(6-chloropyridin-3-yl)sulfonyl]-1-(2-fluoro-3-methylphenyl)-1H-pyrazol-3-yl}methyl)methylcarbamate

To a solution of tert-butyl ({5-[(6-chloropyridin-3-yl)thio]-1-(2-fluoro-3-methylphenyl)-1H-pyrazol-3-yl}methyl)methylcarbamate (1.0 g) in ethyl acetate (15 mL) was added 3-chloroperbenzoic acid (2.1 g). The mixture was stirred at room temperature for 18 hr, treated with saturated aqueous sodium thiosulfate solution, and extracted with ethyl acetate. The extract was washed successively with water, saturated aqueous sodium hydrogen carbonate solution and saturated brine, dried over anhydrous sodium sulfate, and concentrated under reduced pressure. The residue was purified by silica gel column chromatography (eluent: hexane-ethyl acetate=4:1→2:1) to give the title compound as colorless crystals (1.0 g, yield 94%).
¹H-NMR(CDCl₃)δ: 1.51(9H,s),2.18-2.19(3H,m),2.89(3H,brs),4.48(2H,br),7.10-7.25(3H,m),7.33-7.39(2H,m),7.71-7.74(1H,m),8.26-8.27(1H,m).

### Reference Example 368

### tert-butyl ({5-[(6-methylpyridin-3-yl)sulfonyl]-1-(2-fluoro-3-methylphenyl)-1H-pyrazol-3-yl}methyl)methylcarbamate

tert-Butyl ({5-[(6-chloropyridin-3-yl)sulfonyl]-1-(2-fluoro-3-methylphenyl)-1H-pyrazol-3-yl}methyl)methylcarbamate (1.0 g), potassium carbonate (335 mg) and methylboronic acid (1.2 g) were suspended in cyclopentylmethyl ether (15 mL), and the suspension was degassed under an argon atmosphere. Tetrakis(triphenylphosphine) palladium(0) (233 mg) was added, and the mixture was futher degassed. The mixture was stirred at 110°C for 1.5 hr, and allowed to cool to room temperature. Water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate =6:1→3:1) and basic silica gel column chromatography (eluent: hexane-ethyl acetate=9:1→4:1) to give the title compound as a colorless oil (503 mg, yield 52%).
¹H-NMR(CDCl₃)δ: 1.50(9H,s),2.15-2.16(3H,m),2.63(3H,s),2.88(3H,brs),4.48(2H,br),7.05-7.23(4H,m),7.27-7.37(1H,m),7.66-7.68(1H,m),8.39-8.40(1H,m).

### Reference Example 369

### tert-butyl ({5-[(6-chloropyridin-3-yl)sulfonyl]-1-(2-fluoro-4-methylphenyl)-1H-pyrazol-3-yl}methyl)methylcarbamate

To a solution of tert-butyl ({5-[(6-chloropyridin-3-yl)thio]-1-(2-fluoro-4-methylphenyl)-1H-pyrazol-3-yl}methyl)methylcarbamate (882 mg) in ethyl acetate (15 mL) was added 3-chloroperbenzoic acid (1.8 g). The mixture was stirred at room temperature for 18 hr, treated with saturated aqueous sodium thiosulfate solution, and extracted with ethyl acetate. The extract was washed successively with water, saturated aqueous sodium hydrogen carbonate solution and saturated brine, dried over anhydrous sodium sulfate, and concentrated under reduced pressure. The residue was purified by silica gel column chromatography (eluent: hexane-ethyl acetate=9:1→4:1) to give the title compound as a colorless amorphous solid (837 mg, yield 89%).
¹H-NMR (CDCl₃) δ: 1.49(9H,s),2.45(3H,s),2.88(3H,brs),4.46(2H,br),6.90-6.93(1H,m),7.05-7.07(2H,m),7.19-7.26(1H,m),7.36-7.39(1H,m),7.75-7.78(1H,m),8.38-8.39(1H,m).

### Reference Example 370

### tert-butyl {[1-(2-fluoro-4-methylphenyl)-5-(pyridin-3-ylsulfonyl)-1H-pyrazol-3-yl]methyl}methylcarbamate

To a solution of tert-butyl ({5-[(6-chloropyridin-3-yl)sulfonyl]-1-(2-fluoro-4-methylphenyl)-1H-pyrazol-3-yl}methyl)methylcarbamate (178 mg) and triethylamine (0.08 mL) in ethanol (4 mL) was added 10% palladium-carbon (50% water-containing product, 29 mg), and the mixture was stirred at room temperature for 1 hr under a hydrogen atmosphere. The insoluble material was filtered off, and the filtrate was concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was dissolved in toluene (3 mL), manganese dioxide (40 mg) was added, and the mixture was stirred at 80°C for 1 hr. The reaction mixture was allowed to cool to room temperature, and filtered, and the filtrate was concentrated under reduced pressure to give the title compound as a yellow oil (155 mg, yield 93%).
¹H-NMR(CDCl₃) δ: 1.49(9H,brs),2.44(3H,s),2.88(3H,brs),4.45(2H,brs),6.86-6.89(1H,m),7.03-7.06(2H,m),7.18-7.23(1H,m),7.34-7.38(1H,m),7.81-7.84(1H,m),8.63-8.64(1H,m),8.77-8.80(1H,m).

### Reference Example 371

### tert-butyl ({5-[(6-methylpyridin-3-yl)sulfonyl]-1-(2-fluoro-4-methylphenyl)-1H-pyrazol-3-yl}methyl)methylcarbamate

tert-Butyl ({5-[(6-chloropyridin-3-yl)sulfonyl]-1-(2-fluoro-4-methylphenyl)-1H-pyrazol-3-yl}methyl)methylcarbamate (838 mg), potassium carbonate (280 mg) and methylboronic acid (1.0 g) were suspended in cyclopentylmethyl ether (15 mL), and the suspension was degassed under an argon atmosphere. Tetrakis(triphenylphosphine) palladium(0) (113 mg) was added, and the mixture was futher degassed. The mixture was stirred at 100°C for 1 hr, and allowed to cool to room temperature. Water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=6:1→3:1) and basic silica gel column chromatography (eluent: hexane-ethyl acetate=9:1→4:1) to give the title compound as a colorless oil (417 mg, yield 52%).
¹H-NMR(CDCl₃)δ: 1.49(9H,s),2.44(3H,s),2.64(3H,s),2.87(3H,brs),4.46(2H,br),6.88 -6.91(1H,m),7.03-7.05(2H,m),7.17-7.23(2H,m),7.69-7.71(1H,m),8.51-8.52(1H,m).

### Reference Example 372

### tert-butyl ({5-[(6-chloropyridin-3-yl)sulfonyl]-1-(2-fluoro-5-methylphenyl)-1H-pyrazol-3-yl}methyl)methylcarbamate

To a solution of tert-butyl ({5-[(6-chloropyridin-3-yl)thiol-1-(2-fluoro-5-methylphenyl)-1H-pyrazol-3-yl}methyl)methylcarbamate (978 mg) in ethyl acetate (10 mL) was added 3-chloroperbenzoic acid (2.0 g). The mixture was stirred at room temperature for 18 hr, treated with saturated aqueous sodium thiosulfate solution, and extracted with ethyl acetate. The extract was washed successively with water, saturated aqueous sodium hydrogen carbonate solution and saturated brine, dried over anhydrous sodium sulfate, and concentrated under reduced pressure. The residue was purified by silica gel column chromatography (eluent: hexane-ethyl acetate=4:1→2:1) to give the title compound as colorless crystals (938 mg, yield 90%).
¹H-NMR(CDCl₃)δ: 1.50(9H,s),2.37(3H,s),2.89(3H,brs),4.47(2H,br),6.96-7.02(1H,m),7.07-7.15(2H,m),7.28-7.39(2H,m),7.75-7.77(1H,m),8.36-8.37(1H,m).

### Reference Example 373

### tert-butyl ({5-[(6-methylpyridin-3-yl)sulfonyl]-1-(2-fluoro-5-methylphenyl)-1H-pyrazol-3-yl}methyl)methylcarbamate

tert-Butyl ({5-[(6-chloropyridin-3-yl)sulfonyl]-1-(2-fluoro-5-methylphenyl)-1H-pyrazol-3-yl}methyl)methylcarbamate (938 mg), potassium carbonate (314 mg) and methylboronic acid (1.1 g) were suspended in cyclopentylmethyl ether (15 mL), and the suspension was degassed under an argon atmosphere. Tetrakis(triphenylphosphine) palladium(0) (218 mg) was added, and the mixture was futher degassed. The mixture was stirred at 100°C for 3 hr, and allowed to cool to room temperature. Water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=4:1→>1:1) and basic silica gel column chromatography (eluent: hexane-ethyl acetate=9:1→4:1) to give the title compound as a colorless oil (396 mg, yield 44%).
¹H-NMR(CDCl₃)δ: 1.50(9H,s),2.35(3H,s),2.63(3H,s),2.88(3H,brs),4.46(2H,br),6.96 (1H,t,J=8.4Hz),7.04-7.07(2H,m),7.19(1H,d,J=8.4Hz),7.26-7.31(1H,m),7.67-7.71(1H,m),8.48-8.49(1H,m).

### Reference Example 374

### tert-butyl ({5-[(6-chloropyridin-3-yl)sulfonyl]-1-(3-fluoro-2-methylphenyl)-1H-pyrazol-3-yl}methyl)methylcarbamate

To a solution of tert-butyl ({5-[(6-chloropyridin-3-yl)thio]-1-(3-fluoro-2-methylphenyl)-1H-pyrazol-3-yl}methyl)methylcarbamate (2.84 g) in ethyl acetate (25 mL) was added 3-chloroperbenzoic acid (6.04 g), and the mixture was stirred at room temperature for 2.5 hr. Saturated aqueous sodium thiosulfate solution was added, and the mixture was further stirred for 1 hr, and extracted with ethyl acetate. The separated aqueous layer was extracted again with ethyl acetate, and the combined organic layers were washed with saturated brine, dried over anhydrous sodium sulfate, and concentrated under reduced pressure. The residue was purified by silica gel column chromatography (eluent: hexane-ethyl acetate=9:1→7:3) to give the title compound as a colorless oil (2.57 g, yield 85%).
¹H-NMR(CDCl₃)δ: 1.51(9H,s),1.59(3H,d,J=2.3Hz),2.89(3H,s),4.47(2H,brs),6.84-6.95(1H,m),7.12(1H,brs),7.20-7.27(2H,m),7.35(1H,d,J=8.3Hz),7.64(1H,dd,J=8.3,2.3Hz),8.36(1H, d,J=2.3Hz).

### Reference Example 375

### tert-butyl {[1-(3-fluoro-2-methylphenyl)-5-(pyridin-3-ylsulfonyl)-1H-pyrazol-3-yl]methyl}methylcarbamate

tert-Butyl ({1-(3-fluoro-2-methylphenyl)-5-[(6-methylpyridin-3-yl)sulfonyl]-1H-pyrazol-3-yl}methyl)methylcarbamate (909 mg) and triethylamine (372 mg) were dissolved in ethanol (10 mL), and the solution was stirred for 4 hr under a hydrogen atmosphere (1 atom). The reaction mixture was filtered, and the filtrate was concentrated under reduced pressure. Water was added to the residue, and the mixture was extracted with ethyl acetate. The separated aqueous layer was extracted again with ethyl acetate. The combined organic layers were washed with saturated brine, dried over anhydrous sodium sulfate, and concentrated under reduced pressure. The residue was purified by basic silica gel column chromatography (eluent: hexane-ethyl acetate=17:3→2:1) to give the title compound as a colorless oil (719 mg, yield 85%).
¹H-NMR(CDCl₃)δ: 1.48(3H,d,J=2.1Hz),1.51(9H,s),2.89(3H,brs),4.47(2H,d,J=9.4Hz), 6.86-7.00(1H,m),7.13(1H,d,J=4.0Hz),7.19-7.26(2H,m),7.33(1H,ddd,J=8.1,4.9,0.8Hz),7.69(1H,d,J=8.1Hz),8.6 2(1H,dd,J=2.4,0.7Hz),8.80(1H,dd,J=4.8,1.6Hz).

### Reference Example 376

### tert-butyl ({1-(3-fluoro-2-methylphenyl)-5-[(6-methylpyridin-3-yl)sulfonyl]-1H-pyrazol-3-yl}methyl)methylcarbamate

To a solution of tert-butyl ({5-[(6-chloropyridin-3-yl)sulfonyl]-1-(3-fluoro-2-methylphenyl)-1H-pyrazol-3-yl}methyl)methylcarbamate (1.65 g) in tetrahydrofuran (16 mL) were added [1,3-bis(diphenylphosphino)propane]dichloronickel (II) (181 mg) and methylmagnesium bromide-diethyl ether solution (5.5 mL) under ice-cooling, and the mixture was stirred at room temperature for 2 hr. Water was added to the reaction mixture, and the mixture was extracted with ethyl acetate. The separated aqueous layer was extracted again with ethyl acetate. The combined organic layers were washed with saturated brine, dried over anhydrous sodium sulfate, and concentrated under reduced pressure. The residue was purified by silica gel column chromatography (eluent: hexane-ethyl acetate=19:1→17:3) and basic silica gel column chromatography (eluent: hexane-ethyl acetate=19:1→17:3) to give the title compound as a pale-yellow oil (916 mg, yield 58%).
¹H-NMR(CDCl₃)δ: 1.46-1.55(12H,m),2.63(3H,s),2.88(3H,brs),4.47(2H,brs),6.90-6.99(1H,m),7.09(1H,brs),7.13-7.26(3H,m),7.56(1H,dd,J=8.3,2.3Hz).

### Reference Example 377

### tert-butyl ({5-[(6-chloropyridin-3-yl)sulfonyl]-1-(5-fluoro-2-methylphenyl)-1H-pyrazol-3-yl}methyl)methylcarbamate

To a solution of tert-butyl ({5-[(6-chloropyridin-3-yl)thio]-1-(5-fluoro-2-methylphenyl)-1H-pyrazol-3-yl}methyl)methylcarbamate (3.0 g) in ethyl acetate (30 mL) was added 3-chloroperbenzoic acid (6.3 g). The mixture was stirred at room temperature for 18 hr, treated with saturated aqueous sodium thiosulfate solution, and extracted with ethyl acetate. The extract was washed successively with water, saturated aqueous sodium hydrogen carbonate solution and saturated brine, dried over anhydrous sodium sulfate, and concentrated under reduced pressure. The residue was purified by silica gel column chromatography (eluent: hexane-ethyl acetate=9:1→4:1) to give the title compound as a colorless amorphous solid (3.1 g, yield 94%).
¹H-NMR(CDCl₃)δ: 1.51(9H,s),1.63(3H,s),2.88(3H,brs),4.47(2H,br),6.82-6.85(1H,m),7.11-7.20(3H,m),7.34-7.37(1H,m),7.65-7.68(1H,m),8.36-8.37(1H,m).

### Reference Example 378

### tert-butyl {[1-(5-fluoro-2-methylphenyl)-5-(pyridin-3-ylsulfonyl)-1H-pyrazol-3-yl]methyl}methylcarbamate

tert-Butyl ({5-[(6-chloropyridin-3-yl)sulfonyl]-1-(5-fluoro-2-methylphenyl)-1H-pyrazol-3-yl}methyl)methylcarbamate (419 mg) and triethylamine (171 mg) were dissolved in methanol (5 mL), and the solution was stirred for 1 hr under a hydrogen atmosphere (1 atom). The reaction mixture was filtered, and the filtrate was concentrated under reduced pressure. Water was added to the residue, and the mixture was extracted with ethyl acetate. The extract was washed with saturated brine, dried over anhydrous sodium sulfate, and concentrated under reduced pressure. The residue was dissolved-in toluene (5 mL), manganese dioxide (368 mg) was added, and the mixture was stirred at 80°C for 2 hr. The reaction mixture was filtered through celite, and the filtrate was concentrated under reduced pressure. The residue was purified by basic silica gel column chromatography (eluent: hexane-ethyl acetate=9:1→4:1) to give the title compound as a colorless amorphous solid (194 mg, yield 50%).
¹H-NMR(CDCl₃)δ: 1.51(9H,s),1.57(3H,s),2.88(3H,brs),4.47(2H,br),6.77-6.80(1H,m),7.05-7.20(3H,m),7.32-7.36(1H,m),7.71-7.74(1H,m),8.62-8.63(1H,m),8.79-8.80(1H,m).

### Reference Example 379

### tert-butyl ({5-[(6-methylpyridin-3-yl)sulfonyl]-1-(5-fluoro-2-methylphenyl)-1H-pyrazol-3-yl}methyl)methylcarbamate

tert-Butyl ({5-[(6-chloropyridin-3-yl)sulfonyl]-1-(5-fluoro-2-methylphenyl)-1H-pyrazol-3-yl}methyl)methylcarbamate (1.0 g), potassium carbonate (348 mg) and methylboronic acid (1.3 g) were suspended in cyclopentylmethyl ether (10 mL), and the suspension was degassed under an argon atmosphere. Tetrakis(triphenylphosphine) palladium(0) (243 mg) was added, and the mixture was further degassed. The mixture was stirred at 100°C for 1.5 hr, and allowed to cool to room temperature. Water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl/acetate=4:1→1:1) and basic silica gel column chromatography (eluent: hexane-ethyl acetate=9:1→1:1) to give the title compound as a colorless oil (294 mg, yield 29%).
¹H-NMR(CDCl₃)δ: 1.50(9H,s),1.64(3H,s),2.63(3H,s),2.87(3H,brs),4.47(2H,br),6.72 -6.75(1H,m),7.07(1H,br),7.14-7.18(3H,m),7.58-7.61(1H,m),8.48-8.49(1H,m).

### Reference Example 380

### tert-butyl {[1-(2-chloro-3-fluorophenyl)-5-(pyridin-3-ylsulfonyl)-1H-pyrazol-3-yl]methyl}methylcarbamate

tert-Butyl ({1-(2-chloro-3-fluorophenyl)-5-[(pyridin-3-yl)thio]-1H-pyrazol-3-yl}methyl)methylcarbamate (528 mg) was suspended in a mixed solvent of acetonitrile (10 mL) and water (10 mL), sodium percarbonate (5.6 g) was added at room temperature, and the mixture was stirred for 18 hr. Acetonitrile was evaporated under reduced pressure, and the residue was extracted with ethyl acetate. The extract was washed successively with saturated aqueous sodium thiosulfate solution and saturated brine, dried over anhydrous sodium sulfate, and concentrated under reduced pressure. The residue was purified by basic silica gel column chromatography (eluent: hexane-ethyl acetate=9:1→4:1) to give the title compound as a colorless oil (390 mg, yield 69%).
¹H-NMR(CDCl₃)δ: 1.51(9H,s),2.88(3H,brs),4.49(2H,br),7.14(1H,br),7.26-7.45(4H,m),7.74-7.77(1H,m),8.61-8.62(1H,m),8.80-8.81(1H,m).

### Reference Example 381

### tert-butyl ({1-(2-chloro-3-fluorophenyl)-5-[(6-chloropyridin-3-yl)sulfonyl]-1H-pyrazol-3-yl}methyl)methylcarbamate

To a solution of tert-butyl ({1-(2-chloro-3-fluorophenyl)-5-[(6-chloropyridin-3-yl)thio]-1H-pyrazol-3-yl}methyl)methylcarbamate (1.3 g) in ethyl acetate (15 mL) was added 3-chloroperbenzoic acid (2.5 g). The mixture was stirred at room temperature for 18 hr, treated with saturated aqueous sodium thiosulfate solution, and extracted with ethyl acetate. The extract was washed successively with water, saturated aqueous sodium hydrogen carbonate solution and saturated brine, dried over anhydrous sodium sulfate, and concentrated under reduced pressure. The residue was purified by basic silica gel column chromatography (eluent: hexane-ethyl acetate=9:1→4:1) to give the title compound as colorless crystals (1.1 g, yield 79%).
¹H-NMR(CDCl₃)δ: 1.49(9H,s),2.89(3H,brs),4.46(2H,br),7.03-7.28(3H,m),7.33-7.43(2H,m),7.77-7.81(1H,m),8.43-8.44(1H,m).

### Reference Example 382

### tert-butyl ({1-(2-chloro-3-fluorophenyl)-5-[(6-methylpyridin-3-yl)sulfonyl]-1H-pyrazol-3-yl}methyl)methylcarbamate

To a suspension of tert-butyl ({1-(2-chloro-3 fluorophenyl)-5-[(6-chloropyridin-3-yl)sulfonyl]-1H-pyrazol-3-yl}methyl)methylcarbamate (920 mg) and [1,3-bis(diphenylphosphino)propane]dichloronickel (II) (97 mg) in tetrahydrofuran (10 mL) was added dropwise 35% methylmagnesium bromide-ether solution (3 mL) at 0°C, and the mixture was stirred at room temperature for 3 hr. Saturated aqueous ammonium chloride solution was added, and the mixture was extracted with ethyl acetate under ice-cooling. The extract was washed with saturated brine, dried over anhydrous sodium sulfate, and concentrated under reduced pressure. The residue was purified by silica gel column chromatography (eluent: hexane-ethyl acetate=4:1→1:1) to give the title compound as a yellow oil (613 mg, yield 15%).
¹H-NMR(CDCl₃)δ: 1.51(9H,s),2.63(3H,s),2.87(3H,brs),4.48(2H,br),7.10(1H,br),7.1 8(1H,d,J=8.1Hz),7.31-7.45(3H,m),7.61-7.65(1H,m),8.45-8.46(1H,m).

### Reference Example 383

### (tert-butyl {1-(2-chloro-5-fluorophenyl)-5-[(6-chloropyridin-3-yl)sulfonyl]-1H-pyrazol-3-yl}methyl)methylcarbamate

To a solution of tert-butyl ({1-(2-chloro-5-fluorophenyl)-5-[(6-chloropyridin-3-yl)thio]-1H-pyrazol-3-yl}methyl)methylcarbamate (258 mg) in ethyl acetate (5 mL) was added 3-chloroperbenzoic acid (512 mg). The mixture was stirred at room temperature for 1.5 hr, treated with saturated aqueous sodium thiosulfate solution, and the mixture was extracted with ethyl acetate. The extract was washed successively with water, saturated aqueous sodium hydrogen carbonate solution and saturated brine, dried over anhydrous sodium sulfate, and concentrated under reduced pressure. The residue was purified by basic silica gel column chromatography (eluent: hexane-ethyl acetate= 9:1→4:1) to give the title compound as a colorless oil (226 mg, yield 82%).
¹H-NMR(CDCl₃)δ: 1.50(9H,s),2.87(3H,brs),4.47(2H,brs),7.10(1H,br),7.23-7.28(2H,m),7.33-7.40(2H,m),7.75-7.79(1H,m),8.36-8.38(1H,m).

### Reference Example 384

### tert-butyl ({1-(2-chloro-5-fluorophenyl)-5-[(6-methylpyridin-3-yl)sulfonyl]-1H-pyrazol-3-yl}methyl)methylcarbamate

tert-Butyl ({1-(2-chloro-5-fluorophenyl)-5-[(6-chloropyridin-3-yl)sulfonyl]-1H-pyrazol-3-yl}methyl)methylcarbamate (226 mg), potassium carbonate (121 mg) and methylboronic acid (131 mg) were suspended in cyclopentylmethyl ether (5 mL), and the suspension was degassed under an argon atmosphere. Tetrakis(triphenylphosphine) palladium(0) (51 mg) was added, and the mixture was further degassed. The mixture was stirred at 110°C for 1.5 hr, and allowed to cool to room temperature. Water was added, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=9:1→4:1) to give the title compound as a colorless oil (91 mg, yield 42%).
¹H-NMR(CDCl₃)δ: 1.50(9H,s),2.64(3H,s),2.87(3H,brs),4.48(2H,brs),7.09(1H,br),7. 19-7.36(4H,m),7.68-7.72(1H,m),8.50-8.51(1H,m).

### Reference Example 385

### 2-chloro-3-hydrazinopyridine hydrochloride

To a solution of 2-chloropyridin-3-amine (5.0 g) in concentrated hydrochloric acid (65 mL) was added dropwise a solution of sodium nitrite (3.5 g) in water (8 mL) at -10°C, and the mixture was stirred at the same temperature for 1 hr. A solution of tin(II) chloride (14.8 g) in concentrated hydrochloric acid (16 mL) was added dropwise at -10°C, and the mixture was stirred at 0°C for 2 hr. To the mixture was added 8 mol/L sodium hydroxide solution, and the mixture was extracted with ethyl acetate. The insoluble material was filtered off, and the filtrate was washed with saturated brine, dried over anhydrous magnesium sulfate, and concentrated under reduced pressure. The residue was dissolved in ethyl acetate (10 mL), to the solution was added 4 mol/L hydrogen chloride-ethyl acetate solution (5 mL), and the mixture was concentrated under reduced pressure. The residue was suspended in ethyl acetate and insoluble solid was collected by filtration to give the title compound as a yellow solid (5.9 g, yield 85%).
¹H-NMR(DMSO-d₆)δ: 7.40-7.45(1H,m),7.50-7.55(1H,m),7.98-8.00(1H,m),8.38(1H,br),10.46(3H,br).

### Reference Example 386

### ethyl 1-(3-chloro-2-fluorophenyl)-5-hydroxy-1H-pyrazole-3-carboxylate

A mixture of (3-chloro-2-fluorophenyl)hydrazine hydrochloride (5.0 g), potassium carbonate (7.0 g) and diethyl but-2-ynedioate (4.4 g) in ethanol (60 mL) was refluxed for 14 hr, allowed to cool to room temperature, and acidified with 6 mol/L hydrochloric acid. Ethanol was evaporated under reduced pressure, water was added to the residue, and the mixture was extracted with ethyl acetate. The extract was made basic with 8 mol/L sodium hydroxide solution and washed with ethyl acetate. The aqueous layer was acidified with 6 mol/L hydrochloric acid, and extracted with ethyl acetate. The extract was washed with water, saturated brine, dried over anhydrous magnesium sulfate, and concentrated under reduced pressure. The residue was washed with ethyl acetate-hexane to give the title compound as a white solid (3.6 g, yield 50%).
¹H-NMR(DMSO-d₆)δ: 1.28(3H,t,J=7.2Hz),4.26(2H,q,J=7.2Hz),5.93(1H,s),7.36-7.41(1H,m),7.53-7.58(1H,m),7.72-7.78(1H,m),12.12(1H,brs).

### Reference Example 387

### ethyl 1-(2-chloropyridin-3-yl)-5-hydroxy-1H-pyrazole-3-carboxylate

A mixture of 2-chloro-3-hydrazinopyridine hydrochloride (5.9 g) potassium carbonate (9.1 g) and diethyl but-2-ynedioate (5.6 g) in ethanol (60 mL) was refluxed for 18 hr, allowed to cool to room temperature, treated with 6 mol/L hydrochloric acid. Ethanol was evaporated under reduced pressure, water was added to the residue, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=1:1→ethyl acetate) to give the title compound as a pale-brown solid (3.7 g, yield 42%).
¹H-NMR(DMSO-d₆)δ: 1.29(3H,t,J=7.2Hz),4.36(2H,q,J=7.2Hz),5.92(1H,s),7.61-7.66(1H,m),8.08-8.11(1H,m),8.57-8.59(1H,m),12.11(1H,br).

### Reference Example 388

### ethyl 1-(3-chloro-2-fluorophenyl)-5-{[(trifluoromethyl)sulfonyl]oxy}-1H-pyrazole-3-carboxylate

To a solution of ethyl 1-(3-chloro-2-fluorophenyl)-5-hydroxy-1H-pyrazole-3-carboxylate (1.4 g) in tetrahydrofuran (20 mL) were added triethylamine (0.8 mL) and N-phenylbis(trifluoromethanesulfonimide) (1.8 g) at 0°C, and the mixture was stirred at room temperature for 10 min, and concentrated under reduced pressure. Water was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure to give the title compound as a brown oil (2.7 g, yield quantitative).
¹H-NMR(CDCl₃)δ: 1.42(3H,t,J=7.2Hz),4.44(2H,q,J=7.2Hz),6.85(1H,s),7.23-7.47(2H,m),7.57-7.62(1H,m).

### Reference Example 389

### ethyl 1-(2-chloropyridin-3-yl)-5-{[(trifluoromethyl)sulfonyl]oxy}-1H-pyrazole-3-carboxylate

To a solution of ethyl 1-(2-chloropyridin-3-yl)-5-hydroxy-1H-pyrazole-3-carboxylate (1.0 g) in tetrahydrofuran (10 mL) were added triethylamine (0.63 mL) and N-phenylbis(trifluoromethanesulfonimide) (1.5 g) at 0°C, and the mixture was stirred at room temperature for 10 min, and concentrated under reduced pressure. Water was added to the residue, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=19:1→7:1) to give the title compound as a colorless oil (1.5 g, yield 98%).
¹H-NMR(CDCl₃)δ: 1.42(3H,t,J=7.2Hz),4.45(2H,q,J=7.2Hz),6.87(1H,s),7.46(1H,dd,J= 7.8,4.5Hz),7.88(1H,dd,J=7.8,1.8Hz),8.60(1H,dd,J=4.5,1.8Hz).

### Reference Example 390

### ethyl 1-(3-chloro-2-fluorophenyl)-5-({3-[(2-ethylhexyl)oxy]-3-oxopropyl}thio)-1H-pyrazole-3-carboxylate

A solution of ethyl 1-(3-chloro-2-fluorophenyl)-5-{[(trifluoromethyl)sulfonyl]oxy}-1H-pyrazole-3-carboxylate (2.7 g), 2-ethylhexyl 3-mercaptopropanoate (1.3 g), tris(dibenzylideneacetone)dipalladium(0) (53 mg), 9,9-dimethyl-4,5-bis(diphenylphosphino)xanthine (66 mg) and N-ethyldiisopropylamine (1.9 mL) was stirred in toluene (40 mL) at 105°C for 2 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=5:1) to give the title compound as a yellow oil (2.0 g, yield 75%).
¹H-NMR(CDCl₃)δ: 0.85-0.92(6H,m),1.23-1.38(9H,m),1.41(3H,t,J=7.2Hz),2.57(2H,t,J=7.2Hz),2.96(2H,t,J=7 .2Hz),3.96-3.99(2H,m),4.43(2H,q,J=7.2Hz),7.04(1H,s),7.13-7.28(1H,m),7.35-7.40(1H,m),7.52-7.57(1H,m).

### Reference Example 391

### ethyl 1-(2-chloro-5-fluorophenyl)-5-[(pyridin-3-yl)thio]-1H-pyrazole-3-carboxylate

To a solution of ethyl 1-(2-chloro-5-fluorophenyl)-5-({3-[(2-ethylhexyl)oxy]-3-oxopropyl}thio)-1H-pyrazole-3-carboxylate (840 mg) in ethanol (10 mL) was added sodium ethoxide (177 mg), and the mixture was stirred at room temperature for 1 hr. The mixture was concentrated under reduced pressure, and the residue was dissolved in toluene (10 mL). 3-Iodopyridine (390 mg) was added, and the mixture was degassed. Tris(dibenzylideneacetone)dipalladium(0) (16 mg) and 4,5-bis(diphenylphosphino)-9,9-dimethylxanthine (20 mg) were added, and the mixture was further degassed. The mixture was stirred at 110°C for 18 hr under an argon atmosphere, and allowed to cool to room temperature. Water was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=9:1→3:1) to give the title compound as a pale- yellow oil (455 mg, yield 70%).
¹H-NMR(CDCl₃)δ: 1.41(3H,t,J=7.1Hz),4.43(2H,q,J=7.1Hz),7.01(1H,dd,J=8.0,3.0Hz), 7.12-7.23(3H,m),7.41-7.50(2H,m),8.33(1H,d,J=2.5Hz),8.46(1H,dd,J=4.8,1.5Hz).

### Reference Example 392

### ethyl 1-(3-chloro-2-fluorophenyl)-5-[(pyridin-3-yl)thio]-1H-pyrazole-3-carboxylate

To a solution of ethyl 1-(3-chloro-2-fluorophenyl)-5-({3-[(2-ethylhexyl)oxy]-3-oxopropyl}thio)-1H-pyrazole-3-carboxylate (1.04 g) in ethanol (10 mL) was added sodium ethoxide (292 mg) at 0°C, and the mixture was stirred at room temperature for 1 hr, and concentrated under reduced pressure. A mixture of the residue, 3-iodopyridine (679 mg), tris(dibenzylideneacetone)dipalladium(0) (20 mg) and 9,9-dimethyl-4,5-bis(diphenylphosphino)xanthine (26 mg) was stirred in a mixed solvent of toluene (10 mL) and ethanol (1 mL) at 80°C for 3 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=1:1) to give the title compound as a yellow oil (635 mg, yield 79%).
¹H-NMR(CDCl₃)δ: 1.41(3H,t,J=7.2Hz),4.43(2H,q,J=7.2Hz),7.11-7.24(4H,m),7.39-7.43(1H,m),7.49-7.60(1H,m),8.30(1H,d,J=2.7Hz),8.44-8.46(1H,m).

### Reference Example 393

### ethyl 1-(3-chloro-2-fluorophenyl)-5-[(6-chloropyridin-3-yl)thio]-1H-pyrazole-3-carboxylate

To a solution of ethyl 1-(3-chloro-2-fluorophenyl)-5-({3-[(2-ethylhexyl)oxy]-3-oxopropyl}thio)-1H-pyrazole-3-carboxylate (2.42 g) in ethanol (25 mL) was added sodium ethoxide (688 mg) at 0°C, and the mixture was stirred at room temperature for 1 hr, and concentrated under reduced pressure. A mixture of the residue, 2-chloro-5-iodopyridine (1.27 g), tris(dibenzylideneacetone)dipalladium(0) (45 mg) and 9,9-dimethyl-4,5-bis(diphenylphosphino)xanthine (58 mg) was stirred in toluene (25 mL) at 90°C for 2 hr. The reaction mixture was allowed to cool to room temperature, water was added, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=4:1) to give the title compound as a colorless oil (1.64 g, yield 80%).
¹H-NMR(CDCl₃)δ: 1.41(3H,t,J=7.2Hz),4.43(2H,q,J=7.2Hz),7.13-7.25(4H,m),7.34-7.38(1H,m),7.51-7.56(1H,m),8.05-8.06(1H,m).

### Reference Example 394

### ethyl 5-[(3-fluorophenyl)thio]-1-(2-fluoropyridin-3-yl)- 1H-pyrazole-3-carboxylate

A solution of ethyl 1-(2-fluoropyridin-3-yl)-5-{[(trifluoromethyl)sulfonyl]oxy}-1H-pyrazole-3-carboxylate (192 mg), 3-fluorothiophenol (77 mg) and sodium carbonate (80 mg) in toluene (2.5 mL) was degassed, tris(dibenzylideneacetone)dipalladium(0) (23 mg) and 4,5-bis(diphenylphosphino)-9,9-dimethylxanthine (29 mg) were added, and the mixture was further degassed. The mixture was stirred at 110°C for 3 hr under an argon atmosphere, and allowed to cool to room temperature. Ethyl acetate was added, and the mixture was filtered through celite. The filtrate was concentrated under reduced pressure. The residue was purified by silica gel column chromatography (eluent: hexane-ethyl acetate=49:1→17:3) to give the title compound as a colorless oil (143 mg, yield 79%).
¹H-NMR(CDCl₃)δ: 1.42(3H,t,J=7.2Hz),4.45(2H,q,J=6.9Hz),6.74(1H,dt,J=8.9,2.0Hz), 6.79-6.85(1H,m),6.85-6.95(1H,m),7.19(1H,td,J=8.0,5.9Hz),7.23(1H,s),7.25-7.29(1H,m),7.73(1H,ddd,J=9.1,7.6,1.9Hz),8.22-8.37(1H,m).

### Reference Example 395

### ethyl 1-(2-fluoropyridin-3-yl)-5-[(3-methoxyphenyl)thio]-1H-pyrazole-3-carboxylate

A solution of ethyl 1-(2-fluoropyridin-3-yl)-5-{[(trifluoromethyl)sulfonyl]oxy}-1H-pyrazole-3-carboxylate (192 mg), 3-methoxythiophenol (84 mg) and sodium carbonate (80 mg) in toluene (2.5 mL) was degassed, tris(dibenzylideneacetone)dipalladium(0) (23 mg) and 4,5-bis(diphenylphosphino)-9,9-dimethylxanthine (29 mg) were added, and the mixture was further degassed. The mixture was stirred at 110°C for 5 hr under an argon atmosphere, and allowed to cool to room temperature. Ethyl acetate was added, and the mixture was filtered through celite. The filtrate was concentrated under reduced pressure. The residue was purified by silica gel column chromatography (eluent: hexane-ethyl acetate=9:1→3:2) to give the title compound as a colorless oil (99 mg, yield 53%).
¹H-NMR(CDCl₃)δ: 1.42(3H,t,J=7.1Hz),4.44(2H,q,J=7.1Hz),6.56-6.60(1H,m),6.63(1H,ddd,J=7.8,1.7,1.0Hz),6.73(1H,ddd,J=8.4,2.5, 1.0Hz),7.08-7.16(1H,m),7.18(1H,s),7.20-7.26(1H,m),7.71(1H,ddd,J=9.0,7.5,1.9Hz),8.18-8.32(1H,m).

### Reference Example 396

### ethyl 1-(2-chloropyridin-3-yl)-5-(phenylthio)-1H-pyrazole-3-carboxylate

A solution of ethyl 1-(2-chloropyridin-3-yl)-5-{[(trifluoromethyl)sulfonyl]oxy}-1H-pyrazole-3-carboxylate (600 mg), thiophenol (182 mg) and cesium carbonate (978 mg) in toluene (10 mL) was degassed, tris(dibenzylideneacetone)dipalladium(0) (14 mg) and 4,5-bis(diphenylphosphino)-9,9-dimethylxanthine (17 mg) were added, and the mixture was further degassed. The mixture was stirred at 110°C for 4 hr under an argon atmosphere, and allowed to cool to room temperature. Water and ethyl acetate were added, and the mixture was filtered through celite. The organic layer of the filtrate was washed with saturated brine, dried over anhydrous sodium sulfate, and concentrated under reduced pressure. The residue was purified by silica gel column chromatography (eluent: hexane-ethyl acetate=9:1→3:1) to give the crude title compound as a yellow oil (128 mg, yield 24%).
¹H-NMR(CDCl₃)δ: 1.41(3H,t,J=7.2Hz),4.44(2H,q,J=7.2Hz), 7.06-7.09(1H,m),7.17(1H,s),7.22-7.27(5H,m),7.51(1H,dd,J=7.5,1.8Hz),8.48(1H,dd,J=4.5,1.8Hz).

### Reference Example 397

### {1-(3-chloro-2-fluorophenyl)-5-[(pyridin-3-yl)thio]-1H-pyrazol-3-yl}methanol

A solution of ethyl 1-(3-chloro-2-fluorophenyl)-5-[(pyridin-3-yl)thio]-1H-pyrazole-3-carboxylate (628 mg) in tetrahydrofuran (10 mL) was cooled to -78°C, and 1.5 mol/L diisobutylaluminum hydride-toluene solution (4.4 mL) was added dropwise. The reaction mixture was stirred for 1 hr at 0°C, 1 mol/L sodium hydroxide solution was added, and the mixture was extracted with ethyl acetate. The extract was washed successively with saturated aqueous sodium hydrogen carbonate solution, water and saturated brine, dried over anhydrous sodium sulfate, and concentrated under reduced pressure to give the title compound as a colorless oil (555 mg, yield quantitative).
¹H-NMR(CDCl₃)δ: 4.76(2H,s),6.69(1H,s),7.09-7.27(3H,m),7.35-7.41(1H,m),7.45-7.50(1H,m),8.29(1H,d,J=2.4Hz),8.40-8.42(1H,m),1H: not detected.

### Reference Example 398

### {1-(3-chloro-2-fluorophenyl)-5-[(6-chloropyridin-3-yl)thio]-1H-pyrazol-3-yl}methanol

A solution of ethyl 1-(3-chloro-2-fluorophenyl)-5-[(6-chloropyridin-3-yl)thio]-1H-pyrazole-3-carboxylate (1.08 g) in tetrahydrofuran (13 mL) was cooled to -78°C, and 1.5 mol/L diisobutylaluminum hydride-toluene solution (7 mL) was added dropwise. The reaction mixture was stirred for 1 hr at 0°C, 1 mol/L hydrochloric acid was added, and the mixture was extracted with ethyl acetate. The extract was washed successively with saturated aqueous sodium hydrogen carbonate solution, water and saturated brine, dried over anhydrous sodium sulfate, and concentrated under reduced pressure to give the title compound as a colorless oil (1.06 g, yield quantitative).
¹H-NMR(CDCl₃)δ: 2.10(1H,t,J=6.0Hz),4.75(2H,d,J=6.0Hz),6.69(1H,s),7.11-7.23(3H,m),7.32-7.36(1H,m),7.47-7.52(1H,m),8.04(1H,d,J=2.4Hz).

### Reference Example 399

### {5-[(3-fluorophenyl)thio]-1-(2-fluoropyridin-3-yl)-1H-pyrazol-3-yl}methanol

To a suspension of lithium aluminum hydride (57 mg) in tetrahydrofuran (3 mL) was added dropwise a solution of ethyl 5-[(3-fluorophenyl)thio]-1-(2-fluoropyridin-3-yl)-1H-pyrazole-3-carboxylate (364 mg) in tetrahydrofuran (3 mL) with ice-cooling. The mixture was stirred at 0°C for 2 hr, 1 mol/L sodium hydroxide solution was added, and the mixture was extracted with ethyl acetate. The separated aqueous layer was extracted again with ethyl acetate. The combined organic layers were washed with saturated brine, dried over anhydrous sodium sulfate, concentrated under reduced pressure to give the title compound as a colorless oil (187 mg, yield 51%).
¹H-NMR(CDCl₃)δ: 4.80(2H,s),6.70-6.77(2H,m),6.81(1H,ddd,J=7.9,1.7,0.8Hz),6.83-6.91(1H,m),7.12-7.20(1H,m),7.20-7.25(1H,m),7.67-7.76(1H,m),8.22-8.30(1H,m), 1H: not detected.

### Reference Example 400

### 1-(2-chloro-5-fluorophenyl)-5-[(pyridin-3-yl)thio]-1H-pyrazole-3-carbaldehyde

A solution of ethyl 1-(2-chloro-5-fluorophenyl)-5-[(pyridin-3-yl)thio]-1H-pyrazole-3-carboxylate (455 mg) in tetrahydrofuran (5 mL) was cooled to -78°C, and 1.5 mol/L diisobutylaluminum hydride-toluene solution (3.2 mL) was added dropwise. The reaction mixture was stirred for 3 hr under ice-cooling, sodium sulfate 10 hydrate was added, and the mixture was further stirred at room temperature for 3 hr. The reaction mixture was filtered through celite, and the filtrate was concentrated under reduced pressure. The obtained residue was dissolved in toluene (5 mL), manganese dioxide (696 mg) was added, and the mixture was stirred at 80°C for 1 hr. The reaction mixture was allowed to cool to room temperature, and filtered through celite. The filtrate was concentrated under reduced pressure, and the residue was purified by silica gel column chromatography (eluent: hexane-ethyl acetate=4:1→2:1) to give the title compound as a yellow oil (270 mg, 2step yield 68%).
¹H-NMR(CDCl₃)δ: 7.05(1H,dd,J=8.0,3.0Hz), 7.12(1H,s),7.16-7.24(2H,m),7.45-7.51(2H,m),8.34(1H,d,J=1.6Hz),8.49(1H,dd,J=4.8,1.5Hz),9.99(1H, s).

### Reference Example 401

### 1-(3-chloro-2-fluorophenyl)-5-[(pyridin-3-yl)thio]-1H-pyrazole-3-carbaldehyde

{1-(3-Chloro-2-fluorophenyl)-5-[(pyridin-3-yl)thio]-1H-pyrazol-3-yl}methanol (545 mg) was dissolved in toluene (10 mL), manganese dioxide (952 mg) was added, and the mixture was stirred at 100°C for 2 hr. The reaction mixture was allowed to cool to room temperature, and filtered through celite. The filtrate was concentrated under reduced pressure to give the title compound as a pale-yellow oil (467 mg, yield 86%).
¹H-NMR(CDCl₃)δ: 7.11(1H,s),7.16-7.27(3H,m),7.42-7.46(1H,m),7.53-7.59(1H,m),8.30-8.31(1H,m),8.45-8.47(1H,m),9.98(1H,s).

### Reference Example 402

### 1-(3-chloro-2-fluorophenyl)-5-[(6-chloropyridin-3-yl)thio]-1H-pyrazole-3-carbaldehyde

To a solution of {1-(3-chloro-2-fluorophenyl)-5-[(6-chloropyridin-3-yl)thio]-1H-pyrazol-3-yl}methanol (1.1 g) in dimethylsulfoxide (8 mL) were added triethylamine (8 mL) and sulfur trioxide pyridine complex (1.5 g), and the mixture was stirred at room temperature for 16 hr. Water was added, and the mixture was extracted with ethyl acetate. The extract was washed successively with 1 mol/L hydrochloric acid, water, saturated aqueous sodium hydrogen carbonate solution and saturated brine, dried over anhydrous sodium sulfate, and concentrated under reduced pressure. The residue was washed with diisopropyl ether to give the title compound as a pale-yellow solid (0.91 g, yield 82%).
¹H-NMR(CDCl₃)δ: 7.13(1H,s),7.18-7.28(3H,s),7.37-7.40(1H,m),7.55-7.61(1H,m),8.06(1H,d,J=2.7Hz),9.98(1H,s).

### Reference Example 403

### 5-[(3-fluorophenyl)thio]-1-(2-fluoropyridin-3-yl)-1H-pyrazole-3-carbaldehyde

{5-[(3-Fluorophenyl)thio]-1-(2-fluoropyridin-3-yl)-1H-pyrazol-3-yl}methanol (187 mg) was dissolved in toluene (3 mL), manganese dioxide (510 mg) was added, and the mixture was stirred at 80°C for 15 hr. The reaction mixture was allowed to cool to room temperature, and filtered through celite. The filtrate was concentrated under reduced pressure to give the title compound as a colorless oil (166 mg, yield 89%).
¹H-NMR(CDCl₃)δ: 6.76(1H,dt,J=8.7,2.1Hz),6.81-6.86(1H,m),6.87-6.98(1H,m),7.15-7.24(2H,m),7.27-7.35(1H,m),7.76(1H,ddd,J=9.2,7.6,1.9Hz),8.35(1H,dt,J=4.9,1.5Hz ),10.01(1H,s).

### Reference Example 404

### tert-butyl ({1-(2-chloro-5-fluorophenyl)-5-[(pyridin-3-yl)thio]-1H-pyrazol-3-yl}methyl)methylcarbamate

1-(2-Chloro-5-fluorophenyl)-5-[(pyridin-3-yl)thio]-1H-pyrazole-3-carbaldehyde (270 mg) was dissolved in a mixed solvent of tetrahydrofuran (5 mL) and methanol (2 mL), 40% methylamine-methanol solution (0.84 mL) was added, and the mixture was stirred at room temperature for 2 hr. To the reaction mixture was added sodium borohydride (78 mg) under ice-cooling. The mixture was stirred at room temperature for 4 hr, and the solvent was evaporated under reduced pressure. Water and ethyl acetate were added to the residue, di-tert-butyl bicarbonate (202 mg) was added, and the mixture was stirred for 1 hr. The ethyl acetate layer was washed with saturated brine, dried over anhydrous sodium sulfate, and concentrated under reduced pressure. The residue was purified by silica gel column chromatography (eluent: hexane-ethyl acetate=9:1→4:1) to give the title compound as a pale-yellow oil (221 mg, yield 61%).
¹H-NMR(CDCl₃)δ: 1.45-1.54(9H,m),2.89(3H,brs),4.45(2H,brs),6.51-6.69(1H,m),7.00(1H,dd,J=8.1,2.9Hz),7.06-7.23(2H,m),7.34-7.49(2H,m),8.30(1H,d,J=1.9Hz),8.39-8.48(1H,m).

### Reference Example 405

### tert-butyl ({1-(3-chloro-2-fluorophenyl)-5-[(pyridin-3-yl)thio]-1H-pyrazol-3-yl}methyl)methylcarbamate

To a solution of 1-(3-chloro-2-fluorophenyl)-5-[(pyridin-3-yl)thio]-1H-pyrazole-3-carbaldehy (465 mg) in a mixed solvent of tetrahydrofuran (4 mL) and methanol (4 mL) was added 40% methylamine-methanol solution (1.4 mL) at 0°C, and the mixture was stirred at room temperature for 12 hr. The mixture was concentrated under reduced pressure. To a solution of the residue in methanol (4 mL) was added sodium borohydride (63 mg) at 0°C. The mixture was stirred at room temperature for 4 hr, and the solvent was concentrated under reduced pressure. Water was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure. To a solution of the residue in ethyl acetate (6 mL) was added di-tert-butyl bicarbonate (0.3 mL). The mixture was stirred for 30 min, and concentrated under reduced pressure. The residue was purified by silica gel column chromatography (eluent: hexane-ethyl acetate=1:1) to give the title compound as a pale-yellow oil (430 mg, yield 69%).
¹H-NMR(CDCl₃)δ: 1.48(9H,s),2.89(3H,brs),4.46(2H,brs),6.50-6.70(1H,m),7.08-7.21(3H,m),7.34-7.38(1H,m),7.44-7.49(1H,m),8.27(1H,d,J=1.8Hz),8.40-8.41(1H,m).

### Reference Example 406

### tert-butyl ({1-(3-chloro-2-fluorophenyl)-5-[(6-chloropyridin-3-yl)thio]-1H-pyrazol-3-yl}methyl)methylcarbamate

To a solution of 1-(3-chloro-2-fluorophenyl)-5-[(6-chloropyridin-3-yl)thio]-1H-pyrazole-3-carbaldehyde (893 mg) in a mixed solvent of tetrahydrofuran (10 mL) and methanol (10 mL) was added 40% methylamine-methanol solution (2.4 mL) at 0°C, and the mixture was stirred at room temperature for 2 hr. The mixture was concentrated under reduced pressure. To a solution of the residue in a mixed solvent of tetrahydrofuran (10 mL) and methanol (10 mL) was added sodium borohydride (201 mg) at 0°C. The mixture was stirred at room temperature for 30 min, and the solvent was concentrated under reduced pressure. Water was added to the residue, and the mixture was extracted with ethyl acetate. To the extract was added 1 mol/L hydrochloric acid, and the aqueous layer was washed with ethyl acetate. The aqueous layer was made basic with 8 mol/L sodium hydroxide solution and extracted with ethyl acetate. The extract was washed successively with saturated aqueous sodium hydrogen carbonate solution, water and saturated brine, dried over anhydrous sodium sulfate, and concentrated under reduced pressure. To a solution of the residue in ethyl acetate (4 mL) was added di-tert-butyl bicarbonate (0.24 mL). After the mixture was stirred for 30 min, water was added, and the mixture was extracted with ethyl acetate. The extract was washed successively with saturated aqueous sodium hydrogen carbonate solution, water and saturated brine, dried over anhydrous sodium sulfate, and concentrated under reduced pressure to give the title compound as a pale-yellow oil (545 mg, yield 47%).
¹H-NMR(CDCl₃)δ: 1.48(9H,s),2.88(3H,brs),4.45(2H,brs),6.57-6.63(1H,m),7.11-7.22(3H,m),7.30-7.34(1H,m),7.46-7.51(1H,m),8.10(1H,d,J=2.7Hz).

### Reference Example 407

### tert-butyl 5-[(3-fluorophenyl)thio]-1-(2-fluoropyridin-3-yl)-1H-pyrazol-3-yl}methyl)methylcarbamate

To a solution of ({5-[(3-fluorophenyl)thio]-1-(2-fluoropyridin-3-yl)-1H-pyrazole-3-carbaldehyde (166 mg) in methanol (3 mL) were added methylammonium chloride (39 mg), anhydrous magnesium sulfate (94 mg) and triethylamine (58 mg), and the mixture was stirred at room temperature for 1 hr. Sodium borohydride (24 mg) was added under ice-cooling, and the mixture was further stirred at room temperature for 1 hr. The solvent was evaporated under reduced pressure, ethyl acetate (2 mL) and water (2 mL) were added to the residue. To the mixture was added di-tert-butyl bicarbonate (171 mg). Ethyl acetate layer and aqueous layer were separated and the aqueous layer was extracted again with ethyl acetate. The combined organic layers were washed with saturated brine, dried over anhydrous sodium sulfate, and concentrated under reduced pressure. The residue was purified by silica gel column chromatography (eluent: hexane-ethyl acetate=9:1→4:1) to give the title compound as a colorless oil (170 mg, yield 75%).
¹H-NMR(CDCl₃)δ: 1.49(9H,s),2.92(3H,brs),4.48(2H,brs),6.54-6.74(2H,m),6.79(1H,dd,J=7.8,1.5Hz),6.85(1H,td,J=8.3,2.3Hz),7.1 1-7.19(1H,m),7.20-7.25(1H,m),7.70(1H,ddd,J=9.2,7.6,1.9Hz),8.25(1H,dt,J=4.7,1.4Hz ).

### Reference Example 408

### tert-butyl {[1-(2-chloro-5-fluorophenyl)-5-(pyridin-3-ylsulfonyl)-1H-pyrazol-3-yl]methyl}methylcarbamate

tert-Butyl ({1-(2-chloro-5-fluorophenyl)-5-[(pyridin-3-yl)thio]-1H-pyrazol-3-yl}methyl)methylcarbamate (221 mg) was suspended in a mixed solvent of acetonitrile (2 mL) and water (1 mL), sodium percarbonate (771 mg) was added at room temperature, and the mixture was stirred for 24 hr. To the mixture was added water, and the mixture was extracted with ethyl acetate. The extract was washed successively with saturated aqueous sodium thiosulfate solution and saturated brine, dried over anhydrous sodium sulfate, and concentrated under reduced pressure. The residue was purified by silica gel column chromatography (eluent: hexane-ethyl acetate=9:1→3:1) to give the title compound as a colorless oil (182 mg, yield 77%).
¹H-NMR(CDCl₃)δ: 1.51(9H,s),2.88(3H,brs),4.48(2H,brs),7.07-7.19(1H,m),7.20-7.42(4H,m),7.83(1H,d,J=8.0Hz),8.64(1H,d,J=2.5Hz),8.81(1H,dd,J= 4.5,1.5Hz).

### Reference Example 409

### tert-butyl {[1-(3-chloro-2-fluorophenyl)-5-(pyridin-3-ylsulfonyl)-1H-pyrazol-3-yl]methyl}methylcarbamate

tert-Butyl ({1-(3-chloro-2-fluorophenyl)-5-[(pyridin-3-yl)thio]-1H-pyrazol-3-yl}methyl)methylcarbamate (425 mg) was suspended in a mixed solvent of acetonitrile (8 mL) and water (5 mL), sodium percarbonate (4.6 g) was added at room temperature. The mixture was stirred for 18 hr, and filtered, and the filtrate was concentrated under reduced pressure. To the mixture was added water, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by basic silica gel column chromatography (eluent: hexane-ethyl acetate=1:1) to give the title compound as a colorless oil (381 mg, yield 83%).
¹H-NMR(CDCl₃)δ: 1.50(9H,s),2.88(3H,brs),4.47(2H,brs),7.13(1H,brs),7.20-7.25(1H,m),7.30-7.40(2H,m),7.54-7.60(1H,m),7.77-7.80(1H,m),8.66-8.67(1H,m),8.80-8.82(1H,m).

### Reference Example 410

### tert-butyl ({1-(3-chloro-2-fluorophenyl)-5-[(6-chloropyridin-3-yl)sulfonyl]-1H-pyrazol-3-yl}methyl)methylcarbamate

To a solution of tert-butyl ({1-(3-chloro-2-fluorophenyl)-5-[(6-chloropyridin-3-yl)thio]-1H-pyrazol-3-yl}methyl)methylcarbamate (545 mg) in ethyl acetate (8 mL) was added 3-chloroperbenzoic acid (968 mg). The mixture was stirred at room temperature for 12 hr, treated with saturated aqueous sodium thiosulfate solution, and extracted with ethyl acetate. The extract was washed successively with water, saturated aqueous sodium hydrogen carbonate solution and saturated brine, dried over anhydrous sodium sulfate, and concentrated under reduced pressure. The residue was purified by silica gel column chromatography (eluent: hexane-ethyl acetate=4:1) to give the title compound as colorless crystals (433 mg, yield 85%).
¹H-NMR(CDCl₃)δ: 1.50(9H,s),2.89(3H,brs),4.46(2H,br),7.11(1H,brs),7.21-7.27(1H,m),7.32-7.36(1H,m),7.38-7.41(1H,m),7.56-7.61(1H,m),7.71-7.75(1H,m),8.40(1H,d,J=2.7Hz).

### Reference Example 411

### tert-butyl ({1-(3-chloro-2-fluorophenyl)-5-[(6-methylpyridin-3-yl)sulfonyl]-1H-pyrazol-3-yl}methyl)methylcarbamate

To a suspension of tert-butyl ({1-(3-chloro-2-fluorophenyl)-5-[(6-chloropyridin-3-yl)sulfonyl]-1H-pyrazol-3-yl}methyl)methylcarbamate (427 mg) and [1,3-bis(diphenylphosphino)propane]dichloronickel (II) (44 mg) in tetrahydrofuran (5 mL) was added dropwise 35% methylmagnesium bromide-ether solution (1.1 mL) at 0°C, and the mixture was stirred at room temperature for 2 hr. Saturated aqueous ammonium chloride solution was added, and the mixture was extracted with ethyl acetate. The extract was washed successively with saturated aqueous sodium hydroxide solution, water and saturated brine, dried over anhydrous sodium sulfate, and concentrated under reduced pressure. The residue was purified by silica gel column chromatography (eluent: hexane-ethyl acetate=1:1) to give the title compound as a yellow oil (237 mg, yield 58%).
¹H-NMR(CDCl₃) δ: 1.50(9H,s),2.64(3H,s),2.88(3H,brs),4.45(2H,brs),7.09(1H,brs),7 .19-7.22(2H,m),7.34-7.38(1H,m),7.53-7.58(1H,m),7.63-7.66(1H,m),8.48(1H,d,J=2.7Hz).

### Reference Example 412

### tert-butyl 5-[(3-fluorophenyl)sulfonyl]-1-(2-fluoropyridin-3-yl)-1H-pyrazol-3-yl}methyl)methylcarbamate

To a solution of tert-butyl ({5-[(3-fluorophenyl)thio]-1-(2-fluoropyridin-3-yl)-1H-pyrazol-3-yl}methyl)methylcarbamate (170 mg) in ethyl acetate (4 mL) was added 3-chloroperbenzoic acid (417 mg). The mixture was stirred at room temperature for 12 hr, treated with saturated aqueous sodium thiosulfate solution, and extracted with ethyl acetate. The extract was washed successively with water, saturated aqueous sodium hydrogen carbonate solution and saturated brine, dried over anhydrous sodium sulfate, and concentrated under reduced pressure. The residue was purified by silica gel column chromatography (eluent: hexane-ethyl acetate=9:1→7:3) to give the title compound as a colorless oil (167 mg, yield 92%).
¹H-NMR(CDCl₃)δ: 1.49(9H,s),2.90(3H,brs),4.46(2H,brs),7.08(1H,brs),7.22(1H,dt,J =7.7,2.1Hz),7.27-7.51(4H,m),7.90(1H,ddd,J=9.1,7.6,1.9Hz),8.36(1H,dt,J=4.8,1.6Hz ).

### Reference Example 413

### ethyl 1-(2-fluoropyridin-3-yl)-5-[(3-methoxyphenyl)sulfonyl]-1H-pyrazole-3-carboxylate

To a solution of ethyl 1-(2-fluoropyridin-3-yl)-5-[(3-methoxyphenyl)thiol-1H-pyrazole-3-carboxylate (268 mg) in ethyl acetate (3 mL) was added 3-chloroperbenzoic acid (762 mg). The mixture was stirred at room temperature for 3 hr, treated with saturated aqueous sodium thiosulfate solution, and extracted with ethyl acetate. The extract was washed successively with water, saturated aqueous sodium hydrogen carbonate solution and saturated brine, dried over anhydrous magnesium sulfate, and concentrated under reduced pressure. The residue was purified by silica gel column chromatography (eluent: hexane-ethyl acetate=17:3→1:1) to give the title compound as a colorless solid (289 mg, yield 99%).
¹H-NMR(CDCl₃)δ: 1.40(3H,t,J=7.2Hz),3.79(3H,s),4.44(2H,q,J=7.2Hz),7.00-7.07(1H,m),7.09-7.21(2H,m),7.32-7.45(2H,m),7.59(1H,s),7.90(1H,ddd,J=9.1,7.6,1.9Hz),8.38(1H,dt, J=4.5,1.7Hz).

### Reference Example 414

### ethyl 1-(2-chloropyridin-3-yl)-5-(phenylsulfonyl)-1H-pyrazole-3-carboxylate

To a solution of ethyl 1-(2-chloropyridin-3-yl)-5-(phenylthio)-1H-pyrazole-3-carboxylate (228 mg) in ethyl acetate (5 mL) was added 3-chloroperbenzoic acid (609 mg). The mixture was stirred at room temperature for 16 hr, treated with saturated aqueous sodium thiosulfate solution, and extracted with ethyl acetate. The extract was washed successively with saturated aqueous sodium hydrogen carbonate solution, water and saturated brine, dried over anhydrous sodium sulfate, and concentrated under reduced pressure. The residue was purified by basic silica gel column chromatography (eluent: hexane-ethyl acetate=9:1→3:1) to give the title compound as colorless crystals (198 mg, yield 79%).
¹H-NMR(CDCl₃)δ: 1.41(3H,t,J=7.2Hz),4.44(2H,q,J=7.2Hz),7.41-7.51(5H,m),7.60-7.66(2H,m),7.87(1H,dd,J=7.8,1.8Hz),8.56(1H,dd,J=4.8,1.8Hz).

### Reference Example 415

### {1-(2-fluoropyridin-3-yl)-5-[(3-methoxyphenyl)sulfonyl]-1H-pyrazol-3-yl}methanol

To a solution of ethyl 1-(2-fluoropyridin-3-yl)-5-[(3-methoxyphenyl)sulfonyl]-1H-pyrazole-3-carboxylate (283 mg) in tetrahydrofuran (3.5 mL) was added dropwise 1.5 mol/L diisobutylaluminum hydride-toluene solution (1.9 mL) at -78°C. The reaction mixture was allowed to room temperature and stirred at the same temperature for 4 hr. Sodium sulfate 10 hydrate was added, and the mixture was further stirred at room temperature for 1.5 hr. The reaction mixture was filtered through celite, and the filtrate was concentrated under reduced pressure. The residue was purified by silica gel column chromatography (eluent: hexane-ethyl acetate=7:3→3:7) to give the title compound as a colorless oil (173 mg, yield 68%).
¹H-NMR(CDCl₃) δ: 2.00(1H,t,J=6.0Hz),3.78(3H,s),4.77(2H,d,J=6.0Hz),7.01-7.04(1H,m),7.09-7.12(1H,m),7.13-7.16(2H,m),7.31-7.39(2H,m),7.84-7.93(1H,m),8.31-8.38(1H,m).

### Reference Example 416

### [1-(2-chloropyridin-3-yl)-5-(phenylsulfonyl)-1H-pyrazol-3-yl]methanol

A solution of ethyl 1-(2-chloropyridin-3-yl)-5-(phenylsulfonyl)-1H-pyrazole-3-carboxylate (573 mg) in tetrahydrofuran (5 mL) was cooled to -78°C, and 1.5 mol/L diisobutylaluminum hydride-toluene solution (3.9 mL) was added dropwise. The reaction mixture was allowed to warm to 0°C and stirred at the same temperature for 3 hr. Sodium sulfate 10 hydrate was added, and the mixture was further stirred at room temperature for 3 hr. The reaction mixture was filtered through celite, and the filtrate was concentrated under reduced pressure. The residue was purified by silica gel column chromatography (eluent: hexane-ethyl acetate=4:1→2:1) to give the title compound as a colorless oil (463 mg, yield 91%).
¹H-NMR(CDCl₃)δ:2.09(1H,brs),4.78(2H,d,J=5.5Hz),7.19(1H,s),7.35-7.53(5H,m),7.55-7.67(1H,m),7.86(1H,dd,J=7.8,1.8Hz),8.54(1H,dd,J=4.8,1.8Hz).

### Reference Example 417

### 1-(2-fluoropyridin-3-yl)-5-[(3-methoxyphenyl)sulfonyl]-1H-pyrazole-3-carbaldehyde

{1-(2-Fluoropyridin-3-yl)-5-[(3-methoxyphenyl)sulfonyl]-1H-pyrazol-3-yl}methanol(173 mg) was dissolved in toluene (3 mL), manganese dioxide (332 mg) was added, and the mixture was stirred at 90°C for 18 hr. The reaction mixture was allowed to cool to room temperature, and filtered through celite. The filtrate was concentrated under reduced pressure to give the title compound as a colorless oil (167 mg, yield 97%).
¹H-NMR(CDCl₃)δ: 3.79(3H,s),6.96-7.06(1H,m),7.08-7.20(2H,m),7.31-7.46(2H,m),7.53(1H,s),7.90-8.01(1H,m),8.35-8.48(1H,m),10.00(1H,s).

### Reference Example 418

### 1-(2-chloropyridin-3-yl)-5-(phenylsulfonyl)-1H-pyrazole-3-carbaldehyde

[1-(2-Chloropyridin-3-yl)-5-(phenylsulfonyl)-1H-pyrazol-3-yl]methanol (463 mg) was dissolved in a mixed solvent of toluene (5 mL) and acetone (5 mL), manganese dioxide (765 mg) was added, and the mixture was stirred at 80°C for 14 hr. The reaction mixture was allowed to cool to room temperature, and filtered through celite. The filtrate was concentrated under reduced pressure. The residue was purified by silica gel column chromatography (eluent: hexane-ethyl acetate=4:1→2:1) to give the title compound as a colorless oil (360 mg, yield 78%).
¹H-NMR(CDCl₃)δ: 7.41-7.54(5H,m),7.59(1H,s),7.62-7.69(1H,m),7.91-7.97(1H,m),8.61(1H,dd,J=4.9,1.9Hz),10.01(1H,s).

### Reference Example 419

### tert-butyl {[1-(2-fluoropyridin-3-yl)-5-(phenylsulfonyl)-1H-pyrazole-3-yl]methyl}methylcarbamate

To a solution of 1-(2-fluoropyridin-3-yl)-5-(phenylsulfonyl)-1H-pyrazole-3-carbaldehyde (393 mg) in methanol (5 mL) were added methylammonium chloride (88 mg), anhydrous magnesium sulfate (215 mg) and triethylamine (133 mg), and the mixture was stirred at room temperature for 1 hr. Sodium borohydride (54 mg) was added under ice-cooling, and the mixture was further stirred at room temperature for 1 hr. The solvent was evaporated under reduced pressure, and ethyl acetate and water were added to the residue. To the mixture was added di-tert-butyl bicarbonate (312 mg). After the mixture was stirred at room temperature for 30 min, the organic layer was separated. The organic layer was washed with saturated brine, dried over anhydrous sodium sulfate, and concentrated under reduced pressure. The residue was purified by basic silica gel column chromatography (eluent: hexane-ethyl acetate=9:1→3:1) to give the title compound as a colorless oil (403 mg, yield 76%).
¹H-NMR(CDCl₃)δ: 1.49(9H,s),2.89(3H,brs),4.44(2H,brs),7.30-7.66(7H,m),7.85-7.96(1H,m),8.33(1H,dt,J=3.3,1.6Hz).

### Reference Example 420

### tert-butyl ({1-(2-fluoropyridin-3-yl)-5-[(3-methoxyphenyl)sulfonyl]-1H-pyrazole-3-yl}methyl)methylcarbamate

To a solution of 1-(2-fluoropyridin-3-yl)-5-[(3-methoxyphenyl)sulfonyl]-1H-pyrazole-3-carbaldehyde (167 mg) in methanol (2.5 mL) were added methylammonium chloride (34 mg), anhydrous magnesium sulfate (84 mg) and triethylamine (52 mg), and the mixture was stirred at room temperature for 1.5 hr. Sodium borohydride (21 mg) was added under ice-cooling, and the mixture was further stirred at room temperature for 1 hr. The solvent was evaporated under reduced pressure, and ethyl acetate (3 mL) and water (3 mL) were added to the residue. To the mixture was added di-tert-butyl bicarbonate (151 mg). The ethyl acetate layer and aqueous layer were separated and the aqueous layer was extracted again with ethyl acetate. The combined organic layers were washed with saturated brine, dried over anhydrous sodium sulfate, and concentrated under reduced pressure. The residue was purified by basic silica gel column chromatography (eluent: hexane-ethyl acetate=19:1→7:3) to give the title compound as a colorless oil (190 mg, yield 86%).
¹H-NMR(CDCl₃) δ: 1.49(9H,s),2.90(3H,brs),3.77(3H,s),4.45(2H,brs),6.95-7.09(2H,m),7.08-7.18(2H,m),7.29-7.41(2H,m),7.79-7.98(1H,m),8.34(1H,dt,J=4.8,1.4Hz).

### Reference Example 421

### tert-butyl {[1-(2-methoxypyridin-3-yl)-5-(phenylsulfonyl)-1H-pyrazole-3-yl]methyl}methylcarbamate

tert-Butyl {[1-(2-fluoropyridin-3-yl)-5-(phenylsulfonyl)-1H-pyrazole-3-yl]methyl}methylcarbamate (403 mg) was dissolved in methanol (3 mL), 28% sodium methoxide-methanol solution (2 mL) was added at room temperature. The mixture was stirred for 2 hr, and concentrated under reduced pressure. Water was added to the residue, and the mixture was extracted with ethyl acetate. The extract was washed with saturated brine, dried over anhydrous sodium sulfate, and concentrated under reduced pressure The residue was purified by silica gel column chromatography (eluent: hexane-ethyl acetate=7:1→3:1) to give the title compound as a colorless oil (323 mg, yield 78%).
¹H-NMR(CDCl₃)δ: 1.51(9H,s),2.89(3H,brs),3.44(3H,s),4.43(2H,brs),6.96-7.13(2H,m),7.35-7.50(4H,m),7.53-7.61(1H,m),7.62-7.69(1H,m),8.25(1H,dd,J=4.9,1.9Hz).

### Reference Example 422

### tert-butyl {[1-(2-chloropyridin-3-yl)-5-(phenylsulfonyl)-1H-pyrazole-3-yl]methyl}methylcarbamate

1-[1-(2-Chloropyridin-3-yl)-5-(phenylsulfonyl)-1H-pyrazole-3-yl]-N-methylmethanamine hydrochloride (181 mg) was suspended in ethyl acetate (10 mL), saturated aqueous sodium hydrogen carbonate solution (10 mL) and di-tert-butyl bicarbonate (119 mg) were added. The mixture was stirred at room temperature for 15 min, and the organic layer was separated, washed with saturated brine, dried over anhydrous sodium sulfate, and concentrated under reduced pressure. The residue was purified by silica gel column chromatography (eluent: hexane-ethyl acetate=7:1→3:1) to give the title compound as a colorless oil (172 mg, yield 82%).
¹H-NMR(CDCl₃)δ:1.45-1.54(9H,m),2.88(3H,brs),4.49(2H,brs),6.98-7.17(1H,m),7.36-7.52(5H,m),7.55-7.68(1H,m),7.88(1H,dd,J=8.0,1.4Hz),8.53(1H,dd,J=4.8,1.8Hz).

### Reference Example 423

### tert-butyl {[1-(2-cyanopyridin-3-yl)-5-(phenylsulfonyl)-1H-pyrazole-3-yl]methyl}methylcarbamate

tert-Butyl {[1-(2-chloropyridin-3-yl)-5-(phenylsulfonyl)-1H-pyrazole-3-yl]methyl}methylcarbamate (172 mg) was dissolved in N,N-dimethylformamide (3 mL), zinc cyanide (87 mg) and tetrakis(triphenylphosphine)palladium(0) (86 mg) were added. The mixture was microwaved at 140°C for 3 hr. The mixture was cooled to room temperature, water was added, and the mixture was extracted with ethyl acetate. The organic layer was washed with water and saturated brine, dried over anhydrous sodium sulfate, and concentrated under reduced pressure. The residue was purified by silica gel column chromatography (eluent: hexane-ethyl acetate= 9:1→4:1) to give the title compound as a colorless oil (154 mg, yield 91%).
¹H-NMR(CDCl₃)δ: 1.49 (9H, s),2.89(3H, brs),4.49 (2H, brs), 7.41-7.54(5H,m),7.60-7.74(2H,m),8.04(1H,s),8.77-8.83(1H,m).

### Example 1

### 1-[4-(2-fluorophenyl)-5-(pyridin-3-ylsulfonyl)-2-thienyl]-N-methylmethanamine fumarate

4-(2-Fluorophenyl)-5-(pyridin-3-ylsulfonyl)thiophene-2-carbaldehyde (361 mg) was dissolved in a mixed solvent of tetrahydrofuran (10 mL) and methanol (3 mL), and 40% methylamine-methanol solution (1.1 mL) was added. After stirring overnight at room temperature, the reaction mixture was concentrated under reduced pressure. The residue was dissolved in methanol, sodium borohydride (840 mg) was added under ice-cooling, and the mixture was further stirred at room temperature for 4 hr. The solvent was evaporated under reduced pressure, water was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=1:1→1:9) to give the free base of the title compound (194 mg, yield 51%). To a solution of fumaric acid (62 mg) in ethanol (10 mL) was added a solution of the obtained free base in ethyl acetate (5 mL), and the solvent was evaporated under reduced pressure. The obtained crude crystals were recrystallized from a mixed solvent of ethanol and water to give the title compound as colorless crystals (153 mg, yield 59%).
¹H-NMR(DMSO-d₆)δ: 2.38(3H,s), 3.99(2H,s), 6.58(2H,s), 7.12-7.28(4H,m), 7.46-7.56(2H,m), 7.83-7.87(1H,m), 8.49-8.50(1H,m), 8.78-8.80(1H,m),3H not detected.

### Example 2

### 1-[4-(2-fluoropyridin-3-yl)-5-(phenylsulfonyl)-2-thienyl]-N-methylmethanamine hydrochloride

tert-Butyl {[4-(2-fluoropyridin-3-yl)-5-(phenylsulfonyl)-2-thienyl]methyl}methylcarbamate (165 mg) was dissolved in a mixed solvent of ethyl acetate (3 mL) and 2-propanol (3 mL), and 4 mol/L hydrogen chloride-ethyl acetate solution (5 mL) was added at room temperature. After stirring for 3 hr, the reaction mixture was concentrated under reduced pressure. The residue was recrystallized from ethanol to give the title compound as colorless crystals (88.5 mg, yield 62%).
¹H-NMR(DMSO-d₆)δ: 2.57(3H,s),4.42(2H,s), 7.44-7.56(5H,m), 7.66-7.72(1H,m),7.81-7.87(1H,m),8.32-8.34(1H,m),9.37(2H,br).

### Example 3

### 3-{5-[(methylamino)methyl]-2-(phenylsulfonyl)-3-thienyl}pyridine-2-carbonitrile hydrochloride

tert-Butyl {[4-(2-cyanopyridin-3-yl)-5-(phenylsulfonyl)-2-thienyl]methyl}methylcarbamate (238 mg) was dissolved in a mixed solvent of ethyl acetate (3 mL) and 2-propanol (3 mL), and 4 mol/L hydrogen chloride-ethyl acetate solution (3 mL) was added at room temperature. After stirring for 3 hr, the reaction mixture was concentrated under reduced pressure. The residue was recrystallized from ethanol to give the title compound as colorless crystals (136 mg, yield 66%). ¹H-NMR(DMSO-d₆)δ: 2.54(3H,s),4.47(2H,s), 7.40-7.43(2H,m), 7.50-7.56(3H,m), 7.68-7.74(1H,m),7.82-7.92(2H,m), 8.82-8.84(1H,m), 9.58(2H,brs).

### Example 4

### 1-{4-(2-fluorophenyl)-5-[(3-methoxyphenyl)sulfonyl]-2-thienyl}-N-methylmethanamine fumarate

tert-Butyl ({4-(2-fluorophenyl)-5-[(3-methoxyphenyl)sulfonyl]-2-thienyl}methyl)methylcarbamate (308 mg) was dissolved in a mixed solvent of ethyl acetate (3 mL) and ethanol (2 mL), and 4 mol/L hydrogen chloride-ethyl acetate solution (5 mL) was added at room temperature. After stirring for 1.5 hr, the reaction mixture was concentrated under reduced pressure. Saturated aqueous sodium hydrogen carbonate solution was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=1:1→1:9) to give the free base of the title compound. To a solution of fumaric acid (73 mg) in ethanol (10 mL) was added a solution of the obtained free base in ethyl acetate (5 mL), and the solvent was evaporated under reduced pressure. The obtained crude crystals were recrystallized from ethanol to give the title compound as colorless crystals (216 mg, yield 68%).
¹H-NMR(DMSO-d₆)δ: 2.37(3H,s), 3.69(3H,s), 3.96(2H,s), 6.58(2H,s), 6.82-6.83(1H,m), 7.06-7.10(2H,m), 7.17-7.27(4H,m), 7.39-7.56(2H,m),3H not detected.

### Example 5

### 1-{5-[(3-fluorophenyl)sulfonyl]-4-(2-fluoropyridin-3-yl)-2-thienyl}-N-methylmethanamine hydrochloride

tert-Butyl ({5-[(3-fluorophenyl)sulfonyl]-4-(2-fluoropyridin-3-yl)-2-thienyl}methyl)methylcarbamate (140 mg) was dissolved in a mixed solvent of ethyl acetate (2 mL) and 2-propanol (1 mL), and 4 mol/L hydrogen chloride-ethyl acetate solution (3 mL) was added at room temperature. After stirring for 3 hr, the reaction mixture was concentrated under reduced pressure. The residue was recrystallized from ethanol to give the title compound as colorless crystals (77.8 mg, yield 64%).
¹H-NMR(DMSO-d₆)δ: 2.57(3H,s), 4.43(2H,s), 7.18-7.22(1H,m), 7.35-7.38(1H,m), 7.46-7.51(2H,m), 7.56-7.63(2H,m), 7.83-7.89(1H,m), 8.34-8.37(1H,m), 9.34(2H,brs).

### Example 6

### 1-{4-(2-chloropyridin-3-yl)-5-[(3-fluorophenyl)sulfonyl]-2-thienyl}-N-methylmethanamine hydrochloride

tert-Butyl ({4-(2-chloropyridin-3-yl)-5-[(3-fluorophenyl)sulfonyl]-2-thienyl}methyl)methylcarbamate (186 mg) was dissolved in a mixed solvent of ethyl acetate (3 mL) and 2-propanol (2 mL), and 4 mol/L hydrogen chloride-ethyl acetate solution (3 mL) was added at room temperature. After stirring for 4 hr, the reaction mixture was concentrated under reduced pressure. The residue was recrystallized from ethanol to give the title compound as colorless crystals (82.4 mg, yield 51%).
¹H-NMR(DMSO-d₆)δ: 2.56(3H,s), 4.44(2H,s), 7.08-7.12(1H,m), 7.29-7.32(1H,m), 7.44(1H,s), 7.55-7.62(3H,m), 7.77-7.80(1H,m), 8.51-8.53(1H,m), 9.38(2H,brs).

### Example 7

### 1-[4-(2-fluorophenyl)-3-methyl-5-(phenylsulfonyl)-2-thienyl]-N-methylmethanamine hydrochloride

tert-Butyl {[4-(2-fluorophenyl)-3-methyl-5-(phenylsulfonyl)-2-thienyl]methyl}methylcarbamate (140 mg) was dissolved in a mixed solvent of ethyl acetate (1 mL) and ethanol (0.3 mL), and 4 mol/L hydrogen chloride-ethyl acetate solution (1.4 mL) was added at room temperature. After stirring for 4 hr, the reaction mixture was concentrated under reduced pressure. The residue was solidified with diisopropyl ether to give the title compound as a colorless solid (81 mg, yield 67%).
¹H-NMR(DMSO-d₆)δ: 1.90(3H,s), 2.62(3H,s), 4.40(2H,s), 7.01-7.10(1H,m), 7.19(1H,t,J=9.1Hz), 7.29(1H,t,J=7.0Hz), 7.34-7.41(2H,m), 7.42-7.60(3H,m), 7.62-7.72(1H,m),9.09(2H,brs).

### Example 8

### N-methyl-1-[3-methyl-4-(2-methylphenyl)-5-(phenylsulfonyl)-2-thienyl]methanamine hydrochloride

tert-Butyl methyl{[3-methyl-4-(2-methylphenyl)-5-(phenylsulfonyl)-2-thienyl]methyl}carbamate (135 mg) was dissolved in a mixed solvent of ethyl acetate (1 mL) and ethanol (0.3 mL), and 4 mol/L hydrogen chloride-ethyl acetate solution (1.35 mL) was added at room temperature. After stirring for 4 hr, the reaction mixture was concentrated under reduced pressure. The residue was solidified with diisopropyl ether to give the title compound as a colorless solid (94 mg, yield 71%).
¹H-NMR(DMSO-d₆)δ: 1.45(3H,s), 1.81(3H,s), 2.61(3H,s), 4.40(2H,s), 6.78(1H,d,J=6.4Hz), 7.14-7.26(2H,m), 7.26-7.32(2H,m), 7.32-7.39(1H,m),7.41-7.51(2H,m), 7.67(1H,t,J=7.6Hz), 9.17(2H,brs).

### Example 9

### 1-[4-(2-fluoropyridin-3-yl)-3-methyl-5-(phenylsulfonyl)-2-thienyl]-N-methylmethanamine hydrochloride

tert-Butyl {[4-(2-fluoropyridin-3-yl)-3-methyl-5-(phenylsulfonyl)-2-thienyl]methyl}methylcarbamate (48 mg) was dissolved in a mixed solvent of ethyl acetate (0.5 mL) and ethanol (0.1 mL), and 4 mol/L hydrogen chloride-ethyl acetate solution (0.48 mL) was added at room temperature. After stirring for 4 hr, the reaction mixture was concentrated under reduced pressure. The residue was solidified with diisopropyl ether to give the title compound as a colorless solid (38 mg, yield 92%).
¹H-NMR(DMSO-d₆)δ: 1.92(3H,s), 2.62(3H,s), 4.42(2H,s), 7.36-7.44(2H,m), 7.45-7.57(3H,m), 7.64-7.78(2H,m), 8.36(1H,d,J=1.1Hz), 9.08(2H,brs).

### Example 10

### 1-[4-(2-chloropyridin-3-yl)-3-methyl-5-(phenylsulfonyl)-2-thienyl]-N-methylmethanamine hydrochloride

tert-Butyl {[4-(2-chloropyridin-3-yl)-3-methyl-5-(phenylsulfonyl)-2-thienyl]methyl}methylcarbamate (53 mg) was dissolved in a mixed solvent of ethyl acetate (0.5 mL) and ethanol (0.1 mL), and 4 mol/L hydrogen chloride-ethyl acetate solution (0.53 mL) was added at room temperature. After stirring for 4 hr, the reaction mixture was concentrated under reduced pressure. The residue was solidified with diisopropyl ether to give the title compound as a colorless solid (38 mg, yield 83%).
¹H-NMR(DMSO-d₆)δ: 1.91(3H,s), 2.62(3H,brs), 4.45(2H,brs), 7.35-7.42(2H,m), 7.48-7.56(2H,m), 7.56-7.61(1H,m), 7.62-7.76(2H,m), 8.54(1H,dd,J=4.8,2.0Hz), 9.04(2H,brs).

### Example 11

### 1-{4-(2-fluoropyridin-3-yl)-5-[(3-methoxyphenyl)sulfonyl]-2-thienyl}-N-methylmethanamine hydrochloride

To a solution of tert-butyl ({4-(2-fluoropyridin-3-yl)-5-[(3-methoxyphenyl)sulfonyl]-2-thienyl}methyl)methylcarbamate (225 mg) in ethanol (4 mL) was added 4 mol/L hydrogen chloride-ethyl acetate solution (4 mL), and the mixture was stirred at room temperature for 3 hr. The reaction mixture was concentrated under reduced pressure, and the residue was recrystallized from ethanol to give the title compound as colorless crystals (109 mg, yield 55%).
¹H-NMR(DMSO-d₆)δ: 2.57(3H,s), 3.72(3H,s), 4.42(2H,s),6.85-6.86(1H,m), 7.09-7.12(1H,m), 7.24-7.27(1H,m), 7.43-7.51(3H,m), 7.83-7.89(1H,m), 8.33-8.35(1H,m), 9.30(2H,brs).

### Example 12

### 1-[4-(2-fluorophenyl)-5-(phenylsulfonyl)-1,3-thiazol-2-yl]-N-methylmethanamine hydrochloride

To a solution of tert-butyl {[4-(2-fluorophenyl)-5-(phenylsulfonyl)-1,3-thiazol-2-yl]methyl}methylcarbamate (250 mg) in ethanol (3 mL) was added 4 mol/L hydrogen chloride-ethyl acetate solution (4 mL), and the mixture was stirred at room temperature for 2 hr. The reaction mixture was concentrated under reduced pressure, and the residue was recrystallized from ethanol to give the title compound as colorless crystals (142 mg, yield 66%).
¹H-NMR(DMSO-d₆)δ: 2.62(3H,s), 4.64(2H,s), 7.22-7.36(3H,m), 7.52-7.59(5H,m), 7.69-7.74(1H,m), 9.68(2H,brs).

### Example 13

### 1-{4-(2-fluorophenyl)-5-[(3-methoxyphenyl)sulfonyl]-1,3-thiazol-2-yl}-N-methylmethanamine hydrochloride

To a solution of tert-butyl ({4-(2-fluorophenyl)-5-[(3-methoxyphenyl)sulfonyl]-1,3-thiazol-2-yl}methyl)methylcarbamate (178 mg) in ethanol (3 mL) was added 4 mol/L hydrogen chloride-ethyl acetate solution (3 mL), and the mixture was stirred at room temperature for 2 hr. The reaction mixture was concentrated under reduced pressure, and the residue was recrystallized from ethanol to give the title compound as colorless crystals (98 mg, yield 64%).
¹H-NMR(CDCl₃)δ: 2.76(3H,s), 3.72(3H,s), 4.53(2H,s), 6.98-7.07(3H,m), 7.16-7.31(3H,m), 7.41-7.46(2H,m), 10.26(2H,brs).

### Example 14

### 1-[4-(2-fluoropyridin-3-yl)-5-(phenylsulfonyl)-1,3-thiazol-2-yl]-N-methylmethanamine hydrochloride

To a solution of tert-butyl {[4-(2-fluoropyridin-3-yl)-5-(phenylsulfonyl)-1,3-thiazol-2-yl]methyl}methylcarbamate (211 mg) in ethanol (5 mL) was added 4 mol/L hydrogen chloride-ethyl acetate solution (5 mL), and the mixture was stirred at room temperature for 3 hr. The reaction mixture was concentrated under reduced pressure, and the residue was recrystallized from ethanol-ethyl acetate to give the title compound as colorless crystals (95 mg, yield 53%).
¹H-NMR(CDCl₃)δ: 2.81(3H,s), 4.58(2H,s), 7.29-7.34(1H,m), 7.41-7.46(2H,m), 7.56-7.64(3H,m), 7.99-8.05(1H,m), 8.29-8.31(1H,m), 10.27(2H,brs).

### Example 15

### 1-{4-(2-fluoropyridin-3-yl)-5-[(3-methoxyphenyl)sulfonyl]-1,3-thiazol-2-yl}-N-methylmethanamine hydrochloride

To a solution of tert-butyl ({4-(2-fluoropyridin-3-yl)-5-[(3-methoxyphenyl)sulfonyl]-1,3-thiazol-2-yl}methyl)methylcarbamate (199 mg) in ethanol (2 mL) was added 4 mol/L hydrogen chloride-ethyl acetate solution (2 mL), and the mixture was stirred at room temperature for 4 hr. The reaction mixture was concentrated under reduced pressure, and the residue was recrystallized from ethanol-ethyl acetate to give the title compound as colorless crystals (85 mg, yield 49%).
¹H-NMR(CDCl₃)δ: 2.80(3H,s), 3.77(3H,s), 4.57(2H,s), 7.08-7.11(2H,m), 7.19-7.22(1H,m), 7.29-7.36(2H,m), 7.99-8.05(1H,m) 8.29-8.31(1H,m), 10.26(2H,brs).

### Example 16

### 1-[5-[(3-chlorophenyl)sulfony]-4-(2-fluoropyridin-3-yl)-1,3-thiazol-2-yl]-N-methylmethanamine hydrochloride

To a solution of tert-butyl {[5-[(3-chlorophenyl)sulfonyl]-4-(2-fluoropyridin-3-yl)-1,3-thiazol-2-yl]methyl}methylcarbamate (137 mg) in ethanol (2 mL) was added 4 mol/L hydrogen chloride-ethyl acetate solution (2 mL), and the mixture was stirred at room temperature for 2 hr. The reaction mixture was concentrated under reduced pressure, and the residue was recrystallized from ethanol-ethyl acetate to give the title compound as colorless crystals (42 mg, yield 36%).
¹H-NMR(CDCl₃)δ: 2.82(3H,s), 4.59(2H,s), 7.32-7.42(2H,m), 7.51-7.56(3H,m), 7.98-8.04(1H,m), 8.33-8.34(1H,m), 10.31(2H,brs).

### Example 17

### 1-[5-[(3-fluorophenyl)sulfonyl]-4-(2-fluoropyridin-3-yl)-1,3-thiazol-2-yl]-N-methylmethanamine hydrochloride

To a solution of tert-butyl {[5-[(3-fluorophenyl)sulfonyl]-4-(2-fluoropyridin-3-yl)-1,3-thiazol-2-yl]methyl}methylcarbamate (69 mg) in ethanol (1 mL) was added 4 mol/L hydrogen chloride-ethyl acetate solution (2 mL), and the mixture was stirred at room temperature for 2 hr. The reaction mixture was concentrated under reduced pressure, and the residue was recrystallized from ethanol-ethyl acetate to give the title compound as colorless crystals (22 mg, yield 37%).
¹H-NMR(CDCl₃)δ: 2.82(3H,s), 4.57(2H,s), 7.27-7.37(3H,m), 7.43-7.49(2H,m), 8.00-8.06(1H,m), 8.33-8.34(1H,m), 10.24(2H,brs).

### Example 18

### 1-[2-(2-fluorophenyl)-1-(2-thienylsulfonyl)-1H-imidazol-4-yl]-N-methylmethanamine fumarate

2-(2-Fluorophenyl)-1-(2-thienylsulfonyl)-1H-imidazole-4-carbaldehyde (200 mg) was dissolved in a solution of methylamine hydrochloride (401 mg) in methanol (20 mL), and the mixture was stirred for 5 min. Sodium triacetoxyborohydride (378 mg) was added, and the mixture was stirred for 30 min. The reaction mixture was concentrated under reduced pressure at 30°C or less, saturated aqueous sodium hydrogen carbonate solution was added to the residue, and the mixture was extracted with ethyl acetate. The extract was washed with saturated brine, dried over anhydrous magnesium sulfate, and concentrated under reduced pressure. The residue was purified by basic silica gel column chromatography (eluent: ethyl acetate-methanol=97:3), a solution of fumaric acid (104 mg) in methanol (5 mL) was added, and the mixture was concentrated under reduced pressure. The residue was crystallized from ethyl acetate-methanol (9:1) to give the title compound as colorless crystals (86 mg, yield 31%).
¹H-NMR(DMSO-d₆)δ: 2.43(3H,s), 3.86(2H,s), 6.50(2H,s), 7.24(1H,dd,J=4.9,3.8Hz), 7.27-7.39(3H,m), 7.59-7.68(2H,m), 7.89(1H,s), 8.22(1H,dd,J=4.9,1.5Hz),3H not detected.

### Example 19

### 1-[4-(2-fluorophenyl)-5-(phenylsulfonyl)thiophen-2-yl]-N-methylmethanamine hydrochloride

4-(2-Fluorophenyl)-5-(phenylsulfonyl)thiophene-2-carbaldehyde (200 mg) was dissolved in a mixed solvent of tetrahydrofuran (3 mL) and methanol (1 mL), and 40% methylamine-methanol solution (0.6 mL) was added at room temperature. The mixture was stirred for 18 hr, sodium borohydride (66 mg) was added under ice-cooling, and the mixture was further stirred at room temperature for 30 min. The solvent was evaporated under reduced pressure, water was added to the residue, and the mixture was extracted with ethyl acetate. The extract was washed with saturated brine, dried over anhydrous sodium sulfate, and concentrated under reduced pressure. The residue was purified by silica gel column chromatography (eluent: ethyl acetate→methyl acetate:methanol=99:1) to give the free base of the title compound as a pale-yellow oil. The obtained free base was dissolved in ethyl acetate (5 mL), and 4 mol/L hydrogen chloride-ethyl acetate solution (3 mL) was added. The reaction mixture was concentrated under reduced pressure, and the residue was crystallized from a mixed solvent of ethyl acetate and 2-propanol, and recrystallized from a mixed solvent of ethyl acetate and ethanol to give the title compound as colorless crystals (86 mg, yield 38%).
¹H-NMR(DMSO-d₆)δ: 2.57(3H,s),4.40(2H,s),7.17-7.29(3H,m),7.36(1H,s),7.43-7.55(5H,m),7.64-7.69(1H,m),9.22(2H,brs).

### Example 20 (comparative)

### N-{[4-(2-fluorophenyl)-5-(phenylsulfonyl)thiophen-2-yl]methyl}ethaneamine hydrochloride

4-(2-Fluorophenyl)-5-(phenylsulfonyl)thiophene-2-carbaldehyde (200 mg) was dissolved in a mixed solvent of tetrahydrofuran (1 mL) and methanol (1 mL), and 2 mol/L ethylamine-tetrahydrofuran solution (2.9 mL) was added at room temperature. The reaction mixture was stirred for 18 hr, and concentrated under reduced pressure. The residue was dissolved in methanol (3 mL), sodium borohydride (66 mg) was added under ice-cooling, and the mixture was further stirred at room temperature for 30 min. The solvent was evaporated under reduced pressure, water was added to the residue, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=1:4→1:9) to give the free base of the title compound as a pale-yellow oil. The obtained free base was dissolved in ethyl acetate (5 mL), and 4 mol/L hydrogen chloride-ethyl acetate solution (3 mL) was added. The reaction mixture was concentrated under reduced pressure, and the residue was recrystallized from a mixed solvent of ethyl acetate and ethanol to give the title compound as colorless crystals (109 mg, yield 46%).
¹H-NMR(DMSO-d₆)8: 1.21(3H,t,J=7.2Hz),2.98(2H,t,J=7.2Hz),4.42(2H,brs),7.17-7.30(3H,m),7.38(1H,s),7.45-7.56(5H,m),7.64-7.70(1H,m),9.23(2H,brs).

### Example 21

### 1-[4-(2-fluorophenyl)-5-(phenylsulfonyl)thiophen-2-yl]-N,N-dimethylmethanamine hydrochloride

4-(2-Fluorophenyl)-5-(phenylsulfonyl)thiophene-2-carbaldehyde (200 mg) was dissolved in a mixed solvent of tetrahydrofuran (1 mL) and methanol (1 mL), and 2 mol/L N-dimethylamine-tetrahydrofuran solution (2.9 mL) was added at room temperature. The reaction mixture was stirred for 18 hr, and concentrated under reduced pressure. The residue was dissolved in methanol (3 mL), sodium borohydride (66 mg) was added under ice-cooling, and the mixture was further stirred at room temperature for 30 min. The solvent was evaporated under reduced pressure, water was added to the residue, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=3:1→1:2) and basic silica gel column chromatography (eluent: hexane-ethyl acetate=9:1→4:1) to give the free base of the title compound as a colorless oil. The obtained free base was dissolved in ethyl acetate (5 mL), and 4 mol/L hydrogen chloride-ethyl acetate solution (3 mL) was added. The reaction mixture was concentrated under reduced pressure, and the residue was recrystallized from a mixed solvent of ethyl acetate and ethanol to give the title compound as colorless crystals (62 mg, yield 26%).
¹H-NMR(DMSO-d₆)δ: 2.74(6H, brs), 4.55(2H, br), 7.16-7.28(3H,m),7.40(1H,brs),7.43-7.55(5H,m),7.64-7.70(1H,m),10.50(1H,brs).

### Example 22 (comparative)

### 1-{[4-(2-fluoropyridin-3-yl)-5-(phenylsulfonyl)thiophen-2-yl]methyl}azetidin-3-ol

To a solution of 4-(2-fluoropyridin-3-yl)-5-(phenylsulfonyl)thiophene-2-carbaldehyde (104 mg) in methanol (2 mL) was added 3-azetidinol (109 mg), and the mixture was stirred at room temperature for 0.5 hr. Sodium triacetoxyborohydride (159 mg) was added to the reaction mixture under ice-cooling, and the mixture was stirred at room temperature for 18 hr. Saturated aqueous sodium hydrogen carbonate solution was added to the reaction mixture, and the mixture was stirred at room temperature for 0.5 hr, and extracted with ethyl acetate. The extract was washed with saturated brine, dried over anhydrous magnesium sulfate, and concentrated under reduced pressure. The residue was purified by silica gel column chromatography (eluent: hexane-ethyl acetate=1:1→ethyl acetate→ethyl acetate-methanol=19:1) to give the title compound as a white powder (21 mg, yield 17%).
¹H-NMR(CDCl₃)δ: 1.71-1.97(1H,m), 2.99-3.08(2H,m), 3.70-3.87(4H,m),4.44-4.56(1H,m),6.83(1H,s),7.27-7.31(1H,m),7.32-7.42(2H,m),7.47-7.56(3H,m),7.89-8.00(1H,m),8.21-8.28(1H,m).

### Example 23

### 1-[4-(2-fluoropyridin-3-yl)-5-{[3-(methylsulfonyl)phenyl]sulfonyl}thiophen-2-yl]-N-methylmethanamine hydrochloride

4-(2-Fluoropyridin-3-yl)-5-{[3-(methylsulfonyl)phenyl]sulfonyl}thiophene-2-carbaldehyde (297 mg) was dissolved in a mixed solvent of tetrahydrofuran (5 mL) and methanol (2 mL), and 40% methylamine-methanol solution (0.7 mL) was added at room temperature. The reaction mixture was stirred for 18 hr, and concentrated under reduced pressure. The residue was dissolved again in a mixed solvent of tetrahydrofuran (5 mL) and methanol (2 mL), sodium borohydride (79 mg) was added ice-cooling, and the mixture was further stirred at room temperature for 2 hr. The reaction mixture was treated with 1 mol/L hydrochloric acid under ice-cooling, and the solvent was evaporated under reduced pressure. Aqueous sodium hydrogen carbonate solution was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=4:1→1:3) to give the free base of the title compound as a pale-yellow oil. The obtained free base was dissolved in ethyl acetate (5 mL), and 4 mol/L hydrogen chloride-ethyl acetate solution (3 mL) was added. The reaction mixture was concentrated under reduced pressure, and the residue was recrystallized from a mixed solvent of ethyl acetate and ethanol to give the title compound as colorless crystals (191 mg, yield 57%).
¹H-NMR(DMSO-d₆)δ: 2.57(3H, s), 3.30(3H, s), 4.44(2H, s) 7.46-7.50(2H,m),7.83-7.89(4H,m),8.26-8.35(2H,m),9.33(2H,brs).

### Example 24

### 1-{4-(2-fluoropyridin-3-yl)-5-[(6-methoxypyridin-2-yl)sulfonyl]thiophen-2-yl}-N-methylmethanamine fumarate

4-(2-Fluoropyridin-3-yl)-5-[(6-methoxypyridin-2-yl)sulfonyl]thiophene-2-carbaldehyde (363 mg) was dissolved in a mixed solvent of tetrahydrofuran (5 mL) and methanol (2 mL), and 40% methylamine-methanol solution (1.0 mL) was added at room temperature. The reaction mixture was stirred for 18 hr, and concentrated under reduced pressure. The residue was dissolved again in a mixed solvent of tetrahydrofuran (5 mL) and methanol (2 mL), sodium borohydride (69 mg) was added under ice-cooling, and the mixture was stirred at room temperature for 1 hr. Water was added to the reaction mixture, and the mixture was extracted with ethyl acetate. The extract was washed with saturated brine, dried over anhydrous sodium sulfate, and concentrated under reduced pressure. The residue was purified by silica gel column chromatography (eluent: ethyl acetate→ethyl acetate-methanol=99:1) to give the free base of the title compound as a colorless oil (171 mg). The obtained free base was dissolved in ethyl acetate (5 mL), and the solution was added to a solution of fumaric acid (24 mg) in ethanol (10 mL). The solvent was evaporated under reduced pressure, and the residue was recrystallized from ethanol to give the title compound as colorless crystals (86 mg, yield 18%).
¹H-NMR(DMSO-d₆)δ: 2.36(3H,s),3.78(3H,s),3.97(2H,s),6.58(2H,s),7.09(1H,d,J=5.1Hz) ,7.17(1H,s),7.36-7.40(2H,m),7.85-7.93(2H,m),8.24-8.25(1H,m),3H: not detected.

### Example 25

### 1-{4-(2-fluoropyridin-3-yl)-5-[(6-methylpyridin-3-yl)sulfonyl]thiophen-2-yl}-N-methylmethanamine fumarate

4-(2-Fluoropyridin-3-yl)-5-[(6-methylpyridin-3-yl)sulfonyl]thiophene-2-carbaldehyde (650 mg) was dissolved in a solution of methylamine hydrochloride (1.21 g) in methanol (30 mL), and anhydrous magnesium sulfate (2.0 g) was added. Then sodium triacetoxyborohydride (1.14 g) was added, and the mixture was stirred for 10 min. Anhydrous magnesium sulfate (2.0 g) and methylamine hydrochloride (1.21 g) were added to the reaction mixture, and the mixture was stirred for 10 min. Sodium triacetoxyborohydride (1.14 g) was added, and the mixture was stirred for 10 min. Anhydrous magnesium sulfate (2.0 g) and methylamine hydrochloride (1.21 g) were added again to the reaction mixture, and the mixture was stirred for 10 min. Sodium triacetoxyborohydride (1.14 g) was added, and the mixture was stirred for 10 min. Saturated aqueous sodium hydrogen carbonate solution was added to the reaction mixture, and the mixture was extracted with ethyl acetate. The extract was washed with saturated brine, dried over anhydrous magnesium sulfate, and concentrated under reduced pressure. The residue was purified by basic silica gel column chromatography (eluent: ethyl acetate→ethyl acetate-methanol=9:1), a methanol solution of fumaric acid (100 mg) was added, and the mixture was concentrated under reduced pressure. The residue was crystallized from ethyl acetate-methanol (4:1) to give the title compound as colorless crystals (300 mg, yield 34%).
¹H-NMR(DMSO-d₆)δ: 2.38(3H,s),2.54(3H,s),3.98(2H,s),6.59(2H,s),7.17(1H,s),7.41-7.49(2H,m),7.78(1H,dd,J=8.3,2.7Hz),7.86(1H,ddd,J=9.7,7.6,2.1Hz ),8.31-8.35(1H,m),8.45(1H,d,J=2.3Hz), 3H not detected.

### Example 26

### 1-{4-(2-fluoropyridin-3-yl)-5-[(6-methoxypyridin-3-yl)sulfonyl]thiophen-2-yl}-N-methylmethanamine fumarate

4-(2-Fluoropyridin-3-yl)-5-[(6-methoxypyridin-3-yl)sulfonyl]thiophene-2-carbaldehyde (515 mg) was dissolved in a mixed solvent of tetrahydrofuran (5 mL) and methanol (2 mL), and 40% methylamine-methanol solution (1.3 mL) was added at room temperature. The reaction mixture was stirred for 18 hr, and concentrated under reduced pressure. The residue was dissolved again in a mixed solvent of tetrahydrofuran (5 mL) and methanol (2 mL), sodium borohydride (69 mg) was added under ice-cooling, and the mixture was stirred at room temperature for 1 hr. Water was added to the reaction mixture, and the mixture was extracted with ethyl acetate. The extract was washed with saturated brine, dried over anhydrous sodium sulfate, and concentrated under reduced pressure. The residue was purified by silica gel column chromatography (eluent: ethyl acetate→ethyl acetate-methanol=99:1) to give the free base of the title compound as a colorless oil (171 mg). The obtained free base was dissolved in ethyl acetate (5 mL), and the solution was added to a solution of fumaric acid (50 mg) in ethanol (10 mL). The solvent was evaporated under reduced pressure, and the residue was recrystallized from ethanol to give the title compound as colorless crystals (165 mg, yield 27%).
¹H-NMR(DMSO-d₆)δ: 2.36(3H,s),3.91(3H,s),3.94(2H,s),6.59(2H,s),6.92-6.95(1H,m),7.14(1H,s),7.43-7.47(1H,m),7.71-7.75(1H,m),7.82-7.88(1H,m),8.20-8.21(1H,m),8.31-8.32(1H,m),3H: not detected.

### Example 27

### 1-{4-(2-fluoropyridin-3-yl)-5-[(1-methyl-1H-pyrazol-4-yl)sulfonyl]thiophen-2-yl}-N-methylmethanamine hydrochloride

4-(2-Fluoropyridin-3-yl)-5-[(1-methyl-1H-pyrazol-4-yl)sulfonyl]thiophene-2-carbaldehyde (294 mg) was dissolved in a mixed solvent of tetrahydrofuran (5 mL) and methanol (2 mL), and 40% methylamine-methanol solution (0.8 mL) was added at room temperature. The reaction mixture was stirred for 18 hr, and concentrated under reduced pressure. The residue was dissolved again in methanol (2 mL), sodium borohydride (95 mg) was added at room temperature, and the mixture was further stirred for 1 hr. The reaction mixture was treated with 1 mol/L hydrochloric acid under ice-cooling, and the solvent was evaporated under reduced pressure. Aqueous sodium hydrogen carbonate solution was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=2:1→1:2) to give the free base of the title compound as a pale-yellow oil. The obtained free base was dissolved in ethyl acetate (5 mL), and 4 mol/L hydrogen chloride-ethyl acetate solution (3 mL) was added. The reaction mixture was concentrated under reduced pressure, and the residue was recrystallized from ethanol to give the title compound as colorless crystals (153 mg, yield 45%).
¹H-NMR(DMSO-dₛ)δ:2.56(3H,s),3.82(3H,s),4.40(2H,s),7.42-7.51(3H,m),7.86-7.92(1H,m),8.15(1H,s),8.34-8.36(1H,m), 9.41(2H,brs).

### Example 28

### 1-[4-(2-fluoropyridin-3-yl)-5-(1H-pyrrol-1-ylsulfonyl)thiophen-2-yl]-N-methylmethanamine fumarate

4-(2-Fluoropyridin-3-yl)-5-(1H-pyrrol-1-ylsulfonyl)thiophene-2-carbaldehyde (210 mg) was dissolved in a solution of methylamine hydrochloride (422 mg) in methanol (20 mL), sodium triacetoxyborohydride (400 mg) was added, and the mixture was stirred for 10 min. Anhydrous magnesium sulfate (2.0 g) and methylamine hydrochloride (422 mg) were added to the reaction mixture, and the mixture was stirred for about 1 min. Sodium triacetoxyborohydride (400 mg) was added, and the mixture was further stirred for 1 hr, and concentrated under reduced pressure at 30°C. Saturated aqueous sodium hydrogen carbonate solution was added to the residue, and the mixture was extracted with ethyl acetate. The extract was washed with saturated brine, dried over anhydrous magnesium sulfate, and concentrated under reduced pressure. The residue was purified by basic silica gel column chromatography (eluent: ethyl acetate→ethyl acetate-methanol 19:1), and a methanol solution of fumaric acid (17 mg) was added. The mixture was concentrated under reduced pressure, and the residue was crystallized from ethyl acetate-methanol (9:1) to give the title compound as colorless crystals (37 mg, yield 13%).
¹H-NMR(DMSO-d₆)δ:2.35(3H,s),3.93(2H,s),6.21-6.40(2H,m),6.60(2H,s),6.81-6.96(2H,m),7.16(1H,d,J=0.8Hz),7.44-7.51(1H,m),7.84-7.91(1H,m),8.32-8.38(1H,m),3H: not detected.

### Example 29 (comparative)

### (2-fluoro-3-{5-[(methylamino)methyl]-2-(phenylsulfonyl)thiophen-3-yl}phenyl)methanol fumaric acid

tert-Butyl ({4-[2-fluoro-3-(hydroxymethyl)phenyl]-5-(phenylsulfonyl)thiophen-2-yl}methyl)methylcarbamate (137 mg) was dissolved in a mixed solvent of ethyl acetate (1 mL) and ethanol (1 mL), and 4 mol/L hydrogen chloride-ethyl acetate solution (3 mL) was added. The mixture was stirred at room temperature for 4 hr, and concentrated under reduced pressure. Saturated aqueous sodium hydrogen carbonate solution was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=2:1→1:4) to give the free base of the title compound as a colorless oil (70 mg). The obtained free base was dissolved in ethyl acetate (5 mL), and the solution was added to a solution of fumaric acid (21 mg) in ethanol (10 mL). The solvent was evaporated under reduced pressure, and the residue was recrystallized from a mixed solvent of ethyl acetate and ethanol to give the title compound as colorless crystals (63 mg, yield 45%).
¹H-NMR(DMSO-d₆)δ: 2.37(3H,s),3.96(2H,s),4.42(2H,s),6.57(2H,s),7.04-7.09(2H,m),7.19-7.24(1H,m),7.41-7.55(5H,m),7.61-7.66(1H,m),4H: not detected.

### Example 30

### N-methyl-1-[4-(1-methyl-1H-pyrazol-5-yl)-5-(phenylsulfonyl)thiophen-2-yl]methanamine fumarate

tert-Butyl methyl{[4-(1-methyl-1H-pyrazol-5-yl)-5-(phenylsulfonyl)thiophen-2-yl]methyl}carbamate (169 mg) was dissolved in a mixed solvent of ethyl acetate (1 mL) and ethanol (1 mL), and 4 mol/L hydrogen chloride-ethyl acetate solution (3 mL) was added. The mixture was stirred at room temperature for 3 hr, and concentrated under reduced pressure. Saturated aqueous sodium hydrogen carbonate solution was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=1:1→1:9) to give the free base of the title compound as a colorless oil (150 mg). The obtained free base was dissolved in ethyl acetate (5 mL), and the solution was added to a solution of fumaric acid (44 mg) in ethanol (10 mL). The solvent was evaporated under reduced pressure, and the residue was recrystallized from a mixed solvent of ethyl acetate and ethanol to give the title compound as colorless crystals (110 mg, yield 62%).
¹H-NMR(DMSO-d₆)δ: 2.37(3H,s),3.24(3H,s),3.96(2H,s),6.18(1H,d,J=1.8Hz),6.58(2H,s) ,7.15(1H,s),7.44-7.54(5H,m),7.64-7.69(1H,m),3H: not detected.

### Example 31

### N-methyl-1-[4-(1-methyl-1H-imidazol-2-yl)-5-(phenylsulfonyl)thiophen-2-yl]methanamine dihydrochloride

tert-Butyl methyl{[4-(1-methyl-1H-imidazol-2-yl)-5-(phenylsulfonyl)thiophen-2-yl]methyl}carbamate (313 mg) was dissolved in a mixed solvent of ethyl acetate (1 mL) and ethanol (1 mL), and 4 mol/L hydrogen chloride-ethyl acetate solution (5 mL) was added. The mixture was stirred at room temperature for 2 hr, and concentrated under reduced pressure. The residue was recrystallized from a mixed solvent of ethyl acetate and ethanol to give the title compound as colorless crystals (171 mg, yield 57%).
¹H-NMR(DMSO-d₆)δ: 2.55(3H,br), 3.40(3H,s), 4.47(2H,br), 7.62-7.86(8H,m),9.79(2H,brs),1H: not detected.

### Example 32

### N-methyl-1-[4-(1-methyl-1H-imidazol-5-yl)-5-(phenylsulfonyl)thiophen-2-yl]methanamine fumarate

tert-Butyl methyl{[4-(1-methyl-1H-imidazol-5-yl)-5-(phenylsulfonyl)thiophen-2-yl]methyl}carbamate (295 mg) was dissolved in a mixed solvent of ethyl acetate (1 mL) and ethanol (1 mL), and 4 mol/L hydrogen chloride-ethyl acetate solution (5 mL) was added. The mixture was stirred at room temperature for 1 hr, and concentrated under reduced pressure. Saturated aqueous sodium hydrogen carbonate solution was added to the residue, and the mixture was extracted with ethyl acetate. The extract was washed with saturated brine, dried over anhydrous sodium sulfate, and concentrated under reduced pressure to give the free base of the title compound as a pale-yellow oil (193 mg). The obtained free base was dissolved in ethyl acetate (5 mL), and the solution was added to a solution of fumaric acid (66 mg) in ethanol (10 mL). The solvent was evaporated under reduced pressure, and the residue was recrystallized from ethanol to give the title compound as colorless crystals (171 mg, yield 58%).
¹H-NMR(DMSO-d₆)δ: 2.38(3H,s),3.04(3H,s),3.99(2H,s),6.57(2H,s),6.86(1H,s),7.16(1H ,s),7.41-7.52(4H,m),7.63-7.68(2H,m),3H: not detected.

### Example 33 (comparative)

### 1-{5-[(methylamino)methyl]-2-(phenylsulfonyl)thiophen-3-yl}piperidin-2-one hydrochloride

tert-Butyl methyl{[4-(2-oxopiperidin-1-yl)-5-(phenylsulfonyl)thiophen-2-yl]methyl}carbamate (394 mg) was dissolved in a mixed solvent of ethyl acetate (4 mL) and ethanol (1 mL), and 4 mol/L hydrogen chloride-ethyl acetate solution (4 mL) was added. The mixture was stirred at room temperature for 3 hr, and concentrated under reduced pressure. The residue was recrystallized from a mixed solvent of ethanol and water to give the title compound as colorless crystals (241 mg, yield 71%).
¹H-NMR(DMSO-d₆)8: 1.80-1.93(4H,m),2.26(2H,t,J=6.3Hz),2.55(3H,s),3.43-3.47(2H,m),4.34(2H,s),7.24(1H,s),7.60-7.7.75(3H,m),7.88(2H,d,J=8.4Hz),9.22(2H,brs).

### Example 34

### 3-{2-[(3-fluorophenyl)sulfonyl]-5-[(methylamino)methyl]thiophen-3-yl}pyridine-2-carbonitrile hydrochloride

tert-Butyl ({4-(2-cyanopyridin-3-yl)-5-[(3-fluorophenyl)sulfonyl]thiophen-2-yl}methyl)methylcarbamate (198 mg) was dissolved in a mixed solvent of ethyl acetate (3 mL) and 2-propanol (2 mL), and 4 mol/L hydrogen chloride-ethyl acetate solution (5 mL) was added. The mixture was stirred at room temperature for 4 hr, and concentrated under reduced pressure. The residue was recrystallized from a mixed solvent of ethyl acetate and ethanol to give the title compound as colorless crystals (86 mg, yield 50%).
¹H-NMR(DMSO-d₆)δ:2.55(3H,s),4.48(2H,s),7.14-7.18(1H,m),7.27-7.30(1H,m),7.56-7.63(3H,m),7.84-7.95(2H,m),8.83-8.86(1H,m),9.39(2H,brs).

### Example 35

### 1-[4-(2-chloropyridin-3-yl)-5-(pyridin-3-ylsulfonyl)thiophen-2-yl]-N-methylmethanamine fumarate

tert-Butyl {[4-(2-chloropyridin-3-yl)-5-(pyridin-3-ylsulfonyl)thiophen-2-yl]methyl}methylcarbamate (136 mg) was dissolved in a mixed solvent of ethyl acetate (2 mL) and 2-propanol (1 mL), and 4 mol/L hydrogen chloride-ethyl acetate solution (3 mL) was added. The mixture was stirred at room temperature for 3 hr, and concentrated under reduced pressure. Saturated aqueous sodium hydrogen carbonate solution was added to the residue, and the mixture was extracted with ethyl acetate. The extract was washed with saturated brine, dried over anhydrous sodium sulfate, and concentrated under reduced pressure to give the free base of the title compound as a colorless oil (94 mg). The obtained free base was dissolved in ethyl acetate (5 mL), and the solution was added to a solution of fumaric acid (29 mg) in ethanol (5 mL). The solvent was evaporated under reduced pressure, and the residue was recrystallized from a mixed solvent of ethanol and water to give the title compound as colorless crystals (89 mg, yield 63%).
¹H-NMR(DMSO-d₆)δ: 2.37(3H,s),3.97(2H,s),6.59(2H,s),7.15(1H,s),7.51-7.59(2H,m),7.76-7.85(2H,m),8.48-8.50(2H,m),8.81-8.84(1H,m),3H: not detected.

### Example 36

### 2-{5-[(methylamino)methyl]-2-(pyridin-3-ylsulfonyl)thiophen-3-yl}benzonitrile hydrochloride

tert-Butyl {[4-(2-cyanophenyl)-5-(pyridin-3-ylsulfonyl)thiophen-2-yl]methyl}methylcarbamate (150 mg) was dissolved in a mixed solvent of ethyl acetate (3 mL) and ethanol (1 mL), and 4 mol/L hydrogen chloride-ethyl acetate solution (1 mL) was added. The reaction mixture was stirred at room temperature for 6 hr, and concentrated under reduced pressure. The residue was recrystallized from a mixed solvent of ethanol and water to give the title compound as colorless crystals (93 mg, yield 71%).
¹H-NMR(DMSO-d₆)δ: 2.55(3H,s), 4.47(2H,s), 7.38-7.41(1H,m),7.49(1H,s),7.54-7.59(1H,m),7.67-7.72(1H,m),7.77-7.88(3H,m),8.43-8.44(1H,m),8.83-8.85(1H,m),9.41(2H,brs).

### Example 37

### 3-({3-(2-fluoropyridin-3-yl)-5-[(methylamino)methyl]thiophen-2-yl}sulfonyl)benzonitrile hydrochloride

tert-Butyl ({5-[(3-cyanophenyl)sulfonyl]-4-(2-fluoropyridin-3-yl)thiophen-2-yl}methyl)methylcarbamate (335 mg) was dissolved in a mixed solvent of ethyl acetate (3 mL) and 2-propanol (1 mL), and 4 mol/L hydrogen chloride-ethyl acetate solution (3 mL) was added. The reaction mixture was stirred at room temperature for 6 hr, and concentrated under reduced pressure. The residue was recrystallized from ethanol to give the title compound as colorless crystals (165 mg, yield 57%).
¹H-NMR(DMSO-d₆)δ: 2.57(3H,s), 4.44(2H,s), 7.47-7.52(1H,m), 7.74-7.89(1H,m),8.18-8.21(1H,m),8.35-8.37(1H,m),9.41(2H,brs).

### Example 38

### [3-({3-(2-fluoropyridin-3-yl)-5-[(methylamino)methyl]thiophen-2-yl}sulfonyl)phenyl]methanol fumarate

tert-Butyl {[4-(2-fluoropyridin-3-yl)-5-{[3-(hydroxymethyl)phenyl]sulfonyl}thiophen-2-yl]methyl}methylcarbamate (136 mg) was dissolved in 2-propanol (1 mL), and 4 mol/L hydrogen chloride-1,4-dioxane solution (3 mL) was added at room temperature. The mixture was stirred at room temperature for 3 hr, and concentrated under reduced pressure. Saturated aqueous sodium hydrogen carbonate solution was added to the residue, and the mixture was extracted with ethyl acetate. The extract was washed with saturated brine, dried over anhydrous sodium sulfate, and concentrated under reduced pressure to give the free base of the title compound as a colorless oil (185 mg). The obtained free base was dissolved in ethyl acetate (5 mL), and the solution was added to a solution of fumaric acid (52 mg) in ethanol (10 mL). The solvent was evaporated under reduced pressure, and the residue was recrystallized from ethanol to give the title compound as colorless crystals (122 mg, yield 54%).
¹H-NMR(DMSO-d₆)δ: 2.36(3H,s),3.94(2H,s),4.48(2H,s),6.58(2H,s),7.13(1H,s),7.31-7.33(1H,m),7.41-7.49(3H,m),7.56-7.58(1H,m),7.76-7.83(1H,m),8.30-8.31(1H,m),4H: not detected.

### Example 39

### 1-[4-(2-fluoropyridin-3-yl)-5-(thiophen-3-ylsulfonyl)thiophen-2-yl]-N-methylmethanamine hydrochloride

tert-Butyl{[4-(2-fluoropyridin-3-yl)-5-(thiophen-3-ylsulfonyl)thiophen-2-yl]methyl}methylcarbamate (211 mg) was dissolved in a mixed solvent of ethyl acetate (1 mL) and ethanol (1 mL), and 4 mol/L hydrogen chloride-ethyl acetate solution (2 mL) was added. The mixture was stirred at room temperature for 3 hr, and concentrated under reduced pressure, and the residue was recrystallized from a mixed solvent of ethanol and ethyl acetate to give the title compound as colorless crystals (162 mg, yield 89%).
¹H-NMR(DMSO-d₆)δ: 2.58(3H,s),4.43(2H,s),7.03(1H,dd,J=5.3,1.3Hz),7.43-7.51(2H,m),7.75(1H,dd,J=5.1,3.0Hz),7.82-7.91(1H,m),8.06(1H,dd,J=3.0,1.3Hz),8.32-8.37(1H,m),9.33(2H,brs).

### Example 40

### 1-{4-(2-fluoropyridin-3-yl)-5-[(2-methylfuran-3-yl)sulfonyl]thiophen-2-yl}-N-methylmethanamine hydrochloride

tert-Butyl ({4-(2-fluoropyridin-3-yl)-5-[(2-methylfuran-3-yl)sulfonyl]thiophen-2-yl}methyl)methylcarbamate (187 mg) was dissolved in a mixed solvent of ethyl acetate (1 mL) and ethanol (1 mL), and 4 mol/L hydrogen chloride-ethyl acetate solution (2 mL) was added. The mixture was stirred at room temperature for 3 hr, and concentrated under reduced pressure, and the residue was recrystallized from a mixed solvent of ethanol and ethyl acetate to give the title compound as colorless crystals (142 mg, yield 88%).
¹H-NMR(DMSO-d₆)δ: 2.18(3H,s),2.58(3H,s),4.43(2H,s),6.31(1H,d,J=2.1Hz),7.44(1H,s) ,7.46-7.54(1H,m),7.68(1H,d,J=2.1Hz),7.83-7.93(1H,m),8.32-8.38(1H,m),9.18(2H,brs).

### Example 41

### 1-[4-(2-fluoropyridin-3-yl)-5-(1,3-thiazol-2-ylsulfonyl)thiophen-2-yl]-N-methylmethanamine hydrochloride

tert-Butyl {[4-(2-fluoropyridin-3-yl)-5-(1,3-thiazol-2-ylsulfonyl)thiophen-2-yl]methyl}methylcarbamate (83.5 mg) was dissolved in ethyl acetate (1 mL), and 4 mol/L hydrogen chloride-ethyl acetate solution (1 mL) was added. The mixture was stirred at room temperature for 4 hr, and concentrated under reduced pressure, and the residue was recrystallized from a mixed solvent of ethanol and ethyl acetate to give the title compound as colorless crystals (70.1 mg, yield 97%).
¹H-NMR(DMSO-d₆)δ: 2.59(3H,s), 4.46(2H,s), 7.44-7.53(2H,m), 7.89-7.98(1H,m),8.14(1H,d,J=3.0Hz),8.27(1H,d,J=3.0Hz),8.31-8.40(1H,m),9.22(2H,brs).

### Example 42

### 1-[4-(2-fluoropyridin-3-yl)-5-(1H-imidazol-2-ylsulfonyl)thiophen-2-yl]-N-methylmethanamine hydrochloride

tert-Butyl {[4-(2-fluoropyridin-3-yl)-5-(1H-imidazol-2-ylsulfonyl)thiophen-2-yl]methyl}methylcarbamate (121 mg) was dissolved in a mixed solvent of ethyl acetate (1.5 mL) and ethanol (1.5 mL), and 4 mol/L hydrogen chloride-ethyl acetate solution (3 mL) was added. The mixture was stirred at room temperature for 3 hr, and concentrated under reduced pressure, and the residue was recrystallized from a mixed solvent of ethanol and ethyl acetate to give the title compound as colorless crystals (75.6 mg, yield 73%).
¹H-NMR(DMSO-d₆)δ:2.59(3H,s),4.44(2H,s),7.15-7.49(4H,m),7.83-7.92(1H,m),8.29-8.35(1H,m),9.28(2H,brs),13.81(1H,brs).

### Example 43

### 1-{4-(2-fluoropyridin-3-yl)-5-[(1-methyl-1H-imidazol-2-yl)sulfonyl]thiophen-2-yl}-N-methylmethanamine hydrochloride

tert-Butyl ({4-(2-fluoropyridin-3-yl)-5-[(1-methyl-1H-imidazol-2-yl)sulfonyl]thiophen-2-yl}methyl)methylcarbamate (29.7 mg) was dissolved in a mixed solvent of ethyl acetate (0.5 mL) and ethanol (0.5 mL), and 4 mol/L hydrogen chloride-ethyl acetate solution (1 mL) was added. The mixture was stirred at room temperature for 3 hr, and concentrated under reduced pressure, and the residue was recrystallized from a mixed solvent of ethanol and ethyl acetate to give the title compound as colorless crystals (22.9 mg, yield 89%).
¹H-NMR(DMSO-d₆)δ: 2.60(3H,brs),3.65(3H,s),4.46(2H,brs),7.10(1H,d,J=0.8Hz),7.40-7.51(3H,m),7.75-7.87(1H,m),8.28-8.36(1H,m),9.31(2H,brs).

### Example 44

### 1-{5-[(2-chloropyridin-4-yl)sulfonyl]-4-(2-fluoropyridin-3-yl)thiophen-2-yl}-N-methylmethanamine hydrochloride

tert-Butyl ({5-[(2-chloropyridin-4-yl)sulfonyl]-4-(2-fluoropyridin-3-yl)thiophen-2-yl}methyl)methylcarbamate (70 mg) was dissolved in a mixed solvent of ethyl acetate (1 mL) and ethanol (1 mL), and 4 mol/L hydrogen chloride-ethyl acetate solution (2 mL) was added. The mixture was stirred at room temperature for 3 hr, and concentrated under reduced pressure, and the residue was recrystallized from a mixed solvent of ethanol and ethyl acetate to give the title compound as colorless crystals (53.3 mg, yield 85%).
¹H-NMR(DMSO-d₆)δ: 2.59(3H,s),4.47(2H,s),7.41(1H,d,J=1.5Hz),7.48-7.56(3H,m),7.85-7.94(1H,m),8.36-8.42(1H,m),8.69(1H,d,J=5.1Hz),9.28(2H,brs),

### Example 45

### 1-[4-(2-fluoropyridin-3-yl)-5-(pyridin-4-ylsulfonyl)thiophen-2-yl]-N-methylmethanamine dihydrochloride

tert-Butyl {[4-(2-fluoropyridin-3-yl)-5-(pyridin-4-ylsulfonyl)thiophen-2-yl]methyl}methylcarbamate (80 mg) was dissolved in a mixed solvent of ethyl acetate (1 mL) and ethanol (1 mL), and 4 mol/L hydrogen chloride-ethyl acetate solution (2 mL) was added. The mixture was stirred at room temperature for 3 hr, and concentrated under reduced pressure, and the residue was recrystallized from a mixed solvent of ethanol and ethyl acetate to give the title compound as colorless crystals (64.9 mg, yield 87%).
¹H-NMR(DMSO-d₆)δ: 2.58(3H,t,J=5.3Hz), 4.46(2H,t,J=5.7Hz), 7.43-7.54(4H,m),7.82-7.91(1H,m),8.34-8.40(1H,m),8.81-8.86(2H,m),9.42(2H,brs),1H: not detected.

### Example 46

### 1-{4-(2-fluoropyridin-3-yl)-5-[(1-oxidepyridin-4-yl)sulfonyl]thiophen-2-yl}-N-methylmethanamine hydrochloride

tert-Butyl ({4-(2-fluoropyridin-3-yl)-5-[(1-oxidepyridin-4-yl)sulfonyl]thiophen-2-yl}methyl)methylcarbamate (16.7 mg) was dissolved in ethyl acetate (1 mL), and 4 mol/L hydrogen chloride-ethyl acetate solution (1 mL) was added. The mixture was stirred at room temperature for 4 hr, and concentrated under reduced pressure, and the residue was recrystallized from a mixed solvent of ethanol and ethyl acetate to give the title compound as colorless crystals (9.5 mg, yield 66%).
¹H-NMR(DMSO-d₆)δ: 2.59(3H,brs),4.46(2H,brs),7.38-7.45(2H,m),7.46-7.54(2H,m),7.85-7.93(1H,m),8.28-8.34(2H,m),8.35-8.42(1H,m),9.30(2H,brs).

### Example 47

### 1-{5-[(6-chloropyridin-3-yl)sulfonyl]-4-(2-fluoropyridin-3-yl)thiophen-2-yl}-N-methylmethanamine hydrochloride

tert-Butyl ({5-[(6-chloropyridin-3-yl)sulfonyl]-4-(2-fluoropyridin-3-yl)thiophen-2-yl}methyl)methylcarbamate (75 mg) was dissolved in a mixed solvent of ethyl acetate (1 mL) and ethanol (1 mL), and 4 mol/L hydrogen chloride-ethyl acetate solution (2 mL) was added. The mixture was stirred at room temperature for 3 hr, and concentrated under reduced pressure, and the residue was recrystallized from a mixed solvent of ethanol and ethyl acetate to give the title compound as colorless crystals (53.1 mg, yield 81%). ¹H-NMR(DMSO-d₆)δ: 2.58(3H,s), 4.45(2H,s), 7.45-7.54(2H,m), 7.73-7.79(1H,m),7.85-7.93(1H,m),7.93-7.99(1H,m),8.36-8.40(1H,m),8.46(1H,d,J=2.6Hz),9.24(2H,brs).

### Example 48

### 1-[4-(2-fluoropyridin-3-yl)-5-(pyridin-3-ylsulfonyl)thiophen-2-yl]-N-methylmethanamine fumarate

4-(2-Fluoropyridin-3-yl)-5-(pyridin-3-ylsulfonyl)thiophene-2-carbaldehyde (214 mg) was dissolved in a mixed solvent of tetrahydrofuran (3 mL) and methanol (1 mL), and 40% methylamine-methanol solution (0.6 mL) was added at room temperature. The mixture was stirred for 3 hr, sodium borohydride (70 mg) was added under ice-cooling, and the mixture was further stirred at room temperature for 2 hr. The solvent was evaporated under reduced pressure, water was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=3:1→1:3) to give the free base of the title compound as a pale-yellow oil (86 mg). The obtained free base was dissolved in ethyl acetate (5 mL), and the solution was added to a solution of fumaric acid (27 mg) in ethanol (5 mL). The solvent was evaporated under reduced pressure, and the residue was recrystallized from ethanol to give the title compound as colorless crystals (82 mg, yield 28%). melting point 194-197°C.
¹H-NMR(DMSO-d₆)δ: 2.36(3H,s),3.94(2H,s),6.59(2H,s),7.16(1H,s),7.45-7.48(1H,m),7.56-7.60(1H,m),7.82-7.92(2H,m),8.31-8.32(1H,m),8.57-8.58(1H,m)<8.81-8.83(1H,m),3H: not detected.

### Example 49

### 1-[4-(2-fluoropyridin-3-yl)-5-(pyridin-2-ylsulfonyl)thiophen-2-yl]-N-methylmethanamine fumarate

To a solution of tert-butyl {[4-(2-fluoropyridin-3-yl)-5-(pyridin-2-ylsulfonyl)thiophen-2-yl]methyl}methylcarbamate (228 mg) in ethyl acetate (4 mL) was added 4 mol/L hydrogen chloride-ethyl acetate solution (6 mL), and the mixture was stirred at room temperature for 3 hr, and concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure to give the free base of the title compound as a yellow oil (145 mg). To a solution of the obtained free base (145 mg) in ethyl acetate (3 mL) was added a solution of fumaric acid (48 mg) in ethanol (3 mL), and the mixture was stirred at room temperature for 15 min. The reaction mixture was concentrated under reduced pressure, and the residue was recrystallized from ethanol to give the title compound as a colorless solid (92 mg, yield 39%).
¹H-NMR(DMSO-d₆)δ: 2.39(3H,s),4.00(2H,s),6.58(2H,s),7.18(1H,m),7.35-7.39(1H,m),7.65-7.74(2H,m),7.80-7.86(1H,m),7.99-8.05(1H,m),8.23-8.25(1H,m),8.68(1H,d,J=3.9Hz),3H: not detected.

### Example 50

### 1,1-dideutero-1-[4-(2-fluoropyridin-3-yl)-5-(pyridin-3-ylsulfonyl)thiophen-2-yl]-N-methylmethanamine fumarate

tert-Butyl {dideutero[4-(2-fluoropyridin-3-yl)-5-(pyridin-3-ylsulfonyl)thiophen-2-yl]methyl}methylcarbamate (134 mg) was dissolved in a mixed solvent of ethyl acetate (3 mL) and ethanol (1 mL), and 4 mol/L hydrogen chloride-ethyl acetate solution (3 mL) was added. The mixture was stirred at room temperature for 4 hr, and concentrated under reduced pressure. Saturated aqueous sodium hydrogen carbonate solution was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=2:1→1:2) to give the free base of the title compound as a colorless oil (87 mg). The obtained free base was dissolved in ethyl acetate (5 mL), and the solution was added to a solution of fumaric acid (28 mg) in ethanol (5 mL). The solvent was evaporated under reduced pressure, and the residue was recrystallized from ethanol to give the title compound as colorless crystals (91 mg, yield 66%).
¹H-NMR(DMSO-d₆)δ: 2.37(3H,s)6.59(2H,s),7.18(1H,s),7.43-7.48(1H,m),7.56-7.61(1H,m),7.83-7.92(2H,m),8.31-8.33(1H,m),8.57-8.58(1H,m),8.81-8.84(1H,m),3H: not detected.

### Example 51

### 1-[3-({4-(2-fluorophenyl)-2-[(methylamino)methyl]-1,3-thiazol-5-yl}sulfonyl)phenyl]pyrrolidin-2-one hydrochloride

To a solution of tert-butyl {[4-(2-fluorophenyl)-5-{[3-(2-oxopyrrolidin-1-yl)phenyl]sulfonyl}-1,3-thiazol-2-yl]methyl}methylcarbamate (82 mg) in ethanol (2 mL) was added 4 mol/L hydrogen chloride-ethyl acetate solution (2 mL), and the mixture was stirred at room temperature for 4 hr. The reaction mixture was concentrated under reduced pressure, and the residue was recrystallized from ethanol-water to give the title compound as colorless crystals (37 mg, yield 50%).
¹H-NMR(DMSO-d₆)δ: 2.03-2.13(2H,m), 2.51-2.56(2H,m),2.62(3H,s),3.77(2H,t,J=7.2Hz),4.64(2H,s),7.22-7.36(4H,m),7.54-7.62(2H,m),7.80(1H,dd,J=8.1,2.4Hz),8.09(1H,s),9.55(2H,brs).

### Example 52

### 1-{4-(2-fluorophenyl)-5-[(3-(pyrrolidin-1-yl)phenyl)sulfonyl]-1,3-thiazol-2-yl}-N-methylmethanamine

To a solution of tert-Butyl ({4-(2-fluorophenyl)-5-[(3-(pyrrolidin-1-yl)phenyl)sulfonyl]-1,3-thiazol-2-yl}methyl)methylcarbamate (172 mg) in ethanol (2 mL) was added 4 mol/L hydrogen chloride-ethyl acetate solution (2 mL), and the mixture was stirred at room temperature for 5 hr, and concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: ethyl acetate), and recrystallized from ethyl acetate to give the title compound as a colorless solid (53 mg, yield 38%).
¹H-NMR(CDCl₃)δ: 1.98-2.02(4H,m),2.57(3H,s),3.17-3.21(4H,m),4.05(2H,s),6.61-6.64(1H,m),6.71-6.73(1H,m),6.86-6.89(1H,m),7.02-7.08(1H,m),7.16-7.21(2H,m),7.37-7.44(2H,m),1H: not detected.

### Example 53

### 1-[4-(2-fluorophenyl)-5-{[3-(pyrrolidin-1-ylcarbonyl)phenyl]sulfonyl}-1,3-thiazol-2-yl]-N-methylmethanamine hydrochloride

To a solution of tert-butyl {[4-(2-fluorophenyl)-5-{[3-(pyrrolidin-1-ylcarbonyl)phenyl]sulfonyl}-1,3-thiazol-2-yl]methyl}methylcarbamate (106 mg) in ethanol (3 mL) was added 4 mol/L hydrogen chloride-ethyl acetate solution (4 mL), and the mixture was stirred at room temperature for 3 hr. The reaction mixture was concentrated under reduced pressure, and the residue was recrystallized from ethanol-water to give the title compound as colorless crystals (47 mg, yield 49%).
¹H-NMR(DMSO-d₆)δ: 1.89-1.90(4H,m),2.62(3H,s),3.25(2H,t,J=6.6Hz),3.46(2H,t,J=6.6Hz),4. 64(2H,s),7.21-7.38(3H,m),7.54-7.68(4H,m),7.86-7.88(1H,m),9.54(2H,brs).

### Example 54

### 1-[4-(2-fluorophenyl)-5-{[3-(pyrrolidin-1-ylmethyl)phenyl]sulfonyl}-1,3-thiazol-2-yl]-N-methylmethanamine dihydrochloride

To a solution of tert-butyl {[4-(2-fluorophenyl)-5-{[3-(pyrrolidin-1-ylmethyl)phenyl]sulfonyl}-1,3-thiazol-2-yl]methyl}methylcarbamate (125 mg) in ethanol (2 mL) was added 4 mol/L hydrogen chloride-ethyl acetate solution (4 mL), and the mixture was stirred at room temperature for 2 hr, and concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane:ethyl acetate=1:4) to give the free base of the title compound (86 mg). To a solution of the obtained free base (59 mg) in a mixed solvent of ethyl acetate (2 mL) and ethanol (2 mL) was added 4 mol/L hydrogen chloride-ethyl acetate solution (4 mL), and the mixture was stirred at room temperature for 15 min. The reaction mixture was concentrated under reduced pressure, and the residue was recrystallized from ethanol to give the title compound as colorless crystals (38 mg, yield 14%).
¹H-NMR(DMSO-d₆)δ: 1.87-2.01(4H,m), 2.63(3H,s), 3.00(2H,brs), 4.38(2H,brs), 4.65(2H,brs), 7 .22-7.33(3H,m),7.57-7.65(3H,m),7.91(1H,s),8.02(1H,brs),9.54(1H,brs),11.07(1H,brs), 1H: not detected.

### Example 55

### 1-{5-[(6-chloropyridin-3-yl)sulfonyl]-4-(2-fluorophenyl)-1,3-thiazol-2-yl}-N-methylmethanamine hydrochloride

To a solution of tert-butyl ({5-[(6-chloropyridin-3-yl)sulfonyl]-4-(2-fluorophenyl)-1,3-thiazol-2-yl}methyl)methylcarbamate (89 mg) in a mixed solvent of ethyl acetate (4 mL) and ethanol (4 mL) was added 4 mol/L hydrogen chloride-ethyl acetate solution (4 mL), and the mixture was stirred at room temperature for 3 hr. The reaction mixture was concentrated under reduced pressure, and the residue was recrystallized from ethanol to give the title compound as colorless crystals (38 mg, yield 49%).
¹H-NMR(DMSO-d₆)δ: 2.63(3H,s),4.67(2H,s),7.26-7.39(3H,m),7.58-7.65(1H,m),7.74(1H,d,J=8.4Hz),8.03(1H,dd,J=8.4,2.4Hz),8.51(1H, d,J=2.4Hz),9.60(2H,brs).

### Example 56

### 1-[4-(2-fluorophenyl)-5-(pyridin-3-ylsulfonyl)-1,3-thiazol-2-yl]-N-methylmethanamine fumarate

To a solution of tert-butyl {[4-(2-fluorophenyl)-5-(pyridin-3-ylsulfonyl)-1,3-thiazol-2-yl]methyl}methylcarbamate (213 mg) in ethanol (3 mL) was added 4 mol/L hydrogen chloride-ethyl acetate solution (5 mL), and the mixture was stirred at room temperature for 4 hr, and concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure to give the free base of the title compound as a yellow oil (132 mg). To a solution of the obtained free base (128 mg) in ethyl acetate (2 mL) was added a solution of fumaric acid (42 mg) in ethanol (2 mL), and the mixture was stirred at room temperature for 15 min. The reaction mixture was concentrated under reduced pressure, and the residue was recrystallized from ethanol-diisopropyl ether to give the title compound as a colorless solid (94 mg, yield 43%).
¹H-NMR(DMSO-d₆)δ: 2.42(3H,s),3.98(2H,s),6.60(2H,s),7.20-7.35(3H,m),7.55-7.61(2H,m),7.98-8.00(1H,m),8.63-8.64(1H,m),8.83-8.85(1H,m),3H: not detected.

### Example 57

### 1-{4-(2-fluorophenyl)-5-[(1-methyl-1H-pyrazol-4-yl)sulfonyl]-1,3-thiazol-2-yl}-N-methylmethanamine hydrochloride

To a solution of tert-butyl ({4-(2-fluorophenyl)-5-[(1-methyl-1H-pyrazol-4-yl)sulfonyl]-1,3-thiazol-2-yl}methyl)methylcarbamate (178 mg) in a mixed solvent of ethyl acetate (2 mL) and ethanol (2 mL) was added 4 mol/L hydrogen chloride-ethyl acetate solution (6 mL), and the mixture was stirred at room temperature for 3 hr. The reaction mixture was concentrated under reduced pressure, and the residue was recrystallized from ethanol to give the title compound as colorless crystals (90 mg, yield 59%).
¹H-NMR(DMSO-d₆)δ: 2.63(3H,s),3.82(3H,s),4.63(2H,s),7.28-7.42(3H,m),7.54(1H,s),7.56-7.64(1H,m),8.19(1H,s),9.55(2H,brs).

### Example 58

### 1-{5-[(3,4-dimethoxyphenyl)sulfonyl]-4-(2-fluoropyridin-3-yl)-1,3-thiazol-2-yl}-N-methylmethanamine hydrochloride

To a solution of tert-butyl ({5-[(3,4-dimethoxyphenyl)sulfonyl]-4-(2-fluoropyridin-3-yl)-1,3-thiazol-2-yl}methyl)methylcarbamate (197 mg) in ethanol (3 mL) was added 4 mol/L hydrogen chloride-ethyl acetate solution (3 mL), and the mixture was stirred at room temperature for 3 hr. The reaction mixture was concentrated under reduced pressure, and the residue was recrystallized from ethanol to give the title compound as colorless crystals (105 mg, yield 60%).
¹H-NMR(DMSO-d₆)δ: 2.63(3H,s),3.71(3H,s),3.84(3H,s),4.62(2H,s),6.93(1H,d,J=2.1Hz) ,7.12-7.15(1H,m),7.28-7.31(1H,m),7.52-7.56(1H,m),7.98-8.04(1H,m),8.41-8.43(1H,m),9.27(2H,brs).

### Example 59

### 1-{5-[(6-chloropyridin-3-yl)sulfonyl]-4-(2-fluoropyridin-3-yl)-1,3-thiazol-2-yl}-N-methylmethanamine hydrochloride

To a solution of tert-butyl ({5-[(6-chloropyridin-3-yl)sulfonyl]-4-(2-fluoropyridin-3-yl)-1,3-thiazol-2-yl}methyl)methylcarbamate (202 mg) in a mixed solvent of ethyl acetate (30 mL) and 2-propanol (10 mL) was added 4 mol/L hydrogen chloride-ethyl acetate solution (6 mL), and the mixture was stirred at room temperature for 2 hr. The reaction mixture was concentrated under reduced pressure, and the residue was recrystallized from ethanol to give the title compound as colorless crystals (123 mg, yield 69%).
¹H-NMR(DMSO-d₆)δ: 2.63(3H,s), 4.67(2H,s),7.53-7.58(1H,m),7.79-7.82(1H,m,),8.01-8.07(1H,m),8.09-8.13(1H,m),8.44-8.45(1H,m),8.63-8.64(1H,m),9.46(2H,brs).

### Example 60

### 1-[4-(2-fluoropyridin-3-yl)-5-(pyridin-3-ylsulfonyl)-1,3-thiazol-2-yl]-N-methylmethanamine

To a solution of tert-butyl {[4-(2-fluoropyridin-3-yl)-5-(pyridin-3-ylsulfonyl)-1,3-thiazol-2-yl]methyl}methylcarbamate (361 mg) in a mixed solvent of ethyl acetate (2 mL) and 2-propanol (5 mL) was added 4 mol/L hydrogen chloride-ethyl acetate solution (5 mL), and the mixture was stirred at room temperature for 3 hr, and concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: ethyl acetate:methanol=20:1), and recrystallized from ethyl acetate-hexane to give the title compound as a colorless solid (119 mg, yield 42%).
¹H-NMR(CDCl₃)δ: 1.80(1H,brs),2.60(3H,s),4.08(2H,s),7.25-7.42(2H,m),7.91-7.98(2H,m),8.32-8.35(1H,m),8.78-8.80(1H,m),8.91-8.92(1H,m).

### Example 61

### 1-{5-[(2-chloropyridin-4-yl)sulfonyl]-4-(2-fluoropyridin-3-yl)-1,3-thiazol-2-yl}-N-methylmethanamine hydrochloride

To a solution of tert-butyl ({5-[(2-chloropyridin-4-yl)sulfonyl]-4-(2-fluoropyridin-3-yl)-1,3-thiazol-2-yl}methyl)methylcarbamate (149 mg) in a mixed solvent of ethyl acetate (2 mL) and 2-propanol (2 mL) was added 4 mol/L hydrogen chloride-ethyl acetate solution (4 mL), and the mixture was stirred at room temperature for 3 hr. The reaction mixture was concentrated under reduced pressure, and the residue was recrystallized from 2-propanol-diisopropyl ether to give the title compound as colorless crystals (83 mg, yield 64%.
¹H-NMR(DMSO-d₆)δ: 2.64(3H,s),4.69(2H,s),7.54-7.59(1H,m),7.65-7.68(2H,m,),8.02-8.08(1H,m),8.45-8.46(1H,m),8.72-8.74(1H,m),9.53(2H,brs).

### Example 62

### 1-[5-(2-fluorophenyl)-4-(phenylsulfonyl)furan-2-yl]-N-methylmethanamine hydrochloride

tert-Butyl {[5-(2-fluorophenyl)-4-(phenylsulfonyl)furan-2-yl]methyl}methylcarbamate (326 mg) was dissolved in a mixed solvent of ethyl acetate (2 mL) and ethanol (1 mL), and 4 mol/L hydrogen chloride-ethyl acetate solution (3 mL) was added. The mixture was stirred at room temperature for 2 hr, and concentrated under reduced pressure. The residue was recrystallized from a mixed solvent of ethyl acetate and ethanol to give the title compound as colorless crystals (204 mg, yield 73%).
¹H-NMR(DMSO-d₆)δ: 2.56(3H,s),4.26(2H,s),7.17(1H,s),7.36-7.41(2H,m),7.58-7.82(7H,m),9.28(2H,brs).

### Example 63

### N-methyl-1-[5-(2-methylphenyl)-4-(phenylsulfonyl)furan-2-yl]methanamine hydrochloride

tert-Butyl methyl{[5-(2-methylphenyl)-4-(phenylsulfonyl)furan-2-yl]methyl}carbamate (284 mg) was dissolved in a mixed solvent of ethyl acetate (3 mL) and ethanol(1 mL), and 4 mol/L hydrogen chloride-ethyl acetate solution (3 mL) was added. The mixture was stirred at room temperature for 3 hr, and concentrated under reduced pressure. The residue was crystallized from ethyl acetate, and recrystallized from a mixed solvent of ethyl acetate and ethanol to give the title compound as colorless crystals (86 mg, yield 35%).
¹H-NMR (DMSO-d₆)δ: 1.92(3H,s),2.53(3H,s),4.25(2H,s),7.15(1H,s),7.25-7.33(3H,m),7.44-7.70(6H,m),9.34(2H,brs).

### Example 64

### 1-[5-(2-fluorophenyl)-4-(pyridin-3-ylsulfonyl)thiophen-2-yl]-N-methylmethanamine fumarate

To a solution of tert-butyl {[5-(2-fluorophenyl)-4-(pyridin-3-ylsulfonyl)thiophen-2-yl]methyl}methylcarbamate (135 mg) in ethanol (2 mL) was added 4 mol/L hydrogen chloride-ethyl acetate solution (2 mL), and the mixture was stirred at room temperature for 3 hr, and concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure to give the free base of the title compound as a yellow oil (100 mg). To a solution of the obtained free base (97 mg) in ethyl acetate (2 mL) was added a solution of fumaric acid (33 mg) in ethanol (2 mL), and the mixture was stirred at room temperature for 2 hr. The reaction mixture was concentrated under reduced pressure, and the residue was recrystallized from ethanol-water to give the title compound as a colorless solid (67 mg, yield 48%).
¹H-NMR(DMSO-_{d}6)δ: 2.35(3H,s),3.93(2H,s),6.58(2H,s),7.23-7.36(3H,m),7.54-7.61(3H,m),7.99-8.02(1H,m),8.72(1H,d,J=2.1Hz),8.82-8.84(1H,m),3H: not detected.

### Example 65

### 1-[5-(2-fluoropyridin-3-yl)-4-(pyridin-3-ylsulfonyl)thiophen-2-yl]-N-methylmethanamine fumarate

To a solution of tert-butyl {[5-(2-fluoropyridin-3-yl)-4-(pyridin-3-ylsulfonyl)thiophen-2-yl]methyl}methylcarbamate (145 mg) in a mixed solvent of ethyl acetate (1 mL) and 2-propanol (2 mL) was added 4 mol/L hydrogen chloride-ethyl acetate solution (3 mL), and the mixture was stirred at room temperature for 3 hr, and concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure to give the free base of the title compound as a yellow oil (93 mg). To a solution of the obtained free base (91 mg) in ethyl acetate (2 mL) was added a solution of fumaric acid (31 mg) in ethanol (2 mL), and the mixture was stirred at room temperature for 15 min. The reaction mixture was concentrated under reduced pressure, and the residue was recrystallized from ethanol-water to give the title compound as a colorless solid (76 mg, yield 52%). melting point 196-197°C.
¹H-NMR(DMSO-d₆)δ: 2.35(3H,s),3.94(2H,s),6.58(2H,s),7.46-7.50(1H,m),7.59(1H,s),7.62-7.66(1H,m),7.96-8.03(1H,m),8.05-8.09(1H,m),8.36-8.37(1H,m),8.81-8.82(1H,m),8.85-8.87(1H,m),3H: not detected.

### Example 66

### 1-[5-(2-chloropyridin-3-yl)-4-(pyridin-3-ylsulfonyl)thiophen-2-yl]-N-methylmethanamine fumarate

To a solution of tert-butyl {[5-(2-chloropyridin-3-yl)-4-(pyridin-3-ylsulfonyl)thiophen-2-yl]methyl}methylcarbamate (81 mg) in ethyl acetate (2 mL) was added 4 mol/L hydrogen chloride-ethyl acetate solution (2 mL), and the mixture was stirred at room temperature for 3 hr, and concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure to give the free base of the title compound as a yellow oil (53 mg). To a solution of the obtained free base (51 mg) in ethyl acetate (2 mL) was added a solution of fumaric acid (16 mg) in ethanol (2 mL), and the mixture was stirred at room temperature for 15 min. The reaction mixture was concentrated under reduced pressure, and the residue was recrystallized from ethanol to give the title compound as a colorless solid (25 mg, yield 30%).
¹H-NMR(DMSO-d₆)δ: 2.33(3H,s),3.92(2H,s),6.58(2H,s),7.53-7.63(3H,m),7.90-7.93(1H,m),7.98-8.01(1H,m),8.51-8.54(1H,m),8.72-8.73(1H,m),8.83-8.85(1H,m),3H: not detected.

### Example 67

### 1-[2-(2-fluorophenyl)-1-(phenylsulfonyl)-1H-imidazol-4-yl]-N-methylmethanamine fumarate

2-(2-Fluorophenyl)-1-(phenylsulfonyl)-1H-imidazole-4-carbaldehyde (310 mg) was dissolved in a solution of methylamine hydrochloride (634 mg) in methanol (31 mL), and the solution was stirred for about 5 min. Sodium triacetoxyborohydride (995 mg) was added, and the mixture was stirred for 1 hr. Saturated aqueous sodium hydrogen carbonate solution was added to the reaction mixture, and the mixture was extracted with ethyl acetate. The extract was washed with saturated brine, dried over anhydrous magnesium sulfate, and concentrated under reduced pressure. The residue was purified by basic silica gel column chromatography (eluent: ethyl acetate-methanol=19:1), a methanol solution of fumaric acid (109 mg) was added, and the mixture was concentrated under reduced pressure. The residue was crystallized from ethyl acetate-methanol (4:1) to give the title compound as colorless crystals (189 mg, yield 44%).
¹H-NMR(DMSO-d₆)δ: 2.44(3H,s),3.87(2H,s),6.51(2H,s),7.22-7.32(3H,m),7.58-7.67(5H,m),7.76-7.85(1H,m),7.96(1H,s),3H: not detected.

### Example 68

### 1-[2-(2-fluorophenyl)-1-(thiophen-3-ylsulfonyl)-1H-imidazol-4-yl]-N-methylmethanamine fumarate

2-(2-Fluorophenyl)-1-(thiophen-3-ylsulfonyl)-1H-imidazole-4-carbaldehyde (280 mg) was dissolved in a solution of methylamine hydrochloride (562 mg) in methanol (20 mL), and the mixture was stirred for 5 min. Sodium triacetoxyborohydride (530 mg) was added, and the mixture was stirred for 15 min. The reaction mixture was concentrated under reduced pressure to about 1/4 volume, saturated aqueous sodium hydrogen carbonate solution was added to the residue, and the mixture was extracted with ethyl acetate. The extract was washed with saturated brine, dried over anhydrous magnesium sulfate, and concentrated under reduced pressure. The residue was purified by basic silica gel column chromatography (eluent: ethyl acetate-methanol=97:3), a solution of fumaric acid (97 mg) in methanol (5 mL) was added, and the mixture was concentrated under reduced pressure. The residue was crystallized from ethyl acetate-methanol (9:1) to give the title compound as colorless crystals (190 mg, yield 49%).
¹H-NMR(DMSO-d₆)δ: 2.45(3H,s),3.87(2H,s),6.50(2H,s),7.22(1H,dd,J=5.3,1.5Hz),7.25-7.35(3H,m),7.58-7.66(1H,m),7.84(1H,dd,J=4.9,3.0Hz),7.92(1H,s),8.29(1H,dd,J=2.8 ,1.3Hz),3H: not detected.

### Example 69

### 1-{2-(2-fluorophenyl)-1-[(5-methylthiophen-2-yl)sulfonyl]-1H-imidazol-4-yl}-N-methylmethanamine fumarate

2-(2-Fluorophenyl)-1-[(5-methylthiophen-2-yl)sulfonyl]-1H-imidazole-4-carbaldehyde (340 mg) was dissolved in a solution of methylamine hydrochloride (660 mg) in methanol (30 mL), and the mixture was stirred for about 5 min. Sodium triacetoxyborohydride (620 mg) was added, and the mixture was stirred for 1 hr. The reaction mixture was concentrated under reduced pressure, saturated aqueous sodium hydrogen carbonate solution was added, and the mixture was extracted with ethyl acetate. The extract was washed with saturated brine, dried over anhydrous magnesium sulfate, and concentrated under reduced pressure. The residue was purified by basic silica gel column chromatography (eluent: ethyl acetate-methanol=99:1→19:1), a methanol solution of fumaric acid (113 mg) was added, and the mixture was concentrated under reduced pressure. The residue was crystallized from ethyl acetate-methanol (9:1) to give the title compound as colorless crystals (201 mg, yield 43%).
¹H-NMR(DMSO-d₆)δ: 2.45(3H,s),2.52(3H,s),3.88(2H,s),6.50(2H,s),6.98(1H,dd,J=4.0,1 .0Hz),7.28-7.40(3H,m),7.46(1H,d,J=3.8Hz),7.56-7.68(1H,m),7.86(1H,s),3H: not detected.

### Example 70

### 1-[2-(2-fluorophenyl)-1-(furan-3-ylsulfonyl)-1H-imidazol-4-yl]-N-methylmethanamine fumarate

2-(2-Fluorophenyl)-1-(furan-3-ylsulfonyl)-1H-imidazole-4-carbaldehyde (270 mg) was dissolved in a solution of methylamine hydrochloride (570 mg) in methanol (20 mL), and the mixture was stirred for 5 min. Sodium triacetoxyborohydride (536 mg) was added, and the mixture was stirred for 30 min. The reaction mixture was concentrated under reduced pressure, saturated aqueous sodium hydrogen carbonate solution was added to the residue, and the mixture was extracted with ethyl acetate. The extract was washed with saturated brine, dried over anhydrous magnesium sulfate, and concentrated under reduced pressure. The residue was purified by basic silica gel column chromatography (eluent: ethyl acetate-methanol=97:3), a solution of fumaric acid (98 mg) in methanol (5 mL) was added, and the mixture was concentrated under reduced pressure. The residue was crystallized from ethyl acetate-methanol (9:1) to give the title compound as colorless crystals (207 mg, yield 54%).
¹H-NMR(DMSO-d₆)δ: 2.45(3H,s),3.88(2H,s),6.50(2H,s),6.74-6.80(1H,m),7.27-7.40(3H,m),7.57-7.66(1H,m),7.90(1H,s),7.98(1H,t,J=1.9Hz),8.42(1H,s),3H: not detected.

### Example 71

### 1-{2-(2-fluorophenyl)-1-[(1-methyl-1H-pyrazol-5-yl)sulfonyl]-1H-imidazol-4-yl}-N-methylmethanamine fumarate

2-(2-Fluorophenyl)-1-[(1-methyl-1H-pyrazol-5-yl)sulfonyl]-1H-imidazole-4-carbaldehyde (330 mg) was dissolved in a solution of methylamine hydrochloride (670 mg) in methanol (30 mL), and the mixture was stirred for about 5 min. Sodium triacetoxyborohydride (630 mg) was added, and the mixture was stirred for 1 hr. The reaction mixture was concentrated under reduced pressure, saturated aqueous sodium hydrogen carbonate solution was added, and the mixture was extracted with ethyl acetate. The extract was washed with saturated brine, dried over anhydrous magnesium sulfate, and concentrated under reduced pressure. The residue was purified by basic silica gel column chromatography (eluent: ethyl acetate-methanol=49:1→19:1), a methanol solution of fumaric acid (115 mg) was added, and the mixture was concentrated under reduced pressure. The residue was crystallized from ethyl acetate-methanol (4:1) to give the title compound as colorless crystals (310 mg, yield 68%).
¹H-NMR(DMSO-d₆)δ: 2.48(3H,s),3.78(3H,s),3.94(2H,s),6.50(2H,s),6.63(1H,d,J=2.3Hz) ,7.24-7.37(3H,m),7.56-7.69(2H,m),8.02(1H,s),3H: not detected.

### Example 72

### 1-{2-(2-fluorophenyl)-1-[(3-methylpiperidin-1-yl)sulfonyl]-1H-imidazol-4-yl}-N-methylmethanamine 1.5fumarate

2-(2-Fluorophenyl)-1-[(3-methylpiperidin-1-yl)sulfonyl]-1H-imidazole-4-carbaldehyde (140 mg) was dissolved in a solution of methylamine hydrochloride (270 mg) in methanol (20 mL), and the mixture was stirred for about 5 min. Sodium triacetoxyborohydride (424 mg) was added, and the mixture was stirred for 1 hr. Saturated aqueous sodium hydrogen carbonate solution was added to the reaction mixture, and the mixture was extracted with ethyl acetate. The extract was washed with saturated brine, dried over anhydrous magnesium sulfate, and concentrated under reduced pressure. The residue was purified by basic silica gel column chromatography (eluent: ethyl acetate-methanol=99:1→19:1), a methanol solution of fumaric acid (46 mg) was added, and the mixture was concentrated under reduced pressure. The residue was crystallized from ethyl acetate-methanol (9:1) to give the title compound as colorless crystals (32 mg, yield 15%).
¹H-NMR(DMSO-d₆)δ: 0.80(3H,d,J=6.8Hz),0.88-1.02(1H,m),1.29-1.67(4H,m),2.27(1H,t,J=11.2Hz),2.50(3H,s),2.58(1H,td,J=12.0,2. 5Hz),3.27-3.38(2H,m),3.94(2H,s),6.52(3H,s),7.26-7.37(2H,m),7.47-7.65(2H,m),7.75(1H,s),4H: not detected.

### Example 73

### 1-{2-(2,3-difluorophenyl)-1-[(5-methylthiophen-2-yl)sulfonyl]-1H-imidazol-4-yl}-N-methylmethanamine fumarate

2-(2,3-Difluorophenyl)-1-[(5-methylthiophen-2-yl)sulfonyl]-1H-imidazole-4-carbaldehyde (200 mg) was dissolved in a solution of methylamine hydrochloride (367 mg) in methanol (20 mL), and the mixture was stirred for about 5 min. Sodium triacetoxyborohydride (345 mg) was added, and the mixture was stirred for 1 hr. The reaction mixture was concentrated under reduced pressure, saturated aqueous sodium hydrogen carbonate solution was added, and the mixture was extracted with ethyl acetate. The extract was washed with saturated brine, dried over anhydrous magnesium sulfate, and concentrated under reduced pressure. The residue was purified by basic silica gel column chromatography (eluent: ethyl acetate-methanol=99:1→19:1), a methanol solution of fumaric acid (63 mg) was added, and the mixture was concentrated under reduced pressure. The residue was crystallized from ethyl acetate-methanol (9:1) to give the title compound as colorless crystals (95 mg, yield 35%).
¹H-NMR(DMSO-d₆)δ: 2.43(3H,s),2.53(3H,s),3.84(2H,s),6.51(2H,s),7.00(1H,dd,J=4.0,0 .9Hz),7.18-7.25(1H,m),7.32-7.38(1H,m),7.53(1H,d,J=4.0Hz),7.63-7.74(1H,m),7.88(1H,s),3H: not detected.

### Example 74

### 1-[1-(2-fluorophenyl)-5-(phenylsulfonyl)-1H-pyrazol-3-yl]-N-methylmethanamine hydrochloride

To a suspension of lithium aluminum hydride (31 mg) in tetrahydrofuran (2 mL) was added aluminum chloride (36 mg) under ice-cooling under an argon atmosphere, and the mixture was stirred at room temperature for 30 min. A solution of 1-(2-fluorophenyl)-N-methyl-5-(phenylsulfonyl)-1H-pyrazole-3-carboxamide (39 mg) in tetrahydrofuran (1 mL) was added to the reaction mixture, and the mixture was stirred at room temperature for 18 hr. 15% Aqueous sodium hydroxide solution (0.067 mL), water (0.067 mL) and 15% aqueous sodium hydroxide solution (0.201 mL) were successively added under ice-cooling to the reaction mixture. Then celite and anhydrous magnesium sulfate were added, and the mixture was stirred at room temperature for 30 min. The insoluble material was filtered, and washed with ethyl acetate, and the filtrate was concentrated under reduced pressure. The residue was purified by basic silica gel column chromatography (eluent: hexane-ethyl acetate=3:2→ethyl acetate) to give the free base of the title compound as a colorless oil (29.9 mg). The obtained free base (29.9 mg) was dissolved in a mixed solvent of ethyl acetate (1 mL) and ethanol (1 mL), and 4 mol/L hydrogen chloride-ethyl acetate solution (2 mL) was added. The mixture was stirred at room temperature for 3 hr, and concentrated under reduced pressure, and the residue was solidified with diethyl ether and hexane to give the title compound as a white powder (27.4 mg, yield 66%).
¹H-NMR(DMSO-d₆)δ: 2.60(3H,s),4.25(2H,s),7.30-7.41(3H,m),7.42-7.61(5H,m),7.62-7.82(2H,m),9.13(2H,brs).

### Example 75

### 1-{1-(2-fluorophenyl)-5-[(3-methoxyphenyl)sulfonyl]-1H-pyrazol-3-yl}-N-methylmethanamine fumarate

To a suspension of lithium aluminum hydride (77.5 mg) in tetrahydrofuran (3 mL) was added aluminum chloride (91 mg) under ice-cooling under an argon atmosphere, and the mixture was stirred at room temperature for 30 min. A solution of 1-(2-fluorophenyl)-5-[(3-methoxyphenyl)sulfonyl]-N-methyl-1H-pyrazole-3-carboxamide (136 mg) in tetrahydrofuran (2 mL) was added to the reaction mixture, and the mixture was stirred at room temperature for 18 hr. 15% Aqueous sodium hydroxide solution (0.168 mL), water (0.168 mL) and 15% aqueous sodium hydroxide solution (0.504 mL) were successively added to the reaction mixture under ice-cooling. Then celite and anhydrous magnesium sulfate were added, and the mixture was stirred at room temperature for 30 min. The insoluble material was filtered, and washed with ethyl acetate, and the filtrate was concentrated under reduced pressure. The residue was purified by basic silica gel column chromatography (eluent: hexane-ethyl acetate=1:1→ethyl acetate) to give the free base of the title compound as a colorless oil (78 mg). The obtained free base (78 mg) was dissolved in ethyl acetate (1 mL), and a solution of fumaric acid (24.1 mg) in ethanol (2 mL) was added. The reaction mixture was concentrated under reduced pressure, and the residue was recrystallized from a mixed solvent of ethanol and ethyl acetate to give the title compound as colorless crystals (48.4 mg, yield 29%).
¹H-NMR(DMSO-d₆)δ: 2.37(3H,s),3.73(3H,s),3.84(2H,s),6.53(2H,s),6.85-6.91(1H,m),7.09-7.1.7(1H,m),7.25-7.39(5H,m),7.44-7.54(1H,m),7.59-7.69(1H,m),3H: not detected.

### Example 76

### 1-{5-[(3-methoxyphenyl)sulfonyl]-1-(2-methylphenyl)-1H-pyrazol-3-yl}-N-methylmethanamine hydrochloride

To a suspension of lithium aluminum hydride (95 mg) in tetrahydrofuran (10 mL) was added aluminum chloride (1.0 g) under ice-cooling, and the mixture was stirred at the same temperature for 30 min. A solution of 5-[(3-methoxyphenyl)sulfonyl]-N-methyl-1-(2-methylphenyl)-1H-pyrazole-3-carboxamide (1.75 g) in tetrahydrofuran (5 mL) was added dropwise under ice-cooling, and the mixture was stirred at room temperature for 6 hr. The reaction mixture was cooled again, treated with 4 mol/L aqueous sodium hydroxide solution, dried over anhydrous sodium sulfate, and filtered. The filtrate was concentrated under reduced pressure, and ethyl acetate and 1 mol/L aqueous sodium hydroxide solution were added to the residue. The ethyl acetate layer was washed with saturated brine, dried over anhydrous sodium sulfate, and concentrated under reduced pressure. The residue was purified by basic silica gel column chromatography (eluent: hexane-ethyl acetate=2:1→1:4) to give the free base of the title compound as a colorless oil (225 mg). To a solution of the obtained free base in ethyl acetate (3 mL) was added 4 mol/L hydrogen chloride-ethyl acetate solution (3 mL). The solvent was evaporated under reduced pressure, and the residue was crystallized from a mixed solvent of ethyl acetate and diisopropyl ether, and recrystallized from a mixed solvent of ethyl acetate and ethanol to give the title compound as colorless crystals (137 mg, yield 26%).
¹H-NMR(DMSO-d₆)δ: 1.49(3H,s),2.58(3H,s),3.70(3H,s),4.23(2H,s),6.71-6.73(1H,s),7.05-7.09(2H,m),7.25-7.33(3H,m),7.43-7.50(3H,m),9.24(2H,br).

### Example 77

### N-methyl-1-{1-(2-methylphenyl)-5-[(6-methylpyridin-3-yl)sulfonyl]-1H-pyrazol-3-yl}methanamine fumarate

To a suspension of aluminum chloride (122 mg) in tetrahydrofuran (5 mL) was slowly added lithium aluminum hydride (38 mg) at 0°C, and the mixture was stirred at the same temperature for 10 min. A solution of N-methyl-1-(2-methylphenyl)-5-[(6-methylpyridin-3-yl)sulfonyl]-1H-pyrazole-3-carboxamide (113 mg) in tetrahydrofuran (2 mL) was added dropwise at 0°C to the obtained suspension, and the mixture was stirred at room temperature for 1 hr. 8 mol/L Aqueous sodium hydroxide solution was added to the reaction mixture, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=1:1) to give the free base of the title compound as a yellow oil (59 mg). To a solution of the obtained free base (58 mg) in ethyl acetate (2 mL) was added a solution of fumaric acid (20 mg) in ethanol (2 mL), and the mixture was stirred at room temperature for 15 min. The reaction mixture was concentrated under reduced pressure, and the residue was recrystallized from ethanol-water to give the title compound as a colorless solid (43 mg, yield 29%).
¹H-NMR(DMSO-d₆)δ: 1.51(3H,s),2.41(3H,s),2.56(3H,s),3.93(2H,s),6.51(2H,s),7.06-7.08(1H,m),7.26-7.32(3H,m),7.39-7.51(2H,m),7.65-7.69(1H,m),8.28-8.29(1H,m),3H: not detected.

### Example 78

### 1-[1-(2,6-difluorophenyl)-5-(phenylsulfonyl)-1H-pyrazol-3-yl]-N-methylmethanamine fumarate

To a suspension of lithium aluminum hydride (118 mg) in tetrahydrofuran (3 mL) was added aluminum chloride (137 mg) under ice-cooling under an argon atmosphere, and the mixture was stirred at room temperature for 30 min. A solution of 1-(2,6-difluorophenyl)-N-methyl-5-(phenylsulfonyl)-1H-pyrazole-3-carboxamide (195 mg) in tetrahydrofuran (1 mL) was added to the reaction mixture, and the mixture was stirred for 2 hr under ice-cooling. Water (0.255 mL), 15% aqueous sodium hydroxide solution (0.255 mL) and water (0.765 mL) were successively added to the reaction mixture under ice-cooling. Then celite and anhydrous magnesium sulfate were added, and the mixture was stirred at room temperature for 30 min. The insoluble material was filtered, and washed with ethyl acetate, and the filtrate was concentrated under reduced pressure. The residue was purified by basic silica gel column chromatography (eluent: hexane-ethyl acetate=7:3→ethyl acetate) to give the free base of the title compound as a colorless oil (167 mg). The obtained free base (163 mg) was dissolved in ethyl acetate (2 mL), and a solution of fumaric acid (52.1 mg) in ethanol (2 mL) was added. The reaction mixture was concentrated under reduced pressure, and the residue was recrystallized from a mixed solvent of ethanol and ethyl acetate to give the title compound as colorless crystals (153 mg, yield 63%).
¹H-NMR(DMSO-d₆)δ: 2.38(3H,s),3.89(2H,s),6.54(2H,s),7.25-7.34(2H,m),7.37(1H,s),7.48-7.64(4H,m),7.68-7.81(2H,m),3H: not detected.

### Example 79

### 1-[1-(2-fluoropyridin-3-yl)-5-(phenylsulfonyl)-1H-pyrazol-3-yl]-N-methylmethanamine hydrochloride

To a solution of 1-(2-fluoropyridin-3-yl)-5-(phenylsulfonyl)-1H-pyrazole-3-carbaldehyde (800 mg) in methanol (10 mL) were added methylammonium chloride (172 mg), anhydrous magnesium sulfate (417 mg) and triethylamine (257 mg), and the mixture was stirred at room temperature for 1 hr. Sodium borohydride (105 mg) was added under ice-cooling, and the mixture was further stirred at room temperature for 1 hr. The solvent was evaporated under reduced pressure, water was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=1:1→1:4) to give the free base of the title compound as a colorless oil (658 mg). The obtained free base was dissolved in ethyl acetate (5 mL), and 4 mol/L hydrochloric acid-ethyl acetate solution was added. The solvent was evaporated under reduced pressure, and the residue was recrystallized from ethanol to give the title compound as colorless crystals (531 mg, yield 56%).
¹H-NMR(DMSO-d₆)δ: 2.59(3H,s),4.25(2H,s),7.49-7.60(6H,m),7.76-7.81(1H,m),8.07-8.12(1H,m),8.48-8.50(1H,m),9.26(2H,br).

### Example 80

### 1-{5-[(6-chloropyridin-3-yl)sulfonyl]-1-(2-fluorophenyl)-1H-pyrazol-3-yl}-N-methylmethanamine hydrochloride

tert-Butyl ({5-[(6-chloropyridin-3-yl)sulfonyl]-1-(2-fluorophenyl)-1H-pyrazol-3-yl}methyl)methylcarbamate (56.5 mg) was dissolved in ethyl acetate (1 mL), and 4 mol/L hydrogen chloride-ethyl acetate solution (1 mL) was added. The mixture was stirred at room temperature for 2 hr, and concentrated under reduced pressure, and the residue was recrystallized from a mixed solvent of isopropyl alcohol and ethyl acetate to give the title compound as colorless crystals (39.9 mg, yield 81%).
¹H-NMR(DMSO-d₆)δ: 2.59(3H,s),4.26(2H,s),7.34-7.52(3H,m),7.61(1H,s),7.67-7.76(1H,m),7.77-7.82(1H,m),8.00(1H,dd,J=8.5,2.6Hz),8.47(1H,d,J=2.3Hz),9.35(2H, brs).

### Example 81

### 1-[1-(2-fluorophenyl)-5-(pyridin-3-ylsulfonyl)-1H-pyrazol-3-yl]-N-methylmethanamine hydrochloride

tert-Butyl {[1-(2-fluorophenyl)-5-(pyridin-3-ylsulfonyl)-1H-pyrazol-3-yl]methyl}methylcarbamate (205 mg) was dissolved in a mixed solvent of ethyl acetate (1 mL) and ethanol (1 mL), and 4 mol/L hydrogen chloride-ethyl acetate solution (2 mL) was added. The mixture was stirred at room temperature for 2 hr, and concentrated under reduced pressure, and the residue was recrystallized from a mixed solvent of ethanol and ethyl acetate to give the title compound as colorless crystals (142 mg, yield 80%). melting point 203-206°C
¹H-NMR(DMSO-d₆)δ: 2.60(3H,s),4.27(2H,s),7.32-7.53(3H,m),7.57(1H,s),7.61-7.75(2H,m),7.93-7.99(1H,m),8.59-8.62(1H,m),8.92(1H,dd,J=4.8,1.6Hz),9.17(2H,brs).

### Example 82

### 1-{1-(2-fluorophenyl)-5-[(6-methylpyridin-3-yl)sulfonyl]-1H-pyrazol-3-yl}-N-methylmethanamine fumarate

tert-Butyl ({1-(2-fluorophenyl)-5-[(6-methylpyridin-3-yl)sulfonyl]-1H-pyrazol-3-yl}methyl)methylcarbamate (75 mg) was dissolved in a mixed solvent of ethyl acetate (1 mL) and ethanol (1 mL), and 4 mol/L hydrogen chloride-ethyl acetate solution (2 mL) was added. The mixture was stirred at room temperature for 2 hr, and concentrated under reduced pressure. Saturated aqueous sodium hydrogen carbonate solution was added to the obtained residue, and the mixture was extracted with ethyl acetate. The separated aqueous layer was extracted again with ethyl acetate. The combined organic layers were washed with saturated brine, dried over anhydrous sodium sulfate, and concentrated under reduced pressure. The obtained residue was purified by basic silica gel column chromatography (eluent: hexane-ethyl acetate=1:1→ethyl acetate) to give the free base of the title compound as a colorless oil (50 mg). The obtained free base (50 mg) was dissolved in ethyl acetate (1 mL), and a solution of fumaric acid (16.1 mg) in ethanol (1 mL) was added. The reaction mixture was concentrated under reduced pressure, and the residue was recrystallized from a mixed solvent of ethanol and ethyl acetate to give the title compound as colorless crystals (52 mg, yield 79%).
¹H-NMR(DMSO-d₆)δ: 2.39(3H,s),2.57(3H,s),3.88(2H,s),6.54(2H,s),7.30-7.42(4H,m),7.47(1H,d,J=8.3Hz),7.61-7.72(1H,m),7.82(1H,dd,J=8.3,2.7Hz),8.45(1H,d,J=2.3Hz),3H: not detected.

### Example 83

### 1-{1-(2-fluorophenyl)-5-[(6-methoxypyridin-3-yl)sulfonyl]-1H-pyrazol-3-yl}-N-methylmethanamine hydrochloride

tert-Butyl ({1-(2-fluorophenyl)-5-[(6-methoxypyridin-3-yl)sulfonyl]-1H-pyrazol-3-yl}methyl)methylcarbamate (65 mg) was dissolved in ethyl acetate (1 mL), and 4 mol/L hydrogen chloride-ethyl acetate solution (1 mL) was added. The mixture was stirred at room temperature for 2 hr, and concentrated under reduced pressure, and the residue was recrystallized from a mixed solvent of isopropyl alcohol and ethyl acetate to give the title compound as colorless crystals (44 mg, yield 78%).
¹H-NMR(DMSO-d₆)δ: 2.59(3H,s),3.95(3H,s),4.25(2H,s),6.99(1H,d,J=8.9Hz),7.32-7.53(4H,m),7.65-7.75(1H,m),7.77(1H,dd,J=8.9,2.7Hz),8.18(1H,d,J=2.4Hz),9.26(2H, brs).

### Example 84

### 1-{5-[(6-ethoxypyridin-3-yl)sulfonyl]-1-(2-fluorophenyl)-1H-pyrazol-3-yl}-N-methylmethanamine hydrochloride

tert-Butyl ({5-[(6-ethoxypyridin-3-yl)sulfonyl]-1-(2-fluorophenyl)-1H-pyrazol-3-yl}methyl)methylcarbamate (108 mg) was dissolved in a mixed solvent of ethyl acetate (1 mL) and ethanol (1 mL), and 4 mol/L hydrogen chloride-ethyl acetate solution (2 mL) was added. The mixture was stirred at room temperature for 2 hr, and concentrated under reduced pressure, and the residue was recrystallized from a mixed solvent of ethanol and ethyl acetate to give the title compound as colorless crystals (75 mg, yield 80%).
¹H-NMR(DMSO-d₆)δ: 1.34(3H,t,J=7.1Hz),2.60(3H,s),4.25(2Hs,),4.39(2H,q,J=7.1Hz),6. 92-6.98(1H,m),7.33-7.45(3H,m),7.46(1H,s),7.65-7.73(1H,m),7.75(1H,dd,J=8.9,2.6Hz),8.15(1H,d,J=2.3Hz),9.15(2H, brs).

### Example 85

### 5-({1-(2-fluorophenyl)-3-[(methylamino)methyl]-1H-pyrazol-5-yl}sulfonyl)pyridine-2-carbonitrile hydrochloride

tert-Butyl ({5-[(6-cyanopyridin-3-yl)sulfonyl]-1-(2-fluorophenyl)-1H-pyrazol-3-yl}methyl)methylcarbamate (72 mg) was dissolved in a mixed solvent of ethyl acetate (1 mL) and ethanol (1 mL), and 4 mol/L hydrogen chloride-ethyl acetate solution (2 mL) was added. The mixture was stirred at room temperature for 2 hr, and concentrated under reduced pressure, and the residue was recrystallized from a mixed solvent of isopropyl alcohol and ethyl acetate to give the title compound as colorless crystals (47 mg, yield 76%).
¹H-NMR(DMSO-d₆)δ: 2.58(3H,s),4.26(2H,s),7.32-7.49(3H,m),7.64-7.78(2H,m),8.13-8.36(2H,m),8.79(1H,d,J=1.3Hz),9.48(2H,brs).

### Example 86

### 3-({1-(2-chlorophenyl)-3-[(methylamino)methyl]-1H-pyrazol-5-yl}sulfonyl)benzonitrile hydrochloride

To a solution of tert-butyl ({1-(2-chlorophenyl)-5-[(3-cyanophenyl)sulfonyl]-1H-pyrazol-3-yl}methyl)methylcarbamate (226 mg) in ethanol (4 mL) was added 4 mol/L hydrogen chloride-ethyl acetate solution (4 mL) at 0°C, and the mixture was stirred at room temperature for 4 hr. The reaction mixture was concentrated under reduced pressure, and the residue was recrystallized from ethanol to give the title compound as colorless crystals (87 mg, yield 45%).
¹H-NMR(DMSO-d₆)δ: 2.56(3H,s),4.26(2H,s),7.47-7.85(8H,m),8.22-8.25(1H,m),9.46(2H,brs).

### Example 87

### 1-[1-(2-chlorophenyl)-5-(pyridin-3-ylsulfonyl)-1H-pyrazol-3-yl]-N-methylmethanamine fumarate

tert-Butyl {[1-(2-chlorophenyl)-5-(pyridin-3-ylsulfonyl)-1H-pyrazol-3-yl]methyl}methylcarbamate (123 mg) was dissolved in a mixed solvent of ethyl acetate (2 mL) and ethanol (1 mL), and 4 mol/L hydrogen chloride-ethyl acetate solution (3 mL) was added. The mixture was stirred at room temperature for 3 hr, and concentrated under reduced pressure. Saturated aqueous sodium hydrogen carbonate solution was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=1:1→1:4) to give the free base of the title compound as a colorless oil (69.6 mg). The obtained free base was dissolved in ethyl acetate (5 mL), and the solution was added to a solution of fumaric acid (22.3 mg) in ethanol (5 mL). The solvent was evaporated under reduced pressure, and the residue was recrystallized from ethanol to give the title compound as colorless crystals (49.3 mg, yield 28%). melting point 198-201°C.
¹H-NMR(DMSO-d₆)δ: 2.37(3H,s),3.87(2H,s),6.53(2H,s),7.38(1H,s),7.46-7.64(5H,m),7.87-7.97(1H,m),8.51-8.52(1H,m),8.86-8.88(1H,m),3H: not detected.

### Example 88

### 1-{1-(2-chlorophenyl)-5-[(5-fluoropyridin-3-yl)sulfonyl]-1H-pyrazol-3-yl}-N-methylmethanamine fumarate

tert-Butyl ({1-(2-chlorophenyl)-5-[(5-fluoropyridin-3-yl)sulfonyl]-1H-pyrazol-3-yl}methyl)methylcarbamate (117 mg) was dissolved in a mixed solvent of ethyl acetate (2 mL) and ethanol (1 mL), and 4 mol/L hydrogen chloride-ethyl acetate solution (3 mL) was added. The mixture was stirred at room temperature for 3 hr, and concentrated under reduced pressure. Saturated aqueous sodium hydrogen carbonate solution was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=2:1→1:3) to give the free base of the title compound as a colorless oil (74.5 mg). The obtained free base was dissolved in ethyl acetate (5 mL), and the solution was added to a solution of fumaric acid (22.7 mg) in ethanol (5 mL). The solvent was evaporated under reduced pressure, and the residue was recrystallized from a mixed solvent of ethanol and water to give the title compound as colorless crystals (60.5 mg, yield 50%).
¹H-NMR(DMSO-d₆)δ: 2.38(3H,s),3.89(2H,s),6.53(2H,s),7.44(1H,s),7.52-7.57(3H,m),7.63-7.68(1H,m),7.82-7.86(1H,m),8.43(1H,s),8.96-8.97(1H,m),3H: not detected.

### Example 89

### 1-{1-(2-chlorophenyl)-5-[(6-methylpyridin-3-yl)sulfonyl]-1H-pyrazol-3-yl}-N-methylmethanamine fumarate

tert-Butyl ({1-(2-chlorophenyl)-5-[(6-methylpyridin-3-yl)sulfonyl]-1H-pyrazol-3-yl}methyl)methylcarbamate (76.6 mg) was dissolved in a mixed solvent of ethyl acetate (2 mL) and ethanol (2 mL), and 4 mol/L hydrogen chloride-ethyl acetate solution (3 mL) was added. The mixture was stirred at room temperature for 3 hr, and concentrated under reduced pressure. Saturated aqueous sodium hydrogen carbonate solution was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=4:1→1:3) to give the free base of the title compound as a colorless oil (54.9 mg). A solution of the obtained free base in ethyl acetate (5 mL) was added to a solution of fumaric acid (17.1 mg) in ethanol (5 mL). The solvent was evaporated under reduced pressure, and the residue was recrystallized from a mixed solvent of ethanol and water to give the title compound as colorless crystals (49.5 mg, yield 62%). melting point 196-199°C.
¹H-NMR(DMSO-d₆)δ: 2.37(3H,s),2.54(3H,s),3.89(2H,s),6.52(1H,s),7.34(1H,s),7.42-7.56(4H,m),7.63-7.65(1H,m),7.73-7.77(1H,m),8.38(1H,d,J=2.7Hz),3H: not detected.

### Example 90

### 1-{1-(2-chlorophenyl)-5-[(6-methoxypyridin-3-yl)sulfonyl]-1H-pyrazol-3-yl}-N-methylmethanamine fumarate

tert-Butyl ({1-(2-chlorophenyl)-5-[(6-methoxypyridin-3-yl)sulfonyl]-1H-pyrazol-3-yl}methyl)methylcarbamate (204 mg) was dissolved in a mixed solvent of ethyl acetate (3 mL) and 2-propanol (1 mL), and 4 mol/L hydrogen chloride-ethyl acetate solution (2 mL) was added. The mixture was stirred at room temperature for 4 hr, and concentrated under reduced pressure. Saturated aqueous sodium hydrogen carbonate solution was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=1:1→1:4) to give the free base of the title compound as a colorless oil (118 mg). The obtained free base was dissolved in ethyl acetate (5 mL), and the solution was added to a solution of fumaric acid (34.8 mg) in ethanol (5 mL). The solvent was evaporated under reduced pressure, and the residue was recrystallized from ethanol to give the title compound as colorless crystals (115 mg, yield 55%).
¹H-NMR(DMSO-d₆)δ: 2.39(3H,s),3.91(2H,s),3.93(3H,s),6.52(2H,s),6.94(1H,d,J=9.0Hz) ,7.31(1H,s),7.46-7.73(5H,m),8.08-8.09(1H,m),3H: not detected.

### Example 91

### 1-[1-(2-chlorophenyl)-5-(pyridin-4-ylsulfonyl)-1H-pyrazol-3-yl]-N-methylmethanamine fumarate

tert-Butyl {[1-(2-chlorophenyl)-5-(pyridin-4-ylsulfonyl)-1H-pyrazol-3-yl]methyl}methylcarbamate (152 mg) was dissolved in a mixed solvent of ethyl acetate (3 mL) and ethanol (1 mL), and 4 mol/L hydrogen chloride-ethyl acetate solution (3 mL) was added. The mixture was stirred at room temperature for 2 hr, and concentrated under reduced pressure. Saturated aqueous sodium hydrogen carbonate solution was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=1:1→1:4) to give the free base of the title compound as a colorless oil (93 mg). The obtained free base was dissolved in ethyl acetate (5 mL), and the solution was added to a solution of fumaric acid (29.7 mg) in ethanol (10 mL). The solvent was evaporated under reduced pressure, and the residue was recrystallized from ethanol to give the title compound as colorless crystals (96 mg, yield 62%.
¹H-NMR(DMSO-d₆)δ: 2.37(3H,s),3.88(2H,s),6.54(2H,s),7.42-7.66(7H,m),8.81-8.83(2H,m),3H: not detected.

### Example 92

### 1-{1-(2-chlorophenyl)-5-[(2-methylpyridin-4-yl)sulfonyl]-1H-pyrazol-3-yl}-N-methylmethanamine fumarate

tert-Butyl ({1-(2-chlorophenyl)-5-[(2-methylpyridin-4-yl)sulfonyl]-1H-pyrazol-3-yl}methyl)methylcarbamate (60 mg) was dissolved in a mixed solvent of ethyl acetate (2 mL) and ethanol (1 mL), and 4 mol/L hydrogen chloride-ethyl acetate solution (2 mL) was added. The mixture was stirred at room temperature for 2 hr, and concentrated under reduced pressure. Saturated aqueous sodium hydrogen carbonate solution was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=2:1→1:2) to give the free base of the title compound as a colorless oil (35 mg). The obtained free base was dissolved in ethyl acetate (5 mL), and the solution was added to a solution of fumaric acid (10.8 mg) in ethanol (10 mL). The solvent was evaporated under reduced pressure, and the residue was recrystallized from ethanol to give the title compound as colorless crystals (30 mg, yield 49%).
¹H-NMR(DMSO-d₆)δ: 2.37(3H,s),2.50(3H,s),3.87(2H,s),6.53(2H,s),7.14(1H,brs),7.26-7.27(1H,m),7.38(1H,brs),7.43-7.67(4H,m),8.66(1H,d,J=4.8Hz),3H: not detected.

### Example 93

### 1-{1-(2-chlorophenyl)-5-[(2-methoxypyridin-4-yl)sulfonyl]-1H-pyrazol-3-yl}-N-methylmethanamine fumarate

tert-Butyl ({1-(2-chlorophenyl)-5-[(2-methoxypyridin-4-yl)sulfonyl]-1H-pyrazol-3-yl}methyl)methylcarbamate (195 mg) was dissolved in a mixed solvent of ethyl acetate (3 mL) and ethanol (2 mL), and 4 mol/L hydrogen chloride-ethyl acetate solution (3 mL) was added. The reaction mixture was stirred at room temperature for 6 hr, and concentrated under reduced pressure. Saturated aqueous sodium hydrogen carbonate solution was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=6:1→1:3) to give the free base of the title compound as a colorless oil (131 mg). The obtained free base was dissolved in ethyl acetate (5 mL), and the solution was added to a solution of fumaric acid (38.7 mg) in ethanol (10 mL). The solvent was evaporated under reduced pressure, and the residue was recrystallized from a mixed solvent of ethanol and water to give the title compound as colorless crystals (126 mg, yield 62%).
¹H-NMR(DMSO-d₆)δ: 2.39(3H,s),3.90(3H,s),3.93(2H,s),6.53(2H,s),6.65-6.66(1H,m),7.05-7.07(1H,m),7.43-7.64(5H,m),8.39(1H,d,J=5.1Hz),3H: not detected.

### Example 94

### 1-[1-(2-chlorophenyl)-5-(pyridin-2-ylsulfonyl)-1H-pyrazol-3-yl]-N-methylmethanamine hydrochloride

To a solution of tert-butyl {[1-(2-chlorophenyl)-5-(pyridin-2-ylsulfonyl)-1H-pyrazol-3-yl]methyl}methylcarbamate (103 mg) in ethanol (1 mL) was added 4 mol/L hydrogen chloride-ethyl acetate solution (2 mL), and the mixture was stirred at room temperature for 3 hr. The reaction mixture was concentrated under reduced pressure, and the residue was recrystallized from ethanol-water to give the title compound as colorless crystals (37 mg, yield 43%).
¹H-NMR(DMSO-d₆)δ: 2.58(3H,s),4.28(2H,s),7.32-7.34(1H,m),7.40-7.45(1H,m),7.52-7.62(4H,m),7.73-7.77(1H,m),7.99-8.05(1H,m),8.74(1H,d,J=4.8Hz),9.41(2H,brs).

### Example 95

### 1-{1-(2-chlorophenyl)-5-[(6-methylpyridin-2-yl)sulfonyl]-1H-pyrazol-3-yl}-N-methylmethanamine hydrochloride

To a solution of tert-butyl ({1-(2-chlorophenyl)-5-[(6-methylpyridin-2-yl)sulfonyl]-1H-pyrazol-3-yl}methyl)methylcarbamate (368 mg) in ethanol (4 mL) was added 4 mol/L hydrogen chloride-ethyl acetate solution (4 mL), and the mixture was stirred at room temperature for 4 hr. The reaction mixture was concentrated under reduced pressure, and the residue was recrystallized from ethanol-water to give the title compound as colorless crystals (180 mg, yield 56%).
¹H-NMR(DMSO-d₆)δ: 2.50(3H,s),2.57(3H,s),4.27(2H,s),7.38-7.60(7H,m),7.87(1H,t,J=7.8Hz),9.45(2H,brs).

### Example 96

### 1-{1-(2-chlorophenyl)-5-[(5-methylpyridin-2-yl)sulfonyl]-1H-pyrazol-3-yl}-N-methylmethanamine hydrochloride

To a solution of tert-butyl ({1-(2-chlorophenyl)-5-[(5-methylpyridin-2-yl)sulfonyl]-1H-pyrazol-3-yl}methyl)methylcarbamate (420 mg) in a mixed solvent of ethanol (3 mL) and ethyl acetate (3 mL) was added 4 mol/L hydrogen chloride-ethyl acetate solution (4 mL) at 0°C, and the mixture was stirred at room temperature for 4 hr. The reaction mixture was concentrated under reduced pressure, and the residue was recrystallized from ethanol to give the title compound as colorless crystals (236 mg, yield 63%).
¹H-NMR(DMSO-d₆)δ: 2,40(3H,s),2.58(3H,s),4.27(2H,s),7.29-7.31(1H,m),7.39-7.58(5H,m),7.79-7.83(1H,m),8.57(1H,s),9.15(2H,brs).

### Example 97

### 1-{1-(2-chlorophenyl)-5-[(6-methoxypyridin-2-yl)sulfonyl]-1H-pyrazol-3-yl}-N-methylmethanamine hydrochloride

To a solution of tert-butyl ({1-(2-chlorophenyl)-5-[(6-methoxypyridin-2-yl)sulfonyl]-1H-pyrazol-3-yl}methyl)methylcarbamate (396 mg) in a mixed solvent of ethanol (3 mL) and ethyl acetate (3 mL) was added 4 mol/L hydrogen chloride-ethyl acetate solution (4 mL) at 0°C, and the mixture was stirred at room temperature for 2 hr. The reaction mixture was concentrated under reduced pressure, and the residue was recrystallized from ethanol to give the title compound as colorless crystals (234 mg, yield 86%).
¹H-NMR(DMSO-d₆)δ: 2.57(3H,s),3.80(3H,s),4.28(2H,s),7.16(1H,dd,J=8.4,0.6Hz),7.22(' 1H,dd,J=7.2,0.6Hz),7.36-7.48(2H,m),7.51-7.61(3H,m),7.86(1H,dd,J=8.4,7.2Hz),9.37(2H,brs).

### Example 98

### 1-[1-(2,3-difluorophenyl)-5-(pyridin-3-ylsulfonyl)-1H-pyrazol-3-yl]-N-methylmethanamine fumarate

To a solution of tert-butyl {[1-(2,3-difluorophenyl)-5-(phenylsulfonyl)-1H-pyrazol-3-yl]methyl}methylcarbamate (211 mg) in a mixed solvent of ethyl acetate (3 mL) and ethanol (3 mL) was added 4 mol/L hydrogen chloride-ethyl acetate solution (4 mL), and the mixture was stirred at room temperature for 3 hr, and concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by basic silica gel column chromatography (eluent: hexane-ethyl acetate=1:4) to give the free base of the title compound as a yellow oil (104 mg). To a solution of the obtained free base (98 mg) in ethyl acetate (2 mL) was added a solution of fumaric acid (34 mg) in ethanol (2 mL), and the mixture was stirred at room temperature for 1 hr. The reaction mixture was concentrated under reduced pressure, and the residue was recrystallized from ethanol-water to give the title compound as a colorless solid (81 mg, yield 38%).
¹H-NMR(DMSO-d₆)δ: 2.38(3H,s),3.87(2H,s),6.54(2H,s),7.24-7.49(3H,m),7.61-7.66(1H,m),7.71-7.79(1H,m),7.98-8.02(1H,m),8.63-8.64(1H,m),8.89-8.91(1H,m),3H: not detected.

### Example 99

### 1-{1-(2,3-difluorophenyl)-5-[(6-methylpyridin-3-yl)sulfonyl]-1H-pyrazol-3-yl}-N-methylmethanamine fumarate

tert-Butyl ({5-[(6-methylpyridin-3-yl)sulfonyl]-1-(2,3-difluorophenyl)-1H-pyrazol-3-yl}methyl)methylcarbamate (71 mg) was dissolved in a mixed solvent of ethyl acetate (3 mL) and ethanol (1 mL), and 4 mol/L hydrogen chloride-ethyl acetate solution (3 mL) was added. The mixture was stirred at room temperature for 2 hr, and concentrated under reduced pressure. Saturated aqueous sodium hydrogen carbonate solution was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=1:1-+1:4) to give the free base of the title compound as a colorless oil (42 mg). The obtained free base was dissolved in ethyl acetate (5 mL), and the solution was added to a solution of fumaric acid (13 mg) in ethanol (10 mL). The solvent was evaporated under reduced pressure, and the residue was recrystallized from a mixed solvent of ethanol and water to give the title compound as colorless crystals (38 mg, yield 52%). melting point 199-202°C.
¹H-NMR(DMSO-d₆)δ: 2.37(3H,s),2.57(3H,s),3.86(2H,s),6.54(2H,s),7.24-7.29(1H,m),7.32-7.41(2H,m),7.48(1H,d,J=8.4Hz),7.70-7.79(1H,m),7.85-7.88(1H,m),8.48-8.49(1H,m),3H: not detected.

### Example 100

### 1-{1-(2,4-difluorophenyl)-5-[(6-methylpyridin-3-yl)sulfonyl]-1H-pyrazol-3-yl}-N-methylmethanamine fumarate

tert-Butyl ({5-[(6-methylpyridin-3-yl)sulfonyl]-1-(2,4-difluorophenyl)-1H-pyrazol-3-yl}methyl)methylcarbamate (129 mg) was dissolved in a mixed solvent of ethyl acetate (3 mL) and ethanol (1 mL), and 4 mol/L hydrogen chloride-ethyl acetate solution (3 mL) was added. The mixture was stirred at room temperature for 4 hr, and concentrated under reduced pressure. Saturated aqueous sodium hydrogen carbonate solution was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=2:1→1:4) to give the free base of the title compound as a colorless oil (83 mg). The obtained free base was dissolved in ethyl acetate (5 mL), and the solution was added to a solution of fumaric acid (25 mg) in ethanol (5 mL). The solvent was evaporated under reduced pressure, and the residue was recrystallized from ethanol to give the title compound as colorless crystals (80 mg, yield 60%).
¹H-NMR(DMSO-d₆)δ: 2.38(3H,s),2.58(3H,s),3.87(2H,s),6.53(2H,s),7.22-7.29(1H,m),7.35(1H,s),7.45-7.53(3H,m),7.83-7.87(1H,m),8.50-8.51(1H,m),3H: not detected.

### Example 101

### 1-[1-(2,5-difluorophenyl)-5-(phenylsulfonyl)-1H-pyrazol-3-yl]-N-methylmethanamine hydrochloride

To a solution of tert-butyl ({5-(phenylsulfonyl)-1-(2,5-difluorophenyl)-1H-pyrazol-3-yl}methyl)methylcarbamate (571 mg) in a mixed solvent of ethanol (4 mL) and ethyl acetate (4 mL) was added 4 mol/L hydrogen chloride-ethyl acetate solution (4 mL) at 0°C, and the mixture was stirred at room temperature for 3 hr. The reaction mixture was concentrated under reduced pressure, and the residue was recrystallized from ethanol to give the title compound as colorless crystals (340 mg, yield 69%).
¹H-NMR(DMSO-d₆)δ: 2.57(3H,s),4.24(2H,s),7.34-7.62(8H,m),7.75-7.79(1H,m),9.42(2H,brs).

### Example 102

### 3-({1-(2,5-difluorophenyl)-3-[(methylamino)methyl]-1H-pyrazol-5-yl}sulfonyl)benzonitrile succinate

To a solution of tert-butyl ({5-[(3-cyanophenyl)sulfonyl]-1-(2,5-difluorophenyl)-1H-pyrazol-3-yl}methyl)methylcarbamate (455 mg) in a mixed solvent of ethyl acetate (2 mL) and ethanol (2 mL) was added 4 mol/L hydrogen chloride-ethyl acetate solution (6 mL), and the mixture was stirred at room temperature for 2 hr, and concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure to give the free base of the title compound as a yellow oil (316 mg). To a solution of the obtained free base (315 mg) in ethyl acetate (4 mL) was added a solution of succinic acid (98 mg) in ethanol (4 mL), and the mixture was stirred at room temperature for 15 min. The reaction mixture was concentrated under reduced pressure, and the residue was recrystallized from ethanol to give the title compound as a colorless solid (232 mg, yield 49%).
¹H-NMR(DMSO-d₆)δ: 2.36-2.37(7H,m),3.81(2H,s),7.39-7.49(3H,m),7.53-7.61(1H,m),7.77-7.82(1H,m),7.90-7.93(1H,m),8.00-8.01(1H,m),8.22-8,26(1H,m),3H: not detected.

### Example 103

### 1-[1-(2,5-difluorophenyl)-5-(pyridin-3-ylsulfonyl)-1H-pyrazol-3-yl]-N-methylmethanamine fumarate

To a solution of tert-butyl {[1-(2,5-difluorophenyl)-5-(pyridin-3-ylsulfonyl)-1H-pyrazol-3-yl]methyl}methylcarbamate (212 mg) in a mixed solvent of ethyl acetate (3 mL) and ethanol (3 mL) was added 4 mol/L hydrogen chloride-ethyl acetate solution (4 mL), and the mixture was stirred at room temperature for 5 hr, and concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by basic silica gel column chromatography (eluent: hexane-ethyl acetate=1:2) to give the free base of the title compound as a yellow oil (105 mg). To a solution of the obtained free base (99 mg) in ethyl acetate (2 mL) was added a solution of fumaric acid (33 mg) in ethanol (2 mL), and the mixture was stirred at room temperature for 1 hr. The reaction mixture was concentrated under reduced pressure, and the residue was recrystallized from ethanol-water to give the title compound as a colorless solid (87 mg, yield 39%).
¹H-NMR(DMSO-d₆)δ: 2.40(3H,s),3.91(2H,s),6.53(2H,s),7.42-7.49(3H,m),7.53-7.66(2H,m),8.00-8.04(1H,m),8.68-8.69(1H,m),8.90-8.92(1H,m),3H: not detected.

### Example 104

### 1-{1-(2,5-difluorophenyl)-5-[(6-methylpyridin-3-yl)sulfonyl]-1H-pyrazol-3-yl}-N-methylmethanamine fumarate

tert-Butyl ({5-[(6-methylpyridin-3-yl)sulfonyl]-1-(2,5-difluorophenyl)-1H-pyrazol-3-yl}methyl)methylcarbamat (154 mg) was dissolved in a mixed solvent of ethyl acetate (3 mL) and ethanol (1 mL), and 4 mol/L hydrogen chloride-ethyl acetate solution (3 mL) was added. The mixture was stirred at room temperature for 2 hr, and concentrated under reduced pressure. Saturated aqueous sodium hydrogen carbonate solution was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=1:1→1:4) to give the free base of the title compound as a colorless oil (108 mg). The obtained free base was dissolved in ethyl acetate (5 mL), and the solution was added to a solution of fumaric acid (33 mg) in ethanol (5 mL). The solvent was evaporated under reduced pressure, and the residue was recrystallized from a mixed solvent of ethanol and water to give the title compound as colorless crystals (99 mg, yield 62%).
¹H-NMR(DMSO-d₆)δ: 2.36(3H,s),2.58(3H,s),3.83(2H,s),6.53(2H,s),7.34(1H,s),7.43-7.57(4H,m),7.87-7.90(1H,m),8.53-8.54(1H,m),3H: not detected.

### Example 105

### 1-{1-(2-fluoro-3-methylphenyl)-5-[(6-methylpyridin-3-yl)sulfonyl]-1H-pyrazol-3-yl}-N-methylmethanamine fumarate

tert-Butyl ({5-[(6-methylpyridin-3-yl)sulfonyl]-1-(2-fluoro-3-methylphenyl)-1H-pyrazol-3-yl}methyl)methylcarbamate (503 mg) was dissolved in a mixed solvent of ethyl acetate (5 mL) and ethanol (2 mL), and 4 mol/L hydrogen chloride-ethyl acetate solution (5 mL) was added. The mixture was stirred at room temperature for 2 hr, and concentrated under reduced pressure. Saturated aqueous sodium hydrogen carbonate solution was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=3:1→1:3) to give the free base of the title compound as a colorless oil (330 mg). The obtained free base was dissolved in ethyl acetate (5 mL), and the solution was added to a solution of fumaric acid (102 mg) in ethanol (10 mL). The solvent was evaporated under reduced pressure, and the residue was recrystallized from a mixed solvent of ethanol and water to give the title compound as colorless crystals (335 mg, yield 64%) .
¹H-NMR(DMSO=d₆)δ: 2.14(3H,d,J=1.8Hz),2.41(3H,s),2.57(3H,s),3.92(2H,s),6.52(2H,s) ,7.21-7.28(2H,m),7.36(1H,s),7.44-7.55(2H,m),7.75-7.79(1H,m),8.34-8.35(1H,m),3H: not detected.

### Example 106

### 1-[1-(2-fluoro-4-methylphenyl)-5-(pyridin-3-ylsulfonyl)-1H-pyrazol-3-yl]-N-methylmethanamine fumarate

To a solution of tert-butyl {[1-(2-fluoro-4-methylphenyl)-5-(pyridin-3-ylsulfonyl)-1H-pyrazol-3-yl]methyl}methylcarbamate (152 mg) in a mixed solvent of ethyl acetate (1 mL) and ethanol (2 mL) was added 4 mol/L hydrogen chloride-ethyl acetate solution (3 mL), and the mixture was stirred at room temperature for 3 hr, and concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure. The residue was purified by basic silica gel column chromatography (eluent: ethyl acetate) to give the free base of the title compound as a yellow oil (83 mg). To a solution of the obtained free base (82 mg) in ethyl acetate (2 mL) was added a solution of fumaric acid (27 mg) in ethanol (2 mL), and the mixture was stirred at room temperature for 15 min. The reaction mixture was concentrated under reduced pressure, and the residue was recrystallized from ethanol-water to give the title compound as a colorless solid (50 mg, yield 30%).
¹H-NMR(DMSO-d₆)δ: 2.42(6H,s),3.95(2H,s),6.52(2H,s),7.13-7.25(3H,m),7.41(1H,s),7.60-7.64(1H,m),7.92-7.96(1H,m),8.60-8.61(1H,m),8.88-8.90(1H,m),3H: not detected.

### Example 107

### 1-{1-(2-fluoro-4-methylphenyl)-5-[(6-methylpyridin-3-yl)sulfonyl]-1H-pyrazol-3-yl}-N-methylmethamine 0.5fumarate

tert-Butyl ({5-[(6-methylpyridin-3-yl)sulfonyl]-1-(2-fluoro-4-methylphenyl)-1H-pyrazol-3-yl}methyl)methylcarbamate (417 mg) was dissolved in a mixed solvent of ethyl acetate (3 mL) and ethanol (2 mL), and 4 mol/L hydrogen chloride-ethyl acetate solution (5 mL) was added. The mixture was stirred at room temperature for 3 hr, and concentrated under reduced pressure. Saturated aqueous sodium hydrogen carbonate solution was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=2:1→1:2) to give the free base of the title compound as a colorless oil (332 mg). The obtained free base was dissolved in ethyl acetate (5 mL), and the solution was added to a solution of fumaric acid (102 mg) in ethanol (10 mL). The solvent was evaporated under reduced pressure, and the residue was recrystallized from a mixed solvent of ethanol and water to give the title compound as colorless crystals (209 mg, yield 55%) .
¹H-NMR(DMSO-d₆)δ: 2.34(3H,s),2.42(3H,s),2.57(3H,s),3.79(2H,s),6.49(2H,s),7.12-7.24(3H,m),7.29(1H,s),7.46(1H,d,J=8.4Hz),7.79-7.83(1H,m),8.46-8.47(1H,m),2H: not detected.

### Example 108

### 1-{1-(2-fluoro-5-methylphenyl)-5-[(6-methylpyridin-3-yl)sulfonyl]-1H-pyrazol-3-yl}-N-methylmethanamine fumarate

tert-Butyl ({5-[(6-methylpyridin-3-yl)sulfonyl]-1-(2-fluoro-5-methylphenyl)-1H-pyrazol-3-yl}methyl)methylcarbamate (396 mg) was dissolved in a mixed solvent of ethyl acetate (3 mL) and ethanol (2 mL), and 4 mol/L hydrogen chloride-ethyl acetate solution (5 mL) was added. The reaction mixture was stirred at room temperature for 6 hr, and concentrated under reduced pressure. Saturated aqueous sodium hydrogen carbonate solution was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=1:1→1:4) to give the free base of the title compound as a colorless oil (268 mg). The obtained free base was dissolved in ethyl acetate (5 mL), and the solution was added to a solution of fumaric acid (83 mg) in ethanol (10 mL). The solvent was evaporated under reduced pressure, and the residue was recrystallized from a mixed solvent of ethanol and water to give the title compound as colorless crystals (247 mg, yield 61%).
¹H-NMR(DMSO-d₆)δ: 2.26(3H,s),2.38(3H,s),2.57(3H,s),3.86(2H,s),6.52(2H,s),6.95-6.97(1H,m),7.21-7.27(1H,m),7.33(1H,s),7.40-7.48(2H,m),7.78-7.82(1H,m),8.42-8.43(1H,m),3H: not detected.

### Example 109

### 1-[1-(3-fluoro-2-methylphenyl)-5-(pyridin-3-ylsulfonyl)-1H-pyrazol-3-yl]-N-methylmethanamine fumarate

To a solution of tert-butyl {[1-(3-fluoro-2-methylphenyl)-5-(pyridin-3-ylsulfonyl)-1H-pyrazol-3-yl]methyl}methylcarbamate (719 mg) in a mixed solvent of ethyl acetate (2 mL) and ethanol (1 mL) was added 4 mol/L hydrogen chloride-ethyl acetate solution (3 mL), and the mixture was stirred at room temperature for 3 hr. The reaction mixture was concentrated under reduced pressure, the residue was diluted with saturated aqueous sodium hydrogen carbonate, and the mixture was extracted with ethyl acetate. The separated aqueous layer was extracted again with ethyl acetate. The combined organic layers were washed with saturated brine, dried over anhydrous magnesium sulfate, and concentrated under reduced pressure to give the free base of the title compound as a pale-yellow oil (542 mg, yield 96%). A solution of the obtained free base in ethyl acetate (5 mL) was added dropwise to a solution of fumaric acid (175 mg) in ethanol (5 mL), and the mixture was concentrated under reduced pressure. The residue was recrystallized from ethanol-water to give the title compound as a white solid (542 mg, yield 81%).
¹H-NMR(DMSO-d₆)δ: 1.37(3H,d,J=1.9Hz),2.41(3H,s),3.93(2H,s),6.53(2H,s),7.02(1H,d, J=7.7Hz),7.30-7.50(3H,m),7.60(1H,ddd,J=8.2,4.8,0.8Hz),7.88(1H,ddd,J=8.2,2.4, 1.6Hz),8.49(1H,dd,J=2.4,0.7Hz),8.89(1H,dd,J=4.9,1.5Hz),3H: not detected.

### Example 110

### 1-{1-(3-fluoro-2-methylphenyl)-5-[(6-methylpyridin-3-yl)sulfonyl]-1H-pyrazol-3-yl}-N-methylmethanamine fumarate

To a solution of tert-butyl ({1-(3-fluoro-2-methylphenyl)-5-[(6-methylpyridin-3-yl)sulfonyl]-1H-pyrazol-3-yl}methyl)methylcarbamate (916 mg) in a mixed solvent of ethyl acetate (4 mL) and 2-propanol (2 mL) was added 4 mol/L hydrogen chloride-ethyl acetate solution (4 mL), and the mixture was stirred at room temperature for 74 hr. The reaction mixture was concentrated under reduced pressure, the residue was diluted with saturated aqueous sodium hydrogen carbonate, and the mixture was extracted with ethyl acetate. The separated aqueous layer was extracted again with ethyl acetate. The combined organic layers were washed with saturated brine, dried over anhydrous magnesium sulfate, concentrated under reduced pressure to give the free base of the title compound as a pale-yellow oil (626 mg). A solution of the obtained free base in ethyl acetate (5 mL) was added dropwise to a solution of fumaric acid (194 mg) in ethanol (5 mL), and the mixture was concentrated under reduced pressure. The residue was recrystallized from ethanol-water to give the title compound as a white solid (737 mg, yield 78%).
¹H-NMR (DMSO-d₆)δ: 1.40(3H,d,J=1.9Hz),2.45(3H,s),2.56(3H,s),4.00(2H,s),6.51(2H,s) ,7.02(1H,d,J=7.7Hz),7.33-7.50(4H,m),7.75(1H,dd,J=8.3,2.4Hz),8.34(1H,d,J=2.3Hz),3H: not detected.

### Example 111

### 1-[1-(5-fluoro-2-methylphenyl)-5-(pyridin-3-ylsulfonyl)-1H-pyrazol-3-yl]-N-methylmethanamine fumarate

tert-Butyl {[1-(5-fluoro-2-methylphenyl)-5-(pyridin-3-ylsulfonyl)-1H-pyrazol-3-yl]methyl}methylcarbamate (194 mg) was dissolved in a mixed solvent of ethyl acetate (3 mL) and ethanol (1 mL), and 4 mol/L hydrogen chloride-ethyl acetate solution (3 mL) was added. The mixture was stirred at room temperature for 2 hr, and concentrated under reduced pressure. Saturated aqueous sodium hydrogen carbonate solution was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=1:1→1:4) to give the free base of the title compound as a colorless oil (138 mg). The obtained free base was dissolved in ethyl acetate (5 mL), and the solution was added to a solution of fumaric acid (44 mg) in ethanol (10 mL). The solvent was evaporated under reduced pressure, and the residue was recrystallized from a mixed solvent of ethanol and water to give the title compound as colorless crystals (117 mg, yield 58%).
¹H-NMR(DMSO-d₆)δ: 1.48(3H,s),2.37(3H,s),3.86(2H,s),6.53(2H,s),7.03-7.06(1H,m),7.29-7.42(2H,m),7.57-7.62(1H,m),7.87-7.91(1H,m),8.51-8.52(1H,m),8.87-8.89(1H,m),3H: not detected.

### Example 112

### 1-{1-(5-fluoro-2-methylphenyl)-5-[(6-methylpyridin-3-yl)sulfonyl]-1H-pyrazol-3-yl}-N-methylmethanamine fumarate

tert-Butyl ({5-[(6-methylpyridin-3-yl)sulfonyl]-1-(5-fluoro-2-methylphenyl)-1H-pyrazol-3-yl}methyl)methylcarbamate (294 mg) was dissolved in a mixed solvent of ethyl acetate (3 mL) and ethanol (1 mL), and 4 mol/L hydrogen chloride-ethyl acetate solution (3 mL) was added. The mixture was stirred at room temperature for 2 hr, and concentrated under reduced pressure. Saturated aqueous sodium hydrogen carbonate solution was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=1:1→1:4) to give the free base of the title compound as a colorless oil (182 mg). The obtained free base was dissolved in ethyl acetate (5 mL), and the solution was added to a solution of fumaric acid (56 mg) in ethanol (10 mL). The solvent was evaporated under reduced pressure, and the residue was recrystallized from a mixed solvent of ethanol and water to give the title compound as colorless crystals (192 mg, yield 63%).
¹H-NMR(DMSO-d₆)δ: 1.51(3H,s),2.39(3H,s),2.56(3H,s),3.89(2H,s),6.52(2H,s),7.00-7.04(1H,m),7.31-7.45(4H,m),7.73-7.77(1H,m),8.36-8.37(1H,m),3H: not detected.

### Example 113

### 1-[1-(2-chloro-3-fluorophenyl)-5-(pyridin-3-ylsulfonyl)-1H-pyrazol-3-yl]-N-methylmethanamine fumarate

tert-Butyl {[1-(2-chloro-3-fluorophenyl)-5-(pyridin-3-ylsulfonyl)-1H-pyrazol-3-yl]methyl}methylcarbama (390 mg) was dissolved in a mixed solvent of ethyl acetate (3 mL) and ethanol (2 mL), and 4 mol/L hydrogen chloride-ethyl acetate solution (3 mL) was added. The mixture was stirred at room temperature for 2 hr, and concentrated under reduced pressure. Saturated aqueous sodium hydrogen carbonate solution was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=2:1→1:4) to give the free base of the title compound as a colorless oil (261 mg). The obtained free base was dissolved in ethyl acetate (5 mL), and the solution was added to a solution of fumaric acid (80 mg) in ethanol (10 mL). The solvent was evaporated under reduced pressure, and the residue was recrystallized from a mixed solvent of ethanol and water to give the title compound as colorless crystals (232 mg, yield 58%).
¹H-NMR(DMSO-d₆)δ: 2,38(3H,s),3.90 (2H,s),6.53 (2H,s),7.38-7.43(2H,m),7.56-7.63(2H,m),7.70-7.76(1H,m),7.92-7.96(1H,m),8.56-8.57(1H,m),8.89-8.91(1H,m),3H: not detected.

### Example 114

### 1-{1-(2-chloro-3-fluorophenyl)-5-[(6-methylpyridin-3-yl)sulfonyl]-1H-pyrazol-3-yl}-N-methylmethanamine fumarate

tert-Butyl ({1-(2-chloro-3-fluorophenyl)-5-[(6-methylpyridin-3-yl)sulfonyl]-1H-pyrazol-3-yl}methyl)methylcarbamate (459 mg) was dissolved in a mixed solvent of ethyl acetate (3 mL) and ethanol (2 mL), and 4 mol/L hydrogen chloride-ethyl acetate solution (3 mL) was added. The mixture was stirred at room temperature for 2 hr, and concentrated under reduced pressure. Saturated aqueous sodium hydrogen carbonate solution was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=2:1→1:4) to give the free base of the title compound as a colorless oil (313 mg). The obtained free base was dissolved in ethyl acetate (5 mL), and the solution was added to a solution of fumaric acid (92 mg) in ethanol (10 mL). The solvent was evaporated under reduced pressure, and the residue was recrystallized from a mixed solvent of ethanol and water to give the title compound as colorless crystals (303 mg, yield 64%).
¹H-NMR(DMSO-d₆)δ: 2.37(3H,s),2.57(3H,s),3.89(2H,s),6.53(2H,s),7.37-7.40(2H,m),7.45-7.47(1H,m),7.55-7.62(1H,m),7.70-7.83(2H,m),8.41-8.42(1H,m),3H: not detected.

### Example 115

### 1-{1-(2-chloro-5-fluorophenyl)-5-[(6-methylpyridin-3-yl)sulfonyl]-1H-pyrazol-3-yl}-N-methylmethanamine fumarate

tert-Butyl ({1-(2-chloro-5-fluorophenyl)-5-[(6-methylpyridin-3-yl)sulfonyl]-1H-pyrazol-3-yl}methyl)methylcarbamate (91 mg) was dissolved in a mixed solvent of ethyl acetate (3 mL) and ethanol (1 mL), and 4 mol/L hydrogen chloride-ethyl acetate solution (3 mL) was added. The mixture was stirred at room temperature for 3 hr, and concentrated under reduced pressure. Saturated aqueous sodium hydrogen carbonate solution was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=2:1→1:3) to give the free base of the title compound as a colorless oil (62 mg). The obtained free base was dissolved in ethyl acetate (5 mL), and the solution was added to a solution of fumaric acid (18 mg) in ethanol (10 mL). The solvent was evaporated under reduced pressure, and the residue was recrystallized from a mixed solvent of ethanol and water to give the title compound as colorless crystals (61 mg, yield 65%).
¹H-NMR(DMSO-d₆)δ: 2.35(3H,s),2.57(3H,s),3.84(2H,s),6.53(2H,s),7.34(1H,s),7.46(1H ,d,J=8.4Hz),7.54-7.63(3H,m),7.82-7.84(1H,m),8.47-8.48(1H,m), 3H: not detected.

### Example 116

### 1-[1-(2-chloro-5-fluorophenyl)-5-(pyridin-3-ylsulfonyl)-1H-pyrazol-3-yl]-N-methylmethanamine fumarate

To a solution of tert-butyl {[1-(2-chloro-5-fluorophenyl)-5-(pyridin-3-ylsulfonyl)-1H-pyrazol-3-yl]methyl}methylcarbamate (182 mg) in a mixed solvent of ethyl acetate (2 mL) and ethanol (1 mL) was added 4 mol/L hydrogen chloride-ethyl acetate solution (3 mL), and the mixture was stirred at room temperature for 1.5 hr, and concentrated under reduced pressure. Saturated aqueous sodium hydrogen carbonate solution was added to the residue, and the mixture was extracted with ethyl acetate. The extract was washed successively with saturated aqueous sodium hydrogen carbonate solution, water and saturated brine, dried over anhydrous sodium sulfate, and concentrated under reduced pressure to give the free base of the title compound as a colorless oil (141 mg). The obtained free base was dissolved in ethyl acetate (2 mL), and the solution was added to a solution of fumaric acid (43 mg) in ethanol (2 mL). The mixture was concentrated under reduced pressure, and the residue was recrystallized from ethanol-water to give the title compound as a colorless solid (127 mg, yield 68%).
¹H-NMR(DMSO-d₆)δ: 2.34(3H,s),3.84(2H,s),6.53(2H,s),7.38(1H,s),7.52-7.65(4H,m),7.93-8.00(1H,m),8.60-8.64(1H,m),8.86-8.91(1H,m),3H: not detected.

### Example 117

### 1-[1-(3-chloro-2-fluorophenyl)-5-(pyridin-3-ylsulfonyl)-1H-pyrazol-3-yl]-N-methylmethanamine L(+)-tartrate

To a solution of tert-butyl {[1-(3-chloro-2-fluorophenyl)-5-(pyridin-3-ylsulfonyl)-1H-pyrazol-3-yl]methyl}methylcarbamate (376 mg) in a mixed solvent of ethyl acetate (3 mL) and ethanol (3 mL) was added 4 mol/L hydrogen chloride-ethyl acetate solution (4 mL), and the mixture was stirred at room temperature for 4 hr, and concentrated under reduced pressure. Saturated aqueous sodium hydrogen carbonate solution was added to the residue, and the mixture was extracted with ethyl acetate. To the extract was added 1 mol/L hydrochloric acid, and the aqueous layer was washed with ethyl acetate. The aqueous layer was made basic with 8 mol/L sodium hydroxide solution and extracted with ethyl acetate. The extract was washed successively with saturated aqueous sodium hydrogen carbonate solution, water and saturated brine, dried over anhydrous sodium sulfate, and concentrated under reduced pressure to give the free base of the title compound as a colorless oil (237 mg). To a solution of the obtained free base (236 mg) in ethyl acetate (3 mL) was added a solution of L(+)-tartaric acid (101 mg) in ethanol (3 mL), and the mixture was stirred at room temperature for 30 min. The reaction mixture was concentrated under reduced pressure, and the residue was recrystallized from ethanol-water to give the title compound as a colorless solid (300 mg, yield 76%).
¹H-NMR(DMSO-d₆) δ: 2.48(3H,s),4.00(2H,s) ,4.05(2H,s), 7.38-7.50(3H,m),7.61-7.66(1H,m),7.86-7.91(1H,m),7.95-7.99(1H,m),8.64(1H,d,J=2.1Hz), 8.90-8.92 (1H,m), 5H: not detected.

### Example 118

### 1-{1-(3-chloro-2-fluorophenyl)-5-[(6-methylpyridin-3-yl)sulfonyl]-1H-pyrazol-3-yl}-N-methylmethanamine L(+)-tartrate

To a solution of tert-butyl ({1-(3-chloro-2-fluorophenyl)-5-[(6-methylpyridin-3-yl)sulfonyl]-1H-pyrazol-3-yl}methyl)methylcarbamate (235 mg) in a mixed solvent of ethyl acetate (2 mL) and ethanol (2 mL) was added 4 mol/L hydrogen chloride-ethyl acetate solution (4 mL), and the mixture was stirred at room temperature for 3 hr, and concentrated under reduced pressure. Saturated aqueous sodium hydrogen carbonate solution was added to the residue, and the mixture was extracted with ethyl acetate. To the extract was added 1 mol/L hydrochloric acid, and the aqueous layer was separated. The organic layer was washed with 1 mol/L hydrochloric acid. The combined aqueous layers were made basic with saturated aqueous sodium hydrogen carbonate solution and extracted with ethyl acetate. The extract was washed with saturated brine, dried over anhydrous sodium sulfate, and concentrated under reduced pressure to give the free base of the title compound as a yellow oil (175 mg). To a solution of the obtained free base (167 mg) in ethyl acetate (2 mL) was added a solution of L(+)-tartaric acid (66 mg) in ethanol (2 mL), and the mixture was stirred at room temperature for 30 min. The reaction mixture was concentrated under reduced pressure, and the residue was recrystallized from ethanol-water to give the title compound as a colorless solid (145 mg, yield 56%).
¹H-NMR (DMSO-d₆) δ: 2.47(3H,s),2.58(3H,s),3.99(2H,s),4.04(2H,s),7.39-7.49(4H,m),7.81-7.90(2H,m),8.45-8.46(1H,m),5H: not detected.

### Example 119

### 1-{5-[(3-fluorophenyl)sulfonyl]-1-(2-fluoropyridin-3-yl)-1H-pyrazol-3-yl}-N-methylmethanamine hydrochloride

To a solution of tert-butyl ({5-[(3-fluorophenyl)sulfonyl]-1-(2-fluoropyridin-3-yl)-1H-pyrazol-3-yl}methyl)methylcarbamate (167 mg) in a mixed solvent of ethyl acetate (2 mL) and 2-propanol (1 mL) was added 4 mol/L hydrogen chloride-ethyl acetate solution (3 mL). The mixture was stirred at room temperature for 1 hr, and concentrated under reduced pressure, and the residue was recrystallized from ethanol-water to give the title compound as colorless crystals (111 mg, yield 77%).
¹H-NMR(DMSO-d₆)δ:2.59(3H,s),4.26(2H,s),7.36-7.42(1H,m),7.42-7.49(1H,m),7.58(1H,s),7.62(1H,ddd,J=7.8,4.9,0.9Hz),7.66-7.74(2H,m),8.14(1H,ddd,J=9.6,7.7,1.7Hz),8.51(1H,dt,J=4.7,1.6Hz ), 9.26 (2H, brs).

### Example 120

### 1-{1-(2-fluoropyridin-3-yl)-5-[(3-methoxyphenyl)sulfonyl]-1H-pyrazol-3-yl}-N-methylmethanamine hydrochloride

To a solution of tert-butyl ({1-(2-fluoropyridin-3-yl)-5-[(3-methoxyphenyl)sulfonyl]-1H-pyrazol-3-yl}methyl)methylcarbamate (189mg) in a mixed solvent of ethyl acetate (2 mL) and 2-propanol (1 mL) was added 4 mol/L hydrogen chloride-ethyl acetate solution (3 mL). The mixture was stirred at room temperature for 1 hr, and concentrated under reduced pressure, and the residue was recrystallized from ethanol to give the title compound as colorless crystals (147 mg, yield 89%).
¹H-NMR(DMSO-d₆)δ: 2.59(3H,s),3.77(3H,s),4.25(2H,s),6.95(1H,s),7.16(1H,d,J=7.5Hz) ,7.35(1H,dd,J=8.3,2.6Hz),7.50-7.58(2H,m),7.61(1H,dd,J=7.5,4.9Hz),8.12(1H,t,J=8.3Hz),8.50(1H, d,J=4.9Hz),9.32(2H,brs).

### Example 121

### 1-[1-(2-chloropyridin-3-yl)-5-(phenylsulfonyl)-1H-pyrazol-3-yl]-N-methylmethanamine hydrochloride

To a solution of 1-(2-chloropyridin-3-yl)-5-(phenylsulfonyl)-1H-pyrazole-3-carbaldehyde (360 mg) in methanol (5 mL) were added methylammonium chloride (77 mg), anhydrous magnesium sulfate (188 mg) and triethylamine (116 mg), and the mixture was stirred at room temperature for 1 hr. Sodium borohydride (49 mg) was added under ice-cooling, and the mixture was further stirred at room temperature for 1 hr. The solvent was evaporated under reduced pressure, water was added to the residue, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=1:1→1:4) to give the free base of the title compound as a colorless oil (300 mg). The obtained free base was dissolved in a mixed solvent of ethyl acetate (2 mL) and ethanol (1 mL), 4 mol/L hydrochloric acid-ethyl acetate solution (3 mL) was added. The solvent was evaporated under reduced pressure, and the residue was recrystallized from ethanol to give the title compound as colorless crystals (149 mg, yield 36%).
¹H-NMR(DMSO-d₆)δ: 2.55(3H,s) ,4.24(2H,s), 7.47-7.59(5H,m), 7.66-7.71(1H,m),7.72-7.82(1H,m),8.00-8.08(1H,m),8.61-8.70(1 H,m),9.34(2H,brs).

### Example 122

### 1-[1-(2-methoxypyridin-3-yl)-5-(phenylsulfonyl)-1H-pyrazol-3-yl]-N-methylmethanamine fumarate

To a solution of tert-butyl {[1-(2-methoxypyridin-3-yl)-5-(phenylsulfonyl)-1H-pyrazole-3-yl]methyl}methylcarbamate (323 mg) in a mixed solvent of ethyl acetate (2 mL) and ethanol (1 mL) was added 4 mol/L hydrogen chloride-ethyl acetate solution (3 mL). The mixture was stirred at room temperature for 2 hr, and concentrated under reduced pressure. Saturated aqueous sodium hydrogen carbonate solution was added to the residue, and the mixture was extracted with ethyl acetate. The extract was washed with saturated brine, dried over anhydrous sodium sulfate, and concentrated under reduced pressure to give the free base of the title compound (225 mg). The obtained free base (225 mg) was dissolved in ethyl acetate (2 mL), and the solution was added to a solution of fumaric acid (73 mg) in ethanol (10 mL). The mixture was concentrated under reduced pressure, and the residue was recrystallized from ethanol-water to give the title compound as a colorless solid (196 mg, yield 59%).
¹H-NMR(DMSO-d₆)δ: 2.42(3H,s),3.40(3H,s),3.92(2H,s),6.52(2H,s),7.08-7.20(1H,m),7.29(1H,s),7.42-7.50(2H,m),7.55(2H,t,J=7.8Hz),7.67-7.80(2H,m),8.29-8.39(1H,m),3H: not detected.

### Example 123

### 3-{3-[(methylamino)methyl]-5-(phenylsufonyl)-1H-pyrazol-1-yl}pyridine-2-carbonitrile hydrochloride

To a solution of tert-butyl {[1-(2-cyanopyridin-3-yl)-5-(phenylsulfonyl)-1H-pyrazole-3-yl]methyl}methylcarbamate (145 mg) in a mixed solvent of ethyl acetate (2 mL) and ethanol (1 mL) was added 4 mol/L hydrogen chloride-ethyl acetate solution (3 mL). The mixture was stirred at room temperature for 2 hr, and concentrated under reduced pressure, and the residue was recrystallized from ethanol to give the title compound as colorless crystals (69 mg, yield 52%).
¹H-NMR(DMSO-d₆)δ: 2.56(3H,s)4.28(2H,s)7.48-7.54(2H,m)7.54-7.63(3H,m),7.74-7.83(1H,m),8.01(1H,dd,J=8.2,4.9Hz),8.22(1H,dd,J=8.4,1.5Hz),8.9 8(1H,dd,J=4.8,1.5Hz),9.25(2H,brs).

The structures of the compounds described in Reference Examples are shown in Tables 1-40.

**Table 1**

| | | | | |
|---|---|---|---|---|
| 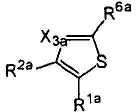 | | | | |

| Ref. No. | R^{1a} | R^{2a} | X₃ₐ | R^{6a} |
|---|---|---|---|---|
| 1 | 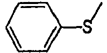 | Br | CH | CHO |
| 2 | 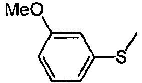 | Br | CH | CHO |
| 3 | 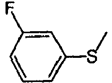 | Br | CH | CHO |
| 4 | 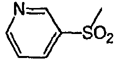 | Br | CH | CHO |
| 5 | 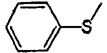 | Br | CMe | CO₂Me |
| 6 | H | 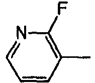 | CH | CHO |
| 7 | H | 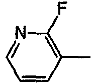 | CH | 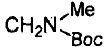 |
| 8 | Br | 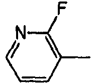 | CH | 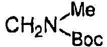 |
| 9 | 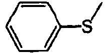 | Br | CH | 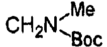 |
| 10 | 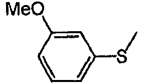 | Br | CH | 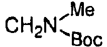 |
| 11 | 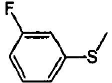 | Br | CH | 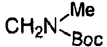 |

**Table 2**

| | | | | |
|---|---|---|---|---|
| 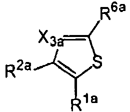 | | | | |

| Ref. No. | R^{1a} | R^{2a} | X₃ₐ | R^{6a} |
|---|---|---|---|---|
| 12 | 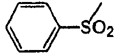 | Br | CH | 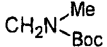 |
| 13 | 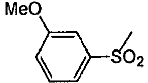 | Br | CH | 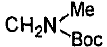 |
| 14 | 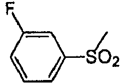 | Br | CH | 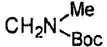 |
| 15 | 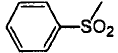 | Br | CMe | CO₂Me |
| 16 | 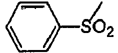 | Br | CMe | CONHMe |
| 17 | 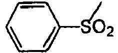 | Br | CMe | 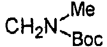 |
| 18 | 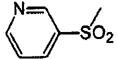 | 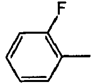 | CH | CHO |
| 19 | 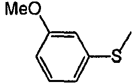 | 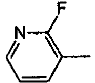 | CH | 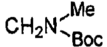 |
| 20 | 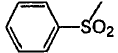 | 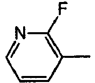 | CH | 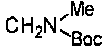 |
| 21 | 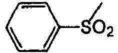 | 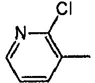 | CH | 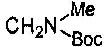 |
| 22 | 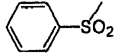 | 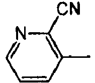 | CH | 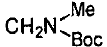 |

**Table 3**

| | | | | |
|---|---|---|---|---|
| 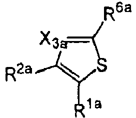 | | | | |

| Ref. No. | R^{1a} | R^{2a} | X₃ₐ | R^{6a} |
|---|---|---|---|---|
| 23 | 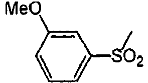 | 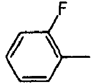 | CH | 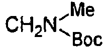 |
| 24 | 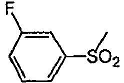 | 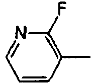 | CH | 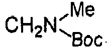 |
| 25 | 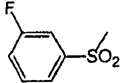 | 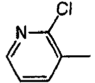 | CH | 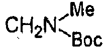 |
| 26 | 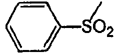 | 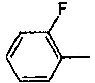 | CMe | 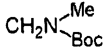 |
| 27 | 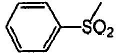 | 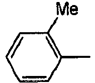 | CMe | 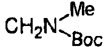 |
| 28 | 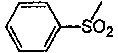 | 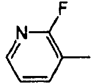 | CMe | 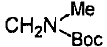 |
| 29 | 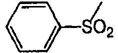 | 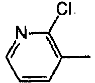 | CMe | 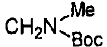 |
| 30 | 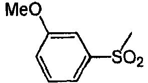 | 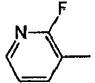 | CH | 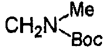 |
| 41 | 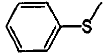 | 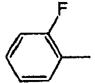 | N | 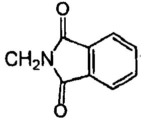 |
| 42 | H | 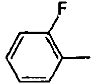 | N | 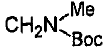 |

**Table 4**

| | | | | |
|---|---|---|---|---|
| 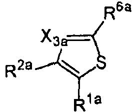 | | | | |

| Ref. No. | R^{1a} | R^{2a} | X₃ₐ | R^{6a} |
|---|---|---|---|---|
| 43 | H | 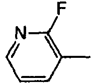 | N | 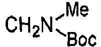 |
| 44 | Br | 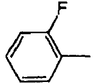 | N | 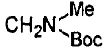 |
| 45 | Br | 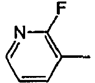 | N | 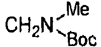 |
| 46 | 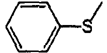 | 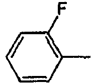 | N | CH₂NH₂ |
| 47 | 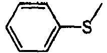 | 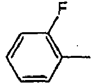 | N | 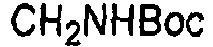 |
| 48 | 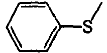 | 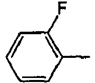 | N | 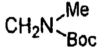 |
| 49 | 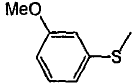 | 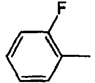 | N | 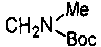 |
| 50 | 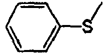 | 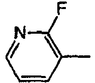 | N | 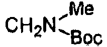 |
| 51 | 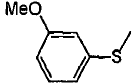 | 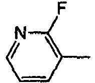 | N | 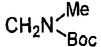 |
| 52 | 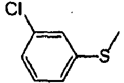 | 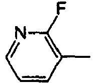 | N | 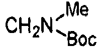 |
| 53 | 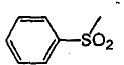 | 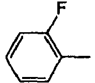 | N | 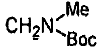 |

**Table 5**

| | | | | |
|---|---|---|---|---|
| 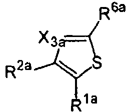 | | | | |

| Ref. No. | R^{1a} | R^{2a} | X₃ₐ | R^{6a} |
|---|---|---|---|---|
| 54 | 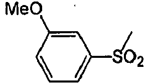 | 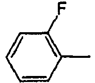 | N | 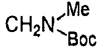 |
| 55 | 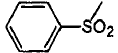 | 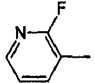 | N | 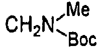 |
| 56 | 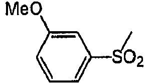 | 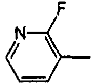 | N | 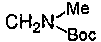 |
| 57 | 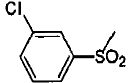 | 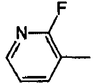 | N | 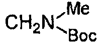 |
| 58 | 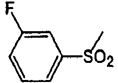 | 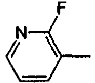 | N | 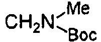 |

**Table 6**

| | | | | |
|---|---|---|---|---|
| 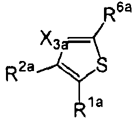 | | | | |

| Ref. No. | R^{1a} | R^{2a} | X₃ₐ | R^{6a} |
|---|---|---|---|---|
| 62 | Br | 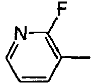 | CH | CHO |
| 63 | Br | 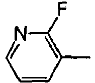 | CH | CO₂H |
| 64 | Br | 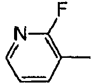 | CH | CD₂OH |
| 65 | Br | 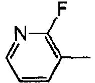 | CH | CDO |
| 73 | 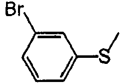 | 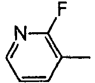 | CH | CHO |
| 74 | 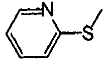 | 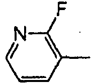 | CH | CHO |
| 75 | 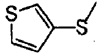 | 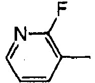 | CH | CHO |
| 76 | 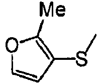 | 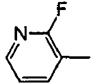 | CH | CHO |
| 77 | 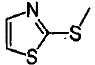 | 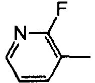 | CH | CHO |
| 78 | 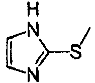 | 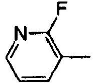 | CH | CHO |
| 79 | 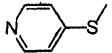 | 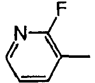 | CH | CHO |
| 80 | 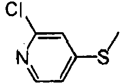 | 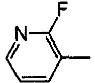 | CH | CHO |

**Table 7**

| | | | | |
|---|---|---|---|---|
| 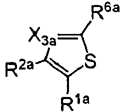 | | | | |

| Ref. No. | R^{1a} | R^{2a} | X₃ₐ | R^{6a} |
|---|---|---|---|---|
| 81 | 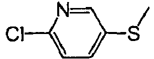 | 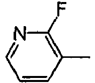 | CH | CHO |
| 82 | 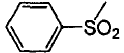 | Br | CH | CHO |
| 83 | 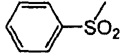 | 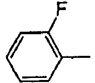 | CH | CHO |
| 84 | 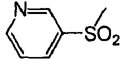 | 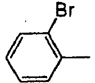 | CH | CHO |
| 85 | 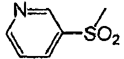 | 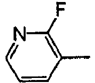 | CH | CHO |
| 86 | 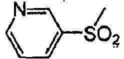 | 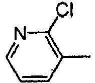 | CH | CHO |
| 87 | 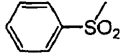 | 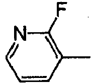 | CH | CHO |
| 88 | 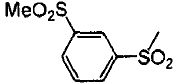 | 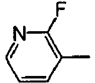 | CH | CHO |
| 89 | 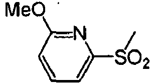 | 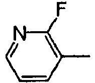 | CH | CHO |
| 90 | 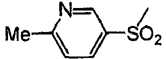 | 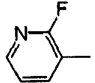 | CH | CHO |
| 91 | 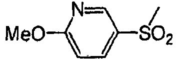 | 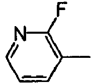 | CH | CHO |
| 92 | 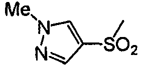 | 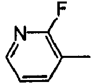 | CH | CHO |

**Table 8**

| | | | | |
|---|---|---|---|---|
| 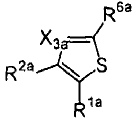 | | | | |

| Ref. No. | R^{1a} | R^{2a} | X₃ₐ | R^{6a} |
|---|---|---|---|---|
| 93 | 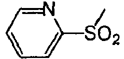 | 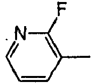 | CH | CDO |
| 94 | 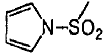 | Br | CH | H |
| 95 | 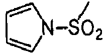 | 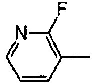 | CH | H |
| 96 | 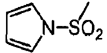 | 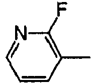 | CH | CHO |
| 97 | 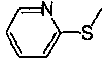 | 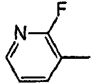 | CH | CH₂NHMe |
| 98 | 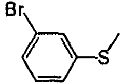 | 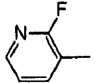 | CH | 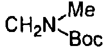 |
| 99 | 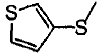 | 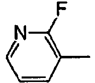 | CH | 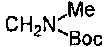 |
| 100 | 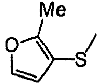 | 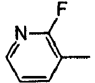 | CH | 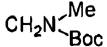 |
| 101 | 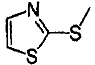 | 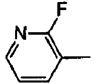 | CH | 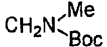 |
| 102 | 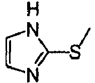 | 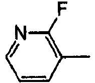 | CH | 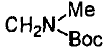 |
| 103 | 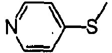 | 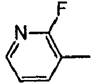 | CH | 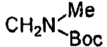 |
| 104 | 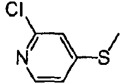 | 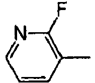 | CH | 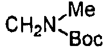 |

**Table 9**

| | | | | |
|---|---|---|---|---|
| 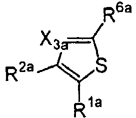 | | | | |

| Ref. No. | R^{1a} | R^{2a} | X₃ₐ | R^{6a} |
|---|---|---|---|---|
| 105 | 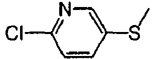 | 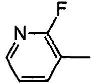 | CH | 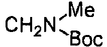 |
| 106 | 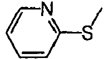 | 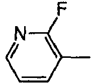 | CH | 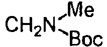 |
| 107 | 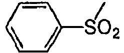 | 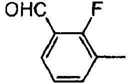 | CH | 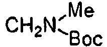 |
| 108 | 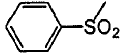 | 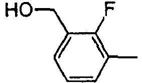 | CH | 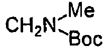 |
| 109 | 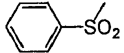 | 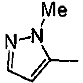 | CH | 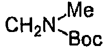 |
| 110 | 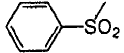 | 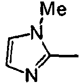 | CH | 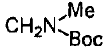 |
| 111 | 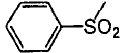 | 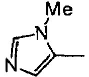 | CH | 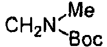 |
| 112 | 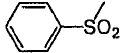 | 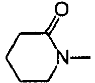 | CH | 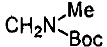 |
| 113 | 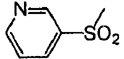 | 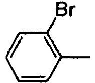 | CH | 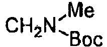 |
| 114 | 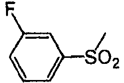 | 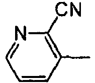 | CH | 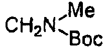 |
| 115 | 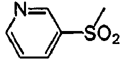 | 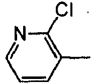 | CH | 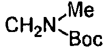 |
| 116 | 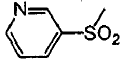 | 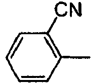 | CH | 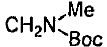 |

**Table 10**

| | | | | |
|---|---|---|---|---|
| 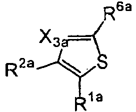 | | | | |

| Ref. No. | R^{1a} | R^{2a} | X₃ₐ | R^{6a} |
|---|---|---|---|---|
| 117 | 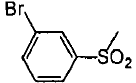 | 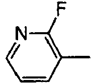 | CH | 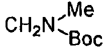 |
| 118 | 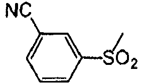 | 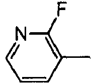 | CH | 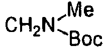 |
| 119 | 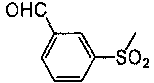 | 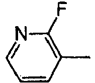 | CH | 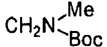 |
| 120 | 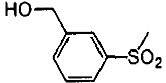 | 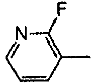 | CH | 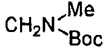 |
| 121 | 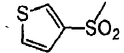 | 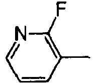 | CH | 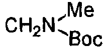 |
| 122 | 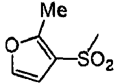 | 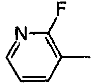 | CH | 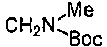 |
| 123 | 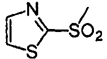 | 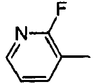 | CH | 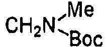 |
| 124 | 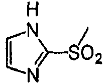 | 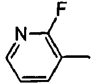 | CH | 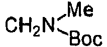 |
| 125 | 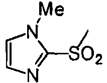 | 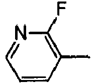 | CH | 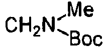 |
| 126 | 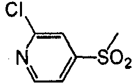 | 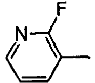 | CH | 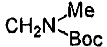 |
| 127 | 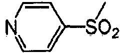 | 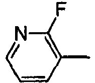 | CH | 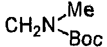 |
| 128 | 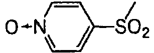 | 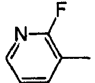 | CH | 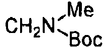 |

**Table 11**

| | | | | |
|---|---|---|---|---|
| 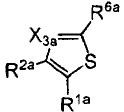 | | | | |

| Ref. No. | R^{1a} | R^{2a} | X₃ₐ | R^{6a} |
|---|---|---|---|---|
| 129 | 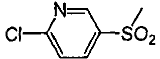 | 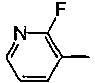 | CH | 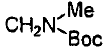 |
| 130 | 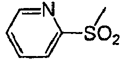 | 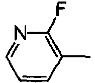 | CH | 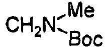 |
| 131 | 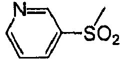 | 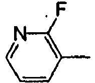 | CH | 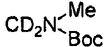 |
| 132 | 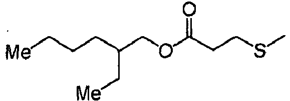 | 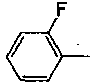 | N | 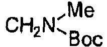 |
| 133 | 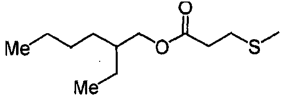 | 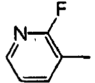 | N | 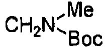 |
| 135 | 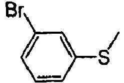 | 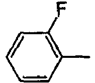 | N | 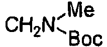 |
| 136 | 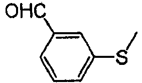 | 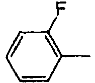 | N | 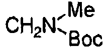 |
| 137 | 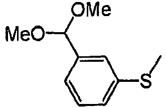 | 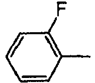 | N | 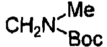 |
| 138 | 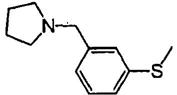 | 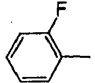 | N | 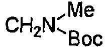 |
| 139 | 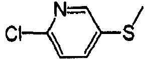 | 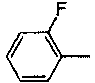 | N | 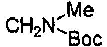 |
| 140 | 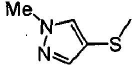 | 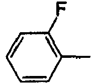 | N | 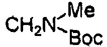 |

**Table 12**

| | | | | |
|---|---|---|---|---|
| 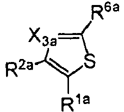 | | | | |

| Ref. No. | R^{1a} | R^{2a} | X₃ₐ | R^{6a} |
|---|---|---|---|---|
| 141 | 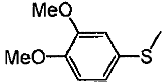 | 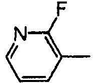 | N | 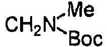 |
| 142 | 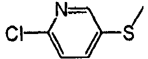 | 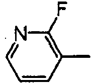 | N | 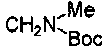 |
| 143 | 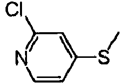 | 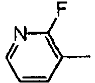 | N | 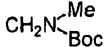 |
| 144 | 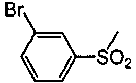 | 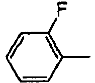 | N | 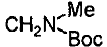 |
| 145 | 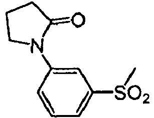 | 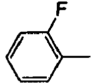 | N | 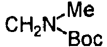 |
| 146 | 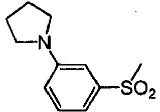 | 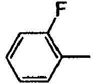 | N | 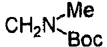 |
| 147 | 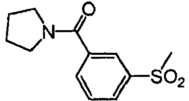 | 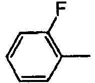 | N | 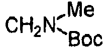 |
| 148 | 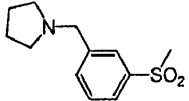 | 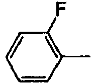 | N | 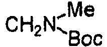 |
| 149 | 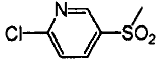 | 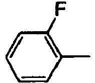 | N | 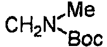 |
| 150 | 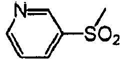 | 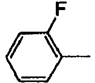 | N | 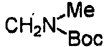 |

**Table 13**

| | | | | |
|---|---|---|---|---|
| 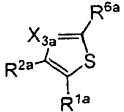 | | | | |

| Ref. No. | R^{1a} | R^{2a} | X₃ₐ | R^{6a} |
|---|---|---|---|---|
| 151 | 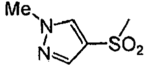 | 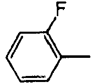 | N | 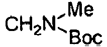 |
| 152 | 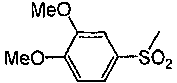 | 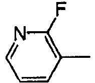 | N | 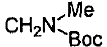 |
| 153 | 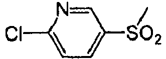 | 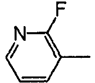 | N | 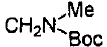 |
| 154 | 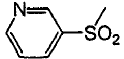 | 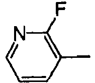 | N | 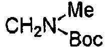 |
| 155 | 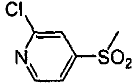 | 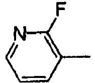 | N | 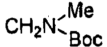 |

**Table 14**

| | | | | | |
|---|---|---|---|---|---|
| 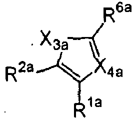 | | | | | |

| Ref. No. | R^{1a} | R^{2a} | X₃ₐ | X₄ₐ | R^{6a} |
|---|---|---|---|---|---|
| 156 | H | 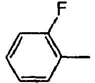 | O | CH | CHO |
| 157 | H | 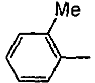 | O | CH | CHO |
| 158 | Br | 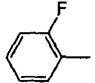 | O | CH | CHO |
| 159 | Br | 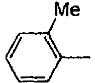 | O | CH | CHO |
| 160 | 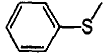 | 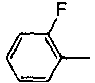 | O | CH | CHO |
| 161 | 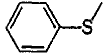 | 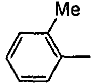 | O | CH | CHO |
| 162 | 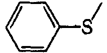 | 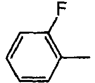 | O | CH | 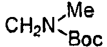 |
| 163 | 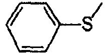 | 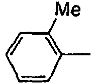 | O | CH | 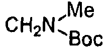 |
| 164 | 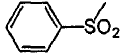 | 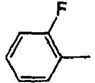 | O | CH | 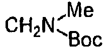 |
| 165 | 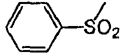 | 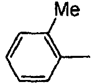 | O | CH | 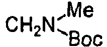 |

**Table 15**

| | | | | | |
|---|---|---|---|---|---|
| 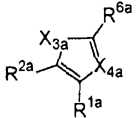 | | | | | |

| Ref. No. | R^{1a} | R^{2a} | X₃ₐ | X₄ₐ | R^{6a} |
|---|---|---|---|---|---|
| 166 | Br | H | S | CH | 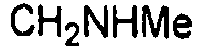 |
| 167 | Br | H | S | CH | 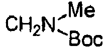 |
| 168 | 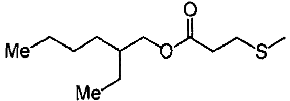 | H | S | CH | 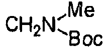 |
| 169 | 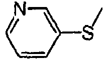 | H | S | CH | 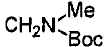 |
| 170 | 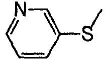 | Br | S | CH | 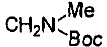 |
| 171 | 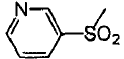 | Br | S | CH | 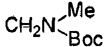 |
| 172 | 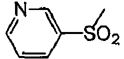 | 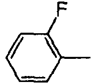 | S | CH | 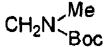 |
| 173 | 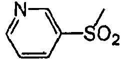 | 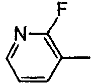 | S | CH | 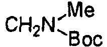 |
| 174 | 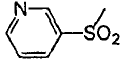 | 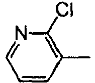 | S | CH | 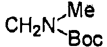 |

**Table 16**

| | | | | |
|---|---|---|---|---|
| 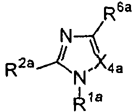 | | | | |

| Ref. No. | R^{1a} | R^{2a} | X₄ₐ | R^{6a} |
|---|---|---|---|---|
| 175 | H | 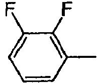 | CH | CH₂OH |
| 176 | H | 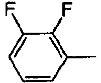 | CH | CHO |
| 177 | 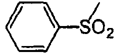 | 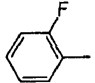 | CH | CHO |
| 178 | 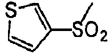 | 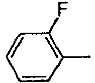 | CH | CHO |
| 179 | 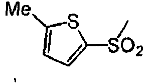 | 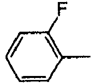 | CH | CHO |
| 180 | 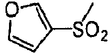 | 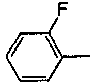 | CH | CHO |
| 181 | 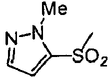 | 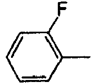 | CH | CHO |
| 182 | 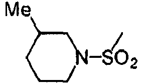 | 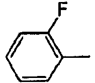 | CH | CHO |
| 183 | 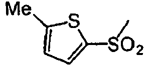 | 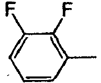 | CH | CHO |

**Table 17**

| | | | | |
|---|---|---|---|---|
| 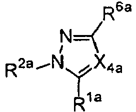 | | | | |

| Ref. No. | R^{1a} | R^{2a} | X₄ₐ | R^{6a} |
|---|---|---|---|---|
| 192 | OH | 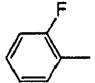 | CH | CO₂Et |
| 193 | Br | 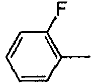 | CH | CO₂Et |
| 194 | OH | 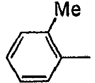 | CH | CO₂Et |
| 195 | NH₂ | 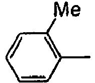 | CH | CO₂Et |
| 196 | NH₂ | 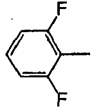 | CH | CO₂Et |
| 197 | I | 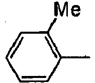 | CH | CO₂Et |
| 198 | I | 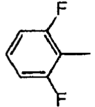 | CH | CO₂Et |
| 199 | OH | 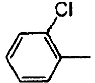 | CH | CO₂Et |
| 200 | OH | 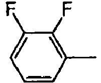 | CH | CO₂Et |
| 201 | OH | 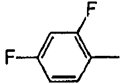 | CH | CO₂Et |
| 202 | OH | 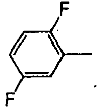 | CH | CO₂Et |

**Table 18**

| | | | | |
|---|---|---|---|---|
| 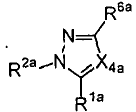 | | | | |

| Ref. No. | R^{1a} | R^{2a} | X₄ₐ | R^{6a} |
|---|---|---|---|---|
| 203 | OH | 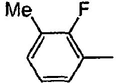 | CH | CO₂Et |
| 204 | OH | 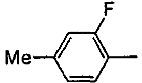 | CH | CO₂Et |
| 205 | OH | 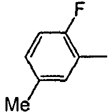 | CH | CO₂Et |
| 206 | OH | 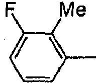 | CH | CO₂Et |
| 207 | OH | 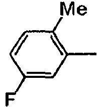 | CH | CO₂Et |
| 208 | OH | 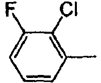 | CH | CO₂Et |
| 209 | OH | 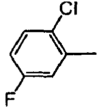 | CH | CO₂Et |
| 210 | OH | 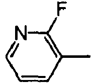 | CH | CO₂Et |
| 211 | OTf | 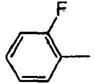 | CH | CO₂Et |
| 212 | OTf | 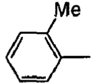 | CH | CO₂Et |
| 213 | OTf | 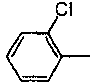 | CH | CO₂Et |

**Table 19**

| | | | | |
|---|---|---|---|---|
| 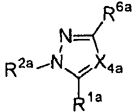 | | | | |

| Ref. No. | R^{1a} | R^{2a} | X₄ₐ | R^{6a} |
|---|---|---|---|---|
| 214 | OTf | 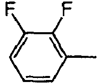 | CH | CO₂Et |
| 215 | OTf | 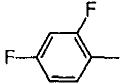 | CH | CO₂Et |
| 216 | OTf | 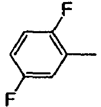 | CH | CO₂Et |
| 217 | OTf | 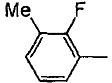 | CH | CO₂Et |
| 218 | OTf | 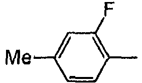 | CH | CO₂Et |
| 219 | OTf | 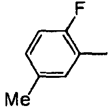 | CH | CO₂Et |
| 220 | OTf | 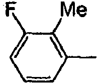 | CH | CO₂Et |
| 221 | OTf | 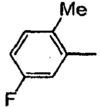 | CH | CO₂Et |
| 222 | OTf | 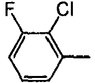 | CH | CO₂Et |
| 223 | OTf | 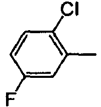 | CH | CO₂Et |
| 224 | OTf | 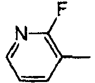 | CH | CO₂Et |

**Table 20**

| | | | | |
|---|---|---|---|---|
| 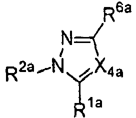 | | | | |

| Ref. No. | R^{1a} | R^{2a} | X₄ₐ | R^{6a} |
|---|---|---|---|---|
| 225 | 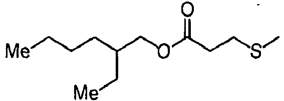 | 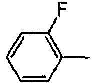 | CH | CO₂Et |
| 226 | 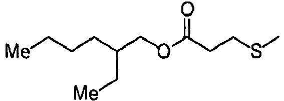 | 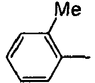 | CH | CO₂Et |
| 227 | 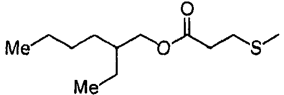 | 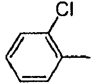 | CH | CO₂Et |
| 228 | 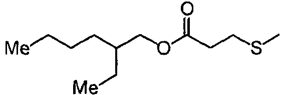 | 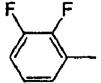 | CH | CO₂Et |
| 229 | 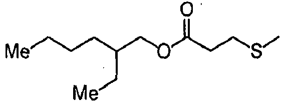 | 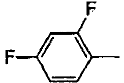 | CH | CO₂Et |
| 230 | 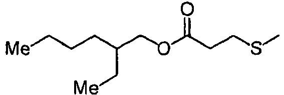 | 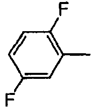 | CH | CO₂Et |
| 231 | 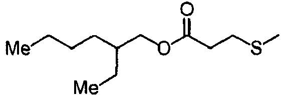 | 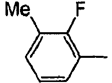 | CH | CO₂Et |
| 232 | 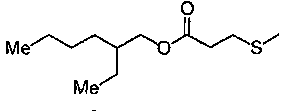 | 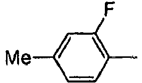 | CH | CO₂Et |
| 233 | 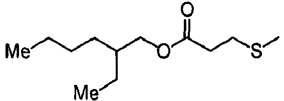 | 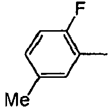 | CH | CO₂Et |
| 234 | 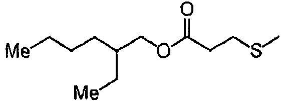 | 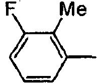 | CH | CO₂Et |

**Table 21**

| | | | | |
|---|---|---|---|---|
| 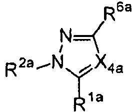 | | | | |

| Ref. No. | R^{1a} | R^{2a} | X₄ₐ | R^{6a} |
|---|---|---|---|---|
| 235 | 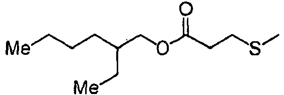 | 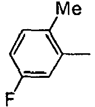 | CH | CO₂Et |
| 236 | 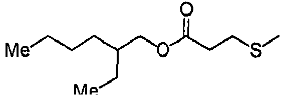 | 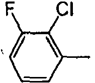 | CH | CO₂Et |
| 237 | 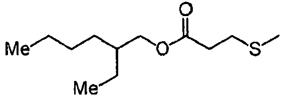 | 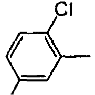 | CH | CO₂Et |
| 238 | 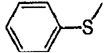 | 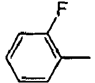 | CH | CO₂Et |
| 239 | 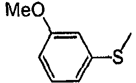 | 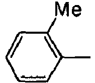 | CH | CO₂Et |
| 240 | 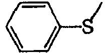 | 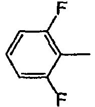 | CH | CO₂Et |
| 241 | 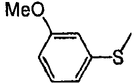 | 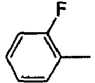 | CH | CO₂Et |
| 242 | 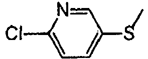 | 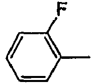 | CH | CO₂Et |
| 243 | 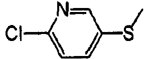 | 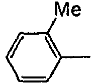 | CHO | CO₂Et |
| 244 | 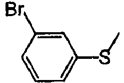 | 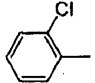 | CH | CO₂Et |
| 245 | 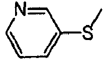 | 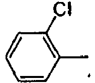 | CH | CO₂Et |

**Table 22**

| | | | | |
|---|---|---|---|---|
| 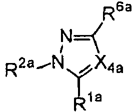 | | | | |

| Ref. No. | R^{1a} | R^{2a} | X₄ₐ | R^{6a} |
|---|---|---|---|---|
| 246 | 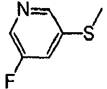 | 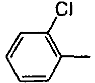 | CH | CO₂Et |
| 247 | 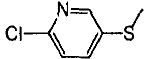 | 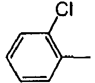 | CH | CO₂Et |
| 248 | 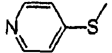 | 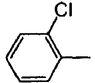 | CH | CO₂Et |
| 249 | 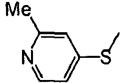 | 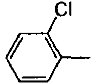 | CH | CO₂Et |
| 250 | 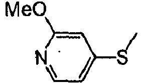 | 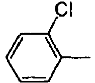 | CH | CO₂Et |
| 251 | 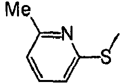 | 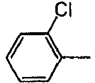 | CH | CO₂Et |
| 252 | 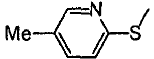 | 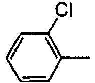 | CH | CO₂Et |
| 253 | 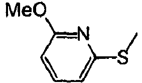 | 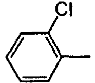 | CH | CO₂Et |
| 254 | 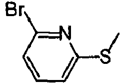 | 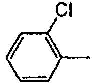 | CH | CO₂Et |
| 255 | 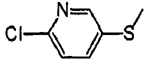 | 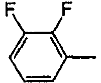 | CH | CO₂Et |

**Table 23**

| | | | | |
|---|---|---|---|---|
| 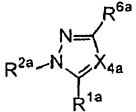 | | | | |

| Ref. No. | R^{1a} | R^{2a} | X₄ₐ | R^{6a} |
|---|---|---|---|---|
| 256 | 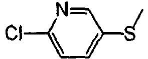 | 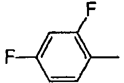 | CH | CO₂Et |
| 257 | 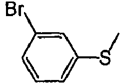 | 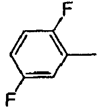 | CH | CO₂Et |
| 258 | 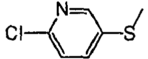 | 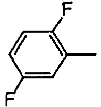 | CH | CO₂Et |
| 259 | 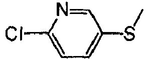 | 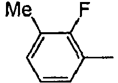 | CH | CO₂Et |
| 260 | 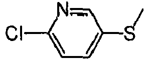 | 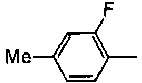 | CH | CO₂Et |
| 261 | 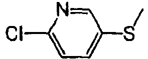 | 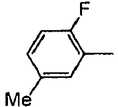 | CH | CO₂Et |
| 262 | 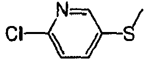 | 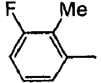 | CH | CO₂Et |
| 263 | 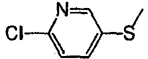 | 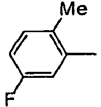 | CH | CO₂Et |
| 264 | 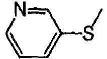 | 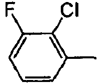 | CH | CO₂Et |

**Table 24**

| | | | | |
|---|---|---|---|---|
| 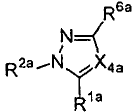 | | | | |

| Ref. No. | R^{1a} | R^{2a} | X₄ₐ | R^{6a} |
|---|---|---|---|---|
| 265 | 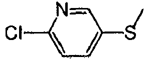 | 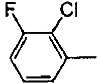 | CH | CO₂Et |
| 266 | 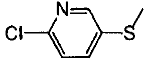 | 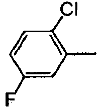 | CH | CO₂Et |
| 267 | 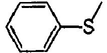 | 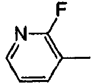 | CH | CO₂Et |
| 268 | 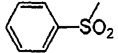 | 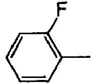 | CH | CO₂Et |
| 269 | 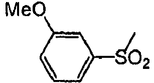 | 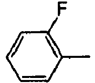 | CH | CO₂Et |
| 270 | 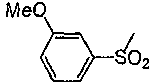 | 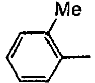 | CH | CO₂Et |
| 271 | 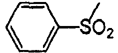 | 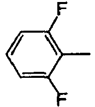 | CH | CO₂Et |
| 272 | 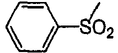 | 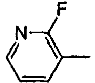 | CH | CO₂Et |
| 273 | 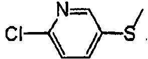 | 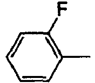 | CH | CONHMe |
| 274 | 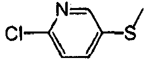 | 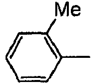 | CH | CONHMe |

**Table 25**

| | | | | |
|---|---|---|---|---|
| 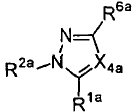 | | | | |

| Ref. No. | R^{1a} | R^{2a} | X₄ₐ | R^{6a} |
|---|---|---|---|---|
| 275 | 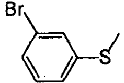 | 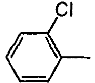 | CH | CONHMe |
| 276 | 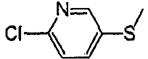 | 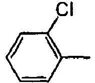 | CH | CONHMe |
| 277 | 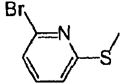 | 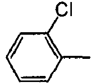 | CH | CONHMe |
| 278 | 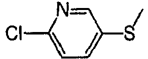 | 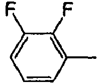 | CH | CONHMe |
| 279 | 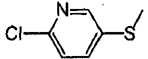 | 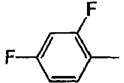 | CH | CONHMe |
| 280 | 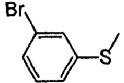 | 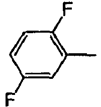 | CH | CONHMe |
| 281 | 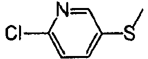 | 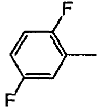 | CH | CONHMe |
| 282 | 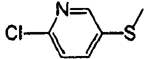 | 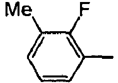 | CH | CONHMe |
| 283 | 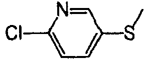 | 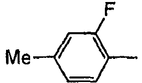 | CH | CONHMe |
| 284 | 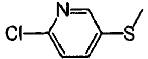 | 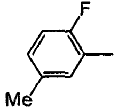 | CH | CONHMe |

**Table 26**

| | | | | |
|---|---|---|---|---|
| 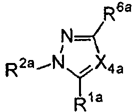 | | | | |

| Ref. No. | R^{1a} | R^{2a} | X₄ₐ | R^{6a} |
|---|---|---|---|---|
| 285 | 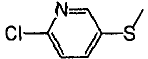 | 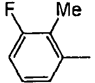 | CH | CONHMe |
| 286 | 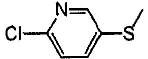 | 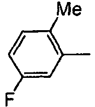 | CH | CONHMe |
| 287 | 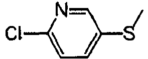 | 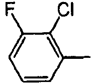 | CH | CONHMe |
| 288 | 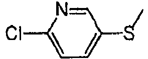 | 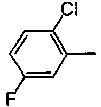 | CH | CONHMe |
| 289 | 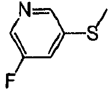 | 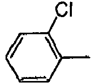 | CH | CH₂OH |
| 290 | 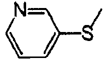 | 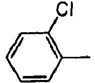 | CH | CHO |
| 291 | 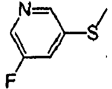 | 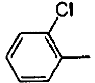 | CH | CHO |
| 292 | 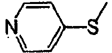 | 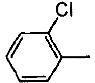 | CH | CHO |
| 293 | 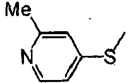 | 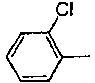 | CH | CHO |
| 294 | 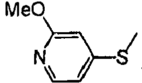 | 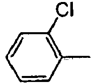 | CH | CHO |

**Table 27**

| | | | | |
|---|---|---|---|---|
| 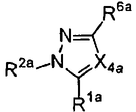 | | | | |

| Ref. No. | R^{1a} | R^{2a} | X₄ₐ | R^{6a} |
|---|---|---|---|---|
| 295 | 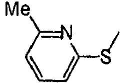 | 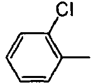 | CH | CHO |
| 296 | 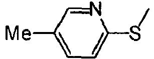 | 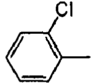 | CH | CHO |
| 297 | 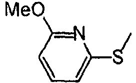 | 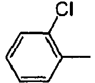 | CH | CHO |
| 298 | 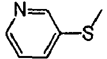 | 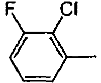 | CH | CHO |
| 299 | 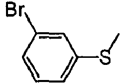 | 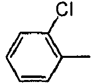 | CH | CH₂NHMe |
| 300 | 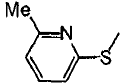 | 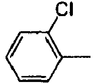 | CH | CH₂NHMe |
| 301 | 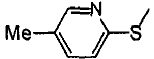 | 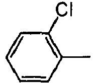 | CH | CH₂NHMe |
| 302 | 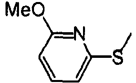 | 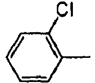 | CH | CH₂NHMe |
| 303 | 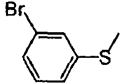 | 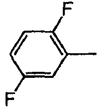 | CH | CH₂NHMe |
| 304 | 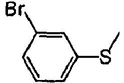 | 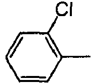 | CH | 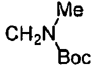 |
| 305 | 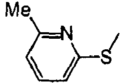 | 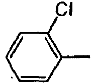 | CH | 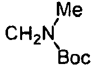 |

**Table 28**

| | | | | |
|---|---|---|---|---|
| 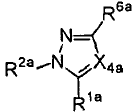 | | | | |

| Ref. No. | R^{1a} | R^{2a} | X₄ₐ | R^{6a} |
|---|---|---|---|---|
| 306 | 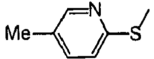 | 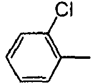 | CH | 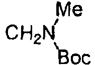 |
| 307 | 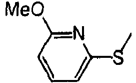 | 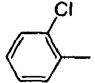 | CH | 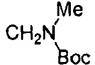 |
| 308 | 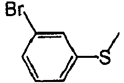 | 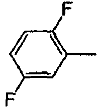 | CH | 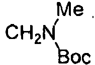 |
| 309 | 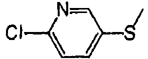 | 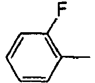 | CH | 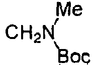 |
| 310 | 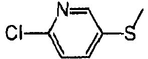 | 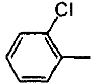 | CH | 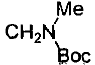 |
| 311 | 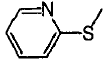 | 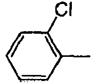 | CH | 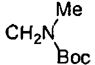 |
| 312 | 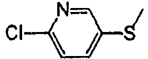 | 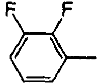 | CH | 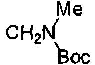 |
| 313 | 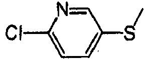 | 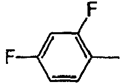 | CH | 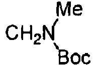 |
| 314 | 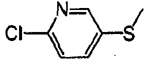 | 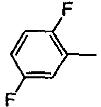 | CH | 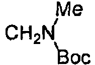 |
| 315 | 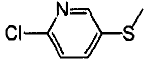 | 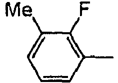 | CH | 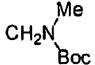 |
| 316 | 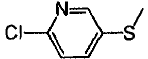 | 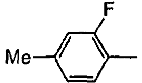 | CH | 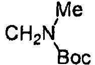 |

**Table 29**

| | | | | |
|---|---|---|---|---|
| 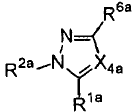 | | | | |

| Ref. No. | R^{1a} | R^{2a} | X₄ₐ | R^{6a} |
|---|---|---|---|---|
| 317 | 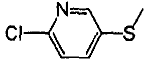 | 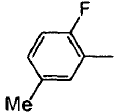 | CH | 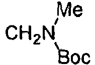 |
| 318 | 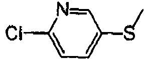 | 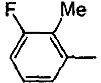 | CH | 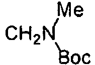 |
| 319 | 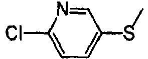 | 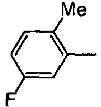 | CH | 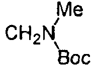 |
| 320 | 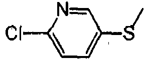 | 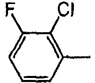 | CH | 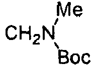 |
| 321 | 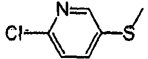 | 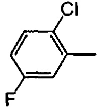 | CH | 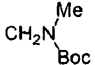 |
| 322 | 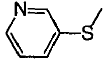 | 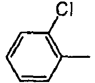 | CH | 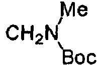 |
| 323 | 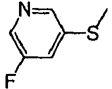 | 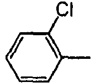 | CH | 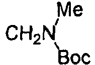 |
| 324 | 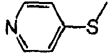 | 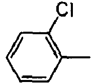 | CH | 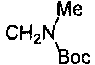 |
| 325 | 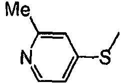 | 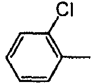 | CH | 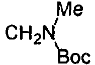 |
| 326 | 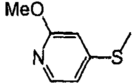 | 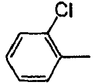 | CH | 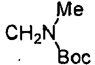 |

**Table 30**

| | | | | |
|---|---|---|---|---|
| 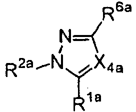 | | | | |

| Ref. No. | R^{1a} | R^{2a} | X₄ₐ | R^{6a} |
|---|---|---|---|---|
| 327 | 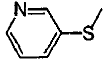 | 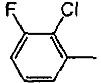 | CH | 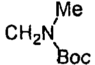 |
| 328 | 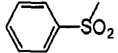 | 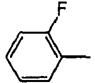 | CH | CONHMe |
| 329 | 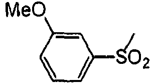 | 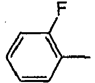 | CH | CONHMe |
| 330 | 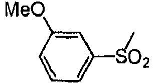 | 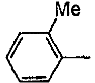 | CH | CONHMe |
| 331 | 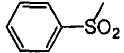 | 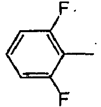 | CH | CONHMe |
| 332 | 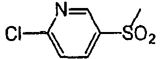 | 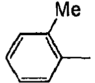 | CH | CONHMe |
| 333 | 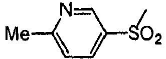 | 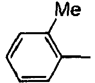 | CH | CONHMe |
| 334 | 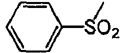 | 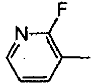 | CH | CH₂OH |
| 335 | 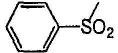 | 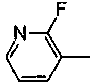 | CH | CHO |
| 336 | 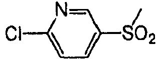 | 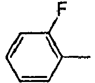 | CH | 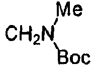 |
| 337 | 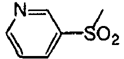 | 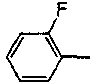 | CH | 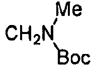 |

**Table 31**

| | | | | |
|---|---|---|---|---|
| 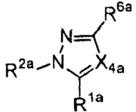 | | | | |

| Ref. No. | R^{1a} | R^{2a} | X₄ₐ | R^{6a} |
|---|---|---|---|---|
| 338 | 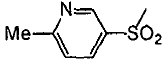 | 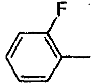 | CH | 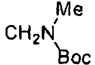 |
| 339 | 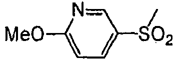 | 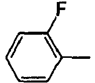 | CH | 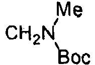 |
| 340 | 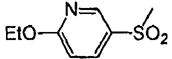 | 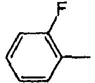 | CH | 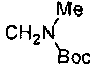 |
| 341 | 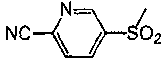 | 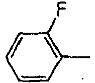 | CH | 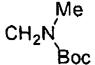 |
| 342 | 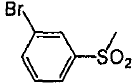 | 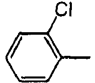 | CH | 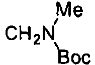 |
| 343 | 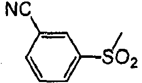 | 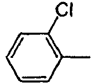 | CH | 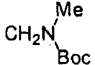 |
| 344 | 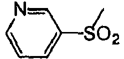 | 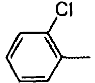 | CH | 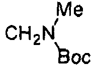 |
| 345 | 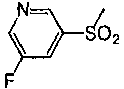 | 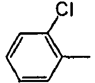 | CH | 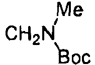 |
| 346 | 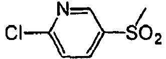 | 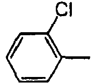 | CH | 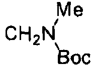 |
| 347 | 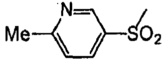 | 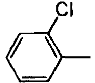 | CH | 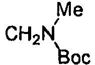 |
| 348 | 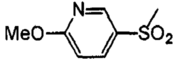 | 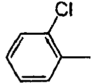 | CH | 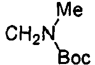 |

**Table 32**

| | | | | |
|---|---|---|---|---|
| 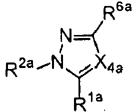 | | | | |

| Ref. No. | R^{1a} | R^{2a} | X₄ₐ | R^{6a} |
|---|---|---|---|---|
| 349 | 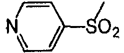 | 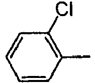 | CH | 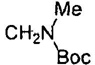 |
| 350 | 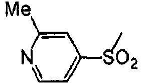 | 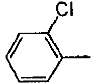 | CH | 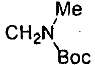 |
| 351 | 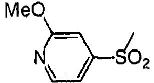 | 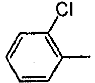 | CH | 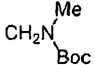 |
| 352 | 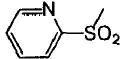 | 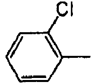 | CH | 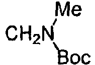 |
| 353 | 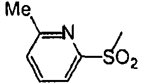 | 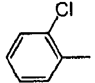 | CH | 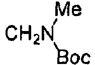 |
| 354 | 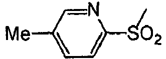 | 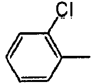 | CH | 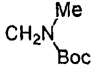 |
| 355 | 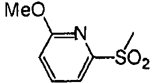 | 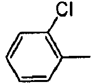 | CH | 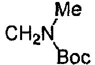 |
| 356 | 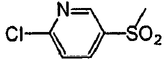 | 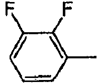 | CH | 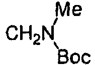 |
| 357 | 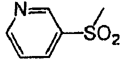 | 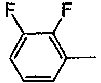 | CH | 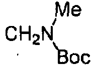 |
| 358 | 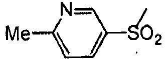 | 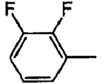 | CH | 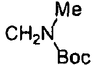 |
| 359 | 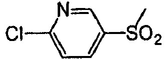 | 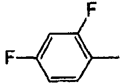 | CH | 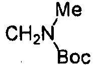 |

**Table 33**

| | | | | |
|---|---|---|---|---|
| 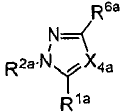 | | | | |

| Ref. No. | R^{1a} | R^{2a} | X₄ₐ | R^{6a} |
|---|---|---|---|---|
| 360 | 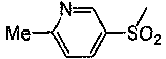 | 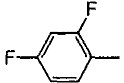 | CH | 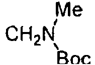 |
| 361 | 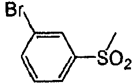 | 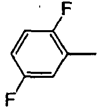 | CH | 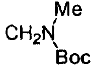 |
| 362 | 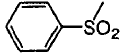 | 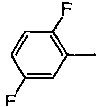 | CH | 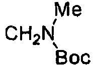 |
| 363 | 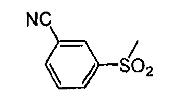 | 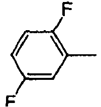 | CH | 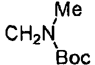 |
| 364 | 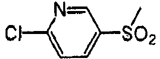 | 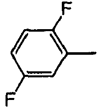 | CH | 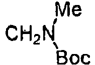 |
| 365 | 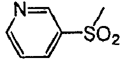 | 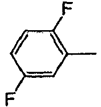 | CH | 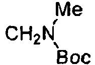 |
| 366 | 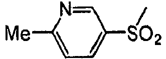 | 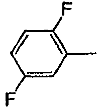 | CH | 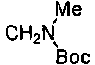 |
| 367 | 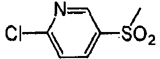 | 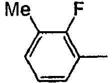 | CH | 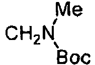 |
| 368 | 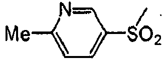 | 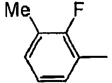 | CH | 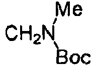 |
| 369 | 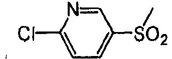 | 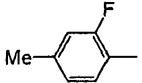 | CH | 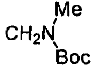 |

**Table 34**

| | | | | |
|---|---|---|---|---|
| 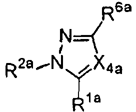 | | | | |

| Ref. No. | R^{1a} | R^{2a} | X₄ₐ | R^{6a} |
|---|---|---|---|---|
| 370 | 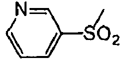 | 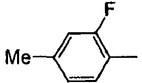 | CH | 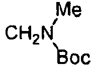 |
| 371 | 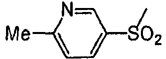 | 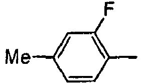 | CH | 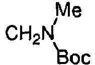 |
| 372 | 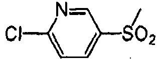 | 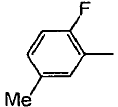 | CH | 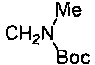 |
| 373 | 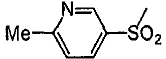 | 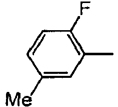 | CH | 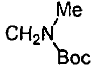 |
| 374 | 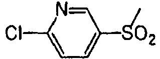 | 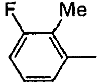 | CH | 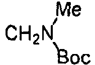 |
| 375 | 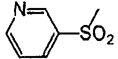 | 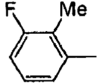 | CH | 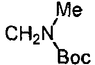 |
| 376 | 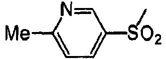 | 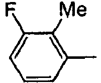 | CH | 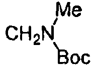 |
| 377 | 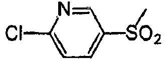 | 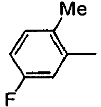 | CH | 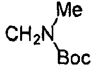 |
| 378 | 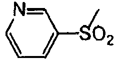 | 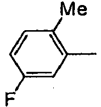 | CH | 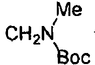 |
| 379 | 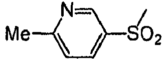 | 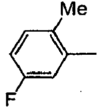 | CH | 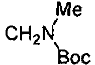 |

**Table 35**

| | | | | |
|---|---|---|---|---|
| 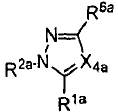 | | | | |

| Ref. No. | R^{1a} | R^{2a} | X₄ₐ | R^{6a} |
|---|---|---|---|---|
| 380 | 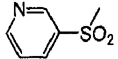 | 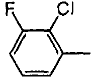 | CH | 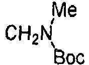 |
| 381 | 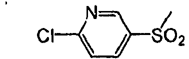 | 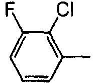 | CH | 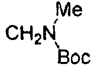 |
| 382 | 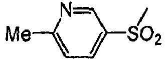 | 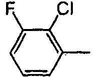 | CH | 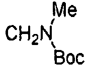 |
| 383 | 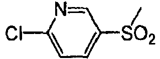 | 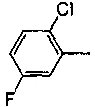 | CH | 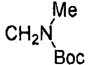 |
| 384 | 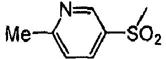 | 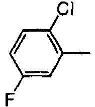 | CH | 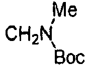 |

**Table 36**

| Ref. No. | | Ref. No. | | addition salt |
|---|---|---|---|---|
| 66 | 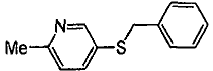 | 184 | 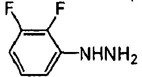 | HCl |
| 67 | 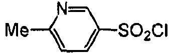 | 185 | 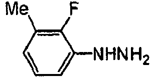 | |
| 68 | 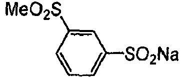 | 186 | 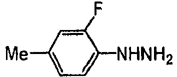 | |
| 69 | 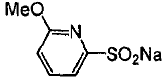 | 187 | 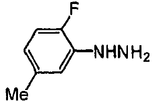 | |
| 70 | 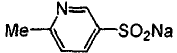 | 188 | 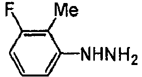 | HCl |
| 71 | 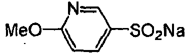 | 189 | 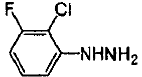 | HCl |
| 72 | 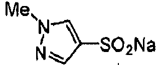 | 190 | 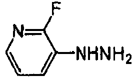 | |
| 134 | 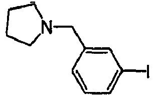 | 191 | 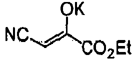 | |

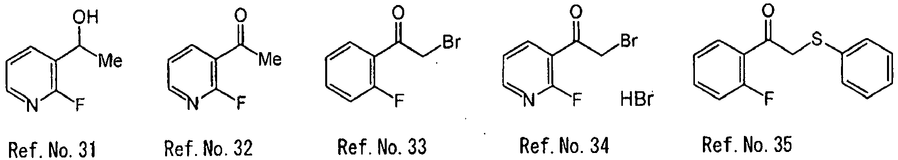

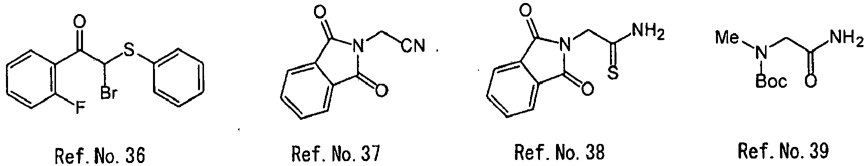

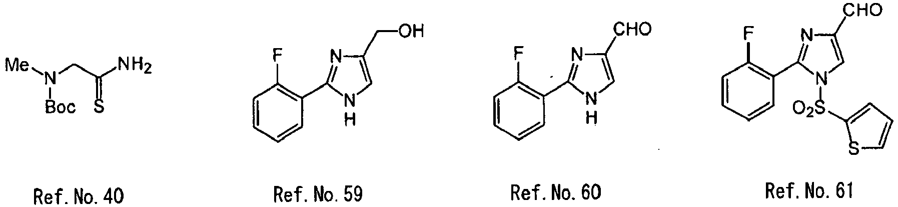

**Table 37**

| | | | | |
|---|---|---|---|---|
| 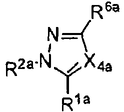 | | | | |

| Ref. Nb. | R^{1a} | R^{2a} | X₄ₐ | R^{6a} |
|---|---|---|---|---|
| 386 | OH | 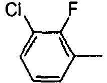 | CH | CO₂Et |
| 387 | OH | 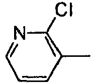 | CH | CO₂Et |
| 388 | OTf | 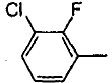 | CH | CO₂Et |
| 389 | OTf | 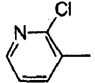 | CH | CO₂Et |
| 390 | 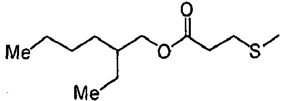 | 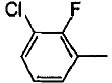 | CH | CO₂Et |
| 391 | 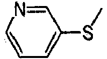 | 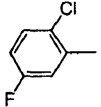 | CH | CO₂Et |
| 392 | 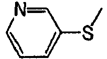 | 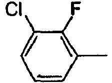 | CH | CO₂Et |
| 393 | 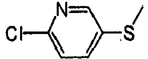 | 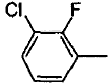 | CH | CO₂Et |
| 394 | 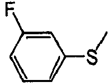 | 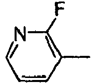 | CH | CO₂Et |
| 395 | 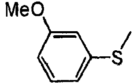 | 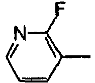 | CH | CO₂Et |
| 396 | 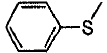 | 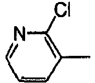 | CH | CO₂Et |

**Table 38**

| | | | | |
|---|---|---|---|---|
| 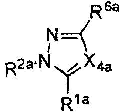 | | | | |

| Ref. Nb. | R^{1a} | R^{2a} | X₄ₐ | R^{6a} |
|---|---|---|---|---|
| 397 | 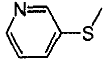 | 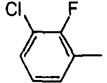 | CH | CH₂OH |
| 398 | 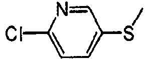 | 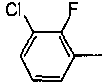 | CH | CH₂OH |
| 399 | 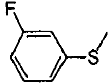 | 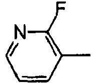 | CH | CH₂OH |
| 400 | 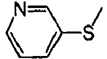 | 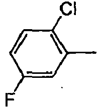 | CH | CHO |
| 401 | 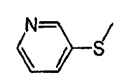 | 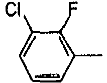 | CH | CHO |
| 402 | 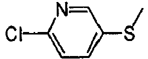 | 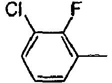 | CH | CHO |
| 403 | 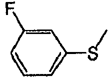 | 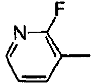 | CH | CHO |
| 404 | 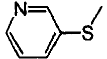 | 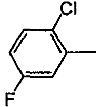 | CH | 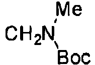 |
| 405 | 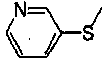 | 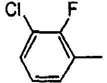 | CH, | 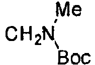 |
| 406 | 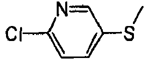 | 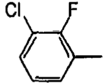 | CH | 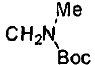 |

**Table 39**

| | | | | |
|---|---|---|---|---|
| 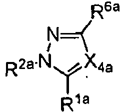 | | | | |

| Ref. No. | R^{1a} | R^{2a} | X₄ₐ | R^{6a} |
|---|---|---|---|---|
| 407 | 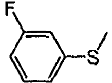 | 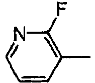 | CH | 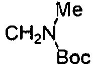 |
| 408 | 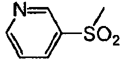 | 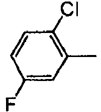 | CH | 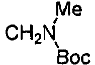 |
| 409 | 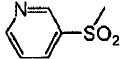 | 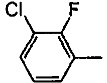 | CH | 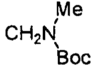 |
| 410 | 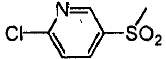 | 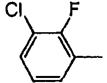 | CH | 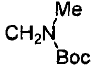 |
| 411 | 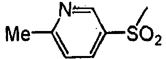 | 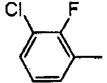 | CH | 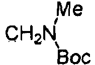 |
| 412 | 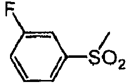 | 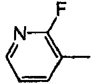 | CH | 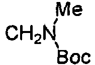 |
| 413 | 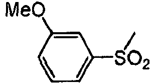 | 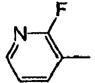 | CH | CO₂Et |
| 414 | 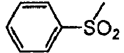 | 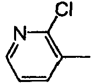 | CH | CO₂Et |
| 415 | 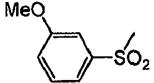 | 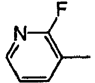 | CH | CH₂OH |
| 416 | 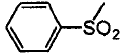 | 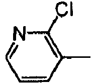 | CH | CH₂OH |

**Table 40**

| | | | | |
|---|---|---|---|---|
| 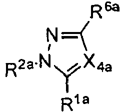 | | | | |

| Ref. Nb. | R^{1a} | R^{2a} | X₄ₐ | R^{6a} |
|---|---|---|---|---|
| 417 | 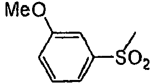 | 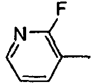 | CH | CHO |
| 418 | 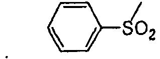 | 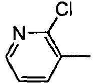 | CH | CHO |
| 419 | 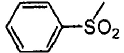 | 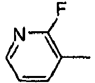 | CH | 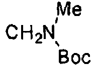 |
| 420 | 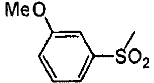 | 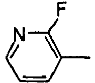 | CH | 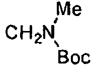 |
| 421 | 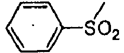 | 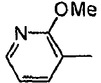 | CH | 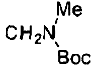 |
| 422 | 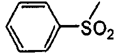 | 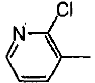 | CH | 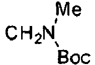 |
| 423 | 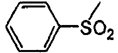 | 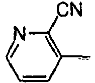 | CH | 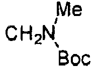 |

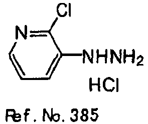

The structures of the compounds described in Examples are shown in Tables 41-53.

**Table 41**

| | | | | |
|---|---|---|---|---|
| 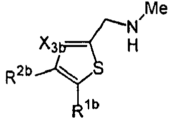 | | | | |

| Ex. No. | R^{1b} | R^{2b} | X_{3b} | addition salt |
|---|---|---|---|---|
| 1 | 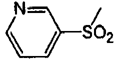 | 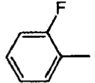 | CH | 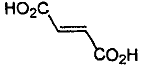 |
| 2 | 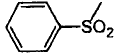 | 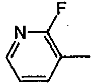 | CH | HCl |
| 3 | 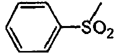 | 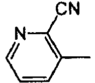 | CH | HCl |
| 4 | 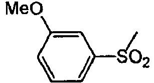 | 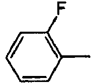 | CH | 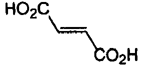 |
| 5 | 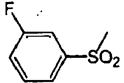 | 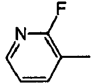 | CH | HCl |
| 6 | 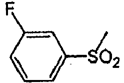 | 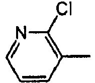 | CH | HCl |
| 7 | 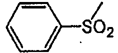 | 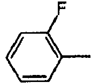 | CMe | HCl |
| 8 | 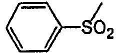 | 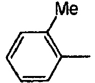 | CMe | HCl |
| 9 | 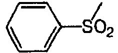 | 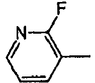 | CMe | HCl |
| 10 | 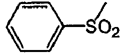 | 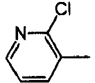 | CMe | HCl |
| 11 | 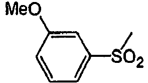 | 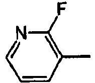 | CH | HCl |

**Table 42**

| | | | | |
|---|---|---|---|---|
| 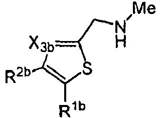 | | | | |

| Ex. No. | R^{1b} | R^{2b} | X_{3b} | addition salt |
|---|---|---|---|---|
| 12 | 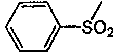 | 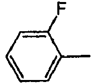 | N | HCl |
| 13 | 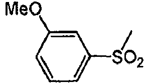 | 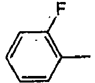 | N | HCl |
| 14 | 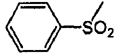 | 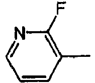 | N | HCl |
| 15 | 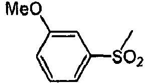 | 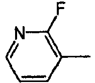 | N | HCl |
| 16 | 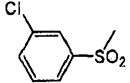 | 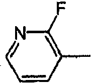 | N | HCl |
| 17 | 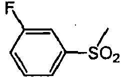 | 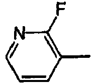 | N | HCl |

**Table 43**

| | | | | | |
|---|---|---|---|---|---|
| 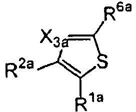 | | | | | |

| Ex. No. | R^{1a} | R^{2a} | X₃ₐ | R^{6a} | addition salt |
|---|---|---|---|---|---|
| 19 | 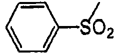 | 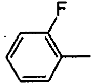 | CH | CH₂NHMe | HCl |
| 20 (comparative) | 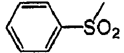 | 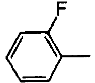 | CH | CH₂NHEt | HCl |
| 21 | 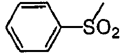 | 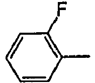 | CH | CH₂NMe₂ | HCl |
| 22 (comparative) | 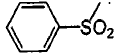 | 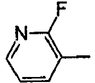 | CH | 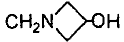 | |
| 23 | 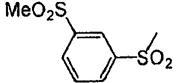 | 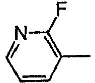 | CH | CH₂NHMe | HCl |
| 24 | 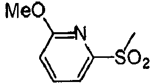 | 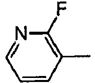 | CH | CH₂NHMe | 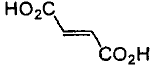 |
| 25 | 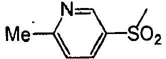 | 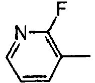 | CH | CH₂NHMe | 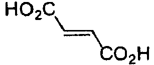 |
| 26 | 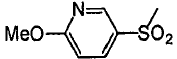 | 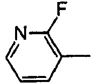 | CH | CH₂NHMe | 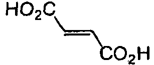 |
| 27 | 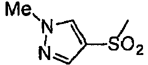 | 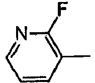 | CH | CH₂NHMe | HCl |
| 28 | 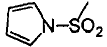 | 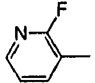 | CH | CH₂NHMe | HCl |
| 29 (comparative) | 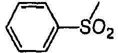 | 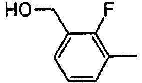 | CH | CH₂NHMe | 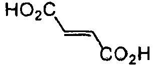 |
| 30 | 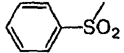 | 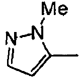 | CH | CH₂NHMe | 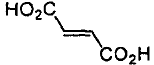 |

**Table 44**

| | | | | | |
|---|---|---|---|---|---|
| 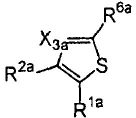 | | | | | |

| Ex. No. | R^{1a} | R^{2a} | X₃ₐ | R^{6a} | addition salt |
|---|---|---|---|---|---|
| 31 | 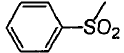 | 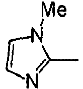 | CH | CH₂NHMe | ₂HCl |
| 32 | 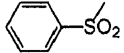 | 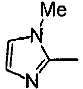 | CH | CH₂NHMe | 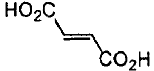 |
| 33 (comparative) | 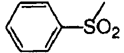 | 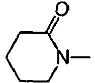 | CH | CH₂NHMe | HCl |
| 34 | 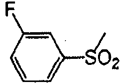 | 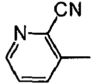 | CH | CH₂NHMe | HCl |
| 35 | 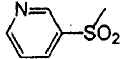 | 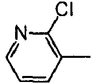 | CH | CH₂NHMe | 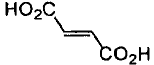 |
| 36 | 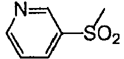 | 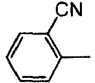 | CH | CH₂NHMe | HCl |
| 37 | 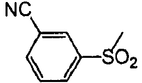 | 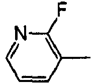 | CH | CH₂NHMe | HCl |
| 38 | 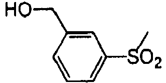 | 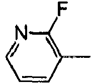 | CH | CH₂NHMe | 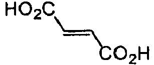 |
| 39 | 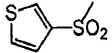 | 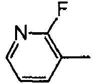 | CH | CH₂NHMe | HCl |

**Table 45**

| | | | | | |
|---|---|---|---|---|---|
| 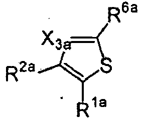 | | | | | |

| Ex. No. | R^{1a} | R^{2a} | X₃ₐ | R^{6a} | addition salt |
|---|---|---|---|---|---|
| 40 | 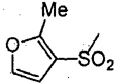 | 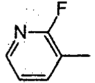 | CH | CH₂NHMe | HCl |
| 41 | 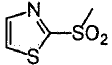 | 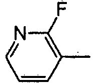 | CH | CH₂NHMe | HCl |
| 42 | 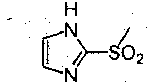 | 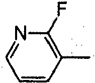 | CH | CH₂NHMe | HCl |
| 43 | 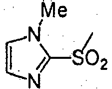 | 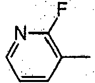 | CH | CH₂NHMe | HCl |
| 44 | 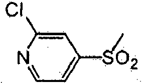 | 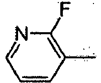 | CH | CH₂NHMe | HCl |
| 45 | 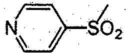 | 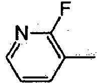 | CH | CH₂NHMe | ₂HCl |
| 46 (comparative) | 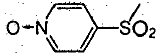 | 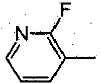 | CH | CH₂NHMe | HCl |
| 47 | 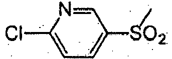 | 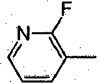 | CH | CH₂NHMe | HCl |
| 48 | 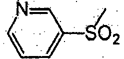 | 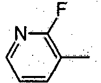 | CH | CH₂NHMe | 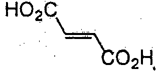 |
| 49 | 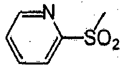 | 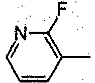 | CH | CH₂NHMe | 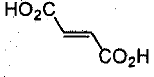 |
| 50 | 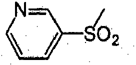 | 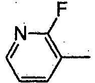 | CH | CD₂NHMe | 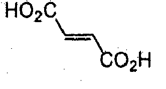 |

**Table 46**

| | | | | | |
|---|---|---|---|---|---|
| 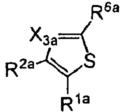 | | | | | |

| Ex. No. | R^{1a} | R^{2a} | X₃ₐ | R^{6a} | addition salt |
|---|---|---|---|---|---|
| 51 | 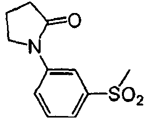 | 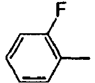 | N | CH₂NHMe | HCl |
| 52 | 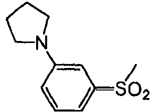 | 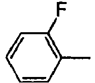 | N | CH₂NHMe | |
| 53 | 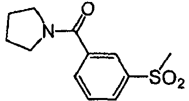 | 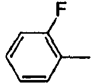 | N | CH₂NHMe | HC |
| 54 | 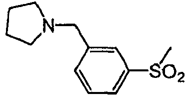 | 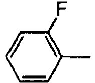 | N | CH₂NHMe | 2HCl |
| 55 | 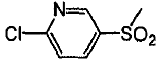 | 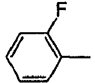 | N | CH₂NHMe | HCl |
| 56 | 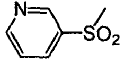 | 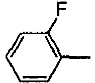 | N | CH₂NHMe | 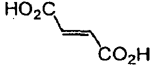 |
| 57 | 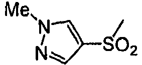 | 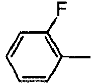 | N | CH₂NHMe | HCl |
| 58 | 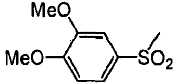 | 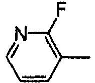 | N | CH₂NHMe | HCl |
| 59 | 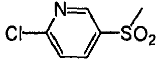 | 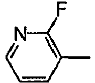 | N | CH₂NHMe | HCl |
| 60 | 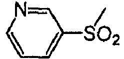 | 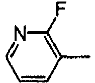 | N | CH₂NHMe | |
| 61 | 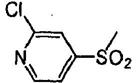 | 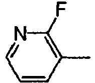 | N | CH₂NHMe | HCl |

**Table 47**

| | | | | | | |
|---|---|---|---|---|---|---|
| 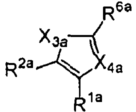 | | | | | | |

| Ex. No. | R^{1a} | R^{2a} | X₃ₐ | X₄ₐ | R^{6a} | addition salt |
|---|---|---|---|---|---|---|
| 62 | 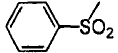 | 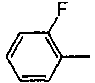 | O | CH | CH₂NHMe | HCl |
| 63 | 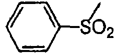 | 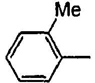 | O | CH | CH₂NHMe | HCl |
| 64 | 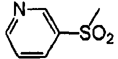 | 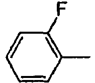 | S | CH | CH₂NHMe | 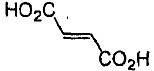 |
| 65 | 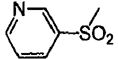 | 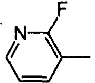 | S | CH | CH₂NHMe | 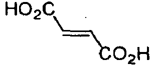 |
| 66 | 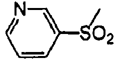 | 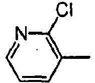 | S | CH | CH₂NHMe | 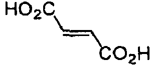 |

**Table 48**

| | | | | | |
|---|---|---|---|---|---|
| 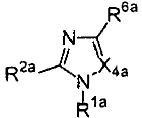 | | | | | |

| Ex. No. | R^{1a} | R^{2a} | X₄ₐ | R^{6a} | addition salt |
|---|---|---|---|---|---|
| 67 | 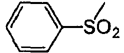 | 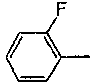 | CH | CH₂NHMe | 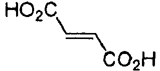 |
| 68 | 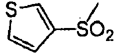 | 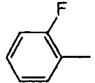 | CH | CH₂NHMe | 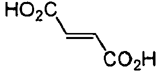 |
| 69 | 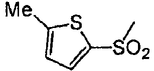 | 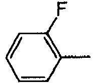 | CH | CH₂NHMe | 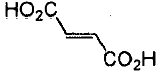 |
| 70 | 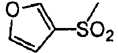 | 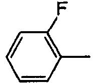 | CH | CH₂NHMe | 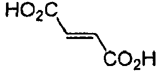 |
| 71 | 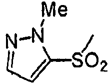 | 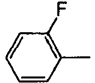 | CH | CH₂NHMe | 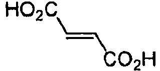 |
| 72 | 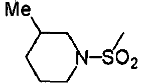 | 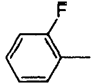 | CH | CH₂NHMe | 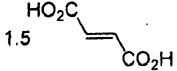 |
| 73 | 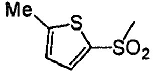 | 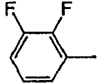 | CH | CH₂NHMe | 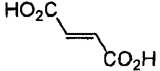 |

**Table 49**

| | | | | | |
|---|---|---|---|---|---|
| 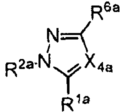 | | | | | |

| Ex. No. | R^{1a} | R^{2a} | X₄ₐ | R^{6a} | addition salt |
|---|---|---|---|---|---|
| 74 | 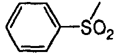 | 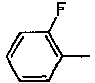 | CH | CH₂NHMe | HCl |
| 75 | 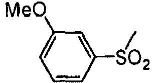 | 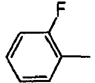 | CH | CH₂NHMe | 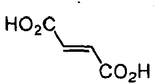 |
| 76 | 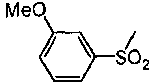 | 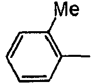 | CH | CH₂NHMe | HCl |
| 77 | 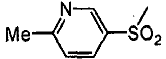 | 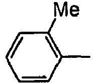 | CH | CH₂NHMe | 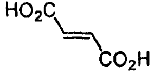 |
| 78 | 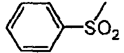 | 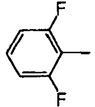 | CH | CH₂NHMe | 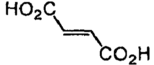 |
| 79 | 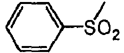 | 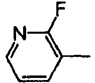 | CH | CH₂NHMe | HCl |
| 80 | 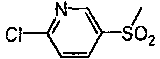 | 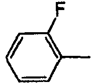 | CH | CH₂NHMe | NCl |
| 81 | 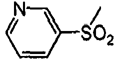 | 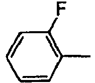 | CH | CH₂NHMe | HCl |
| 82 | 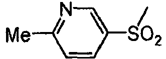 | 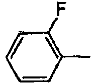 | CH | CH₂NHMe | 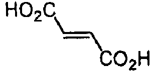 |
| 83 | 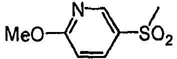 | 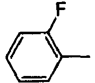 | CH | CH₂NHMe | HCl |
| 84 | 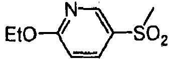 | 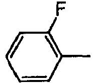 | CH | CH₂NHMe | HCl |

**Table 50**

| | | | | | |
|---|---|---|---|---|---|
| 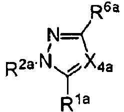 | | | | | |

| Ex. No. | R^{1a} | R^{2a} | X₄ₐ | R^{6a} | addition salt |
|---|---|---|---|---|---|
| 85 | 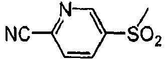 | 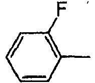 | CH | CH₂NHMe | HCl |
| 86 | 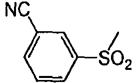 | 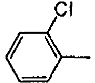 | CH | CH₂NHMe | HCl |
| 87 | 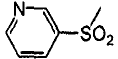 | 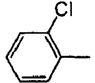 | CH | CH₂NHMe | 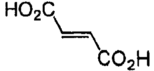 |
| 88 | 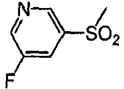 | 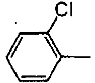 | CH | CH₂NHMe | 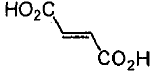 |
| 89 | 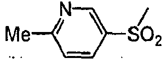 | 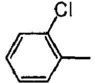 | CH | CH₂NHMe | 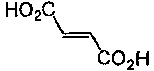 |
| 90 | 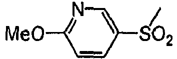 | 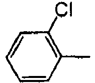 | CH | CH₂NHMe | 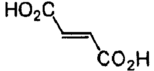 |
| 91 | 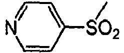 | 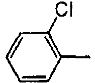 | CH | CH₂NHMe | 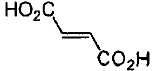 |
| 92 | 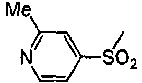 | 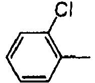 | CH | CH₂NHMe | 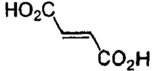 |
| 93 | 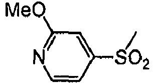 | 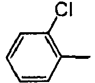 | CH | CH₂NHMe | 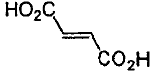 |
| 94 | 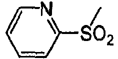 | 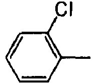 | CH | CH₂NHMe | HCl |
| 95 | 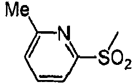 | 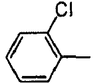 | CH | CH₂NHMe | HCl |

**Table 51**

| | | | | | |
|---|---|---|---|---|---|
| 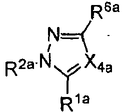 | | | | | |

| Ex. No. | R^{1a} | R^{2a} | X₄ₐ | R^{6a} | addition salt |
|---|---|---|---|---|---|
| 96 | 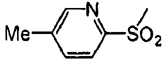 | 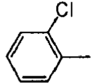 | CH | CH₂NHMe | HCl |
| 97 | 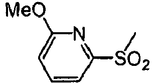 | 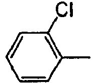 | CH | CH₂NHMe | HCl |
| 98 | 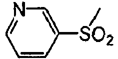 | 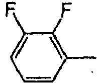 | CH | CH₂NHMe | 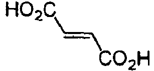 |
| 99 | 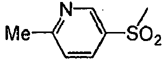 | 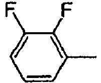 | CH | CH₂NHMe | 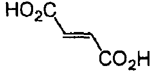 |
| 100 | 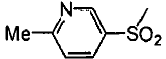 | 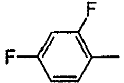 | CH | CH₂NHMe | 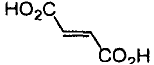 |
| 101 | 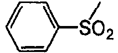 | 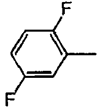 | CH | CH₂NHMe | HCl |
| 102 | 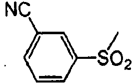 | 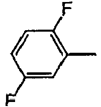 | CH | CH₂NHMe | 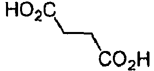 |
| 103 | 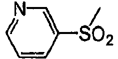 | 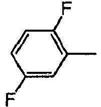 | CH | CH₂NHMe | 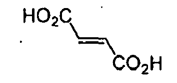 |
| 104 | 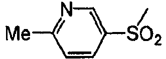 | 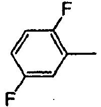 | CH | CH₂NHMe | 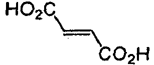 |
| 105 | 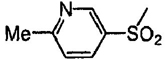 | 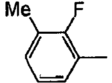 | CH | CH₂NHMe | 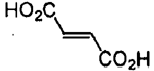 |
| 106 | 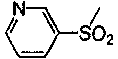 | 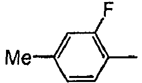 | CH | CH₂NHMe | 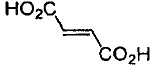 |

**Table 52**

| | | | | | |
|---|---|---|---|---|---|
| 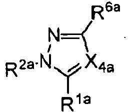 | | | | | |

| Ex. No. | R^{1a} | R^{2a} | X₄ₐ | R^{6a} | addition salt |
|---|---|---|---|---|---|
| 107 | 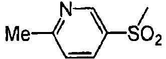 | 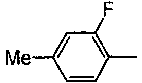 | CH | CH₂NHMe | 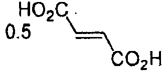 |
| 108 | 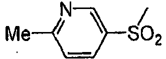 | 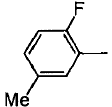 | CH | CH₂NHMe | 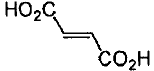 |
| 109 | 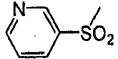 | 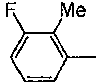 | CH | CH₂NHMe | 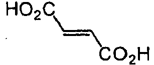 |
| 110 | 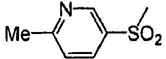 | 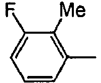 | CH | CH₂NHMe | 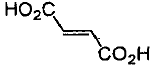 |
| 111 | 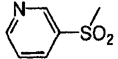 | 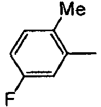 | CH | CH₂NHMe | 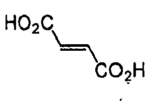 |
| 112 | 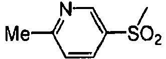 | 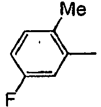 | CH | CH₂NHMe | 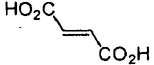 |
| 113 | 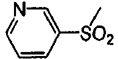 | 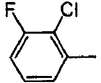 | CH | CH₂NHMe | 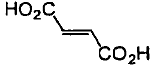 |
| 114 | 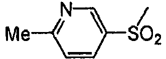 | 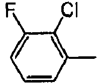 | CH | CH₂NHMe | 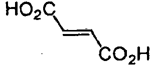 |
| 115 | 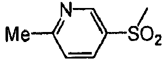 | 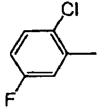 | CH | CH₂NHMe | 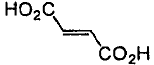 |

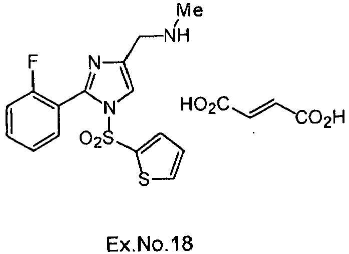

**Table 53**

| | | | | | |
|---|---|---|---|---|---|
| 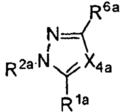 | | | | | |

| Ex. No. | R^{1a} | R^{2a} | X₄ₐ | R^{6a} | additi on salt |
|---|---|---|---|---|---|
| 116 | 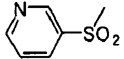 | 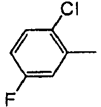 | CH | CH₂NHMe | 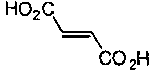 |
| 117 | 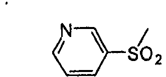 | 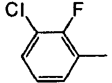 | CH | CH₂NHMe | 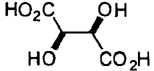 |
| 118 | 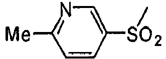 | 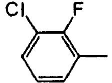 | CH | CH₂NHMe | 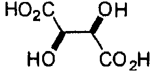 |
| 119 | 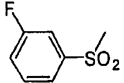 | 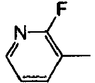 | CH | CH₂NHMe | HCl |
| 120 | 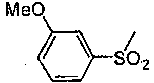 | 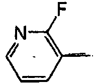 | CH | CH₂NHMe | HCl |
| 121 | 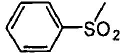 | 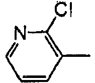 | CH | CH₂NHMe | HCl |
| 122 | 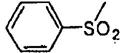 | 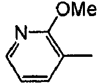 | CH | CH₂NHMe | 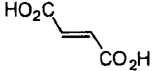 |
| 123 | 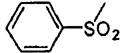 | 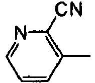 | CH | CH₂NHMe | HCl |

### Experimental Example

### Proton potassium-adenosine triphosphatase (H⁺,K⁺-ATPase) inhibitory activity test

According to the method [Biochim. Biophys. Acta, 728, 31 (1983)] of Wallmark et al., a gastric mucous membrane microsomal fraction was prepared from the stomach of swine. First, the stomach was removed, washed with tap water, and immersed in 3 mol/L brine, and the surface of the mucous membrane was wiped with a paper towel. The gastric mucous membrane was detached, chopped, and homogenized in a 0.25 mol/L saccharose solution (pH 6.8) containing 1 mmol/L EDTA and 10 mmol/L tris-hydrochloric acid using polytron (Kinematica). The obtained homogenate was centrifuged at 20,000xg for 30 min and the supernatant was centrifuged at 100,000×g for 90 min. The precipitate was suspended in 0.25 mol/L saccharose solution, the suspension was superimposed on a 0.25 mol/L saccharose solution containing 7.5% Ficoll, and centrifuged at 100,000xg for 5 hr. The fraction containing the interface between the both layers was recovered, and centrifugally washed with 0.25 mol/L saccharose solution.

The obtained microsomal fraction was used as a proton, potassium-adenosine triphosphatase standard product.

To 40 µL of a 50 mmol/L HEPES-tris buffer (5 mmol/L magnesium chloride, 10 mmol/L potassium chloride, 10 0µmol/L valinomycin, pH=6.5) containing 2.5 µg/mL (based on the protein concentration) of the enzyme standard product was added a test compound (5 µL) dissolved in a 10% aqueous dimethyl sulfoxide solution, and the mixture was incubated at 37°C for 30 min. The enzyme reaction was started by adding 5 µL of a 2 mmol/L adenosine triphosphate tris salt solution (50 mmol/L HEPES-tris buffer (5 mmol/L magnesium chloride, pH 6.5)). The enzyme reaction was carried out at 37°C for 20 min, and 15 µL of a malachite green solution (0.12% malachite green solution in sulfuric acid (2.5 mol/L), 7.5% ammonium molybdate and 11% Tween 20 were mixed at a ratio of 100:25:2) was added to quench the reaction. After allowing to stand at room temperature for 15 min, the resulting reaction product of inorganic phosphorus with malachite green was colorimetrically determined at a wavelength of 610 nm. In addition, the amount of the inorganic phosphoric acid in the reaction solution free of potassium chloride was measured in the same manner, which was subtracted from the inorganic phosphoric acid amount in the presence of potassium chloride to determine the proton, potassium-adenosine triphosphatase activity. The inhibitory rate (%) was determined from the activity value of the control and the activity values of various concentrations of the test compound, and the 50% inhibitory concentration (IC₅₀) of the proton, potassium-adenosine triphosphatase was determined. The results are shown in Table 54.

**Table 54**

| Example Compound | IC₅₀ (nM) |
|---|---|
| 2 | 32 |
| 4 | 64 |
| 13 | 46 |
| 18 | 64 |
| 19 | 88 |
| 48 | 240 |
| 56 | 240 |
| 60 | 310 |
| 65 | 250 |
| 67 | 28 |
| 79 | 190 |
| 81 | 130 |
| 86 | 84 |
| 87 | 86 |
| 89 | 110 |
| 98 | 220 |
| 99 | 76 |
| 113 | 180 |

From the results of Table 54, it is clear that compound (I) of the present invention has a superior H⁺/K⁺-ATPase inhibitory activity.

### Industrial Applicability

Compound (I) of the present invention shows a superior proton pump inhibitory effect, which is a clinically useful agent for the prophylaxis or treatment of peptic ulcer (e.g., gastric ulcer, duodenal ulcer, anastomotic ulcer, ulcer caused by non-steroidal anti-inflammatory agent, ulcer due to postoperative stress etc.), Zollinger-Ellison syndrome, gastritis, erosive esophagitis, reflux esophagitis, symptomatic gastroesophageal reflux disease (Symptomatic GERD), Barrettesophagus, functional dyspepsia, gastric cancer, stomach MALT lymphoma or hyperacidity; or as a suppressant of upper gastrointestinal hemorrhage due to peptic ulcer, acute stress ulcer, hemorrhagic gastritis or invasive stress.

## Claims

1. A compound represented by the formula (I): 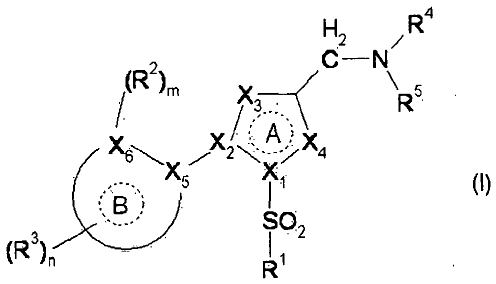 which is selected from
(1) a compound represented by the formula (Ia-20): 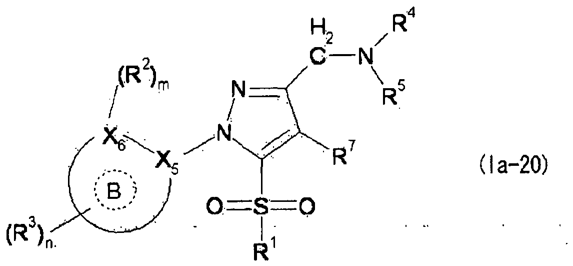 wherein
ring B is a phenyl group, a cyclopentyl group, a cyclohexyl group, a pyrrolyl group, a pyrazolyl group, a thiazolyl group, an imidazolyl group, an oxazolyl group, a thienyl group, a furyl group, a pyridyl group, a pyrrolidinyl group or a piperidyl group;
X₅ is a carbon atom or a nitrogen atom, and X₆ is a carbon atom, a nitrogen atom, an oxygen atom or a sulfur atom;
R¹ is a phenyl group, a cyclopentyl group, a cyclohexyl group, a pyrrolyl group, a pyrazolyl group, a thiazolyl group, an imidazolyl group, an oxazolyl group, a thienyl group, a furyl group, a pyridyl group, a pyrrolidinyl group or a piperidyl group, each of which is optionally substituted by 1 to 3 substituents selected from (i) a halogen atom, (ii) hydroxy, (iii) cyano, (iv) C₁₋₆ alkyl optionally substituted by 1 to 5 halogen atoms, (v) C₁₋₆ alkoxy optionally substituted by 1 to 5 halogen atoms, (vi) amino optionally mono- or di-substituted by C₁₋₆ alkyl, (vii) oxo, (viii) carbamoyl, (ix) mono-C₁₋₆ alkyl-carbamoyl, (x) di-C₁₋₆ alkyl-carbamoyl, (xi) C₁₋₆ alkylsulfonyl and (xii) C₁₋₆ alkyl-carbonylamino;
R² is a halogen atom, a cyano group, a trifluoromethyl group, a methyl group, an ethyl group, a methoxy group or an ethoxy group;
R³ is a halogen atom, a cyano group, a trifluoromethyl group, a methyl group, an ethyl group or a methoxy group;
R⁴ and R⁵ are the same or different and each is a hydrogen atom or a methyl group;
R⁷ is a hydrogen atom, a halogen atom, a methyl group or a cyano group;
m is 0 or 1, provided that when ring B is a phenyl group, a pyrrolyl group, a pyrazolyl group, a thiazolyl group, an imidazolyl group, an oxazolyl group, a thienyl group, a furyl group or a pyridyl group, then m should be 1; and
n is an integer of 0 to 3;
(2) a compound represented by the formula (Ia-1): 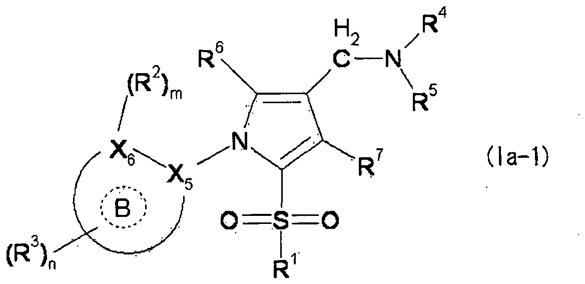 wherein
ring B is a phenyl group, a cyclopentyl group, a cyclohexyl group, a pyrrolyl group, a pyrazolyl group, a thiazolyl group, an imidazolyl group, an oxazolyl group, a thienyl group, a furyl group, a pyridyl group, a pyrrolidinyl group or a piperidyl group;
X₅ is a carbon atom or a nitrogen atom, and X₆ is a carbon atom, a nitrogen atom, an oxygen atom or a sulfur atom;
R¹ is a phenyl group, a cyclopentyl group, a cyclohexyl group, a pyrrolyl group, a pyrazolyl group, a thiazolyl group, an imidazolyl group, an oxazolyl group, a thienyl group, a furyl group, a pyridyl group, a pyrrolidinyl group or a piperidyl group, each of which is optionally substituted by 1 to 3 substituents selected from (i) a halogen atom, (ii) hydroxy, (iii) cyano, (iv) C₁₋₆ alkyl optionally substituted by 1 to 5 halogen atoms, (v) C₁₋₆ alkoxy optionally substituted by 1 to 5 halogen atoms, (vi) amino optionally mono- or di-substituted by C₁₋₆ alkyl, (vii) oxo, (viii) carbamoyl, (ix) mono-C₁₋₆ alkyl-carbamoyl, (x) di-C₁₋₆ alkyl-carbamoyl, (xi) C₁₋₆ alkylsulfonyl and (xii) C₁₋₆ alkyl-carbonylamino;
R² is a halogen atom, a cyano group, a trifluoromethyl group, a methyl group, an ethyl group, a methoxy group or an ethoxy group;
R³ is a halogen atom, a cyano group, a trifluoromethyl group, a methyl group, an ethyl group or a methoxy group;
R⁴ and R⁵ are the same or different and each is a hydrogen atom or a methyl group;
R⁶ and R⁷ are the same or different and each is a hydrogen atom, a halogen atom, a methyl group or a cyano group;
m is 0 or 1, provided that when ring B is a phenyl group, a pyrrolyl group, a pyrazolyl group, a thiazolyl group, an imidazolyl group, an oxazolyl group, a thienyl group, a furyl group or a pyridyl group, then m should be 1; and
n is an integer of 0 to 3;
(3) a compound represented by the formula (Ia-9): 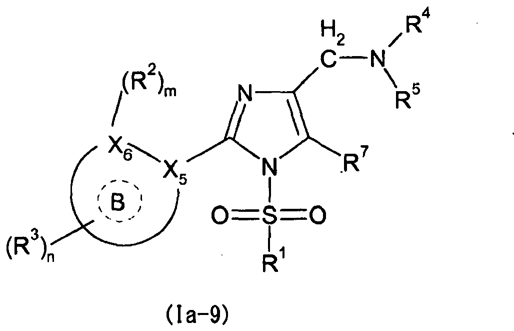 wherein
ring B is a phenyl group, a cyclopentyl group, a cyclohexyl group, a pyrrolyl group, a pyrazolyl group, a thiazolyl group, an imidazolyl group, an oxazolyl group, a thienyl group, a furyl group, a pyridyl group, a pyrrolidinyl group or a piperidyl group;
X₅ is a carbon atom or a nitrogen atom, and X₆ is a carbon atom, a nitrogen atom, an oxygen atom or a sulfur atom;
R¹ is a phenyl group, a cyclopentyl group, a cyclohexyl group, a pyrrolyl group, a pyrazolyl group, a thiazolyl group, an imidazolyl group, an oxazolyl group, a thienyl group, a furyl group, a pyridyl group, a pyrrolidinyl group or a piperidyl group, each of which is optionally substituted by 1 to 3 substituents selected from (i) a halogen atom, (ii) hydroxy, (iii) cyano, (iv) C₁₋₆ alkyl optionally substituted by 1 to 5 halogen atoms, (v) C₁₋₆ alkoxy optionally substituted by 1 to 5 halogen atoms, (vi) amino optionally mono- or di-substituted by C₁₋₆ alkyl, (vii) oxo, (viii) carbamoyl, (ix) mono-C₁₋₆ alkyl-carbamoyl, (x) di-C₁₋₆ alkyl-carbamoyl, (xi) C₁₋₆ alkylsulfonyl and (xii) C₁₋₆ alkyl-carbonylamino;
R² is a halogen atom, a cyano group, a trifluoromethyl group, a methyl group, an ethyl group, a methoxy group or an ethoxy group;
R³ is a halogen atom, a cyano group, a trifluoromethyl group, a methyl group, an ethyl group or a methoxy group;
R⁴ and R⁵ are the same or different and each is a hydrogen atom or a methyl group;
R⁷ is a hydrogen atom, a halogen atom, a methyl group or a cyano group;
m is 0 or 1, provided that when ring B is a phenyl group, a pyrrolyl group, a pyrazolyl group, a thiazolyl group, an imidazolyl group, an oxazolyl group, a thienyl group, a furyl group or a pyridyl group, then m should be 1; and
n is an integer of 0 to 3;
(4) a compound represented by the formula (Ia-30): 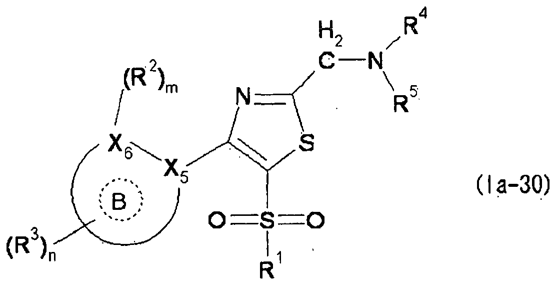 wherein
ring B is a phenyl group, a cyclopentyl group, a cyclohexyl group, a pyrrolyl group, a pyrazolyl group, a thiazolyl group, an imidazolyl group, an oxazolyl group, a thienyl group, a furyl group, a pyridyl group, a pyrrolidinyl group or a piperidyl group;
X₅ is a carbon atom or a nitrogen atom, and X₆ is a carbon atom, a nitrogen atom, an oxygen atom or a sulfur atom;
R¹ is a phenyl group, a cyclopentyl group, a cyclohexyl group, a pyrrolyl group, a pyrazolyl group, a thiazolyl group, an imidazolyl group, an oxazolyl group, a thienyl group, a furyl group, a pyridyl group, a pyrrolidinyl group or a piperidyl group, each of which is optionally substituted by 1 to 3 substituents selected from (i) a halogen atom, (ii) hydroxy, (iii) cyano, (iv) C₁₋₆ alkyl optionally substituted by 1 to 5 substituents selected from a halogen atom and a non-aromatic heterocyclic group, (v) C₁₋₆ alkoxy optionally substituted by 1 to 5 halogen atoms, (vi) amino optionally mono- or di-substituted by C₁₋₆ alkyl, (vii) oxo, (viii) carbamoyl, (ix) mono-C₁₋₆ alkyl-carbamoyl, (x) di-C₁₋₆ alkyl-carbamoyl, (xi) C₁₋₆ alkylsulfonyl, (xii) C₁₋₆ alkyl-carbonylamino, (xiii) a non-aromatic heterocyclic group optionally substituted by oxo and (xiv) a 5- or 10-membered heterocyclyl-carbonyl containing, besides carbon atoms, 1 or 2 kinds of 1 to 4 heteroatoms selected from a nitrogen atom, a sulfur atom and an oxygen atom;
R² is a halogen atom, a cyano group, a trifluoromethyl group, a methyl group, an ethyl group, a methoxy group or an ethoxy group;
R³ is a halogen atom, a cyano group, a trifluoromethyl group, a methyl group, an ethyl group or a methoxy group;
R⁴ and R⁵ are the same or different and each is a hydrogen atom or a methyl group;
m is 0 or 1, provided that when ring B is a phenyl group, a pyrrolyl group, a pyrazolyl group, a thiazolyl group, an imidazolyl group, an oxazolyl group, a thienyl group, a furyl group or a pyridyl group, then m should be 1; and
n is an integer of 0 to 3;
(5) a compound represented by the formula (Ia-31): 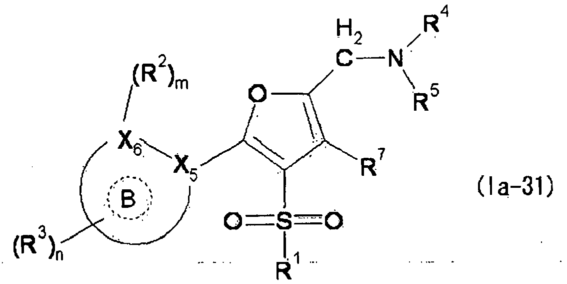 wherein
ring B is a phenyl group, a cyclopentyl group, a cyclohexyl group, a pyrrolyl group, a pyrazolyl group, a thiazolyl group, an imidazolyl group, an oxazolyl group, a thienyl group, a furyl group, a pyridyl group, a pyrrolidinyl group or a piperidyl group;
X₅ is a carbon atom or a nitrogen atom, and X₆ is a carbon atom, a nitrogen atom, an oxygen atom or a sulfur atom;
R¹ is a phenyl group, a cyclopentyl group, a cyclohexyl group, a pyrrolyl group, a pyrazolyl group, a thiazolyl group, an imidazolyl group, an oxazolyl group, a thienyl,group, a furyl group, a pyridyl group, a pyrrolidinyl group or a piperidyl group, each of which is optionally substituted by 1 to 3 substituents selected from (i) a halogen atom, (ii) hydroxy, (iii) cyano, (iv) C₁₋₆ alkyl optionally substituted by 1 to 5 halogen atoms, (v) C₁₋₆ alkoxy optionally substituted by 1 to 5 halogen atoms, (vi) amino optionally mono- or di-substituted by C₁₋₆ alkyl, (vii) oxo, (viii) carbamoyl, (ix) mono-C₁₋₆ alkyl-carbamoyl, (x) di-C₁₋₆ alkyl-carbamoyl, (xi) C₁₋₆ alkylsulfonyl and (xii) C₁₋₆ alkyl-carbonylamino;
R² is a halogen atom, a cyano group, a trifluoromethyl group, a methyl group, an ethyl group, a methoxy group or an ethoxy group;
R³ is a halogen atom, a cyano group, a trifluoromethyl group, a methyl group, an ethyl group or a methoxy group;
R⁴ and R⁵ are the same or different and each is a hydrogen atom or a methyl group;
R⁷ is a hydrogen atom, a halogen atom, a methyl group or a cyano group;
m is 0 or 1, provided that when ring B is a phenyl group, a pyrrolyl group, a pyrazolyl group, a thiazolyl group, an imidazolyl group, an oxazolyl group, a thienyl group, a furyl group or a pyridyl group, then m should be 1; and
n is an integer of 0 to 3;
(6) a compound represented by the formula (Ia-33) : 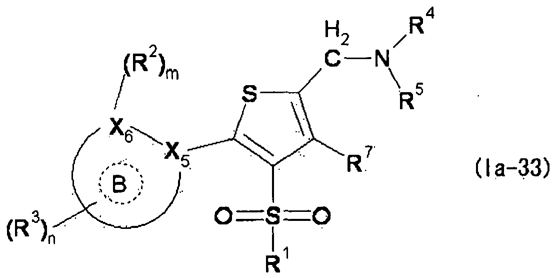 wherein
ring B is a phenyl group, a cyclopentyl group, a cyclohexyl group, a pyrrolyl group, a pyrazolyl group, a thiazolyl group, an imidazolyl group, an oxazolyl group, a thienyl group, a furyl group, a pyridyl group, a pyrrolidinyl group or a piperidyl group;
X₅ is a carbon atom or a nitrogen atom, and X₆ is a carbon atom, a nitrogen atom, an oxygen atom or a sulfur atom;
R¹ is a phenyl group, a cyclopentyl group, a cyclohexyl group, a pyrrolyl group, a pyrazolyl group, a thiazolyl group, an imidazolyl group, an oxazolyl group, a thienyl group, a furyl group, a pyridyl group, a pyrrolidinyl group or a piperidyl group, each of which is optionally substituted by 1 to 3 substituents selected from (i) a halogen atom, (ii) hydroxy, (iii) cyano, (iv) C₁₋₆ alkyl optionally substituted by 1 to 5 halogen atoms, (v) C₁₋₆ alkoxy optionally substituted by 1 to 5 halogen atoms, (vi) amino optionally mono- or di-substituted by C₁₋₆ alkyl, (vii) oxo, (viii) carbamoyl, (ix) mono-C₁₋₆ alkyl-carbamoyl, (x) di-C₁₋₆ alkyl-carbamoyl, (xi) C₁₋₆ alkylsulfonyl and (xii) C₁₋₆ alkyl-carbonylamino;
R² is a halogen atom, a cyano group, a trifluoromethyl group, a methyl group, an ethyl group, a methoxy group or an ethoxy group;
R³ is a halogen atom, a cyano group, a trifluoromethyl group, a methyl group, an ethyl group or a methoxy group;
R⁴ and R⁵ are the same or different and each is a hydrogen atom or a methyl group;
R⁷ is a hydrogen atom, a halogen atom, a methyl group or a cyano group;
m is 0 or 1, provided that when ring B is a phenyl group, a pyrrolyl group, a pyrazolyl group, a thiazolyl group, an imidazolyl group, an oxazolyl group, a thienyl group, a furyl group or a pyridyl group, then m should be 1; and
n is an integer of 0 to 3; and
(7) a compound represented by the formula (Ia-34): 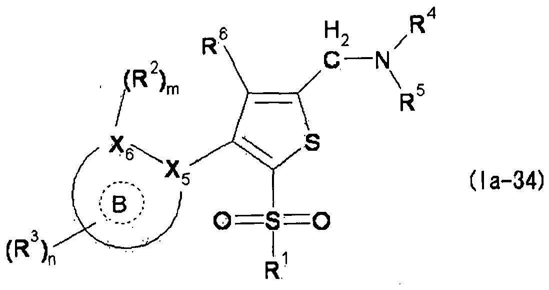 wherein
ring B is a phenyl group, a cyclopentyl group, a cyclohexyl group, a pyrrolyl group, a pyrazolyl group, a thiazolyl group, an imidazolyl group, an oxazolyl group, a thienyl group, a furyl group, a pyridyl group, a pyrrolidinyl group or a piperidyl group;
X₅ is a carbon atom or a nitrogen atom, and X₆ is a carbon atom, a nitrogen atom, an oxygen atom or a sulfur atom;
R¹ is a phenyl group, a cyclopentyl group, a cyclohexyl group, a pyrrolyl group, a pyrazolyl group, a thiazolyl group, an imidazolyl group, an oxazolyl group, a thienyl group, a furyl group, a pyridyl group, a pyrrolidinyl group or a piperidyl group, each of which is optionally substituted by 1 to 3 substituents selected from (i) a halogen atom, (ii) hydroxy, (iii) cyano, (iv) C₁₋₆ alkyl optionally substituted by 1 to 5 substituents selected from a halogen atom and hydroxy, (v) C₁₋₆ alkoxy optionally substituted by 1 to 5 halogen atoms, (vi) amino optionally mono- or di-substituted by C₁₋₆ alkyl, (vii) oxo, (viii) carbamoyl, (ix) mono-C₁₋₆ alkyl-carbamoyl, (x) di-C₁₋₆ alkyl-carbamoyl, (xi) C₁₋₆ alkylsulfonyl and (xii) C₁₋₆ alkyl-carbonylamino;
R² is a halogen atom, a cyano group, a trifluoromethyl group, a methyl group, an ethyl group, a methoxy group or an ethoxy group;
R³ is a halogen atom, a cyano group, a trifluoromethyl group, a methyl group, an ethyl group or a methoxy group;
R⁴ and R⁵ are the same or different and each is a hydrogen atom or a methyl group;
R⁶ is a hydrogen atom, a halogen atom, a methyl group or a cyano group;
m is 0 or 1, provided that when ring B is a phenyl group, a pyrrolyl group, a pyrazolyl group, a thiazolyl group, an imidazolyl group, an oxazolyl group, a thienyl group, a furyl group or a pyridyl group, then m should be 1; and
n is an integer of 0 to 3;
or a salt thereof.

2. The compound of claim 1, wherein R⁶ and R⁷ are the same or different and each is a hydrogen atom or a halogen atom.

3. The compound of claim 1, wherein R² is a halogen atom, a cyano group, a trifluoromethyl group, a methyl group or an ethyl group.

4. The compound or salt of claim 1, which is 1-[4-(2-fluoropyridin-3-yl)-5-(pyridin-3-ylsulfonyl)thiophen-2-yl]-N-methylmethanamine or a salt thereof.

5. The compound or salt of claim 1, which is 1-[5-(2-fluoropyridin-3-yl)-4-(pyridin-3-ylsulfonyl)thiophen-2-yl]-N-methylmethanamine or a salt thereof.

6. The compound or salt of claim 1, which is 1-[1-(2-fluoropyridin-3-yl)-5-(phenylsulfonyl)-1H-pyrazol-3-yl]-N-methylmethanamine or a salt thereof.

7. The compound or salt of claim 1, which is 1-[1-(2-fluorophenyl)-5-(pyridin-3-ylsulfonyl)-1H-pyrazol-3-yl]-N-methylmethanamine or a salt thereof.

8. The compound or salt of claim 1, which is 1-[1-(2-chlorophenyl)-5-(pyridin-3-ylsulfonyl)-1H-pyrazol-3-yl]-N-methylmethanamine or a salt thereof.

9. The compound or salt of claim 1, which is 1-{1-(2-chlorophenyl)-5-[(6-methylpyridin-3-yl)sulfonyl]-1H-pyrazol-3-yl}-N-methylmethanamine or a salt thereof.

10. The compound or salt of claim 1, which is 1-[1-(2,3-difluorophenyl)-5-(pyridin-3-ylsulfonyl)-1H-pyrazol-3-yl]-N-methylmethanamine or a salt thereof.

11. The compound or salt of claim 1, which is 1-{1-(2,3-difluorophenyl)-5-[(6-methylpyridin-3-yl)sulfonyl]-1H-pyrazol-3-yl}-N-methylmethanamine or a salt thereof.

12. A pharmaceutical agent comprising the compound or salt of any one of claims 1 to 11.

13. The pharmaceutical agent of claim 12, for use in the prophylaxis or treatment of peptic ulcer, Zollinger-Ellison syndrome, gastritis, reflux esophagitis, symptomatic gastroesophageal reflux disease (symptomatic GERD), Barrettesophagus, functional dyspepsia, gastric cancer, stomach MALT lymphoma, or ulcer caused by non-steroidal anti-inflammatory agent, gastric hyperacidity or ulcer due to postoperative stress; or as an inhibitor of upper gastrointestinal hemorrhage due to peptic ulcer, acute stress ulcer, hemorrhagic gastritis or invasive stress.

14. Use of the compound or salt of any one of claims 1 to 11 for the production of an agent for the prophylaxis or treatment of peptic ulcer, Zollinger-Ellison syndrome, gastritis, reflux esophagitis, symptomatic gastroesophageal reflux disease (symptomatic GERD), Barrettesophagus, functional dyspepsia, gastric cancer, stomach MALT lymphoma, or ulcer caused by non-steroidal anti-inflammatory agent, gastric hyperacidity or ulcer due to postoperative stress; or for the inhibition of upper gastrointestinal hemorrhage due to peptic ulcer, acute stress ulcer, hemorrhagic gastritis or invasive stress.

## Patentansprüche

1. Verbindung, dargestellt durch die Formel (I): 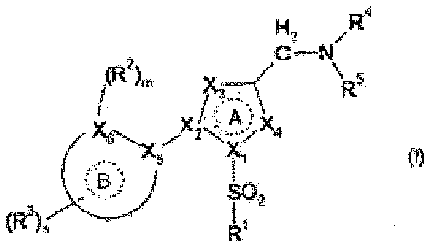 die ausgewählt ist aus
(1) einer Verbindung, die durch die Formel (Ia-20) dargestellt wird: 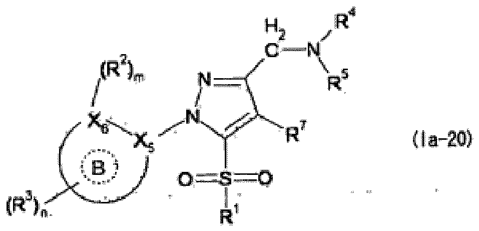 wobei
Ring B eine Phenylgruppe, Cyclopentylgruppe, Cyclohexylgruppe, Pyrrolylgruppe, Pyrazolylgruppe, Thiazolylgruppe, Imidazolylgruppe, Oxazolylgruppe, Thienylgruppe, Furylgruppe, Pyridylgruppe, Pyrrolidinylgruppe oder Piperidylgruppe ist;
X₅ ein Kohlenstoffatom oder ein Stickstoffatom ist und X₆ ein Kohlenstoffatom, ein Stickstoffatom, ein Sauerstoffatom oder ein Schwefelatom ist;
R¹ eine Phenylgruppe, Cyclopentylgruppe, Cyclohexylgruppe, Pyrrolylgruppe, Pyrazolylgruppe, Thiazolylgruppe, Imidazolylgruppe, Oxazolylgruppe, Thienylgruppe, Furylgruppe, Pyridylgruppe, Pyrrolidinylgruppe oder Piperidylgruppe ist, die jeweils gegebenenfalls mit 1 bis 3 Substituenten substituiert ist, die ausgewählt sind aus (i) einem Halogenatom, (ii) Hydroxy, (iii) Cyano, (iv) C₁₋₆-Alkyl, das gegebenenfalls mit 1 bis 5 Halogenatomen substituiert ist, (v) C₁₋₆-Alkoxy, das gegebenenfalls mit 1 bis 5 Halogenatomen substituiert ist, (vi) Amino, das gegebenenfalls mit C₁₋₆-Alkyl mono- oder disubstituiert ist, (vii) Oxo, (viii) Carbamoyl, (ix) MonO-C₁₋₆-alkylcarbamoyl, (x) Di-C₁₋₆-alkylcarbamoyl, (xi) C₁₋₆-Alkylsulfonyl und (xii) C₁₋₆-Alkylcarbonylamino;
R² ein Halogenatom, eine Cyanogruppe, eine Trifluormethylgruppe, eine Methylgruppe, eine Ethylgruppe, eine Methoxygruppe oder eine Ethoxygruppe ist;
R³ ein Halogenatom, eine Cyanogruppe, eine Trifluormethylgruppe, eine Methylgruppe, eine Ethylgruppe oder eine Methoxygruppe ist;
R⁴ und R⁵ gleich oder verschieden sind und jeweils ein Wasserstoffatom oder eine Methylgruppe sind;
R⁷ ein Wasserstoffatom, ein Halogenatom, eine Methylgruppe oder eine Cyanogruppe ist;
m = 0 oder 1 ist, mit der Maßgabe, dass dann, wenn Ring B eine Phenylgruppe, eine Pyrrolylgruppe, eine Pyrazolylgruppe, eine Thiazolylgruppe, eine Imidazolylgruppe, eine Oxazolylgruppe, eine Thienylgruppe, eine Furylgruppe oder eine Pyridylgruppe ist, m = 1 ist; und
n eine ganze Zahl von 0 bis 3 ist;
(2) einer Verbindung, die durch die Formel (Ia-1) dargestellt wird: 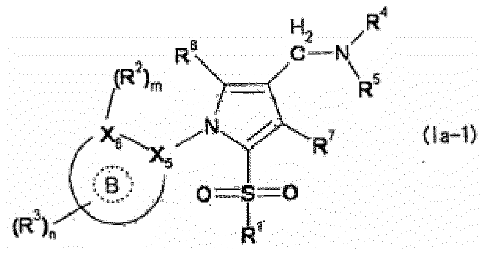 wobei
Ring B eine Phenylgruppe, Cyclopentylgruppe, Cyclohexylgruppe, Pyrrolylgruppe, Pyrazolylgruppe, Thiazolylgruppe, Imidazolylgruppe, Oxazolylgruppe, Thienylgruppe, Furylgruppe, Pyridylgruppe, Pyrrolidinylgruppe oder Piperidylgruppe ist;
X₅ ein Kohlenstoffatom oder ein Stickstoffatom ist und X₆ ein Kohlenstoffatom, ein Stickstoffatom, ein Sauerstoffatom oder ein Schwefelatom ist;
R¹ eine Phenylgruppe, Cyclopentylgruppe, Cyclohexylgruppe, Pyrrolylgruppe, Pyrazolylgruppe, Thiazolylgruppe, Imidazolylgruppe, Oxazolylgruppe, Thienylgruppe, Furylgruppe, Pyridylgruppe, Pyrrolidinylgruppe oder Piperidylgruppe ist, die jeweils gegebenenfalls mit 1 bis 3 Substituenten substituiert ist, die ausgewählt sind aus (i) einem Halogenatom, (ii) Hydroxy, (iii) Cyano, (iv) C₁₋₆-Alkyl, das gegebenenfalls mit 1 bis 5 Halogenatomen substituiert ist, (v) C₁₋₆-Alkoxy, das gegebenenfalls mit 1 bis 5 Halogenatomen substituiert ist, (vi) Amino, das gegebenenfalls mit C₁₋₆-Alkyl mono- oder disubstituiert ist, (vii) Oxo, (viii) Carbamoyl, (ix) Mono-C₁₋₆-alkylcarbamoyl, (x) Di-C₁₋₆-alkylcarbamoyl, (xi) C₁₋₆-Alkylsulfonyl und (xii) C₁₋₆-Alkylcarbonylamino;
R² ein Halogenatom, eine Cyanogruppe, eine Trifluormethylgruppe, eine Methylgruppe, eine Ethylgruppe, eine Methoxygruppe oder eine Ethoxygruppe ist;
R³ ein Halogenatom, eine Cyanogruppe, eine Trifluormethylgruppe, eine Methylgruppe, eine Ethylgruppe oder eine Methoxygruppe ist;
R⁴ und R⁵ gleich oder verschieden sind und jeweils ein Wasserstoffatom oder eine Methylgruppe sind;
R⁶ und R⁷ gleich oder verschieden sind und jeweils ein Wasserstoffatom, ein Halogenatom, eine Methylgruppe oder eine Cyanogruppe sind;
m = 0 oder 1 ist, mit der Maßgabe, dass dann, wenn Ring B eine Phenylgruppe, eine Pyrrolylgruppe, eine Pyrazolylgruppe, eine Thiazolylgruppe, eine Imidazolylgruppe, eine Oxazolylgruppe, eine Thienylgruppe, eine Furylgruppe oder eine Pyridylgruppe ist, m = 1 ist; und
n eine ganze Zahl von 0 bis 3 ist;
(3) einer Verbindung, die durch die Formel (Ia-9) dargestellt wird: 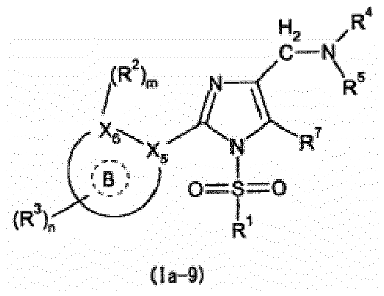 wobei
Ring B eine Phenylgruppe, Cyclopentylgruppe, Cyclohexylgruppe, Pyrrolylgruppe, Pyrazolylgruppe, Thiazolylgruppe, Imidazolylgruppe, Oxazolylgruppe, Thienylgruppe, Furylgruppe, Pyridylgruppe, Pyrrolidinylgruppe oder Piperidylgruppe ist;
Xs ein Kohlenstoffatom oder ein Stickstoffatom ist und X₆ ein Kohlenstoffatom, ein Stickstoffatom, ein Sauerstoffatom oder ein Schwefelatom ist;
R¹ eine Phenylgruppe, Cyclopentylgruppe, Cyclohexylgruppe, Pyrrolylgruppe, Pyrazolylgruppe, Thiazolylgruppe, Imidazolylgruppe, Oxazolylgruppe, Thienylgruppe, Furylgruppe, Pyridylgruppe, Pyrrolidinylgruppe oder Piperidylgruppe ist, die jeweils gegebenenfalls mit 1 bis 3 Substituenten substituiert ist, die ausgewählt sind aus (i) einem Halogenatom, (ii) Hydroxy, (iii) Cyano, (iv) C₁₋₆-Alkyl, das gegebenenfalls mit 1 bis 5 Halogenatomen substituiert ist, (v) C₁₋₆-Alkoxy, das gegebenenfalls mit 1 bis 5 Halogenatomen substituiert ist, (vi) Amino, das gegebenenfalls mit C₁₋₆-Alkyl mono- oder disubstituiert ist, (vii) Oxo, (viii) Carbamoyl, (ix) MonO-C₁₋₆-alkylcarbamoyl, (x) Di-C₁₋₆-alkylcarbamoyl, (xi) C₁₋₆-Alkylsulfonyl und (xii) C₁₋₆-Alkylcarbonylamino;
R² ein Halogenatom, eine Cyanogruppe, eine Trifluormethylgruppe, eine Methylgruppe, eine Ethylgruppe, eine Methoxygruppe oder eine Ethoxygruppe ist;
R³ ein Halogenatom, eine Cyanogruppe, eine Trifluormethylgruppe, eine Methylgruppe, eine Ethylgruppe oder eine Methoxygruppe ist;
R⁴ und R⁵ gleich oder verschieden sind und jeweils ein Wasserstoffatom oder eine Methylgruppe sind;
R⁷ ein Wasserstoffatom, ein Halogenatom, eine Methylgruppe oder eine Cyanogruppe ist;
m = 0 oder 1 ist, mit der Maßgabe, dass dann, wenn Ring B eine Phenylgruppe, eine Pyrrolylgruppe, eine Pyrazolylgruppe, eine Thiazolylgruppe, eine Imidazolylgruppe, eine Oxazolylgruppe, eine Thienylgruppe, eine Furylgruppe oder eine Pyridylgruppe ist, m = 1 ist; und
n eine ganze Zahl von 0 bis 3 ist;
(4) einer Verbindung, die durch die Formel (Ia-30) dargestellt wird: 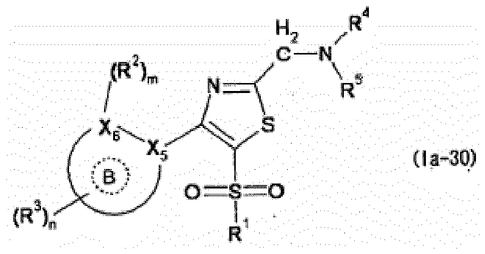 wobei
Ring B eine Phenylgruppe, Cyclopentylgruppe, Cyclohexylgruppe, Pyrrolylgruppe, Pyrazolylgruppe, Thiazolylgruppe, Imidazolylgruppe, Oxazolylgruppe, Thienylgruppe, Furylgruppe, Pyridylgruppe, Pyrrolidinylgruppe oder Piperidylgruppe ist;
X₅ ein Kohlenstoffatom oder ein Stickstoffatom ist und X₆ ein Kohlenstoffatom, ein Stickstoffatom, ein Sauerstoffatom oder ein Schwefelatom ist;
R¹ eine Phenylgruppe, Cyclopentylgruppe, Cyclohexylgruppe, Pyrrolylgruppe, Pyrazolylgruppe, Thiazolylgruppe, Imidazolylgruppe, Oxazolylgruppe, Thienylgruppe, Furylgruppe, Pyridylgruppe, Pyrrolidinylgruppe oder Piperidylgruppe ist, die jeweils gegebenenfalls mit 1 bis 3 Substituenten substituiert ist, die ausgewählt sind aus (i) einem Halogenatom, (ii) Hydroxy, (iii) Cyano, (iv) C₁₋₆-Alkyl, das gegebenenfalls mit 1 bis 5 Substituenten substituiert ist, die aus einem Halogenatom und einer nichtaromatischen heterocyclischen Gruppe ausgewählt sind, (v) C₁₋₆-Alkoxy, das gegebenenfalls mit 1 bis 5 Halogenatomen substituiert ist, (vi) Amino, das gegebenenfalls mit C₁₋₆-Alkyl mono- oder disubstituiert ist, (vii) Oxo, (viii) Carbamoyl, (ix) MonO-C₁₋₆-alkylcarbamoyl, (x) Di-C₁₋₆-alkylcarbamoyl, (xi) C₁₋₆-Alkylsulfonyl, (xii) C₁₋₆-Alkylcarbonylamino, (xiii) einer nichtaromatischen heterocyclischen Gruppe, die gegebenenfalls mit Oxo substituiert ist, und (xiv) einem fünf- oder zehngliedrigen Heterocyclylcarbonyl, das neben Kohlenstoffatomen 1 oder 2 Arten von 1 bis 4 Heteroatomen enthält, die aus einem Stickstoffatom, einem Schwefelatom und einem Sauerstoffatom ausgewählt sind;
R² ein Halogenatom, eine Cyanogruppe, eine Trifluormethylgruppe, eine Methylgruppe, eine Ethylgruppe, eine Methoxygruppe oder eine Ethoxygruppe ist;
R³ ein Halogenatom, eine Cyanogruppe, eine Trifluormethylgruppe, eine Methylgruppe, eine Ethylgruppe oder eine Methoxygruppe ist;
R⁴ und R⁵ gleich oder verschieden sind und jeweils ein Wasserstoffatom oder eine Methylgruppe sind;
m = 0 oder 1 ist, mit der Maßgabe, dass dann, wenn Ring B eine Phenylgruppe, eine Pyrrolylgruppe, eine Pyrazolylgruppe, eine Thiazolylgruppe, eine Imidazolylgruppe, eine Oxazolylgruppe, eine Thienylgruppe, eine Furylgruppe oder eine Pyridylgruppe ist, m = 1 ist; und
n eine ganze Zahl von 0 bis 3 ist;
(5) einer Verbindung, die durch die Formel (Ia-31) dargestellt wird: 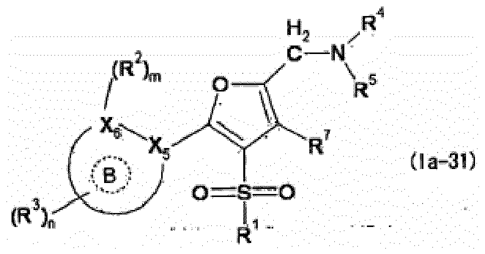 wobei
Ring B eine Phenylgruppe, Cyclopentylgruppe, Cyclohexylgruppe, Pyrrolylgruppe, Pyrazolylgruppe, Thiazolylgruppe, Imidazolylgruppe, Oxazolylgruppe, Thienylgruppe, Furylgruppe, Pyridylgruppe, Pyrrolidinylgruppe oder Piperidylgruppe ist;
X₅ ein Kohlenstoffatom oder ein Stickstoffatom ist und X₆ ein Kohlenstoffatom, ein Stickstoffatom, ein Sauerstoffatom oder ein Schwefelatom ist;
R¹ eine Phenylgruppe, Cyclopentylgruppe, Cyclohexylgruppe, Pyrrolylgruppe, Pyrazolylgruppe, Thiazolylgruppe, Imidazolylgruppe, Oxazolylgruppe, Thienylgruppe, Furylgruppe, Pyridylgruppe, Pyrrolidinylgruppe oder Piperidylgruppe ist, die jeweils gegebenenfalls mit 1 bis 3 Substituenten substituiert ist, die ausgewählt sind aus (i) einem Halogenatom, (ii) Hydroxy, (iii) Cyano, (iv) C₁₋₆-Alkyl, das gegebenenfalls mit 1 bis 5 Halogenatomen substituiert ist, (v) C₁₋₆-Alkoxy, das gegebenenfalls mit 1 bis 5 Halogenatomen substituiert ist, (vi) Amino, das gegebenenfalls mit C₁₋₆-Alkyl mono- oder disubstituiert ist, (vii) Oxo, (viii) Carbamoyl, (ix) Mono-C₁₋₆-alkylcarbamoyl, (x) Di-C₁₋₆-alkylcarbamoyl, (xi) C₁₋₆-Alkylsulfonyl und (xii) C₁₋₆-Alkylcarbonylamino;
R² ein Halogenatom, eine Cyanogruppe, eine Trifluormethylgruppe, eine Methylgruppe, eine Ethylgruppe, eine Methoxygruppe oder eine Ethoxygruppe ist;
R³ ein Halogenatom, eine Cyanogruppe, eine Trifluormethylgruppe, eine Methylgruppe, eine Ethylgruppe oder eine Methoxygruppe ist;
R⁴ und R⁵ gleich oder verschieden sind und jeweils ein Wasserstoffatom oder eine Methylgruppe sind;
R⁷ ein Wasserstoffatom, ein Halogenatom, eine Methylgruppe oder eine Cyanogruppe ist;
m = 0 oder 1 ist, mit der Maßgabe, dass dann, wenn Ring B eine Phenylgruppe, eine Pyrrolylgruppe, eine Pyrazolylgruppe, eine Thiazolylgruppe, eine Imidazolylgruppe, eine Oxazolylgruppe, eine Thienylgruppe, eine Furylgruppe oder eine Pyridylgruppe ist, m = 1 ist; und
n eine ganze Zahl von 0 bis 3 ist;
(6) einer Verbindung, die durch die Formel (Ia-33) dargestellt wird: 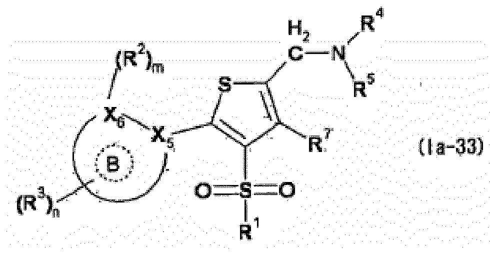 wobei
Ring B eine Phenylgruppe, Cyclopentylgruppe, Cyclohexylgruppe, Pyrrolylgruppe, Pyrazolylgruppe, Thiazolylgruppe, Imidazolylgruppe, Oxazolylgruppe, Thienylgruppe, Furylgruppe, Pyridylgruppe, Pyrrolidinylgruppe oder Piperidylgruppe ist;
X₅ ein Kohlenstoffatom oder ein Stickstoffatom ist und X₆ ein Kohlenstoffatom, ein Stickstoffatom, ein Sauerstoffatom oder ein Schwefelatom ist;
R¹ eine Phenylgruppe, Cyclopentylgruppe, Cyclohexylgruppe, Pyrrolylgruppe, Pyrazolylgruppe, Thiazolylgruppe, Imidazolylgruppe, Oxazolylgruppe, Thienylgruppe, Furylgruppe, Pyridylgruppe, Pyrrolidinylgruppe oder Piperidylgruppe ist, die jeweils gegebenenfalls mit 1 bis 3 Substituenten substituiert ist, die ausgewählt sind aus (i) einem Halogenatom, (ii) Hydroxy, (iii) Cyano, (iv) C₁₋₆-Alkyl, das gegebenenfalls mit 1 bis 5 Halogenatomen substituiert ist, (v) C₁₋₆-Alkoxy, das gegebenenfalls mit 1 bis 5 Halogenatomen substituiert ist, (vi) Amino, das gegebenenfalls mit C₁₋₆-Alkyl mono- oder disubstituiert ist, (vii) Oxo, (viii) Carbamoyl, (ix) Mono-C₁₋₆-alkylcarbamoyl, (x) Di-C₁₋₆-alkylcarbamoyl, (xi) C₁₋₆-Alkylsulfonyl und (xii) C₁₋₆-Alkylcarbonylamino;
R² ein Halogenatom, eine Cyanogruppe, eine Trifluormethylgruppe, eine Methylgruppe, eine Ethylgruppe, eine Methoxygruppe oder eine Ethoxygruppe ist;
R³ ein Halogenatom, eine Cyanogruppe, eine Trifluormethylgruppe, eine Methylgruppe, eine Ethylgruppe oder eine Methoxygruppe ist;
R⁴ und R⁵ gleich oder verschieden sind und jeweils ein Wasserstoffatom oder eine Methylgruppe sind;
R⁷ ein Wasserstoffatom, ein Halogenatom, eine Methylgruppe oder eine Cyanogruppe ist;
m = 0 oder 1 ist, mit der Maßgabe, dass dann, wenn Ring B eine Phenylgruppe, eine Pyrrolylgruppe, eine Pyrazolylgruppe, eine Thiazolylgruppe, eine Imidazolylgruppe, eine Oxazolylgruppe, eine Thienylgruppe, eine Furylgruppe oder eine Pyridylgruppe ist, m = 1 ist; und
n eine ganze Zahl von 0 bis 3 ist;
(7) einer Verbindung, die durch die Formel (Ia-34) dargestellt wird: 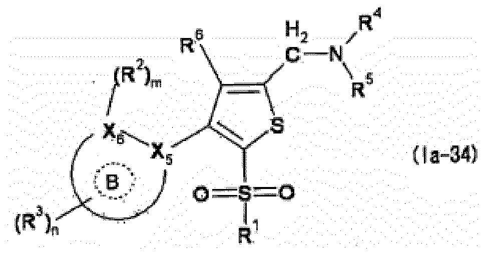 wobei
Ring B eine Phenylgruppe, Cyclopentylgruppe, Cyclohexylgruppe, Pyrrolylgruppe, Pyrazolylgruppe, Thiazolylgruppe, Imidazolylgruppe, Oxazolylgruppe, Thienylgruppe, Furylgruppe, Pyridylgruppe, Pyrrolidinylgruppe oder Piperidylgruppe ist;
X₅ ein Kohlenstoffatom oder ein Stickstoffatom ist und X₆ ein Kohlenstoffatom, ein Stickstoffatom, ein Sauerstoffatom oder ein Schwefelatom ist;
R¹ eine Phenylgruppe, Cyclopentylgruppe, Cyclohexylgruppe, Pyrrolylgruppe, Pyrazolylgruppe, Thiazolylgruppe, Imidazolylgruppe, Oxazolylgruppe, Thienylgruppe, Furylgruppe, Pyridylgruppe, Pyrrolidinylgruppe oder Piperidylgruppe ist, die jeweils gegebenenfalls mit 1 bis 3 Substituenten substituiert ist, die ausgewählt sind aus (i) einem Halogenatom, (ii) Hydroxy, (iii) Cyano, (iv) C₁₋₆-Alkyl, das gegebenenfalls mit 1 bis 5 Substituenten substituiert ist, die aus einem Halogenatom und Hydroxy ausgewählt sind, (v) C₁₋₆-Alkoxy, das gegebenenfalls mit 1 bis 5 Halogenatomen substituiert ist, (vi) Amino, das gegebenenfalls mit C₁₋₆-Alkyl mono- oder disubstituiert ist, (vii) Oxo, (viii) Carbamoyl, (ix) Mono-C₁₋₆-alkylcarbamoyl, (x) Di-C₁₋₆-alkylcarbamoyl, (xi) C₁₋₆-Alkylsulfonyl und (xii) C₁₋₆-Alkylcarbonylamino;
R² ein Halogenatom, eine Cyanogruppe, eine Trifluormethylgruppe, eine Methylgruppe, eine Ethylgruppe, eine Methoxygruppe oder eine Ethoxygruppe ist;
R³ ein Halogenatom, eine Cyanogruppe, eine Trifluormethylgruppe, eine Methylgruppe, eine Ethylgruppe oder eine Methoxygruppe ist;
R⁴ und R⁵ gleich oder verschieden sind und jeweils ein Wasserstoffatom oder eine Methylgruppe sind;
R⁶ ein Wasserstoffatom, ein Halogenatom, eine Methylgruppe oder eine Cyanogruppe ist;
m = 0 oder 1 ist, mit der Maßgabe, dass dann, wenn Ring B eine Phenylgruppe, eine Pyrrolylgruppe, eine Pyrazolylgruppe, eine Thiazolylgruppe, eine Imidazolylgruppe, eine Oxazolylgruppe, eine Thienylgruppe, eine Furylgruppe oder eine Pyridylgruppe ist, m = 1 ist; und
n eine ganze Zahl von 0 bis 3 ist;
oder ein Salz davon.

2. Verbindung gemäß Anspruch 1, wobei R⁶ und R⁷ gleich oder verschieden sind und jeweils ein Wasserstoffatom oder ein Halogenatom sind.

3. Verbindung gemäß Anspruch 1, wobei R² ein Halogenatom, eine Cyanogruppe, eine Trifluormethylgruppe, eine Methylgruppe oder eine Ethylgruppe ist.

4. Verbindung oder Salz gemäß Anspruch 1, bei der es sich um 1-[4-(2-Fluorpyridin-3-yl)-5-(pyridin-3-ylsulfonyl)thiophen-2-yl]-N-methylmethanamin oder ein Salz davon handelt.

5. Verbindung oder Salz gemäß Anspruch 1, bei der es sich um 1-[5-(2-Fluorpyridin-3-yl)-4-(pyridin-3-ylsulfonyl)thiophen-2-yl]-N-methylmethanamin oder ein Salz davon handelt.

6. Verbindung oder Salz gemäß Anspruch 1, bei der es sich um 1-[1-(2-Fluorpyridin-3-yl)-5-(phenylsulfonyl)-1H-pyrazol-3-yl]-N-methylmethanamin oder ein Salz davon handelt.

7. Verbindung oder Salz gemäß Anspruch 1, bei der es sich um 1-[1-(2-Fluorphenyl)-5-(pyridin-3-ylsulfonyl)-1H-pyrazol-3-yl]-N-methylmethanamin oder ein Salz davon handelt.

8. Verbindung oder Salz gemäß Anspruch 1, bei der es sich um 1-[1-(2-Chlorphenyl)-5-(pyridin-3-ylsulfonyl)-1H-pyrazol-3-yl]-N-methylmethanamin oder ein Salz davon handelt.

9. Verbindung oder Salz gemäß Anspruch 1, bei der es sich um 1-{1-(2-Chlorphenyl)-5-[(6-methylpyridin-3-yl)sulfonyl]-1H-pyrazol-3-yl}-N-methylmethanamin oder ein Salz davon handelt.

10. Verbindung oder Salz gemäß Anspruch 1, bei der es sich um 1-[1-(2,3-Difluorphenyl)-5-(pyridin-3-ylsulfonyl)-1H-pyrazol-3-yl]-N-methylmethanamin oder ein Salz davon handelt.

11. Verbindung oder Salz gemäß Anspruch 1, bei der es sich um 1-{1-(2,3-Difluorphenyl)-5-[(6-methylpyridin-3-yl)sulfonyl]-1H-pyrazol-3-yl}-N-methylmethanamin oder ein Salz davon handelt.

12. Pharmazeutisches Mittel, das die Verbindung oder das Salz gemäß einem der Ansprüche 1 bis 11 umfasst.

13. Pharmazeutisches Mittel gemäß Anspruch 12 zur Verwendung bei der Prophylaxe oder Behandlung von peptischem Geschwür, Zollinger-Ellison-Syndrom, Gastritis, Refluxösophagitis, symptomatischer gastroösophagealer Refluxkrankheit (symptomatischer GERD), Barrett-Ösophagus, funktioneller Dyspepsie, Magenkrebs, Magen-MALT-Lymphom oder Ulcus, der durch ein nichtsteroidales entzündungshemmendes Mittel oder Übersäuerung verursacht ist, oder Ulcus aufgrund von postoperativem Stress, oder als Inhibitor einer oberen gastrointestinalen Blutung aufgrund von peptischem Geschwür, akutem Stress-Ulcus, hämorrhagischer Gastritis oder invasivem Stress.

14. Verwendung der Verbindung oder des Salzes gemäß einem der Ansprüche 1 bis 11 zur Herstellung eines Mittels für die Prophylaxe oder Behandlung von peptischem Geschwür, Zollinger-Ellison-Syndrom, Gastritis, Refluxösophagitis, symptomatischer gastroösophagealer Refluxkrankheit (symptomatischer GERD), Barrett-Ösophagus, funktioneller Dyspepsie, Magenkrebs, Magen-MALT-Lymphom oder Ulcus, der durch ein nichtsteroidales entzündungshemmendes Mittel oder Übersäuerung verursacht ist, oder Ulcus aufgrund von postoperativem Stress, oder zur Hemmung einer oberen gastrointestinalen Blutung aufgrund von peptischem Geschwür, akutem Stress-Ulcus, hämorrhagischer Gastritis oder invasivem Stress.

## Revendications

1. Composé représenté par la formule (I) : 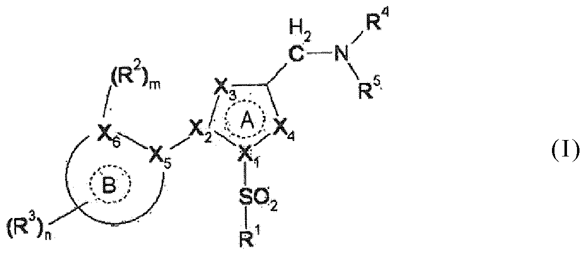 qui est choisi parmi :
1) un composé représenté par la formule (Ia-20) : 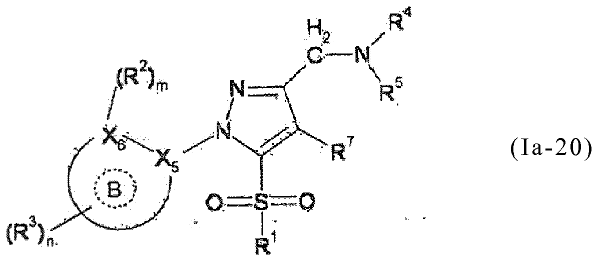 dans laquelle
- le cycle B est un groupe phényle, un groupe cyclopentyle, un groupe cyclohexyle, un groupe pyrrolyle, un groupe pyrazolyle, un groupe thiazolyle, un groupe imidazolyle, un groupe oxazolyle, un groupe thiényle, un groupe furyle, un groupe pyridyle, un groupe pyrrolidinyle, ou un groupe pipéridinyle,
- X₅ représente un atome de carbone ou un atome d'azote, et X₆ représente un atome de carbone, un atome d'azote, un atome d'oxygène ou un atome de soufre,
- R¹ représente un groupe phényle, un groupe cyclopentyle, un groupe cyclohexyle, un groupe pyrrolyle, un groupe pyrazolyle, un groupe thiazolyle, un groupe imidazolyle, un groupe oxazolyle, un groupe thiényle, un groupe furyle, un groupe pyridyle, un groupe pyrrolidinyle, ou un groupe pipéridinyle, chacun de ces groupes pouvant, en option, porter de 1 à 3 substituant(s) choisi(s) parmi les suivants :
i) un atome d'halogène,
ii) un groupe hydroxyle,
iii) un groupe cyano,
iv) un groupe alkyle en C₁₋₆, en option porteur, en tant que substituant(s), de 1 à 5 atome(s) d'halogène,
v) un groupe alcoxy en C₁₋₆, en option porteur, en tant que substituant(s), de 1 à 5 atome(s) d'halogène,
vi) un groupe amino, en option porteur, en tant que substituant(s), d'un ou deux groupe(s) alkyle en C₁₋₆,
vii) un substituant oxo,
viii) un groupe carbamyle,
ix) un groupe mono(alkyle en C₁₋₆)-carbamyle,
x) un groupe di(alkyle en C₁₋₆)-carbamyle,
xi) un groupe (alkyle en C₁₋₆)-sulfonyle,
xii) et un groupe (alkyle en C₁₋₆)-carbonyl-amino,
- R² représente un atome d'halogène, un groupe cyano, un groupe trifluorométhyle, un groupe méthyle, un groupe éthyle, un groupe méthoxy, ou un groupe éthoxy,
- R³ représente un atome d'halogène, un groupe cyano, un groupe trifluorométhyle, un groupe méthyle, un groupe éthyle, ou un groupe méthoxy,
- R⁴ et R⁵ représentent des entités identiques ou différentes, et chacun d'eux représente un atome d'hydrogène ou un groupe méthyle,
- R⁷ représente un atome d'hydrogène, un atome d'halogène, un groupe méthyle, ou un groupe cyano,
- l'indice m vaut 0 ou 1, sous réserve que cet indice m vaille 1 si le cycle B est un groupe phényle, un groupe pyrrolyle, un groupe pyrazolyle, un groupe thiazolyle, un groupe imidazolyle, un groupe oxazolyle, un groupe thiényle, un groupe furyle, ou un groupe pyridyle,
- et l'indice n est un nombre entier valant de 0 à 3 ;
2) un composé représenté par la formule (Ia-1) : 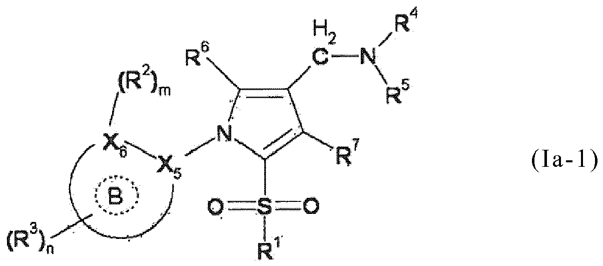 dans laquelle
- le cycle B est un groupe phényle, un groupe cyclopentyle, un groupe cyclohexyle, un groupe pyrrolyle, un groupe pyrazolyle, un groupe thiazolyle, un groupe imidazolyle, un groupe oxazolyle, un groupe thiényle, un groupe furyle, un groupe pyridyle, un groupe pyrrolidinyle, ou un groupe pipéridinyle,
- X₅ représente un atome de carbone ou un atome d'azote, et X₆ représente un atome de carbone, un atome d'azote, un atome d'oxygène ou un atome de soufre,
- R¹ représente un groupe phényle, un groupe cyclopentyle, un groupe cyclohexyle, un groupe pyrrolyle, un groupe pyrazolyle, un groupe thiazolyle, un groupe imidazolyle, un groupe oxazolyle, un groupe thiényle, un groupe furyle, un groupe pyridyle, un groupe pyrrolidinyle, ou un groupe pipéridinyle, chacun de ces groupes pouvant, en option, porter de 1 à 3 substituant(s) choisi(s) parmi les suivants :
i) un atome d'halogène,
ii) un groupe hydroxyle,
iii) un groupe cyano,
iv) un groupe alkyle en C₁₋₆, en option porteur, en tant que substituant(s), de 1 à 5 atome(s) d'halogène,
v) un groupe alcoxy en C₁₋₆, en option porteur, en tant que substituant(s), de 1 à 5 atome(s) d'halogène,
vi) un groupe amino, en option porteur, en tant que substituant(s), d'un ou deux groupe(s) alkyle en C₁₋₆,
vii) un substituant oxo,
viii) un groupe carbamyle,
ix) un groupe mono(alkyle en C₁₋₆)-carbamyle,
x) un groupe di(alkyle en C₁₋₆)-carbamyle,
xi) un groupe (alkyle en C₁₋₆)-sulfonyle,
xii) et un groupe (alkyle en C₁₋₆)-carbonyl-amino,
- R² représente un atome d'halogène, un groupe cyano, un groupe trifluorométhyle, un groupe méthyle, un groupe éthyle, un groupe méthoxy, ou un groupe éthoxy,
- R³ représente un atome d'halogène, un groupe cyano, un groupe trifluorométhyle, un groupe méthyle, un groupe éthyle, ou un groupe méthoxy,
- R⁴ et R⁵ représentent des entités identiques ou différentes, et chacun d'eux représente un atome d'hydrogène ou un groupe méthyle,
- R⁶ et R⁷ représentent des entités identiques ou différentes, et chacun d'eux représente un atome d'hydrogène, un atome d'halogène, un groupe méthyle, ou un groupe cyano,
- l'indice m vaut 0 ou 1, sous réserve que cet indice m vaille 1 si le cycle B est un groupe phényle, un groupe pyrrolyle, un groupe pyrazolyle, un groupe thiazolyle, un groupe imidazolyle, un groupe oxazolyle, un groupe thiényle, un groupe furyle, ou un groupe pyridyle,
- et l'indice n est un nombre entier valant de 0 à 3 ;
3) un composé représenté par la formule (Ia-9) : 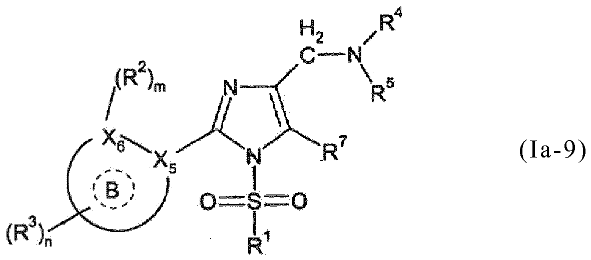 dans laquelle
- le cycle B est un groupe phényle, un groupe cyclopentyle, un groupe cyclohexyle, un groupe pyrrolyle, un groupe pyrazolyle, un groupe thiazolyle, un groupe imidazolyle, un groupe oxazolyle, un groupe thiényle, un groupe furyle, un groupe pyridyle, un groupe pyrrolidinyle, ou un groupe pipéridinyle,
- X₅ représente un atome de carbone ou un atome d'azote, et X₆ représente un atome de carbone, un atome d'azote, un atome d'oxygène ou un atome de soufre,
- R¹ représente un groupe phényle, un groupe cyclopentyle, un groupe cyclohexyle, un groupe pyrrolyle, un groupe pyrazolyle, un groupe thiazolyle, un groupe imidazolyle, un groupe oxazolyle, un groupe thiényle, un groupe furyle, un groupe pyridyle, un groupe pyrrolidinyle, ou un groupe pipéridinyle, chacun de ces groupes pouvant, en option, porter de 1 à 3 substituant(s) choisi(s) parmi les suivants :
i) un atome d'halogène,
ii) un groupe hydroxyle,
iii) un groupe cyano,
iv) un groupe alkyle en C₁₋₆, en option porteur, en tant que substituant(s), de 1 à 5 atome(s) d'halogène,
v) un groupe alcoxy en C₁₋₆, en option porteur, en tant que substituant(s), de 1 à 5 atome(s) d'halogène,
vi) un groupe amino, en option porteur, en tant que substituant(s), d'un ou deux groupe(s) alkyle en C₁₋₆,
vii) un substituant oxo,
viii) un groupe carbamyle,
ix) un groupe mono(alkyle en C₁₋₆)-carbamyle,
x) un groupe di(alkyle en C₁₋₆)-carbamyle,
xi) un groupe (alkyle en C₁₋₆)-sulfonyle,
xii) et un groupe (alkyle en C₁₋₆)-carbonyl-amino,
- R² représente un atome d'halogène, un groupe cyano, un groupe trifluorométhyle, un groupe méthyle, un groupe éthyle, un groupe méthoxy, ou un groupe éthoxy,
- R³ représente un atome d'halogène, un groupe cyano, un groupe trifluorométhyle, un groupe méthyle, un groupe éthyle, ou un groupe méthoxy,
- R⁴ et R⁵ représentent des entités identiques ou différentes, et chacun d'eux représente un atome d'hydrogène ou un groupe méthyle,
- R⁷ représente un atome d'hydrogène, un atome d'halogène, un groupe méthyle, ou un groupe cyano,
- l'indice m vaut 0 ou 1, sous réserve que cet indice m vaille 1 si le cycle B est un groupe phényle, un groupe pyrrolyle, un groupe pyrazolyle, un groupe thiazolyle, un groupe imidazolyle, un groupe oxazolyle, un groupe thiényle, un groupe furyle, ou un groupe pyridyle,
- et l'indice n est un nombre entier valant de 0 à 3 ;
4) un composé représenté par la formule (Ia-30) : 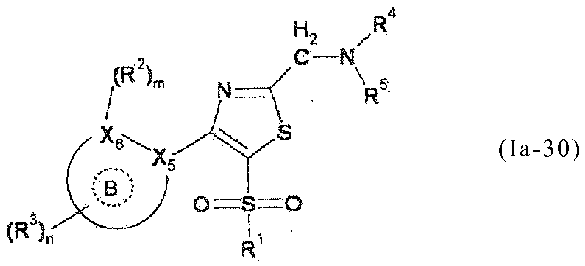 dans laquelle
- le cycle B est un groupe phényle, un groupe cyclopentyle, un groupe cyclohexyle, un groupe pyrrolyle, un groupe pyrazolyle, un groupe thiazolyle, un groupe imidazolyle, un groupe oxazolyle, un groupe thiényle, un groupe furyle, un groupe pyridyle, un groupe pyrrolidinyle, ou un groupe pipéridinyle,
- X₅ représente un atome de carbone ou un atome d'azote, et X₆ représente un atome de carbone, un atome d'azote, un atome d'oxygène ou un atome de soufre,
- R¹ représente un groupe phényle, un groupe cyclopentyle, un groupe cyclohexyle, un groupe pyrrolyle, un groupe pyrazolyle, un groupe thiazolyle, un groupe imidazolyle, un groupe oxazolyle, un groupe thiényle, un groupe furyle, un groupe pyridyle, un groupe pyrrolidinyle, ou un groupe pipéridinyle, chacun de ces groupes pouvant, en option, porter de 1 à 3 substituant(s) choisi(s) parmi les suivants :
i) un atome d'halogène,
ii) un groupe hydroxyle,
iii) un groupe cyano,
iv) un groupe alkyle en C₁₋₆, en option porteur de 1 à 5 substituant(s) choisi(s) parmi les atomes d'halogène et les groupes hétérocycliques non-aromatiques,
v) un groupe alcoxy en C₁₋₆, en option porteur, en tant que substituant(s), de 1 à 5 atome(s) d'halogène,
vi) un groupe amino, en option porteur, en tant que substituant(s), d'un ou deux groupe(s) alkyle en C₁₋₆,
vii) un substituant oxo,
viii) un groupe carbamyle,
ix) un groupe mono(alkyle en C₁₋₆)-carbamyle,
x) un groupe di(alkyle en C₁₋₆)-carbamyle,
xi) un groupe (alkyle en C₁₋₆)-sulfonyle,
xii) un groupe (alkyle en C₁₋₆)-carbonyl-amino,
xiii) un groupe hétérocyclique non aromatique, en option porteur d'un substituant oxo,
xiv) et un groupe hétérocyclyl-carbonyle, comportant 5 ou 10 chaînons de cycle et, outre des atomes de carbone, de 1 à 4 hétéroatome(s) d'un ou de deux type(s), choisi(s) parmi des atomes d'azote, de soufre et d'oxygène,
- R² représente un atome d'halogène, un groupe cyano, un groupe trifluorométhyle, un groupe méthyle, un groupe éthyle, un groupe méthoxy, ou un groupe éthoxy,
- R³ représente un atome d'halogène, un groupe cyano, un groupe trifluorométhyle, un groupe méthyle, un groupe éthyle, ou un groupe méthoxy,
- R⁴ et R⁵ représentent des entités identiques ou différentes, et chacun d'eux représente un atome d'hydrogène ou un groupe méthyle,
- l'indice m vaut 0 ou 1, sous réserve que cet indice m vaille 1 si le cycle B est un groupe phényle, un groupe pyrrolyle, un groupe pyrazolyle, un groupe thiazolyle, un groupe imidazolyle, un groupe oxazolyle, un groupe thiényle, un groupe furyle, ou un groupe pyridyle,
- et l'indice n est un nombre entier valant de 0 à 3 ;
5) un composé représenté par la formule (Ia-31) : 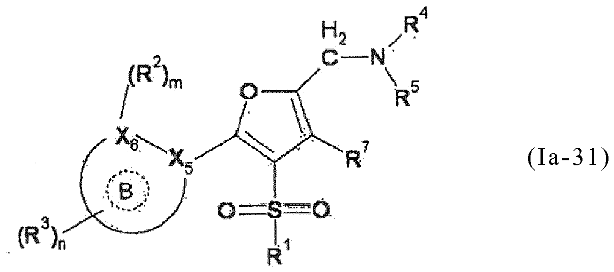 dans laquelle
- le cycle B est un groupe phényle, un groupe cyclopentyle, un groupe cyclohexyle, un groupe pyrrolyle, un groupe pyrazolyle, un groupe thiazolyle, un groupe imidazolyle, un groupe oxazolyle, un groupe thiényle, un groupe furyle, un groupe pyridyle, un groupe pyrrolidinyle, ou un groupe pipéridinyle,
- X₅ représente un atome de carbone ou un atome d'azote, et X₆ représente un atome de carbone, un atome d'azote, un atome d'oxygène ou un atome de soufre,
- R¹ représente un groupe phényle, un groupe cyclopentyle, un groupe cyclohexyle, un groupe pyrrolyle, un groupe pyrazolyle, un groupe thiazolyle, un groupe imidazolyle, un groupe oxazolyle, un groupe thiényle, un groupe furyle, un groupe pyridyle, un groupe pyrrolidinyle, ou un groupe pipéridinyle, chacun de ces groupes pouvant, en option, porter de 1 à 3 substituant(s) choisi(s) parmi les suivants :
i) un atome d'halogène,
ii) un groupe hydroxyle,
iii) un groupe cyano,
iv) un groupe alkyle en C₁₋₆, en option porteur, en tant que substituant(s), de 1 à 5 atome(s) d'halogène,
v) un groupe alcoxy en C₁₋₆, en option porteur, en tant que substituant(s), de 1 à 5 atome(s) d'halogène,
vi) un groupe amino, en option porteur, en tant que substituant(s), d'un ou deux groupe(s) alkyle en C₁₋₆,
vii) un substituant oxo,
viii) un groupe carbamyle,
ix) un groupe mono(alkyle en C₁₋₆)-carbamyle,
x) un groupe di(alkyle en C₁₋₆)-carbamyle,
xi) un groupe (alkyle en C₁₋₆)-sulfonyle,
xii) et un groupe (alkyle en C₁₋₆)-carbonyl-amino,
- R² représente un atome d'halogène, un groupe cyano, un groupe trifluorométhyle, un groupe méthyle, un groupe éthyle, un groupe méthoxy, ou un groupe éthoxy,
- R³ représente un atome d'halogène, un groupe cyano, un groupe trifluorométhyle, un groupe méthyle, un groupe éthyle, ou un groupe méthoxy,
- R⁴ et R⁵ représentent des entités identiques ou différentes, et chacun d'eux représente un atome d'hydrogène ou un groupe méthyle,
- R⁷ représente un atome d'hydrogène, un atome d'halogène, un groupe méthyle, ou un groupe cyano,
- l'indice m vaut 0 ou 1, sous réserve que cet indice m vaille 1 si le cycle B est un groupe phényle, un groupe pyrrolyle, un groupe pyrazolyle, un groupe thiazolyle, un groupe imidazolyle, un groupe oxazolyle, un groupe thiényle, un groupe furyle, ou un groupe pyridyle,
- et l'indice n est un nombre entier valant de 0 à 3 ;
6) un composé représenté par la formule (Ia-33) : 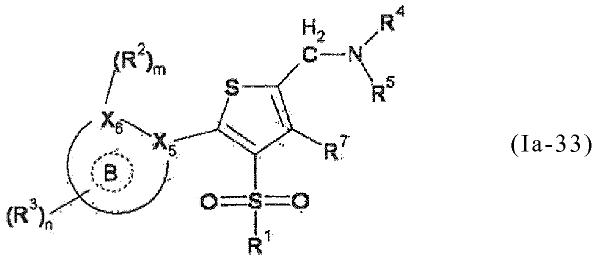 dans laquelle
- le cycle B est un groupe phényle, un groupe cyclopentyle, un groupe cyclohexyle, un groupe pyrrolyle, un groupe pyrazolyle, un groupe thiazolyle, un groupe imidazolyle, un groupe oxazolyle, un groupe thiényle, un groupe furyle, un groupe pyridyle, un groupe pyrrolidinyle, ou un groupe pipéridinyle,
- X₅ représente un atome de carbone ou un atome d'azote, et X₆ représente un atome de carbone, un atome d'azote, un atome d'oxygène ou un atome de soufre,
- R¹ représente un groupe phényle, un groupe cyclopentyle, un groupe cyclohexyle, un groupe pyrrolyle, un groupe pyrazolyle, un groupe thiazolyle, un groupe imidazolyle, un groupe oxazolyle, un groupe thiényle, un groupe furyle, un groupe pyridyle, un groupe pyrrolidinyle, ou un groupe pipéridinyle, chacun de ces groupes pouvant, en option, porter de 1 à 3 substituant(s) choisi(s) parmi les suivants :
i) un atome d'halogène,
ii) un groupe hydroxyle,
iii) un groupe cyano,
iv) un groupe alkyle en C₁₋₆, en option porteur, en tant que substituant(s), de 1 à 5 atome(s) d'halogène,
v) un groupe alcoxy en C₁₋₆, en option porteur, en tant que substituant(s), de 1 à 5 atome(s) d'halogène,
vi) un groupe amino, en option porteur, en tant que substituant(s), d'un ou deux groupe(s) alkyle en C₁₋₆,
vii) un substituant oxo,
viii) un groupe carbamyle,
ix) un groupe mono(alkyle en C₁₋₆)-carbamyle,
x) un groupe di(alkyle en C₁₋₆)-carbamyle,
xi) un groupe (alkyle en C₁₋₆)-sulfonyle,
xii) et un groupe (alkyle en C₁₋₆)-carbonyl-amino,
- R² représente un atome d'halogène, un groupe cyano, un groupe trifluorométhyle, un groupe méthyle, un groupe éthyle, un groupe méthoxy, ou un groupe éthoxy,
- R³ représente un atome d'halogène, un groupe cyano, un groupe trifluorométhyle, un groupe méthyle, un groupe éthyle, ou un groupe méthoxy,
- R⁴ et R⁵ représentent des entités identiques ou différentes, et chacun d'eux représente un atome d'hydrogène ou un groupe méthyle,
- R⁷ représente un atome d'hydrogène, un atome d'halogène, un groupe méthyle, ou un groupe cyano,
- l'indice m vaut 0 ou 1, sous réserve que cet indice m vaille 1 si le cycle B est un groupe phényle, un groupe pyrrolyle, un groupe pyrazolyle, un groupe thiazolyle, un groupe imidazolyle, un groupe oxazolyle, un groupe thiényle, un groupe furyle, ou un groupe pyridyle,
- et l'indice n est un nombre entier valant de 0 à 3 ;
7) et un composé représenté par la formule (Ia-34) : 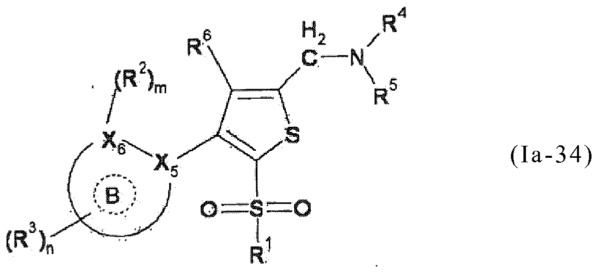 dans laquelle
- le cycle B est un groupe phényle, un groupe cyclopentyle, un groupe cyclohexyle, un groupe pyrrolyle, un groupe pyrazolyle, un groupe thiazolyle, un groupe imidazolyle, un groupe oxazolyle, un groupe thiényle, un groupe furyle, un groupe pyridyle, un groupe pyrrolidinyle, ou un groupe pipéridinyle,
- X₅ représente un atome de carbone ou un atome d'azote, et X₆ représente un atome de carbone, un atome d'azote, un atome d'oxygène ou un atome de soufre,
- R¹ représente un groupe phényle, un groupe cyclopentyle, un groupe cyclohexyle, un groupe pyrrolyle, un groupe pyrazolyle, un groupe thiazolyle, un groupe imidazolyle, un groupe oxazolyle, un groupe thiényle, un groupe furyle, un groupe pyridyle, un groupe pyrrolidinyle, ou un groupe pipéridinyle, chacun de ces groupes pouvant, en option, porter de 1 à 3 substituant(s) choisi(s) parmi les suivants :
i) un atome d'halogène,
ii) un groupe hydroxyle,
iii) un groupe cyano,
iv) un groupe alkyle en C₁₋₆, en option porteur de 1 à 5 substituant(s) choisi(s) parmi les atomes d'halogène et un groupe hydroxyle,
v) un groupe alcoxy en C₁₋₆, en option porteur de 1 à 5 substituant(s) choisi(s) parmi les atomes d'halogène et un groupe hydroxyle,
vi) un groupe amino, en option porteur, en tant que substituant(s), d'un ou deux groupe(s) alkyle en C₁₋₆,
vii) un substituant oxo,
viii) un groupe carbamyle,
ix) un groupe mono(alkyle en C₁₋₆)-carbamyle,
x) un groupe di(alkyle en C₁₋₆)-carbamyle,
xi) un groupe (alkyle en C₁₋₆)-sulfonyle,
xii) et un groupe (alkyle en C₁₋₆)-carbonyl-amino,
- R² représente un atome d'halogène, un groupe cyano, un groupe trifluorométhyle, un groupe méthyle, un groupe éthyle, un groupe méthoxy, ou un groupe éthoxy,
- R³ représente un atome d'halogène, un groupe cyano, un groupe trifluorométhyle, un groupe méthyle, un groupe éthyle, ou un groupe méthoxy,
- R⁴ et R⁵ représentent des entités identiques ou différentes, et chacun d'eux représente un atome d'hydrogène ou un groupe méthyle,
- R⁶ représente un atome d'hydrogène, un atome d'halogène, un groupe méthyle, ou un groupe cyano,
- l'indice m vaut 0 ou 1, sous réserve que cet indice m vaille 1 si le cycle B est un groupe phényle, un groupe pyrrolyle, un groupe pyrazolyle, un groupe thiazolyle, un groupe imidazolyle, un groupe oxazolyle, un groupe thiényle, un groupe furyle, ou un groupe pyridyle,
- et l'indice n est un nombre entier valant de 0 à 3 ;
ou sel d'un tel composé.

2. Composé conforme à la revendication 1, dans lequel R⁶ et R⁷ représentent des entités identiques ou différentes, et chacun d'eux représente un atome d'hydrogène ou un atome d'halogène.

3. Composé conforme à la revendication 1, dans lequel R² représente un atome d'halogène, un groupe cyano, un groupe trifluorométhyle, un groupe méthyle, ou un groupe éthyle.

4. Composé ou sel conforme à la revendication 1, qui est la 1-[4-(2-fluoro-pyridin-3-yl)-5-(pyridin-3-yl-sulfonyl)-thiophèn-2-yl]-N-méthyl-méthanamine ou un sel de celle-ci.

5. Composé ou sel conforme à la revendication 1, qui est la 1-[5-(2-fluoro-pyridin-3-yl)-4-(pyridin-3-yl-sulfonyl)-thiophèn-2-yl]-N-méthyl-méthanamine ou un sel de celle-ci.

6. Composé ou sel conforme à la revendication 1, qui est la 1-[1-(2-fluoro-pyridin-3-yl)-5-(phényl-sulfonyl)-1H-pyrazol-3-yl]-N-méthyl-méthanamine ou un sel de celle-ci.

7. Composé ou sel conforme à la revendication 1, qui est la 1-[1-(2-fluoro-phényl)-5-(pyridin-3-yl-sulfonyl)-1H-pyrazol-3-yl]-N-méthyl-méthanamine ou un sel de celle-ci.

8. Composé ou sel conforme à la revendication 1, qui est la 1-[1-(2-chloro-phényl)-5-(pyridin-3-yl-sulfonyl)-1H-pyrazol-3-yl]-N-méthyl-méthanamine ou un sel de celle-ci.

9. Composé ou sel conforme à la revendication 1, qui est la 1-[1-(2-chloro-phényl)-5-(6-méthyl-pyridin-3-yl-sulfonyl)-1H-pyrazol-3-yl]-N-méthyl-méthanamine ou un sel de celle-ci.

10. Composé ou sel conforme à la revendication 1, qui est la 1-[1-(2,3-difluoro-phényl)-5-(pyridin-3-yl-sulfonyl)-1H-pyrazol-3-yl]-N-méthyl-méthanamine ou un sel de celle-ci.

11. Composé ou sel conforme à la revendication 1, qui est la 1-[1-(2,3-difluoro-phényl)-5-(6-méthyl-pyridin-3-yl-sulfonyl)-1H-pyrazol-3-yl]-N-méthyl-méthanamine ou un sel de celle-ci.

12. Agent pharmaceutique comprenant un composé ou un sel conforme à l'une des revendications 1 à 11.

13. Agent pharmaceutique conforme à la revendication 12 pour utilisation dans la prophylaxie ou le traitement d'un ulcère peptique, du syndrome de Zollinger-Ellison, d'une gastrite, d'une oesophagite par reflux, de la maladie de reflux gastro-oesophagien symptomatique (RGO symptomatique), d'un ulcère de Barret, d'une dyspepsie fonctionnelle, d'un cancer de l'estomac, d'un lymphome MALT de l'estomac, d'un ulcère causé par un agent anti-inflammatoire non-stéroïdien ou une hyperacidité gastrique, ou d'un ulcère dû à un stress post-opératoire, ou bien en tant qu'inhibiteur d'hémorragies digestives hautes dues à un ulcère peptique, à un ulcère de stress aigu, à une gastrite hémorragique ou à un stress invasif.

14. Utilisation d'un composé ou d'un sel conforme à l'une des revendications 1 à 11 en vue de la production d'un agent destiné à la prophylaxie ou au traitement d'un ulcère peptique, du syndrome de Zollinger-Ellison, d'une gastrite, d'une oesophagite par reflux, de la maladie de reflux gastro-oesophagien symptomatique (RGO symptomatique), d'un ulcère de Barret, d'une dyspepsie fonctionnelle, d'un cancer de l'estomac, d'un lymphome MALT de l'estomac, d'un ulcère causé par un agent anti-inflammatoire non-stéroïdien ou une hyperacidité gastrique, ou d'un ulcère dû à un stress post-opératoire, ou conçu pour inhiber des hémorragies digestives hautes dues à un ulcère peptique, à un ulcère de stress aigu, à une gastrite hémorragique ou à un stress invasif.
